(19) Europäisches Patentamt / European Patent Office / Office européen des brevets

(11) EP 2 540 832 A1

(12) EUROPEAN PATENT APPLICATION

(43) Date of publication:
**02.01.2013 Bulletin 2013/01**

(51) Int Cl.:
***C12N 15/82*** *(2006.01)* ***C12N 9/12*** *(2006.01)*

(21) Application number: **12185028.3**

(22) Date of filing: **02.08.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**12.10.2006 US 851258 P**
**01.12.2006 US 868095 P**
**12.10.2006 US 851250 P**
**12.10.2006 US 851265 P**
**17.11.2006 US 859717 P**
**10.08.2006 US 836804 P**
**06.10.2006 EP 06121928**
**27.10.2006 EP 06123066**
**31.10.2006 EP 06123237**
**05.10.2006 EP 06121856**
**02.08.2006 EP 06118347**
**20.09.2006 EP 06120994**

(62) Document number(s) of the earlier application(s) in accordance with Art. 76 EPC:
**07788181.1 / 2 054 516**

(71) Applicant: **CropDesign N.V.**
**9052 Zwijnaarde (BE)**

(72) Inventor: **Sanz Molinero, Ana Isabel**
**28035 Madrid (ES)**

(74) Representative: **Hennin, Caroline Marie Odile**
**BASF SE**
**Global Intellectual Property**
**Carl-Bosch-Strasse 38**
**67056 Ludwigshafen (DE)**

Remarks:
This application was filed on 19-09-2012 as a divisional application to the application mentioned under INID code 62.

(54) **Plants transformed with a small inducible kinase having improved yield related traits and a method for making the same**

(57) The present invention relates generally to the field of molecular biology and concerns a method for enhancing various economically important characteristics in plants. More specifically, the present invention concerns a method for improving yield-related traits, such as enhanced yield and/or enhanced growth, or modified content of storage compounds in plants by modulating expression in a plant of a nucleic acid encoding a sall inducible kinase (SIK) polypeptide. The present invention also concerns plants having modulated expression of a nucleic acid encoding a SIK polypeptide, which plants have improved characterisitics relative to control plants.

EP 2 540 832 A1

## Description

The present invention relates generally to the field of molecular biology and concerns a method for improving various plant characteristics by modulating expression in a plant of a nucleic acid encoding a GRP (Growth Related Protein). The present invention also concerns plants having modulated expression of a nucleic acid encoding a GRP polypeptide, which plants have improved characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various characteristics may be improved in plants by modulating expression in a plant of a nucleic acid encoding a GRP polypeptide in a plant. The GRP polypeptide may be a small inducible kinase (SIK). The improved characteristics comprise yield related traits, such as enhanced yield and/or enhanced growth, or modified content of storage compounds.

**Background**

SIK

Plant breeders are often interested in improving specific aspects of yield depending on the crop or plant in question, and the part of that plant or crop which is of economic value. For example, for certain crops, or for certain end uses, a plant breeder may look specifically for improvements in plant biomass (weight) of one or more parts of a plant, which may include aboveground (harvestable) parts and/or (harvestable) parts below ground. This is particularly relevant where the aboveground parts or below ground parts of a plant are for consumption. For many crops, particularly cereals, it is an improvement in seed yield that is highly desirable. Increased seed yield may manifest itself in many ways, with each individual aspect of seed yield being of varying importance to a plant breeder depending on the crop or plant in question and its end use. For example, seed yield may be manifested as or result from a) an increase in seed biomass (total seed weight) which may be on an individual seed basis and/or per plant and/or per square meter; b) increased number of flowers per plant; c) increased number of (filled) seeds; d) increased seed filling rate (which is typically expressed as the ratio between the number of filled seeds divided by the total number of seeds; e) increased harvest index, which is typically expressed as a ratio of the yield of harvestable parts, such as seeds, divided by the total biomass; and f) increased thousand kernel weight (TKW), which is typically extrapolated from the number of filled seeds counted and their total weight.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

It would be of great advantage to a plant breeder to be able to be able to pick and choose the aspects of seed yield to be altered. It would be highly desirable to be able to pick off the shelf, so to speak, a gene suitable for altering a particular aspect, or component, of seed yield. For example an increase in the fill rate, combined with increased thousand kernel weight would be highly desirable for a crop such as corn. For rice and wheat a combination of increased fill rate, harvest index and increased thousand kernel weight would be highly desirable.

Published International patent Application, WO 02/074801, in the name of Genomine Inc., describes an AtSIK protein from *Arabidopsis thaliana* and a gene encoding said protein. It is mentioned that plants may be made resistant to osmotic stress by inhibiting expression of AtSIK, and that as a consequence the productivity of a plant may be increased. What is not mentioned however is which aspects of yield may be modified by inhibiting expression of AtSIK.

Summary

Surprisingly, it has now been found that modulating expression of a nucleic acid encoding a GRP polypeptide gives plants having improved characteristics relative to control plants.

In one aspect of the present invention, it has now been found that modulating expression in a plant of a SIK nucleic acid and/or a SIK polypeptide gives plants having various improved yield-related traits relative to control plants, wherein overexpression of a SIK-encoding nucleic acid in a plant gives increased number of flowers per plant relative to control plants, and wherein the reduction or substantial elimination of a SIK nucleic acid gives increased thousand kernel weight, increased harvest index and increased fill rate relative to corresponding wild type plants. The present invention also provides nucleic acid sequences and constructs useful in performing such methods.

Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1**: Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\%\ formamide$$

2) DNA-RNA or RNA-RNA hybrids:

$$Tm = 79.8 + 18.5\ (\log_{10}[Na^+]^a) + 0.58\ (\%G/C^b) + 11.8\ (\%G/C^b)^2 - 820/L^c$$

3) oligo-DNA or oligo-RNA[d] hybrids:

For <20 nucleotides: $T_m$= 2 ($I_n$)
For 20-35 nucleotides: $T_m$= 22 + 1.46 (In)

a or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions. For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are shown in Tables 2b to 2e below. Further examples of seed-specific promoters are given in Qing Qu and Takaiwa (Plant Biotechnol. J. 2, 113-125, 2004), which disclosure is incorporated by reference herein as if fully set forth.

**Table 2b:** Examples of seed-specific promoters

| **Gene source** | **Reference** |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |

(continued)

| Gene source | Reference |
|---|---|
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2c:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |

(continued)

| Gene source | Reference |
|---|---|
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2d:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2e:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus),*

*Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

## Detailed description of the invention

<u>Detailed description for the SIK polypeptide</u>

It has now been found that modulating expression in a plant of a SIK nucleic acid and/or a SIK polypeptide gives plants having various improved yield-related traits relative to control plants, wherein overexpression of a SIK-encoding nucleic acid in a plant gives increased number of flowers per plant relative to control plants, and wherein the reduction or substantial elimination of a SIK nucleic acid gives increased thousand kernel weight, increased harvest index and increased fill rate relative to corresponding wild type plants.

Therefore, the invention provides a method for improving yield-related traits in plants relative to control plants, comprising modulating expression in a plant of a SIK nucleic acid and/or a SIK polypeptide, wherein the modulated expression is overexpression of a SIK-encoding nucleic acid in a plant resulting in an increased number of flowers per plant relative to control plants, and wherein the modulated expression is a reduction or substantial elimination of a SIK nucleic acid resulting in an increased thousand kernel weight (TKW), increased harvest index (HI) and increased fill rate relative to corresponding wild type plants.

The term "modulation" as defined herein is taken to mean a change in the level of gene expression in comparison to a control plant. In the case where a SIK-encoding nucleic acid is being used to increase the number of flowers per plant, the expression level is increased, and in the case where a SIK nucleic acid is being used to increase TKW, HI or fill rate, the expression level is decreased. The original, unmodulated expression may be of any kind of expression of a structural RNA (rRNA, tRNA) or mRNA with subsequent translation.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

The various improved yield-related traits are improved by at least 5%, 6%, 7%, 8%, 9% or 10%, preferably at least 15% or 20%, more preferably 25%, 30%, 35%, 40%, 45%, 50%, 55%, 60%, 65% or 70% compared to control plants.

The improvement may be in one or more of the following: increased number of flowers per plant, increased seed filling rate, which as defined herein is the ratio between the number of filled seeds divided by the total number of seeds; increased harvest index, which as defined herein is the ratio of the yield of harvestable parts, such as seeds, divided by the total biomass; and increased thousand kernel weight (TKW), which as defined herein is derived by extrapolating the number of filled seeds counted and their total weight. Increased TKW may result from an increased seed size and/or seed weight, and may also result from an increase in embryo and/or endosperm size.

An increase in thousand kernel weight, increased harvest index and increased fill rate rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased thousand kernel weight, increased harvest index and increased fill rate relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased thousand kernel weight, increased harvest index and increased fill rate relative to control plants grown under comparable conditions. Therefore, according to the present invention, there is provided a method for increasing thousand kernel weight, increased harvest index and increased fill rate in plants grown under non-stress conditions or under mild drought conditions, which method comprises increasing expression in a plant of a nucleic acid encoding a SIK polypeptide.

Performance of the methods of the invention gives plants grown under conditions of nutrient deficiency, particularly under conditions of nitrogen deficiency, increased thousand kernel weight, increased harvest index and increased fill rate relative to control plants grown under comparable conditions. Therefore, according to the present invention, there is provided a method for increasing thousand kernel weight, increased harvest index and increased fill rate in plants grown under conditions of nutrient deficiency, which method comprises increasing expression in a plant of a nucleic acid encoding a SIK polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a SIK polypeptide as defined above.

According to one aspect of the present invention, a reduction or substantial elimination of a SIK nucleic acid results in one or more of an increased thousand kernel weight (TKW), increased harvest index (HI) and increased fill rate relative to control plants.

Reference herein to a "SIK nucleic acid" is taken to mean a polymeric form of a deoxyribonucleotide or a ribonucleotide polymer of any length, either double- or single-stranded, or analogues thereof, that has the essential characteristic of a natural ribonucleotide in that it can hybridise to SIK nucleic acid sequences in a manner similar to naturally occurring polynucleotides.

Reference herein to "a reduction or substantial elimination of a SIK nucleic acid or gene" and to an "endogenous" SIK gene is as described in the definitions section.

For the reduction or substantial elimination of expression an endogenous SIK gene in a plant, a sufficient length of substantially contiguous nucleotides of a SIK nucleic acid sequence is required. The stretch of substantially contiguous nucleotides may be derived from any SIK nucleic acid, preferably a SIK nucleic acid represented by any one of SEQ ID NO 213 to 225 or any one of the nucleic acid sequences given in Table 3 or Table 4 below. A SIK nucleic acid sequence encoding a (functional) polypeptide is not a requirement for the various methods discussed herein for the reduction or substantial elimination of expression of an endogenous SIK gene.

This reduction or substantial elimination may be achieved using routine tools and techniques, as described above. A preferred method for the reduction or substantial elimination of expression of a SIK nucleic acid is by introducing and expressing in a plant a genetic construct into which the SIK nucleic acid is cloned as an inverted repeat (in part or completely), separated by a spacer (non-coding DNA).

According to another aspect of the present invention, overexpression in a plant of a SIK-encoding nucleic acid results in an increased number of flowers per plant relative to control plants.

A preferred method for overexpression of a SIK-encoding nucleic acid is by introducing and expressing in a plant a nucleic acid encoding a SIK polypeptide as defined below.

A "SIK-encoding nucleic acid" encodes a "SIK polypeptide" or "SIK amino acid sequence" which as defined herein is taken to mean a polypeptide according to SEQ ID NO: 210 and orthologues and paralogues thereof as defined herein.

The SIK polypeptide represented by SEQ ID NO: 210 and orthologues and paralogues thereof may typically also comprise the following features.

ATP-binding region

ATP-binding region VSESLTSTSYKASFRDDFTDPKTIEAIVSRL (Motif 1) or an ATP-binding region having in increasing order of preference at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to Motif I.

Serine threonine kinase active site signature

Serine threonine kinase active site signature AMYNDFSTSNIQI with conserved D residue (Motif 2) or a serine threonine kinase active site signature having in increasing order of preference at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to Motif II.

Serine-rich domain

An N-terminal serine-rich domain.

Myristoylation sites

The orthologues and paralogues may further comprise one or more myristoylation sites that could serve to anchor the protein to the membrane.

Kinase activity

Furthermore, the SIK orthologues and paralogues will exhibit kinase activity of which can readily be determined using routine tools and techniques. Several assays are available (for example Current Protocols in Molecular Biology, Volumes 1 and 2, Ausubel et al. (1994), Current Protocols; or online such as http://www.protocol-online.org).

In brief, the kinase assay generally involves (1) bringing the kinase protein into contact with a substrate polypeptide containing the target site to be phosphorylated; (2) allowing phosphorylation of the target site in an appropriate kinase buffer under appropriate conditions; (3) separating phosphorylated products from non-phosphorylated substrate after a suitable reaction period. The presence or absence of kinase activity is determined by the presence or absence of a phosphorylated target. In addition, quantitative measurements may be performed.

Purified SIK proteins, or cell extracts containing or enriched in the SIK protein could be used as source for the kinase protein. As a substrate, small peptides are particularly well suited. The peptide must comprise one or more serine, threonine, or tyrosine residues in a phosphorylation site motif. A compilation of phosphorylation sites may be found in Biochimica et Biophysica Acta 1314, 191-225, (1996). In addition, the peptide substrates may advantageously have a net positive charge to facilitate binding to phosphocellulose filters, (allowing to separate the phosphorylated from non-phosphorylated peptides and to detect the phosphorylated peptides). If a phosphorylation site motif is not known, a general tyrosine kinase substrate may be used. For example, "Src-related peptide" (RRLIEDAEYAARG) is a substrate for many receptor and non-receptor tyrosine kinases). To determine the kinetic parameters for phosphorylation of the synthetic peptide, a range of peptide concentrations is required. For initial reactions, a peptide concentration of 0.7-1.5 mM may be used.

For each kinase enzyme, it is important to determine the optimal buffer, ionic strength, and pH for activity. A standard 5x Kinase Buffer generally contains 5 mg/ml BSA (Bovine Serum Albumin preventing kinase adsorption to the assay tube), 150 mM Tris-Cl (pH 7.5), 100 mM $MgCl_2$. Divalent cations are required for most tyrosine kinases, although some tyrosine kinases (for example, insulin-, IGF-1-, and PDGF receptor kinases) require $MnCl_2$ instead of $MgCl_2$ (or in addition to $MgCl_2$). The optimal concentrations of divalent cations must be determined empirically for each protein kinase.

A commonly used donor of the phophoryl group is radio-labelled [gamma-$^{32}$P]ATP (normally at 0.2 mM final concentra-

tion). The amount of $^{32}P$ incorporated in the peptides may be determined by measuring activity on the nitrocellulose dry pads in a scintillation counter.

Alternatively or additionally, the activity of a SIK orthologue or paralogue may be assayed by overexpression in a plant of a nucleic acid encoding a SIK polypeptide under the control of a constitutive promoter to check for increase the number of flowers per plant relative to control plants, and also by the reduction or substantial elimination of a SIK nucleic acid under the control of a constitutive promoter to check for one or more of an increase in thousand kernel weight (TKW), harvest index (HI) and fill rate in plants relative to control plants.

The invention is illustrated by transforming plants with the *Oryza sativa* sequence represented by SEQ ID NO: 209, encoding the polypeptide sequence of SEQ ID NO: 210, however performance of the invention is not restricted to these sequences. The methods of the invention may advantageously be performed using any nucleic acid encoding a SIK polypeptide as defined herein, such as any of the nucleic acid sequences given in Table 5 and Table 6. The examples of orthologues and paralogues of SEQ ID NO: 210 given in Table 5 and Table 6 were obtained from The Institute of Genetic research (TIGR). The % identity given in Table 6 is on a nucleotide level. The accession number given starting with 'TC' identifies the sequence as found through TIGR. In case of partial nucleic acids, it would be well within the capabilities of a person skilled in the art to obtain the full length sequence (or at least a sufficient length of sequence for performing the methods of the invention).

**Table 3:** Examples of putative SIK orthologues and paralogues

| # | TC | Putative function |
|---|---|---|
| 1 | Arabidopsis\|TC272322 | (Q9C9Y3) Hypothetical protein F17O14.23 |
| 2 | Barley\|TC134680 | (Q8RUD7) Similar to protein kinase AtSIK (P0485B12.21 protein) |
| 3 | Cotton\|TC30779 | (Q9C9Y3) Hypothetical protein F17O14.23 |
| 4 | Grape\|TC43027 | (Q94CI5) Protein kinase AtSIK |
| 5 | L.japonicus\|TC17767 | (Q94CI5) Protein kinase AtSIK |
| 6 | Lettuce\|TC15801 | protein kinase AtSIK |
| 7 | Maize\|TC255367 | (Q8RUD7) Similar to protein kinase AtSIK (P0485B12.21 protein) |
| 8 | Maize\|TC272965 | (Q8RUD7) Similar to protein kinase AtSIK (P0485B12.21 protein) |
| 9 | Medicago\|TC96175 | (Q9C9Y3) Hypothetical protein F17O14.23 |
| 10 | Pepper\|TC5595 | (Q94CI5) Protein kinase AtSIK |
| 11 | Potato\|TC117743 | Protein kinase domain putative |
| 12 | Rice\|TC268277 | (Q8RUD7) Similar to protein kinase AtSIK (P0485B12.21 protein) |
| 13 | S.officinarum\|TC67974 | Similar to protein kinase AtSIK (P0485B12.21 protein) |
| 14 | Sorghum\|TC103780 | (Q8RUD7) Similar to protein kinase AtSIK (P0485B12.21 protein) |
| 15 | Soybean \|TC229774 | (Q94CI5) Protein kinase AtSIK |
| 16 | Tomato\|TC158378 | 68416.m01018 protein kinase family protein contains protein kinase domain Pfam:PF00069 |
| 17 | Tomato\|TC166426 | 68416.m01018 protein kinase family protein contains protein kinase domain Pfam:PF00069 |
| 18 | Wheat\|TC257477 | (Q8RUD7) Similar to protein kinase AtSIK (P0485B12.21 protein) |

**Table 4:** Examples of putative SIK orthologues and paralogues.

| Sequence1 | Sequence2 | % Identity | Match length | p-value | Recip. best hits |
|---|---|---|---|---|---|
| Barley\|TC134680 | Arabidopsis\|TC272322 | 63 | 1030 | 6.80E-66 | * |
| Cotton\|TC30779 | Arabidopsis\|TC272322 | 71 | 1008 | 9.40E-103 | * |
| Cotton\|TC30779 | Barley\|TC134680 | 68 | 971 | 8.30E-80 | * |

(continued)

| Sequence1 | Sequence2 | % Identity | Match length | p-value | Recip. best hits |
|---|---|---|---|---|---|
| Grape\|TC43027 | Arabidopsis\|TC272322 | 60 | 619 | 4.30E-25 | * |
| Grape\|TC43027 | Cotton\|TC30779 | 80 | 245 | 5.60E-29 | * |
| Grape\|TC43027 | Maize\|TC272965 | 58 | 483 | 3.20E-13 | * |
| Grape\|TC43027 | Rice\|TC268277 | 66 | 318 | 3.50E-19 | * |
| Grape\|TC43027 | S.officinarum\|TC67974 | 63 | 372 | 2.40E-17 | * |
| Grape\|TC43027 | Soybean\|TC229774 | 77 | 276 | 4.60E-30 | * |
| Grape\|TC43027 | Tomato\|TC158378 | 69 | 420 | 9.20E-33 | * |
| Grape\|TC43027 | Tomato\|TC166426 | 75 | 241 | 1.80E-23 | |
| L.japonicus\|TC17767 | Arabidopsis\|TC272322 | 72 | 659 | 1.30E-64 | * |
| L.japonicus\|TC17767 | Barley\|TC134680 | 67 | 659 | 4.40E-53 | * |
| L.japonicus\|TC17767 | Cotton\|TC30779 | 80 | 654 | 9.20E-89 | * |
| Lettuce\|TC15801 | Arabidopsis\|TC272322 | 69 | 529 | 8.90E-44 | * |
| Lettuce\|TC15801 | Cotton\|TC30779 | 77 | 501 | 1.20E-58 | * |
| Lettuce\|TC15801 | L.japonicus\|TC17767 | 75 | 412 | 2.20E-45 | * |
| Maize\|TC255367 | Arabidopsis\|TC272322 | 62 | 528 | 5.50E-24 | * |
| Maize\|TC255367 | Barleyl\|TC134680 | 81 | 451 | 1.10E-60 | * |
| Maize\|TC255367 | Cotton\|TC30779 | 63 | 738 | 6.90E-44 | * |
| Maize\|TC255367 | L.japonicus\|TC17767 | 66 | 366 | 2.20E-24 | * |
| Maize\|TC255367 | Lettuce\|TC15801 | 65 | 455 | 4.10E-30 | * |
| Maize\|TC272965 | Barleyl\|TC134680 | 68 | 623 | 2.90E-43 | |
| Medicago\|TC96175 | Arabidopsis\|TC272322 | 67 | 1245 | 2.70E-102 | * |
| Medicago\|TC96175 | Barley\|TC134680 | 65 | 1069 | 2.50E-75 | * |
| Medicago\|TC96175 | Cotton\|TC30779 | 76 | 1041 | 2.20E-125 | * |
| Medicago\|TC96175 | L.japonicus\|TC17767 | 89 | 666 | 3.50E-114 | * |
| Medicago\|TC96175 | Lettuce\|TC15801 | 73 | 504 | 1.00E-51 | * |
| Medicago\|TC96175 | Maize\|TC255367 | 62 | 813 | 3.60E-41 | * |
| Pepper\|TC5595 | Arabidopsis\|TC272322 | 66 | 696 | 6.40E-49 | * |
| Pepper\|TC5595 | Barley\|TC134680 | 66 | 510 | 2.90E-38 | * |
| Pepper\|TC5595 | Cotton\|TC30779 | 76 | 553 | 1.60E-66 | * |
| Pepper\|TC5595 | L.japonicus\|TC17767 | 75 | 474 | 4.50E-53 | * |
| Pepper\|TC5595 | Lettuce\|TC15801 | 75 | 541 | 3.30E-60 | * |
| Pepper\|TC5595 | Maize\|TC255367 | 66 | 462 | 1.00E-29 | * |
| Pepper\|TC5595 | Medicago\|TC96175 | 67 | 800 | 1.30E-60 | * |
| Potato\|TC117743 | Arabidopsis\|TC272322 | 64 | 1082 | 8.80E-65 | * |
| Potato\|TC117743 | Barley\|TC134680 | 66 | 565 | 1.30E-39 | * |
| Potato\|TC117743 | Cotton\|TC30779 | 77 | 587 | 1.80E-70 | * |
| Potato\|TC117743 | L.japonicus\|TC17767 | 75 | 496 | 3.60E-54 | * |
| Potato\|TC117743 | Lettuce\|TC15801 | 77 | 543 | 2.30E-65 | * |

(continued)

| Sequence1 | Sequence2 | % Identity | Match length | p-value | Recip. best hits |
|---|---|---|---|---|---|
| Potato\|TC117743 | Maize\|TC255367 | 67 | 444 | 1.30E-33 | * |
| Potato\|TC117743 | Medicago\|TC96175 | 68 | 804 | 4.20E-64 | * |
| Potato\|TC117743 | Pepper\|TC5595 | 82 | 826 | 1.60E-121 | * |
| Rice\|TC268277 | Arabidopsis\|TC272322 | 63 | 1019 | 3.10E-66 | * |
| Rice\|TC268277 | Barley\|TC134680 | 78 | 1371 | 5.70E-179 | * |
| Rice\|TC268277 | Cotton\|TC30779 | 64 | 1436 | 3.40E-101 | * |
| Rice\|TC268277 | L.japonicus\|TC17767 | 70 | 656 | 1.90E-61 | * |
| Rice\|TC268277 | Maize\|TC255367 | 81 | 860 | 4.50E-121 | * |
| Rice\|TC268277 | Maize\|TC272965 | 74 | 646 | 4.00E-68 | |
| Rice\|TC268277 | Medicago\|TC96175 | 63 | 1684 | 3.00E-104 | * |
| Rice\|TC268277 | Pepper\|TC5595 | 65 | 600 | 5.50E-40 | * |
| Rice\|TC268277 | Potato\|TC117743 | 65 | 691 | 3.80E-45 | * |
| S.officinarum\|TC67974 | Arabidopsis\|TC272322 | 63 | 938 | 1.50E-59 | * |
| S.officinarum\|TC67974 | Barley\|TC134680 | 76 | 1294 | 2.60E-151 | * |
| S.officinarum\|TC67974 | Cotton\|TC30779 | 66 | 876 | 5.10E-70 | * |
| S.officinarum\|TC67974 | L.japonicus\|TC17767 | 69 | 656 | 4.70E-57 | * |
| S.officinarum\|TC67974 | Lettuce\|TC15801 | 66 | 390 | 4.70E-28 | * |
| S.officinarum\|TC67974 | Maize\|TC255367 | 96 | 367 | 2.80E-72 | |
| S.officinarum\|TC67974 | Maize\|TC272965 | 90 | 649 | 2.20E-112 | * |
| S.officinarum\|TC67974 | Medicago\|TC96175 | 64 | 1140 | 7.80E-75 | * |
| S.officinarum\|TC67974 | Pepper\|TC5595 | 66 | 471 | 1.80E-33 | * |
| S.officinarum\|TC67974 | Potato\|TC117743 | 67 | 460 | 1.00E-35 | * |
| S.officinarum\|TC67974 | Rice\|TC268277 | 82 | 1338 | 1.20E-194 | * |
| S.officinarum\|TC67974 | Sorghum\|TC103780 | 95 | 1359 | 8.70E-277 | * |
| S.officinarum\|TC67974 | Soybean\|TC229774 | 70 | 750 | 1.70E-67 | * |
| Sorghum\|TC103780 | Arabidopsis\|TC272322 | 63 | 1057 | 1.40E-65 | * |
| Sorghum\|TC103780 | Barley\|TC134680 | 75 | 1421 | 5.10E-163 | * |
| Sorghum\|TC103780 | Cotton\|TC30779 | 64 | 1333 | 8.60E-91 | * |
| Sorghum\|TC103780 | L.japonicus\|TC17767 | 69 | 656 | 6.40E-57 | * |
| Sorghum\|TC103780 | Lettuce\|TC15801 | 64 | 493 | 3.70E-30 | * |
| Sorghum\|TC103780 | Maize\|TC255367 | 92 | 948 | 1.70E-180 | * |
| Sorghum\|TC103780 | Maize\|TC272965 | 89 | 651 | 2.40E-110 | |
| Sorghum\|TC103780 | Medicago\|TC96175 | 62 | 1653 | 5.10E-96 | * |
| Sorghum\|TC103780 | Pepper\|TC5595 | 64 | 580 | 2.60E-36 | * |
| Sorghum\|TC103780 | Potato\|TC117743 | 65 | 602 | 6.50E-39 | * |
| Sorghum\|TC103780 | Rice\|TC268277 | 80 | 1852 | 1.40E-260 | * |
| Soybean\|TC229774 | Arabidopsis\|TC272322 | 72 | 757 | 1.10E-77 | * |
| Soybean\|TC229774 | Barley\|TC134680 | 69 | 756 | 5.70E-67 | * |

(continued)

| Sequence1 | Sequence2 | % Identity | Match length | p-value | Recip. best hits |
|---|---|---|---|---|---|
| Soybean\|TC229774 | Cotton\|TC30779 | 78 | 737 | 3.40E-96 | * |
| Soybean\|TC229774 | L.japonicus\|TC17767 | 90 | 495 | 4.10E-87 | * |
| Soybean\|TC229774 | Lettuce\|TC15801 | 77 | 249 | 1.90E-26 | * |
| Soybean\|TC229774 | Maize\|TC255367 | 71 | 203 | 2.00E-15 | * |
| Soybean\|TC229774 | Medicago\|TC96175 | 87 | 717 | 2.10E-118 | * |
| Soybean\|TC229774 | Pepper\|TC5595 | 76 | 308 | 1.50E-32 | * |
| Soybean\|TC229774 | Potato\|TC117743 | 76 | 332 | 1.80E-35 | * |
| Soybean\|TC229774 | Rice\|TC268277 | 71 | 744 | 4.20E-74 | * |
| Soybean\|TC229774 | Sorghum\|TC103780 | 69 | 757 | 1.30E-65 | * |
| Tomato\|TC158378 | Arabidopsis\|TC272322 | 71 | 686 | 3.10E-64 | * |
| Tomato\|TC158378 | Barley\|TC134680 | 65 | 907 | 8.80E-63 | * |
| Tomato\|TC158378 | Cotton\|TC30779 | 78 | 669 | 2.90E-86 | * |
| Tomato\|TC158378 | L.japonicus\|TC17767 | 78 | 427 | 1.70E-52 | * |
| Tomato\|TC158378 | Lettuce\|TC15801 | 81 | 181 | 3.90E-21 | |
| Tomato\|TC158378 | Maize\|TC272965 | 60 | 428 | 4.80E-14 | * |
| Tomato\|TC158378 | Medicago\|TC96175 | 68 | 908 | 4.60E-76 | * |
| Tomato\|TC158378 | Pepper\|TC5595 | 84 | 243 | 6.40E-34 | |
| Tomato\|TC158378 | Potato\|TC117743 | 96 | 264 | 8.90E-50 | * |
| Tomato\|TC158378 | Rice\|TC268277 | 66 | 897 | 1.70E-64 | * |
| Tomato\|TC158378 | S.officinarum\|TC67974 | 65 | 906 | 2.40E-60 | * |
| Tomato\|TC158378 | Soybean\|TC229774 | 74 | 700 | 4.80E-78 | * |
| Tomato\|TC166426 | Arabidopsis\|TC272322 | 70 | 675 | 1.80E-62 | |
| Tomato\|TC166426 | Barley\|TC134680 | 67 | 680 | 1.40E-54 | |
| Tomato\|TC166426 | Cotton\|TC30779 | 77 | 679 | 3.80E-85 | |
| Tomato\|TC166426 | L.japonicus\|TC17767 | 77 | 440 | 1.30E-50 | |
| Tomato\|TC166426 | Lettuce\|TC15801 | 81 | 193 | 6.50E-23 | * |
| Tomato\|TC166426 | Medicago\|TC96175 | 73 | 675 | 1.30E-69 | |
| Tomato\|TC166426 | Pepper\|TC5595 | 95 | 255 | 1.10E-46 | * |
| Tomato\|TC166426 | Potato\|TC117743 | 85 | 276 | 2.40E-39 | |
| Tomato\|TC166426 | Rice\|TC268277 | 67 | 680 | 2.70E-55 | |
| Tomato\|TC166426 | S.officinarum\|TC67974 | 67 | 680 | 4.90E-53 | |
| Tomato\|TC166426 | Sorghum\|TC103780 | 67 | 680 | 1.60E-53 | * |
| Tomato\|TC166426 | Soybean\|TC229774 | 73 | 680 | 2.30E-72 | |
| Wheat\|TC257477 | Barley\|TC134680 | 91 | 672 | 7.00E-119 | * |
| Wheat\|TC257477 | Maize\|TC272965 | 69 | 639 | 2.80E-49 | * |
| Wheat\|TC257477 | Rice\|TC268277 | 68 | 647 | 4.10E-47 | * |
| Wheat\|TC257477 | S.officinarum\|TC67974 | 67 | 648 | 4.10E-46 | * |
| Wheat\|TC257477 | Sorghum\|TC103780 | 69 | 648 | 4.20E-49 | * |

(continued)

| Sequence1 | Sequence2 | % Identity | Match length | p-value | Recip. best hits |
|---|---|---|---|---|---|
| Wheat\|TC257477 | Tomato\|TC158378 | 61 | 441 | 1.20E-18 | * |

Orthologous and paralogous SIK polypeptides may readily be found, and nucleic acids encoding such orthologues and paralogues would be useful in performing the methods of the invention.

Orthologues and paralogues may easily be found by performing a so-called reciprocal blast search. Typically this involves a first BLAST involving BLASTing a query sequence (for example, SEQ ID NO: 209 or SEQ ID NO: 210) against any sequence database, such as the publicly available NCBI database which may be found at: http://www.ncbi.nlm.nih.gov. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 209 or SEQ ID NO: 210, the second BLAST would therefore be against *Oryza* sequences). The results of the first and second BLASTs are then compared. A paralogue is identified if a high-ranking hit from the first blast is from the same species as from which the query sequence is derived, a BLAST back then ideally results in the query sequence being among the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. Preferably the score is greater than 50, more preferably greater than 100; and preferably the E-value is less than e-5, more preferably less than e-6. In the case of large families, ClustalW may be used, followed by a neighbour joining tree, to help visualize clustering of related genes and to identify orthologues and paralogues.

Orthologues and paralogues may also be identified using the BLAST procedure described below. Homologues (or homologous proteins, encompassing orthologues and paralogues) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-410) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 4, 29, 2003). Minor manual editing may be performed to optimise alignment between conserved motifs, as would be apparent to a person skilled in the art. Furthermore, instead of using full-length sequences for the identification of homologues, specific domains may be used as well. The sequence identity values, which are indicated below as a percentage were determined over the entire SIK nucleic acid or amino acid sequence using the programs mentioned above using the default parameters.

Preferably, the SIK polypeptides encoded by the SIK-encoding nucleic acids useful in the methods of the present invention have, in increasing order of preference, at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to the polypeptide of SEQ ID NO: 210.

Alternatively, the sequence identity among homologues may be determined using a specific domain. Any given domain may be identified and delineated using the databases and tools for protein identification listed above, and/or methods for the alignment of sequences for comparison. In some instances, default parameters may be adjusted to modify the stringency of the search. For example using BLAST, the statistical significance threshold (called "expect" value) for reporting matches against database sequences may be increased to show less stringent matches. In this way, short nearly exact matches may be identified.

The SIK polypeptides may be identifiable by the presence of an ATP-binding region, a serine threonine kinase active site signature, an N-terminal serine-rich domain and one or more myristoylation sites. The terms "domain" and "motif" are defined above. Specialist databases exist for the identification of domains, such as SMART (Schultz et al.

(1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp. 53-61, AAAI Press, Menlo Park; Hulo et al., Nucl. Acids. Res. 32:D134-D137, (2004)) or Pfam (Bateman et al., Nucleic Acids Research 30(1): 276-280 (2002)). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)). However, the various domains may also easily be identified upon sequence alignment of a putative SIK polypeptide with SIK polypeptides known in the art.

Also useful in the methods of the invention are nucleic acids encoding homologues of a SIK polypeptide represented by SEQ ID NO: 210 or orthologues or paralogues thereof as defined above.

Also useful in the methods of the invention are nucleic acids encoding derivatives of SEQ ID NO: 210 or nucleic acids encoding derivatives of orthologues, paralogues or homologues of SEQ ID NO: 210.

Nucleic acids encoding SIK polypeptides defined herein need not be full-length nucleic acids, since performance of the methods of the invention does not rely on the use of full length nucleic acid sequences. Examples of nucleic acids suitable for use in performing the methods of the invention include the nucleic acid sequences given in Table 3 and Table 4, but are not limited to those sequences. Nucleic acid variants may also be useful in practising the methods of the invention. Examples of such nucleic acid variants include portions of nucleic acids encoding a SIK polypeptide as defined herein, splice variants of nucleic acids encoding a SIK polypeptide as defined herein, allelic variants of nucleic acids encoding a SIK polypeptide as defined herein and variants of nucleic acids encoding a SIK polypeptide as defined herein that are obtained by gene shuffling. The terms portion, splice variant, allelic variant and gene shuffling are as described herein.

According to the present invention, there is provided a method for increasing the number of flowers per plant relative to control plants, comprising introducing and expressing in a plant a portion of any one of the nucleic acid sequences given in Table 3 or Table 4, or a portion of a nucleic acid encoding an orthologue, paralogue or homologue of any of the amino acid sequences given in Table 3 or Table 4.

Portions useful in the methods of the invention, encode a polypeptide having substantially the same biological activity as the SIK polypeptide represented by any of the amino acid sequences given in Table 3 or Table 4. Preferably, the portion is a portion of any one of the nucleic acids given in Table 3 or Table 4, or a portion of any one of the nucleic acids sequences represented by SEQ ID NO: 213 to 225. The portion is typically at least 300 consecutive nucleotides in length, preferably at least 400 consecutive nucleotides in length, more preferably at least 500 consecutive nucleotides in length, the consecutive nucleotides being of any one of the nucleic acid sequences given in Table 3 or Table 4 or any one of the nucleic acids sequences represented by SEQ ID NO: 213 to 225. Most preferably the portion is a portion of the nucleic acid of SEQ ID NO: 209. Preferably, the portion encodes an amino acid sequence comprising any one or more of an ATP-binding region, a serine threonine kinase active site signature, an N-terminal serine-rich domain, one or more myristoylation sites and kinase activity.

A portion of a nucleic acid encoding a SIK polypeptide as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the portion.

Another nucleic acid variant useful in the methods of the invention is a nucleic acid capable of hybridising, under reduced stringency conditions, preferably under stringent conditions, with a nucleic acid encoding a SIK polypeptide as defined herein, or with a portion as defined herein.

Hybridising sequences useful in the methods of the invention, encode a polypeptide having substantially the same biological activity as the SIK polypeptide represented by any of the amino acid sequences given in Table 3 or Table 4. The hybridising sequence is typically at least 300 consecutive nucleotides in length, preferably at least 400 consecutive nucleotides in length, more preferably at least 500 consecutive nucleotides in length, the consecutive nucleotides being of any one of the nucleic acid sequences given in Table 3 or Table 4. Preferably, the hybridising sequence is one that is capable of hybridising to any of the nucleic acids given in Table 3 or Table 4, or to a portion of any of these sequences, a portion being as defined above. Most preferably, the hybridising sequence is capable of hybridising to a nucleic acid as represented by SEQ ID NO: 209 or to a portion thereof. Preferably, the hybridising sequence encodes an amino acid sequence comprising any one or more of an ATP-binding region, a serine threonine kinase active site signature, an N-terminal serine-rich domain, one or more myristoylation sites and kinase activity.

According to the present invention, there is provided a method for increasing the number of flowers per plant, comprising introducing and expressing in a plant a nucleic acid capable of hybridizing to any one of the nucleic acids given in Table 35 or Table 4 or any one of the nucleic acids sequences represented by SEQ ID NO: 213 to 225, or comprising introducing and expressing in a plant a nucleic acid capable of hybridising to a nucleic acid encoding an orthologue, paralogue or homologue of any of the nucleic acid sequences given in Table 3 or Table 4 or any one of the nucleic acids sequences represented by SEQ ID NO: 213 to 225.

Another nucleic acid variant useful in the methods of the invention is a splice variant encoding a SIK polypeptide as defined hereinabove, the term "splice variant" being as defined above.

According to the present invention, there is provided a method for increasing the number of flowers per plant, comprising introducing and expressing in a plant a splice variant of any one of the nucleic acid sequences given in Table 3 or Table 4 or a splice variant of any one of the nucleic acids represented by SEQ ID NO: 213 to 225, or a splice variant of a nucleic acid encoding an orthologue, paralogue or homologue of any of the amino acid sequences given in Table 3 or Table 4 or any one of the nucleic acids sequences represented by SEQ ID NO: 213 to 225.

Preferred splice variants are splice variants of a nucleic acid represented by SEQ ID NO: 209 or a splice variant encoding an orthologue or paralogue of SEQ ID NO: 210. Preferably, the amino acid encoded by the splice variant comprises any one or more of an ATP-binding region, a serine threonine kinase active site signature, an N-terminal serine-rich domain, one or more myristoylation sites and kinase activity.

Another nucleic acid variant useful in performing the methods of the invention is an allelic variant of a nucleic acid encoding a SIK polypeptide as defined hereinabove.

According to the present invention, there is provided a method for increasing the number of flowers per plant, comprising introducing and expressing in a plant an allelic variant of any one of the nucleic acids given in Table 3 or Table 4 or of any one of the nucleic acid sequences represented by SEQ ID NO: 213 to 225, or comprising introducing and expressing in a plant an allelic variant of a nucleic acid encoding an orthologue, paralogue or homologue of any of the amino acid sequences given in Table 3 or Table 4 or of any one of the nucleic acids sequences represented by SEQ ID NO: 213 to 225.

Preferably, the allelic variant is an allelic variant of SEQ ID NO: 209 or an allelic variant of a nucleic acid encoding an orthologue or paralogue or homologue of SEQ ID NO: 210. Preferably, the amino acid encoded by the allelic variant comprises any one or more of an ATP-binding region, a serine threonine kinase active site signature, an N-terminal serine-rich domain, one or more myristoylation sites and kinase activity.

A further nucleic acid variant useful in the methods of the invention is a nucleic acid variant obtained by gene shuffling. Gene shuffling or directed evolution may also be used to generate variants of nucleic acids encoding SIK polypeptides.

According to the present invention, there is provided a method for increasing the number of flowers per plant, comprising introducing and expressing in a plant a variant of any one of the nucleic acid sequences given in Table 3 or Table 4, or of any one of the nucleic acids sequences represented by SEQ ID NO: 213 to 225, or comprising introducing and expressing in a plant a variant of a nucleic acid encoding an orthologue, paralogue or homologue of any of the amino acid sequences given in Table 3 or Table 4 or of any one of the nucleic acids sequences represented by SEQ ID NO: 213 to 225, which variant nucleic acid is obtained by gene shuffling. Preferably, the variant nucleic acid obtained by gene shuffling encodes an amino acid comprising any one or more of an ATP-binding region, a serine threonine kinase active site signature, an N-terminal serine-rich domain, one or more myristoylation sites and kinase activity.

Furthermore, nucleic acid variants may also be obtained by site-directed mutagenesis. Several methods are available to achieve site-directed mutagenesis, the most common being PCR based methods (current protocols in molecular biology. Wiley Eds.

SIK nucleic acids encoding SIK-like polypeptides may be derived from any natural or artificial source. The SIK nucleic acid may be modified from its native form in composition and/or genomic environment through deliberate human manipulation. Preferably the SIK-encoding nucleic acid is from a plant, further preferably from a monocotyledonous plant, more preferably from the family of Poaceae, most preferably the nucleic acid is from *Oryza sativa.*

Any reference herein to a SIK polypeptide is therefore taken to mean a SIK polypeptide as defined above. Any SIK nucleic acid encoding such a SIK polypeptide is suitable for use in performing the methods of the invention to increase the number of flowers per plant.

The present invention also encompasses plants or parts thereof (including seeds) obtainable by the methods according to the present invention. The plants or parts thereof comprise a nucleic acid transgene encoding a SIK polypeptide as defined above.

The invention also provides genetic constructs and vectors to facilitate introduction and/or expression of the SIK nucleic acid sequences useful in the methods according to the invention, in a plant.

Therefore, there is provided a gene construct comprising:

(i) a SIK nucleic acid as defined hereinabove or a SIK-encoding nucleic acid;
(ii) one or more control sequences operably linked to the SIK nucleic acid of (i).

A preferred construct in the case of reduction or substantial elimination of a SIK nucleic acid to give increased thousand kernel weight, increased harvest index and increased fill rate is one comprising an inverted repeat of a SIK nucleic acid, preferably capable of forming a hairpin structure, which inverted repeat is under the control of a constitutive promoter.

Constructs useful in the methods according to the present invention may be constructed using recombinant DNA technology well known to persons skilled in the art. The gene constructs may be inserted into vectors, which may be commercially available, suitable for transforming into plants and suitable for transcribing of the gene of interest in the transformed cells. The invention therefore provides use of a gene construct as defined hereinabove in the methods of the invention.

Plants are transformed with a vector comprising the sequence of interest. The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are defined above.

Advantageously, any type of promoter may be used in the methods o the present invention. The term "promoter" refers to a nucleic acid control sequence located upstream from the transcriptional start of a gene and which is involved in recognising and binding of RNA polymerase and other proteins, thereby directing transcription of an operably linked nucleic acid. The promoter may be a constitutive promoter. Alternatively, the promoter may be an inducible promoter. Additionally or alternatively, the promoter may be a tissue-specific promoter. Promoters able to initiate transcription in certain tissues only are referred to herein as "tissue-specific", similarly, promoters able to initiate transcription in certain cells only are referred to herein as "cell-specific".

Preferably, the nucleic acid sequence is operably linked to a constitutive promoter. Preferably the promoter is derived from a plant, more preferably the promoter is from a monocotyledonous plant if a monocotyledonous plant is to be transformed. Further preferably, the constitutive promoter is a GOS2 promoter that is represented by a nucleic acid sequence substantially similar to SEQ ID NO: 56 or 226. Most preferably the GOS2 promoter is as represented by SEQ ID NO: 56 or 226. It should be clear that the applicability of the present invention is not restricted to the SIK nucleic acid sequence as represented by SEQ ID NO: 209, nor is the applicability of the invention restricted to expression of a SIK nucleic acid sequence when driven by a GOS2 promoter. Examples of other constitutive promoters that may also be used to drive expression of a SIK nucleic acid sequence are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The expression of a SIK nucleic acid or SIK polypeptide may also be modulated by introducing a genetic modification, within the locus of a SIK gene. The locus of a gene as defined herein is taken to mean a genomic region, which includes the gene of interest and 10 kb up- or down stream of the coding region.

The genetic modification may be introduced, for example, by any one (or more) of the following methods: T-DNA tagging, TILLING and homologous recombination. Following introduction of the genetic modification, there follows a step of selecting for suitable modulated expression.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera Helianthus, Tagetes e.g. the species Helianthus annus [sunflower], Tagetes lucida, Tagetes erecta or Tagetes tenuifolia [Marigold], Brassicaceae such as the genera Brassica, Arabadopsis e.g. the species Brassica napus, Brassica rapa ssp. [canola, oilseed rape, turnip rape] or Arabidopsis thaliana. Fabaceae such as the genera Glycine e.g. the species Glycine max, Soja hispida or Soja max [soybean]. Linaceae such as the genera Linum e.g. the species Linum usitatissimum, [flax, linseed]; Poaceae such as the genera Hordeum, Secale, Avena, Sorghum, Oryza, Zea, Triticum e.g. the species Hordeum vulgare [barley]; Secale cereale [rye], Avena sativa, Avena fatua, Avena byzantina, Avena fatua var. sativa, Avena hybrida [oat], Sorghum bicolor [Sorghum, millet], Oryza sativa, Oryza latifolia [rice], Zea mays [corn, maize] Triticum aestivum, Triticum durum, Triticum turgidum, Triticum hybernum, Triticum macha, Triticum sativum or Triticum vulgare [wheat, bread wheat, common wheat]; Solanaceae such as the genera Solanum, Lycopersicon e.g. the species Solanum tuberosum [potato], Lycopersicon esculentum, Lycopersicon lycopersicum., Lycopersicon pyriforme, Solanum integrifolium or Solanum lycopersicum [tomato].

The present invention also encompasses plants obtainable by the methods according to the present invention. The present invention therefore provides plants, plant parts or plant cells thereof obtainable by the method according to the present invention, which plants or parts or cells thereof comprise a SIK nucleic acid transgene (which may encode a SIK polypeptide as defined above).

The invention furthermore provides a method for the production of transgenic plants having the various improved yield-related traits mentioned above, comprising introduction and expression in a plant SIK nucleic acid as defined.

Host plants for the nucleic acids or the vector used in the method according to the invention, the expression cassette or construct or vector are, in principle, advantageously all plants, which are capable of synthesizing the polypeptides used in the inventive method.

More specifically, the present invention provides a method for the production of transgenic plants having various improved yield-related traits relative to control plants, which method comprises:

(i) introducing and expressing a SIK nucleic acid in a plant cell; and
(ii) cultivating the plant cell under conditions promoting plant growth and development;
(iii) obtaining plants having increased number of flowers per plant.

Also provided is a method for the production of transgenic plants having various improved yield-related traits relative to control plants, which method comprises:

(i) introducing into a plant cell a construct for downregulating SIK gene expression; and
(ii) cultivating the plant cell under conditions promoting plant growth and development;
(iii) obtaining plants having one or more of increased thousand kernel weight, incraesd harvest index and increased fill rate.

The nucleic acid may be introduced directly into a plant cell or into the plant itself (including introduction into a tissue, organ or any other part of a plant). According to a preferred feature of the present invention, the nucleic acid is preferably introduced into a plant by transformation. Further preferably the construct for downregulating SIK gene expression and introduced into the plant cell or plant comprise an inverted repeat of the SIK nucleic acid or a part thereof.

Generally after transformation, plant cells or cell groupings are selected for the presence of one or more markers which are encoded by plant-expressible genes co-transferred with the gene of interest, following which the transformed material is regenerated into a whole plant. As mentioned Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (Carthamus tinctorius) or cocoa beans, for example by bathing scarred leaves or leaf segments in an agrobacterial solution and subsequently growing them in suitable media.

The genetically modified plant cells can be regenerated via all methods with which the skilled worker is familiar. Suitable methods can be found in the abovementioned publications by S.D. Kung and R. Wu, Potrykus or Höfgen and Willmitzer.

To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated SIK nucleic acid as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

The present invention also encompasses use of SIK nucleic acids and SIK polypeptides in improving various yield-related traits as mentioned above.

Nucleic acids encoding SIK polypeptides may find use in breeding programmes in which a DNA marker is identified which may be genetically linked to a SIK gene. The nucleic acids/genes may be used to define a molecular marker. This DNA marker may then be used in breeding programmes to select plants having increased yield as defined hereinabove in the methods of the invention.

Allelic variants of a SIK nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

A SIK nucleic acid may also be used as probes for genetically and physically mapping the genes that they are a part of, and as markers for traits linked to those genes. Such information may be useful in plant breeding in order to develop lines with desired phenotypes. Such use of SIK nucleic acids requires only a nucleic acid sequence of at least 15 nucleotides in length. The SIK nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SIK nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the SIK nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (Plant Mol. Biol. Reporter 4: 37-41, 1986). Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having altered yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

Description of figures

The present invention will now be described with reference to the following figures in which:

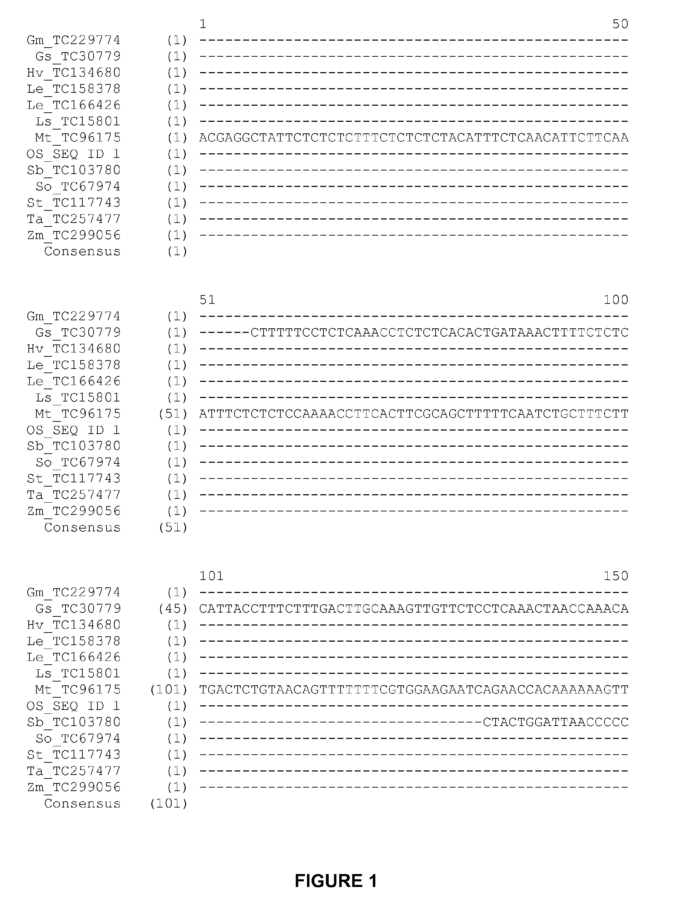

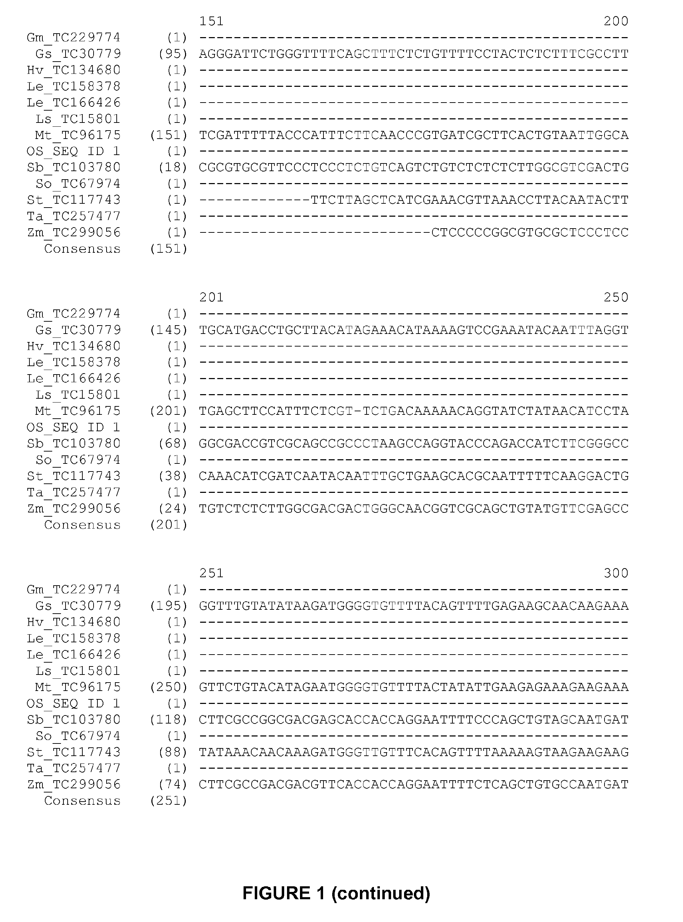

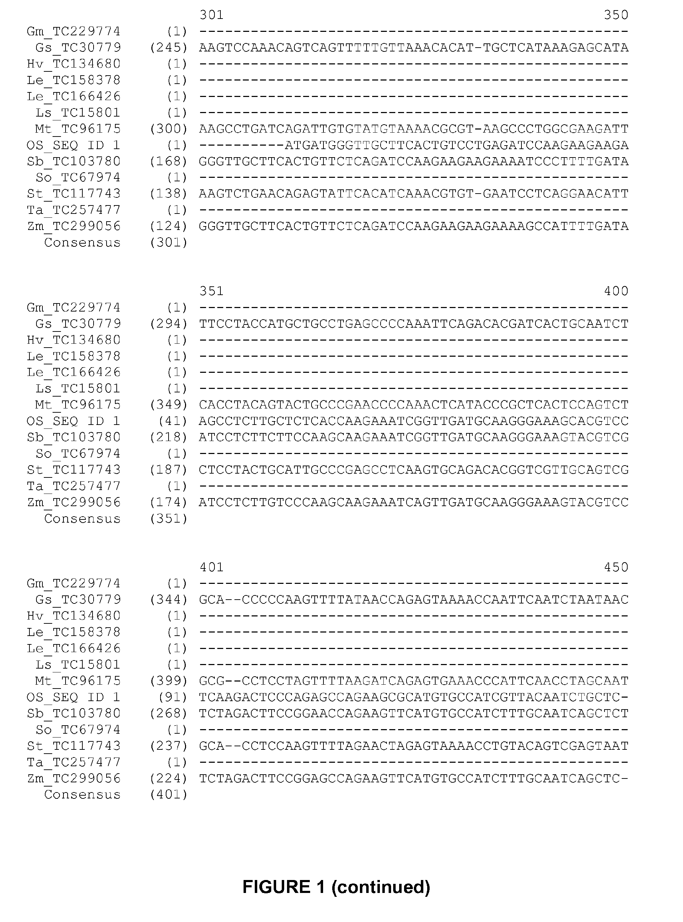

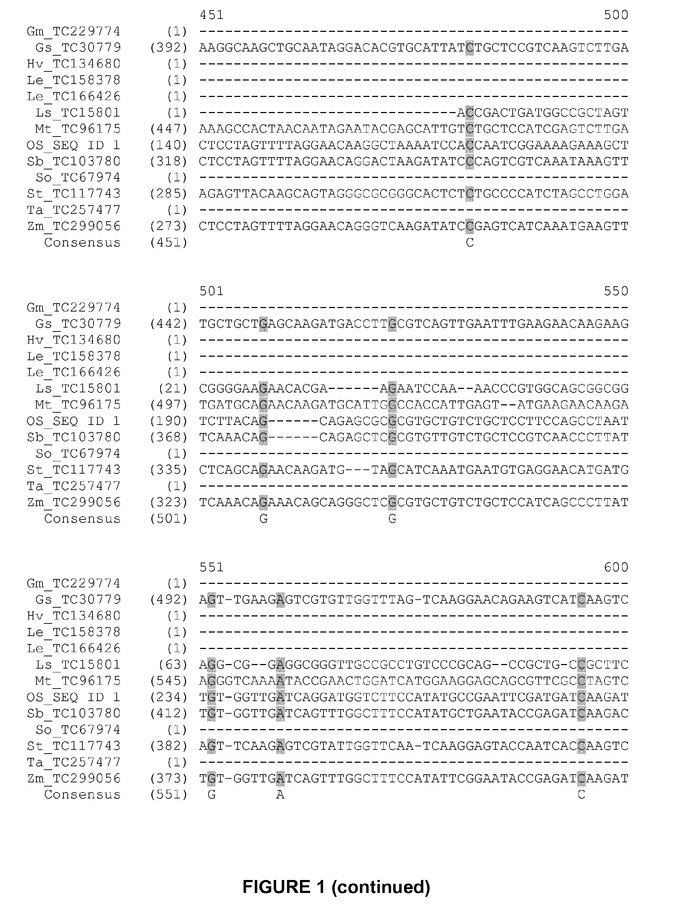

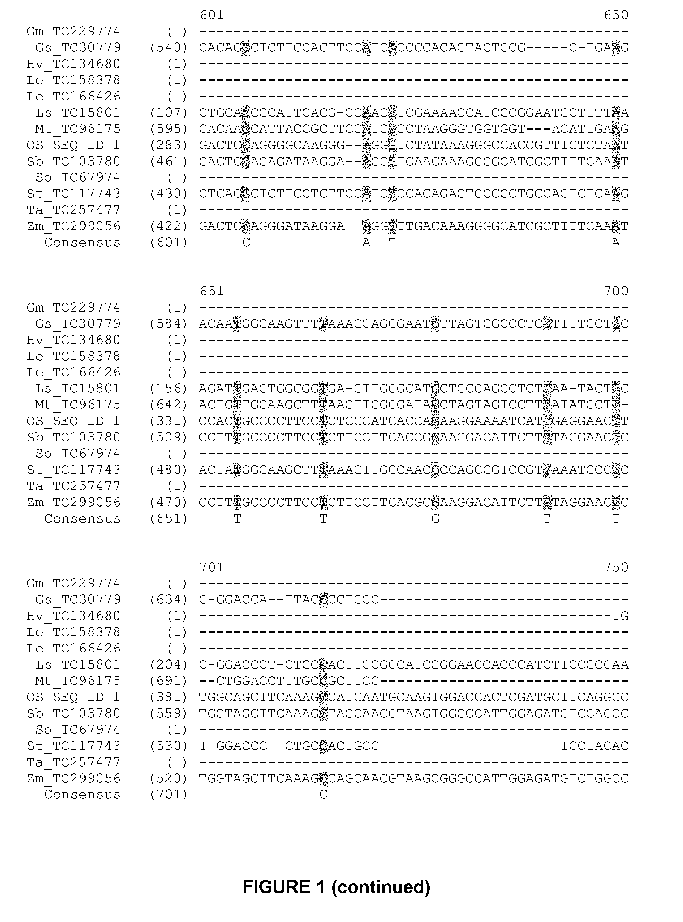

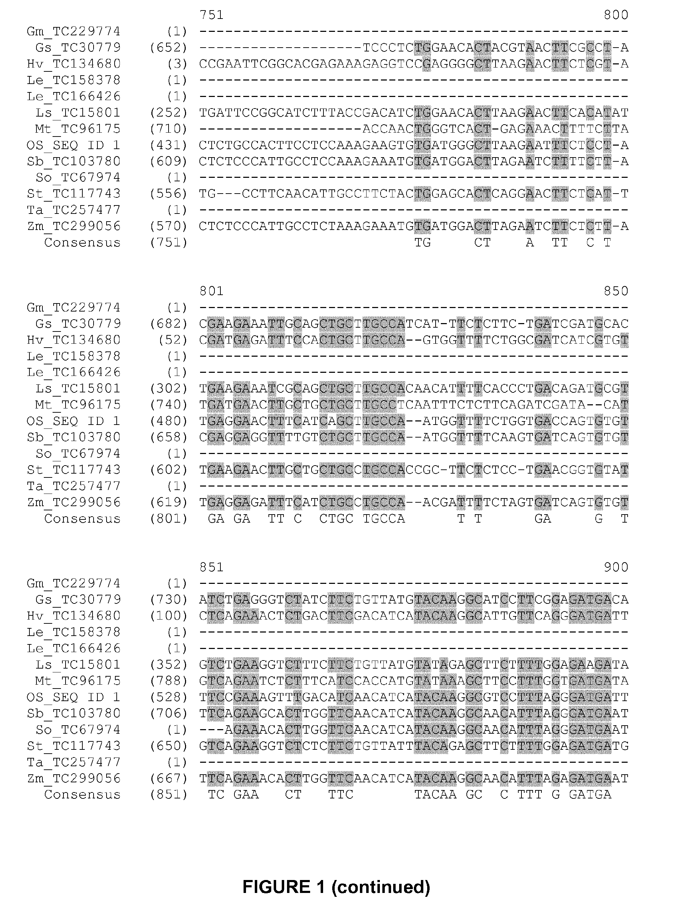

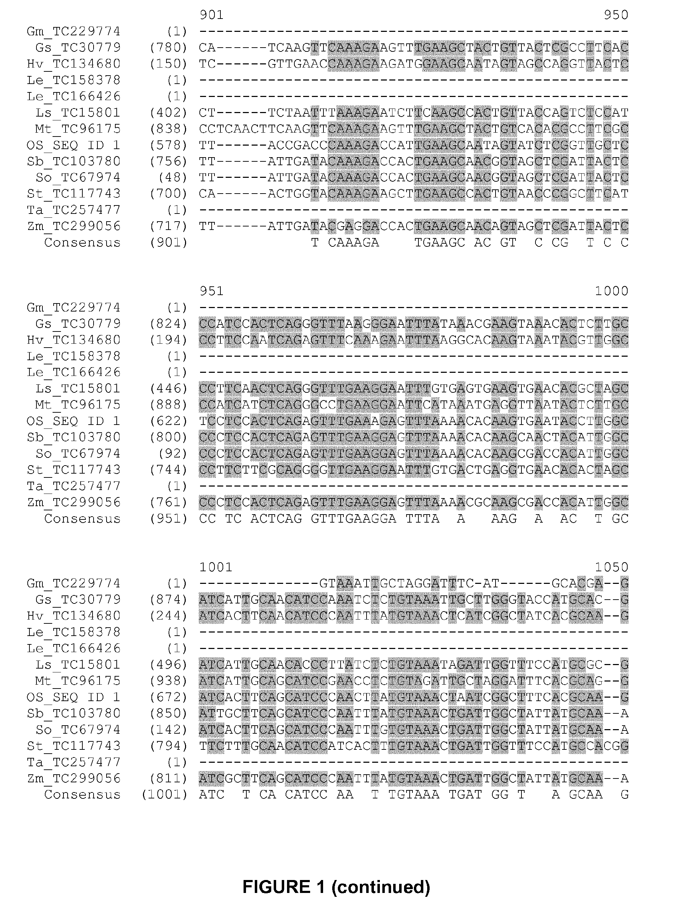

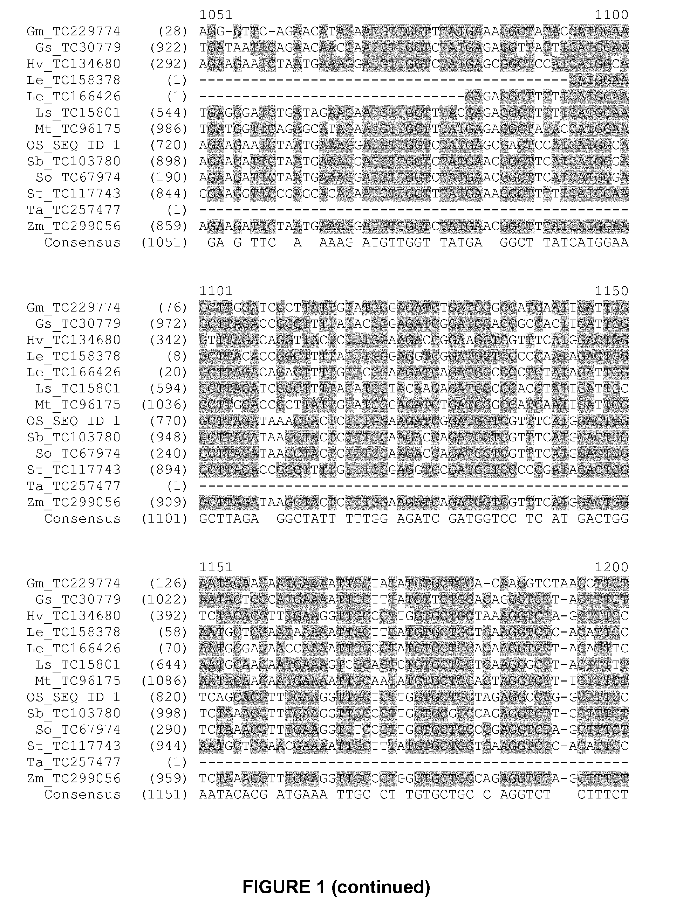

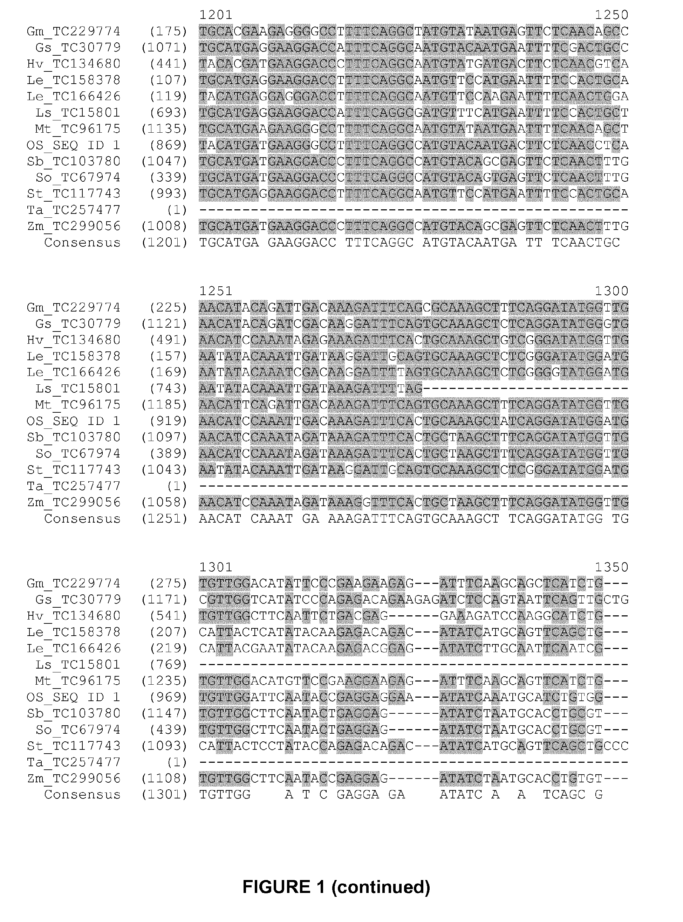

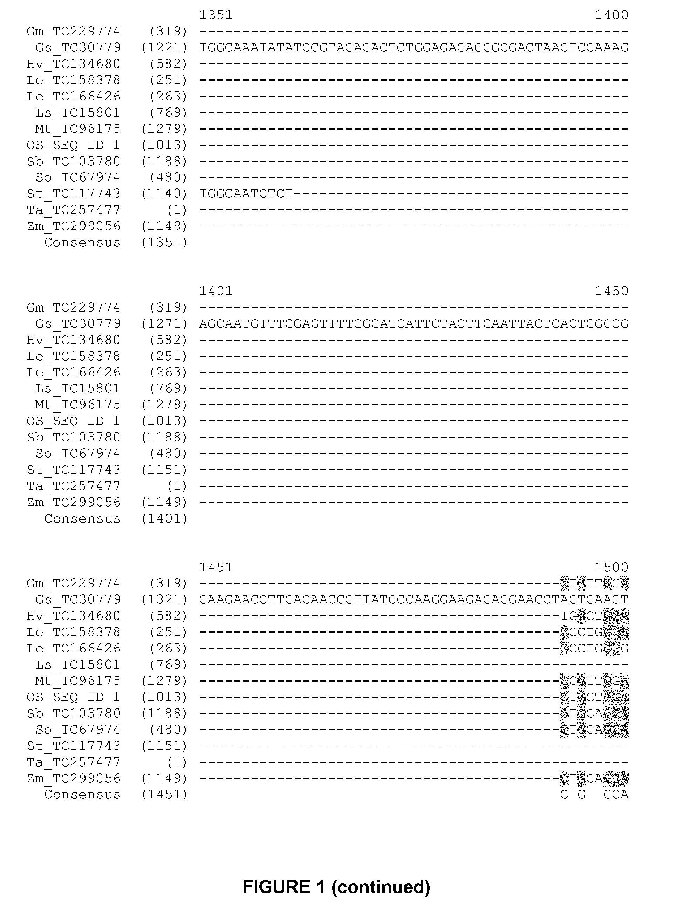

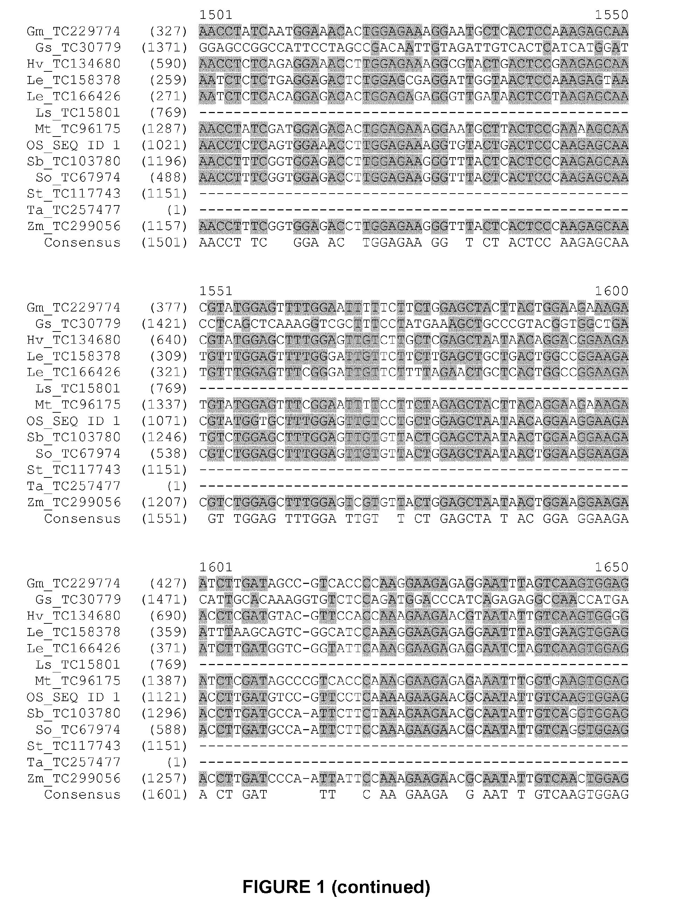

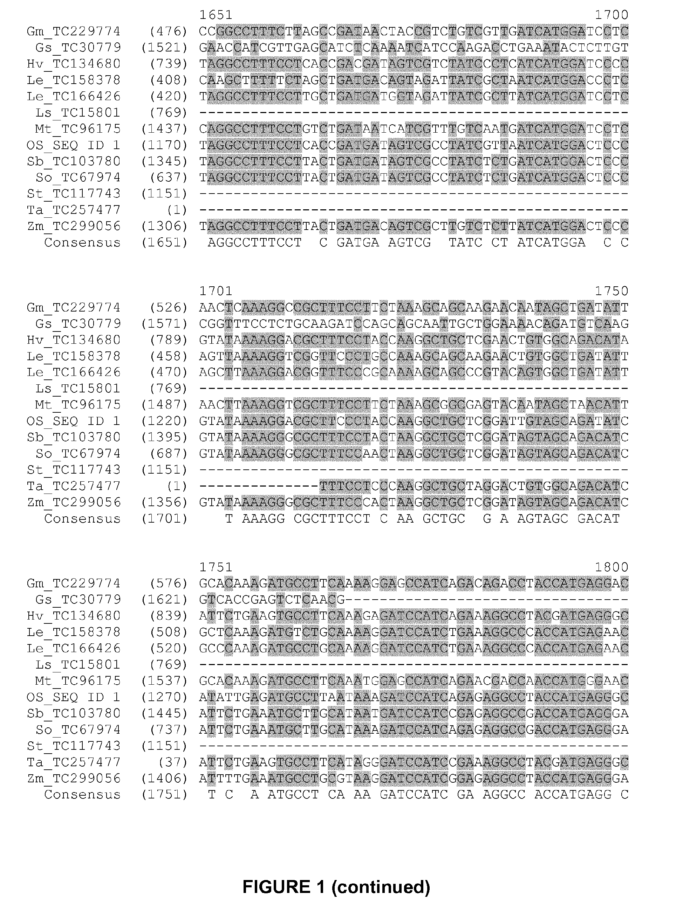

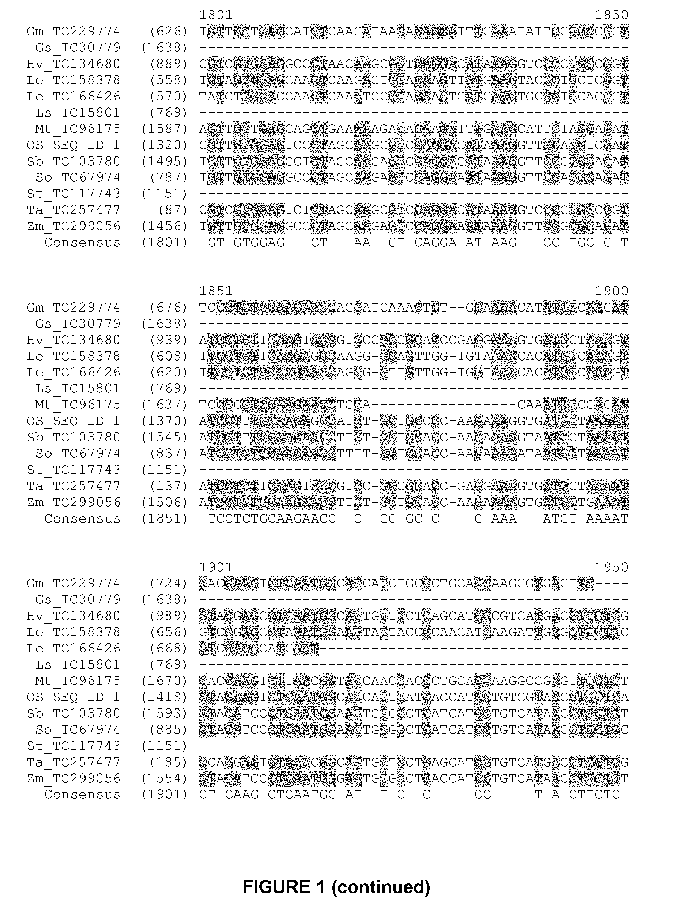

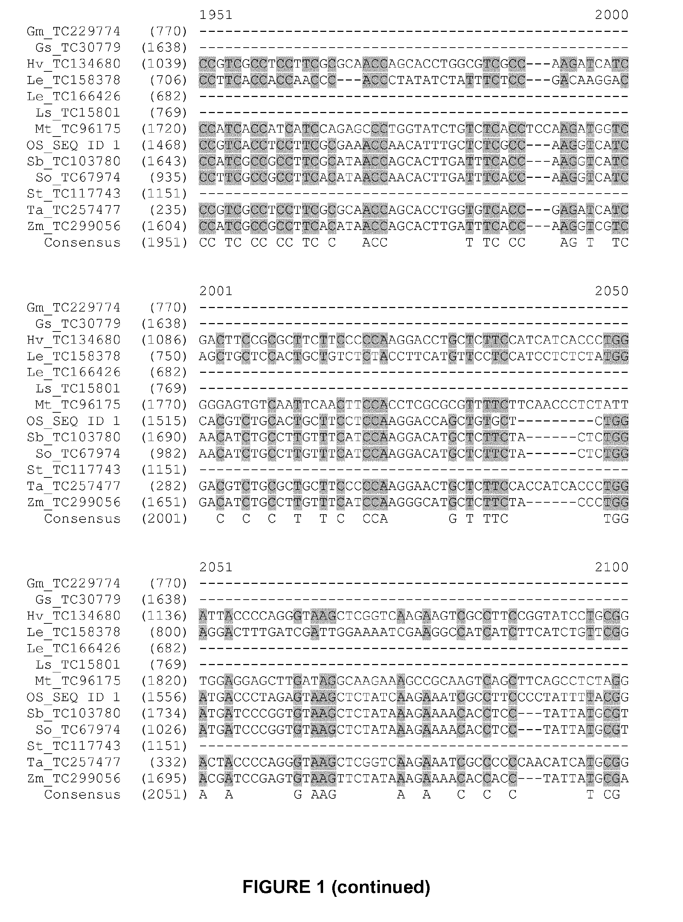

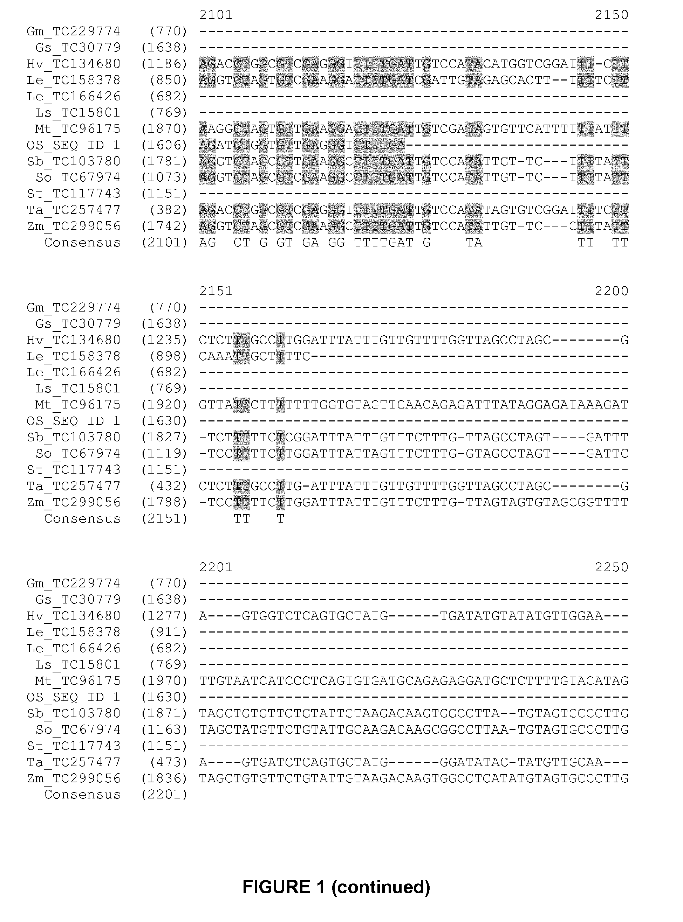

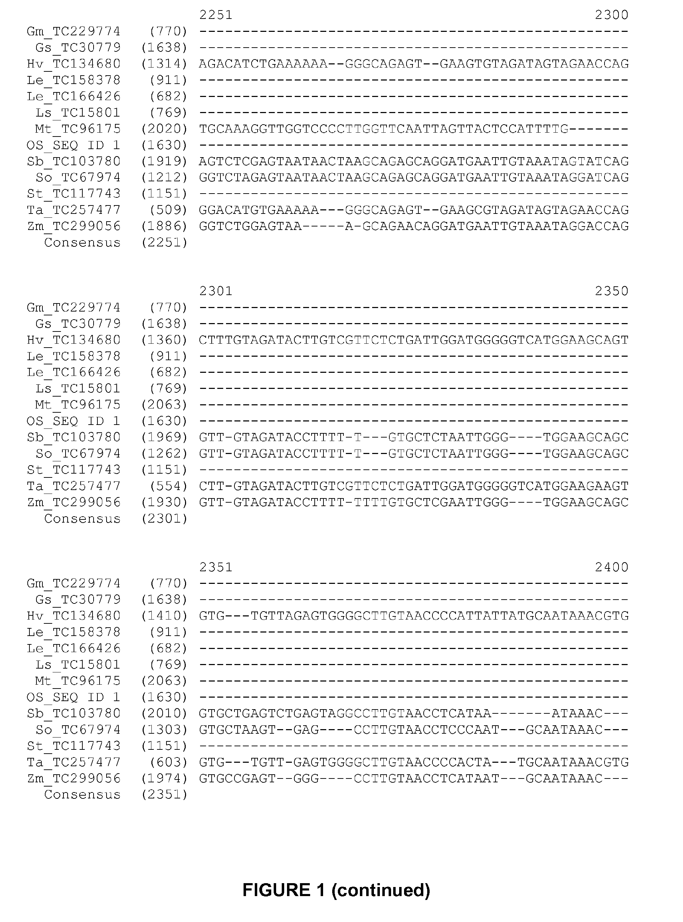

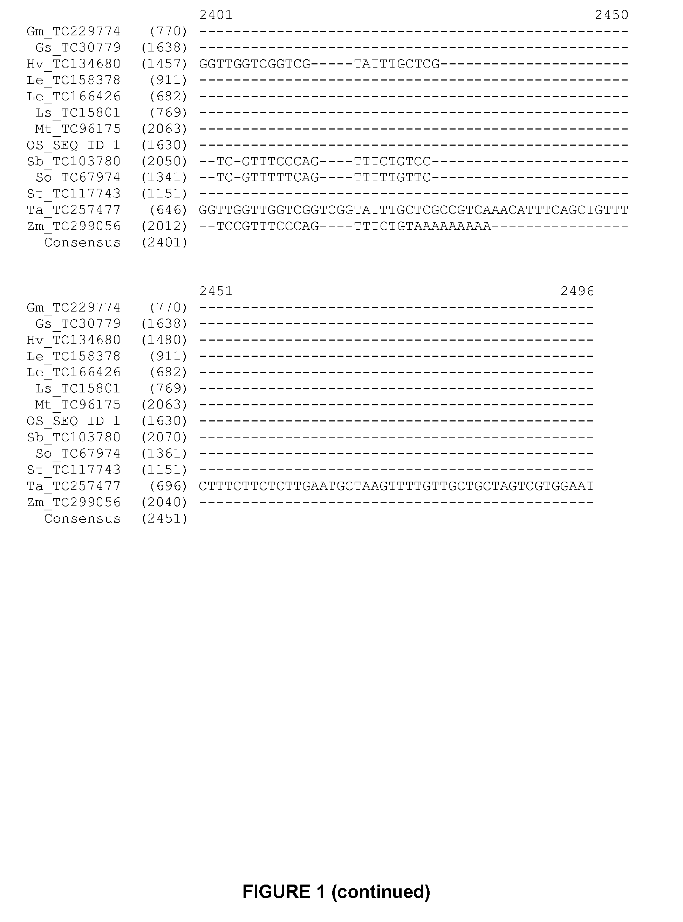

**Figure 1** is an alignment of SIK nucleic acids encoding putative SIK orthologues described in Table 3.

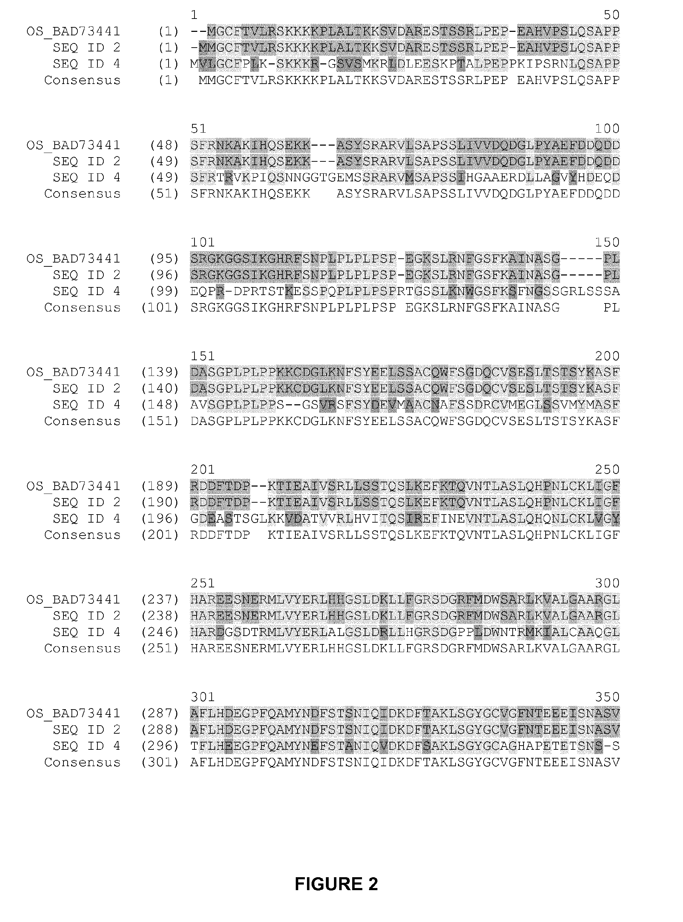

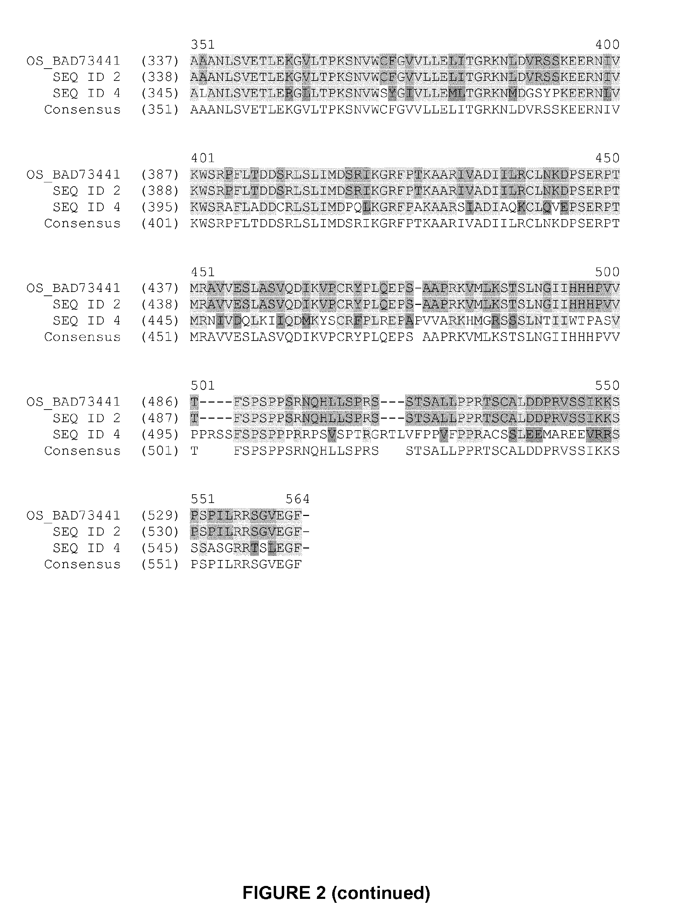

**Figure 2** is an alignment of rice SIK polypeptide (SEQ ID NO: 2) and *Arabidopsis* SIK polypeptide (SEQ ID NO: 4) and OSSIK polypeptide having NCBI accession number *OS*_BAD73441.
**Figure 3** represents the binary for vector endogenous gene silencing in *Oryza sativa* using a SIK nucleic acid represented by SEQ ID NO: 1 using a hairpin construct under the control of a constitutive promoter, GOS2.
**Figure 4** shows a binary vector for expression in *Oryza sativa* of a SIK-encoding nucleic acid from *Oryza sativa* under the control of a GOS2 promoter.

**Examples**

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

<u>Example 1: Gene Cloning</u>

The rice SIK gene was amplified by PCR with primers (SEQ ID NO: 227; sense, start codon in bold, AttB1 site in italic: 5'-ggggacaagtttgtacaaaaaagcaggcttaaacaatgatgggttgcttcactgtc-3') and (SEQ ID NO: 228; reverse, complementary, AttB2 site in italic: 5'-ggggaccactttgtacaagaaagctgggtatggacaatcaaaaccctca-3'), which include the AttB sites for Gateway recombination, were used for PCR amplification. PCR was performed using Hifi Taq DNA polymerase under standard conditions. The PCR fragment was purified using standard methods. The first step of the Gateway procedure, the BP reaction, was then performed, during which the PCR fragment recombines *in vivo* with the pDONR201 plasmid to produce, according to the Gateway terminology, an "entry clone". Plasmid pDONR201 was purchased from Invitrogen, as part of the Gateway@ technology.

<u>Example 2: Vector Construction Downregulation</u>

The entry clone was subsequently used in an LR reaction with a destination vector used for Oryza sativa transformation for the downregulation construct. This vectors contained within the T-DNA borders: a plant selectable marker; a screenable marker expression cassette; and a Gateway cassette intended for LR in vivo recombination so as to integrate the sequence of interest from the entry clone in sense or anti sense orientation. A rice GOS2 promoter (SEQ ID NO: 226) for constitutive expression was located upstream of this Gateway cassette.

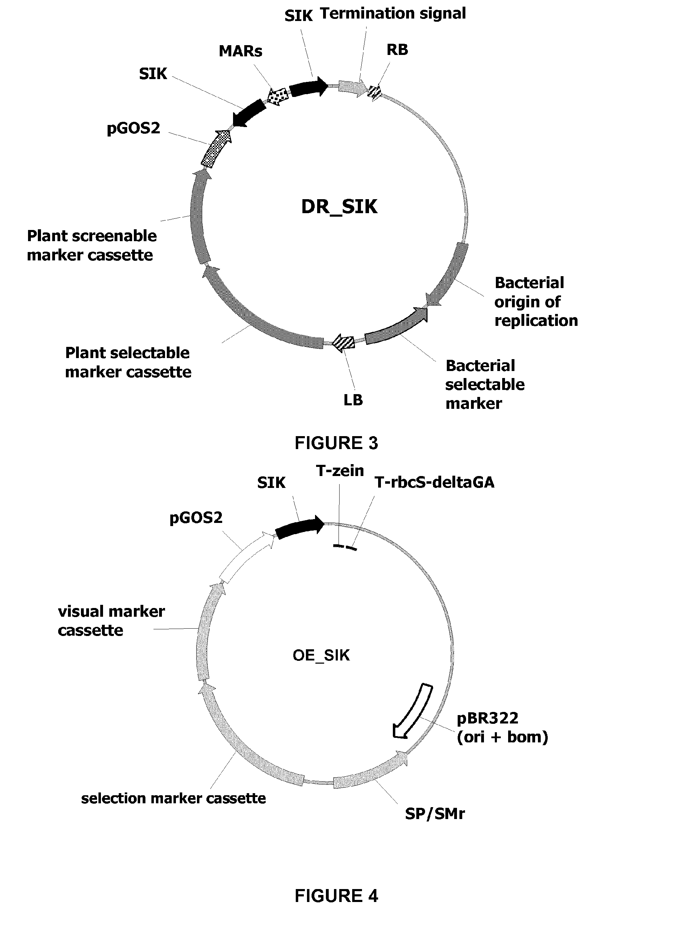

After the LR recombination step, the resulting expression vector (Figure 3) was transformed into Agrobacterium strain LBA4044 and subsequently to Oryza sativa plants. Transformed rice plants were allowed to grow and were then examined for the parameters described in Example 4.

Example 3: Vector Construction Overexpression

The entry clone was subsequently used in an LR reaction with a destination vector used for plant (Oryza sativa) transformation. This vector contained within the T-DNA borders: a plant selectable marker; a screenable marker expression cassette; and a Gateway cassette intended for LR in vivo recombination with the sequence of interest already cloned in the entry clone. A rice GOS2 promoter for constitutive expression was located upstream of this Gateway cassette.

After the LR recombination step, the resulting expression vector (Figure 4) was transformed into Agrobacterium strain LBA4044. Transformed colonies were grown on YEP media and selected by respective antibiotics for two days at 28°C. These Agrobacterium cultures were used for the plant transformation. Transformed rice plants were allowed to grow and were then examined for the parameters described in Example 4.

Example 4: Evaluation of plants transformed with SIK in anti sense orientation

Approximately 15 to 20 independent T0 rice transformants were generated. The primary transformants were transferred from a tissue culture chamber to a greenhouse for growing and harvest of T1 seed. Six events for which the T1 progeny segregated 3:1 for presence/absence of the transgene, were retained. For each of these events, approximately 10 T1 seedlings containing the transgene (hetero- and homozygotes) and approximately 10 T1 seedlings lacking the transgene (nullizygotes) were selected by monitoring visual marker expression. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C, night time temperature= 22°C, relative humidity= 60-70%. Plants were grown under optimal watering conditions until they approached the heading stage when irrigation was withheld. Humidity probes were inserted in randomly chosen pots to monitor the soil water content (SWC). When SWC dropped below 20%, the plants were automatically re-watered to achieve optimal watering conditions again. Transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts aboveground. The Areamax is the aboveground area at the time point at which the plant had reached its maximal leafy biomass.

The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of the following seed-related parameters:

The flowers-per-panicle is a parameter estimating the average number of florets per panicle on a plant, derived from the number of total seeds divided by the number of first panicles. The tallest panicle and all the panicles that overlapped with the tallest panicle when aligned vertically, were considered as first panicles and were counted manually. The number of filled seeds was determined by counting the number of filled husks that remained after the separation step. The total seed yield (total seed weight) was measured by weighing all filled husks harvested from a plant. Total seed number per plant was measured by counting the number of husks harvested from a plant and corresponds to the number of florets per plant. These parameters were derived in an automated way from the digital images using image analysis software and were analysed statistically. Individual seed parameters (including width, length, area, weight) were measured using a custom-made device consisting of two main components, a weighing and imaging device, coupled to software for image analysis. The harvest index in the present invention is defined as the ratio between the total seed yield (g) and the above ground area ($mm^2$), multiplied by a factor 106.

A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value was obtained by comparing the t-value to the t-distribution or alternatively, by comparing the F-value to the F-distribution. The p-value then gives the probability that the null hypothesis (i.e., that there is no effect of the transgene) is correct.

Example 5: Results

1. Thousand Kernel Weight

The transgenic lines transformed with the downregulation construct gave an overall percentage increase of 3% compared to controls with the best line giving a 10% increase compared to controls.

2. Harvest Index

The transgenic lines transformed with the downregulation construct gave an overall percentage increase of 16% compared to controls with the best line giving a 61 % increase compared to controls.

3. Fill rate

The transgenic lines transformed with the downregulation construct gave an overall percentage increase of 14% compared to controls with the best line giving a 41 % increase compared to controls.

4. Flowers per panicle (number of flowers per plant)

The transgenic lines transformed with the downregulation construct gave an overall percentage decrease of -3% compared to controls with the best line giving a -11 % decrease compared to controls. A decrease in the number of flowers may be important for grasses (when used for lawns for example).

The transgenic lines transformed with the overexpression construct gave an overall percentage increase of 6% compared to controls with the best line giving a 14% increase compared to controls.

## Claims

1. Method for improving various yield-related traits in plants relative to control plants, comprising modulating expression in a plant of a small inducible kinase (SIK) nucleic acid and/or a SIK polypeptide, wherein said yield-related trait is an increase in the number of flowers per plant when said modulated expression consists of overexpression in a plant of a nucleic acid encoding a SIK polypeptide, and wherein said yield-related trait is one or more of increased thousand kernel weight (TKW), increased harvest index (HI) and increased fill rate when said modulated expression consists of a reduction or substantial elimination of expression of an endogenous SIK gene in a plant.

2. Method according to claim 1, wherein said reduction or substantial elimination of expression of an endogenous SIK gene is effected by RNA-mediated silencing of gene expression.

3. Method according to claim 2, wherein said RNA-mediated silencing is effected by co-suppression, or is effected by use of antisense SIK nucleic acid sequences.

4. Method according to claims 1 or 2, wherein said reduction or substantial elimination of expression of an endogenous SIK gene is effected using an inverted repeat of a SIK nucleic acid, preferably capable of forming a hairpin structure.

5. Method according to any one of claims 1 to 4, wherein said endogenous SIK gene or SIK nucleic acid used in the reduction or substantial elimination of expression of an endogenous SIK gene is from a plant source or artificial source.

6. Method according to claim 5, wherein said SIK nucleic acid is from a monocotyledonous plant and is used for transformation of monocotyledonous plants, or is from a dicotyledonous plant and is used for transformation of dicotyledonous plants.

7. Method according to claim 6, wherein said SIK nucleic acid is from the family Poaceae and is used for transformation of plants of the family Poaceae, more preferably wherein said SIK nucleic acid is from rice and is used to transform rice plants.

8. Method according to any one of claims 5 to 7, wherein said nucleic acid comprises a sufficient length of substantially contiguous nucleotides of SEQ ID NO: 209 or SEQ ID NO: 211 or any one of SEQ ID NOs 213-225 or any one of the nucleic acids given in Table 3 or Table 4.

9. Method according to claim 1, wherein said overexpression is effected by introducing and expressing in a plant a nucleic acid encoding a SIK polypeptide.

10. Method according to claim 1 or 9, wherein said SIK polypeptide is represented by SEQ ID NO: 210 or an orthologue or paralogue thereof.

11. Method according to claim 1, 9 or 10, wherein said SIK polypeptide comprises one or more of, preferably all of the following:

(i) an ATP-binding region;
(ii) a serine threonine kinase active site signature;
(iii) a serine-rich domain;
(iv) one or more myristoylation sites;
(v) kinase activity.

12. Plant, plant part or plant cell thereof obtainable by a method according to any one of claims 1 to 8 wherein said plant, plant part or plant cell thereof has reduced expression of an endogenous SIK gene using a SIK nucleic acid sequence, and having one or more of increased thousand kernel weight (TKW), increased harvest index (HI) and increased fill rate relative to control plants.

13. Plant, plant part or plant cell thereof obtainable by a method according to any one of claims 9 to 11, wherein said plant, plant part or plant cell thereof has increased expression of a SIK-encoding nucleic acid relative to control plants and has an increased number of flowers per plants relative to control plants.

14. Construct comprising:

(a) a nucleic acid encoding a SIK polypeptide represented by SEQ ID NO: 210 or an orthologue or paralogue thereof;
(b) one or more control sequences capable of driving expression of the nucleic acid sequence of (a); and optionally
(c) a transcription termination sequence.

15. Construct for reduced expression in a plant of an endogenous SIK gene comprising one or more control sequences, a SIK nucleic acid, and optionally a transcription termination sequence.

16. Construct according to claim 15, wherein said SIK nucleic acid is an inverted repeat, preferably capable of forming a hairpin structure, which inverted repeat is under the control of a constitutive promoter.

**17.** Construct according to claim 16, wherein said constitutive promoter is a GOS2 promoter from rice, preferably a promoter having comparable expression profile to the promoter of SEQ ID NO: 56 or SEQ ID NO: 226.

**18.** Plant, plant part or plant cell transformed with a construct according to any one of claims 14 to 17.

**19.** Method for the production of a transgenic plant having increased number of flowers per plant relative to control plants, which method comprises:

(i) introducing and expressing in a plant a nucleic acid encoding a SIK polypeptide represented by SEQ ID NO: 210 or an orthologue or paralogue thereof; and
(ii) cultivating the plant cell under conditions promoting plant growth and development.

**20.** Method for the production of transgenic plants having one or more of increased thousand kernel weight (TKW), increased harvest index (HI) and increased fill rate increased yield relative to control plants, which method comprises:

(i) introducing and expressing in a plant, plant part or plant cell a genetic construct comprising one or more control sequences for reducing expression in a plant of an endogenous SIK gene using a SIK nucleic acid sequence; and
(ii) cultivating the plant, plant part or plant cell under conditions promoting plant growth and development.

**21.** Transgenic plant having an increased number of flowers per plant relative to control plants **characterised in that** said plant has increased expression of a nucleic acid encoding a SIK polypeptide compared to control plants.

**22.** Transgenic plant having one or more of increased thousand kernel weight (TKW), increased harvest index (HI) and increased fill rate yield relative to control plants, **characterised in that** said plant has reduced expression of an endogenous SIK gene compared to control plants.

**23.** Harvestable parts of a transgenic plant according to claim 12, 13, 18, 21 or 22.

**24.** Products derived from a plant according to claim 12, 13, 18, 21 or 22 or from harvestable parts according to claim 23.

**25.** Use of a SIK-encoding nucleic acid for increasing the number of flowers per plant relative to control plants by overexpression of a SIK-encoding nucleic acid in a plant.

**26.** Use of a SIK nucleic acid for increasing one or more of thousand kernel weight (TKW), harvest index (HI) and fill rate yield relative to control plants by the reduction or substantial elimination in a plant of an endogenous SIK gene.

```
                           1                                                50
Gm_TC229774        (1)     --------------------------------------------------
 Gs_TC30779        (1)     --------------------------------------------------
Hv_TC134680        (1)     --------------------------------------------------
Le_TC158378        (1)     --------------------------------------------------
Le_TC166426        (1)     --------------------------------------------------
 Ls_TC15801        (1)     --------------------------------------------------
 Mt_TC96175        (1)     ACGAGGCTATTCTCTCTTTCTCTCTCTCTACATTTCTCAACATTCTTCAA
OS_SEQ ID 1        (1)     --------------------------------------------------
Sb_TC103780        (1)     --------------------------------------------------
 So_TC67974        (1)     --------------------------------------------------
St_TC117743        (1)     --------------------------------------------------
Ta_TC257477        (1)     --------------------------------------------------
Zm_TC299056        (1)     --------------------------------------------------
  Consensus        (1)

                           51                                              100
Gm_TC229774        (1)     --------------------------------------------------
 Gs_TC30779        (1)     ------CTTTTTCCTCTCAAACCTCTCTCACACTGATAAACTTTTCTCTC
Hv_TC134680        (1)     --------------------------------------------------
Le_TC158378        (1)     --------------------------------------------------
Le_TC166426        (1)     --------------------------------------------------
 Ls_TC15801        (1)     --------------------------------------------------
 Mt_TC96175       (51)     ATTTCTCTCTCCAAAACCTTCACTTCGCAGCTTTTTCAATCTGCTTTCTT
OS_SEQ ID 1        (1)     --------------------------------------------------
Sb_TC103780        (1)     --------------------------------------------------
 So_TC67974        (1)     --------------------------------------------------
St_TC117743        (1)     --------------------------------------------------
Ta_TC257477        (1)     --------------------------------------------------
Zm_TC299056        (1)     --------------------------------------------------
  Consensus       (51)

                           101                                             150
Gm_TC229774        (1)     --------------------------------------------------
 Gs_TC30779       (45)     CATTACCTTTCTTTGACTTGCAAAGTTGTTCTCCTCAAACTAACCAAACA
Hv_TC134680        (1)     --------------------------------------------------
Le_TC158378        (1)     --------------------------------------------------
Le_TC166426        (1)     --------------------------------------------------
 Ls_TC15801        (1)     --------------------------------------------------
 Mt_TC96175      (101)     TGACTCTGTAACAGTTTTTTTCGTGGAAGAATCAGAACCACAAAAAAGTT
OS_SEQ ID 1        (1)     --------------------------------------------------
Sb_TC103780        (1)     ---------------------------------CTACTGGATTAACCCCC
 So_TC67974        (1)     --------------------------------------------------
St_TC117743        (1)     --------------------------------------------------
Ta_TC257477        (1)     --------------------------------------------------
Zm_TC299056        (1)     --------------------------------------------------
  Consensus      (101)
```

## FIGURE 1

```
                         151                                              200
Gm_TC229774        (1)   --------------------------------------------------
 Gs_TC30779       (95)   AGGGATTCTGGGTTTTCAGCTTTCTCTGTTTTCCTACTCTCTTTCGCCTT
Hv_TC134680        (1)   --------------------------------------------------
Le_TC158378        (1)   --------------------------------------------------
Le_TC166426        (1)   --------------------------------------------------
 Ls_TC15801        (1)   --------------------------------------------------
 Mt_TC96175      (151)   TCGATTTTTACCCATTTCTTCAACCCGTGATCGCTTCACTGTAATTGGCA
OS_SEQ ID 1        (1)   --------------------------------------------------
Sb_TC103780       (18)   CGCGTGCGTTCCCTCCCTCTGTCAGTCTGTCTCTCTCTTGGCGTCGACTG
 So_TC67974        (1)   --------------------------------------------------
St_TC117743        (1)   -------------TTCTTAGCTCATCGAAACGTTAAACCTTACAATACTT
Ta_TC257477        (1)   --------------------------------------------------
Zm_TC299056        (1)   ---------------------------CTCCCCCGGCGTGCGCTCCCTCC
  Consensus      (151)

                         201                                              250
Gm_TC229774        (1)   --------------------------------------------------
 Gs_TC30779      (145)   TGCATGACCTGCTTACATAGAAACATAAAAGTCCGAAATACAATTTAGGT
Hv_TC134680        (1)   --------------------------------------------------
Le_TC158378        (1)   --------------------------------------------------
Le_TC166426        (1)   --------------------------------------------------
 Ls_TC15801        (1)   --------------------------------------------------
 Mt_TC96175      (201)   TGAGCTTCCATTTCTCGT-TCTGACAAAAACAGGTATCTATAACATCCTA
OS_SEQ ID 1        (1)   --------------------------------------------------
Sb_TC103780       (68)   GGCGACCGTCGCAGCCGCCCTAAGCCAGGTACCCAGACCATCTTCGGGCC
 So_TC67974        (1)   --------------------------------------------------
St_TC117743       (38)   CAAACATCGATCAATACAATTTGCTGAAGCACGCAATTTTTCAAGGACTG
Ta_TC257477        (1)   --------------------------------------------------
Zm_TC299056       (24)   TGTCTCTCTTGGCGACGACTGGGCAACGGTCGCAGCTGTATGTTCGAGCC
  Consensus      (201)

                         251                                              300
Gm_TC229774        (1)   --------------------------------------------------
 Gs_TC30779      (195)   GGTTTGTATATAAGATGGGGTGTTTTACAGTTTTGAGAAGCAACAAGAAA
Hv_TC134680        (1)   --------------------------------------------------
Le_TC158378        (1)   --------------------------------------------------
Le_TC166426        (1)   --------------------------------------------------
 Ls_TC15801        (1)   --------------------------------------------------
 Mt_TC96175      (250)   GTTCTGTACATAGAATGGGGTGTTTTACTATATTGAAGAGAAAGAAGAAA
OS_SEQ ID 1        (1)   --------------------------------------------------
Sb_TC103780      (118)   CTTCGCCGGCGACGAGCACCACCAGGAATTTTCCCAGCTGTAGCAATGAT
 So_TC67974        (1)   --------------------------------------------------
St_TC117743       (88)   TATAAACAACAAAGATGGGTTGTTTCACAGTTTTAAAAAGTAAGAAGAAG
Ta_TC257477        (1)   --------------------------------------------------
Zm_TC299056       (74)   CTTCGCCGACGACGTTCACCACCAGGAATTTTCTCAGCTGTGCCAATGAT
  Consensus      (251)
```

## FIGURE 1 (continued)

```
                       301                                              350
Gm_TC229774      (1)   --------------------------------------------------
 Gs_TC30779    (245)   AAGTCCAAACAGTCAGTTTTTGTTAAACACAT-TGCTCATAAAGAGCATA
Hv_TC134680      (1)   --------------------------------------------------
Le_TC158378      (1)   --------------------------------------------------
Le_TC166426      (1)   --------------------------------------------------
 Ls_TC15801      (1)   --------------------------------------------------
 Mt_TC96175    (300)   AAGCCTGATCAGATTGTGTATGTAAAACGCGT-AAGCCCTGGCGAAGATT
OS_SEQ ID 1      (1)   ----------ATGATGGGTTGCTTCACTGTCCTGAGATCCAAGAAGAAGA
Sb_TC103780    (168)   GGGTTGCTTCACTGTTCTCAGATCCAAGAAGAAGAAAATCCCTTTTGATA
 So_TC67974      (1)   --------------------------------------------------
St_TC117743    (138)   AAGTCTGAACAGAGTATTCACATCAAACGTGT-GAATCCTCAGGAACATT
Ta_TC257477      (1)   --------------------------------------------------
Zm_TC299056    (124)   GGGTTGCTTCACTGTTCTCAGATCCAAGAAGAAGAAAAGCCATTTTGATA
  Consensus    (301)

                       351                                              400
Gm_TC229774      (1)   --------------------------------------------------
 Gs_TC30779    (294)   TTCCTACCATGCTGCCTGAGCCCCAAATTCAGACACGATCACTGCAATCT
Hv_TC134680      (1)   --------------------------------------------------
Le_TC158378      (1)   --------------------------------------------------
Le_TC166426      (1)   --------------------------------------------------
 Ls_TC15801      (1)   --------------------------------------------------
 Mt_TC96175    (349)   CACCTACAGTACTGCCCGAACCCCAAACTCATACCCGCTCACTCCAGTCT
OS_SEQ ID 1     (41)   AGCCTCTTGCTCTCACCAAGAAATCGGTTGATGCAAGGGAAAGCACGTCC
Sb_TC103780    (218)   ATCCTCTTCTTCCAAGCAAGAAATCGGTTGATGCAAGGGAAAGTACGTCG
 So_TC67974      (1)   --------------------------------------------------
St_TC117743    (187)   CTCCTACTGCATTGCCCGAGCCTCAAGTGCAGACACGGTCGTTGCAGTCG
Ta_TC257477      (1)   --------------------------------------------------
Zm_TC299056    (174)   ATCCTCTTGTCCCAAGCAAGAAATCAGTTGATGCAAGGGAAAGTACGTCC
  Consensus    (351)

                       401                                              450
Gm_TC229774      (1)   --------------------------------------------------
 Gs_TC30779    (344)   GCA--CCCCCAAGTTTTATAACCAGAGTAAAACCAATTCAATCTAATAAC
Hv_TC134680      (1)   --------------------------------------------------
Le_TC158378      (1)   --------------------------------------------------
Le_TC166426      (1)   --------------------------------------------------
 Ls_TC15801      (1)   --------------------------------------------------
 Mt_TC96175    (399)   GCG--CCTCCTAGTTTTAAGATCAGAGTGAAACCCATTCAACCTAGCAAT
OS_SEQ ID 1     (91)   TCAAGACTCCCAGAGCCAGAAGCGCATGTGCCATCGTTACAATCTGCTC-
Sb_TC103780    (268)   TCTAGACTTCCGGAACCAGAAGTTCATGTGCCATCTTTGCAATCAGCTCT
 So_TC67974      (1)   --------------------------------------------------
St_TC117743    (237)   GCA--CCTCCAAGTTTTAGAACTAGAGTAAAACCTGTACAGTCGAGTAAT
Ta_TC257477      (1)   --------------------------------------------------
Zm_TC299056    (224)   TCTAGACTTCCGGAGCCAGAAGTTCATGTGCCATCTTTGCAATCAGCTC-
  Consensus    (401)
```

## FIGURE 1 (continued)

```
                       451                                               500
Gm_TC229774      (1)   --------------------------------------------------
 Gs_TC30779    (392)   AAGGCAAGCTGCAATAGGACACGTGCATTATCTGCTCCGTCAAGTCTTGA
Hv_TC134680      (1)   --------------------------------------------------
Le_TC158378      (1)   --------------------------------------------------
Le_TC166426      (1)   --------------------------------------------------
 Ls_TC15801      (1)   ------------------------------ACCGACTGATGGCCGCTAGT
 Mt_TC96175    (447)   AAAGCCACTAACAATAGAATACGAGCATTGTCTGCTCCATCGAGTCTTGA
OS_SEQ ID 1    (140)   CTCCTAGTTTTAGGAACAAGGCTAAAATCCACCAATCGGAAAAGAAAGCT
Sb_TC103780    (318)   CTCCTAGTTTTAGGAACAGGACTAAGATATCCCAGTCGTCAAATAAAGTT
 So_TC67974      (1)   --------------------------------------------------
St_TC117743    (285)   AGAGTTACAAGCAGTAGGGCGCGGGCACTCTCTGCCCCATCTAGCCTGGA
Ta_TC257477      (1)   --------------------------------------------------
Zm_TC299056    (273)   CTCCTAGTTTTAGGAACAGGGTCAAGATATCCGAGTCATCAAATGAAGTT
  Consensus    (451)                                  C

                       501                                               550
Gm_TC229774      (1)   --------------------------------------------------
 Gs_TC30779    (442)   TGCTGCTGAGCAAGATGACCTTGCGTCAGTTGAATTTGAAGAACAAGAAG
Hv_TC134680      (1)   --------------------------------------------------
Le_TC158378      (1)   --------------------------------------------------
Le_TC166426      (1)   --------------------------------------------------
 Ls_TC15801     (21)   CGGGGAAGAACACGA------AGAATCCAA--AACCCGTGGCAGCGGCGG
 Mt_TC96175    (497)   TGATGCAGAACAAGATGCATTGGCCACCATTGAGT--ATGAAGAACAAGA
OS_SEQ ID 1    (190)   TCTTACAG------CAGAGCGCGCGTGCTGTCTGCTCCTTCCAGCCTAAT
Sb_TC103780    (368)   TCAAACAG------CAGAGCTCGCGTGTTGTCTGCTCCGTCAACCCTTAT
 So_TC67974      (1)   --------------------------------------------------
St_TC117743    (335)   CTCAGCAGAACAAGATG---TAGCATCAAATGAATGTGAGGAACATGATG
Ta_TC257477      (1)   --------------------------------------------------
Zm_TC299056    (323)   TCAAACAGAAACAGCAGGGCTCGCGTGCTGTCTGCTCCATCAGCCCTTAT
  Consensus    (501)          G              G

                       551                                               600
Gm_TC229774      (1)   --------------------------------------------------
 Gs_TC30779    (492)   AGT-TGAAGAGTCGTGTTGGTTTAG-TCAAGGAACAGAAGTCATCAAGTC
Hv_TC134680      (1)   --------------------------------------------------
Le_TC158378      (1)   --------------------------------------------------
Le_TC166426      (1)   --------------------------------------------------
 Ls_TC15801     (63)   AGG-CG--GAGGCGGGTTGCCGCCTGTCCCGCAG--CCGCTG-CCGCTTC
 Mt_TC96175    (545)   AGGGTCAAAATACCGAACTGGATCATGGAAGGAGCAGCGTTCGCCTAGTC
OS_SEQ ID 1    (234)   TGT-GGTTGATCAGGATGGTCTTCCATATGCCGAATTCGATGATCAAGAT
Sb_TC103780    (412)   TGT-GGTTGATCAGTTTGGCTTTCCATATGCTGAATACCGAGATCAAGAC
 So_TC67974      (1)   --------------------------------------------------
St_TC117743    (382)   AGT-TCAAGAGTCGTATTGGTTCAA-TCAAGGAGTACCAATCACCAAGTC
Ta_TC257477      (1)   --------------------------------------------------
Zm_TC299056    (373)   TGT-GGTTGATCAGTTTGGCTTTCCATATTCGGAATACCGAGATCAAGAT
  Consensus    (551)    G       A                                  C
```

**FIGURE 1 (continued)**

```
                         601                                              650
Gm_TC229774       (1)    --------------------------------------------------
 Gs_TC30779     (540)    CACAGCCTCTTCCACTTCCATCTCCCCACAGTACTGCG-----C-TGAAG
Hv_TC134680       (1)    --------------------------------------------------
Le_TC158378       (1)    --------------------------------------------------
Le_TC166426       (1)    --------------------------------------------------
 Ls_TC15801     (107)    CTGCACCGCATTCACG-CCAACTTCGAAAACCATCGCGGAATGCTTTTAA
 Mt_TC96175     (595)    CACAACCATTACCGCTTCCATCTCCTAAGGGTGGTGGT---ACATTGAAG
OS_SEQ ID 1     (283)    GACTCCAGGGGCAAGGG--AGGTTCTATAAAGGGCCACCGTTTCTCTAAT
Sb_TC103780     (461)    GACTCCAGAGATAAGGA--AGGTTCAACAAAGGGGCATCGCTTTTCAAAT
 So_TC67974       (1)    --------------------------------------------------
St_TC117743     (430)    CTCAGCCTCTTCCTCTTCCATCTCCACAGAGTGCCGCTGCCACTCTCAAG
Ta_TC257477       (1)    --------------------------------------------------
Zm_TC299056     (422)    GACTCCAGGGATAAGGA--AGGTTTGACAAAGGGGCATCGCTTTTCAAAT
  Consensus     (601)         C             A  T                         A

                         651                                              700
Gm_TC229774       (1)    --------------------------------------------------
 Gs_TC30779     (584)    ACAATGGGAAGTTTTAAAGCAGGGAATGTTAGTGGCCCTCTTTTTGCTTC
Hv_TC134680       (1)    --------------------------------------------------
Le_TC158378       (1)    --------------------------------------------------
Le_TC166426       (1)    --------------------------------------------------
 Ls_TC15801     (156)    AGATTGAGTGGCGGTGA-GTTGGGCATGCTGCCAGCCTCTTAA-TACTTC
 Mt_TC96175     (642)    ACTGTTGGAAGCTTTAAGTTGGGGATAGCTAGTAGTCCTTTATATGCTT-
OS_SEQ ID 1     (331)    CCACTGCCCCTTCCTCTCCCATCACCAGAAGGAAAATCATTGAGGAACTT
Sb_TC103780     (509)    CCTTTGCCCCTTCCTCTTCCTTCACCGGAAGGACATTCTTTTAGGAACTC
 So_TC67974       (1)    --------------------------------------------------
St_TC117743     (480)    ACTATGGGAAGCTTTAAAGTTGGCAACGCCAGCGGTCCGTTAAATGCCTC
Ta_TC257477       (1)    --------------------------------------------------
Zm_TC299056     (470)    CCTTTGCCCCTTCCTCTTCCTTCACGCGAAGGACATTCTTTTAGGAACTC
  Consensus     (651)        T         T            G            T       T

                         701                                              750
Gm_TC229774       (1)    --------------------------------------------------
 Gs_TC30779     (634)    G-GGACCA--TTACCCCTGCC-----------------------------
Hv_TC134680       (1)    ------------------------------------------------TG
Le_TC158378       (1)    --------------------------------------------------
Le_TC166426       (1)    --------------------------------------------------
 Ls_TC15801     (204)    C-GGACCCT-CTGCCACTTCCGCCATCGGGAACCACCCATCTTCCGCCAA
 Mt_TC96175     (691)    --CTGGACCTTTGCCGCTTCC-----------------------------
OS_SEQ ID 1     (381)    TGGCAGCTTCAAAGCCATCAATGCAAGTGGACCACTCGATGCTTCAGGCC
Sb_TC103780     (559)    TGGTAGCTTCAAAGCTAGCAACGTAAGTGGGCCATTGGAGATGTCCAGCC
 So_TC67974       (1)    --------------------------------------------------
St_TC117743     (530)    T-GGACCC--CTGCCACTGCC---------------------TCCTACAC
Ta_TC257477       (1)    --------------------------------------------------
Zm_TC299056     (520)    TGGTAGCTTCAAAGCCAGCAACGTAAGCGGGCCATTGGAGATGTCTGGCC
  Consensus     (701)                  C
```

**FIGURE 1 (continued)**

```
                      751                                               800
Gm_TC229774      (1)  --------------------------------------------------
 Gs_TC30779    (652)  -------------------TCCCTCTGGAACACTACGTAACTTCGCCT-A
Hv_TC134680      (3)  CCGAATTCGGCACGAGAAAGAGGTCCGAGGGGCTTAAGAACTTCTCGT-A
Le_TC158378      (1)  --------------------------------------------------
Le_TC166426      (1)  --------------------------------------------------
 Ls_TC15801    (252)  TGATTCCGGCATCTTTACCGACATCTGGAACACTTAAGAACTTCACATAT
  Mt_TC96175   (710)  -------------------ACCAACTGGGTCACT-GAGAAACTTTTCTTA
OS_SEQ ID 1    (431)  CTCTGCCACTTCCTCCAAAGAAGTGTGATGGGCTTAAGAATTTCTCCT-A
Sb_TC103780    (609)  CTCTCCCATTGCCTCCAAAGAAATGTGATGGACTTAGAATCTTTTCTT-A
 So_TC67974      (1)  --------------------------------------------------
St_TC117743    (556)  TG---CCTTCAACATTGCCTTCTACTGGAGCACTCAGGAACTTCTCAT-T
Ta_TC257477      (1)  --------------------------------------------------
Zm_TC299056    (570)  CTCTCCCATTGCCTCTAAAGAAATGTGATGGACTTAGAATCTTCTCTT-A
  Consensus    (751)                           TG     CT    A  TT  C T

                      801                                               850
Gm_TC229774      (1)  --------------------------------------------------
 Gs_TC30779    (682)  CGAAGAAATTGCAGCTGCTTGCCATCAT-TTCTCTTC-TGATCGATGCAC
Hv_TC134680     (52)  CGATGAGATTTCCACTGCTTGCCA--GTGGTTTTCTGGCGATCATCGTGT
Le_TC158378      (1)  --------------------------------------------------
Le_TC166426      (1)  --------------------------------------------------
 Ls_TC15801    (302)  TGAAGAAATCGCAGCTGCTTGCCACAACATTTTCACCCTGACAGATGCGT
  Mt_TC96175   (740)  TGATGAACTTGCTGCTGCTTGCCTCAATTTCTCTTCAGATCGATA--CAT
OS_SEQ ID 1    (480)  TGAGGAACTTTCATCAGCTTGCCA--ATGGTTTTCTGGTGACCAGTGTGT
Sb_TC103780    (658)  CGAGGAGGTTTTGTCTGCTTGCCA--ATGGTTTTCAAGTGATCAGTGTGT
 So_TC67974      (1)  --------------------------------------------------
St_TC117743    (602)  TGAAGAACTTGCTGCTGCCTGCCACCGC-TTCTCTCC-TGAACGGTGTAT
Ta_TC257477      (1)  --------------------------------------------------
Zm_TC299056    (619)  TGAGGAGATTTCATCTGCCTGCCA--ACGATTTTCTAGTGATCAGTGTGT
  Consensus    (801)   GA GA  TT C  CTGC TGCCA      T T      GA     G  T

                      851                                               900
Gm_TC229774      (1)  --------------------------------------------------
 Gs_TC30779    (730)  ATCTGAGGGTCTATCTTCTGTTATGTACAAGGCATCCTTCGGAGATGACA
Hv_TC134680    (100)  CTCAGAAACTCTGACTTCGACATCATACAAGGCATTGTTCAGGGATGATT
Le_TC158378      (1)  --------------------------------------------------
Le_TC166426      (1)  --------------------------------------------------
 Ls_TC15801    (352)  GTCTGAAGGTCTTTCTTCTGTTATGTATAGAGCTTCTTTTGGAGAAGATA
  Mt_TC96175   (788)  GTCAGAATCTCTTTCATCCACCATGTATAAAGCTTCCTTTGGTGATGATA
OS_SEQ ID 1    (528)  TTCCGAAAGTTTGACATCAACATCATACAAGGCGTCCTTTAGGGATGATT
Sb_TC103780    (706)  TTCAGAAGCACTTGGTTCAACATCATACAAGGCAACATTTAGCGATCAAT
 So_TC67974      (1)  ---AGAAACACTTGGTTCAACATCATACAAGGCAACATTTAGGGATGAAT
St_TC117743    (650)  GTCAGAAGGTCTCTCTTCTGTTATTTACAGAGCTTCTTTTGGAGATGATG
Ta_TC257477      (1)  --------------------------------------------------
Zm_TC299056    (667)  TTCAGAAACACTTGGTTCAACATCATACAAGGCAACATTTAGAGATGAAT
  Consensus    (851)   TC GAA    CT    TTC         TACAA GC   C TTT G GATGA
```

## FIGURE 1 (continued)

```
                        901                                               950
Gm_TC229774       (1)   --------------------------------------------------
 Gs_TC30779     (780)   CA------TCAAGTTCAAAGAAGTTTGAAGCTACTGTTACTCGCCTTCAC
Hv_TC134680     (150)   TC------GTTGAACCAAAGAAGATGGAAGCAATAGTAGCCAGGTTACTC
Le_TC158378       (1)   --------------------------------------------------
Le_TC166426       (1)   --------------------------------------------------
  Ls_TC15801    (402)   CT------TCTAATTTAAAGAATCTTCAAGCCACTGTTACCAGTCTCCAT
  Mt_TC96175    (838)   CCTCAACTTCAAGTTCAAAGAAGTTTGAAGCTACTGTCACACGCCTTCGC
OS_SEQ ID 1     (578)   TT------ACCGACCCAAAGACCATTGAAGCAATAGTATCTCGGTTGCTC
Sb_TC103780     (756)   TT------ATTGATACAAAGACCACTGAAGCAACGGTAGCTCGATTACTC
  So_TC67974     (48)   TT------ATTGATACAAAGACCACTGAAGCAACGGTAGCTCGATTACTC
St_TC117743     (700)   CA------ACTGGTACAAAGAAGCTTGAAGCCACTGTAACCCGGCTTCAT
Ta_TC257477       (1)   --------------------------------------------------
Zm_TC299056     (717)   TT------ATTGATACGAGGACCACTGAAGCAACAGTAGCTCGATTACTC
  Consensus     (901)                 T CAAAGA    TGAAGC AC GT  C CG  T C C

                        951                                              1000
Gm_TC229774       (1)   --------------------------------------------------
 Gs_TC30779     (824)   CCATCCACTCAGGGTTTAAGGGAATTTATAAACGAAGTAAACACTCTTGC
Hv_TC134680     (194)   CCTTCCAATCAGAGTTTCAAAGAATTTAAGGCACAAGTAAATACGTTGGC
Le_TC158378       (1)   --------------------------------------------------
Le_TC166426       (1)   --------------------------------------------------
  Ls_TC15801    (446)   CCTTCAACTCAGGGTTTGAAGGAATTTGTGAGTGAAGTGAACACGCTAGC
  Mt_TC96175    (888)   CCATCATCTCAGGGCCTGAAGGAATTCATAAATGAGGTTAATACTCTTGC
OS_SEQ ID 1     (622)   TCCTCCACTCAGAGTTTGAAAGAGTTTAAAACACAAGTGAATACCTTGGC
Sb_TC103780     (800)   CCCTCCACTCAGAGTTTGAAGGAGTTTAAAACACAAGCAACTACATTGGC
  So_TC67974     (92)   CCCTCCACTCAGAGTTTGAAGGAGTTTAAAACACAAGCGACCACATTGGC
St_TC117743     (744)   CCTTCTTCGCAGGGGTTGAAGGAATTTGTGACTGAGGTGAACACACTAGC
Ta_TC257477       (1)   --------------------------------------------------
Zm_TC299056     (761)   CCCTCCACTCAGAGTTTGAAGGAGTTTAAAACGCAAGCGACCACATTGGC
  Consensus     (951)   CC TC ACTCAG GTTTGAAGGA TTTA  A   AAG  A  AC  T GC

                        1001                                             1050
Gm_TC229774       (1)   --------------GTAAATTGCTAGGATTTC-AT------GCACGA--G
 Gs_TC30779     (874)   ATCATTGCAACATCCAAATCTCTGTAAATTGCTTGGGTACCATGCAC--G
Hv_TC134680     (244)   ATCACTTCAACATCCCAATTTATGTAAACTCATCGGCTATCACGCAA--G
Le_TC158378       (1)   --------------------------------------------------
Le_TC166426       (1)   --------------------------------------------------
  Ls_TC15801    (496)   ATCATTGCAACACCCTTATCTCTGTAAATAGATTGGTTTCCATGCGC--G
  Mt_TC96175    (938)   ATCATTGCAGCATCCGAACCTCTGTAGATTGCTAGGATTTCACGCAG--G
OS_SEQ ID 1     (672)   ATCACTTCAGCATCCCAACTTATGTAAACTAATCGGCTTTCACGCAA--G
Sb_TC103780     (850)   ATTGCTTCAGCATCCCAATTTATGTAAACTGATTGGCTATTATGCAA--A
  So_TC67974    (142)   ATCACTTCAGCATCCCAATTTGTGTAAACTGATTGGCTATTATGCAA--A
St_TC117743     (794)   TTCTTTGCAACATCCATCACTTTGTAAACTGATTGGTTTCCATGCCACGG
Ta_TC257477       (1)   --------------------------------------------------
Zm_TC299056     (811)   ATCGCTTCAGCATCCCAATTTATGTAAACTGATTGGCTATTATGCAA--A
  Consensus    (1001)   ATC   T CA CATCC AA   T TGTAAA TGAT GG T    A GCAA  G
```

FIGURE 1 (continued)

```
                           1051                                              1100
Gm_TC229774       (28)  AGG-GTTC-AGAACATAGAATGTTGGTTTATGAAAGGCTATACCATGGAA
 Gs_TC30779      (922)  TGATAATTCAGAACAACGAATGTTGGTCTATGAGAGGTTATTTCATGGAA
Hv_TC134680      (292)  AGAAGAATCTAATGAAAGGATGTTGGTCTATGAGCGGCTCCATCATGGCA
Le_TC158378        (1)  -------------------------------------------CATGGAA
Le_TC166426        (1)  -------------------------------GAGAGGCTTTTTCATGGAA
 Ls_TC15801      (544)  TGAGGGATCTGATAGAAGAATGTTGGTTTACGAGAGGCTTTTTCATGGAA
 Mt_TC96175      (986)  TGATGGTTCAGAGCATAGAATGTTGGTTTATGAGAGGCTATACCATGGAA
OS_SEQ ID 1      (720)  AGAAGAATCTAATGAAAGGATGTTGGTCTATGAGCGACTCCATCATGGCA
Sb_TC103780      (898)  AGAAGATTCTAATGAAAGGATGTTGGTCTATGAACGGCTTCATCATGGGA
 So_TC67974      (190)  AGAAGATTCTAATGAAAGGATGTTGGTCTATGAACGGCTTCATCATGGGA
St_TC117743      (844)  GGAAGGTTCCGAGCACAGAATGTTGGTTTATGAAAGGCTTTTTCATGGAA
Ta_TC257477        (1)  --------------------------------------------------
Zm_TC299056      (859)  AGAAGATTCTAATGAAAGGATGTTGGTCTATGAACGGCTTTATCATGGAA
  Consensus     (1051)   GA G TTC  A  AAAG ATGTTGGT TATGA  GGCT TATCATGGAA

                           1101                                              1150
Gm_TC229774       (76)  GCTTGGATCGCTTATTGTATGGGAGATCTGATGGGCCATCAATTGATTGG
 Gs_TC30779      (972)  GCTTAGACCGGCTTTTATACGGGAGATCGGATGGACCGCCACTTGATTGG
Hv_TC134680      (342)  GTTTAGACAGGTTACTCTTTGGAAGACCGGAAGGTCGTTTCATGGACTGG
Le_TC158378        (8)  GCTTACACCGGCTTTTATTTGGGAGGTCGGATGGTCCCCCAATAGACTGG
Le_TC166426       (20)  GCTTAGACAGACTTTTGTTCGGAAGATCAGATGGCCCCTCTATAGATTGG
 Ls_TC15801      (594)  GCTTAGATCGGCTTTTATATGGTACAACAGATGGCCCACCTATTGATTGC
 Mt_TC96175     (1036)  GCTTGGACCGCTTATTGTATGGGAGATCTGATGGGCCATCAATTGATTGG
OS_SEQ ID 1      (770)  GCTTAGATAAACTACTCTTTGGAAGATCGGATGGTCGTTTCATGGACTGG
Sb_TC103780      (948)  GCTTAGATAAGCTACTCTTTGGAAGACCAGATGGTCGTTTCATGGACTGG
 So_TC67974      (240)  GCTTAGATAAGCTACTCTTTGGAAGACCAGATGGTCGTTTCATGGACTGG
St_TC117743      (894)  GCTTAGACCGGCTTTTGTTTGGGAGGTCCGATGGTCCCCCGATAGACTGG
Ta_TC257477        (1)  --------------------------------------------------
Zm_TC299056      (909)  GCTTAGATAAGCTACTCTTTGGAAGATCAGATGGTCGTTTCATGGACTGG
  Consensus     (1101)  GCTTAGA  GGCTATT TTTGG AGATC GATGGTCC TC AT GACTGG

                           1151                                              1200
Gm_TC229774      (126)  AATACAAGAATGAAAATTGCTATATGTGCTGCA-CAAGGTCTAACCTTCT
 Gs_TC30779     (1022)  AATACTCGCATGAAAATTGCTTTATGTTCTGCACAGGGTCTT-ACTTTCT
Hv_TC134680      (392)  TCTACACGTTTGAAGGTTGCCCTTGGTGCTGCTAAAGGTCTA-GCTTTCC
Le_TC158378       (58)  AATGCTCGAATAAAAATTGCTTTATGTGCTGCTCAAGGTCTC-ACATTCC
Le_TC166426       (70)  AATGCGAGAACCAAAATTGCCCTATGTGCTGCACAAGGTCTT-ACATTTC
 Ls_TC15801      (644)  AATGCAAGAATGAAAGTCGCACTCTGTGCTGCTCAAGGGCTT-ACTTTTT
 Mt_TC96175     (1086)  AATACAAGAATGAAAATTGCAATATGTGCTGCACTAGGTCTT-TCTTTCT
OS_SEQ ID 1      (820)  TCAGCACGTTTGAAGGTTGCTCTTGGTGCTGCTAGAGGCCTG-GCTTTCC
Sb_TC103780      (998)  TCTAAACGTTTGAAGGTTGCCCTTGGTGCGGCCAGAGGTCTT-GCTTTCT
 So_TC67974      (290)  TCTAAACGTTTGAAGGTTTCCCTTGGTGCTGCCCGAGGTCTA-GCTTTCT
St_TC117743      (944)  AATGCTCGAACGAAAATTGCTTTATGTGCTGCTCAAGGTCTC-ACATTCC
Ta_TC257477        (1)  --------------------------------------------------
Zm_TC299056      (959)  TCTAAACGTTTGAAGGTTGCCCTGGGTGCTGCCAGAGGTCTA-GCTTTCT
  Consensus     (1151)  AATACACG ATGAAA TTGC CT TGTGCTGC C AGGTCT   CTTTCT
```

## FIGURE 1 (continued)

```
                   1201                                               1250
Gm_TC229774    (175) TGCACGAAGAGGGGCCTTTTCAGGCTATGTATAATGAGTTCTCAACAGCC
 Gs_TC30779   (1071) TGCATGAGGAAGGACCATTTCAGGCAATGTACAATGAATTTTCGACTGCC
Hv_TC134680    (441) TACACGATGAAGGACCCTTTCAGGCAATGTATGATGACTTCTCAACGTCA
Le_TC158378    (107) TGCATGAGGAAGGACCTTTTCAGGCAATGTTCCATGAATTTTCCACTGCA
Le_TC166426    (119) TACATGAGGAGGGACCTTTTCAGGCAATGTTCCAAGAATTTTCAACTGGA
 Ls_TC15801    (693) TGCATGAGGAAGGACCATTTCAGGCGATGTTTCATGAATTTTCCACTGCT
 Mt_TC96175   (1135) TGCATGAAGAAGGGCCTTTTCAGGCAATGTATAATGAATTTTCAACAGCT
OS_SEQ ID 1    (869) TACATGATGAAGGGCCTTTTCAGGCCATGTACAATGACTTCTCAACCTCA
Sb_TC103780   (1047) TGCATGATGAAGGACCCTTTCAGGCCATGTACAGCGAGTTCTCAACTTTG
 So_TC67974    (339) TGCATGATGAAGGACCCTTTCAGGCCATGTACAGTGAGTTCTCAACTTTG
St_TC117743    (993) TGCATGAGGAAGGACCTTTTCAGGCAATGTTCCATGAATTTTCCACTGCA
Ta_TC257477      (1) --------------------------------------------------
Zm_TC299056   (1008) TGCATGATGAAGGACCCTTTCAGGCCATGTACAGCGAGTTCTCAACTTTG
  Consensus   (1201) TGCATGA GAAGGACC TTTCAGGC ATGTACAATGA TT TCAACTGC

                   1251                                               1300
Gm_TC229774    (225) AACATACAGATTGACAAAGATTTCAGCGCAAAGCTTTCAGGATATGGTTG
 Gs_TC30779   (1121) AACATACAGATCGACAAGGATTTCAGTGCAAAGCTCTCAGGATATGGGTG
Hv_TC134680    (491) AACATCCAAATAGAGAAAGATTTCACTGCAAAGCTGTCGGGATATGGTTG
Le_TC158378    (157) AATATACAAATTGATAAGGATTGCAGTGCAAAGCTCTCGGGATATGGATG
Le_TC166426    (169) AATATACAAATCGACAAGGATTTTAGTGCAAAGCTCTCGGGGTATGGATG
 Ls_TC15801    (743) AATATACAAATTGATAAAGATTTTAG------------------------
 Mt_TC96175   (1185) AACATTCAGATTGACAAAGATTTCAGTGCAAAGCTTTCAGGATATGGTTG
OS_SEQ ID 1    (919) AACATCCAAATTGACAAAGATTTCACTGCAAAGCTATCAGGATATGGATG
Sb_TC103780   (1097) AACATCCAAATAGATAAAGATTTCACTGCTAAGCTTTCAGGATATGGTTG
 So_TC67974    (389) AACATCCAAATAGATAAAGATTTCACTGCTAAGCTTTCAGGATATGGTTG
St_TC117743   (1043) AATATACAAATTGATAAGCATTGCAGTGCAAAGCTCTCGGGATATGGATG
Ta_TC257477      (1) --------------------------------------------------
Zm_TC299056   (1058) AACATCCAAATAGATAAAGGTTTCACTGCTAAGCTTTCAGGATATGGTTG
  Consensus   (1251) AACAT CAAAT GA AAAGATTTCAGTGCAAAGCT TCAGGATATGG TG

                   1301                                               1350
Gm_TC229774    (275) TGTTGGACATATTCCCGAAGAAGAG---ATTTCAAGCAGCTCATCTG---
 Gs_TC30779   (1171) CGTTGGTCATATCCCAGAGACAGAAGAGATCTCCAGTAATTCAGTTGCTG
Hv_TC134680    (541) TGTTGGCTTCAATTCTGACGAG------GAAAGATCCAAGGCATCTG---
Le_TC158378    (207) CATTACTCATATACAAGAGACAGAC---ATATCATGCAGTTCAGCTG---
Le_TC166426    (219) CATTACGAATATACAAGAGACGGAG---ATATCTTGCAATTCAATCG---
 Ls_TC15801    (769) --------------------------------------------------
 Mt_TC96175   (1235) TGTTGGACATGTTCCGAAGCAAGAG---ATTTCAAGCAGTTCATCTG---
OS_SEQ ID 1    (969) TGTTGGATTCAATACCGAGGAGGAA---ATATCAAATGCATCTGTGG---
Sb_TC103780   (1147) TGTTGGCTTCAATACTGAGCAG------ATATCTAATGCACCTGCGT---
 So_TC67974    (439) TGTTGGCTTCAATACTGAGGAG------ATATCTAATGCACCTGCGT---
St_TC117743   (1093) CATTACTCCTATACCAGAGACAGAC---ATATCATGCAGTTCAGCTGCCC
Ta_TC257477      (1) --------------------------------------------------
Zm_TC299056   (1108) TGTTGGCTTCAATACCGAGGAG------ATATCTAATGCACCTGTGT---
  Consensus   (1301) TGTTGG    A T C GAGGA GA    ATATC A  A  TCAGC G
```

## FIGURE 1 (continued)

```
                              1351                                            1400
Gm_TC229774    (319)  --------------------------------------------------
 Gs_TC30779   (1221)  TGGCAAATATATCCGTAGAGACTCTGGAGAGAGGGCGACTAACTCCAAAG
Hv_TC134680    (582)  --------------------------------------------------
Le_TC158378    (251)  --------------------------------------------------
Le_TC166426    (263)  --------------------------------------------------
  Ls_TC15801   (769)  --------------------------------------------------
  Mt_TC96175  (1279)  --------------------------------------------------
OS_SEQ ID 1   (1013)  --------------------------------------------------
Sb_TC103780   (1188)  --------------------------------------------------
  So_TC67974   (480)  --------------------------------------------------
St_TC117743   (1140)  TGGCAATCTCT---------------------------------------
Ta_TC257477      (1)  --------------------------------------------------
Zm_TC299056   (1149)  --------------------------------------------------
   Consensus  (1351)

                              1401                                            1450
Gm_TC229774    (319)  --------------------------------------------------
 Gs_TC30779   (1271)  AGCAATGTTTGGAGTTTTGGGATCATTCTACTTGAATTACTCACTGGCCG
Hv_TC134680    (582)  --------------------------------------------------
Le_TC158378    (251)  --------------------------------------------------
Le_TC166426    (263)  --------------------------------------------------
  Ls_TC15801   (769)  --------------------------------------------------
  Mt_TC96175  (1279)  --------------------------------------------------
OS_SEQ ID 1   (1013)  --------------------------------------------------
Sb_TC103780   (1188)  --------------------------------------------------
  So_TC67974   (480)  --------------------------------------------------
St_TC117743   (1151)  --------------------------------------------------
Ta_TC257477      (1)  --------------------------------------------------
Zm_TC299056   (1149)  --------------------------------------------------
   Consensus  (1401)

                              1451                                            1500
Gm_TC229774    (319)  ------------------------------------------CTGTTGGA
 Gs_TC30779   (1321)  GAAGAACCTTGACAACCGTTATCCCAAGGAAGAGAGGAACCTAGTGAAGT
Hv_TC134680    (582)  ------------------------------------------TGGCTGCA
Le_TC158378    (251)  ------------------------------------------CCCTGGCA
Le_TC166426    (263)  ------------------------------------------CCCTGGCG
  Ls_TC15801   (769)  --------------------------------------------------
  Mt_TC96175  (1279)  ------------------------------------------CCGTTGGA
OS_SEQ ID 1   (1013)  ------------------------------------------CTGCTGCA
Sb_TC103780   (1188)  ------------------------------------------CTGCAGCA
  So_TC67974   (480)  ------------------------------------------CTGCAGCA
St_TC117743   (1151)  --------------------------------------------------
Ta_TC257477      (1)  --------------------------------------------------
Zm_TC299056   (1149)  ------------------------------------------CTGCAGCA
   Consensus  (1451)                                            C G  GCA
```

**FIGURE 1 (continued)**

```
                    1501                                              1550
Gm_TC229774   (327) AACCTATCAATGGAAACACTGGAGAAAGGAATGCTCACTCCAAAGAGCAA
 Gs_TC30779  (1371) GGAGCCGGCCATTCCTAGCCGACAATTGTAGATTGTCACTCATCATGGAT
Hv_TC134680   (590) AACCTCTCAGAGGAAACCTTGGAGAAAGGCGTACTGACTCCGAAGAGCAA
Le_TC158378   (259) AATCTCTCTGAGGAGACTCTGGAGCGAGGATTGGTAACTCCAAAGAGTAA
Le_TC166426   (271) AATCTCTCACAGGAGACACTGGAGAGAGGGTTGATAACTCCTAAGAGCAA
  Ls_TC15801  (769) --------------------------------------------------
  Mt_TC96175 (1287) AACCTATCGATGGAGACACTGGAGAAAGGAATGCTTACTCCGAAAAGCAA
OS_SEQ ID 1  (1021) AACCTCTCAGTGGAAACCTTGGAGAAAGGTGTACTGACTCCCAAGAGCAA
Sb_TC103780  (1196) AACCTTTCGGTGGAGACCTTGGAGAAGGGTTTACTCACTCCCAAGAGCAA
  So_TC67974  (488) AACCTTTCGGTGGAGACCTTGGAGAAGGGTTTACTCACTCCCAAGAGCAA
St_TC117743  (1151) --------------------------------------------------
Ta_TC257477     (1) --------------------------------------------------
Zm_TC299056  (1157) AACCTTTCGGTGGAGACCTTGGAGAAGGGTTTACTCACTCCCAAGAGCAA
   Consensus (1501) AACCT TC   GGA AC  TGGAGAA GG  T CT ACTCC AAGAGCAA

                    1551                                              1600
Gm_TC229774   (377) CGTATGGAGTTTTGGAATTTTTCTTCTGGAGCTACTTACTGGAAGAAAGA
 Gs_TC30779  (1421) CCTCAGCTCAAAGGTCGCTTTCCTATGAAAGCTGCCCGTACGGTGGCTGA
Hv_TC134680   (640) CGTATGGAGCTTTGGAGTTGTCTTGCTCGAGCTAATAACAGGACGGAAGA
Le_TC158378   (309) TGTTTGGAGTTTTGGGATTGTTCTTCTTGAGCTGCTGACTGGCCGGAAGA
Le_TC166426   (321) TGTTTGGAGTTTCGGGATTGTTCTTTTAGAACTGCTCACTGGCCGGAAGA
  Ls_TC15801  (769) --------------------------------------------------
  Mt_TC96175 (1337) TGTATGGAGTTTCGGAATTTTCCTTCTAGAGCTACTTACAGGAAGAAAGA
OS_SEQ ID 1  (1071) CGTATGGTGCTTTGGAGTTGTCCTGCTGGAGCTAATAACAGGAAGGAAGA
Sb_TC103780  (1246) TGTCTGGAGCTTTGGAGTTGTGTTACTGGAGCTAATAACTGGAAGGAAGA
  So_TC67974  (538) CGTCTGGAGCTTTGGAGTTGTGTTACTGGAGCTAATAACTGGAAGGAAGA
St_TC117743  (1151) --------------------------------------------------
Ta_TC257477     (1) --------------------------------------------------
Zm_TC299056  (1207) CGTCTGGAGCTTTGGAGTCGTGTTACTGGAGCTAATAACTGGAAGGAAGA
   Consensus (1551)  GT TGGAG TTTGGA TTGT  T CT GAGCTA T AC GGA GGAAGA

                    1601                                              1650
Gm_TC229774   (427) ATCTTGATAGCC-GTCACCCCAAGGAAGAGAGGAATTTAGTCAAGTGGAG
 Gs_TC30779  (1471) CATTGCACAAAGGTGTCTCCAGATGGACCCATCAGAGAGGCCAACCATGA
Hv_TC134680   (690) ACCTCGATGTAC-GTTCCACCAAAGAAGAACGTAATATTGTCAAGTGGGG
Le_TC158378   (359) ATTTAAGCAGTC-GGCATCCAAAGGAAGAGAGGAATTTAGTGAAGTGGAG
Le_TC166426   (371) ATCTTGATGGTC-GGTATTCAAAGGAAGAGAGGAATCTAGTCAAGTGGAG
  Ls_TC15801  (769) --------------------------------------------------
  Mt_TC96175 (1387) ATCTCGATAGCCCGTCACCCAAAGGAAGAGAGAAATTTGGTGAAGTGGAG
OS_SEQ ID 1  (1121) ACCTTGATGTCC-GTTCCTCAAAAGAAGAACGCAATATTGTCAAGTGGAG
Sb_TC103780  (1296) ACCTTGATGCCA-ATTCTTCTAAAGAAGAACGCAATATTGTCAGGTGGAG
  So_TC67974  (588) ACCTTGATGCCA-ATTCTTCCAAAGAAGAACGCAATATTGTCAGGTGGAG
St_TC117743  (1151) --------------------------------------------------
Ta_TC257477     (1) --------------------------------------------------
Zm_TC299056  (1257) ACCTTGATCCCA-ATTATTCCAAAGAAGAACGCAATATTGTCAACTGGAG
   Consensus (1601) A CT GAT       TT   C AA GAAGA  G AAT T GTCAAGTGGAG
```

**FIGURE 1 (continued)**

```
                    1651                                              1700
Gm_TC229774   (476) CCGGCCTTTCTTAGCCGATAACTACCGTCTGTCGTTGATCATGGATCCTC
 Gs_TC30779  (1521) GAACCATCGTTGAGCATCTCAAAATCATCCAAGACCTGAAATACTCTTGT
Hv_TC134680   (739) TAGGCCTTTCCTCACCGACGATAGTCGTCTATCCCTCATCATGGATCCCC
Le_TC158378   (408) CAAGCTTTTTCTAGCTGATGACAGTAGATTATCGCTAATCATGGACCCTC
Le_TC166426   (420) TAGGCCTTTCCTTGCTGATGATGGTAGATTATCGCTTATCATGGATCCTC
 Ls_TC15801   (769) --------------------------------------------------
 Mt_TC96175  (1437) CAGGCCTTTCCTGTCTGATAATCATCGTTTGTCAATGATCATGGATCCTC
OS_SEQ ID 1  (1170) TAGGCCTTTCCTCACCGATGATAGTCGCCTATCGTTAATCATGGACTCCC
Sb_TC103780  (1345) TAGGCCTTTCCTTACTGATGATAGTCGCCTATCTCTGATCATGGACTCCC
 So_TC67974   (637) TAGGCCTTTCCTTACTGATGATAGTCGCCTATCTCTGATCATGGACTCCC
St_TC117743  (1151) --------------------------------------------------
Ta_TC257477     (1) --------------------------------------------------
Zm_TC299056  (1306) TAGGCCTTTCCTTACTGATGACAGTCGCTTGTCTCTTATCATGGACTCCC
  Consensus  (1651)  AGGCCTTTCCT  C GATGA AGTCG  TATC CT ATCATGGA  C C

                    1701                                              1750
Gm_TC229774   (526) AACTCAAAGGCCGCTTTCCTTCTAAAGCAGCAAGAACAATAGCTGATATT
 Gs_TC30779  (1571) CGGTTTCCTCTGCAAGATCCAGCAGCAATTGCTGGAAAACAGATGTCAAG
Hv_TC134680   (789) GTATAAAAGGACGCTTTCCTACCAAGGCTGCTCGAACTGTGGCAGACATA
Le_TC158378   (458) AGTTAAAAGGTCGGTTCCCTGCCAAAGCAGCAAGAACTGTGGCTGATATT
Le_TC166426   (470) AGCTTAAAGGACGGTTTCCCGCAAAAGCAGCCCGTACAGTGGCTGATATT
 Ls_TC15801   (769) --------------------------------------------------
 Mt_TC96175  (1487) AACTTAAAGGTCGCTTTCCTTCTAAAGCGGCGAGTACAATAGCTAACATT
OS_SEQ ID 1  (1220) GTATAAAAGGACGCTTCCCTACCAAGGCTGCTCGGATTGTAGCAGATATC
Sb_TC103780  (1395) GTATAAAAGGGCGCTTTCCTACTAAGGCTGCTCGGATAGTAGCAGACATC
 So_TC67974   (687) GTATAAAAGGGCGCTTTCCAACTAAGGCTGCTCGGATAGTAGCAGACATC
St_TC117743  (1151) --------------------------------------------------
Ta_TC257477     (1) --------------TTTCCTCCCAAGGCTGCTAGGACTGTGGCAGACATC
Zm_TC299056  (1356) GTATAAAAGGGCGCTTTCCCACTAAGGCTGCTCGGATAGTAGCAGACATC
  Consensus  (1701)    T AAAGG CGCTTTCCT C AA GCTGC  G A AGTAGC GACAT

                    1751                                              1800
Gm_TC229774   (576) GCACAAAGATGCCTTCAAAAGGAGCCATCAGACAGACCTACCATGAGGAC
 Gs_TC30779  (1621) GTCACCGAGTCTCAACG---------------------------------
Hv_TC134680   (839) ATTCTGAAGTGCCTTCAAAGAGATCCATCAGAAAGGCCTACGATGAGGGC
Le_TC158378   (508) GCTCAAAGATGTCTGCAAAAGGATCCATCTGAAAGGCCCACCATGAGAAC
Le_TC166426   (520) GCCCAAAGATGCCTGCAAAAGGATCCATCTGAAAGGCCCACCATGAGAAC
 Ls_TC15801   (769) --------------------------------------------------
 Mt_TC96175  (1537) GCACAAAGATGCCTTCAAATGGAGCCATCAGAACGACCAACCATGGGAAC
OS_SEQ ID 1  (1270) ATATTGAGATGCCTTAATAAAGATCCATCAGAGAGGCCTACCATGAGGGC
Sb_TC103780  (1445) ATTCTGAAATGCTTGCATAATGATCCATCCGAGAGGCCGACCATGAGGGA
 So_TC67974   (737) ATTCTGAAATGCTTGCATAAAGATCCATCAGAGAGGCCGACCATGAGGGA
St_TC117743  (1151) --------------------------------------------------
Ta_TC257477    (37) ATTCTGAAGTGCCTTCATAGGGATCCATCCGAAAGGCCTACGATGAGGGC
Zm_TC299056  (1406) ATTTTGAAATGCCTGCGTAAGGATCCATCGGAGAGGCCTACCATGAGGGA
  Consensus  (1751)  T C  A ATGCCT CA AA GATCCATC GA AGGCC ACCATGAGG C
```

**FIGURE 1 (continued)**

```
                         1801                                              1850
Gm_TC229774     (626) TGTTGTTGAGCATCTCAAGATAATACAGGATTTGAAATATTCGTGCCGGT
 Gs_TC30779    (1638) --------------------------------------------------
Hv_TC134680     (889) CGTCGTGGAGGCCCTAACAAGCGTTCAGGACATAAAGGTCCCCTGCCGGT
Le_TC158378     (558) TGTAGTGGAGCAACTCAAGACTGTACAAGTTATGAAGTACCCTTCTCGGT
Le_TC166426     (570) TATCTTGGACCAACTCAAATCCGTACAAGTGATGAAGTGCCCTTCACGGT
 Ls_TC15801     (769) --------------------------------------------------
 Mt_TC96175    (1587) AGTTGTTGAGCAGCTGAAAAAGATACAAGATTTGAAGCATTCTAGCAGAT
OS_SEQ ID 1    (1320) CGTTGTGGAGTCCCTAGCAAGCGTCCAGGACATAAAGGTTCCATGTCGAT
Sb_TC103780    (1495) TGTTGTGGAGGCTCTAGCAAGAGTCCAGGAGATAAAGGTTCCGTGCAGAT
 So_TC67974     (787) TGTTGTGGAGGCCCTAGCAAGAGTCCAGGAAATAAAGGTTCCATGCAGAT
St_TC117743    (1151) --------------------------------------------------
Ta_TC257477      (87) CGTCGTGGAGTCTCTAGCAAGCGTCCAGGACATAAAGGTCCCCTGCCGGT
Zm_TC299056    (1456) TGTTGTGGAGGCCCTAGCAAGAGTCCAGGAAATAAAGGTTCCGTGCAGAT
  Consensus    (1801)  GT GTGGAG   CT   AA  GT CAGGA AT AAG   CC TGC G T

                         1851                                              1900
Gm_TC229774     (676) TCCCTCTGCAAGAACCAGCATCAAACTCT--GGAAAACATATGTCAAGAT
 Gs_TC30779    (1638) --------------------------------------------------
Hv_TC134680     (939) ATCCTCTTCAAGTACCGTCCCGCCGCACCCGAGGAAAGTGATGCTAAAGT
Le_TC158378     (608) TTCCTCTTCAAGAGCCAAGG-GCAGTTGG-TGTAAAACACATGTCAAAGT
Le_TC166426     (620) TTCCTCTGCAAGAACCAGCG-GTTGTTGG-TGGTAAACACATGTCAAAGT
 Ls_TC15801     (769) --------------------------------------------------
 Mt_TC96175    (1637) TCCCGCTGCAAGAACCTGCA-----------------CAAATGTCGAGCAT
OS_SEQ ID 1    (1370) ATCCTTTGCAAGAGCCATCT-GCTGCCCC-AAGAAAGGTGATGTTAAAAT
Sb_TC103780    (1545) ATCCTTTGCAAGAACCTTCT-GCTGCACC-AAGAAAAGTAATGCTAAAAT
 So_TC67974     (837) ATCCTCTGCAAGAACCTTTT-GCTGCACC-AAGAAAAATAATGTTAAAAT
St_TC117743    (1151) --------------------------------------------------
Ta_TC257477     (137) ATCCTCTTCAAGTACCGTCC-GCCGCACC-GAGGAAAGTGATGCTAAAAT
Zm_TC299056    (1506) ATCCTCTGCAAGAACCTTCT-GCTGCACC-AAGAAAAGTGATGTTGAAAT
  Consensus    (1851)  TCCTCTGCAAGAACC  C  GC GC C    G AAA   ATGT AAAAT

                         1901                                              1950
Gm_TC229774     (724) CACCAAGTCTCAATGGCATCATCTGCCCTGCACCAAGGGTGAGTTT----
 Gs_TC30779    (1638) --------------------------------------------------
Hv_TC134680     (989) CTACGAGCCTCAATGGCATTGTTCCTCAGCATCCCGTCATGACCTTCTCG
Le_TC158378     (656) GTCCGAGCCTAAATGGAATTATTACCCCAACATCAAGATTGAGCTTCTCC
Le_TC166426     (668) CTCCAAGCATGAAT------------------------------------
 Ls_TC15801     (769) --------------------------------------------------
 Mt_TC96175    (1670) CACCAAGTCTTAACGGTATCAACCACCCTGCACCAAGGCCGAGTTTCTCT
OS_SEQ ID 1    (1418) CTACAAGTCTCAATGGCATCATTCATCACCATCCTGTCGTAACCTTCTCA
Sb_TC103780    (1593) CTACATCCCTCAATGGAATTGTGCCTCATCATCCTGTCATAACCTTCTCT
 So_TC67974     (885) CTACATCCCTCAATGGAATTGTGCCTCATCATCCTGTCATAACCTTCTCC
St_TC117743    (1151) --------------------------------------------------
Ta_TC257477     (185) CCACGAGTCTCAACGGCATTGTTCCTCAGCATCCTGTCATGACCTTCTCG
Zm_TC299056    (1554) CTACATCCCTCAATGGGATTGTGCCTCACCATCCTGTCATAACCTTCTCT
  Consensus    (1901) CT CAAG CTCAATGG AT  T C  C     CC      T A CTTCTC
```

## FIGURE 1 (continued)

```
                        1951                                              2000
Gm_TC229774     (770)   --------------------------------------------------
 Gs_TC30779    (1638)   --------------------------------------------------
Hv_TC134680    (1039)   CCGTCGCCTCCTTCGCGCAACCAGCACCTGGCGTCGCC---AAGATCATC
Le_TC158378     (706)   CCTTCACCACCAACCC---ACCCTATATCTATTTCTCC---GACAAGGAC
Le_TC166426     (682)   --------------------------------------------------
 Ls_TC15801     (769)   --------------------------------------------------
 Mt_TC96175    (1720)   CCATCACCATCATCCAGAGCCCTGGTATCTGTCTCACCTCCAAGATGGTC
OS_SEQ ID 1    (1468)   CCGTCACCTCCTTCGCGAAACCAACATTTGCTCTCGCC---AAGGTCATC
Sb_TC103780    (1643)   CCATCGCCGCCTTCGCATAACCAGCACTTGATTTCACC---AAGGTCATC
 So_TC67974     (935)   CCTTCGCCGCCTTCACATAACCAACACTTGATTTCACC---AAGGTCATC
St_TC117743    (1151)   --------------------------------------------------
Ta_TC257477     (235)   CCGTCGCCTCCTTCGCGCAACCAGCACCTGGTGTCACC---GAGATCATC
Zm_TC299056    (1604)   CCATCGCCGCCTTCACATAACCAGCACTTGATTTCACC---AAGGTCGTC
  Consensus    (1951)   CC TC CC CC TC C   ACC          T TC CC    AG T  TC

                        2001                                              2050
Gm_TC229774     (770)   --------------------------------------------------
 Gs_TC30779    (1638)   --------------------------------------------------
Hv_TC134680    (1086)   GACTTCCGCGCTTCTTCCCCCAAGGACCTGCTCTTCCATCATCACCCTGG
Le_TC158378     (750)   AGCTGCTCCACTGCTGTCTCTACCTTCATGTTCCTCCATCCTCTCTATGG
Le_TC166426     (682)   --------------------------------------------------
 Ls_TC15801     (769)   --------------------------------------------------
 Mt_TC96175    (1770)   GGGAGTGTCAATTCAACTTCCACCTCGCGCGTTTTCTTCAACCCTCTATT
OS_SEQ ID 1    (1515)   CACGTCTGCACTGCTTCCTCCAAGGACCAGCTGTGCT---------CTGG
Sb_TC103780    (1690)   AACATCTGCCTTGTTTCATCCAAGGACATGCTCTTCTA------CTCTGG
 So_TC67974     (982)   AACATCTGCCTTGTTTCATCCAAGGACATGCTCTTCTA------CTCTGG
St_TC117743    (1151)   --------------------------------------------------
Ta_TC257477     (282)   GACGTCTGCGCTGCTTCCCCCAAGGAACTGCTCTTCCACCATCACCCTGG
Zm_TC299056    (1651)   GACATCTGCCTTGTTTCATCCAAGGGCATGCTCTTCTA------CCCTGG
  Consensus    (2001)     C  C  C  T  T C  CCA       G T TTC            TGG

                        2051                                              2100
Gm_TC229774     (770)   --------------------------------------------------
 Gs_TC30779    (1638)   --------------------------------------------------
Hv_TC134680    (1136)   ATTACCCCAGGGTAAGCTCGGTCAAGAAGTCGCCTTCCGGTATCCTGCGG
Le_TC158378     (800)   AGGACTTTGATCGATTGGAAAATCGAAGGCCATCATCTTCATCTGTTCGG
Le_TC166426     (682)   --------------------------------------------------
 Ls_TC15801     (769)   --------------------------------------------------
 Mt_TC96175    (1820)   TGGAGGAGCTTGATAGGCAAGAAAGCCGCAAGTCAGCTTCAGCCTCTAGG
OS_SEQ ID 1    (1556)   ATGACCCTAGAGTAAGCTCTATCAAGAAATCGCCTTCCCCTATTTTACGG
Sb_TC103780    (1734)   ATGATCCCGGTGTAAGCTCTATAAAGAAAACACCTCC---TATTATGCGT
 So_TC67974    (1026)   ATGATCCCGGTGTAAGCTCTATAAAGAAAACACCTCC---TATTATGCGT
St_TC117743    (1151)   --------------------------------------------------
Ta_TC257477     (332)   ACTACCCCAGGGTAAGCTCGGTCAAGAAATCGCCCCCAACATCATGCGG
Zm_TC299056    (1695)   ACGATCCGAGTGTAAGTTCTATAAAGAAAACACCACC---TATTATGCGA
  Consensus    (2051)   A  A       G AAG       A  A   C  C  C         T CG
```

FIGURE 1 (continued)

```
                         2101                                              2150
Gm_TC229774     (770)    --------------------------------------------------
 Gs_TC30779    (1638)    --------------------------------------------------
Hv_TC134680    (1186)    AGACCTGGCGTCGAGGGTTTTTGATTGTCCATACATGGTCGGATTT-CTT
Le_TC158378     (850)    AGGTCTAGTGTCGAAGGATTTGATCGATTGTAGAGCACTT--TTTTCTT
Le_TC166426     (682)    --------------------------------------------------
 Ls_TC15801     (769)    --------------------------------------------------
 Mt_TC96175    (1870)    AAGGCTAGTGTTGAAGGATTTTGATTGTCGATAGTGTTCATTTTTTATTT
OS_SEQ ID 1    (1606)    AGATCTGGTGTTGAGGGTTTTTGA--------------------------
Sb_TC103780    (1781)    AGGTCTAGCGTTGAAGGCTTTTGATTGTCCATATTGT-TC---TTTTATT
 So_TC67974    (1073)    AGGTCTAGCGTCGAAGGCTTTTGATTGTCCATATTGT-TC---TTTTATT
St_TC117743    (1151)    --------------------------------------------------
Ta_TC257477     (382)    AGACCTGGCGTCGAGGGTTTTTGATTGTCCATATAGTGTCGGATTTTCTT
Zm_TC299056    (1742)    AGGTCTAGCGTCGAAGGCTTTTGATTGTCCATATTGT-TC---CTTTATT
  Consensus    (2101)    AG  CT G GT GA GG TTTTGAT G    TA            TT  TT
```

```
                         2151                                              2200
Gm_TC229774     (770)    --------------------------------------------------
 Gs_TC30779    (1638)    --------------------------------------------------
Hv_TC134680    (1235)    CTCTTTGCCTTGGATTTATTTGTTGTTTTGGTTAGCCTAGC--------G
Le_TC158378     (898)    CAAATTGCTTTTC-------------------------------------
Le_TC166426     (682)    --------------------------------------------------
 Ls_TC15801     (769)    --------------------------------------------------
 Mt_TC96175    (1920)    GTTATTCTTTTTTTGGTGTAGTTCAACAGAGATTTATAGGAGATAAAGAT
OS_SEQ ID 1    (1630)    --------------------------------------------------
Sb_TC103780    (1827)    -TCTTTTTCTCGGATTTATTTGTTTCTTTG-TTAGCCTAGT----GATTT
 So_TC67974    (1119)    -TCCTTTTCTTGGATTTATTAGTTTCTTTG-GTAGCCTAGT----GATTC
St_TC117743    (1151)    --------------------------------------------------
Ta_TC257477     (432)    CTCTTTGCCTTG-ATTTATTTGTTGTTTTGGTTAGCCTAGC--------G
Zm_TC299056    (1788)    -TCCTTTTCTTGGATTTATTTGTTTCTTTG-TTAGTAGTGTAGCGGTTTT
  Consensus    (2151)        TT   T
```

```
                         2201                                              2250
Gm_TC229774     (770)    --------------------------------------------------
 Gs_TC30779    (1638)    --------------------------------------------------
Hv_TC134680    (1277)    A----GTGGTCTCAGTGCTATG------TGATATGTATATGTTGGAA---
Le_TC158378     (911)    --------------------------------------------------
Le_TC166426     (682)    --------------------------------------------------
 Ls_TC15801     (769)    --------------------------------------------------
 Mt_TC96175    (1970)    TTGTAATCATCCCTCAGTGTGATGCAGAGAGGATGCTCTTTTGTACATAG
OS_SEQ ID 1    (1630)    --------------------------------------------------
Sb_TC103780    (1871)    TAGCTGTGTTCTGTATTGTAAGACAAGTGGCCTTA--TGTAGTGCCCTTG
 So_TC67974    (1163)    TAGCTATGTTCTGTATTGCAAGACAAGCGGCCTTAA-TGTAGTGCCCTTG
St_TC117743    (1151)    --------------------------------------------------
Ta_TC257477     (473)    A----GTGATCTCAGTGCTATG------GGATATAC-TATGTTGCAA---
Zm_TC299056    (1836)    TAGCTGTGTTCTGTATTGTAAGACAAGTGGCCTCATATGTAGTGCCCTTG
  Consensus    (2201)
```

**FIGURE 1 (continued)**

```
                           2251                                              2300
Gm_TC229774     (770)  --------------------------------------------------
 Gs_TC30779    (1638)  --------------------------------------------------
Hv_TC134680    (1314)  AGACATCTGAAAAAA--GGGCAGAGT--GAAGTGTAGATAGTAGAACCAG
Le_TC158378     (911)  --------------------------------------------------
Le_TC166426     (682)  --------------------------------------------------
 Ls_TC15801     (769)  --------------------------------------------------
 Mt_TC96175    (2020)  TGCAAAGGTTGGTCCCCTTGGTTCAATTAGTTACTCCATTTTG-------
OS_SEQ ID 1    (1630)  --------------------------------------------------
Sb_TC103780    (1919)  AGTCTCGAGTAATAACTAAGCAGAGCAGGATGAATTGTAAATAGTATCAG
 So_TC67974    (1212)  GGTCTAGAGTAATAACTAAGCAGAGCAGGATGAATTGTAAATAGGATCAG
St_TC117743    (1151)  --------------------------------------------------
Ta_TC257477     (509)  GGACATGTGAAAAA---GGGCAGAGT--GAAGCGTAGATAGTAGAACCAG
Zm_TC299056    (1886)  GGTCTGGAGTAA-----A-GCAGAACAGGATGAATTGTAAATAGGACCAG
  Consensus    (2251)

                           2301                                              2350
Gm_TC229774     (770)  --------------------------------------------------
 Gs_TC30779    (1638)  --------------------------------------------------
Hv_TC134680    (1360)  CTTTGTAGATACTTGTCGTTCTCTGATTGGATGGGGGTCATGGAAGCAGT
Le_TC158378     (911)  --------------------------------------------------
Le_TC166426     (682)  --------------------------------------------------
 Ls_TC15801     (769)  --------------------------------------------------
 Mt_TC96175    (2063)  --------------------------------------------------
OS_SEQ ID 1    (1630)  --------------------------------------------------
Sb_TC103780    (1969)  GTT-GTAGATACCTTTT-T---GTGCTCTAATTGGG----TGGAAGCAGC
 So_TC67974    (1262)  GTT-GTAGATACCTTTT-T---GTGCTCTAATTGGG----TGGAAGCAGC
St_TC117743    (1151)  --------------------------------------------------
Ta_TC257477     (554)  CTT-GTAGATACTTGTCGTTCTCTGATTGGATGGGGGTCATGGAAGAAGT
Zm_TC299056    (1930)  GTT-GTAGATACCTTTT-TTTGTGCTCGAATTGGG----TGGAAGCAGC
  Consensus    (2301)

                           2351                                              2400
Gm_TC229774     (770)  --------------------------------------------------
 Gs_TC30779    (1638)  --------------------------------------------------
Hv_TC134680    (1410)  GTG---TGTTAGAGTGGGGCTTGTAACCCCATTATTATGCAATAAACGTG
Le_TC158378     (911)  --------------------------------------------------
Le_TC166426     (682)  --------------------------------------------------
 Ls_TC15801     (769)  --------------------------------------------------
 Mt_TC96175    (2063)  --------------------------------------------------
OS_SEQ ID 1    (1630)  --------------------------------------------------
Sb_TC103780    (2010)  GTGCTGAGTCTGAGTAGGCCTTGTAACCTCATAA-------ATAAAC---
 So_TC67974    (1303)  GTGCTAAGT--GAG----CCTTGTAACCTCCCAAT---GCAATAAAC---
St_TC117743    (1151)  --------------------------------------------------
Ta_TC257477     (603)  GTG---TGTT-GAGTGGGGCTTGTAACCCCACTA---TGCAATAAACGTG
Zm_TC299056    (1974)  GTGCCGAGT--GGG----CCTTGTAACCTCATAAT---GCAATAAAC---
  Consensus    (2351)
```

**FIGURE 1 (continued)**

```
                          2401                                             2450
Gm_TC229774     (770) --------------------------------------------------
  Gs_TC30779   (1638) --------------------------------------------------
 Hv_TC134680   (1457) GGTTGGTCGGTCG-----TATTTGCTCG----------------------
 Le_TC158378    (911) --------------------------------------------------
 Le_TC166426    (682) --------------------------------------------------
  Ls_TC15801    (769) --------------------------------------------------
  Mt_TC96175   (2063) --------------------------------------------------
 OS_SEQ ID 1   (1630) --------------------------------------------------
 Sb_TC103780   (2050) --TC-GTTTCCCAG----TTTCTGTCC-----------------------
  So_TC67974   (1341) --TC-GTTTTTCAG----TTTTTGTTC-----------------------
 St_TC117743   (1151) --------------------------------------------------
 Ta_TC257477    (646) GGTTGGTTGGTCGGTCGGTATTTGCTCGCCGTCAAACATTTCAGCTGTTT
 Zm_TC299056   (2012) --TCCGTTTCCCAG----TTTCTGTAAAAAAAAA----------------
   Consensus   (2401)

                          2451                                         2496
Gm_TC229774     (770) ----------------------------------------------
  Gs_TC30779   (1638) ----------------------------------------------
 Hv_TC134680   (1480) ----------------------------------------------
 Le_TC158378    (911) ----------------------------------------------
 Le_TC166426    (682) ----------------------------------------------
  Ls_TC15801    (769) ----------------------------------------------
  Mt_TC96175   (2063) ----------------------------------------------
 OS_SEQ ID 1   (1630) ----------------------------------------------
 Sb_TC103780   (2070) ----------------------------------------------
  So_TC67974   (1361) ----------------------------------------------
 St_TC117743   (1151) ----------------------------------------------
 Ta_TC257477    (696) CTTTCTTCTCTTGAATGCTAAGTTTTGTTGCTGCTAGTCGTGGAAT
 Zm_TC299056   (2040) ----------------------------------------------
   Consensus   (2451)
```

**FIGURE 1 (continued)**

```
                        1                                                50
OS_BAD73441      (1)   --MGCFTVLRSKKKKPLALTKKSVDARESTSSRLPEP-EAHVPSLQSAPP
   SEQ ID 2      (1)   -MMGCFTVLRSKKKKPLALTKKSVDARESTSSRLPEP-EAHVPSLQSAPP
   SEQ ID 4      (1)   MVLGCFPLK-SKKKR-GSVSMKRLDLEESKPTALPEPPKIPSRNLQSAPP
  Consensus      (1)    MMGCFTVLRSKKKKPLALTKKSVDARESTSSRLPEP EAHVPSLQSAPP

                        51                                              100
OS_BAD73441     (48)   SFRNKAKIHQSEKK---ASYSRARVLSAPSSLIVVDQDGLPYAEFDDQDD
   SEQ ID 2     (49)   SFRNKAKIHQSEKK---ASYSRARVLSAPSSLIVVDQDGLPYAEFDDQDD
   SEQ ID 4     (49)   SFRTRVKPIQSNNGGTGEMSSRARVMSAPSSIHGAAERDLLAGVYHDEQD
  Consensus     (51)   SFRNKAKIHQSEKK   ASYSRARVLSAPSSLIVVDQDGLPYAEFDDQDD

                        101                                             150
OS_BAD73441     (95)   SRGKGGSIKGHRFSNPLPLPLPSP-EGKSLRNFGSFKAINASG-----PL
   SEQ ID 2     (96)   SRGKGGSIKGHRFSNPLPLPLPSP-EGKSLRNFGSFKAINASG-----PL
   SEQ ID 4     (99)   EQPR-DPRTSTKESSPQPLPLPSPRTGSSLKNWGSFKSFNGSSGRLSSSA
  Consensus    (101)   SRGKGGSIKGHRFSNPLPLPLPSP EGKSLRNFGSFKAINASG     PL

                        151                                             200
OS_BAD73441    (139)   DASGPLPLPPKKCDGLKNFSYEELSSACQWFSGDQCVSESLTSTSYKASF
   SEQ ID 2    (140)   DASGPLPLPPKKCDGLKNFSYEELSSACQWFSGDQCVSESLTSTSYKASF
   SEQ ID 4    (148)   AVSGPLPLPPS--GSVRSFSYDEVMAACNAFSSDRCVMEGLSSVMYMASF
  Consensus    (151)   DASGPLPLPPKKCDGLKNFSYEELSSACQWFSGDQCVSESLTSTSYKASF

                        201                                             250
OS_BAD73441    (189)   RDDFTDP--KTIEAIVSRLLSSTQSLKEFKTQVNTLASLQHPNLCKLIGF
   SEQ ID 2    (190)   RDDFTDP--KTIEAIVSRLLSSTQSLKEFKTQVNTLASLQHPNLCKLIGF
   SEQ ID 4    (196)   GDEASTSGLKKVDATVVRLHVITQSIREFINEVNTLASLQHQNLCKLVGY
  Consensus    (201)   RDDFTDP  KTIEAIVSRLLSSTQSLKEFKTQVNTLASLQHPNLCKLIGF

                        251                                             300
OS_BAD73441    (237)   HAREESNERMLVYERLHHGSLDKLLFGRSDGRFMDWSARLKVALGAARGL
   SEQ ID 2    (238)   HAREESNERMLVYERLHHGSLDKLLFGRSDGRFMDWSARLKVALGAARGL
   SEQ ID 4    (246)   HARDGSDTRMLVYERLALGSLDRLLHGRSDGPPLDWNTRMKIALCAAQGL
  Consensus    (251)   HAREESNERMLVYERLHHGSLDKLLFGRSDGRFMDWSARLKVALGAARGL

                        301                                             350
OS_BAD73441    (287)   AFLHDEGPFQAMYNDFSTSNIQIDKDFTAKLSGYGCVGFNTEEEISNASV
   SEQ ID 2    (288)   AFLHDEGPFQAMYNDFSTSNIQIDKDFTAKLSGYGCVGFNTEEEISNASV
   SEQ ID 4    (296)   TFLHEEGPFQAMYNEFSTANIQVDKDFSAKLSGYGCAGHAPETETSNS-S
  Consensus    (301)   AFLHDEGPFQAMYNDFSTSNIQIDKDFTAKLSGYGCVGFNTEEEISNASV
```

# FIGURE 2

```
                     351                                               400
OS_BAD73441   (337)  AAANLSVETLEKGVLTPKSNVWCFGVVLLELITGRKNLDVRSSKEERNIV
    SEQ ID 2  (338)  AAANLSVETLEKGVLTPKSNVWCFGVVLLELITGRKNLDVRSSKEERNIV
    SEQ ID 4  (345)  ALANLSVETLERGILTPKSNVWSYGIVLLEMLTGRKNMDGSYPKEERNIV
   Consensus  (351)  AAANLSVETLEKGVLTPKSNVWCFGVVLLELITGRKNLDVRSSKEERNIV

                     401                                               450
OS_BAD73441   (387)  KWSRPFLTDDSRLSLIMDSRIKGRFPTKAARIVADIILRCLNKDPSERPT
    SEQ ID 2  (388)  KWSRPFLTDDSRLSLIMDSRIKGRFPTKAARIVADIILRCLNKDPSERPT
    SEQ ID 4  (395)  KWSRAFLADDCRLSLIMDPQLKGRFPAKAARSIADIAQKCLQVEPSERPT
   Consensus  (401)  KWSRPFLTDDSRLSLIMDSRIKGRFPTKAARIVADIILRCLNKDPSERPT

                     451                                               500
OS_BAD73441   (437)  MRAVVESLASVQDIKVPCRYPLQEPS-AAPRKVMLKSTSLNGIIHHHPVV
    SEQ ID 2  (438)  MRAVVESLASVQDIKVPCRYPLQEPS-AAPRKVMLKSTSLNGIIHHHPVV
    SEQ ID 4  (445)  MRNIVDQLKIIQDMKYSCRFPLREPAPVVARKHMGRSSSLNTIIWTPASV
   Consensus  (451)  MRAVVESLASVQDIKVPCRYPLQEPS AAPRKVMLKSTSLNGIIHHHPVV

                     501                                               550
OS_BAD73441   (486)  T----FSPSPPSRNQHLLSPRS---STSALLPPRTSCALDDPRVSSIKKS
    SEQ ID 2  (487)  T----FSPSPPSRNQHLLSPRS---STSALLPPRTSCALDDPRVSSIKKS
    SEQ ID 4  (495)  PPRSSFSPSPPPRRPSVSPTRGRTLVFPPVFPPRACSSLEEMAREEVRRS
   Consensus  (501)  T    FSPSPPSRNQHLLSPRS   STSALLPPRTSCALDDPRVSSIKKS

                     551          564
OS_BAD73441   (529)  PSPILRRSGVEGF-
    SEQ ID 2  (530)  PSPILRRSGVEGF-
    SEQ ID 4  (545)  SSASGRRTSLEGF-
   Consensus  (551)  PSPILRRSGVEGF
```

FIGURE 2 (continued)

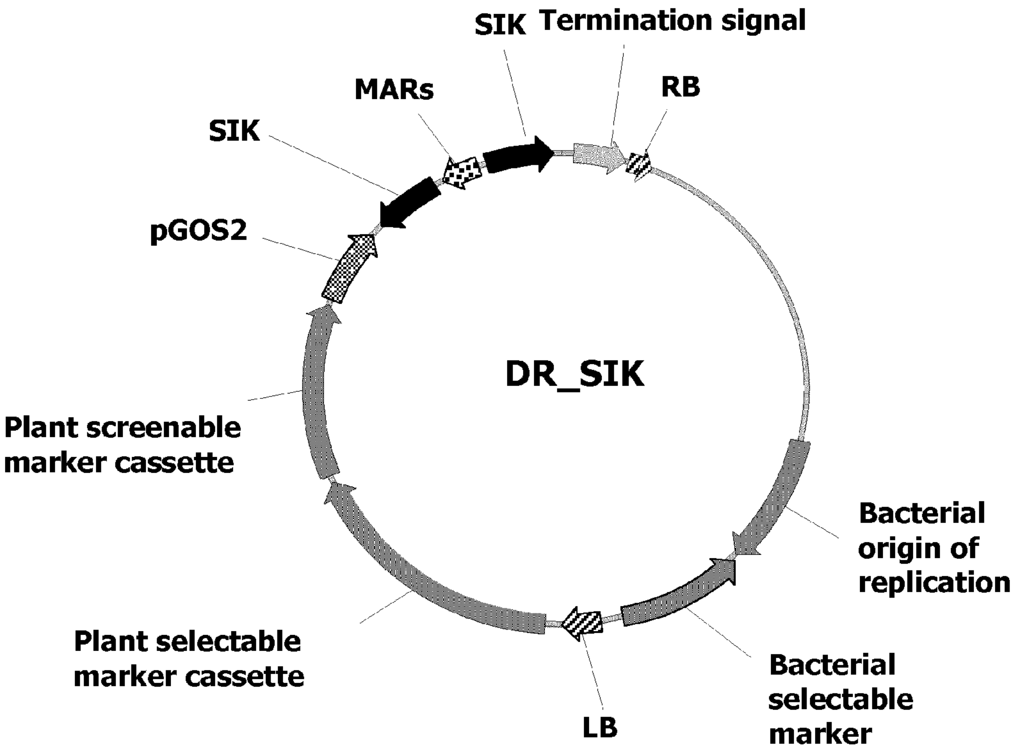


FIGURE 3

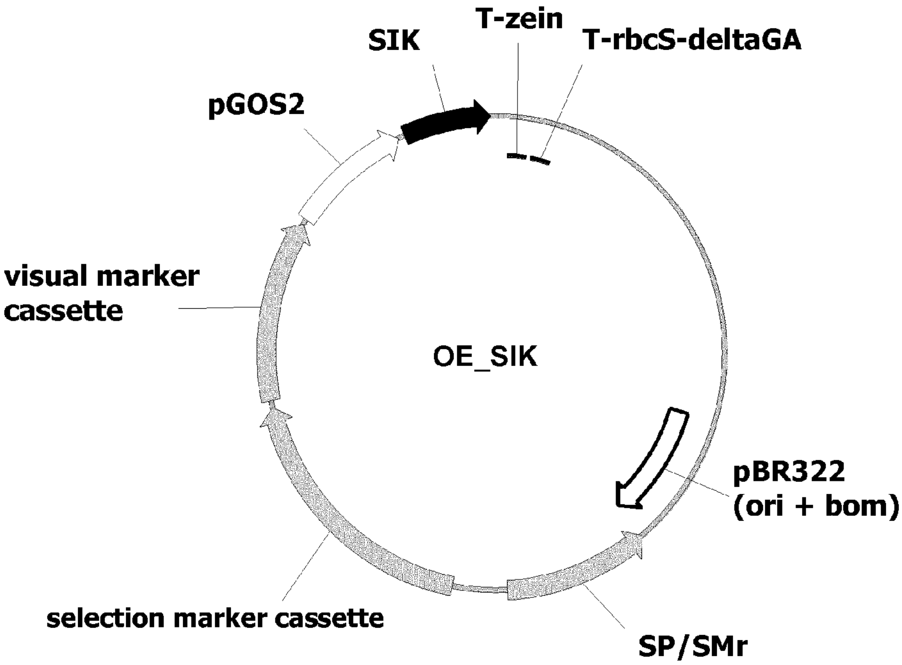


FIGURE 4

Europäisches Patentamt
European Patent Office
Office européen des brevets

# EUROPEAN SEARCH REPORT

Application Number
EP 12 18 5028

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 02/074801 A1 (GENOMINE INC [KR]; HWANG IN-HWAN [KR]; LIM JEONG-HWA [KR]; PIH KYOUNG-) 26 September 2002 (2002-09-26)<br>* the whole document *<br>----- | 14-17 | INV.<br>C12N15/82<br>C12N9/12 |
| A | WO 2006/008271 A1 (CROPDESIGN NV [BE]; FRANKARD VALERIE [BE]; MIRONOV VLADIMIR [BE]) 26 January 2006 (2006-01-26)<br>* page 3, paragraph 2 - page 4, paragraph 1 *<br>----- | 1-26 | |
| A | WO 98/05760 A2 (UNIV KINGSTON [CA]; LEFEBVRE DANIEL D [CA]; MALBOOBI MOHAMMAD A [IR] U) 12 February 1998 (1998-02-12)<br>* claims 19-26 *<br>----- | 1-26 | |
| A | DATABASE UniProt [Online]<br><br>3 October 2006 (2006-10-03),<br>"Os01g0822200 protein.",<br>XP002442037,<br>retrieved from EBI accession no. UNIPROT:Q0JI62<br>Database accession no. Q0JI62<br>* the whole document * | 14-17 | TECHNICAL FIELDS SEARCHED (IPC)<br>C12N |
| A | -& INT RICE GENOME SEQUENCING PROJECT: "The map-based sequence of the rice genome",<br>NATURE (LONDON),<br>vol. 436, no. 7052, August 2005 (2005-08), pages 793-800, XP002442031,<br>ISSN: 0028-0836<br>----- | 14-17 | |

The present search report has been drawn up for all claims

| Place of search | Date of completion of the search | Examiner |
|---|---|---|
| Munich | 23 November 2012 | Bilang, Jürg |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 18 5028

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2012

| Patent document cited in search report | Publication date | Patent family member(s) | Publication date |
|---|---|---|---|
| WO 02074801 A1 | 26-09-2002 | KR 20010035308 A | 07-05-2001 |
| | | US 2005054831 A1 | 10-03-2005 |
| | | WO 02074801 A1 | 26-09-2002 |
| WO 2006008271 A1 | 26-01-2006 | AT 454458 T | 15-01-2010 |
| | | AU 2005263730 A1 | 26-01-2006 |
| | | BR PI0513393 A | 06-05-2008 |
| | | CA 2573676 A1 | 26-01-2006 |
| | | ES 2338028 T3 | 03-05-2010 |
| | | US 2009271895 A1 | 29-10-2009 |
| | | WO 2006008271 A1 | 26-01-2006 |
| WO 9805760 A2 | 12-02-1998 | AU 730471 B2 | 08-03-2001 |
| | | AU 3616797 A | 25-02-1998 |
| | | BR 9710909 A | 17-08-1999 |
| | | CN 1226925 A | 25-08-1999 |
| | | EP 0917564 A2 | 26-05-1999 |
| | | WO 9805760 A2 | 12-02-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02074801 A
- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1


### Non-patent literature cited in the description


- **WANG et al.** *Planta,* 2003, vol. 218, 1-14
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519

- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22

- Techniques for Gene Transfer. **B. JENES et al.** Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **RABBANI et al.** *Plant Physiol,* 2003, vol. 133, 1755-1767
- **AUSUBEL et al.** Current Protocols in Molecular Biology. Current Protocols, 1994, vol. 1, 2
- *Biochimica et Biophysica Acta,* 1996, vol. 1314, 191-225
- **NEEDLEMAN ; WUNSCH.** *J Mol Biol,* 1970, vol. 48, 443-453
- **ALTSCHUL et al.** *J Mol Biol,* 1990, vol. 215, 403-410
- **CAMPANELLA et al.** *BMC Bioinformatics,* 2003, vol. 4, 29
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- current protocols in molecular biology. Wiley
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **R.D.D. CROY.** Plant Molecular Biology Labfax. BIOS Scientific Publications Ltd (UK, 1993

SEQUENCE LISTING

<110> CropDesign N.V.

<120> Plants having increased yield and method for making the same

<130> PF58401-prio

<160> 347

<170> PatentIn version 3.3

<210> 1
<211> 2151
<212> DNA
<213> Arabidopsis thaliana

<400> 1
atgtgctgtg gatcagaccg attaaaccag atcgtgtcat caagatcttc gttgccaatt 60

tctttcgagg aagataacaa tcttgttacc aacacagaca tgaatcactt aacagtcgaa 120

acagaggata cgtttgcgag cttgcttgag cttgcagcta acaacgatgt tgaaggtgta 180

aggctatcta tcgagagaga cccttcttgt gtagacgaag ctggtctctg gtacggtcgt 240

caaaaaggtt ctaaagctat ggtcaacgat tacaggactc cgttgatggt tgctgctact 300

tacggaagca ttgatgtgat caagcttatt gtttctttga ctgatgctga cgtgaaccgt 360

gcttgcggga atgatcagac cactgcgtta cactgcgctg cttctggagg agctgtgaat 420

gctatccaag ttgttaagct gcttcttgca gctggagctg atttgaatct gttggatgct 480

gaaggtcaac gagctggtga tgttattgtt gttcctccta agcttgaagg cgtgaagctg 540

atgcttcagg agcttctttc tgctgatgga tcatctactg cggagcggaa tctacgggtt 600

gtgacaaatg ttccgaatag aagctcatct ccgtgtcatt ctcctactgg agagaatggt 660

ggatcagggt ctggttcacc gctcggctct ccttttaagc tgaaatctac tgaattcaag 720

aaagagtatc cggttgatcc gtctttgcca gatatcaaga acagtatcta cgcgactgat 780

gagtttagaa tgtattcctt caaggtccgg ccttgctctc gtgcttattc acatgattgg 840

actgagtgtc cttttgttca cccgggtgaa aacgcgagga ggagagaccc gaggaagttc 900

cattacagct gcgttccttg cccggatttt aggaaaggag cttgtaggag aggagatatg 960

tgtgagtatg cgcacggtgt gtttgaatgc tggcttcatc cggctcagta caggacccgt 1020

ctttgcaaag atggaacagg ctgtgctcgg cgggtttgtt tctttgcgca tacacccgag 1080

gagcttcgac ctttgtacgc atcaactggt tcagcggttc cttcgcctag atcgaatgct 1140

gattatgcag ctgctttgag tctccttcct ggttctccat caggagtctc tgtcatgtcc 1200

ccgctttccc catcagcagc ggggaacgga atgtctcatt cgaatatggc ttggccacaa 1260

ccaaatgtcc ctgcgttgca cttaccagga agcaatctac agtcaagcag gctaaggtct 1320

tctctcaatg caagggatat cccgacggat gagttcaata tgttagcgga ttacgagcag 1380

cagcaactcc tcaacgagta ttccaatgct ctgagccgtt ctggtcggat gaaatcaatg 1440

cctccttcga atcttgaaga tcttttctca gcagaaggct cttcatctcc ccggttcact 1500

gattccgctt tagcttccgc ggtgttctcg cctacacaca agtcagctgt cttcaaccag 1560

ttccaacaac agcaacagca gcagcagagc atgttgtctc caatcaacac aagcttttct 1620

tcaccaaaga gcgttgacca ctcattgttt tcaggtggag gaagaatgtc tcctcggaat 1680

gttgttgaac caatatcacc catgagtgct cgggtttcca tgttggctca gtgcgtgaag 1740

caacaacaac agcaacagca gcagcagcag cagcaacatc agttccgtag ccttagctcc 1800

agagagctca gaacaaactc tagcccaatc gttggttcac cggtaaacaa caacacatgg 1860

tcatcaaaat ggggatcttc aaatggtcaa ccggattggg gaatgagctc agaagcactt 1920

ggtaagttga gatcttcgtc atcgtttgat ggtgatgagc ctgatgtgtc atgggtccag 1980

tcactggtga aggagactcc agcagaagcc aaagagaaag cagcaacatc ttcctcaggg 2040

gaacacgtga tgaagcagcc aaatccggtt gaaccggtaa tggatcatgc tgggctagaa 2100

gcttggattg agcaaatgca gctcgatcag cttgtggctc agcagaattg a 2151

<210> 2
<211> 716
<212> PRT
<213> Arabidopsis thaliana

<400> 2

Met Cys Cys Gly Ser Asp Arg Leu Asn Gln Ile Val Ser Ser Arg Ser
1 5 10 15

Ser Leu Pro Ile Ser Phe Glu Glu Asp Asn Asn Leu Val Thr Asn Thr
20 25 30

Asp Met Asn His Leu Thr Val Glu Thr Glu Asp Thr Phe Ala Ser Leu
35 40 45

Leu Glu Leu Ala Ala Asn Asn Asp Val Glu Gly Val Arg Leu Ser Ile
50 55 60

Glu Arg Asp Pro Ser Cys Val Asp Glu Ala Gly Leu Trp Tyr Gly Arg
65 70 75 80

Gln Lys Gly Ser Lys Ala Met Val Asn Asp Tyr Arg Thr Pro Leu Met
85 90 95

Val Ala Ala Thr Tyr Gly Ser Ile Asp Val Ile Lys Leu Ile Val Ser
100 105 110

Leu Thr Asp Ala Asp Val Asn Arg Ala Cys Gly Asn Asp Gln Thr Thr
115 120 125

```
Ala Leu His Cys Ala Ala Ser Gly Gly Ala Val Asn Ala Ile Gln Val
    130                 135                 140

Val Lys Leu Leu Leu Ala Ala Gly Ala Asp Leu Asn Leu Leu Asp Ala
145                 150                 155                 160

Glu Gly Gln Arg Ala Gly Asp Val Ile Val Val Pro Pro Lys Leu Glu
                165                 170                 175

Gly Val Lys Leu Met Leu Gln Glu Leu Leu Ser Ala Asp Gly Ser Ser
            180                 185                 190

Thr Ala Glu Arg Asn Leu Arg Val Val Thr Asn Val Pro Asn Arg Ser
        195                 200                 205

Ser Ser Pro Cys His Ser Pro Thr Gly Glu Asn Gly Gly Ser Gly Ser
    210                 215                 220

Gly Ser Pro Leu Gly Ser Pro Phe Lys Leu Lys Ser Thr Glu Phe Lys
225                 230                 235                 240

Lys Glu Tyr Pro Val Asp Pro Ser Leu Pro Asp Ile Lys Asn Ser Ile
                245                 250                 255

Tyr Ala Thr Asp Glu Phe Arg Met Tyr Ser Phe Lys Val Arg Pro Cys
            260                 265                 270

Ser Arg Ala Tyr Ser His Asp Trp Thr Glu Cys Pro Phe Val His Pro
        275                 280                 285

Gly Glu Asn Ala Arg Arg Arg Asp Pro Arg Lys Phe His Tyr Ser Cys
    290                 295                 300

Val Pro Cys Pro Asp Phe Arg Lys Gly Ala Cys Arg Arg Gly Asp Met
305                 310                 315                 320

Cys Glu Tyr Ala His Gly Val Phe Glu Cys Trp Leu His Pro Ala Gln
                325                 330                 335

Tyr Arg Thr Arg Leu Cys Lys Asp Gly Thr Gly Cys Ala Arg Arg Val
            340                 345                 350

Cys Phe Phe Ala His Thr Pro Glu Glu Leu Arg Pro Leu Tyr Ala Ser
        355                 360                 365

Thr Gly Ser Ala Val Pro Ser Pro Arg Ser Asn Ala Asp Tyr Ala Ala
    370                 375                 380

Ala Leu Ser Leu Leu Pro Gly Ser Pro Ser Gly Val Ser Val Met Ser
```

385 390 395 400

Pro Leu Ser Pro Ser Ala Ala Gly Asn Gly Met Ser His Ser Asn Met
405 410 415

Ala Trp Pro Gln Pro Asn Val Pro Ala Leu His Leu Pro Gly Ser Asn
420 425 430

Leu Gln Ser Ser Arg Leu Arg Ser Ser Leu Asn Ala Arg Asp Ile Pro
435 440 445

Thr Asp Glu Phe Asn Met Leu Ala Asp Tyr Glu Gln Gln Gln Leu Leu
450 455 460

Asn Glu Tyr Ser Asn Ala Leu Ser Arg Ser Gly Arg Met Lys Ser Met
465 470 475 480

Pro Pro Ser Asn Leu Glu Asp Leu Phe Ser Ala Glu Gly Ser Ser Ser
485 490 495

Pro Arg Phe Thr Asp Ser Ala Leu Ala Ser Ala Val Phe Ser Pro Thr
500 505 510

His Lys Ser Ala Val Phe Asn Gln Phe Gln Gln Gln Gln Gln Gln Gln
515 520 525

Gln Ser Met Leu Ser Pro Ile Asn Thr Ser Phe Ser Ser Pro Lys Ser
530 535 540

Val Asp His Ser Leu Phe Ser Gly Gly Gly Arg Met Ser Pro Arg Asn
545 550 555 560

Val Val Glu Pro Ile Ser Pro Met Ser Ala Arg Val Ser Met Leu Ala
565 570 575

Gln Cys Val Lys Gln Gln Gln Gln Gln Gln Gln Gln Gln Gln Gln Gln
580 585 590

His Gln Phe Arg Ser Leu Ser Ser Arg Glu Leu Arg Thr Asn Ser Ser
595 600 605

Pro Ile Val Gly Ser Pro Val Asn Asn Asn Thr Trp Ser Ser Lys Trp
610 615 620

Gly Ser Ser Asn Gly Gln Pro Asp Trp Gly Met Ser Ser Glu Ala Leu
625 630 635 640

Gly Lys Leu Arg Ser Ser Ser Ser Phe Asp Gly Asp Glu Pro Asp Val
645 650 655

```
Ser Trp Val Gln Ser Leu Val Lys Glu Thr Pro Ala Glu Ala Lys Glu
            660                 665                 670


Lys Ala Ala Thr Ser Ser Ser Gly Glu His Val Met Lys Gln Pro Asn
        675                 680                 685


Pro Val Glu Pro Val Met Asp His Ala Gly Leu Glu Ala Trp Ile Glu
    690                 695                 700


Gln Met Gln Leu Asp Gln Leu Val Ala Gln Gln Asn
705                 710                 715


<210>  3
<211>  13
<212>  PRT
<213>  Artificial sequence

<220>
<223>  motif 1


<220>
<221>  VARIANT
<222>  (1)..(1)
<223>  \replace = "Ala"

<220>
<221>  VARIANT
<222>  (7)..(7)
<223>  \replace = "Thr"

<400>  3

Pro Cys Ser Arg Ala Tyr Ser His Asp Trp Thr Glu Cys
1               5                   10


<210>  4
<211>  12
<212>  PRT
<213>  Artificial sequence

<220>
<223>  motif 2

<400>  4

His Pro Gly Glu Asn Ala Arg Arg Arg Asp Pro Arg
1               5                   10


<210>  5
<211>  19
<212>  PRT
<213>  Artificial sequence

<220>
<223>  motif 3
```

<220>
<221> VARIANT
<222> (3)..(3)
<223> \replace = "Ile"

<220>
<221> VARIANT
<222> (6)..(6)
<223> \replace = "Ser"

<220>
<221> VARIANT
<222> (11)..(11)
<223> \replace = "Ser"

<220>
<221> VARIANT
<222> (14)..(14)
<223> \replace = "Lys"

<400> 5

His Gly Val Phe Glu Cys Trp Leu His Pro Ala Gln Tyr Arg Thr Arg
1 5 10 15

Leu Cys Lys

<210> 6
<211> 5
<212> PRT
<213> Artificial sequence

<220>
<223> motif 4

<400> 6

Cys Phe Phe Ala His
1 5

<210> 7
<211> 54
<212> DNA
<213> Artificial sequence

<220>
<223> primer: prm06717

<400> 7
ggggacaagt ttgtacaaaa aagcaggctt aaacaatgtg ctgtggatca gacc 54

<210> 8
<211> 50
<212> DNA
<213> Artificial sequence

<220>
<223> primer: prm06718

<400> 8
ggggaccact ttgtacaaga aagctgggtg gttaggtctc tcaattctgc 50

<210> 9
<211> 981
<212> DNA
<213> Oryza sativa

<400> 9
aagaggcaag agcatccgta ttaaccagcc ttttgagact tgagagtgtg tgtgactcga 60

tccagcgtag tttcagttcg tgtgttggtg agtgattcca gccaagtttg cgatggcttc 120

tcagcaggaa cgggctagct accacgccgg cgagaccaag gcccgcgccg aggagaagac 180

ggggcgcatg atgggcacgg cgcaggagaa ggcgcgggag gccaaggaca cggcgtccga 240

cgccgcgggg cgcgcgatgg gcaggggaca cggcgccaag gaggcgacca aggagaaggc 300

gtacgagacc aaggacgcga ccaaggagaa ggcgtacgag gcaaaggacg cggcctccga 360

cgccaccggc cgcgccatgg acaagggccg cggcgccgcg ggcgccacga gggacaaggc 420

gtacgatgcc aaggacaggg cggctgacac ggcgcagtcc gccgccgacc gcgcccgcga 480

cggcgccggg cagaccggga gctacattgg acagaccgcc gaggccgcca agcagaaagc 540

ggccggcgcc gcgcagtacg ccaaggagac cgcgatcgcc ggcaaggaca agaccggcgc 600

cgtgctccag caggcagggg agcaggtgaa gagcgtggcg gtgggggcga aggacgcggt 660

gatgtacacg ctcgggatgt caggcgataa caagaacaac gccgctgccg gcaaggacac 720

cagcacctac aagcctggaa ctgggagtga ctaccagtaa tacggtagaa gaagcatgtg 780

tcgtctttgg cactgatgcc aaagtgtacg tgttgtatcc tctttttaa gtttcagctc 840

gacttcgacg tgttcggtgt cacactttgg tttttcagtt gtgctcaact gttcatgttt 900

ctggttccat ggagggccag tgtggaggtc aatgtttaag ctttcgtttt aaaatctgat 960

aataaagttg gttaagacct g 981

<210> 10
<211> 3372
<212> DNA
<213> Eucalyptus grandis

<400> 10
ggaagacgaa gagcaacaaa ataggtctca ctccctcctc tctcctctct cctctctctt 60

ctttctctct cttctgcttc taacaaagtc tcttccttga gagacagggc tgcgtcgtcg 120

tctttctctc tcctcgctgc gagcttctga gaaagttcaa ttctttttct cttgttctct 180

ctctctaccc ttctgggtac cactgtgaag ctccggtctt ttctattttt ttttttttg 240

ggctatctgg gtctgggcaa atccatcgcg cgctctgctc tggactgaga ggccgtcagt 300

ggctttagat ctgcgacgcc tcttgcttgc tcagtgagct gggctagttc aaatcgacga 360

agaaagcatg cgctagtgat tggtgtgggt aatacactgc attcgatctc tactaagtat 420

```
ccccaagtat actaagatcc cgttctcagc catgagtcaa ctgaccattc agactgagga     480
cacttttgcc agcttgcttg agcttgctgc taacaacgac acagaatctt tcggacggtg     540
tgtggaacgt gatccttcga gcatagatga aattggatat tggtatggtc gccaaaaggg     600
ttcgaagcag gtggtcaata tgcaaagaac tcctcttatg gtggctgcta catatggtag     660
tgttgatgta atgagactca ttctttgcct atctgatgct gatgtgaatc gaacctgcag     720
cacagacaag agcacagccc ttcactgtgc tgcctctggt ggtgctgtga atgctgtaga     780
tgctgtgagg ctactcctgt cagctggtgc tgacccaagt ttagcagatg ctaacggtca     840
gcggcctgtg gatgttattg ttgttcctcc aaagctcctt tcaataaagt ttgctcttga     900
agagctcttg tcgaccgaag gatctgtaaa tgaacacaat ctgagagtgt ccgtagccac     960
ttccaattca acctctcccc cactttcatc ttccccggat aatggttccc cagcatctgc    1020
taattgttct tcccccaaga actcaaagtt aagtgatgcc cctgttcttt atgcatcaga    1080
aaagaaggaa tacccggtgg atccatctct tccagatatc aagaatagca tttactcaac    1140
agatgaattc cgaatgtatt cttttaaagt gcggccttgt tcacgagcgt actcgcatga    1200
ttggacggag tgcccttttg ttcatccagg ggagaatgcc cgtagaaggg atccaaggaa    1260
gttccactac agctgtgtcc cttgccctga tttccggaag ggtgcttgta gacgtggaga    1320
tatgtgtgaa tatgctcatg gtgtttttga gtgctggctc catcctgctc agtatcggac    1380
tcgattatgc aaggatggta caagttgtgc tcggagagtg tgcttctttg cccacacgga    1440
gcaagagctg cgtccattgt acgtctccac tggttctgct gttccgtctc ctcgctcgag    1500
tacctctgga gctgctgcca tggattttgc tgcagccatg agcctcttac ctggttcccc    1560
atcatcagta tccatcatgt ccccttcacc cttcactcct cccatgtctc catctgctaa    1620
tggtatttct cacccatctg ttgcctggcc ccagcaaaat gtaccaactt tgcatcttcc    1680
cggaagcaat cttcagtcca gccgcttgag atcttctctt aatgcaagag atattcctca    1740
ggaggatttt gacttgctgt cagattatga tgtgcaacag cagcagctcc taaatgagtt    1800
ttccatcctt tcacaacaat cgatgggtgc taattccttg aaccgttctg gtcggctgaa    1860
aactttgacc ccctcaaacc ttgatgatct cttctctgct gagagctcat cccctcgcta    1920
cgctgatcaa gccctggctt ctgctgtttt ttcaccaacg cacaaatctg cagtaatcaa    1980
tcaatttcag cagcagcagc agagcatgtt atcacccatc aacacaacct tctctcctaa    2040
gagtgtcgac caccctttgt tgcaagcgtc tttcggtgtt caatctgggc gaatgtcccc    2100
tcgtaacatg gatcccatct ctcctataag ttctcgtgtg tcgatgttgg cccaacgaga    2160
gaaacagcaa cagcaattac gcagcctaag ctctcgtgaa ctcggttcca attcagccgc    2220
cattgtgggt tcccccgtgg gttcttggtc gaaatgggga gctacaaatg ggaaaccaga    2280
ctgggctgtt agtgcagatg aactaggtaa gcttcgcagg tctaattcat ttgagcttgg    2340
gaacaatggt gaggagccag atctttcatg ggttcaatcc ctcgttaaag aatctcctac    2400
```

cgagatgaaa gaaaagcttt cgtcaactct ctctggtgtt ccagcccccg ctacatccag 2460

tgaggttccg agtatcagct cgcagatgga atcggttgat cacgaagtgc taggagcatg 2520

gctccagcag atgcagctcg atccgctcgt ggctcagcaa aactaggttg ttttttttcc 2580

tacatggcct tgaggaagta gacagcggaa agtttttttt ggtaaatact atgtttttttc 2640

tggaaatttt tgatgctggg ggtggggtct ggaagaagat aacaaggcag gaaaggggtc 2700

agtgaagtca ctggagaaaa ggaattcatt tttaaccatt ttatcattct attacaacag 2760

aaagtaggga aaaaaaagga agaccctctg ggttatgaag agaaattaaa cccaggctag 2820

gcgttctcct ttctaatatt tccaatttta ggtccatatt actgtcattt cctttttgcc 2880

gtcttatcat atttcatcaa aatggaactg gggactaatg tttgttccat tctttcgctc 2940

ttctgattta tttgcaccct tggggtaaga tcaaaagaga aattatgatc attttctttt 3000

gaggatattt tttttttccca atatttgtga gaatgaaagt taagagggga tatgatgtgt 3060

ctggtgttgt agtatgaaaa accaataacc gagttcacct gttgctgctg gtggtagaag 3120

aagtggagaa gaagctatga tcctttgatg taacagtcaa tcaaacattt taataccttt 3180

attttttgtt tcctcatgta atccatcctt tgtgattgtc ctctctctct ctctctctct 3240

ctctctctcc ctccccgtgt tctttcttca taagcgtctt gcttgtcgat ctgtaaatta 3300

ttgaaaaggg tcatggaaag ccgtgccggt gtggattctc atttttgcaa aaaaaaaaaa 3360

aaaaaaaaaa aa 3372

<210> 11
<211> 704
<212> PRT
<213> Eucalyptus grandis

<400> 11

Met Ser Gln Leu Thr Ile Gln Thr Glu Asp Thr Phe Ala Ser Leu Leu
1 5 10 15

Glu Leu Ala Ala Asn Asn Asp Thr Glu Ser Phe Gly Arg Cys Val Glu
20 25 30

Arg Asp Pro Ser Ser Ile Asp Glu Ile Gly Tyr Trp Tyr Gly Arg Gln
35 40 45

Lys Gly Ser Lys Gln Val Val Asn Met Gln Arg Thr Pro Leu Met Val
50 55 60

Ala Ala Thr Tyr Gly Ser Val Asp Val Met Arg Leu Ile Leu Cys Leu
65 70 75 80

Ser Asp Ala Asp Val Asn Arg Thr Cys Ser Thr Asp Lys Ser Thr Ala
85 90 95

Leu His Cys Ala Ala Ser Gly Gly Ala Val Asn Ala Val Asp Ala Val
            100                 105                 110

Arg Leu Leu Leu Ser Ala Gly Ala Asp Pro Ser Leu Ala Asp Ala Asn
        115                 120                 125

Gly Gln Arg Pro Val Asp Val Ile Val Val Pro Pro Lys Leu Leu Ser
    130                 135                 140

Ile Lys Phe Ala Leu Glu Glu Leu Leu Ser Thr Glu Gly Ser Val Asn
145                 150                 155                 160

Glu His Asn Leu Arg Val Ser Val Ala Thr Ser Asn Ser Thr Ser Pro
                165                 170                 175

Pro Leu Ser Ser Ser Pro Asp Asn Gly Ser Pro Ala Ser Ala Asn Cys
            180                 185                 190

Ser Ser Pro Lys Asn Ser Lys Leu Ser Asp Ala Pro Val Leu Tyr Ala
        195                 200                 205

Ser Glu Lys Lys Glu Tyr Pro Val Asp Pro Ser Leu Pro Asp Ile Lys
    210                 215                 220

Asn Ser Ile Tyr Ser Thr Asp Glu Phe Arg Met Tyr Ser Phe Lys Val
225                 230                 235                 240

Arg Pro Cys Ser Arg Ala Tyr Ser His Asp Trp Thr Glu Cys Pro Phe
                245                 250                 255

Val His Pro Gly Glu Asn Ala Arg Arg Arg Asp Pro Arg Lys Phe His
            260                 265                 270

Tyr Ser Cys Val Pro Cys Pro Asp Phe Arg Lys Gly Ala Cys Arg Arg
        275                 280                 285

Gly Asp Met Cys Glu Tyr Ala His Gly Val Phe Glu Cys Trp Leu His
    290                 295                 300

Pro Ala Gln Tyr Arg Thr Arg Leu Cys Lys Asp Gly Thr Ser Cys Ala
305                 310                 315                 320

Arg Arg Val Cys Phe Phe Ala His Thr Glu Gln Glu Leu Arg Pro Leu
                325                 330                 335

Tyr Val Ser Thr Gly Ser Ala Val Pro Ser Pro Arg Ser Ser Thr Ser
            340                 345                 350

Gly Ala Ala Ala Met Asp Phe Ala Ala Ala Met Ser Leu Leu Pro Gly
355 360 365

Ser Pro Ser Ser Val Ser Ile Met Ser Pro Ser Pro Phe Thr Pro Pro
370 375 380

Met Ser Pro Ser Ala Asn Gly Ile Ser His Pro Ser Val Ala Trp Pro
385 390 395 400

Gln Gln Asn Val Pro Thr Leu His Leu Pro Gly Ser Asn Leu Gln Ser
405 410 415

Ser Arg Leu Arg Ser Ser Leu Asn Ala Arg Asp Ile Pro Gln Glu Asp
420 425 430

Phe Asp Leu Leu Ser Asp Tyr Asp Val Gln Gln Gln Gln Leu Leu Asn
435 440 445

Glu Phe Ser Ile Leu Ser Gln Gln Ser Met Gly Ala Asn Ser Leu Asn
450 455 460

Arg Ser Gly Arg Leu Lys Thr Leu Thr Pro Ser Asn Leu Asp Asp Leu
465 470 475 480

Phe Ser Ala Glu Ser Ser Ser Pro Arg Tyr Ala Asp Gln Ala Leu Ala
485 490 495

Ser Ala Val Phe Ser Pro Thr His Lys Ser Ala Val Ile Asn Gln Phe
500 505 510

Gln Gln Gln Gln Gln Ser Met Leu Ser Pro Ile Asn Thr Thr Phe Ser
515 520 525

Pro Lys Ser Val Asp His Pro Leu Leu Gln Ala Ser Phe Gly Val Gln
530 535 540

Ser Gly Arg Met Ser Pro Arg Asn Met Asp Pro Ile Ser Pro Ile Ser
545 550 555 560

Ser Arg Val Ser Met Leu Ala Gln Arg Glu Lys Gln Gln Gln Gln Leu
565 570 575

Arg Ser Leu Ser Ser Arg Glu Leu Gly Ser Asn Ser Ala Ala Ile Val
580 585 590

Gly Ser Pro Val Gly Ser Trp Ser Lys Trp Gly Ala Thr Asn Gly Lys
595 600 605

Pro Asp Trp Ala Val Ser Ala Asp Glu Leu Gly Lys Leu Arg Arg Ser
    610                 615                 620

Asn Ser Phe Glu Leu Gly Asn Asn Gly Glu Glu Pro Asp Leu Ser Trp
625                 630                 635                 640

Val Gln Ser Leu Val Lys Glu Ser Pro Thr Glu Met Lys Glu Lys Leu
                645                 650                 655

Ser Ser Thr Leu Ser Gly Val Pro Ala Pro Ala Thr Ser Ser Glu Val
            660                 665                 670

Pro Ser Ile Ser Ser Gln Met Glu Ser Val Asp His Glu Val Leu Gly
        675                 680                 685

Ala Trp Leu Gln Gln Met Gln Leu Asp Pro Leu Val Ala Gln Gln Asn
    690                 695                 700

<210> 12
<211> 1860
<212> DNA
<213> Oryza sativa

<400> 12
atgggggagc ctgggggcgc cgaggcggcc gtctccgcga ggctgctcga gctggcggcc 60

gacgacaacg cggcggggct cggggagctc ctcgcggcgt ggccctccct cgccgacgag 120

cccgcgccgt ggtacacccc ggcgcggggc gcggagccgc tgaccccgct catggtcgcc 180

gccgtgtacg gctcggtggg ctgcctcgac gcgctcctct cgccgcccta cctcgtggac 240

cccaaccgcg cctcggcgtc gtcgctctcc accccgctcc acctcgccgc cgcgggcggg 300

tccgcctccg cccccgcggc ggtctcccgc ctcctcgccg ccggcgccga cccggccctc 360

ctcgaccacc tccagcgccg ggcgtccgac ctcgtcgcgc tcccgcccaa ctcgctcccg 420

ctcaagaacc acctcctctc cctcctcggc gcccgcaagg agtggcctcc cgacccctcc 480

ctccccgaca tcaagaacgg cgcctacgcc tccgacgact tcaggatgta ctcgttcaag 540

gtgcgcgcgt gctcgcgggc ctactcccat gactggacgg agtgcccctt cgtccacccc 600

ggcgagaacg cgcggcggcg cgacccgagg aagtaccact acagctgcgt gccgtgcccg 660

gagttcaaga agggggccgg gtgcaggaga ggggacatgt gcgagtacgc gcacggggtg 720

ttcgagagct ggctccaccc ggcgcagtac cggacgcgcc tctgcaagga cggcgtcggc 780

tgcgcccgcc gcgtctgctt cttcgcccac acgcccgacg agctccgccc gctctacgtc 840

tccacgggct ccgccgtgcc gtcgccgcgc ggggcgttgg agatggcggc ggcggcggcg 900

gcgatgggga tggggctgtc gtcgccgggg tcgtcgtcgt tcacgccgcc gctatcgccg 960

tcggccggcg gggcggggg cgggggcggg ggcagcggcg gcggcggcgc gtggccgcag 1020

cagccgagcg tgccggcgct ctgcctgccc gggagcgccg ggaacctcca cctgagccgg 1080

ctgcgcacgt cgctgagcgc gcgcgacatg gccgtcgacg agctgctcgc cgcggcggcg 1140

gcggcggcgg actacgacgg cctcgtcgcc tccccgcct ccatccggtc cgcgaggggg 1200

aaggcgcttg tgccgtcaaa tctcgacgag ctcttctccg ctgagctcgc cgccgccgcg 1260

gcgtcgcgct cgccgcgcta cgccgaccaa ggcggcgccg cgttctcccc gacccgcaag 1320

gccaccgtgc tcaaccaatt ccagctgcag cagcagcata gcttgctctc gccgcgggcg 1380

gccgcggtga caccagagcc ggtctcccca atgagctccc gcctcctcgc cgcgctggcg 1440

cagcgggaga agatgcagca gcagacgctg cggagcatga gctcacggga cctcggcaac 1500

gccgcgtcgc tgctggtcgg ctcgccggtg agctcgagca tgtccaaatg gggggttcccc 1560

tccggcaacc cggactgggg cgccgacgac gaggagctcg gccgcctcaa gcgttgctcc 1620

tcgttcgagc tccggtccgg agccgccaat ggcaaccatg agcctgacct ctcatgggtc 1680

aacaccctag tgaaggagcc gacaccggag aagatgatga cgacgacatc ggcaatggat 1740

tccattggca tcttgggaca gaacacaagc cgtgatcaca tcgtcggagg cgaggatgac 1800

actgccggag tcatcagcag ctggcttgaa cagctccagc tcgatgagat ggttgtctag 1860

<210> 13
<211> 619
<212> PRT
<213> Oryza sativa

<400> 13

Met Gly Glu Pro Gly Gly Ala Glu Ala Ala Val Ser Ala Arg Leu Leu
1 5 10 15

Glu Leu Ala Ala Asp Asp Asn Ala Ala Gly Leu Gly Glu Leu Leu Ala
20 25 30

Ala Trp Pro Ser Leu Ala Asp Glu Pro Ala Pro Trp Tyr Thr Pro Ala
35 40 45

Arg Gly Ala Glu Pro Leu Thr Pro Leu Met Val Ala Ala Val Tyr Gly
50 55 60

Ser Val Gly Cys Leu Asp Ala Leu Leu Ser Pro Pro Tyr Leu Val Asp
65 70 75 80

Pro Asn Arg Ala Ser Ala Ser Ser Leu Ser Thr Pro Leu His Leu Ala
85 90 95

Ala Ala Gly Gly Ser Ala Ser Ala Pro Ala Ala Val Ser Arg Leu Leu
100 105 110

Ala Ala Gly Ala Asp Pro Ala Leu Leu Asp His Leu Gln Arg Arg Ala
115 120 125

```
Ser Asp Leu Val Ala Leu Pro Pro Asn Ser Leu Pro Leu Lys Asn His
    130                 135                 140

Leu Leu Ser Leu Leu Gly Ala Arg Lys Glu Trp Pro Pro Asp Pro Ser
145                 150                 155                 160

Leu Pro Asp Ile Lys Asn Gly Ala Tyr Ala Ser Asp Asp Phe Arg Met
                165                 170                 175

Tyr Ser Phe Lys Val Arg Ala Cys Ser Arg Ala Tyr Ser His Asp Trp
            180                 185                 190

Thr Glu Cys Pro Phe Val His Pro Gly Glu Asn Ala Arg Arg Arg Asp
        195                 200                 205

Pro Arg Lys Tyr His Tyr Ser Cys Val Pro Cys Pro Glu Phe Lys Lys
    210                 215                 220

Gly Ala Gly Cys Arg Arg Gly Asp Met Cys Glu Tyr Ala His Gly Val
225                 230                 235                 240

Phe Glu Ser Trp Leu His Pro Ala Gln Tyr Arg Thr Arg Leu Cys Lys
                245                 250                 255

Asp Gly Val Gly Cys Ala Arg Arg Val Cys Phe Phe Ala His Thr Pro
            260                 265                 270

Asp Glu Leu Arg Pro Leu Tyr Val Ser Thr Gly Ser Ala Val Pro Ser
        275                 280                 285

Pro Arg Gly Ala Leu Glu Met Ala Ala Ala Ala Ala Ala Met Gly Met
    290                 295                 300

Gly Leu Ser Ser Pro Gly Ser Ser Ser Phe Thr Pro Pro Leu Ser Pro
305                 310                 315                 320

Ser Ala Gly Gly Gly Gly Gly Gly Gly Gly Gly Ser Gly Gly Gly Gly
                325                 330                 335

Ala Trp Pro Gln Gln Pro Ser Val Pro Ala Leu Cys Leu Pro Gly Ser
            340                 345                 350

Ala Gly Asn Leu His Leu Ser Arg Leu Arg Thr Ser Leu Ser Ala Arg
        355                 360                 365

Asp Met Ala Val Asp Glu Leu Leu Ala Ala Ala Ala Ala Ala Ala Asp
    370                 375                 380
```

```
Tyr Asp Gly Leu Val Ala Ser Pro Ala Ser Ile Arg Ser Ala Arg Gly
385                 390                 395                 400

Lys Ala Leu Val Pro Ser Asn Leu Asp Glu Leu Phe Ser Ala Glu Leu
                405                 410                 415

Ala Ala Ala Ala Ala Ser Arg Ser Pro Arg Tyr Ala Asp Gln Gly Gly
            420                 425                 430

Ala Ala Phe Ser Pro Thr Arg Lys Ala Thr Val Leu Asn Gln Phe Gln
        435                 440                 445

Leu Gln Gln Gln His Ser Leu Leu Ser Pro Arg Ala Ala Ala Val Thr
    450                 455                 460

Pro Glu Pro Val Ser Pro Met Ser Ser Arg Leu Leu Ala Ala Leu Ala
465                 470                 475                 480

Gln Arg Glu Lys Met Gln Gln Gln Thr Leu Arg Ser Met Ser Ser Arg
                485                 490                 495

Asp Leu Gly Asn Ala Ala Ser Leu Leu Val Gly Ser Pro Val Ser Ser
            500                 505                 510

Ser Met Ser Lys Trp Gly Phe Pro Ser Gly Asn Pro Asp Trp Gly Ala
        515                 520                 525

Asp Asp Glu Glu Leu Gly Arg Leu Lys Arg Cys Ser Ser Phe Glu Leu
    530                 535                 540

Arg Ser Gly Ala Ala Asn Gly Asn His Glu Pro Asp Leu Ser Trp Val
545                 550                 555                 560

Asn Thr Leu Val Lys Glu Pro Thr Pro Glu Lys Met Met Thr Thr Thr
                565                 570                 575

Ser Ala Met Asp Ser Ile Gly Ile Leu Gly Gln Asn Thr Ser Arg Asp
            580                 585                 590

His Ile Val Gly Gly Glu Asp Asp Thr Ala Gly Val Ile Ser Ser Trp
        595                 600                 605

Leu Glu Gln Leu Gln Leu Asp Glu Met Val Val
    610                 615
```

<210>  14
<211>  2106
<212>  DNA
<213>  Medicago truncatula

<400> 14
atgaaaaatc taactgttcg tactgatgat tctttttcca gcttacttga acatgcttct 60
aacaatgatt ttgaagattt caaggtagct ctagatagtg atgcttcact tattaatgaa 120
gttggcttct ggtatgtccg tcaaaaggga tctaaccaaa ttgttcttga gcaccgaacc 180
cctttaatgg tggctgcttc ctatgggagt attgatattc taaagcttat actctcatat 240
cccgaggctg atgttaattt ctcctgtgga actgataaaa gcactgctct tcactgtgct 300
gcctcaagtg gttcagttaa tgctgttgat gctataaaat tgcttttatc agctggtgct 360
gatatcaatt ctgtggatgc taatgggaaa cgccctgtgg atgttatcgt tgttcctatt 420
gttgttcctc ataagctcga aggtgttaaa acaattcttg aagaacttct ctcagacagt 480
gcttctgaag gatctgtgga tgattgctct cttcccctgt ctcttatttc atcgagtcct 540
ggttcatctg cccctttatc atctgctgaa aatggatctc catcctctcc tgtggctccc 600
aagtttacag atacagctgt taattctaca tcagaaaaga aagagtatcc agttgaccca 660
tctcttcctg acataaaaaa cagcatgtat gccacagatg aattccgcat gtattcattc 720
aaggttcgtc cttgttctcg tgcatactct catgattgga ctgagtgtcc ttttgtgcat 780
cctggagaga atgctcgaag gagagaccct agaaagtttc actacagctg tgtgccatgc 840
cctgatttta ggaaaggggc ttgccgacgt tcggatatgt gtgaatatgc tcatggagta 900
ttcgagtgct ggctacaccc agctcagtat cggacaaggc tgtgcaaaga cggtatgggt 960
tgtaaccgaa gggtgtgctt cttcgctcac tcacctgaag agctgcgtcc gctgtatgtg 1020
tccactggtt ctgctgttcc ttcaccccga tcagctgctt ctactgctaa tgtcatggac 1080
atggctgctg ctatgagcct tttccctggt tcaccatcat caatctcttt gatgtctcaa 1140
tcaccctttg cacagcctcc tctatctcca tctgcaaatg gcaataatgc ttggccacag 1200
cccaatgtgc cagctcttca tttaccagga agcattaatc aaactagtcg tttgagatct 1260
tctcttagtg cccgtgatat gccacacgac gacttcaaca atatgttgca agactttgat 1320
gggcagcagc agatactaaa tgacttgagc tgtttctcac agccccgtcc tggtgctatt 1380
tcagttggtc gatctggccg ccctaaaaca ctaactccct caaatctgga tgatcttttt 1440
tgtgctgaga ttgcttcatc tcctaggtat tccgaccccg ctgcggcttc tgtattttcc 1500
ccaacacaca aatctgctgt cttcaaccag tttcaacagc ttcaaagctc cttatcaccc 1560
atcaacacaa atgtcatgtc tcctacaaac gtagagcatc ccctgttcca ccaggcttca 1620
tatggtctct cttctcctgg aaggatgtca ccaagaagta tggaagccct atctccaatg 1680
agttctcggc tgtcagcttt tgctcagcgt gagaaacaac agcagcagca gcaacagctg 1740
cgtagcctca gctcaagaga actcggtgct aacaatcctc tctcagctgt tgggtcccct 1800
gttaactcct ggtccaagtg gggatcatcc cctattggaa aagctgattg gtcggtaaat 1860
ccaaatgact tcggtcaaac acagagatca acttcttttg agcatggaaa caatggagaa 1920

gagcctgatg taggttgggt ccattccctt gtcaaggatc ccacacctga gaagaaagag 1980

aagcttgcag gttccggccc aattccatcc gttgaaaaga atcccaatcc tcaagcggac 2040

ggcattgatc actctgtttt gggagcttgg ctcgagcaac tgcagctgga tcaacttgta 2100

gtctag 2106

<210> 15
<211> 701
<212> PRT
<213> Medicago truncatula

<400> 15

Met Lys Asn Leu Thr Val Arg Thr Asp Asp Ser Phe Ser Ser Leu Leu
1               5                   10                  15

Glu His Ala Ser Asn Asn Asp Phe Glu Asp Phe Lys Val Ala Leu Asp
            20                  25                  30

Ser Asp Ala Ser Leu Ile Asn Glu Val Gly Phe Trp Tyr Val Arg Gln
        35                  40                  45

Lys Gly Ser Asn Gln Ile Val Leu Glu His Arg Thr Pro Leu Met Val
    50                  55                  60

Ala Ala Ser Tyr Gly Ser Ile Asp Ile Leu Lys Leu Ile Leu Ser Tyr
65                  70                  75                  80

Pro Glu Ala Asp Val Asn Phe Ser Cys Gly Thr Asp Lys Ser Thr Ala
                85                  90                  95

Leu His Cys Ala Ala Ser Ser Gly Ser Val Asn Ala Val Asp Ala Ile
            100                 105                 110

Lys Leu Leu Leu Ser Ala Gly Ala Asp Ile Asn Ser Val Asp Ala Asn
        115                 120                 125

Gly Lys Arg Pro Val Asp Val Ile Val Val Pro Ile Val Val Pro His
    130                 135                 140

Lys Leu Glu Gly Val Lys Thr Ile Leu Glu Glu Leu Leu Ser Asp Ser
145                 150                 155                 160

Ala Ser Glu Gly Ser Val Asp Asp Cys Ser Leu Pro Leu Ser Leu Ile
                165                 170                 175

Ser Ser Ser Pro Gly Ser Ser Ala Pro Leu Ser Ser Ala Glu Asn Gly
            180                 185                 190

Ser Pro Ser Ser Pro Val Ala Pro Lys Phe Thr Asp Thr Ala Val Asn
195 200 205

Ser Thr Ser Glu Lys Lys Glu Tyr Pro Val Asp Pro Ser Leu Pro Asp
210 215 220

Ile Lys Asn Ser Met Tyr Ala Thr Asp Glu Phe Arg Met Tyr Ser Phe
225 230 235 240

Lys Val Arg Pro Cys Ser Arg Ala Tyr Ser His Asp Trp Thr Glu Cys
245 250 255

Pro Phe Val His Pro Gly Glu Asn Ala Arg Arg Arg Asp Pro Arg Lys
260 265 270

Phe His Tyr Ser Cys Val Pro Cys Pro Asp Phe Arg Lys Gly Ala Cys
275 280 285

Arg Arg Ser Asp Met Cys Glu Tyr Ala His Gly Val Phe Glu Cys Trp
290 295 300

Leu His Pro Ala Gln Tyr Arg Thr Arg Leu Cys Lys Asp Gly Met Gly
305 310 315 320

Cys Asn Arg Arg Val Cys Phe Phe Ala His Ser Pro Glu Glu Leu Arg
325 330 335

Pro Leu Tyr Val Ser Thr Gly Ser Ala Val Pro Ser Pro Arg Ser Ala
340 345 350

Ala Ser Thr Ala Asn Val Met Asp Met Ala Ala Ala Met Ser Leu Phe
355 360 365

Pro Gly Ser Pro Ser Ser Ile Ser Leu Met Ser Gln Ser Pro Phe Ala
370 375 380

Gln Pro Pro Leu Ser Pro Ser Ala Asn Gly Asn Asn Ala Trp Pro Gln
385 390 395 400

Pro Asn Val Pro Ala Leu His Leu Pro Gly Ser Ile Asn Gln Thr Ser
405 410 415

Arg Leu Arg Ser Ser Leu Ser Ala Arg Asp Met Pro His Asp Asp Phe
420 425 430

Asn Asn Met Leu Gln Asp Phe Asp Gly Gln Gln Gln Ile Leu Asn Asp
435 440 445

Leu Ser Cys Phe Ser Gln Pro Arg Pro Gly Ala Ile Ser Val Gly Arg

450 455 460

Ser Gly Arg Pro Lys Thr Leu Thr Pro Ser Asn Leu Asp Asp Leu Phe
465 470 475 480

Cys Ala Glu Ile Ala Ser Ser Pro Arg Tyr Ser Asp Pro Ala Ala Ala
485 490 495

Ser Val Phe Ser Pro Thr His Lys Ser Ala Val Phe Asn Gln Phe Gln
500 505 510

Gln Leu Gln Ser Ser Leu Ser Pro Ile Asn Thr Asn Val Met Ser Pro
515 520 525

Thr Asn Val Glu His Pro Leu Phe His Gln Ala Ser Tyr Gly Leu Ser
530 535 540

Ser Pro Gly Arg Met Ser Pro Arg Ser Met Glu Ala Leu Ser Pro Met
545 550 555 560

Ser Ser Arg Leu Ser Ala Phe Ala Gln Arg Glu Lys Gln Gln Gln Gln
565 570 575

Gln Gln Gln Leu Arg Ser Leu Ser Ser Arg Glu Leu Gly Ala Asn Asn
580 585 590

Pro Leu Ser Ala Val Gly Ser Pro Val Asn Ser Trp Ser Lys Trp Gly
595 600 605

Ser Ser Pro Ile Gly Lys Ala Asp Trp Ser Val Asn Pro Asn Asp Phe
610 615 620

Gly Gln Thr Gln Arg Ser Thr Ser Phe Glu His Gly Asn Asn Gly Glu
625 630 635 640

Glu Pro Asp Val Gly Trp Val His Ser Leu Val Lys Asp Pro Thr Pro
645 650 655

Glu Lys Lys Glu Lys Leu Ala Gly Ser Gly Pro Ile Pro Ser Val Glu
660 665 670

Lys Asn Pro Asn Pro Gln Ala Asp Gly Ile Asp His Ser Val Leu Gly
675 680 685

Ala Trp Leu Glu Gln Leu Gln Leu Asp Gln Leu Val Val
690 695 700

<210> 16
<211> 2841

<212> DNA
<213> Oryza sativa

<400> 16
atgaacggca cgccgatctc cgcgtccgcc gcggccggcg tcgacggagt cggcgcggcg 60

gtggcgctgg cggccgcgac caagaagagt gccgccgcgg cggccgccgt cgccgagatg 120

gcgaaaaccc tcaccgtcga cacggacgac gccttcgcgg ggctcctcga gctcgccgcg 180

gacgacgacg cggagggcct gcgccgcgcg ctggagcgcg ccccgcccgc cgccgcggac 240

gaggcgggcc tctggtacgg ccgccgcaag gtcctcgagc accgcacgcc gctgatggtc 300

gcggccacct atggcagcct cgcggtgctt cgcctgctgc tgtccctccc gtccgtcgat 360

gtcaatcgcc gctgtggctc cgacggcacc accgccctcc actgtgcggc gtctggtggc 420

tcgccgtctt gtgtggaggc cgtcaagctg ctgcttgctg ctggggctga tgctgatgcc 480

acggatgctt ccggatatcg tccagctgat gtgatctctg ttcctccaaa gatgtttgac 540

gccaagattg ccctccaaga tcttcttgga tgcccaaagg ctgggcatgg cgttctccgg 600

gtggtgacaa gggccgcaaa ctctatgttg tcacctgtat catcccctac agcagaagat 660

gcacgatctc catcagctgc tgtgatgatg acgacaaagt ttgcagatct tccaagggtt 720

gtgacatcgg aaaagaaaga atatccagtg gatccgtccc ttcccgatat caagaacagc 780

atctatgctt ccgatgagtt ccgcatgtac tcatttaaga tcaggccatg ctcgcgggcg 840

tactcacatg attggactga gtgcccgttt gttcacccag gggagaacgc acggcgtcgg 900

gaccctcgca agtatcacta cagctgtgtg ccatgccccg actttagaaa gggagtttgc 960

cggcgtggtg acatgtgtga atatgctcat ggcgtgttcg agtgttggct ccatccagca 1020

cagtaccgta ctcgcctttg caaggatggc acaagctgta atcgccgtgt ctgtttcttt 1080

gcgcatacaa ctgatgagct ccgaccacta tatgtttcca ctggatctgc agtaccatcc 1140

ccaagagcct cggcaacagc tacaatggag atggctgcag caatgggctt gatgcctggt 1200

tctccatcat cagtttcagc agtcatgtcc ccatttacac caccaatgtc cccttcaggc 1260

aatgggatgc ccccttcatt gggctggcag cagccaaatg ttccgacact acaccttcca 1320

ggcagcagcc ttcagtcgag ccggctccgt acctcactta gtgcaaggga tatgcctgct 1380

gatgattact ccctgatgca ggatattgat tcacagctta taaatgattt gtgctattca 1440

cgtattggtt catcaacagg aaaccacacg tctcggacca agtccctaaa tccgtcaaac 1500

ttggatgatc tcttctctgc tgagatggtc tcttccccga ggtatagtaa tgctgatcag 1560

ggtggtatgt tttcaccatc tcacaaggct gctttcctta atcagttcca gcaacagcag 1620

caggcacttc tttcaccaat caacacagtc ttctccccga agtctgtgga caaccagcag 1680

ttgccttcac actcatctct gttgcaagca tcacttggta tatcctcccc tggccgcatg 1740

tctcctcgat gtgttgaatc tgggtcccct atgaactctc atcttgctgc tgctcttgct 1800

cagcgtgaga agcaacagca gacaatgaga agtctcagtt ctcgtgatct tgggccgagt 1860

gctgcaagag catcaggtgt tgttggctcc cctctaagct catcatggtc aaagtgggga 1920

tcaccttcag ggacacctga ctggggtgtt aatggtgaag aattgggcaa gcttcgccgg 1980

tcatcatcgt ttgagctgag atctggtggt gatgatccag atctctcttg ggtacacaca 2040

ctggttaagg aatctccacc agagaagcaa gtcactactg ctgaatccat aaactctgtt 2100

ggaccttcac cactgatgcc tcccagtgta agcaacggtg aaggtcctag tctgaatgcc 2160

ccgctggatg ggcatgacca agctgctgtt attggagcat tgcttgaaca gatgcagctt 2220

gatcagcata ttggtagtct agcaacataa gcgctgaatg agcctggaaa gtgcaaggag 2280

ttattattct tagttaatga atttggagta attttttttcc tgttcattaa gatggtcagc 2340

aagcaaaagg atggatagct gatggtggtg attcagagat tggttttctt tactttattg 2400

aggtaaatca tatacattat tgaggttcca gtaggttgaa agattgaagt accttgattg 2460

gggtcgtttc aagaccgacc caggtagaat cgcacccccgg cagcttcaat tcatcggtca 2520

aaaatatttc cctgttttgt taattaaccc cgttaaaaaa gaagactcgt ttggtgtttc 2580

ggaattcttt tctttacctt agcggtgttt attttgttta ttatgatatt gatacttgat 2640

gtactgatgg gtataaggtt ggttaccagg catgctatag tggtatatca agtcccaaag 2700

tattcttttt ctccctttca ccatttgtcg aggatcatac tatggccttg ttttggtcag 2760

atcttgaggc ctgtataatc cttggatttg taataatgta atattgtcat tgaacttaca 2820

ttgctattgt tttgcaatcg c 2841

<210> 17
<211> 749
<212> PRT
<213> Oryza sativa

<400> 17

Met Asn Gly Thr Pro Ile Ser Ala Ser Ala Ala Ala Gly Val Asp Gly
1 5 10 15

Val Gly Ala Ala Val Ala Leu Ala Ala Ala Thr Lys Lys Ser Ala Ala
20 25 30

Ala Ala Ala Ala Val Ala Glu Met Ala Lys Thr Leu Thr Val Asp Thr
35 40 45

Asp Asp Ala Phe Ala Gly Leu Leu Glu Leu Ala Ala Asp Asp Asp Ala
50 55 60

Glu Gly Leu Arg Arg Ala Leu Glu Arg Ala Pro Pro Ala Ala Ala Asp
65 70 75 80

Glu Ala Gly Leu Trp Tyr Gly Arg Arg Lys Val Leu Glu His Arg Thr
85 90 95

```
Pro Leu Met Val Ala Ala Thr Tyr Gly Ser Leu Ala Val Leu Arg Leu
            100                 105                 110

Leu Leu Ser Leu Pro Ser Val Asp Val Asn Arg Arg Cys Gly Ser Asp
        115                 120                 125

Gly Thr Thr Ala Leu His Cys Ala Ala Ser Gly Gly Ser Pro Ser Cys
    130                 135                 140

Val Glu Ala Val Lys Leu Leu Leu Ala Ala Gly Ala Asp Ala Asp Ala
145                 150                 155                 160

Thr Asp Ala Ser Gly Tyr Arg Pro Ala Asp Val Ile Ser Val Pro Pro
                165                 170                 175

Lys Met Phe Asp Ala Lys Ile Ala Leu Gln Asp Leu Leu Gly Cys Pro
            180                 185                 190

Lys Ala Gly His Gly Val Leu Arg Val Val Thr Arg Ala Ala Asn Ser
        195                 200                 205

Met Leu Ser Pro Val Ser Ser Pro Thr Ala Glu Asp Ala Arg Ser Pro
    210                 215                 220

Ser Ala Ala Val Met Met Thr Thr Lys Phe Ala Asp Leu Pro Arg Val
225                 230                 235                 240

Val Thr Ser Glu Lys Lys Glu Tyr Pro Val Asp Pro Ser Leu Pro Asp
                245                 250                 255

Ile Lys Asn Ser Ile Tyr Ala Ser Asp Glu Phe Arg Met Tyr Ser Phe
            260                 265                 270

Lys Ile Arg Pro Cys Ser Arg Ala Tyr Ser His Asp Trp Thr Glu Cys
        275                 280                 285

Pro Phe Val His Pro Gly Glu Asn Ala Arg Arg Arg Asp Pro Arg Lys
    290                 295                 300

Tyr His Tyr Ser Cys Val Pro Cys Pro Asp Phe Arg Lys Gly Val Cys
305                 310                 315                 320

Arg Arg Gly Asp Met Cys Glu Tyr Ala His Gly Val Phe Glu Cys Trp
                325                 330                 335

Leu His Pro Ala Gln Tyr Arg Thr Arg Leu Cys Lys Asp Gly Thr Ser
            340                 345                 350
```

```
Cys Asn Arg Arg Val Cys Phe Phe Ala His Thr Thr Asp Glu Leu Arg
        355                 360                 365

Pro Leu Tyr Val Ser Thr Gly Ser Ala Val Pro Ser Pro Arg Ala Ser
    370                 375                 380

Ala Thr Ala Thr Met Glu Met Ala Ala Ala Met Gly Leu Met Pro Gly
385                 390                 395                 400

Ser Pro Ser Ser Val Ser Ala Val Met Ser Pro Phe Thr Pro Pro Met
                405                 410                 415

Ser Pro Ser Gly Asn Gly Met Pro Pro Ser Leu Gly Trp Gln Gln Pro
            420                 425                 430

Asn Val Pro Thr Leu His Leu Pro Gly Ser Ser Leu Gln Ser Ser Arg
        435                 440                 445

Leu Arg Thr Ser Leu Ser Ala Arg Asp Met Pro Ala Asp Asp Tyr Ser
    450                 455                 460

Leu Met Gln Asp Ile Asp Ser Gln Leu Ile Asn Asp Leu Cys Tyr Ser
465                 470                 475                 480

Arg Ile Gly Ser Ser Thr Gly Asn His Thr Ser Arg Thr Lys Ser Leu
                485                 490                 495

Asn Pro Ser Asn Leu Asp Asp Leu Phe Ser Ala Glu Met Val Ser Ser
            500                 505                 510

Pro Arg Tyr Ser Asn Ala Asp Gln Gly Gly Met Phe Ser Pro Ser His
        515                 520                 525

Lys Ala Ala Phe Leu Asn Gln Phe Gln Gln Gln Gln Gln Ala Leu Leu
    530                 535                 540

Ser Pro Ile Asn Thr Val Phe Ser Pro Lys Ser Val Asp Asn Gln Gln
545                 550                 555                 560

Leu Pro Ser His Ser Ser Leu Leu Gln Ala Ser Leu Gly Ile Ser Ser
                565                 570                 575

Pro Gly Arg Met Ser Pro Arg Cys Val Glu Ser Gly Ser Pro Met Asn
            580                 585                 590

Ser His Leu Ala Ala Ala Leu Ala Gln Arg Glu Lys Gln Gln Gln Thr
        595                 600                 605
```

Met Arg Ser Leu Ser Ser Arg Asp Leu Gly Pro Ser Ala Ala Arg Ala
610 615 620

Ser Gly Val Val Gly Ser Pro Leu Ser Ser Ser Trp Ser Lys Trp Gly
625 630 635 640

Ser Pro Ser Gly Thr Pro Asp Trp Gly Val Asn Gly Glu Glu Leu Gly
645 650 655

Lys Leu Arg Arg Ser Ser Ser Phe Glu Leu Arg Ser Gly Gly Asp Asp
660 665 670

Pro Asp Leu Ser Trp Val His Thr Leu Val Lys Glu Ser Pro Pro Glu
675 680 685

Lys Gln Val Thr Thr Ala Glu Ser Ile Asn Ser Val Gly Pro Ser Pro
690 695 700

Leu Met Pro Pro Ser Val Ser Asn Gly Glu Gly Pro Ser Leu Asn Ala
705 710 715 720

Pro Leu Asp Gly His Asp Gln Ala Ala Val Ile Gly Ala Leu Leu Glu
725 730 735

Gln Met Gln Leu Asp Gln His Ile Gly Ser Leu Ala Thr
740 745

<210> 18
<211> 2769
<212> DNA
<213> Arabidopsis thaliana

<400> 18
acaccagtta ccctctcatc cgttttcgtt tttttttct ctctttcaaa aatctctcag 60

ctgaggttga tcgatcttct tcttcttctt cctcactctt tagatttgtt ccttttgcat 120

tttacacttt tggatctgaa aatgtggttc tctgtttcgc ccgttaccgt ttagattcag 180

ttctgttttt ttcttacccg atcgcttgat tcggactgtg atctttgatc ttttttcttc 240

tccagtgccg tgaaggatgt gtggtcttgc taagaagctg gatatagagg atactttgac 300

atcactgtca gaccaagaga atgaatcttt ggccaaaccc atgaatgatg ctgctgaatg 360

ggaacattcg ttttctgcct tgcttgagtt tgctgcagac aacgatgtgg aggggtttag 420

gcggcaactc tctgatgtgt cttgtatcaa ccagatgggt ctttggtaca gacggcagag 480

gtttgttaga agaatggttc ttgagcaaag aaccccgctg atggttgctt cgttatatgg 540

gagtttagat gttgtgaagt ttattctttc tttcccggaa gcggagttga atctgtcttg 600

tggtcctgat aaaagtactg ctcttcattg cgctgcttct ggtgcttctg tgaattcctt 660

ggatgttgtc aagttgcttt tgagtgtagg agcagatcct aatatccctg atgctcatgg 720

aaatcgtcct gttgatgttc ttgttgtgtc tccacacgct cctggtttga gaaccatcct 780

tgaagagatc ttgaagaaag acgagattat atctgaagat ctgcatgcct cgtcatctag 840

cttgggatca agtttccggt ctctctcatc atcccctgat aatggttcct cgttactctc 900

cttagattca gtatcctctc cgactaagcc acacggtact gatgtaactt tcgcatcaga 960

gaagaaagag tacccaattg atccatcatt gcctgatatc aaaagcggga tttattcaac 1020

cgatgagttt cgtatgttct cgttcaagat ccgcccatgt tctcgagcat attcccatga 1080

ctggactgaa tgtccatttg cacacccagg tgagaatgca aggagaagag acccgaggaa 1140

gtttcactat acgtgtgttc catgcccgga ttttaagaaa ggatcctgta agcaaggtga 1200

tatgtgtgaa tatgctcatg ggtttttga atgctggcta caccctgctc agtacagaac 1260

acgattgtgc aaggacggaa tgggttgcaa ccgaagggtt tgcttctttg ctcacgcaaa 1320

tgaggagttg cgtcccttgt acccttccac aggatctgga ttgccatctc ctcgggcttc 1380

gtctgctgtt tccgcctcta ctatggacat ggcgtcagtt ttgaacatgt taccaggctc 1440

accatctgct gctcaacatt cgttcacccc accaatatct ccttctggaa atggtagtat 1500

gccccattca tcgatgggtt ggcctcagca gaacataccg gcgttgaatc ttcctggaag 1560

caatatccag ttgagtcgtc tgagatcttc tcttaacgct agagatattc cttctgagca 1620

gcttagcatg ctgcatgagt ttgaaatgca acgtcagctt gctggcgata tgcacagtcc 1680

acgctttatg aatcattccg ctcgtcctaa gacactgaac ccttcaaatc tggaggaact 1740

cttctcagct gaggttgcat ctcctcgttt ctctgatcaa cttgctgttt catctgttct 1800

atcgccttcc cacaagtccg cgcttcttaa tcagctgcag aataataagc agagcatgct 1860

ttctcctatc aagacaaatc taatgtcttc tccaaagaat gtggagcaac attctcttct 1920

gcagcaagcc tcgtcacccc gaggcggaga gcctatttcc ccaatgaatg ctcgaatgaa 1980

acagcagcta cattcacgca gcctaagctc ccgtgatttt ggatctagtc tgccccgtga 2040

tttaatgccg actgattctg gttcgccatt aagtccatgg tcaagttggg accagaccca 2100

tggaagcaag gtggattggt cagtccaatc agatgagtta ggtcggttga gaaaatctca 2160

ttccttggct aataacccaa acagggaagc agatgtttca tgggctcagc agatgttaaa 2220

agactcttca tcacctagga acggaaaccg tgttgtgaac atgaatggtg caaggccatt 2280

gactcaaggt ggttcgagtg tgaatcctca caacagtgac actcgtgaga gcgacattct 2340

tgatgcgtgg cttgaacagc tgcacctaga tcgctgagcc tcagctgcga gagagaggtt 2400

cacatttctg tgaagctgtg aaactgatga ttcgtttatt tattattcaa gaaagcaaac 2460

ggaaacaaaa gcaaactccg ggtaagcttt tttcgattct aataacccta aaaggctcag 2520

ttttttcagg cttctttctg aaatttcttt actttcttat ttttatcacc tcattaaatt 2580

aattattgta tcatctctgt tgtaacaatg gccaaagtgc gcctctatta cttcccggat 2640

ttctgattta catttttgt atcctctcag tttgtcaatt gtttctaata tctccttcat 2700

atttgtcaaa gaacactgta tgagaaataa taacatattg tttcagctaa taagattcat 2760

tcatttcct 2769

<210> 19
<211> 706
<212> PRT
<213> Arabidopsis thaliana

<400> 19

Met Cys Gly Leu Ala Lys Lys Leu Asp Ile Glu Asp Thr Leu Thr Ser
1 5 10 15

Leu Ser Asp Gln Glu Asn Glu Ser Leu Ala Lys Pro Met Asn Asp Ala
20 25 30

Ala Glu Trp Glu His Ser Phe Ser Ala Leu Leu Glu Phe Ala Ala Asp
35 40 45

Asn Asp Val Glu Gly Phe Arg Arg Gln Leu Ser Asp Val Ser Cys Ile
50 55 60

Asn Gln Met Gly Leu Trp Tyr Arg Arg Gln Arg Phe Val Arg Arg Met
65 70 75 80

Val Leu Glu Gln Arg Thr Pro Leu Met Val Ala Ser Leu Tyr Gly Ser
85 90 95

Leu Asp Val Val Lys Phe Ile Leu Ser Phe Pro Glu Ala Glu Leu Asn
100 105 110

Leu Ser Cys Gly Pro Asp Lys Ser Thr Ala Leu His Cys Ala Ala Ser
115 120 125

Gly Ala Ser Val Asn Ser Leu Asp Val Val Lys Leu Leu Leu Ser Val
130 135 140

Gly Ala Asp Pro Asn Ile Pro Asp Ala His Gly Asn Arg Pro Val Asp
145 150 155 160

Val Leu Val Val Ser Pro His Ala Pro Gly Leu Arg Thr Ile Leu Glu
165 170 175

Glu Ile Leu Lys Lys Asp Glu Ile Ile Ser Glu Asp Leu His Ala Ser
180 185 190

Ser Ser Ser Leu Gly Ser Ser Phe Arg Ser Leu Ser Ser Ser Pro Asp
195 200 205

Asn Gly Ser Ser Leu Leu Ser Leu Asp Ser Val Ser Ser Pro Thr Lys
    210                 215                 220

Pro His Gly Thr Asp Val Thr Phe Ala Ser Glu Lys Lys Glu Tyr Pro
225                 230                 235                 240

Ile Asp Pro Ser Leu Pro Asp Ile Lys Ser Gly Ile Tyr Ser Thr Asp
                245                 250                 255

Glu Phe Arg Met Phe Ser Phe Lys Ile Arg Pro Cys Ser Arg Ala Tyr
            260                 265                 270

Ser His Asp Trp Thr Glu Cys Pro Phe Ala His Pro Gly Glu Asn Ala
        275                 280                 285

Arg Arg Arg Asp Pro Arg Lys Phe His Tyr Thr Cys Val Pro Cys Pro
    290                 295                 300

Asp Phe Lys Lys Gly Ser Cys Lys Gln Gly Asp Met Cys Glu Tyr Ala
305                 310                 315                 320

His Gly Val Phe Glu Cys Trp Leu His Pro Ala Gln Tyr Arg Thr Arg
                325                 330                 335

Leu Cys Lys Asp Gly Met Gly Cys Asn Arg Arg Val Cys Phe Phe Ala
            340                 345                 350

His Ala Asn Glu Glu Leu Arg Pro Leu Tyr Pro Ser Thr Gly Ser Gly
        355                 360                 365

Leu Pro Ser Pro Arg Ala Ser Ser Ala Val Ser Ala Ser Thr Met Asp
    370                 375                 380

Met Ala Ser Val Leu Asn Met Leu Pro Gly Ser Pro Ser Ala Ala Gln
385                 390                 395                 400

His Ser Phe Thr Pro Pro Ile Ser Pro Ser Gly Asn Gly Ser Met Pro
                405                 410                 415

His Ser Ser Met Gly Trp Pro Gln Gln Asn Ile Pro Ala Leu Asn Leu
            420                 425                 430

Pro Gly Ser Asn Ile Gln Leu Ser Arg Leu Arg Ser Ser Leu Asn Ala
        435                 440                 445

Arg Asp Ile Pro Ser Glu Gln Leu Ser Met Leu His Glu Phe Glu Met
    450                 455                 460

Gln Arg Gln Leu Ala Gly Asp Met His Ser Pro Arg Phe Met Asn His
465 470 475 480

Ser Ala Arg Pro Lys Thr Leu Asn Pro Ser Asn Leu Glu Glu Leu Phe
485 490 495

Ser Ala Glu Val Ala Ser Pro Arg Phe Ser Asp Gln Leu Ala Val Ser
500 505 510

Ser Val Leu Ser Pro Ser His Lys Ser Ala Leu Leu Asn Gln Leu Gln
515 520 525

Asn Asn Lys Gln Ser Met Leu Ser Pro Ile Lys Thr Asn Leu Met Ser
530 535 540

Ser Pro Lys Asn Val Glu Gln His Ser Leu Leu Gln Gln Ala Ser Ser
545 550 555 560

Pro Arg Gly Gly Glu Pro Ile Ser Pro Met Asn Ala Arg Met Lys Gln
565 570 575

Gln Leu His Ser Arg Ser Leu Ser Ser Arg Asp Phe Gly Ser Ser Leu
580 585 590

Pro Arg Asp Leu Met Pro Thr Asp Ser Gly Ser Pro Leu Ser Pro Trp
595 600 605

Ser Ser Trp Asp Gln Thr His Gly Ser Lys Val Asp Trp Ser Val Gln
610 615 620

Ser Asp Glu Leu Gly Arg Leu Arg Lys Ser His Ser Leu Ala Asn Asn
625 630 635 640

Pro Asn Arg Glu Ala Asp Val Ser Trp Ala Gln Gln Met Leu Lys Asp
645 650 655

Ser Ser Ser Pro Arg Asn Gly Asn Arg Val Val Asn Met Asn Gly Ala
660 665 670

Arg Pro Leu Thr Gln Gly Gly Ser Ser Val Asn Pro His Asn Ser Asp
675 680 685

Thr Arg Glu Ser Asp Ile Leu Asp Ala Trp Leu Glu Gln Leu His Leu
690 695 700

Asp Arg
705

<210> 20

<211> 2674
<212> DNA
<213> Oryza sativa

<400> 20
tgcgagtcct cctcctcttc tcgtcgccgt gctactctcg ctttctctct ctctctctct 60

cacttgttcc ccaaggcgag aagcagccgc cgccggcgag cgtcgcgggg gaggggaggg 120

aagggaggga ggagcggtgg atccgggctt gattggattg ggtcggattc gattttggat 180

caaccccgga gggcgggagc ggttgctaca gatgcgttga gctttggtta atctatccgg 240

cgagagataa tgggcgagct tgctgatctc gttgtcgtgc cgtcgcagcc gccgctcgcc 300

ggcggccggc gggacaggct ggcggcgctg ctggagctcg cggcggcgga tgatgttgat 360

gggctcaggg gggcgctcgc ggagggaggc gaggaggcgg cggagttggc tgatggggtc 420

gggctgtggt atggtcggag caaggcgtac gaggcgcgca cgccgctgat ggtggcggcg 480

acgtacggca gcgccggggt ggtctcgctg ctggtgggcc tcggcggttg cgtcgacgtc 540

aaccgtcgcc ctggagccga cggcgccacc gcgctccact gcgccgcctc cggtggctcg 600

cgcaacgccg tcgctgttgt caagctgctt ttggccgctg gcgccgatcc ggccaccccc 660

gattccgccg gccgcttccc cgccgacgtc atcctagctc ctccggcttc gccagatgcc 720

cttggcgatc tcgaggtgct cctcggccgc cgccgagcac tcgccgtggc gacctcggtg 780

gcttcaggtt cgtcatcccc tccgctctcg tcctcaccag atgagggcaa caggtcgccc 840

tcgtcgcgtt cgtcgtcgct gtctcccatc actgtggatc gtgggaagaa ggagtatccg 900

gtggatccaa ctctgccgga catcaagagc agcgtgtatg cttcggatga gttccgcatg 960

tttgcgttca aggtccggcc ctgctcccgt gcctactcac acgactggac tgagtgcccg 1020

tttgtgcacc ccggcgagaa cgcccgccgc cgtgatcccc gcaagcaccc atacactgct 1080

gtgccttgcc ccaactttcg ccggcctggt ggctgcccta gcggcgatag ctgtgagttc 1140

tcgcatggcg tgtttgagag ctggctacac ccatcacagt atcgcacaag gctctgcaag 1200

gagggagcag cttgcgcccg tcgcatttgc ttctttgccc atgatgagga tgagctccgc 1260

catgtgcctc acaacagtgg tgccggcctg ctgtctcccc gcgcttcttc atccattgat 1320

atgactgctg cagctgcgct cgggcttctt ccaggttctc ctaccagaca ctttgcaccg 1380

ccgcctgtgt caccatctgc tgggagcaat ggaggagctg ctgctgcgca ttggctccaa 1440

ggcagtaggc tgcgttcttc tttcaatgca agggatgctg ctgttgatga ccttggcatg 1500

ctcctcgaat gggaatcaca ataccttggg gcactctgcc tgccacccag cagccgcccc 1560

caaccacgcc tttcagctgg tctgagtatc aggccaacaa ttgctccatc caatcttgaa 1620

gacatgtatg cttcagacat ggcaatgtct ccgaggttcc ctaatgacca aggtcactca 1680

gtctactcac cagcccacaa atcagccctc ctcaacaagc ttcatcaaca gaagggcctc 1740

ttatcacctg ttaacaccaa cagaatgtac tccccaaggg ctcttgatcc gtcatctttg 1800

gcacattctc catttggtgg catgtctccc cggtcccccc gtaccatgga acctacatca 1860

cccctaagtg ctcgtgtagg agcccctgcc acacagcggc cttctgttgg ttcaccacgg 1920

aattccagtg cttggggcac cgtggggtcc ccgatgggta aggttgactg ggtgtcgat 1980

agcgaggagc tagtccgctt gagacgccct gcacaaccag ggtttggaga agatgagaca 2040

gatgtatcat gggtgcagtc actggtaagc aatgctgagc ttaatggcaa gaggggcgaa 2100

gtacaaggca tgcctggtac ttctgcattg atgaacaggc ctgacctgaa caatcagggt 2160

gacttgttgg accagacggt gatcggtgct tggcttgagc agatgcacct ggatcagaag 2220

tgatttccaa gggaagccat gaagtcccaa agtggatgaa gcctttattt tgccaaggtt 2280

atttaccaaa gaatagttgt tggtcctagt aaataataat ttattctttt taattcttga 2340

aatttttggt gggcaaagtc agagatggtg gtcaagttca acaaaacatt tggtcacaga 2400

ttggtagctg aaatcagttc cagagattgg taaacaacct cattacttgg ggtcctaact 2460

agtattcttt tgattagctc agatgagtct ttattttagt gggttaaaat tcatatgttc 2520

cccatggtta ttatgtccat gatctcttcc taacaaaaga gagattataa ttgtccattt 2580

ttcatttatc aatgaatgat tttgttaaaa caatgtaagt tacattctta atttttctc 2640

tgttcaatgg aattaccttc cttggttagt cctc 2674

<210> 21
<211> 657
<212> PRT
<213> Oryza sativa

<400> 21

Met Gly Glu Leu Ala Asp Leu Val Val Val Pro Ser Gln Pro Pro Leu
1 5 10 15

Ala Gly Gly Arg Arg Asp Arg Leu Ala Ala Leu Leu Glu Leu Ala Ala
20 25 30

Ala Asp Asp Val Asp Gly Leu Arg Gly Ala Leu Ala Glu Gly Gly Glu
35 40 45

Glu Ala Ala Glu Leu Ala Asp Gly Val Gly Leu Trp Tyr Gly Arg Ser
50 55 60

Lys Ala Tyr Glu Ala Arg Thr Pro Leu Met Val Ala Ala Thr Tyr Gly
65 70 75 80

Ser Ala Gly Val Val Ser Leu Leu Val Gly Leu Gly Gly Cys Val Asp
85 90 95

Val Asn Arg Arg Pro Gly Ala Asp Gly Ala Thr Ala Leu His Cys Ala
100 105 110

```
Ala Ser Gly Gly Ser Arg Asn Ala Val Ala Val Val Lys Leu Leu Leu
        115                 120                 125

Ala Ala Gly Ala Asp Pro Ala Thr Pro Asp Ser Ala Gly Arg Phe Pro
    130                 135                 140

Ala Asp Val Ile Leu Ala Pro Pro Ala Ser Pro Asp Ala Leu Gly Asp
145                 150                 155                 160

Leu Glu Val Leu Leu Gly Arg Arg Arg Ala Leu Ala Val Ala Thr Ser
                165                 170                 175

Val Ala Ser Gly Ser Ser Ser Pro Pro Leu Ser Ser Ser Pro Asp Glu
            180                 185                 190

Gly Asn Arg Ser Pro Ser Ser Arg Ser Ser Ser Leu Ser Pro Ile Thr
        195                 200                 205

Val Asp Arg Gly Lys Lys Glu Tyr Pro Val Asp Pro Thr Leu Pro Asp
    210                 215                 220

Ile Lys Ser Ser Val Tyr Ala Ser Asp Glu Phe Arg Met Phe Ala Phe
225                 230                 235                 240

Lys Val Arg Pro Cys Ser Arg Ala Tyr Ser His Asp Trp Thr Glu Cys
                245                 250                 255

Pro Phe Val His Pro Gly Glu Asn Ala Arg Arg Arg Asp Pro Arg Lys
            260                 265                 270

His Pro Tyr Thr Ala Val Pro Cys Pro Asn Phe Arg Arg Pro Gly Gly
        275                 280                 285

Cys Pro Ser Gly Asp Ser Cys Glu Phe Ser His Gly Val Phe Glu Ser
    290                 295                 300

Trp Leu His Pro Ser Gln Tyr Arg Thr Arg Leu Cys Lys Glu Gly Ala
305                 310                 315                 320

Ala Cys Ala Arg Arg Ile Cys Phe Phe Ala His Asp Glu Asp Glu Leu
                325                 330                 335

Arg His Val Pro His Asn Ser Gly Ala Gly Leu Leu Ser Pro Arg Ala
            340                 345                 350

Ser Ser Ser Ile Asp Met Thr Ala Ala Ala Ala Leu Gly Leu Leu Pro
        355                 360                 365
```

```
Gly Ser Pro Thr Arg His Phe Ala Pro Pro Pro Val Ser Pro Ser Ala
    370                 375                 380

Gly Ser Asn Gly Gly Ala Ala Ala Ala His Trp Leu Gln Gly Ser Arg
385                 390                 395                 400

Leu Arg Ser Ser Phe Asn Ala Arg Asp Ala Ala Val Asp Asp Leu Gly
                405                 410                 415

Met Leu Leu Glu Trp Glu Ser Gln Tyr Leu Gly Ala Leu Cys Leu Pro
            420                 425                 430

Pro Ser Ser Arg Pro Gln Pro Arg Leu Ser Ala Gly Leu Ser Ile Arg
        435                 440                 445

Pro Thr Ile Ala Pro Ser Asn Leu Glu Asp Met Tyr Ala Ser Asp Met
    450                 455                 460

Ala Met Ser Pro Arg Phe Pro Asn Asp Gln Gly His Ser Val Tyr Ser
465                 470                 475                 480

Pro Ala His Lys Ser Ala Leu Leu Asn Lys Leu His Gln Gln Lys Gly
                485                 490                 495

Leu Leu Ser Pro Val Asn Thr Asn Arg Met Tyr Ser Pro Arg Ala Leu
            500                 505                 510

Asp Pro Ser Ser Leu Ala His Ser Pro Phe Gly Gly Met Ser Pro Arg
        515                 520                 525

Ser Pro Arg Thr Met Glu Pro Thr Ser Pro Leu Ser Ala Arg Val Gly
    530                 535                 540

Ala Pro Ala Thr Gln Arg Pro Ser Val Gly Ser Pro Arg Asn Ser Ser
545                 550                 555                 560

Ala Trp Gly Thr Val Gly Ser Pro Met Gly Lys Val Asp Trp Gly Val
                565                 570                 575

Asp Ser Glu Glu Leu Val Arg Leu Arg Arg Pro Ala Gln Pro Gly Phe
            580                 585                 590

Gly Glu Asp Glu Thr Asp Val Ser Trp Val Gln Ser Leu Val Ser Asn
        595                 600                 605

Ala Glu Leu Asn Gly Lys Arg Gly Glu Val Gln Gly Met Pro Gly Thr
    610                 615                 620

Ser Ala Leu Met Asn Arg Pro Asp Leu Asn Asn Gln Gly Asp Leu Leu
```

625 630 635 640

Asp Gln Thr Val Ile Gly Ala Trp Leu Glu Gln Met His Leu Asp Gln
645 650 655

Lys

<210> 22
<211> 2223
<212> DNA
<213> Arabidopsis thaliana

<400> 22
ttcttcaaaa accccaacca cttcttctcc ccaaaaacct ccaaagtttc aatctttact 60

tctctctttt tctccaagtt atcttctttt ctaggaagag atatgtgcgg tgcaaagagc 120

aacctttgct catctaaaac cctaacagaa gtcgaattca tgaggcagaa atcagaagac 180

ggagcttccg ccacgtgtct cctcgaattc gccgcctgtg atgatctttc atcgtttaag 240

agagagatcg aagagaatcc atcggtggag attgatgagt cagggttttg gtattgcaga 300

cgggtcgggt ctaagaagat gggttttgaa gaaagaacac cacttatggt tgctgctatg 360

tatggaagca tggaagtgtt gaattacata attgccacag gaagatccga tgtgaacaga 420

gtttgcagtg acgagaaagt cactgctctt cactgtgcag tttctggctg ttctgtttct 480

atcgttgaga tcatcaagat cttgcttgat gcttctgctt cacctaattg tgttgacgct 540

aatgggaaca aaccggttga tttgttggct aaagattctc ggtttgttcc taaccagagt 600

agaaaggcgg ttgaggtttt actgaccggg attcatggtt cggttatgga agaagaggag 660

gaggaactga agagtgttgt gactaagtat ccagctgatg catcacttcc tgatattaac 720

gaaggtgttt atggaactga tgattttagg atgtttagct ttaaggttaa gccatgttct 780

agggcttatt cacatgattg gactgaatgt ccttttgttc atcctggtga gaatgcaagg 840

aggagagatc ctaggaagta tccttacact tgtgtgcctt gtcccgagtt tcgtaaaggg 900

tcttgtccta aaggagattc gtgtgagtac gcgcacggtg ttttcgagtc ttggcttcac 960

ccggcgcagt ataggacacg gctttgcaaa gatgagactg gttgtgctag gagagtttgt 1020

ttctttgctc atagacggga tgagttaaga ccggttaatg cttctactgg ttctgcaatg 1080

gtttcaccaa ggtcgtctaa tcagtctcct gagatgtctg ttatgtctcc tttgacgctg 1140

ggatcatcgc caatgaactc tcctatggct aatggtgttc ctttgtctcc aagaaatggt 1200

ggtttatggc agaacagagt taatagcctt acaccaccac cgttgcagct taatggtagc 1260

agattgaagt cgactttgag tgctagagat atggatatgg agatggaact taggtttcgc 1320

ggtttggata accggagact tggtgatctc aagccatcca acctcgaaga gactttcgga 1380

tcatatgact cagcttctgt gatgcaactt caatcaccaa gcaggcattc tcagatgaac 1440

```
cactatccgt cttcacctgt gaggcagcct cctcctcatg gattcgaatc ttcagcagcc   1500

atggcagctg cagtgatgaa tgcaagatcc tcagcgtttg cgaaacgcag cttgagtttc   1560

aaaccagctc cagtagcttc taatgtctcc gattggggat caccaaatgg gaagcttgag   1620

tggggaatgc aaagagatga gctgaacaag ttgaggagaa gtgcctcctt cggcattcat   1680

ggaaacaaca acaacagtgt gtcacgccct gctagagact acagtgacga gccagatgtg   1740

tcgtgggtga actcactggt gaaagagaat gcaccagaga gagtgaatga gagggttggg   1800

aatacggtga atggtgcagc gagtagagac aagtttaagc tgccgtcgtg ggcagagcaa   1860

atgtatatag accatgagca gcagattgtg gcataagaag cagaaagaaa gatgtgggat   1920

ttatattgct tttgtcttct gggcctctct acacagaatc taacaaatct ggcaataatt   1980

ctttgatttg tgtttgaccc atagtttggt tactagtata tgttttttta tgttcttttt   2040

ttctttgtca ttctcttgtc cttcgtgaca ctatgtaatg attaaaagca aataattgat   2100

gcatgagttc aaatgttctt tgaaggatcc atcttattag ctttgtaatt gttgtgatat   2160

cttaatctta ttggttacgt atttcaagtg ctttagaaaa aatgggccta agagattttg   2220

ggg                                                                 2223
```

<210> 23
<211> 597
<212> PRT
<213> Arabidopsis thaliana

<400> 23

```
Met Cys Gly Ala Lys Ser Asn Leu Cys Ser Ser Lys Thr Leu Thr Glu
1               5                   10                  15

Val Glu Phe Met Arg Gln Lys Ser Glu Asp Gly Ala Ser Ala Thr Cys
            20                  25                  30

Leu Leu Glu Phe Ala Ala Cys Asp Asp Leu Ser Ser Phe Lys Arg Glu
        35                  40                  45

Ile Glu Glu Asn Pro Ser Val Glu Ile Asp Glu Ser Gly Phe Trp Tyr
    50                  55                  60

Cys Arg Arg Val Gly Ser Lys Lys Met Gly Phe Glu Glu Arg Thr Pro
65                  70                  75                  80

Leu Met Val Ala Ala Met Tyr Gly Ser Met Glu Val Leu Asn Tyr Ile
                85                  90                  95

Ile Ala Thr Gly Arg Ser Asp Val Asn Arg Val Cys Ser Asp Glu Lys
            100                 105                 110

Val Thr Ala Leu His Cys Ala Val Ser Gly Cys Ser Val Ser Ile Val
```

115 120 125

Glu Ile Ile Lys Ile Leu Leu Asp Ala Ser Ala Ser Pro Asn Cys Val
130 135 140

Asp Ala Asn Gly Asn Lys Pro Val Asp Leu Leu Ala Lys Asp Ser Arg
145 150 155 160

Phe Val Pro Asn Gln Ser Arg Lys Ala Val Glu Val Leu Leu Thr Gly
165 170 175

Ile His Gly Ser Val Met Glu Glu Glu Glu Glu Glu Leu Lys Ser Val
180 185 190

Val Thr Lys Tyr Pro Ala Asp Ala Ser Leu Pro Asp Ile Asn Glu Gly
195 200 205

Val Tyr Gly Thr Asp Asp Phe Arg Met Phe Ser Phe Lys Val Lys Pro
210 215 220

Cys Ser Arg Ala Tyr Ser His Asp Trp Thr Glu Cys Pro Phe Val His
225 230 235 240

Pro Gly Glu Asn Ala Arg Arg Arg Asp Pro Arg Lys Tyr Pro Tyr Thr
245 250 255

Cys Val Pro Cys Pro Glu Phe Arg Lys Gly Ser Cys Pro Lys Gly Asp
260 265 270

Ser Cys Glu Tyr Ala His Gly Val Phe Glu Ser Trp Leu His Pro Ala
275 280 285

Gln Tyr Arg Thr Arg Leu Cys Lys Asp Glu Thr Gly Cys Ala Arg Arg
290 295 300

Val Cys Phe Phe Ala His Arg Arg Asp Glu Leu Arg Pro Val Asn Ala
305 310 315 320

Ser Thr Gly Ser Ala Met Val Ser Pro Arg Ser Ser Asn Gln Ser Pro
325 330 335

Glu Met Ser Val Met Ser Pro Leu Thr Leu Gly Ser Ser Pro Met Asn
340 345 350

Ser Pro Met Ala Asn Gly Val Pro Leu Ser Pro Arg Asn Gly Gly Leu
355 360 365

Trp Gln Asn Arg Val Asn Ser Leu Thr Pro Pro Pro Leu Gln Leu Asn
370 375 380

```
Gly Ser Arg Leu Lys Ser Thr Leu Ser Ala Arg Asp Met Asp Met Glu
385                 390                 395                 400

Met Glu Leu Arg Phe Arg Gly Leu Asp Asn Arg Arg Leu Gly Asp Leu
                405                 410                 415

Lys Pro Ser Asn Leu Glu Glu Thr Phe Gly Ser Tyr Asp Ser Ala Ser
            420                 425                 430

Val Met Gln Leu Gln Ser Pro Ser Arg His Ser Gln Met Asn His Tyr
        435                 440                 445

Pro Ser Ser Pro Val Arg Gln Pro Pro Pro His Gly Phe Glu Ser Ser
    450                 455                 460

Ala Ala Met Ala Ala Ala Val Met Asn Ala Arg Ser Ser Ala Phe Ala
465                 470                 475                 480

Lys Arg Ser Leu Ser Phe Lys Pro Ala Pro Val Ala Ser Asn Val Ser
                485                 490                 495

Asp Trp Gly Ser Pro Asn Gly Lys Leu Glu Trp Gly Met Gln Arg Asp
            500                 505                 510

Glu Leu Asn Lys Leu Arg Arg Ser Ala Ser Phe Gly Ile His Gly Asn
        515                 520                 525

Asn Asn Asn Ser Val Ser Arg Pro Ala Arg Asp Tyr Ser Asp Glu Pro
    530                 535                 540

Asp Val Ser Trp Val Asn Ser Leu Val Lys Glu Asn Ala Pro Glu Arg
545                 550                 555                 560

Val Asn Glu Arg Val Gly Asn Thr Val Asn Gly Ala Ala Ser Arg Asp
                565                 570                 575

Lys Phe Lys Leu Pro Ser Trp Ala Glu Gln Met Tyr Ile Asp His Glu
            580                 585                 590

Gln Gln Ile Val Ala
        595
```

<210> 24
<211> 1761
<212> DNA
<213> Arabidopsis thaliana

<400> 24
atggaaaaag atagtattat gtgcagtgga ccaaagagca atctctgctc ttcaagaacc 60

ttaacagaaa tcgaatcaag gcaaaaggaa gaagaaacaa tgcttctcct cgaattcgct 120

gcttgtgatg atcttgactc gttcaagaga gaggttgaag agaaagggct tgatttggat 180

gagtcagggt tatggtattg cagacgtgtc ggttctaaga agatgggtct tgaagaaaga 240

acacctttaa tggttgcagc tatgtatgga agcataaagg ttttgacttt catcgtttcc 300

actggaaaat ctgatgtgaa cagagcttgt ggtgaagaga gagttactcc gcttcactgt 360

gctgttgctg gctgttctgt gaatatgatt gaagtcatca atgtcttgct tgatgcttct 420

gctttggtta actctgttga tgctaatggg aatcaacctt tggatgtgtt tgttcgagtt 480

tcgaggtttg tggctagtcc gaggaggaaa gcggttgagt tgttgctgag aggaggaggt 540

gttggaggat tgatcgatga ggcggttgaa gaagagatca agattgtctc taagtatcca 600

gctgatgctt ctttaccgga tataaacgaa ggggtttatg gaagtgatga gtttaggatg 660

tatagcttta aggttaagcc atgttctagg gcttattctc atgattggac cgagtgtgct 720

tttgttcatc cgggagaaaa tgcgaggagg agagatccga ggaagtatcc ttacacttgt 780

gtcccctgtc ccgagttccg taaaggatca tgcccgaaag gagattcttg cgagtatgct 840

cacggggttt tcgagtcgtg gcttcacccc gcgcagtata aaacccggct ttgtaaagat 900

gaaacgggtt gtgcaaggaa agtttgtttc tttgctcata aacgcgaaga gatgagacct 960

gttaatgctt caactggctc tgccgtggct cagtctccgt ttagcagctt ggagatgatg 1020

ccagggttgt ctcctcttgc ttattcttca ggagtttcga ctcctccggt ttctccaatg 1080

gctaatggtg ttccttcctc tccaagaaac ggcggatcat ggcagaacag agtcaatacc 1140

cttactccac cggctttgca gctcaatggt ggaagcagat tgaagtccac actgagcgct 1200

agagatatcg atatggagat ggagatggaa ttgagactcc gcggttttgg caacaatgtg 1260

gaagagacgt tcgggtctta tgtttcctct ccaagtagga attctcaaat gggtcaaaac 1320

atgaaccaac attatccatc ttccccggtg agacaaccgc catctcaaca cgggttcgaa 1380

tcttcagcag ctgcagcggt tgcagtgatg aaagcgagat caaccgcctt tgcgaaacgt 1440

agcttgagct tcaaaccagc tactcaagca gcaccacagt cgaatctctc ggattgggga 1500

tctccaaacg ggaagctgga atggggaatg aaaggagaag agctgaataa gatgagaaga 1560

agtgtttcct ttggaatcca tggaaacaac aacaataacg cagctagaga ctacagggac 1620

gagccagatg tgtcatgggt taactcttta gttaaagaca gtactgtggt gtctgagaga 1680

agctttggaa tgaatgagag ggttcggata atgtcgtggg ctgagcaaat gtacagagag 1740

aaggagcaga ctgtggtgta a 1761

<210> 25
<211> 586
<212> PRT
<213> Arabidopsis thaliana

<400> 25

Met Glu Lys Asp Ser Ile Met Cys Ser Gly Pro Lys Ser Asn Leu Cys
1               5                   10                  15

Ser Ser Arg Thr Leu Thr Glu Ile Glu Ser Arg Gln Lys Glu Glu Glu
            20                  25                  30

Thr Met Leu Leu Leu Glu Phe Ala Ala Cys Asp Asp Leu Asp Ser Phe
        35                  40                  45

Lys Arg Glu Val Glu Glu Lys Gly Leu Asp Leu Asp Glu Ser Gly Leu
    50                  55                  60

Trp Tyr Cys Arg Arg Val Gly Ser Lys Lys Met Gly Leu Glu Glu Arg
65                  70                  75                  80

Thr Pro Leu Met Val Ala Ala Met Tyr Gly Ser Ile Lys Val Leu Thr
                85                  90                  95

Phe Ile Val Ser Thr Gly Lys Ser Asp Val Asn Arg Ala Cys Gly Glu
            100                 105                 110

Glu Arg Val Thr Pro Leu His Cys Ala Val Ala Gly Cys Ser Val Asn
        115                 120                 125

Met Ile Glu Val Ile Asn Val Leu Leu Asp Ala Ser Ala Leu Val Asn
    130                 135                 140

Ser Val Asp Ala Asn Gly Asn Gln Pro Leu Asp Val Phe Val Arg Val
145                 150                 155                 160

Ser Arg Phe Val Ala Ser Pro Arg Arg Lys Ala Val Glu Leu Leu Leu
                165                 170                 175

Arg Gly Gly Gly Val Gly Gly Leu Ile Asp Glu Ala Val Glu Glu Glu
            180                 185                 190

Ile Lys Ile Val Ser Lys Tyr Pro Ala Asp Ala Ser Leu Pro Asp Ile
        195                 200                 205

Asn Glu Gly Val Tyr Gly Ser Asp Glu Phe Arg Met Tyr Ser Phe Lys
    210                 215                 220

Val Lys Pro Cys Ser Arg Ala Tyr Ser His Asp Trp Thr Glu Cys Ala
225                 230                 235                 240

Phe Val His Pro Gly Glu Asn Ala Arg Arg Arg Asp Pro Arg Lys Tyr
                245                 250                 255

```
Pro Tyr Thr Cys Val Pro Cys Pro Glu Phe Arg Lys Gly Ser Cys Pro
            260                 265                 270

Lys Gly Asp Ser Cys Glu Tyr Ala His Gly Val Phe Glu Ser Trp Leu
        275                 280                 285

His Pro Ala Gln Tyr Lys Thr Arg Leu Cys Lys Asp Glu Thr Gly Cys
    290                 295                 300

Ala Arg Lys Val Cys Phe Phe Ala His Lys Arg Glu Glu Met Arg Pro
305                 310                 315                 320

Val Asn Ala Ser Thr Gly Ser Ala Val Ala Gln Ser Pro Phe Ser Ser
                325                 330                 335

Leu Glu Met Met Pro Gly Leu Ser Pro Leu Ala Tyr Ser Ser Gly Val
            340                 345                 350

Ser Thr Pro Pro Val Ser Pro Met Ala Asn Gly Val Pro Ser Ser Pro
        355                 360                 365

Arg Asn Gly Gly Ser Trp Gln Asn Arg Val Asn Thr Leu Thr Pro Pro
    370                 375                 380

Ala Leu Gln Leu Asn Gly Gly Ser Arg Leu Lys Ser Thr Leu Ser Ala
385                 390                 395                 400

Arg Asp Ile Asp Met Glu Met Glu Met Glu Leu Arg Leu Arg Gly Phe
                405                 410                 415

Gly Asn Asn Val Glu Glu Thr Phe Gly Ser Tyr Val Ser Ser Pro Ser
            420                 425                 430

Arg Asn Ser Gln Met Gly Gln Asn Met Asn Gln His Tyr Pro Ser Ser
        435                 440                 445

Pro Val Arg Gln Pro Pro Ser Gln His Gly Phe Glu Ser Ser Ala Ala
    450                 455                 460

Ala Ala Val Ala Val Met Lys Ala Arg Ser Thr Ala Phe Ala Lys Arg
465                 470                 475                 480

Ser Leu Ser Phe Lys Pro Ala Thr Gln Ala Ala Pro Gln Ser Asn Leu
                485                 490                 495

Ser Asp Trp Gly Ser Pro Asn Gly Lys Leu Glu Trp Gly Met Lys Gly
            500                 505                 510
```

Glu Glu Leu Asn Lys Met Arg Arg Ser Val Ser Phe Gly Ile His Gly
515 520 525

Asn Asn Asn Asn Asn Ala Ala Arg Asp Tyr Arg Asp Glu Pro Asp Val
530 535 540

Ser Trp Val Asn Ser Leu Val Lys Asp Ser Thr Val Val Ser Glu Arg
545 550 555 560

Ser Phe Gly Met Asn Glu Arg Val Arg Ile Met Ser Trp Ala Glu Gln
565 570 575

Met Tyr Arg Glu Lys Glu Gln Thr Val Val
580 585

<210> 26
<211> 2709
<212> DNA
<213> Eucalyptus grandis

<400> 26
cttctgaaag ctttttgact taagacgaga gagaaggaga gaaggtcccc ctcctcgtcc 60

tcgtccccccc gtggattttg aagaagaaaa gtcgcacctt ccttctcctt tcccactcct 120

ccctctgctc gaagcttttc tcttccgcag aattacataa aaacctcgac tttgcgcatc 180

attccgattc acctcacacc ttcactttcc cactcgaggt ctccccctc ttttcctagc 240

tctttccctt tccctccctc tctctcgaga atcgccgcat ttggaggagc tccaatctgc 300

tttgctttgc tttgctctct tcttgctcgg ttccccctca taaggagtcg attatgtgca 360

acggttcttc gaagggtaaa cttttcccct cgagtatggg catggagggc gaattccaca 420

acaaggatgg cgaagcaccc cgtaaatgct ctgccttgct tgaattggca gcctcggacg 480

atctctcgtc gttcaaaagt gaagtggaag agaagggctg cgacgttgat gaggccagct 540

tttggtatgg taggagaatc gggtcgaaga agatgggttt tgaagagagg actccattga 600

tgatctctgc tttgtttgga agcaccaagg tcttgaaata cataatcgag accgccagag 660

ctgatgtcaa caggtcttgt gggtccgaca aggtggccgc cctccattgc gcagccgcgg 720

gtgggtccag ttcttcactt gaaattgtga agctcttgat tgaggcctca gcggatatta 780

attctgtaga tggcaatgga aataggccca tcgacgtgct tgccccggca gggaagtctc 840

gctgcaattc cagaaataag tttgttagat cgttgctgaa aggtgaaaac tatgtcgtgg 900

aaggtgacca atcctttgac atagaaggag aggagaagct agtcgctctt ccaaaggagg 960

gaggcgagaa gaaagagtat cctgttgatg tctctctacc tgacataaac aatgggttct 1020

acagtaccga tgagttccgg atgtatgctt tcaaggtgaa gccttgctcg agggcttact 1080

cccacgactg gaccgagtgc ccgtttgtgc accctgggga gaacgcgagg aggagggacc 1140

cacgcaagta cccttacagc tgtgtccctt gtcctgagtt tcgcaagggt tcgtgcgtaa 1200

gggggggatgc ttgtgagtat gctcatggag tctttgagtc gtggcttcac ccagcgcaat 1260

accgaacccg gctgtgcaag gatgaaactg gttgtactcg caaagtttgc ttctttgctc 1320

acaagtccga agaattgcgt cccgtgtatg cttccacagg ttctgctatg ccctcaccca 1380

agtccttttc agctaatgcc ctagacatga caaccctgag ccccttatcc cttaattcac 1440

catctctgcc tttgcctgct acttccacgc cccccatgtc acctttggct gcctcatctt 1500

cacccaaggg catgaacttg tggcataaca aaattaacct gaccccacca agcctgcagc 1560

ttcctggcag ccggctgaag acggctatga gtgcgcggga cttcgatttt gagttggaat 1620

ttcttgggct ggaaaagcaa gcttctcagc ggcagcaact gatagaagag atttctcgtc 1680

tctcatcgcc ctctcatatg tggaactcgg aatttggcag aaccgcagag ctgaagccca 1740

ctaaccttga tgatgcgttt ggatctcttg acacttctct tttgtctccg ttgcaggggt 1800

cgtcgatgaa aacatcgact cctacccagt tgcaatcccc cacagggctt aaaatttcga 1860

atttgaacca actccgtgcg agctacccgt ctagcagctt gtcgtcctct cctgtgagga 1920

agacctcttc ttttgggttc gactcatcca gtgcagttgc tgcagcagtc atgaactcac 1980

ggtctgctgc tatgacgaag cggagccaga gcttcattga ccgtggagca gtgggtcaac 2040

ggtctggact cattggacct gctaattctg ctcctaggat gtccaacctt tcggactggg 2100

gctcgcctga tgggaagttg gattggggtg ttcaagggga cgagctcaac aagcttagga 2160

agtccgcttc cttcggcttt agaaacaaca gtatggcgaa cccaaacaac gtggcgtctc 2220

ccagtgctga tgagccggac gtgtcgtggg ttggttcatt ggtgaaggat gtggctccgc 2280

ccgaagggta tccacagtat ctgtacatag aacaggagca gatggtggca taactaaagc 2340

gaagagcacc acacgaactc tctcctgatg gcttaagatg acttgtttga cattctttat 2400

attcttacaa acagcgcgtt cttaggagtt agctggagga aagaaggaaa cggtattgag 2460

tttgagattc aggctcttag ctggacagcg aaaatttggg gaaggaagag aatttggttt 2520

cttgcccaac ttagataatg atgcttttga aggcttaaaa gaaagatgaa ggcaaacatt 2580

cttttgttag tattgtatta ttgttttaat ttttcatccc ctctgtcggg gtgtggtggg 2640

tgtcgatgtt tctttcatca gtaaaatata taatgaggtt tactcatcta ttttctacta 2700

aaaaaaaaa 2709

<210> 27
<211> 659
<212> PRT
<213> Eucalyptus grandis

<400> 27

Met Cys Asn Gly Ser Ser Lys Gly Lys Leu Phe Pro Ser Ser Met Gly
1 5 10 15

```
Met Glu Gly Glu Phe His Asn Lys Asp Gly Glu Ala Pro Arg Lys Cys
            20                  25                  30

Ser Ala Leu Leu Glu Leu Ala Ala Ser Asp Asp Leu Ser Ser Phe Lys
        35                  40                  45

Ser Glu Val Glu Glu Lys Gly Cys Asp Val Asp Glu Ala Ser Phe Trp
    50                  55                  60

Tyr Gly Arg Arg Ile Gly Ser Lys Lys Met Gly Phe Glu Glu Arg Thr
65                  70                  75                  80

Pro Leu Met Ile Ser Ala Leu Phe Gly Ser Thr Lys Val Leu Lys Tyr
                85                  90                  95

Ile Ile Glu Thr Ala Arg Ala Asp Val Asn Arg Ser Cys Gly Ser Asp
            100                 105                 110

Lys Val Ala Ala Leu His Cys Ala Ala Ala Gly Gly Ser Ser Ser Ser
        115                 120                 125

Leu Glu Ile Val Lys Leu Leu Ile Glu Ala Ser Ala Asp Ile Asn Ser
    130                 135                 140

Val Asp Gly Asn Gly Asn Arg Pro Ile Asp Val Leu Ala Pro Ala Gly
145                 150                 155                 160

Lys Ser Arg Cys Asn Ser Arg Asn Lys Phe Val Arg Ser Leu Leu Lys
                165                 170                 175

Gly Glu Asn Tyr Val Val Glu Gly Asp Gln Ser Phe Asp Ile Glu Gly
            180                 185                 190

Glu Glu Lys Leu Val Ala Leu Pro Lys Glu Gly Gly Glu Lys Lys Glu
        195                 200                 205

Tyr Pro Val Asp Val Ser Leu Pro Asp Ile Asn Asn Gly Phe Tyr Ser
    210                 215                 220

Thr Asp Glu Phe Arg Met Tyr Ala Phe Lys Val Lys Pro Cys Ser Arg
225                 230                 235                 240

Ala Tyr Ser His Asp Trp Thr Glu Cys Pro Phe Val His Pro Gly Glu
                245                 250                 255

Asn Ala Arg Arg Arg Asp Pro Arg Lys Tyr Pro Tyr Ser Cys Val Pro
            260                 265                 270

Cys Pro Glu Phe Arg Lys Gly Ser Cys Val Arg Gly Asp Ala Cys Glu
```

```
        275                 280                 285

Tyr Ala His Gly Val Phe Glu Ser Trp Leu His Pro Ala Gln Tyr Arg
    290                 295                 300

Thr Arg Leu Cys Lys Asp Glu Thr Gly Cys Thr Arg Lys Val Cys Phe
305                 310                 315                 320

Phe Ala His Lys Ser Glu Glu Leu Arg Pro Val Tyr Ala Ser Thr Gly
                325                 330                 335

Ser Ala Met Pro Ser Pro Lys Ser Phe Ser Ala Asn Ala Leu Asp Met
            340                 345                 350

Thr Thr Leu Ser Pro Leu Ser Leu Asn Ser Pro Ser Leu Pro Leu Pro
        355                 360                 365

Ala Thr Ser Thr Pro Pro Met Ser Pro Leu Ala Ala Ser Ser Ser Pro
    370                 375                 380

Lys Gly Met Asn Leu Trp His Asn Lys Ile Asn Leu Thr Pro Pro Ser
385                 390                 395                 400

Leu Gln Leu Pro Gly Ser Arg Leu Lys Thr Ala Met Ser Ala Arg Asp
                405                 410                 415

Phe Asp Phe Glu Leu Glu Phe Leu Gly Leu Glu Lys Gln Ala Ser Gln
            420                 425                 430

Arg Gln Gln Leu Ile Glu Glu Ile Ser Arg Leu Ser Ser Pro Ser His
        435                 440                 445

Met Trp Asn Ser Glu Phe Gly Arg Thr Ala Glu Leu Lys Pro Thr Asn
    450                 455                 460

Leu Asp Asp Ala Phe Gly Ser Leu Asp Thr Ser Leu Leu Ser Pro Leu
465                 470                 475                 480

Gln Gly Ser Ser Met Lys Thr Ser Thr Pro Thr Gln Leu Gln Ser Pro
                485                 490                 495

Thr Gly Leu Lys Ile Ser Asn Leu Asn Gln Leu Arg Ala Ser Tyr Pro
            500                 505                 510

Ser Ser Ser Leu Ser Ser Ser Pro Val Arg Lys Thr Ser Ser Phe Gly
        515                 520                 525

Phe Asp Ser Ser Ser Ala Val Ala Ala Ala Val Met Asn Ser Arg Ser
    530                 535                 540
```

Ala Ala Met Thr Lys Arg Ser Gln Ser Phe Ile Asp Arg Gly Ala Val
545                 550                 555                 560

Gly Gln Arg Ser Gly Leu Ile Gly Pro Ala Asn Ser Ala Pro Arg Met
                565                 570                 575

Ser Asn Leu Ser Asp Trp Gly Ser Pro Asp Gly Lys Leu Asp Trp Gly
            580                 585                 590

Val Gln Gly Asp Glu Leu Asn Lys Leu Arg Lys Ser Ala Ser Phe Gly
        595                 600                 605

Phe Arg Asn Asn Ser Met Ala Asn Pro Asn Asn Val Ala Ser Pro Ser
    610                 615                 620

Ala Asp Glu Pro Asp Val Ser Trp Val Gly Ser Leu Val Lys Asp Val
625                 630                 635                 640

Ala Pro Pro Glu Gly Tyr Pro Gln Tyr Leu Tyr Ile Glu Gln Glu Gln
                645                 650                 655

Met Val Ala

<210> 28
<211> 2518
<212> DNA
<213> Eucalyptus grandis

<400> 28
tctccttcga gtttctttct tcactagaat tcgctcccga gtctgttgtt gctcgtgtag 60

ttttgcttac tccgtccttc gtttagctcg ctgaccagcg cggagctagg agcggtcgct 120

aaaggattac tcgtacaaaa cgtaaactca gctctgccaa ttttcccatg gagggggaat 180

cttacttcga gaaagatgaa aaatattcta attgctcaat cttgctcgaa ttatctgctt 240

cggacgatct cccagctttt gaaaggaaag cgaaagagaa gggctgtaac attgatggtg 300

ctagcttctg gtacggtaga agaattggct caaggaagat gggtcttgaa gagaggactc 360

ctctcatggt ggcttccttg tttggaagct ctagggttgt gaagtacatt ctcgaatctg 420

gcaaagtcga tgtaaatagg gcttgtggtt cggacaaggt cactgccctt cactgtgctg 480

ttgccagtgg ctctgcttct gcggtggagg ttgtcaagct cttgcttcac gcatctgccg 540

atgctaattg cattgatggc aatggaaaga agccaattga tgtgatagcc cttccattaa 600

agtcacgcgg cgattcaagg aggaagctga tggagctgtt gctgaaaggc gataattctg 660

atggggaatt tgaatcccac gaggagaagc cgattgccgc accgcaagca tccaaagagg 720

gaagcgaaaa gaaagagtat caatttcctg ttgatatctc tctgcctgac ataaatgttg 780

ggatttacag tactgatgag ttcagaatgt atgctttcaa agtaaagcct tgctcgcggg 840
catactccca tgactggaca gagtgcccat ttgttcatcc tggcgagaat gcgaggaggc 900
gggaccctcg caagtacccc tacagctgcg tcccttgccc tgaatttcgg aagggatctt 960
gccaaaaggg tgactcctgt gagtacgcgc acggcgtatt tgagtcgtgg cttcatcctg 1020
cacagtatag aacaagactg tgcaaggatg agactggatg tgctcgcaaa gtttgtttct 1080
ttgctcacaa gcccgaagaa ttaaggcctg tctatgcttc gacgggatca gctatgcctt 1140
ccccaaaatc ctactcatca agtgggctgg acatgtccac attgagtcct ctctcaatca 1200
gttctccgtc agcatcgttg cctgttactt caacagcacc catgtctcct cttgcagcct 1260
cgtcatctcc gatgtctgtg aacatgtggc agagcaaggc taacaagctc tccccgccaa 1320
tgctgcagct ctcaggtagt aggctgaaga ctgctttgag tgctagggac ttggacctgg 1380
agatggaatt gcgtggtcta gagagtcaga tggccactca acagcatcag ttgatggaag 1440
agatatctcg tctctcctca ccatcatcct gctttagtag taggattggg gaagtgaaac 1500
ccactaacct cgatgacgtt tttgggtctc cggatcctgc tttgctgcct caattgcagg 1560
ggctgtcaag accttcaaca ccaagccagt tgcaatctcc aactgggctt cagatgcgcc 1620
agaatgcaac ccagtttcgt ggggcgtacc agagcaatgc aaatgcattg tcatctccag 1680
caatgaagca ggcaccttct tatgggtttg actcatctag tgcagttgca gcagcggtga 1740
tgaattcgag gtcagccgct tttgcgaagc ggagtcagag ttttatcgac aggggaatgg 1800
cgtgccctgg aattgccaat tcttccccta tgatgtcttc agctatgtcg agctggagct 1860
cacctcatgg gaaattggat tggggcgtcc aaggagatga gttgaatagg ctgaggaaag 1920
ctgcttcctt taagatgaga agcagcaccg gagcaggtgc taatactgtc tcggcagcag 1980
ccatggctga tgagccagat atttcttggg tcagttcatt ggttaaggac gtgccttctg 2040
cggaggacgc gatgttcgct gcagagaaag gacagcgcac ttatgggaaa gacatccgcg 2100
aaaggattac cccatgggtg gagcagctgt acagagaagt gccacggatg gcgatgtaag 2160
attgccactg caagtcggat gccttagtat gctgactaat tgatattctt tgcatttgtt 2220
ttgaggcatt tggtagccat tagatacgag aaaaggccaa gcagcaggtg gtgtcttggc 2280
aaggaatagg atgcacatag tctgttatcg agtagaatag acttgggaac aatggttata 2340
gccaaatgtt aaaagttatg atattctttt ccaattcttt ctcttcctca tagtaggttt 2400
ctcaccaagt cttttagtga gagcctgcgg gatgtactat atgtttccct tatgtaacgt 2460
ctcttcgttg aaagaaatgg ctttataata taaagcatca agttttttaa aaaaaaa 2518

<210> 29
<211> 663
<212> PRT
<213> Eucalyptus grandis

<400> 29

Met Glu Gly Glu Ser Tyr Phe Glu Lys Asp Glu Lys Tyr Ser Asn Cys
1 5 10 15

Ser Ile Leu Leu Glu Leu Ser Ala Ser Asp Asp Leu Pro Ala Phe Glu
20 25 30

Arg Lys Ala Lys Glu Lys Gly Cys Asn Ile Asp Gly Ala Ser Phe Trp
35 40 45

Tyr Gly Arg Arg Ile Gly Ser Arg Lys Met Gly Leu Glu Glu Arg Thr
50 55 60

Pro Leu Met Val Ala Ser Leu Phe Gly Ser Ser Arg Val Val Lys Tyr
65 70 75 80

Ile Leu Glu Ser Gly Lys Val Asp Val Asn Arg Ala Cys Gly Ser Asp
85 90 95

Lys Val Thr Ala Leu His Cys Ala Val Ala Ser Gly Ser Ala Ser Ala
100 105 110

Val Glu Val Val Lys Leu Leu Leu His Ala Ser Ala Asp Ala Asn Cys
115 120 125

Ile Asp Gly Asn Gly Lys Lys Pro Ile Asp Val Ile Ala Leu Pro Leu
130 135 140

Lys Ser Arg Gly Asp Ser Arg Arg Lys Leu Met Glu Leu Leu Leu Lys
145 150 155 160

Gly Asp Asn Ser Asp Gly Glu Phe Glu Ser His Glu Glu Lys Pro Ile
165 170 175

Ala Ala Pro Gln Ala Ser Lys Glu Gly Ser Glu Lys Lys Glu Tyr Gln
180 185 190

Phe Pro Val Asp Ile Ser Leu Pro Asp Ile Asn Val Gly Ile Tyr Ser
195 200 205

Thr Asp Glu Phe Arg Met Tyr Ala Phe Lys Val Lys Pro Cys Ser Arg
210 215 220

Ala Tyr Ser His Asp Trp Thr Glu Cys Pro Phe Val His Pro Gly Glu
225 230 235 240

Asn Ala Arg Arg Arg Asp Pro Arg Lys Tyr Pro Tyr Ser Cys Val Pro
245 250 255

```
Cys Pro Glu Phe Arg Lys Gly Ser Cys Gln Lys Gly Asp Ser Cys Glu
            260                 265                 270

Tyr Ala His Gly Val Phe Glu Ser Trp Leu His Pro Ala Gln Tyr Arg
        275                 280                 285

Thr Arg Leu Cys Lys Asp Glu Thr Gly Cys Ala Arg Lys Val Cys Phe
    290                 295                 300

Phe Ala His Lys Pro Glu Glu Leu Arg Pro Val Tyr Ala Ser Thr Gly
305                 310                 315                 320

Ser Ala Met Pro Ser Pro Lys Ser Tyr Ser Ser Ser Gly Leu Asp Met
                325                 330                 335

Ser Thr Leu Ser Pro Leu Ser Ile Ser Ser Pro Ser Ala Ser Leu Pro
            340                 345                 350

Val Thr Ser Thr Ala Pro Met Ser Pro Leu Ala Ala Ser Ser Ser Pro
        355                 360                 365

Met Ser Val Asn Met Trp Gln Ser Lys Ala Asn Lys Leu Ser Pro Pro
    370                 375                 380

Met Leu Gln Leu Ser Gly Ser Arg Leu Lys Thr Ala Leu Ser Ala Arg
385                 390                 395                 400

Asp Leu Asp Leu Glu Met Glu Leu Arg Gly Leu Glu Ser Gln Met Ala
                405                 410                 415

Thr Gln Gln His Gln Leu Met Glu Glu Ile Ser Arg Leu Ser Ser Pro
            420                 425                 430

Ser Ser Cys Phe Ser Ser Arg Ile Gly Glu Val Lys Pro Thr Asn Leu
        435                 440                 445

Asp Asp Val Phe Gly Ser Pro Asp Pro Ala Leu Leu Pro Gln Leu Gln
    450                 455                 460

Gly Leu Ser Arg Pro Ser Thr Pro Ser Gln Leu Gln Ser Pro Thr Gly
465                 470                 475                 480

Leu Gln Met Arg Gln Asn Ala Thr Gln Phe Arg Gly Ala Tyr Gln Ser
                485                 490                 495

Asn Ala Asn Ala Leu Ser Ser Pro Ala Met Lys Gln Ala Pro Ser Tyr
            500                 505                 510
```

Gly Phe Asp Ser Ser Ser Ala Val Ala Ala Ala Val Met Asn Ser Arg
515 520 525

Ser Ala Ala Phe Ala Lys Arg Ser Gln Ser Phe Ile Asp Arg Gly Met
530 535 540

Ala Cys Pro Gly Ile Ala Asn Ser Ser Pro Met Met Ser Ser Ala Met
545 550 555 560

Ser Ser Trp Ser Ser Pro His Gly Lys Leu Asp Trp Gly Val Gln Gly
565 570 575

Asp Glu Leu Asn Arg Leu Arg Lys Ala Ala Ser Phe Lys Met Arg Ser
580 585 590

Ser Thr Gly Ala Gly Ala Asn Thr Val Ser Ala Ala Ala Met Ala Asp
595 600 605

Glu Pro Asp Ile Ser Trp Val Ser Ser Leu Val Lys Asp Val Pro Ser
610 615 620

Ala Glu Asp Ala Met Phe Ala Ala Glu Lys Gly Gln Arg Thr Tyr Gly
625 630 635 640

Lys Asp Ile Arg Glu Arg Ile Thr Pro Trp Val Glu Gln Leu Tyr Arg
645 650 655

Glu Val Pro Arg Met Ala Met
660

<210> 30
<211> 2001
<212> DNA
<213> Triticum aestivum

<220>
<221> misc_feature
<222> (481)..(530)
<223> n is a, c, g, or t

<400> 30
cgaattccgg tcgacgattt ctcgatttcc ttctctataa cacaacgctc tcttctcttg 60

caaccaaagt acttgttcca gtgtctactc tactcaaaaa ggatttggga catcatgtgc 120

agtgattcga aaagtaaact ttcttcccca accctcgtcg tcatggagaa tagtaacatt 180

cagaagcaga atctggatgg tctctacaac tcggttttgc ttgaattgtc tgcatctgat 240

gattatgaag ctttcaaaag agaggtggag gaaaaaggct tagatgtgaa cgaggcaggc 300

ttttggtacg gtagaagaat tgggtcaaag aagatgggat ctgaaacgag gacccctctg 360

atgattgctt ctttgtttgg aagcgccaag gtgctcaatt atattcttct tcagaaagga 420

ggaggtgttg atgtgaacag ggtctgtggt tctgataggg ccactgctct ccattgtgct 480

nnnnnnnnnn nnnnnnnnnn nnnnnnnnnn nnnnnnnnnn nnnnnnnnnn tcccgggtcg 540

cgatttcgta ggagattcac agagagaaaa gaagcaatta gtgataataa gaaagaatac 600

cctgttgata tatcactgcc agacataaac aacggtgtat atggaacaga tgattttagg 660

atgtacaact tcaaggtgaa gccttgctca agggcttact cccatgactg gaccgagtgt 720

ccattcgttc acccagggga gaacgctagg aggagagacc cacggaaata cccttacagc 780

tgtgttcctt gccctgagtt ccgcaaaggg acctgccaga agggtgattc ctgtgagtat 840

gctcatggtg tttttgagtc ctggctgcat cctgcccaat accggacaag gctttgcaag 900

gatgagactg gctgcgctag aaaagtctgc ttctttgccc acaaacctga agagctacgc 960

cctgtgtatg cttccactgg gtcggctatg ccatcaccaa aatcatattc agctagtgga 1020

cttgacatga cagcgatgag tccattggct ctaagttcca catctttgcc taatgccccc 1080

ccgtttccag cctcacccta tcgtgcgccc tcgttcttct ctcagagtga agctgtgcag 1140

aacaaaataa accttactcc accatcgttg cagctccctg gtagccgact gaaggctgct 1200

ttgagtgcca gggatctgga gatggagatg gaactgctcg gtctagaaag ccctgctcgc 1260

caacaacagc agcagcagca acaattgatc gaagagattg ccaggatctc ttccccatct 1320

ttccggagca aggaattcaa taggattgtt gatttgaatc ctactaacct tgatgacctg 1380

ttagcatctg ctgacccttc tgtattttct caactacatg gactttctgt gcaaccttca 1440

acacccacac aaagtgggct tcagatgcgc caaaacatga accacctccg tgcgagttat 1500

ccatccaaca tcccttcctc tcctgtgagg aagccctcag cttttgggtt tgactcatca 1560

gctgctgtgg caactgcagt gatgaattct aggtctgctg ccttcgcaaa gcgaagccaa 1620

agtttcattg atcgtggagc tgcaacccac catcttgggc tgtcttcagc ttccaactct 1680

tcttgcaggg tatcctctac cctttcagat tggagttccc ctaccgggaa actggattgg 1740

ggtgtaaacg gagacaagct gaacaagctg aggaaatcta cttcctttgg attcagaaac 1800

agtggggtaa ctgcatcccc catagcacag cctgaatttg gtgctgagcc ggatgtctca 1860

tgggttcatt cattggttaa agatgttccc tccgagaggt ctgagatatt tggtgctgag 1920

aagcaacaat atgatctcag taaagagatg cttccaccat ggatggagca gctgtatata 1980

gagcaggagc agatggtagc a 2001

<210> 31
<211> 667
<212> PRT
<213> Triticum aestivum

<220>
<221> UNSURE
<222> (161)..(177)

<223> Xaa can be any naturally occurring amino acid

<400> 31

Arg Ile Pro Val Asp Asp Phe Ser Ile Ser Phe Ser Ile Thr Gln Arg
1               5                   10                  15

Ser Leu Leu Leu Gln Pro Lys Tyr Leu Phe Gln Cys Leu Leu Tyr Ser
            20                  25                  30

Lys Arg Ile Trp Asp Ile Met Cys Ser Asp Ser Lys Ser Lys Leu Ser
        35                  40                  45

Ser Pro Thr Leu Val Val Met Glu Asn Ser Asn Ile Gln Lys Gln Asn
    50                  55                  60

Leu Asp Gly Leu Tyr Asn Ser Val Leu Leu Glu Leu Ser Ala Ser Asp
65                  70                  75                  80

Asp Tyr Glu Ala Phe Lys Arg Glu Val Glu Glu Lys Gly Leu Asp Val
                85                  90                  95

Asn Glu Ala Gly Phe Trp Tyr Gly Arg Arg Ile Gly Ser Lys Lys Met
            100                 105                 110

Gly Ser Glu Thr Arg Thr Pro Leu Met Ile Ala Ser Leu Phe Gly Ser
        115                 120                 125

Ala Lys Val Leu Asn Tyr Ile Leu Leu Gln Lys Gly Gly Gly Val Asp
    130                 135                 140

Val Asn Arg Val Cys Gly Ser Asp Arg Ala Thr Ala Leu His Cys Ala
145                 150                 155                 160

Xaa Xaa Xaa Xaa Xaa Xaa Xaa Xaa Xaa Xaa Xaa Xaa Xaa Xaa Xaa Xaa
                165                 170                 175

Xaa Pro Gly Ser Arg Phe Arg Arg Arg Phe Thr Glu Arg Lys Glu Ala
            180                 185                 190

Ile Ser Asp Asn Lys Lys Glu Tyr Pro Val Asp Ile Ser Leu Pro Asp
        195                 200                 205

Ile Asn Asn Gly Val Tyr Gly Thr Asp Asp Phe Arg Met Tyr Asn Phe
    210                 215                 220

Lys Val Lys Pro Cys Ser Arg Ala Tyr Ser His Asp Trp Thr Glu Cys
225                 230                 235                 240

Pro Phe Val His Pro Gly Glu Asn Ala Arg Arg Arg Asp Pro Arg Lys

245 250 255

Tyr Pro Tyr Ser Cys Val Pro Cys Pro Glu Phe Arg Lys Gly Thr Cys
260 265 270

Gln Lys Gly Asp Ser Cys Glu Tyr Ala His Gly Val Phe Glu Ser Trp
275 280 285

Leu His Pro Ala Gln Tyr Arg Thr Arg Leu Cys Lys Asp Glu Thr Gly
290 295 300

Cys Ala Arg Lys Val Cys Phe Phe Ala His Lys Pro Glu Glu Leu Arg
305 310 315 320

Pro Val Tyr Ala Ser Thr Gly Ser Ala Met Pro Ser Pro Lys Ser Tyr
325 330 335

Ser Ala Ser Gly Leu Asp Met Thr Ala Met Ser Pro Leu Ala Leu Ser
340 345 350

Ser Thr Ser Leu Pro Asn Ala Pro Pro Phe Pro Ala Ser Pro Tyr Arg
355 360 365

Ala Pro Ser Phe Phe Ser Gln Ser Glu Ala Val Gln Asn Lys Ile Asn
370 375 380

Leu Thr Pro Pro Ser Leu Gln Leu Pro Gly Ser Arg Leu Lys Ala Ala
385 390 395 400

Leu Ser Ala Arg Asp Leu Glu Met Glu Met Glu Leu Leu Gly Leu Glu
405 410 415

Ser Pro Ala Arg Gln Gln Gln Gln Gln Gln Gln Gln Leu Ile Glu Glu
420 425 430

Ile Ala Arg Ile Ser Ser Pro Ser Phe Arg Ser Lys Glu Phe Asn Arg
435 440 445

Ile Val Asp Leu Asn Pro Thr Asn Leu Asp Asp Leu Leu Ala Ser Ala
450 455 460

Asp Pro Ser Val Phe Ser Gln Leu His Gly Leu Ser Val Gln Pro Ser
465 470 475 480

Thr Pro Thr Gln Ser Gly Leu Gln Met Arg Gln Asn Met Asn His Leu
485 490 495

Arg Ala Ser Tyr Pro Ser Asn Ile Pro Ser Ser Pro Val Arg Lys Pro
500 505 510

Ser Ala Phe Gly Phe Asp Ser Ser Ala Ala Val Ala Thr Ala Val Met
515 520 525

Asn Ser Arg Ser Ala Ala Phe Ala Lys Arg Ser Gln Ser Phe Ile Asp
530 535 540

Arg Gly Ala Ala Thr His His Leu Gly Leu Ser Ser Ala Ser Asn Ser
545 550 555 560

Ser Cys Arg Val Ser Ser Thr Leu Ser Asp Trp Ser Ser Pro Thr Gly
565 570 575

Lys Leu Asp Trp Gly Val Asn Gly Asp Lys Leu Asn Lys Leu Arg Lys
580 585 590

Ser Thr Ser Phe Gly Phe Arg Asn Ser Gly Val Thr Ala Ser Pro Ile
595 600 605

Ala Gln Pro Glu Phe Gly Ala Glu Pro Asp Val Ser Trp Val His Ser
610 615 620

Leu Val Lys Asp Val Pro Ser Glu Arg Ser Glu Ile Phe Gly Ala Glu
625 630 635 640

Lys Gln Gln Tyr Asp Leu Ser Lys Glu Met Leu Pro Pro Trp Met Glu
645 650 655

Gln Leu Tyr Ile Glu Gln Glu Gln Met Val Ala
660 665

<210> 32
<211> 2683
<212> DNA
<213> Eucalyptus grandis

<400> 32
gcaaaggtcg atcacttcct ccctagaaag cgagtgtgga gttgaagctt gataaccaga 60

ggccgcctct cgtctcgtct cgcccgcctg cgcttgctct gctctccgcg tgccaaggga 120

gtgttcctag gtgctgaatc tttccatgtg tagcggttca aaagggaagg gagagtgaat 180

tcgagaagca gaggatgtcg gcccgtcagt tctcgatcct gctcgagtta tctgctgcgg 240

atgatctgac gaactttaag aaagcagttg aggaagacgg ctacgatatt gatgagtcga 300

gcttgtggta tggtaggagg atcgggtcga agaagattgg gcttgaagag agaactcccc 360

tcatgattgc cgcgatgttc ggcagtatgt ccgtgctgga ttatattatc aagtctggcc 420

gggccaatgt aaacaaggcg tgtggttcag atggtgctac cgcgcttcac tgtgctgcgg 480

ctggtggctc ggtacaatct cctgaggtgg tcaagctgtt gcttgattct tcagcgaatg 540

ctaactccat tgatgcgaat gggaaacgag cgggagactt gatttctgag gtctctggtt 600
cgcccttcaa ttcgagaagg aagactttgg atgtcatgtt gactggaggt gggactgttg 660
agtttgttga ggaaacttac aatctgcctg agaatctggg tagtcaaatt gaaggaaacg 720
aacaaagaga gagtccaacg gcccgcgctt ccaaggatgg ttctgaaaag aaagagtatc 780
ctgtcgacct ttctcttccg gacatcaaca atggaatata tagcacagat gagtttagga 840
tgtattcttt caaagtgaag ccttgctcga gagcttactc tcatgactgg actgagtgtc 900
catttgttca ccctggggag aatgcaagac ggcgtgaccc acggaaatat cactacagct 960
gtgtgccttg ccctgagttc cgcaaggggt catgcaggca aggggatggc tgcgagtatg 1020
ctcatggtat atttgagtgc tggcttcacc cagctcaata tcgcacccgt ctctgtaagg 1080
atgagattgg atgcaccaga aaagtctgtt tctttgccca caaacatgaa gagcttcgtc 1140
cattgtatgc atcaactggt tcggcgcttc cttctccaag atcattttcg cccgttgctg 1200
cttctctaga catgggatca ctgagccctc tctctctcgg ttcttcttca gtccggatac 1260
cgccaacttc aacaccacct atgactccat caggggcctc ttctcccctt ggtgggtcga 1320
tgtggaaaag ccaaattaat agcactccgc ctggcttgca gcttccaggt agcaggttga 1380
gaagcgcatt gagtgctaga gacatggatt tagatgttga cttgatcgat ctagaaaata 1440
attatcgttt gcagaagcag ttgctcgaac actttcctga tctgtcctct cctcgtggtt 1500
ggaacaactc ttcatccacc acgtcggctt tccctgagta ttcaggtgac atgactggag 1560
aaataagtag gttaggagta aaaccaaata atctcgagga tagtttcagg tcattggacc 1620
tgaccctctt gtctcagtta caagggctgt cacttgatgg tgcaatatcc cagctgcaat 1680
ctcctactgg aatgaagatt cggcagaaca tgacccagca gctctactca aactatactg 1740
acaagctttc ctcgtcacct agggcaatgc catcatttgg aaccgatcct tccagagctt 1800
cagcagcagc cactctgagt tccaggtcat tggcatttgc aaaaaggagc cacagcttca 1860
ttgagcggag tacagtgaac agtcagtctg gatattcagc aggtgctgct tctccaactg 1920
caaggatgtc ttcccagaat gactggggct cgcccgatgg caaactagac tggggcattc 1980
aaggggagga gctgaacaag ctgaggaaat ctgcatcatt cgggctcagg agcagcagca 2040
accgcttcca tgcgtctgca gattctgcga cagcaactgt aggggaccca gacatgccct 2100
ggattcagtc cttggcaaag gaagccccgt cacaaaaccc tggcaatttt ggagcagagc 2160
atcagcagca gcagcagcag cagcagcagc agcagtatca tcttaattct ggaggtactg 2220
agctgcttcc agcttgggtg gagcagttgt acgcggatca ggagcagatg gtcgcctgag 2280
atcaacattg gcttcttatc taaccactat tagtcatttc gttattgctt taattttttt 2340
tcttctgagt ctagtattaa tgtctaggat tcgaacgaac tggaaaatta aatctagagg 2400
gaagatggga agaaaagagc aggatggaag gtttctgctc ggtccgagat ttctcatagt 2460

ctattataga ctatcgtatt tctcgttctt ttccgtccca atgttcttga tttggttctc 2520

agcatgtttt ctggatgagg cttacaaact atgtaatctt gtcttgctaa aagaatcaga 2580

gctgcacctg caccaaaggt tgtgatacta ccgcttattg atgatgatga taataataat 2640

aattcggaca tttagtacca agtccgatgt ctcaaaaaaa aaa 2683

<210> 33
<211> 694
<212> PRT
<213> Eucalyptus grandis

<400> 33

Met Ser Ala Arg Gln Phe Ser Ile Leu Leu Glu Leu Ser Ala Ala Asp
1 5 10 15

Asp Leu Thr Asn Phe Lys Lys Ala Val Glu Glu Asp Gly Tyr Asp Ile
20 25 30

Asp Glu Ser Ser Leu Trp Tyr Gly Arg Arg Ile Gly Ser Lys Lys Ile
35 40 45

Gly Leu Glu Glu Arg Thr Pro Leu Met Ile Ala Ala Met Phe Gly Ser
50 55 60

Met Ser Val Leu Asp Tyr Ile Ile Lys Ser Gly Arg Ala Asn Val Asn
65 70 75 80

Lys Ala Cys Gly Ser Asp Gly Ala Thr Ala Leu His Cys Ala Ala Ala
85 90 95

Gly Gly Ser Val Gln Ser Pro Glu Val Val Lys Leu Leu Leu Asp Ser
100 105 110

Ser Ala Asn Ala Asn Ser Ile Asp Ala Asn Gly Lys Arg Ala Gly Asp
115 120 125

Leu Ile Ser Glu Val Ser Gly Ser Pro Phe Asn Ser Arg Arg Lys Thr
130 135 140

Leu Asp Val Met Leu Thr Gly Gly Gly Thr Val Glu Phe Val Glu Glu
145 150 155 160

Thr Tyr Asn Leu Pro Glu Asn Leu Gly Ser Gln Ile Glu Gly Asn Glu
165 170 175

Gln Arg Glu Ser Pro Thr Ala Arg Ala Ser Lys Asp Gly Ser Glu Lys
180 185 190

Lys Glu Tyr Pro Val Asp Leu Ser Leu Pro Asp Ile Asn Asn Gly Ile

195 200 205

Tyr Ser Thr Asp Glu Phe Arg Met Tyr Ser Phe Lys Val Lys Pro Cys
210 215 220

Ser Arg Ala Tyr Ser His Asp Trp Thr Glu Cys Pro Phe Val His Pro
225 230 235 240

Gly Glu Asn Ala Arg Arg Arg Asp Pro Arg Lys Tyr His Tyr Ser Cys
245 250 255

Val Pro Cys Pro Glu Phe Arg Lys Gly Ser Cys Arg Gln Gly Asp Gly
260 265 270

Cys Glu Tyr Ala His Gly Ile Phe Glu Cys Trp Leu His Pro Ala Gln
275 280 285

Tyr Arg Thr Arg Leu Cys Lys Asp Glu Ile Gly Cys Thr Arg Lys Val
290 295 300

Cys Phe Phe Ala His Lys His Glu Glu Leu Arg Pro Leu Tyr Ala Ser
305 310 315 320

Thr Gly Ser Ala Leu Pro Ser Pro Arg Ser Phe Ser Pro Val Ala Ala
325 330 335

Ser Leu Asp Met Gly Ser Leu Ser Pro Leu Ser Leu Gly Ser Ser Ser
340 345 350

Val Arg Ile Pro Pro Thr Ser Thr Pro Pro Met Thr Pro Ser Gly Ala
355 360 365

Ser Ser Pro Leu Gly Gly Ser Met Trp Lys Ser Gln Ile Asn Ser Thr
370 375 380

Pro Pro Gly Leu Gln Leu Pro Gly Ser Arg Leu Arg Ser Ala Leu Ser
385 390 395 400

Ala Arg Asp Met Asp Leu Asp Val Asp Leu Ile Asp Leu Glu Asn Asn
405 410 415

Tyr Arg Leu Gln Lys Gln Leu Leu Glu His Phe Pro Asp Leu Ser Ser
420 425 430

Pro Arg Gly Trp Asn Asn Ser Ser Ser Thr Thr Ser Ala Phe Pro Glu
435 440 445

Tyr Ser Gly Asp Met Thr Gly Glu Ile Ser Arg Leu Gly Val Lys Pro
450 455 460

Asn Asn Leu Glu Asp Ser Phe Arg Ser Leu Asp Leu Thr Leu Leu Ser
465 470 475 480

Gln Leu Gln Gly Leu Ser Leu Asp Gly Ala Ile Ser Gln Leu Gln Ser
485 490 495

Pro Thr Gly Met Lys Ile Arg Gln Asn Met Thr Gln Gln Leu Tyr Ser
500 505 510

Asn Tyr Thr Asp Lys Leu Ser Ser Ser Pro Arg Ala Met Pro Ser Phe
515 520 525

Gly Thr Asp Pro Ser Arg Ala Ser Ala Ala Ala Thr Leu Ser Ser Arg
530 535 540

Ser Leu Ala Phe Ala Lys Arg Ser His Ser Phe Ile Glu Arg Ser Thr
545 550 555 560

Val Asn Ser Gln Ser Gly Tyr Ser Ala Gly Ala Ala Ser Pro Thr Ala
565 570 575

Arg Met Ser Ser Gln Asn Asp Trp Gly Ser Pro Asp Gly Lys Leu Asp
580 585 590

Trp Gly Ile Gln Gly Glu Glu Leu Asn Lys Leu Arg Lys Ser Ala Ser
595 600 605

Phe Gly Leu Arg Ser Ser Ser Asn Arg Phe His Ala Ser Ala Asp Ser
610 615 620

Ala Thr Ala Thr Val Gly Asp Pro Asp Met Pro Trp Ile Gln Ser Leu
625 630 635 640

Ala Lys Glu Ala Pro Ser Gln Asn Pro Gly Asn Phe Gly Ala Glu His
645 650 655

Gln Gln Gln Gln Gln Gln Gln Gln Gln Gln Gln Tyr His Leu Asn Ser
660 665 670

Gly Gly Thr Glu Leu Leu Pro Ala Trp Val Glu Gln Leu Tyr Ala Asp
675 680 685

Gln Glu Gln Met Val Ala
690

<210> 34
<211> 2499
<212> DNA

<213> Arabidopsis thaliana

<400> 34
atttgacct taagaagaaa gtgacaagga gaggaagaag aagaaaaaaa acataatttg 60
aggaagaaga aaaaaaattc ggatttgttt tttcaataaa ttgactaatt gagtactcgt 120
ttaaaggaag tgaagagcgg ttttttggta gtggtggtcg agaaaagaga gagtttgtct 180
ctgtgactca gagtgaaatc aatagagcgg gaaaagattg ttgctttttt ttgccatggg 240
agttgatgag ctgtctcacc tcaaattctc tcttctgcta gaatcatcag cctgcaatga 300
tttgtccggt tttaagtctc tagttgaaga agaaggtctt gagagcattg atggctctgg 360
tttgtggtat gggaggagat taggatcaaa gaagatgggt tttgaggaga ggacgcctct 420
tatgattgct gccttgtttg gaagcaaaga ggttgttgat tacatcatta gtactggtct 480
tgttgacgtg aaccgctctt gtggctctga tggtgccacg gctcttcact gtgcggtctc 540
tggcttgtct gccaatagcc ttgagattgt tactcttctg ctgaagggct ctgcgaatcc 600
ggattcttgt gatgcttatg gtaacaagcc tggagatgtg attttccctt gtttgagtcc 660
ggtttttagc gcgaggatga aggttttgga gcgtttgttg aaaggaaatg atgatttgaa 720
tgaagttaat gggcaagaag aaagcgagcc agaggttgag gttgaggttg aggtttcgcc 780
tcctcggggg tctgagagga aggagtatcc ggttgatcca acgcttcctg atatcaagaa 840
cggtgtatat gggacggatg agttccggat gtatgctttc aagatcaagc cgtgctctag 900
agcatactct cacgactgga cggaatgtcc ctttgttcat ccgggtgaga acgcaaggag 960
gcgtgatccg aggaagtacc attatagttg tgtcccttgt cctgaattcc ggaaggggtc 1020
ttgttccaga ggtgatactt gcgagtatgc tcatggtatc tttgagtgct ggcttcaccc 1080
ggctcagtac cggactcgtc tctgcaagga cgagacgaat tgctcgagaa gagtttgttt 1140
ctttgcccac aaacccgagg agctgcgtcc tttgtaccct tcaactggat caggtgttcc 1200
gtccccgcgg tcttccttct catcttgcaa ttcctcgacc gctttcgaca tgggaccgat 1260
tagtccgctt cctatcggag caacaaccac acctcctttg agtcctaacg gtgtatcctc 1320
tccaataggt ggaggaaaaa cgtggatgaa ctggcctaac ataacccctc ctgcattgca 1380
gcttccaggg agcagattga aatctgcatt gaatgcaaga gaaatcgatt tctctgaaga 1440
gatgcaaagt cttacttctc caactacatg gaacaacacg ccaatgtcat ctccattctc 1500
cggaaagggc atgaacaggc ttgcaggagg agcaatgagc ccggtgaata gtctcagtga 1560
tatgtttggg acagaggata atacatcggg tttgcagatc cgacgcagcg tcattaaccc 1620
gcagctgcat tccaacagtc tttcttcatc acctgtggga gccaattctc tgttttcgat 1680
ggattcctcc gcagtcttgg cttcaagagc ggctgaattt gctaaacagc gaagccaaag 1740
cttcatagaa cgcaacaacg gactgaatca ccatcccgca atctcttcca tgactacaac 1800
ttgtttaaac gattggggct cattggatgg gaagcttgac tggagcgtcc aaggagacga 1860

gctacagaag ctcagaaaat ccacttcttt ccgtctcaga gccggtggca tggaatcaag 1920

actgcctaac gaagggactg ggctcgaaga gccagatgtc tcatgggtgg agccgctggt 1980

gaaagagcca caggagacaa gactagctcc ggtttggatg gagcaatcat acatggagac 2040

agaacagacc gtggcttgaa tcaaaagttt tgaactttca ttaaccgttc cacaagaagc 2100

aaagtcagaa agattccgag aggtcgatgc taatctattt cattttattt gtttaatgct 2160

ttgttatttt tctttagaat aaaaagaaaa aattcttagg ggacaaaaga gagttcgttt 2220

gtctctctct ctgtctccaa agaaaaacag aggtgaaaaa aggtttcaaa acctaagaaa 2280

ccttgaatta cctcacctca cttccttgat tctttactat tcacaatgag taatcgattt 2340

tttttttct tggtaacact ctcacgctga atatatatgt tttttagtaa taatataatt 2400

ggaatacaga aatgtattta cacttgtgaa gttagggaaa gtgttgtaat tgtttcttct 2460

aagagttgat ctaagatgtt tgagactata tcttcgctt 2499

<210> 35
<211> 607
<212> PRT
<213> Arabidopsis thaliana

<400> 35

Met Gly Val Asp Glu Leu Ser His Leu Lys Phe Ser Leu Leu Leu Glu
1               5                   10                  15

Ser Ser Ala Cys Asn Asp Leu Ser Gly Phe Lys Ser Leu Val Glu Glu
            20                  25                  30

Glu Gly Leu Glu Ser Ile Asp Gly Ser Gly Leu Trp Tyr Gly Arg Arg
        35                  40                  45

Leu Gly Ser Lys Lys Met Gly Phe Glu Glu Arg Thr Pro Leu Met Ile
    50                  55                  60

Ala Ala Leu Phe Gly Ser Lys Glu Val Val Asp Tyr Ile Ile Ser Thr
65                  70                  75                  80

Gly Leu Val Asp Val Asn Arg Ser Cys Gly Ser Asp Gly Ala Thr Ala
                85                  90                  95

Leu His Cys Ala Val Ser Gly Leu Ser Ala Asn Ser Leu Glu Ile Val
            100                 105                 110

Thr Leu Leu Leu Lys Gly Ser Ala Asn Pro Asp Ser Cys Asp Ala Tyr
        115                 120                 125

Gly Asn Lys Pro Gly Asp Val Ile Phe Pro Cys Leu Ser Pro Val Phe
    130                 135                 140

```
Ser Ala Arg Met Lys Val Leu Glu Arg Leu Leu Lys Gly Asn Asp Asp
145                 150                 155                 160

Leu Asn Glu Val Asn Gly Gln Glu Glu Ser Glu Pro Glu Val Glu Val
                165                 170                 175

Glu Val Glu Val Ser Pro Pro Arg Gly Ser Glu Arg Lys Glu Tyr Pro
            180                 185                 190

Val Asp Pro Thr Leu Pro Asp Ile Lys Asn Gly Val Tyr Gly Thr Asp
        195                 200                 205

Glu Phe Arg Met Tyr Ala Phe Lys Ile Lys Pro Cys Ser Arg Ala Tyr
    210                 215                 220

Ser His Asp Trp Thr Glu Cys Pro Phe Val His Pro Gly Glu Asn Ala
225                 230                 235                 240

Arg Arg Arg Asp Pro Arg Lys Tyr His Tyr Ser Cys Val Pro Cys Pro
                245                 250                 255

Glu Phe Arg Lys Gly Ser Cys Ser Arg Gly Asp Thr Cys Glu Tyr Ala
            260                 265                 270

His Gly Ile Phe Glu Cys Trp Leu His Pro Ala Gln Tyr Arg Thr Arg
        275                 280                 285

Leu Cys Lys Asp Glu Thr Asn Cys Ser Arg Arg Val Cys Phe Phe Ala
    290                 295                 300

His Lys Pro Glu Glu Leu Arg Pro Leu Tyr Pro Ser Thr Gly Ser Gly
305                 310                 315                 320

Val Pro Ser Pro Arg Ser Ser Phe Ser Ser Cys Asn Ser Ser Thr Ala
                325                 330                 335

Phe Asp Met Gly Pro Ile Ser Pro Leu Pro Ile Gly Ala Thr Thr Thr
            340                 345                 350

Pro Pro Leu Ser Pro Asn Gly Val Ser Ser Pro Ile Gly Gly Gly Lys
        355                 360                 365

Thr Trp Met Asn Trp Pro Asn Ile Thr Pro Pro Ala Leu Gln Leu Pro
    370                 375                 380

Gly Ser Arg Leu Lys Ser Ala Leu Asn Ala Arg Glu Ile Asp Phe Ser
385                 390                 395                 400
```

Glu Glu Met Gln Ser Leu Thr Ser Pro Thr Thr Trp Asn Asn Thr Pro
405 410 415

Met Ser Ser Pro Phe Ser Gly Lys Gly Met Asn Arg Leu Ala Gly Gly
420 425 430

Ala Met Ser Pro Val Asn Ser Leu Ser Asp Met Phe Gly Thr Glu Asp
435 440 445

Asn Thr Ser Gly Leu Gln Ile Arg Arg Ser Val Ile Asn Pro Gln Leu
450 455 460

His Ser Asn Ser Leu Ser Ser Ser Pro Val Gly Ala Asn Ser Leu Phe
465 470 475 480

Ser Met Asp Ser Ser Ala Val Leu Ala Ser Arg Ala Ala Glu Phe Ala
485 490 495

Lys Gln Arg Ser Gln Ser Phe Ile Glu Arg Asn Asn Gly Leu Asn His
500 505 510

His Pro Ala Ile Ser Ser Met Thr Thr Thr Cys Leu Asn Asp Trp Gly
515 520 525

Ser Leu Asp Gly Lys Leu Asp Trp Ser Val Gln Gly Asp Glu Leu Gln
530 535 540

Lys Leu Arg Lys Ser Thr Ser Phe Arg Leu Arg Ala Gly Gly Met Glu
545 550 555 560

Ser Arg Leu Pro Asn Glu Gly Thr Gly Leu Glu Glu Pro Asp Val Ser
565 570 575

Trp Val Glu Pro Leu Val Lys Glu Pro Gln Glu Thr Arg Leu Ala Pro
580 585 590

Val Trp Met Glu Gln Ser Tyr Met Glu Thr Glu Gln Thr Val Ala
595 600 605

<210> 36
<211> 1806
<212> DNA
<213> Oryza sativa

<400> 36
atgtgctctg ggccgcgcaa gccgtccaca ccgccgctgc cgcagcagca gaaggaggcg 60

acggtgatgg cggcgtcctt gcttcttgag ctggcggcag cggacgacgt ggcggcggtg 120

aggagggtcg tggaggagga gaaggtgtct cttggcgtgg ctgggttgtg gtatgggcct 180

tcggcgagcg gcgtggcgag gctcgggatg gagcggagga cggcggcgat ggtggcggcg 240

```
ctgtacggga gcacgggggt gcttgggtat gtcgtggcgg cagcgccggc ggaggccgcg     300
cgcgcgtcgg agacggatgg ggccacgccg ctgcacatgg cggctgccgg tggcgcggcg     360
aacgcggtcg cggccacgcg cctgttgctc gccgcggggg cgtcggtcga cgcgctctcg     420
gcttcggggc tccgcgccgg tgacctcctc ccgcgcgcca ccgcggcgga gaaggccatc     480
cggctgctgc tcaagtcgcc ggccgtgtcg ccgtcgtcgt cgccgaagaa gtcggcctcg     540
ccgccgtcgc cgccgccgcc gcaggaggcg aagaaggagt acccgcctga cctgacgctg     600
cccgacctca agagcggact gttcagcacc gacgagttcc gcatgtacag cttcaaggtg     660
aagccgtgct cccgcgccta ctcccatgac tggaccgagt gccccttcgt ccaccccggc     720
gagaacgcgc gccgccgcga ccctcgccgc tactcctaca gctgcgtgcc ttgcccggag     780
ttccgcaagg gcggctcgtg ccgcaagggc gacgcgtgcg agtacgccca tggcgtgttc     840
gagtgctggc tccacccggc gcagtacagg acgcgcctct gcaaggacga ggtcggctgc     900
gcgcgccgca tctgcttctt cgcccacaag cccgacgagc tccgcgccgt caacccctcc     960
gccgtgtccg tcggcatgca gcccaccgta tcgtcgccgc gctcctcgcc gcccaacggg    1020
ctcgacatgg cggcggcggc ggcggcgatg atgagccccg cctggccgtc gtccccagcg    1080
agccgcctca agacggcgct cggcgcgcgg gagctcgact tcgacctcga gatgctcgcg    1140
ctggaccagt accagcagaa gctgttcgac aaggtgtccg gcgcgccgtc gccgagggcg    1200
agctggggcg ccgcggcgaa cggcctcgcc accgcgtcgc cggcgagggc cgtgccggac    1260
tacaccgacc tgctcggctc cgtcgacccg gccatgctgt cccagctcca cgcgctgtcc    1320
ctcaagcagg ccggcgacat gcccgcgtac agctccatgg cggacaccac gcagatgcac    1380
atgccgacct cgccgatggt gggcggcgcg aacaccgcgt tcgggctgga ccactccatg    1440
gcgaaggcga tcatgagctc ccgcgcctcg gcgttcgcca agcgcagcca gagcttcatc    1500
gaccgcggag gccgcgcccc ggcggcgcgt tcgctcatgt cgccggcgac gaccggcgcg    1560
ccgtccattc tctcggactg ggctcgccg gacggcaagc tggactgggg cgtccagggc    1620
gacgagctgc acaagctccg caagtcggcg tcgttcgcgt tccgcggcca atccgccatg    1680
ccggtggcga cgcacgccgc ggcggcggag ccggacgtgt catgggtgaa ctctcttgtc    1740
aaggacggcc acgccgccgg cgacatattc gcgcagtggc cggagcagga gcagatggtg    1800
gcatga                                                               1806
```

<210> 37
<211> 601
<212> PRT
<213> Oryza sativa

<400> 37

```
Met Cys Ser Gly Pro Arg Lys Pro Ser Thr Pro Pro Leu Pro Gln Gln
1               5                   10                  15
```

```
Gln Lys Glu Ala Thr Val Met Ala Ala Ser Leu Leu Leu Glu Leu Ala
            20                  25                  30

Ala Ala Asp Asp Val Ala Ala Val Arg Arg Val Val Glu Glu Glu Lys
        35                  40                  45

Val Ser Leu Gly Val Ala Gly Leu Trp Tyr Gly Pro Ser Ala Ser Gly
    50                  55                  60

Val Ala Arg Leu Gly Met Glu Arg Arg Thr Ala Ala Met Val Ala Ala
65                  70                  75                  80

Leu Tyr Gly Ser Thr Gly Val Leu Gly Tyr Val Val Ala Ala Ala Pro
                85                  90                  95

Ala Glu Ala Ala Arg Ala Ser Glu Thr Asp Gly Ala Thr Pro Leu His
            100                 105                 110

Met Ala Ala Ala Gly Gly Ala Ala Asn Ala Val Ala Ala Thr Arg Leu
        115                 120                 125

Leu Leu Ala Ala Gly Ala Ser Val Asp Ala Leu Ser Ala Ser Gly Leu
    130                 135                 140

Arg Ala Gly Asp Leu Leu Pro Arg Ala Thr Ala Ala Glu Lys Ala Ile
145                 150                 155                 160

Arg Leu Leu Leu Lys Ser Pro Ala Val Ser Pro Ser Ser Ser Pro Lys
                165                 170                 175

Lys Ser Ala Ser Pro Pro Ser Pro Pro Pro Pro Gln Glu Ala Lys Lys
            180                 185                 190

Glu Tyr Pro Pro Asp Leu Thr Leu Pro Asp Leu Lys Ser Gly Leu Phe
        195                 200                 205

Ser Thr Asp Glu Phe Arg Met Tyr Ser Phe Lys Val Lys Pro Cys Ser
    210                 215                 220

Arg Ala Tyr Ser His Asp Trp Thr Glu Cys Pro Phe Val His Pro Gly
225                 230                 235                 240

Glu Asn Ala Arg Arg Arg Asp Pro Arg Arg Tyr Ser Tyr Ser Cys Val
                245                 250                 255

Pro Cys Pro Glu Phe Arg Lys Gly Gly Ser Cys Arg Lys Gly Asp Ala
            260                 265                 270
```

Cys Glu Tyr Ala His Gly Val Phe Glu Cys Trp Leu His Pro Ala Gln
275 280 285

Tyr Arg Thr Arg Leu Cys Lys Asp Glu Val Gly Cys Ala Arg Arg Ile
290 295 300

Cys Phe Phe Ala His Lys Pro Asp Glu Leu Arg Ala Val Asn Pro Ser
305 310 315 320

Ala Val Ser Val Gly Met Gln Pro Thr Val Ser Ser Pro Arg Ser Ser
325 330 335

Pro Pro Asn Gly Leu Asp Met Ala Ala Ala Ala Ala Ala Met Met Ser
340 345 350

Pro Ala Trp Pro Ser Ser Pro Ala Ser Arg Leu Lys Thr Ala Leu Gly
355 360 365

Ala Arg Glu Leu Asp Phe Asp Leu Glu Met Leu Ala Leu Asp Gln Tyr
370 375 380

Gln Gln Lys Leu Phe Asp Lys Val Ser Gly Ala Pro Ser Pro Arg Ala
385 390 395 400

Ser Trp Gly Ala Ala Ala Asn Gly Leu Ala Thr Ala Ser Pro Ala Arg
405 410 415

Ala Val Pro Asp Tyr Thr Asp Leu Leu Gly Ser Val Asp Pro Ala Met
420 425 430

Leu Ser Gln Leu His Ala Leu Ser Leu Lys Gln Ala Gly Asp Met Pro
435 440 445

Ala Tyr Ser Ser Met Ala Asp Thr Thr Gln Met His Met Pro Thr Ser
450 455 460

Pro Met Val Gly Gly Ala Asn Thr Ala Phe Gly Leu Asp His Ser Met
465 470 475 480

Ala Lys Ala Ile Met Ser Ser Arg Ala Ser Ala Phe Ala Lys Arg Ser
485 490 495

Gln Ser Phe Ile Asp Arg Gly Gly Arg Ala Pro Ala Ala Arg Ser Leu
500 505 510

Met Ser Pro Ala Thr Thr Gly Ala Pro Ser Ile Leu Ser Asp Trp Gly
515 520 525

Ser Pro Asp Gly Lys Leu Asp Trp Gly Val Gln Gly Asp Glu Leu His
530 535 540

Lys Leu Arg Lys Ser Ala Ser Phe Ala Phe Arg Gly Gln Ser Ala Met
545 550 555 560

Pro Val Ala Thr His Ala Ala Ala Ala Glu Pro Asp Val Ser Trp Val
565 570 575

Asn Ser Leu Val Lys Asp Gly His Ala Ala Gly Asp Ile Phe Ala Gln
580 585 590

Trp Pro Glu Gln Glu Gln Met Val Ala
595 600

<210> 38
<211> 1692
<212> DNA
<213> Hordeum vulgare

<400> 38
cggcacgagg cacatccatc atctaacctc acctctcctc tcctcccctc tcctcctacc 60
aaacccaaaa ccaagcagag caagagcaag agcaagagca agagcaagca agcatgtgcc 120
ctggcctgcg caacctcgcc gccgccatgc caccctccgc ccacgaccac ccctcctcct 180
acctgctcga gctcgccgcc gacgacgacc tccccgcctt ccgccgcgcc gtccaggagg 240
acaacctctc cctcgacgcc gcatccccga ggtacgagcc atcccccaaa tcagaccaac 300
aacaacaaca cgccccagct cgcgctccac ctgcgcaccc ccgccatggt cgccgcgctc 360
tacggcagca ccaccgtcct ctcctacgtc ctctccatcg cccccctccga ggccgcccgc 420
gcctccgcat ccgacggcgc caccccgctc ctcctcgccc accagggccg cgcgccatcc 480
gcgccccacg ccgcacgcct cctcctcacc gacggcgcat catcgtcctc cctactcgcg 540
ccccaagctc accctctcaa ccaccaaaac caaaaccaaa acagccccac caagaaagac 600
tcgccgccgg actccaggag gaccaccacc aagaaggact actcctccgc ctccgactcc 660
cagacggagg acatcaacgc gggcgtcttc gccaccgacg acttccggat gtacagcttc 720
aaggtgaacc cgtgctcccg cgcctacacg cacgactgga ccgagtgccc cttcgcccac 780
cccggcgaga acgcgcgccg ccgcgacccg cgccgcgtgc catactcgtg cgtcccatgc 840
ccggacttcc gccgcgaccc ggccgcatgc cgcaagggcg acgcctgcga gtacgcgcac 900
ggcgtcttcg agtcatggct ccaccccgcg cagtaccgca ccaggctctg caaggacgag 960
gtcggatgcc cgcgccgcat ctgcttcttc gcgcacggcg cccgacagct acgcgccgtc 1020
aacccctccg ccgcatccat ggactcgcca tccccaactt cctcttcgcc gccgcgaacc 1080
tccaggccgg ccgcgctcac cgcgtcgctc agctcgcggg acctcgactt ggacgccgac 1140
aaccaggccc agtacgcgcg caggatgatg atggccaggg ccaactcccc gccggactac 1200

tcgcccgacc tcgtcgccgc ctacgtacag gcgctctcct ccctgcaaca gcagcagcat 1260

cagcagaacc agcaacagca gcatcagcag cagaaccagc accagcagca acatcagcag 1320

aaccagcacc agcagcatca gcagcaacat cagcagagca tggggatggg ggggctgagc 1380

gcccgcgccg ccgccttcac caaccgcagc cagaccttcg tgcaccgctc tccgtccccg 1440

gctccggcgc ggtcgttcaa gtctccggcg ccgtcgtcca tgctcgcgga ctgggggtcg 1500

ccggacggga agctggactg ggcgtgcag gccgcggagc tgcgcaagtc cacgtctttc 1560

ggagtcagaa gcagcagcag gccgcatcat gagacgacga gggcggagga caacatgtac 1620

ccgtcgtgga tgaaggacgg cagcgatatg ctgctggcgg cgcggtggtc ggacctggag 1680

cagatggtcg cc 1692

<210> 39
<211> 564
<212> PRT
<213> Hordeum vulgare

<400> 39

Arg His Glu Ala His Pro Ser Ser Asn Leu Thr Ser Pro Leu Leu Pro
1 5 10 15

Ser Pro Pro Thr Lys Pro Lys Thr Lys Gln Ser Lys Ser Lys Ser Lys
20 25 30

Ser Lys Ser Lys Gln Ala Cys Ala Leu Ala Cys Ala Thr Ser Pro Pro
35 40 45

Pro Cys His Pro Pro Pro Thr Thr Thr Pro Pro Pro Thr Cys Ser Ser
50 55 60

Ser Pro Pro Thr Thr Thr Ser Pro Pro Ser Ala Ala Pro Ser Arg Arg
65 70 75 80

Thr Thr Ser Pro Ser Thr Pro His Pro Arg Gly Thr Ser His Pro Pro
85 90 95

Asn Gln Thr Asn Asn Asn Asn Thr Pro Gln Leu Ala Leu His Leu Arg
100 105 110

Thr Pro Ala Met Val Ala Ala Leu Tyr Gly Ser Thr Thr Val Leu Ser
115 120 125

Tyr Val Leu Ser Ile Ala Pro Ser Glu Ala Ala Arg Ala Ser Ala Ser
130 135 140

Asp Gly Ala Thr Pro Leu Leu Leu Ala His Gln Gly Arg Ala Pro Ser
145 150 155 160

```
Ala Pro His Ala Ala Arg Leu Leu Leu Thr Asp Gly Ala Ser Ser Ser
                165                 170                 175

Ser Leu Leu Ala Pro Gln Ala His Pro Leu Asn His Gln Asn Gln Asn
            180                 185                 190

Gln Asn Ser Pro Thr Lys Lys Asp Ser Pro Pro Asp Ser Arg Arg Thr
        195                 200                 205

Thr Thr Lys Lys Asp Tyr Ser Ser Ala Ser Asp Ser Gln Thr Glu Asp
    210                 215                 220

Ile Asn Ala Gly Val Phe Ala Thr Asp Asp Phe Arg Met Tyr Ser Phe
225                 230                 235                 240

Lys Val Asn Pro Cys Ser Arg Ala Tyr Thr His Asp Trp Thr Glu Cys
                245                 250                 255

Pro Phe Ala His Pro Gly Glu Asn Ala Arg Arg Arg Asp Pro Arg Arg
            260                 265                 270

Val Pro Tyr Ser Cys Val Pro Cys Pro Asp Phe Arg Arg Asp Pro Ala
        275                 280                 285

Ala Cys Arg Lys Gly Asp Ala Cys Glu Tyr Ala His Gly Val Phe Glu
    290                 295                 300

Ser Trp Leu His Pro Ala Gln Tyr Arg Thr Arg Leu Cys Lys Asp Glu
305                 310                 315                 320

Val Gly Cys Pro Arg Arg Ile Cys Phe Phe Ala His Gly Ala Arg Gln
                325                 330                 335

Leu Arg Ala Val Asn Pro Ser Ala Ala Ser Met Asp Ser Pro Ser Pro
            340                 345                 350

Thr Ser Ser Ser Pro Pro Arg Thr Ser Arg Pro Ala Ala Leu Thr Ala
        355                 360                 365

Ser Leu Ser Ser Arg Asp Leu Asp Leu Asp Ala Asp Asn Gln Ala Gln
    370                 375                 380

Tyr Ala Arg Arg Met Met Met Ala Arg Ala Asn Ser Pro Pro Asp Tyr
385                 390                 395                 400

Ser Pro Asp Leu Val Ala Ala Tyr Val Gln Ala Leu Ser Ser Leu Gln
                405                 410                 415
```

Gln Gln Gln His Gln Gln Asn Gln Gln Gln Gln His Gln Gln Gln Asn
420 425 430

Gln His Gln Gln Gln His Gln Gln Asn Gln His Gln Gln His Gln Gln
435 440 445

Gln His Gln Gln Ser Met Gly Met Gly Gly Leu Ser Ala Arg Ala Ala
450 455 460

Ala Phe Thr Asn Arg Ser Gln Thr Phe Val His Arg Ser Pro Ser Pro
465 470 475 480

Ala Pro Ala Arg Ser Phe Lys Ser Pro Ala Pro Ser Ser Met Leu Ala
485 490 495

Asp Trp Gly Ser Pro Asp Gly Lys Leu Asp Trp Gly Val Gln Ala Ala
500 505 510

Glu Leu Arg Lys Ser Thr Ser Phe Gly Val Arg Ser Ser Ser Arg Pro
515 520 525

His His Glu Thr Thr Arg Ala Glu Asp Asn Met Tyr Pro Ser Trp Met
530 535 540

Lys Asp Gly Ser Asp Met Leu Leu Ala Ala Arg Trp Ser Asp Leu Glu
545 550 555 560

Gln Met Val Ala

<210> 40
<211> 3610
<212> DNA
<213> Pinus radiata

<400> 40
tgtttccagg cgggcactaa agcaagggag ggggtaggct ttactttctg ctctgcgcaa 60

agaacgttga aatcaatcgc cctggctggt ctggcgtgac tactagattc aatttcttca 120

tggccgtctt cacataccca ttctttaccg gttcagagct gtgatcttta tttttaacag 180

ccacaatcat ggtttgtgtt tccagtgtta tgatctgagt gaagttcgtt ctttttctcg 240

tgacccaggc ttgatactag gccggacctt tctgaggtgg aagagatcta tacatttgag 300

gcctattttg tgtagccatg tgtggaggcc cagaacattt gaagcctgcc agcccacacg 360

aaggagaaga taaagtcaaa atggccgaga atcagtctat caaagtgaag gaattgtctg 420

aatcttgttc aagtctacat gaactagctg ctaataatga ccttattggc tttaagaaag 480

caatggagga agaagggtca aagatagatg aggttaactt ttggtacggg aggcagaatg 540

```
gttctaatca gatggtcctg gagcaaagga ctccattgat ggttgctgca ctttatggca    600
gtgtagatgc gctgagttac atcttatcca tttatgtaac ttgtggagca gatgttaacc    660
aagcctgtgg gtcagataac tccactgcct tgcattgtgc ggctgtggga gggtctgcct    720
gtgcagttga aactgtaaaa ttgttacttc atgcaggcag tgatgtgaat cgcttggatg    780
cttatggcag aagaccagca gatgtgatta tggtttctcc taagctaacc gaaatcaagg    840
ccaagctaga agaaatgtta aacgcagctg gttcatgtca aacttctccg gcaaagttgc    900
ctaacatagt ttcagggcca cctgggtttg agtcaaaggg gatggagtcc atgtccccat    960
tgccattgtt gcctctttca ttgtctttag aagcatccaa taatagatca ggttgtgtga   1020
attctccaac atcttcgcca aagtccatgg aagcattaaa gggtttcggt gatgttaatg   1080
agaagaagga atatcctgtg gacccttctt ttccagacat aaagaatagc atctatacta   1140
cagatgaatt tcggatgttt tccttcaagg tgcggccatg ttcacgggca tattctcatg   1200
attggactga atgcccattt gtgcatcctg gtgaaaatgc cagaaggcgg gatccaagaa   1260
ggtatcatta tagctgtgtt ccttgcccag attttcggaa agggacttgt aggcgcagtg   1320
atgtttgtga atatgcacac ggtgtttttg agtgctggtt acatcctgct caatatagga   1380
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc ccctggttct ccctcttcag   1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgatttttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
cgataaattc aagaatggct atggctgctt ttgttcagag ggaaaaacgg agccatagtt   2340
cccgtgactt gggagctaat gtgaatccca gttcatggtc tgattggggc tcgcctacag   2400
gtaaagttga ctgggggggtt caaggagaag agttgagcaa attaagaaag tcggcttcat   2460
ttggtccccg cagttatgaa gaaccggatt tgtcttgggt tcaaacactg gtaaaggaaa   2520
```

ctacaccaga gggtaaagat ggaggaaatg taagctgttc tggggaaact ccacacaagg 2580

ggcaaataga aaatgttgat cattcagttt tgggtgcctg gattgaacag atgcagcttg 2640

atcagattgt agcttgagat taggattatt tatttggagt ggtggtaggg ataggctcat 2700

ttaaaattca atttctcatt ttttactatt tcttttataa aaattcccca ttatagttta 2760

ggaaatagtc tggttttcta cctattatca gaattacacc tgcaggaaat tttggaggaa 2820

agcatgcaaa aagtagatag ggatgttatt cctatcagca ggttgacaag ctgaaaatca 2880

cttgggtggt agaccagaga atgacactat tttttgttga catggcaact gaagatgctg 2940

ttttctttac ttatcattaa caaccctata tatatttgtt ttgaaagaac tgagcggaga 3000

aatgttgtca gttggttact ctgcgcaagg ccttggaaga aatccaagat gtggcatctt 3060

ggtgcatttt taatttatca agtgtgaaat ccataacagg tttcagtgag tgacttctga 3120

ggttgtatat ggaaaaacct atgatgttgg ctgtctactg ctatttttct gtgcctaaac 3180

tgtcaactaa agtttgcagg tggcaatttt gtggcagcat atttgcacat tgaagcggat 3240

ggtctgcacc tgctatagaa gttttcgagt ctgtagaatt tgatggtgca agatgatttt 3300

ctagttgata tatttggaag gctttgccaa agtagtggca tgtacatttt gcaaaaattt 3360

aaaggatggc aatccattgt tttgccatgt agcttcactt tattgattag gtggaaagga 3420

attttgagac acttcaattt gtgcatactt ttgttctgaa ctgcaaaatc agtctcttgt 3480

gatgtcctca aggctattat gctcagggat ttgcctaaaa ccataagtgg ccttagataa 3540

ggtaccattg tattaccttt tattgtttgg atattttatt tatgaaagtg aatttatttt 3600

aaaaaaaaaa 3610

<210> 41
<211> 779
<212> PRT
<213> Pinus radiata

<400> 41

Met Cys Gly Gly Pro Glu His Leu Lys Pro Ala Ser Pro His Glu Gly
1 5 10 15

Glu Asp Lys Val Lys Met Ala Glu Asn Gln Ser Ile Lys Val Lys Glu
20 25 30

Leu Ser Glu Ser Cys Ser Ser Leu His Glu Leu Ala Ala Asn Asn Asp
35 40 45

Leu Ile Gly Phe Lys Lys Ala Met Glu Glu Glu Gly Ser Lys Ile Asp
50 55 60

Glu Val Asn Phe Trp Tyr Gly Arg Gln Asn Gly Ser Asn Gln Met Val
65 70 75 80

```
Leu Glu Gln Arg Thr Pro Leu Met Val Ala Ala Leu Tyr Gly Ser Val
                85                  90                  95

Asp Ala Leu Ser Tyr Ile Leu Ser Ile Tyr Val Thr Cys Gly Ala Asp
            100                 105                 110

Val Asn Gln Ala Cys Gly Ser Asp Asn Ser Thr Ala Leu His Cys Ala
        115                 120                 125

Ala Val Gly Gly Ser Ala Cys Ala Val Glu Thr Val Lys Leu Leu Leu
    130                 135                 140

His Ala Gly Ser Asp Val Asn Arg Leu Asp Ala Tyr Gly Arg Arg Pro
145                 150                 155                 160

Ala Asp Val Ile Met Val Ser Pro Lys Leu Thr Glu Ile Lys Ala Lys
                165                 170                 175

Leu Glu Glu Met Leu Asn Ala Ala Gly Ser Cys Gln Thr Ser Pro Ala
            180                 185                 190

Lys Leu Pro Asn Ile Val Ser Gly Pro Pro Gly Phe Glu Ser Lys Gly
        195                 200                 205

Met Glu Ser Met Ser Pro Leu Pro Leu Leu Pro Leu Ser Leu Ser Leu
    210                 215                 220

Glu Ala Ser Asn Asn Arg Ser Gly Cys Val Asn Ser Pro Thr Ser Ser
225                 230                 235                 240

Pro Lys Ser Met Glu Ala Leu Lys Gly Phe Gly Asp Val Asn Glu Lys
                245                 250                 255

Lys Glu Tyr Pro Val Asp Pro Ser Phe Pro Asp Ile Lys Asn Ser Ile
            260                 265                 270

Tyr Thr Thr Asp Glu Phe Arg Met Phe Ser Phe Lys Val Arg Pro Cys
        275                 280                 285

Ser Arg Ala Tyr Ser His Asp Trp Thr Glu Cys Pro Phe Val His Pro
    290                 295                 300

Gly Glu Asn Ala Arg Arg Arg Asp Pro Arg Arg Tyr His Tyr Ser Cys
305                 310                 315                 320

Val Pro Cys Pro Asp Phe Arg Lys Gly Thr Cys Arg Arg Ser Asp Val
                325                 330                 335
```

```
Cys Glu Tyr Ala His Gly Val Phe Glu Cys Trp Leu His Pro Ala Gln
            340                 345                 350

Tyr Arg Thr Arg Leu Cys Lys Asp Gly Thr Asn Cys Ser Arg Arg Val
        355                 360                 365

Cys Phe Phe Ala His Thr Ser Glu Glu Leu Arg Pro Leu Ile Val Ser
    370                 375                 380

Thr Gly Ser Ala Val Pro Ser Pro Arg Ala Ser Ser Ser Leu Asp Met
385                 390                 395                 400

Thr Ser Val Met Ser Pro Leu Ala Pro Gly Ser Pro Ser Ser Val Ser
                405                 410                 415

Met Met Ser Pro Phe Leu Ser Asn Pro Gln Gln Gly Ser Val Leu Thr
            420                 425                 430

Pro Pro Met Ser Pro Ser Ala Ser Ser Val Asn Gly Tyr Gly Gly Trp
        435                 440                 445

Pro Gln Pro Asn Val Pro Thr Leu His Leu Pro Gly Ser Asn Val Gln
    450                 455                 460

Thr Ser Arg Leu Arg Ala Glu Leu Asn Ala Arg Asp Met Pro Val Glu
465                 470                 475                 480

Asp Ser Pro Arg Ile Ser Asp Tyr Glu Gly Gln Gln Leu Leu Asn Asp
                485                 490                 495

Phe Ser Pro Leu Ser Thr Gln Ala Arg Leu Asn Ala Ala Ala Ala Val
            500                 505                 510

Ile Ser Gly Gly Gly Asn Thr Thr Thr Arg Ser Gly Lys Tyr Lys Ser
        515                 520                 525

His Gly Ile Asn Thr Val Ala Pro Thr Asn Leu Glu Asp Leu Phe Ala
    530                 535                 540

Ser Glu Val Thr Ser Pro Arg Val Ala Val Leu Glu Pro Ser Ile Phe
545                 550                 555                 560

Ser Gln Met Ser Pro Gln Met Gln Ala His Lys Thr Ala Gln Ala Tyr
                565                 570                 575

Met Gln Ile Gln Asn Gln Met Leu Pro Pro Ile Asn Thr Gln Ala Phe
            580                 585                 590
```

Ser Gln Gly Ile Thr Gln Met Gln Gln Ala Ala Ile Glu Pro Gln Ser
595 600 605

Pro Gly His Ser Leu Met Gln Ser Pro Phe Gln Ser Ser Ser Tyr Gly
610 615 620

Leu Gly Ser Pro Gly Arg Met Ser Pro Arg Cys Val Asp Val Glu Arg
625 630 635 640

His Asn Thr Cys Gly Ser Pro Leu Ser Pro Ala Met Ala Ala Thr Ile
645 650 655

Asn Ser Arg Met Ala Met Ala Ala Phe Val Gln Arg Glu Lys Arg Ser
660 665 670

His Ser Ser Arg Asp Leu Gly Ala Asn Val Asn Pro Ser Ser Trp Ser
675 680 685

Asp Trp Gly Ser Pro Thr Gly Lys Val Asp Trp Gly Val Gln Gly Glu
690 695 700

Glu Leu Ser Lys Leu Arg Lys Ser Ala Ser Phe Gly Pro Arg Ser Tyr
705 710 715 720

Glu Glu Pro Asp Leu Ser Trp Val Gln Thr Leu Val Lys Glu Thr Thr
725 730 735

Pro Glu Gly Lys Asp Gly Gly Asn Val Ser Cys Ser Gly Glu Thr Pro
740 745 750

His Lys Gly Gln Ile Glu Asn Val Asp His Ser Val Leu Gly Ala Trp
755 760 765

Ile Glu Gln Met Gln Leu Asp Gln Ile Val Ala
770 775

<210> 42
<211> 3610
<212> DNA
<213> Pinus radiata

<400> 42
tgtttccagg cgggcactaa agcaagggag ggggtaggct ttactttctg ctctgcgcaa 60

agaacgttga aatcaatcgc cctggctggt ctggcgtgac tactagattc aatttcttca 120

tggccgtctt cacataccca ttctttaccg gttcagagct gtgatcttta tttttaacag 180

ccacaatcat ggtttgtgtt tccagtgtta tgatctgagt gaagttcgtt ctttttctcg 240

tgacccaggc ttgatactag gccggacctt tctgaggtgg aagagatcta tacatttgag 300

gcctattttg tgtagccatg tgtggaggcc cagaacattt gaagcctgcc agcccacacg 360

aaggagaaga taaagtcaaa atggccgaga atcagtctat caaagtgaag gaattgtctg 420
aatcttgttc aagtctacat gaactagctg ctaataatga ccttattggc tttaagaaag 480
caatggagga agaagggtca aagatagatg aggttaactt ttggtacggg aggcagaatg 540
gttctaatca gatggtcctg gagcaaagga ctccattgat ggttgctgca ctttatggca 600
gtgtagatgc gctgagttac atcttatcca tttatgtaac ttgtggagca gatgttaacc 660
aagcctgtgg gtcagataac tccactgcct tgcattgtgc ggctgtggga gggtctgcct 720
gtgcagttga aactgtaaaa ttgttacttc atgcaggcag tgatgtgaat cgcttggatg 780
cttatggcag aagaccagca gatgtgatta tggtttctcc taagctaacc gaaatcaagg 840
ccaagctaga agaaatgtta aacgcagctg gttcatgtca aacttctccg gcaaagttgc 900
ctaacatagt ttcagggcca cctgggtttg agtcaaaggg gatggagtcc atgtccccat 960
tgccattgtt gcctctttca ttgtctttag aagcatccaa taatagatca ggttgtgtga 1020
attctccaac atcttcgcca aagtccatgg aagcattaaa gggtttcggt gatgttaatg 1080
agaagaagga atatcctgtg gacccttctt ttccagacat aaagaatagc atctatacta 1140
cagatgaatt tcggatgttt tccttcaagg tgcggccatg ttcacgggca tattctcatg 1200
attggactga atgcccattt gtgcatcctg gtgaaaatgc cagaaggcgg gatccaagaa 1260
ggtatcatta tagctgtgtt ccttgcccag attttcggaa agggacttgt aggcgcagtg 1320
atgtttgtga atatgcacac ggtgtttttg agtgctggtt acatcctgct caatatagga 1380
cacggttgtg caaagatggg actaattgtt cacgtagagt ttgcttcttt gctcacacat 1440
ctgaggaact acgccctctc attgtctcta ctgggtctgc tgttccatcc ccaagggcat 1500
catcatctct ggacatgaca tctgtcatga gtcctcttgc ccctggttct ccctcttcag 1560
tttcaatgat gtcacccttc ctatcaaatc ctcagcaagg cagtgtgctt actccgccta 1620
tgtctccatc agcgtcctct gtaaatggat atggaggctg gccacagcct aatgtaccaa 1680
ccttacacct tcctggtagc aatgttcaaa ccagccgtct tagagcggaa cttaatgcca 1740
gagacatgcc tgttgaggat tctcctcgaa tttcagacta tgaagggcag caactcctga 1800
atgatttttc tccactgtcc acacaagcca ggctgaatgc tgctgctgct gttatatctg 1860
gtggcgggaa caccacaaca aggtctggaa aatacaagag tcacgggatc aatactgttg 1920
ctccaacgaa tcttgaagac ttgtttgcct ccgaggtaac atctcctaga gtagcagttc 1980
ttgaaccttc catcttttct cagatgagtc cccaaatgca agctcataag actgcccagg 2040
catatatgca gattcaaaac cagatgctgc ctcctataaa tacacaggca ttttcgcagg 2100
gaattacaca gatgcagcag gctgcaatag agcctcagag ccctggacat tctttgatgc 2160
aatcaccttt ccaatcttcc tcgtatgggt tgggatcccc tggtagaatg tcacctcgtt 2220
gtgtggatgt ggaacgtcat aatacatgtg ggtctccctt atcaccggct atggctgcaa 2280

cgataaattc aagaatggct atggctgctt ttgttcagag ggaaaaacgg agccatagtt 2340

cccgtgactt gggagctaat gtgaatccca gttcatggtc tgattggggc tcgcctacag 2400

gtaaagttga ctgggggtt caaggagaag agttgagcaa attaagaaag tcggcttcat 2460

ttggtccccg cagttatgaa gaaccggatt tgtcttgggt tcaaacactg gtaaaggaaa 2520

ctacaccaga gggtaaagat ggaggaaatg taagctgttc tggggaaact ccacacaagg 2580

ggcaaataga aaatgttgat cattcagttt tgggtgcctg gattgaacag atgcagcttg 2640

atcagattgt agcttgagat taggattatt tatttggagt ggtggtaggg ataggctcat 2700

ttaaaattca atttctcatt ttttactatt tcttttataa aaattcccca ttatagttta 2760

ggaaatagtc tggttttcta cctattatca gaattacacc tgcaggaaat tttggaggaa 2820

agcatgcaaa aagtagatag ggatgttatt cctatcagca ggttgacaag ctgaaaatca 2880

cttgggtggt agaccagaga atgacactat tttttgttga catggcaact gaagatgctg 2940

ttttctttac ttatcattaa caaccctata tatatttgtt ttgaaagaac tgagcggaga 3000

aatgttgtca gttggttact ctgcgcaagg ccttggaaga aatccaagat gtggcatctt 3060

ggtgcatttt taatttatca agtgtgaaat ccataacagg tttcagtgag tgacttctga 3120

ggttgtatat ggaaaaacct atgatgttgg ctgtctactg ctatttttct gtgcctaaac 3180

tgtcaactaa agtttgcagg tggcaatttt gtggcagcat atttgcacat tgaagcggat 3240

ggtctgcacc tgctatagaa gttttcgagt ctgtagaatt tgatggtgca agatgatttt 3300

ctagttgata tatttggaag gctttgccaa agtagtggca tgtacatttt gcaaaaattt 3360

aaaggatggc aatccattgt tttgccatgt agcttcactt tattgattag gtggaaagga 3420

attttgagac acttcaattt gtgcatactt ttgttctgaa ctgcaaaatc agtctcttgt 3480

gatgtcctca aggctattat gctcagggat ttgcctaaaa ccataagtgg ccttagataa 3540

ggtaccattg tattaccttt tattgtttgg atattttatt tatgaaagtg aatttatttt 3600

aaaaaaaaaa 3610

<210> 43
<211> 749
<212> PRT
<213> Pinus radiata

<400> 43

Met Lys Glu Met Ala Glu Tyr Cys Ser Pro Ala Leu Leu Glu Leu Ala
1 5 10 15

Ala Asn Asn Asp Leu Ser Gly Phe Lys Gln Ala Val Glu Glu Gly Gly
20 25 30

Ser Ser Val Asn Glu Arg Gly Leu Trp Tyr Gly Arg Gln Ile Gly Ser
35 40 45

```
Gly Gln Lys Met Val Leu Glu Gln Arg Thr Pro Leu Met Val Ala Ala
    50                  55                  60

Leu Tyr Gly Ser Leu Asp Val Leu Ser Tyr Met Leu Ser Gly Gly Arg
65                  70                  75                  80

Val Asp Val Asn Gln Ser Cys Gly Ser Asp Met Ser Thr Ala Leu His
                85                  90                  95

Cys Ala Ala Ala Gly Gly Ser Ile Leu Ala Ile Glu Thr Val Gly Met
            100                 105                 110

Leu Ile Lys Ala Gly Ala Asp Val Asn Phe Met Asn Ala Gly Gly Arg
        115                 120                 125

Lys Pro Ala Asp Val Ile Met Val Ser Pro Lys Leu Ala His Phe Lys
    130                 135                 140

Asn Val Leu Glu Asp Leu Leu Ile Met Gly Ser Asn Ser Pro Met Lys
145                 150                 155                 160

Ile Pro Cys Arg Val Ser Gly Ser Gly Phe Tyr Leu Pro Glu Gly Gly
                165                 170                 175

Gly Cys Phe Phe Asp Glu His Gly Cys Val Val Ser Val Pro Thr Ser
            180                 185                 190

Ser Pro Leu Phe Ser Ser Pro Asp Ala Thr Ser Pro Ala Thr Val Asn
        195                 200                 205

Ser Pro Leu Ser Ser Pro Pro Thr Ser Leu Asp Thr Pro Lys Asn Leu
    210                 215                 220

Cys Asp Cys Gly Gln Lys Lys Glu Phe Ala Val Asp Ser Ser Leu Pro
225                 230                 235                 240

Asp Ile Lys Asn Ser Ile Tyr Ser Thr Asp Glu Phe Arg Met Tyr Ser
                245                 250                 255

Phe Lys Val Arg Pro Cys Ser Arg Ala Tyr Ser His Asp Trp Thr Glu
            260                 265                 270

Cys Pro Phe Val His Pro Gly Glu Asn Ala Arg Arg Arg Asp Pro Arg
        275                 280                 285

Lys Tyr His Tyr Ser Cys Val Pro Cys Pro Asp Phe Arg Lys Gly Ala
    290                 295                 300
```

```
Cys Arg Arg Gly Asp Val Cys Glu Tyr Ala His Gly Val Phe Glu Cys
305                 310                 315                 320

Trp Leu His Pro Ala Gln Tyr Arg Thr Arg Leu Cys Lys Asp Gly Thr
                325                 330                 335

Asn Cys Ser Arg Arg Val Cys Phe Phe Ala His Thr Pro Glu Glu Leu
            340                 345                 350

Arg Pro Leu Tyr Pro Pro Ala Cys Ser Ser Met Leu Ser Gln Arg Thr
        355                 360                 365

Thr Met Thr Ser Ser Asp Lys Met Ala Val Met His Pro Leu Ala Pro
    370                 375                 380

Gly Ser Ala Ser Ser Val Leu Met Met Ser Ser Ser Asn Ser Ser Gln
385                 390                 395                 400

Ser Ser Phe Pro Asn Ser Pro Val Ser Pro Leu Ser Ser Ala Asn Thr
                405                 410                 415

Ser Ser His Ser Ser Phe Gly Gly Gly Ser Trp Ala His Pro Asn Leu
            420                 425                 430

Pro Thr Leu His Leu Ser Asn Gly Ala Leu Gln Ala Ser Arg Leu Arg
        435                 440                 445

Thr Ala Val Asn Ala Arg Asp Met His Pro Asp Cys Ser Ile Glu Ser
    450                 455                 460

Gly Asp Tyr Glu Gly Gln Leu Leu Asn Glu Phe Ala Tyr Leu Ser Thr
465                 470                 475                 480

Gln Ala Arg Gly Asn Gly Pro Met Ala Thr Val Ser Ser Ser Gly Asn
                485                 490                 495

Thr Pro Cys Arg Pro Arg Lys Phe Arg Ala His Asn Val Ala Pro Thr
            500                 505                 510

Asn Leu Glu Asp Leu Phe Ala Ser Glu Val Phe Ser Pro Lys Met Thr
        515                 520                 525

Ala Ser Glu Ser Ala Phe Leu Ser Glu Ile Gln Ser His Lys Ser Ala
    530                 535                 540

Gln Leu Ser Pro Gln Leu Gln Ser Gln Met Leu Ser Ser Phe Asn Thr
545                 550                 555                 560

Gln Val Tyr Pro Gln Gly Ser Thr Gln Gly Gln Met His Met Gln His
```

```
                565                 570                 575

Gly Gly Val Asp Cys Gln Ser Pro Ser Val Phe Leu Ser Pro Pro Pro
            580                 585                 590

Val Gln Leu Ala Ser Tyr Ser Leu Ser Ser Leu Gly Pro Leu Ser Ser
        595                 600                 605

Leu Thr Gly Glu Leu Glu Arg Gln Asn Ser Asn Gly Ser Pro Leu Ser
    610                 615                 620

Pro Ile Met Ser Thr Ala Ala Asp Ser Arg Ala Val Ala Phe Ser Gln
625                 630                 635                 640

Arg Asp Lys Gly Ser Ser Arg Ser Gly Asp Leu Gly Gly Ala Thr Thr
                645                 650                 655

Trp Ser Glu Trp Gly Ser Pro Thr Gly Lys Val Asn Trp Gly Ile Arg
            660                 665                 670

Gly Glu Glu Leu Gln Lys Phe Arg Lys Ser Ala Ser Phe Gly Ile Arg
        675                 680                 685

Ser Ser Asp Glu Pro Asp Leu Ser Trp Val Gln Lys Leu Phe Lys Glu
    690                 695                 700

Ala Pro Met Glu Ser Met Asp Arg Gly Thr Met Gly Arg Ser Met Asp
705                 710                 715                 720

Ile Ala Asn Ser Val Gln Met Glu Ala Thr Asp Leu Gly Gly Trp Ile
                725                 730                 735

Ser Gln Ile Asn Pro Asp Gln Val Ala Pro Leu Thr Leu
            740                 745


<210>  44
<211>  711
<212>  PRT
<213>  Glycine max

<400>  44

Lys Ser Ala Asn Asp Lys Glu Met Lys Ser Leu Thr Val Asn Thr Glu
1               5                   10                  15

Asp Ser Phe Ser Ser Leu Leu Glu Leu Ala Ser Asn Asn Asp Ile Glu
            20                  25                  30

Gly Phe Lys Val Leu Leu Glu Lys Asp Ser Ser Ser Ile Asn Glu Val
        35                  40                  45
```

```
Gly Leu Trp Tyr Gly Arg Gln Asn Gly Ser Lys Gln Phe Val Leu Glu
    50                  55                  60

His Arg Thr Pro Leu Met Val Ala Ala Thr Tyr Gly Ser Ile Asp Val
65                  70                  75                  80

Met Lys Ile Ile Leu Leu Cys Pro Glu Ala Asp Val Asn Phe Ala Cys
                85                  90                  95

Gly Ala Asn Lys Thr Thr Ala Leu His Cys Ala Ala Ser Gly Gly Ser
            100                 105                 110

Ala Asn Ala Val Asp Ala Val Lys Ile Leu Leu Ser Ala Gly Ala Asp
        115                 120                 125

Val Asn Gly Val Asp Ala Asn Gly Asn Arg Pro Ile Asp Val Ile Ala
    130                 135                 140

Val Pro Pro Lys Leu Gln Gly Ala Lys Ala Val Leu Glu Glu Leu Leu
145                 150                 155                 160

Ser Asp Ser Ala Ser Glu Gly Ser Ile Gly Glu Phe Ser Val Pro Val
                165                 170                 175

Ser Val Asn Thr Ser Ser Leu Gly Ser Pro Gly His Ser Ser Asn Gly
            180                 185                 190

Met Pro Tyr Thr Pro Ser Ser Ser Pro Pro Ser Pro Val Val Ala Lys
        195                 200                 205

Phe Thr Asp Ala Ala Val Cys Ser Leu Ser Glu Lys Lys Glu Tyr Pro
    210                 215                 220

Ile Asp Pro Ser Leu Pro Asp Ile Lys Asn Ser Ile Tyr Ala Thr Asp
225                 230                 235                 240

Glu Phe Arg Met Phe Ser Phe Lys Val Arg Pro Cys Ser Arg Ala Tyr
                245                 250                 255

Ser His Asp Trp Thr Glu Cys Pro Phe Val His Pro Gly Glu Asn Ala
            260                 265                 270

Arg Arg Arg Asp Pro Arg Lys Phe His Tyr Ser Cys Val Pro Cys Pro
        275                 280                 285

Asp Phe Arg Lys Gly Ala Cys Arg Arg Gly Asp Met Cys Glu Tyr Ala
    290                 295                 300
```

His Gly Val Phe Glu Cys Trp Leu His Pro Ala Gln Tyr Arg Thr Arg
305 310 315 320

Leu Cys Lys Asp Gly Thr Ser Cys Asn Arg Arg Val Cys Phe Phe Ala
325 330 335

His Thr Ala Glu Glu Leu Arg Pro Leu Tyr Val Ser Thr Gly Ser Ala
340 345 350

Val Pro Ser Pro Arg Ser Ser Ala Ser Ala Pro Asn Val Met Asp Met
355 360 365

Ala Ala Ala Met Ser Leu Leu Pro Gly Ser Pro Ser Ser Val Ser Ser
370 375 380

Met Ser Pro Ser His Phe Gly Gln Pro Met Ser Pro Ser Ala Asn Gly
385 390 395 400

Met Ser Leu Ser Ser Ala Trp Ala Gln Pro Asn Val Ser Ala Leu His
405 410 415

Leu Pro Gly Ser Asn Leu Gln Ser Ser Arg Leu Arg Ser Ser Leu Ser
420 425 430

Ala Arg Asp Met Pro Pro Asp Asp Leu Asn Met Met Ser Asp Leu Asp
435 440 445

Gly Gln Gln Gln His Pro Leu Asn Asp Leu Ser Cys Tyr Leu Gln Pro
450 455 460

Arg Pro Gly Ala Gly Ser Val Ser Arg Ser Gly Arg Ser Lys Ile Leu
465 470 475 480

Thr Pro Ser Asn Leu Glu Asp Leu Phe Ser Ala Glu Ile Ser Ser Ser
485 490 495

Pro Arg Tyr Ser Asp Pro Ala Ala Gly Ser Val Phe Ser Pro Thr His
500 505 510

Lys Ser Ala Val Leu Asn Gln Phe Gln Gln Leu Gln Ser Met Leu Ser
515 520 525

Pro Ile Asn Thr Asn Leu Leu Ser Pro Lys Asn Val Glu His Pro Leu
530 535 540

Leu Gln Ala Ser Phe Gly Val Ser Pro Ser Gly Arg Met Ser Pro Arg
545 550 555 560

Ser Val Glu Pro Ile Ser Pro Met Ser Ser Arg Ile Ser Ala Phe Ala

565 570 575

Gln Arg Glu Lys Gln Gln Gln Gln Gln Gln Gln Leu Arg Ser Leu Ser
580 585 590

Ser Arg Asp Leu Gly Ala Asn Ser Pro Ala Ser Leu Val Gly Ser Pro
595 600 605

Ala Asn Pro Trp Ser Lys Trp Gly Ser Pro Asn Gly Lys Ala Asp Trp
610 615 620

Ser Val Asn Gly Asp Thr Leu Gly Arg Gln Met Arg Arg Ser Ser Ser
625 630 635 640

Phe Glu Leu Lys Asn Asn Gly Glu Glu Pro Asp Leu Ser Trp Val Gln
645 650 655

Ser Leu Val Lys Glu Ser Pro Pro Glu Met Ile Lys Glu Lys Phe Ala
660 665 670

Ser Pro Met Pro Thr Ala Ser Ala Asp Gly Pro Asn Ser Asn Ser Gln
675 680 685

Ile Glu Ser Ile Asp His Ser Val Leu Gly Ala Trp Leu Glu Gln Met
690 695 700

Gln Leu Asp Gln Leu Val Val
705 710

<210> 45
<211> 643
<212> PRT
<213> Glycine max

<400> 45

Arg Gly Ser Gly Phe Pro Gly Arg Pro Thr Arg Pro Arg Ser Gly Arg
1 5 10 15

Thr Arg Gly Arg Thr Arg Gly Val Asn Phe Ala Cys Gly Ala Asn Lys
20 25 30

Thr Thr Ala Leu His Cys Ala Ala Ser Gly Ala Ser Thr Lys Ala Val
35 40 45

Asp Ala Val Lys Leu Leu Leu Ser Ala Gly Ala Asp Val Asn Cys Val
50 55 60

Asp Ala Asn Gly Asn Arg Pro Ile Asp Val Ile Ala Val Pro Pro Lys
65 70 75 80

```
Leu Gln Gly Ala Lys Ala Val Leu Glu Glu Leu Leu Ser Asp Asn Ala
                85                  90                  95

Ser Asp Val Ser Val Gly Glu Phe Ser Val Pro Val Ser Val Asn Ser
            100                 105                 110

Ser Ser Pro Gly Ser Pro Ala His Ser Ser Asn Gly Met Pro Tyr Thr
        115                 120                 125

Pro Ser Val Ser Pro Pro Ser Pro Val Ala Ala Lys Phe Thr Asp Ala
    130                 135                 140

Ala Ile Cys Ser Leu Ser Glu Lys Ala Arg Glu Tyr Pro Ile Asp Pro
145                 150                 155                 160

Ser Leu Pro Asp Ile Lys Asn Ser Ile Tyr Ala Thr Asp Glu Phe Arg
                165                 170                 175

Met Phe Ser Phe Lys Val Arg Pro Cys Ser Arg Ala Tyr Ser His Asp
            180                 185                 190

Trp Thr Glu Cys Pro Phe Val His Pro Gly Glu Asn Ala Arg Arg Arg
        195                 200                 205

Asp Pro Arg Lys Phe His Tyr Ser Cys Val Pro Cys Pro Asp Phe Arg
    210                 215                 220

Lys Gly Ala Cys Arg Arg Gly Asp Met Cys Glu Tyr Ala His Gly Val
225                 230                 235                 240

Phe Glu Cys Trp Leu His Pro Ala Gln Tyr Arg Thr Arg Leu Cys Lys
                245                 250                 255

Asp Gly Thr Ser Cys Asn Arg Arg Val Cys Phe Phe Ala His Thr Ala
            260                 265                 270

Glu Glu Leu Arg Pro Leu Tyr Val Ser Thr Gly Ser Ala Ala Pro Ser
        275                 280                 285

Pro Arg Ser Ser Ala Ser Gly Pro Asn Val Met Asp Met Ala Ala Ala
    290                 295                 300

Met Ser Leu Phe Pro Gly Ser Pro Ser Ser Gly Ser Ser Ile Ser Leu
305                 310                 315                 320

Ser Ile Ser Phe Ser Leu Asp Pro Met Ser Pro Ser Ala Asn Gly Met
                325                 330                 335
```

Pro Leu Ser Ser Ala Trp Ala Gln Pro Asn Val Pro Ala Leu His Leu
340 345 350

Pro Gly Ser Asn Leu Gln Ser Ser Arg Leu Arg Ser Ser Leu Ser Ala
355 360 365

Arg Asp Ile Pro Pro Glu Asp Leu Asn Met Met Ser Asp Leu Asp Gly
370 375 380

Gln Gln Gln His His Leu Asn Asp Leu Ser Cys Tyr Ile Gln Pro Arg
385 390 395 400

Pro Gly Ala Ser Ser Val Ser Arg Ser Gly Arg Ser Lys Thr Leu Thr
405 410 415

Pro Ser Asn Leu Glu Glu Leu Phe Ser Ala Glu Ile Ser Leu Ser Pro
420 425 430

Arg Tyr Ser Asp Pro Ala Ala Gly Ser Val Phe Ser Pro Thr His Lys
435 440 445

Ser Ala Val Leu Asn Gln Phe Gln Gln Leu Gln Ser Met Leu Ser Pro
450 455 460

Ile Asn Thr Asn Leu Leu Ser Pro Lys Asn Val Glu His Pro Leu Phe
465 470 475 480

Gln Ala Ser Phe Gly Val Ser Pro Ser Gly Arg Met Ser Pro Arg Ser
485 490 495

Val Glu Pro Ile Ser Pro Met Ser Ala Arg Leu Ser Ala Phe Ala Gln
500 505 510

Arg Glu Lys Gln Gln Gln Gln Leu Arg Ser Val Ser Ser Arg Asp Leu
515 520 525

Gly Ala Asn Ser Pro Ala Ser Leu Val Gly Ser Pro Ala Asn Pro Trp
530 535 540

Ser Lys Trp Gly Ser Pro Ile Gly Lys Ala Asp Trp Ser Val Asn Gly
545 550 555 560

Asp Ser Leu Gly Arg Gln Met Arg Arg Ser Ser Ser Phe Glu Arg Lys
565 570 575

Asn Asn Gly Glu Glu Pro Asp Leu Ser Trp Val Gln Ser Leu Val Lys
580 585 590

Glu Ser Pro Pro Glu Met Ile Lys Glu Lys Phe Ala Ser Pro Met Pro

595 600 605

Thr Ala Ser Ala Asp Gly Pro Asn Ser Asn Ser Gln Ile Glu Ser Ile
610 615 620

Asp His Ser Val Leu Gly Ala Trp Leu Glu Gln Met Gln Leu Asp Gln
625 630 635 640

Leu Val Val

<210> 46
<211> 669
<212> PRT
<213> Glycine max

<400> 46

Ser His Glu Met Asn His Leu Ser Leu Asp Thr Glu Asp Ser Leu Ala
1 5 10 15

Ser Leu Leu Leu Glu Leu Ala Ala Asn Asn Asp Val Ser Gly Phe Lys
20 25 30

Arg Leu Ile Glu Cys Glu Pro Ser Ser Ile Asp Glu Val Gly Leu Trp
35 40 45

Tyr Gly Arg His Lys Glu Ser Lys Lys Met Val Asn Glu Gln Arg Thr
50 55 60

Pro Leu Met Val Ala Ala Thr Tyr Gly Ser Ile Asp Val Met Thr Leu
65 70 75 80

Ile Leu Ser Leu Ser Glu Ala Asp Val Asn Arg Ser Ser Gly Leu Asp
85 90 95

Lys Ser Thr Ala Leu His Cys Ala Ala Ser Gly Gly Ser Glu Asn Ala
100 105 110

Val Asp Ala Val Lys Leu Leu Leu Glu Ala Gly Ala Asp Arg Asn Ser
115 120 125

Val Asp Ala Asn Gly Arg Arg Pro Gly Asp Val Ile Val Ser Pro Pro
130 135 140

Lys Leu Asp Tyr Val Lys Lys Ser Leu Glu Glu Leu Leu Gly Ser Asp
145 150 155 160

Asp Trp Ser Leu Leu Arg Val Met Arg Ser Thr Cys Asn Gly Cys Ser
165 170 175

```
Ala Glu Asp Leu Lys Met Lys Thr Asn Glu Val Ser Glu Lys Lys Glu
            180                 185                 190

Tyr Pro Val Asp Leu Ser Leu Pro Asp Ile Lys Asn Ser Ile Tyr Ser
        195                 200                 205

Ser Asp Glu Phe Arg Met Tyr Ser Phe Lys Val Arg Pro Cys Ser Arg
    210                 215                 220

Ala Tyr Ser His Asp Trp Thr Glu Cys Pro Phe Val His Pro Gly Glu
225                 230                 235                 240

Asn Ala Arg Arg Arg Asp Pro Arg Lys Phe His Tyr Ser Cys Val Pro
                245                 250                 255

Cys Pro Glu Phe Arg Lys Gly Ala Cys Arg Arg Gly Asp Met Cys Glu
            260                 265                 270

Tyr Ala His Gly Val Phe Glu Cys Trp Leu His Pro Ala Gln Tyr Arg
        275                 280                 285

Thr Arg Leu Cys Lys Asp Gly Thr Asn Cys Ala Arg Arg Val Cys Phe
    290                 295                 300

Phe Ala His Thr Asn Glu Glu Leu Arg Pro Leu Tyr Val Ser Thr Gly
305                 310                 315                 320

Ser Ala Val Pro Ser Pro Arg Ser Ser Ala Ser Ser Ala Met Asp Phe
                325                 330                 335

Val Ala Ala Ile Ser Pro Ser Ser Met Ser Val Met Ser Pro Ser Pro
            340                 345                 350

Phe Thr Pro Pro Met Ser Pro Ser Ser Ala Ser Ile Ala Trp Pro Gln
        355                 360                 365

Pro Asn Ile Pro Ala Leu His Leu Pro Gly Ser Asn Phe His Ser Ser
    370                 375                 380

Arg Leu Arg Ser Ser Leu Asn Ala Arg Asp Phe Ser Val Asp Asp Phe
385                 390                 395                 400

Asp Leu Leu Leu Pro Asp Tyr Asp His His His His Gln Gln Gln Gln
                405                 410                 415

Gln Gln Phe Leu Asn Glu Leu Ser Cys Leu Ser Pro His Ala Met Asn
            420                 425                 430
```

Cys Asn Thr Met Asn Arg Ser Gly Arg Met Lys Pro Leu Thr Pro Ser
435 440 445

Asn Leu Asp Asp Leu Phe Ser Ala Glu Ser Ser Ser Pro Arg Tyr Ala
450 455 460

Asp Pro Ala Leu Ala Ser Ala Val Phe Ser Pro Thr His Lys Ser Ala
465 470 475 480

Val Phe Asn Gln Phe Gln His Gln Gln Ser Met Leu Ala Pro Leu Asn
485 490 495

Thr Asn Phe Ala Ser Lys Asn Phe Glu His Pro Leu Leu Gln Ala Ser
500 505 510

Leu Gly Met Ser Pro Arg Asn Val Glu Pro Ile Ser Pro Met Gly Ser
515 520 525

Arg Ile Ser Met Leu Ala Gln Arg Glu Lys Gln Gln Phe Arg Ser Leu
530 535 540

Ser Phe Arg Glu Leu Gly Ser Asn Ser Ala Ala Ala Ser Ala Asp Ser
545 550 555 560

Trp Ser Lys Trp Gly Ser Pro Asn Val Lys Leu Asp Trp Pro Val Gly
565 570 575

Ala Gly Glu Val Gly Lys Leu Arg Arg Ser Ser Ser Phe Glu Leu Gly
580 585 590

Asn Asn Gly Glu Glu Pro Asp Leu Ser Trp Val Gln Ser Leu Val Lys
595 600 605

Glu Ser Pro Ala Glu Val Lys Asp Lys Leu Ala Thr Thr Val Ser Tyr
610 615 620

Val Ala Ala Ala Ala Ala Gly Ser Ser Ser Glu Gly Ser Asn Ile Ser
625 630 635 640

Thr Gln Met Glu Ser Val Val Asp His Ala Val Leu Gly Ala Trp Leu
645 650 655

Glu Gln Met Gln Leu Asp His Leu Val Ala Gln Gln Asn
660 665

<210> 47
<211> 580
<212> PRT
<213> Arabidopsis thaliana

<400> 47

Met Cys Ser Gly Pro Lys Ser Asn Leu Cys Ser Ser Arg Thr Leu Thr
1 5 10 15

Glu Ile Glu Ser Arg Gln Lys Glu Glu Glu Thr Met Leu Leu Leu Glu
20 25 30

Phe Ala Ala Cys Asp Asp Leu Asp Ser Phe Lys Arg Glu Val Glu Glu
35 40 45

Lys Gly Leu Asp Leu Asp Glu Ser Gly Leu Trp Tyr Cys Arg Arg Val
50 55 60

Gly Ser Lys Lys Met Gly Leu Glu Glu Arg Thr Pro Leu Met Val Ala
65 70 75 80

Ala Met Tyr Gly Ser Ile Lys Val Leu Thr Phe Ile Val Ser Thr Gly
85 90 95

Lys Ser Asp Val Asn Arg Ala Cys Gly Glu Glu Arg Val Thr Pro Leu
100 105 110

His Cys Ala Val Ala Gly Cys Ser Val Asn Met Ile Glu Val Ile Asn
115 120 125

Val Leu Leu Asp Ala Ser Ala Leu Val Asn Ser Val Asp Ala Asn Gly
130 135 140

Asn Gln Pro Leu Asp Val Phe Val Arg Val Ser Arg Phe Val Ala Ser
145 150 155 160

Pro Arg Arg Lys Ala Val Glu Leu Leu Leu Arg Gly Gly Gly Val Gly
165 170 175

Gly Leu Ile Asp Glu Ala Val Glu Glu Glu Ile Lys Ile Val Ser Lys
180 185 190

Tyr Pro Ala Asp Ala Ser Leu Pro Asp Ile Asn Glu Gly Val Tyr Gly
195 200 205

Ser Asp Glu Phe Arg Met Tyr Ser Phe Lys Val Lys Pro Cys Ser Arg
210 215 220

Ala Tyr Ser His Asp Trp Thr Glu Cys Ala Phe Val His Pro Gly Glu
225 230 235 240

Asn Ala Arg Arg Arg Asp Pro Arg Lys Tyr Pro Tyr Thr Cys Val Pro
245 250 255

Cys Pro Glu Phe Arg Lys Gly Ser Cys Pro Lys Gly Asp Ser Cys Glu
260 265 270

Tyr Ala His Gly Val Phe Glu Ser Trp Leu His Pro Ala Gln Tyr Lys
275 280 285

Thr Arg Leu Cys Lys Asp Glu Thr Gly Cys Ala Arg Lys Val Cys Phe
290 295 300

Phe Ala His Lys Arg Glu Glu Met Arg Pro Val Asn Ala Ser Thr Gly
305 310 315 320

Ser Ala Val Ala Gln Ser Pro Phe Ser Ser Leu Glu Met Met Pro Gly
325 330 335

Leu Ser Pro Leu Ala Tyr Ser Ser Gly Val Ser Thr Pro Pro Val Ser
340 345 350

Pro Met Ala Asn Gly Val Pro Ser Ser Pro Arg Asn Gly Gly Ser Trp
355 360 365

Gln Asn Arg Val Asn Thr Leu Thr Pro Pro Ala Leu Gln Leu Asn Gly
370 375 380

Gly Ser Arg Leu Lys Ser Thr Leu Ser Ala Arg Asp Ile Asp Met Glu
385 390 395 400

Met Glu Met Glu Leu Arg Leu Arg Gly Phe Gly Asn Asn Val Glu Glu
405 410 415

Thr Phe Gly Ser Tyr Val Ser Ser Pro Ser Arg Asn Ser Gln Met Gly
420 425 430

Gln Asn Met Asn Gln His Tyr Pro Ser Ser Pro Val Arg Gln Pro Pro
435 440 445

Ser Gln His Gly Phe Glu Ser Ser Ala Ala Ala Ala Val Ala Val Met
450 455 460

Lys Ala Arg Ser Thr Ala Phe Ala Lys Arg Ser Leu Ser Phe Lys Pro
465 470 475 480

Ala Thr Gln Ala Ala Pro Gln Ser Asn Leu Ser Asp Trp Gly Ser Pro
485 490 495

Asn Gly Lys Leu Glu Trp Gly Met Lys Gly Glu Glu Leu Asn Lys Met
500 505 510

Arg Arg Ser Val Ser Phe Gly Ile His Gly Asn Asn Asn Asn Asn Ala
515 520 525

Ala Arg Asp Tyr Arg Asp Glu Pro Asp Val Ser Trp Val Asn Ser Leu
530 535 540

Val Lys Asp Ser Thr Val Val Ser Glu Arg Ser Phe Gly Met Asn Glu
545 550 555 560

Arg Val Arg Ile Met Ser Trp Ala Glu Gln Met Tyr Arg Glu Lys Glu
565 570 575

Gln Thr Val Val
580

<210> 48
<211> 719
<212> PRT
<213> Arabidopsis thaliana

<400> 48

Met Cys Cys Gly Ser Asp Arg Leu Asn Gln Ile Val Ser Ser Arg Ser
1 5 10 15

Ser Leu Pro Ile Ser Phe Glu Glu Asp Asn Asn Leu Val Thr Asn Thr
20 25 30

Asp Met Asn His Ile Thr Val Glu Thr Glu Asp Thr Phe Ala Ser Leu
35 40 45

Leu Glu Leu Ala Ala Asn Asn Asp Val Glu Gly Val Arg Leu Ser Ile
50 55 60

Glu Arg Asp Pro Ser Cys Val Asp Glu Ala Gly Leu Trp Tyr Gly Arg
65 70 75 80

Gln Lys Gly Ser Lys Ala Met Val Asn Asp Tyr Arg Thr Pro Leu Met
85 90 95

Val Ala Ala Thr Tyr Gly Ser Ile Asp Val Ile Lys Leu Ile Val Ser
100 105 110

Leu Thr Asp Ala Asp Val Asn Arg Ala Cys Gly Asn Asp Gln Thr Thr
115 120 125

Ala Leu His Cys Ala Ala Ser Gly Gly Ala Val Asn Ala Ile Gln Val
130 135 140

Val Lys Leu Leu Leu Ala Ala Gly Ala Asp Leu Asn Leu Leu Asp Ala
145 150 155 160

```
Glu Gly Gln Arg Ala Gly Asp Val Ile Val Val Pro Pro Lys Leu Glu
                165                 170                 175

Gly Val Lys Leu Met Leu Gln Glu Leu Leu Ser Ala Asp Gly Ser Ser
            180                 185                 190

Thr Ala Glu Arg Asn Leu Arg Val Val Thr Asn Val Pro Asn Arg Ser
        195                 200                 205

Ser Ser Pro Cys His Ser Pro Thr Gly Glu Asn Gly Gly Ser Gly Ser
    210                 215                 220

Gly Ser Pro Leu Gly Ser Pro Phe Lys Leu Lys Ser Thr Glu Phe Lys
225                 230                 235                 240

Lys Glu Tyr Pro Val Asp Pro Ser Leu Pro Asp Ile Lys Asn Ser Ile
                245                 250                 255

Tyr Ala Thr Asp Glu Phe Arg Met Tyr Ser Phe Lys Val Arg Pro Cys
            260                 265                 270

Ser Arg Ala Tyr Ser His Asp Trp Thr Glu Cys Pro Phe Val His Pro
        275                 280                 285

Gly Glu Asn Ala Arg Arg Arg Asp Pro Arg Lys Phe His Tyr Ser Cys
    290                 295                 300

Val Pro Cys Pro Asp Phe Arg Lys Gly Ala Cys Arg Arg Gly Asp Met
305                 310                 315                 320

Cys Glu Tyr Ala His Gly Val Phe Glu Cys Trp Leu His Pro Ala Gln
                325                 330                 335

Tyr Arg Thr Arg Leu Cys Lys Asp Gly Thr Gly Cys Ala Arg Arg Val
            340                 345                 350

Cys Phe Phe Ala His Thr Pro Glu Glu Leu Arg Pro Leu Tyr Ala Ser
        355                 360                 365

Thr Gly Ser Ala Val Pro Ser Pro Arg Ser Asn Ala Asp Tyr Ala Ala
    370                 375                 380

Ala Leu Ser Leu Leu Pro Gly Ser Pro Ser Gly Val Ser Val Met Ser
385                 390                 395                 400

Pro Leu Ser Pro Ser Ala Ala Gly Asn Gly Met Ser His Ser Asn Met
                405                 410                 415
```

Ala Trp Pro Gln Pro Asn Val Pro Ala Leu His Leu Pro Gly Ser Asn
420 425 430

Leu Gln Ser Ser Arg Leu Arg Ser Ser Leu Asn Ala Arg Asp Ile Pro
435 440 445

Thr Asp Glu Phe Asn Met Leu Ala Asp Tyr Glu Gln Gln Gln Leu Leu
450 455 460

Asn Glu Tyr Ser Asn Ala Leu Ser Arg Ser Gly Arg Met Lys Ser Met
465 470 475 480

Pro Pro Ser Asn Leu Glu Asp Leu Phe Ser Ala Glu Gly Ser Ser Ser
485 490 495

Pro Arg Phe Thr Asp Ser Ala Leu Ala Ser Ala Val Phe Ser Pro Thr
500 505 510

His Lys Ser Ala Val Phe Asn Gln Phe Gln Gln Gln Gln Gln Gln Gln
515 520 525

Gln Gln Gln Ser Met Leu Ser Pro Ile Asn Thr Ser Phe Ser Ser Pro
530 535 540

Lys Ser Val Asp His Ser Leu Phe Ser Gly Gly Gly Arg Met Ser Pro
545 550 555 560

Arg Asn Val Val Glu Pro Ile Ser Pro Met Ser Ala Arg Val Ser Met
565 570 575

Leu Ala Gln Cys Val Lys Gln Gln Gln Gln Gln Gln Gln Gln Gln Gln
580 585 590

Gln Gln Gln His Gln Phe Arg Ser Leu Ser Ser Arg Glu Leu Arg Thr
595 600 605

Asn Ser Ser Pro Ile Val Gly Ser Pro Val Asn Asn Asn Thr Trp Ser
610 615 620

Ser Lys Trp Gly Ser Ser Asn Gly Gln Pro Asp Trp Gly Met Ser Ser
625 630 635 640

Glu Ala Leu Gly Lys Leu Arg Ser Ser Ser Ser Phe Asp Gly Asp Glu
645 650 655

Pro Asp Val Ser Trp Val Gln Ser Leu Val Lys Glu Thr Pro Ala Glu
660 665 670

Ala Lys Glu Lys Ala Ala Thr Ser Ser Ser Gly Glu His Val Met Lys
675 680 685

Gln Pro Asn Pro Val Glu Pro Val Met Asp His Ala Gly Leu Glu Ala
690 695 700

Trp Ile Glu Gln Met Gln Leu Asp Gln Leu Val Ala Gln Gln Asn
705 710 715

<210> 49
<211> 686
<212> PRT
<213> Arabidopsis thaliana

<400> 49

Met Asn His Ile Thr Val Glu Thr Glu Asp Thr Phe Ala Ser Leu Leu
1 5 10 15

Glu Leu Ala Ala Asn Asn Asp Val Glu Gly Val Arg Leu Ser Ile Glu
20 25 30

Arg Asp Pro Ser Cys Val Asp Glu Ala Gly Leu Trp Tyr Gly Arg Gln
35 40 45

Lys Gly Ser Lys Ala Met Val Asn Asp Tyr Arg Thr Pro Leu Met Val
50 55 60

Ala Ala Thr Tyr Gly Ser Ile Asp Val Ile Lys Leu Ile Val Ser Leu
65 70 75 80

Thr Asp Ala Asp Val Asn Arg Ala Cys Gly Asn Asp Gln Thr Thr Ala
85 90 95

Leu His Cys Ala Ala Ser Gly Gly Ala Val Asn Ala Ile Gln Val Val
100 105 110

Lys Leu Leu Leu Ala Ala Gly Ala Asp Leu Asn Leu Leu Asp Ala Glu
115 120 125

Gly Gln Arg Ala Gly Asp Val Ile Val Val Pro Pro Lys Leu Glu Gly
130 135 140

Val Lys Leu Met Leu Gln Glu Leu Leu Ser Ala Asp Gly Ser Ser Thr
145 150 155 160

Ala Glu Arg Asn Leu Arg Val Val Thr Asn Val Pro Asn Arg Ser Ser
165 170 175

Ser Pro Cys His Ser Pro Thr Gly Glu Asn Gly Gly Ser Gly Ser Gly
180 185 190

```
Ser Pro Leu Gly Ser Pro Phe Lys Leu Lys Ser Thr Glu Phe Lys Lys
        195                 200                 205

Glu Tyr Pro Val Asp Pro Ser Leu Pro Asp Ile Lys Asn Ser Ile Tyr
    210                 215                 220

Ala Thr Asp Glu Phe Arg Met Tyr Ser Phe Lys Val Arg Pro Cys Ser
225                 230                 235                 240

Arg Ala Tyr Ser His Asp Trp Thr Glu Cys Pro Phe Val His Pro Gly
                245                 250                 255

Glu Asn Ala Arg Arg Arg Asp Pro Arg Lys Phe His Tyr Ser Cys Val
            260                 265                 270

Pro Cys Pro Asp Phe Arg Lys Gly Ala Cys Arg Arg Gly Asp Met Cys
        275                 280                 285

Glu Tyr Ala His Gly Val Phe Glu Cys Trp Leu His Pro Ala Gln Tyr
    290                 295                 300

Arg Thr Arg Leu Cys Lys Asp Gly Thr Gly Cys Ala Arg Arg Val Cys
305                 310                 315                 320

Phe Phe Ala His Thr Pro Glu Glu Leu Arg Pro Leu Tyr Ala Ser Thr
                325                 330                 335

Gly Ser Ala Val Pro Ser Pro Arg Ser Asn Ala Asp Tyr Ala Ala Ala
            340                 345                 350

Leu Ser Leu Leu Pro Gly Ser Pro Ser Gly Val Ser Val Met Ser Pro
        355                 360                 365

Leu Ser Pro Ser Ala Ala Gly Asn Gly Met Ser His Ser Asn Met Ala
    370                 375                 380

Trp Pro Gln Pro Asn Val Pro Ala Leu His Leu Pro Gly Ser Asn Leu
385                 390                 395                 400

Gln Ser Ser Arg Leu Arg Ser Ser Leu Asn Ala Arg Asp Ile Pro Thr
                405                 410                 415

Asp Glu Phe Asn Met Leu Ala Asp Tyr Glu Gln Gln Gln Leu Leu Asn
            420                 425                 430

Glu Tyr Ser Asn Ala Leu Ser Arg Ser Gly Arg Met Lys Ser Met Pro
        435                 440                 445
```

Pro Ser Asn Leu Glu Asp Leu Phe Ser Ala Glu Gly Ser Ser Ser Pro
    450                 455                 460

Arg Phe Thr Asp Ser Ala Leu Ala Ser Ala Val Phe Ser Pro Thr His
465                 470                 475                 480

Lys Ser Ala Val Phe Asn Gln Phe Gln Gln Gln Gln Gln Gln Gln Gln
                485                 490                 495

Gln Gln Ser Met Leu Ser Pro Ile Asn Thr Ser Phe Ser Ser Pro Lys
            500                 505                 510

Ser Val Asp His Ser Leu Phe Ser Gly Gly Gly Arg Met Ser Pro Arg
        515                 520                 525

Asn Val Val Glu Pro Ile Ser Pro Met Ser Ala Arg Val Ser Met Leu
    530                 535                 540

Ala Gln Cys Val Lys Gln Gln Gln Gln Gln Gln Gln Gln Gln Gln Gln
545                 550                 555                 560

Gln Gln His Gln Phe Arg Ser Leu Ser Ser Arg Glu Leu Arg Thr Asn
                565                 570                 575

Ser Ser Pro Ile Val Gly Ser Pro Val Asn Asn Asn Thr Trp Ser Ser
            580                 585                 590

Lys Trp Gly Ser Ser Asn Gly Gln Pro Asp Trp Gly Met Ser Ser Glu
        595                 600                 605

Ala Leu Gly Lys Leu Arg Ser Ser Ser Ser Phe Asp Gly Asp Glu Pro
    610                 615                 620

Asp Val Ser Trp Val Gln Ser Leu Val Lys Glu Thr Pro Ala Glu Ala
625                 630                 635                 640

Lys Glu Lys Ala Ala Thr Ser Ser Ser Gly Glu His Val Met Lys Gln
                645                 650                 655

Pro Asn Pro Val Glu Pro Val Met Asp His Ala Gly Leu Glu Ala Trp
            660                 665                 670

Ile Glu Gln Met Gln Leu Asp Gln Leu Val Ala Gln Gln Asn
        675                 680                 685

<210> 50
<211> 633
<212> PRT
<213> Arabidopsis thaliana

<400> 50

Met Val Asn Asp Tyr Arg Thr Pro Leu Met Val Ala Ala Thr Tyr Gly
1 5 10 15

Ser Ile Asp Val Ile Lys Leu Ile Val Ser Leu Thr Asp Ala Asp Val
20 25 30

Asn Arg Ala Cys Gly Asn Asp Gln Thr Thr Ala Leu His Cys Ala Ala
35 40 45

Ser Gly Gly Ala Val Asn Ala Ile Gln Val Val Lys Leu Leu Leu Ala
50 55 60

Ala Gly Ala Asp Leu Asn Leu Leu Asp Ala Glu Gly Gln Arg Ala Gly
65 70 75 80

Asp Val Ile Val Val Pro Pro Lys Leu Glu Gly Val Lys Leu Met Leu
85 90 95

Gln Glu Leu Leu Ser Ala Asp Gly Ser Ser Thr Ala Glu Arg Asn Leu
100 105 110

Arg Val Val Thr Asn Val Pro Asn Arg Ser Ser Ser Pro Cys His Ser
115 120 125

Pro Thr Gly Glu Asn Gly Gly Ser Gly Ser Gly Ser Pro Leu Gly Ser
130 135 140

Pro Phe Lys Leu Lys Ser Thr Glu Phe Lys Lys Glu Tyr Pro Val Asp
145 150 155 160

Pro Ser Leu Pro Asp Ile Lys Asn Ser Ile Tyr Ala Thr Asp Glu Phe
165 170 175

Arg Met Tyr Ser Phe Lys Val Arg Pro Cys Ser Arg Ala Tyr Ser His
180 185 190

Asp Trp Thr Glu Cys Pro Phe Val His Pro Gly Glu Asn Ala Arg Arg
195 200 205

Arg Asp Pro Arg Lys Phe His Tyr Ser Cys Val Pro Cys Pro Asp Phe
210 215 220

Arg Lys Gly Ala Cys Arg Arg Gly Asp Met Cys Glu Tyr Ala His Gly
225 230 235 240

Val Phe Glu Cys Trp Leu His Pro Ala Gln Tyr Arg Thr Arg Leu Cys
245 250 255

```
Lys Asp Gly Thr Gly Cys Ala Arg Arg Val Cys Phe Phe Ala His Thr
            260                 265                 270

Pro Glu Glu Leu Arg Pro Leu Tyr Ala Ser Thr Gly Ser Ala Val Pro
        275                 280                 285

Ser Pro Arg Ser Asn Ala Asp Tyr Ala Ala Ala Leu Ser Leu Leu Pro
    290                 295                 300

Gly Ser Pro Ser Gly Val Ser Val Met Ser Pro Leu Ser Pro Ser Ala
305                 310                 315                 320

Ala Gly Asn Gly Met Ser His Ser Asn Met Ala Trp Pro Gln Pro Asn
                325                 330                 335

Val Pro Ala Leu His Leu Pro Gly Ser Asn Leu Gln Ser Ser Arg Leu
            340                 345                 350

Arg Ser Ser Leu Asn Ala Arg Asp Ile Pro Thr Asp Glu Phe Asn Met
        355                 360                 365

Leu Ala Asp Tyr Glu Gln Gln Gln Leu Leu Asn Glu Tyr Ser Asn Ala
    370                 375                 380

Leu Ser Arg Ser Gly Arg Met Lys Ser Met Pro Pro Ser Asn Leu Glu
385                 390                 395                 400

Asp Leu Phe Ser Ala Glu Gly Ser Ser Ser Pro Arg Phe Thr Asp Ser
                405                 410                 415

Ala Leu Ala Ser Ala Val Phe Ser Pro Thr His Lys Ser Ala Val Phe
            420                 425                 430

Asn Gln Phe Gln Gln Gln Gln Gln Gln Gln Gln Gln Gln Ser Met Leu
        435                 440                 445

Ser Pro Ile Asn Thr Ser Phe Ser Ser Pro Lys Ser Val Asp His Ser
    450                 455                 460

Leu Phe Ser Gly Gly Gly Arg Met Ser Pro Arg Asn Val Val Glu Pro
465                 470                 475                 480

Ile Ser Pro Met Ser Ala Arg Val Ser Met Leu Ala Gln Cys Val Lys
                485                 490                 495

Gln Gln Gln Gln Gln Gln Gln Gln Gln Gln Gln Gln Gln His Gln Phe
            500                 505                 510
```

Arg Ser Leu Ser Ser Arg Glu Leu Arg Thr Asn Ser Ser Pro Ile Val
515 520 525

Gly Ser Pro Val Asn Asn Asn Thr Trp Ser Ser Lys Trp Gly Ser Ser
530 535 540

Asn Gly Gln Pro Asp Trp Gly Met Ser Ser Glu Ala Leu Gly Lys Leu
545 550 555 560

Arg Ser Ser Ser Ser Phe Asp Gly Asp Glu Pro Asp Val Ser Trp Val
565 570 575

Gln Ser Leu Val Lys Glu Thr Pro Ala Glu Ala Lys Glu Lys Ala Ala
580 585 590

Thr Ser Ser Ser Gly Glu His Val Met Lys Gln Pro Asn Pro Val Glu
595 600 605

Pro Val Met Asp His Ala Gly Leu Glu Ala Trp Ile Glu Gln Met Gln
610 615 620

Leu Asp Gln Leu Val Ala Gln Gln Asn
625 630

<210> 51
<211> 678
<212> PRT
<213> Arabidopsis thaliana

<400> 51

Met Asn Asp Ala Ala Glu Trp Glu His Ser Phe Ser Ala Leu Leu Glu
1 5 10 15

Phe Ala Ala Asp Asn Asp Val Glu Gly Phe Arg Arg Gln Leu Ser Asp
20 25 30

Val Ser Cys Ile Asn Gln Met Gly Leu Trp Tyr Arg Arg Gln Arg Phe
35 40 45

Val Arg Arg Met Val Leu Glu Gln Arg Thr Pro Leu Met Val Ala Ser
50 55 60

Leu Tyr Gly Ser Leu Asp Val Val Lys Phe Ile Leu Ser Phe Pro Glu
65 70 75 80

Ala Glu Leu Asn Leu Ser Cys Gly Pro Asp Lys Ser Thr Ala Leu His
85 90 95

Cys Ala Ala Ser Gly Ala Ser Val Asn Ser Leu Asp Val Val Lys Leu

```
            100                 105                 110

Leu Leu Ser Val Gly Ala Asp Pro Asn Ile Pro Asp Ala His Gly Asn
        115                 120                 125

Arg Pro Val Asp Val Leu Val Val Ser Pro His Ala Pro Gly Leu Arg
    130                 135                 140

Thr Ile Leu Glu Glu Ile Leu Lys Lys Asp Glu Ile Ile Ser Glu Asp
145                 150                 155                 160

Leu His Ala Ser Ser Ser Ser Leu Gly Ser Ser Phe Arg Ser Leu Ser
                165                 170                 175

Ser Ser Pro Asp Asn Gly Ser Ser Leu Leu Ser Leu Asp Ser Val Ser
            180                 185                 190

Ser Pro Thr Lys Pro His Gly Thr Asp Val Thr Phe Ala Ser Glu Lys
        195                 200                 205

Lys Glu Tyr Pro Ile Asp Pro Ser Leu Pro Asp Ile Lys Ser Gly Ile
    210                 215                 220

Tyr Ser Thr Asp Glu Phe Arg Met Phe Ser Phe Lys Ile Arg Pro Cys
225                 230                 235                 240

Ser Arg Ala Tyr Ser His Asp Trp Thr Glu Cys Pro Phe Ala His Pro
                245                 250                 255

Gly Glu Asn Ala Arg Arg Arg Asp Pro Arg Lys Phe His Tyr Thr Cys
            260                 265                 270

Val Pro Cys Pro Asp Phe Lys Lys Gly Ser Cys Lys Gln Gly Asp Met
        275                 280                 285

Cys Glu Tyr Ala His Gly Val Phe Glu Cys Trp Leu His Pro Ala Gln
    290                 295                 300

Tyr Arg Thr Arg Leu Cys Lys Asp Gly Met Gly Cys Asn Arg Arg Val
305                 310                 315                 320

Cys Phe Phe Ala His Ala Asn Glu Glu Leu Arg Pro Leu Tyr Pro Ser
                325                 330                 335

Thr Gly Ser Gly Leu Pro Ser Pro Arg Ala Ser Ser Ala Val Ser Ala
            340                 345                 350

Ser Thr Met Asp Met Ala Ser Val Leu Asn Met Leu Pro Gly Ser Pro
        355                 360                 365
```

```
Ser Ala Ala Gln His Ser Phe Thr Pro Pro Ile Ser Pro Ser Gly Asn
    370                 375                 380

Gly Ser Met Pro His Ser Ser Met Gly Trp Pro Gln Gln Asn Ile Pro
385                 390                 395                 400

Ala Leu Asn Leu Pro Gly Ser Asn Ile Gln Leu Ser Arg Leu Arg Ser
                405                 410                 415

Ser Leu Asn Ala Arg Asp Ile Pro Ser Glu Gln Leu Ser Met Leu His
            420                 425                 430

Glu Phe Glu Met Gln Arg Gln Leu Ala Gly Asp Met His Ser Pro Arg
        435                 440                 445

Phe Met Asn His Ser Ala Arg Pro Lys Thr Leu Asn Pro Ser Asn Leu
    450                 455                 460

Glu Glu Leu Phe Ser Ala Glu Val Ala Ser Pro Arg Phe Ser Asp Gln
465                 470                 475                 480

Leu Ala Val Ser Ser Val Leu Ser Pro Ser His Lys Ser Ala Leu Leu
                485                 490                 495

Asn Gln Leu Gln Asn Asn Lys Gln Ser Met Leu Ser Pro Ile Lys Thr
            500                 505                 510

Asn Leu Met Ser Ser Pro Lys Asn Val Glu Gln His Ser Leu Leu Gln
        515                 520                 525

Gln Ala Ser Ser Pro Arg Gly Gly Glu Pro Ile Ser Pro Met Asn Ala
    530                 535                 540

Arg Met Lys Gln Gln Leu His Ser Arg Ser Leu Ser Ser Arg Asp Phe
545                 550                 555                 560

Gly Ser Ser Leu Pro Arg Asp Leu Met Pro Thr Asp Ser Gly Ser Pro
                565                 570                 575

Leu Ser Pro Trp Ser Ser Trp Asp Gln Thr His Gly Ser Lys Val Asp
            580                 585                 590

Trp Ser Val Gln Ser Asp Glu Leu Gly Arg Leu Arg Lys Ser His Ser
        595                 600                 605

Leu Ala Asn Asn Pro Asn Arg Glu Ala Asp Val Ser Trp Ala Gln Gln
    610                 615                 620
```

Met Leu Lys Asp Ser Ser Ser Pro Arg Asn Gly Asn Arg Val Val Asn
625 630 635 640

Met Asn Gly Ala Arg Pro Leu Thr Gln Gly Gly Ser Ser Val Asn Pro
645 650 655

His Asn Ser Asp Thr Arg Glu Ser Asp Ile Leu Asp Ala Trp Leu Glu
660 665 670

Gln Leu His Leu Asp Arg
675

<210> 52
<211> 640
<212> PRT
<213> Arabidopsis thaliana

<400> 52

Met Gly Leu Trp Tyr Arg Arg Gln Arg Phe Val Arg Arg Met Val Leu
1 5 10 15

Glu Gln Arg Thr Pro Leu Met Val Ala Ser Leu Tyr Gly Ser Leu Asp
20 25 30

Val Val Lys Phe Ile Leu Ser Phe Pro Glu Ala Glu Leu Asn Leu Ser
35 40 45

Cys Gly Pro Asp Lys Ser Thr Ala Leu His Cys Ala Ala Ser Gly Ala
50 55 60

Ser Val Asn Ser Leu Asp Val Val Lys Leu Leu Leu Ser Val Gly Ala
65 70 75 80

Asp Pro Asn Ile Pro Asp Ala His Gly Asn Arg Pro Val Asp Val Leu
85 90 95

Val Val Ser Pro His Ala Pro Gly Leu Arg Thr Ile Leu Glu Glu Ile
100 105 110

Leu Lys Lys Asp Glu Ile Ile Ser Glu Asp Leu His Ala Ser Ser Ser
115 120 125

Ser Leu Gly Ser Ser Phe Arg Ser Leu Ser Ser Ser Pro Asp Asn Gly
130 135 140

Ser Ser Leu Leu Ser Leu Asp Ser Val Ser Ser Pro Thr Lys Pro His
145 150 155 160

Gly Thr Asp Val Thr Phe Ala Ser Glu Lys Lys Glu Tyr Pro Ile Asp

```
            165                 170                 175

Pro Ser Leu Pro Asp Ile Lys Ser Gly Ile Tyr Ser Thr Asp Glu Phe
        180                 185                 190

Arg Met Phe Ser Phe Lys Ile Arg Pro Cys Ser Arg Ala Tyr Ser His
    195                 200                 205

Asp Trp Thr Glu Cys Pro Phe Ala His Pro Gly Glu Asn Ala Arg Arg
210                 215                 220

Arg Asp Pro Arg Lys Phe His Tyr Thr Cys Val Pro Cys Pro Asp Phe
225                 230                 235                 240

Lys Lys Gly Ser Cys Lys Gln Gly Asp Met Cys Glu Tyr Ala His Gly
            245                 250                 255

Val Phe Glu Cys Trp Leu His Pro Ala Gln Tyr Arg Thr Arg Leu Cys
        260                 265                 270

Lys Asp Gly Met Gly Cys Asn Arg Arg Val Cys Phe Phe Ala His Ala
    275                 280                 285

Asn Glu Glu Leu Arg Pro Leu Tyr Pro Ser Thr Gly Ser Gly Leu Pro
290                 295                 300

Ser Pro Arg Ala Ser Ser Ala Val Ser Ala Ser Thr Met Asp Met Ala
305                 310                 315                 320

Ser Val Leu Asn Met Leu Pro Gly Ser Pro Ser Ala Ala Gln His Ser
            325                 330                 335

Phe Thr Pro Pro Ile Ser Pro Ser Gly Asn Gly Ser Met Pro His Ser
        340                 345                 350

Ser Met Gly Trp Pro Gln Gln Asn Ile Pro Ala Leu Asn Leu Pro Gly
    355                 360                 365

Ser Asn Ile Gln Leu Ser Arg Leu Arg Ser Ser Leu Asn Ala Arg Asp
370                 375                 380

Ile Pro Ser Glu Gln Leu Ser Met Leu His Glu Phe Glu Met Gln Arg
385                 390                 395                 400

Gln Leu Ala Gly Asp Met His Ser Pro Arg Phe Met Asn His Ser Ala
            405                 410                 415

Arg Pro Lys Thr Leu Asn Pro Ser Asn Leu Glu Glu Leu Phe Ser Ala
        420                 425                 430
```

Glu Val Ala Ser Pro Arg Phe Ser Asp Gln Leu Ala Val Ser Ser Val
435 440 445

Leu Ser Pro Ser His Lys Ser Ala Leu Leu Asn Gln Leu Gln Asn Asn
450 455 460

Lys Gln Ser Met Leu Ser Pro Ile Lys Thr Asn Leu Met Ser Ser Pro
465 470 475 480

Lys Asn Val Glu Gln His Ser Leu Leu Gln Gln Ala Ser Ser Pro Arg
485 490 495

Gly Gly Glu Pro Ile Ser Pro Met Asn Ala Arg Met Lys Gln Gln Leu
500 505 510

His Ser Arg Ser Leu Ser Ser Arg Asp Phe Gly Ser Ser Leu Pro Arg
515 520 525

Asp Leu Met Pro Thr Asp Ser Gly Ser Pro Leu Ser Pro Trp Ser Ser
530 535 540

Trp Asp Gln Thr His Gly Ser Lys Val Asp Trp Ser Val Gln Ser Asp
545 550 555 560

Glu Leu Gly Arg Leu Arg Lys Ser His Ser Leu Ala Asn Asn Pro Asn
565 570 575

Arg Glu Ala Asp Val Ser Trp Ala Gln Gln Met Leu Lys Asp Ser Ser
580 585 590

Ser Pro Arg Asn Gly Asn Arg Val Val Asn Met Asn Gly Ala Arg Pro
595 600 605

Leu Thr Gln Gly Gly Ser Ser Val Asn Pro His Asn Ser Asp Thr Arg
610 615 620

Glu Ser Asp Ile Leu Asp Ala Trp Leu Glu Gln Leu His Leu Asp Arg
625 630 635 640

<210> 53
<211> 2158
<212> DNA
<213> Oryza sativa

<400> 53
tgctccttct tcattgcaaa gaaagcacca ccttttaaag aatcctcctc acactccatt 60

cttcttaaaa aacccaccac aacacaattc ccacttgttt cttcatcatc acctacttca 120

atcaaaaaac attccaactt tcttctcaat ttcattccag gatagtatta tgtgcagtgg 180

accaaagagc aatctctgct cttcaagaac cttaacagaa atcgaatcaa ggcaaaagga 240
agaagaaaca atgcttctcc tcgaattcgc tgcttgtgat gatcttgact cgttcaagag 300
agaggttgaa gagaaagggc ttgatttgga tgagtcaggg ttatggtatt gcagacgtgt 360
cggttctaag aagatgggtc ttgaagaaag aacacccttta atggttgcag ctatgtatgg 420
aagcataaag gttttgactt tcatcgtttc cactggaaaa tctgatgtga acagagcttg 480
tggtgaagag agagttactc cgcttcactg tgctgttgct ggctgttctg tgaatatgat 540
tgaagtcatc aatgtcttgc ttgatgcttc tgctttggtt aactctgttg atgctaatgg 600
gaatcaacct ttggatgtgt ttgttcgagt ttcgaggttt gtggctagtc cgaggaggaa 660
agcggttgag ttgttgctga gaggaggagg tgttggagga ttgatcgatg aggcggttga 720
agaagagatc aagattgtct ctaagtatcc agctgatgct tctttaccgg atataaacga 780
aggggtttat ggaagtgatg agtttaggat gtatagcttt aaggttaagc catgttctag 840
ggcttattct catgattgga ccgagtgtgc ttttgttcat ccgggagaaa atgcgaggag 900
gagagatccg aggaagtatc cttacacttg tgtcccctgt cccgagttcc gtaaaggatc 960
atgcccgaaa ggagattctt gcgagtatgc tcacggggtt ttcgagtcgt ggcttcaccc 1020
cgcgcagtat aaaacccggc tttgtaaaga tgaaacgggt tgtgcaagga aagtttgttt 1080
ctttgctcat aaacgcgaag agatgagacc tgttaatgct tcaactggct ctgccgtggc 1140
tcagtctccg tttagcagct tggagatgat gccagggttg tctcctcttg cttattcttc 1200
aggagtttcg actcctccgg tttctccaat ggctaatggt gttccttcct ctccaagaaa 1260
cggcggatca tggcagaaca gagtcaatac ccttactcca ccggctttgc agctcaatgg 1320
tggaagcaga ttgaagtcca cactgagcgc tagagatatc gatatggaga tggagatgga 1380
attgagactc cgcggttttg gcaacaatgt ggaagagacg ttcgggtctt atgtttcctc 1440
tccaagtagg aattctcaaa tgggtcaaaa catgaaccaa cattatccat cttccccggt 1500
gagacaaccg ccatctcaac acgggttcga atcttcagca gctgcagcgg ttgcagtgat 1560
gaaagcgaga tcaaccgcct ttgcgaaacg tagcttgagc ttcaaaccag ctactcaagc 1620
agcaccacag tcgaatctct cggattgggg atctccaaac gggaagctgg aatggggaat 1680
gaaaggagaa gagctgaata agatgagaag aagtgtttcc tttggaatcc atggaaacaa 1740
caacaataac gcagctagag actacaggga cgagccagat gtgtcatggg ttaactcttt 1800
agttaaagac agtactgtgg tgtctgagag aagctttgga atgaatgaga gggttcggat 1860
aatgtcgtgg gctgagcaaa tgtacagaga gaaggagcag actgtggtgt aaacacacac 1920
aaagatggtt tcttatatat attgcttttg ggccatctct gcaaatttga ttctttaatt 1980
tttgtgactt tctttagttg ttactgttat tagtagtata tggtttgttg tcactacgag 2040
tctacgtgat gaaaagatag aagttaattg cattagtttc tatattcgtt tctcatcctc 2100

ttgtaattta tcaaaccatg aaatggctaa gcaatccaaa ccgaaaaaaa aaaaaaa     2158

<210>  54
<211>  2193
<212>  DNA
<213>  Oryza sativa

<400>  54
aatccgaaaa gtttctgcac cgttttcacc ccctaactaa caatataggg aacgtgtgct     60

aaatataaaa tgagacctta tatatgtagc gctgataact agaactatgc aagaaaaact    120

catccaccta ctttagtggc aatcgggcta aataaaaaag agtcgctaca ctagtttcgt    180

tttccttagt aattaagtgg gaaaatgaaa tcattattgc ttagaatata cgttcacatc    240

tctgtcatga agttaaatta ttcgaggtag ccataattgt catcaaactc ttcttgaata    300

aaaaaatctt tctagctgaa ctcaatgggt aaagagagag atttttttta aaaaaataga    360

atgaagatat tctgaacgta ttggcaaaga tttaaacata taattatata attttatagt    420

ttgtgcattc gtcatatcgc acatcattaa ggacatgtct tactccatcc caatttttat    480

ttagtaatta aagacaattg acttattttt attatttatc ttttttcgat tagatgcaag    540

gtacttacgc acacactttg tgctcatgtg catgtgtgag tgcacctcct caatacacgt    600

tcaactagca acacatctct aatatcactc gcctatttaa tacatttagg tagcaatatc    660

tgaattcaag cactccacca tcaccagacc acttttaata atatctaaaa tacaaaaaat    720

aattttacag aatagcatga aaagtatgaa acgaactatt taggtttttc acatacaaaa    780

aaaaaaagaa ttttgctcgt gcgcgagcgc caatctccca tattgggcac acaggcaaca    840

acagagtggc tgcccacaga acaacccaca aaaaacgatg atctaacgga ggacagcaag    900

tccgcaacaa ccttttaaca gcaggctttg cggccaggag agaggaggag aggcaaagaa    960

aaccaagcat cctcctcctc ccatctataa attcctcccc ccttttcccc tctctatata   1020

ggaggcatcc aagccaagaa gagggagagc accaaggaca cgcgactagc agaagccgag   1080

cgaccgcctt cttcgatcca tatcttccgg tcgagttctt ggtcgatctc ttccctcctc   1140

cacctcctcc tcacagggta tgtgcccttc ggttgttctt ggatttattg ttctaggttg   1200

tgtagtacgg gcgttgatgt taggaaaggg gatctgtatc tgtgatgatt cctgttcttg   1260

gatttgggat agaggggttc ttgatgttgc atgttatcgg ttcggtttga ttagtagtat   1320

ggttttcaat cgtctggaga gctctatgga aatgaaatgg tttagggtac ggaatcttgc   1380

gattttgtga gtaccttttg tttgaggtaa aatcagagca ccggtgattt tgcttggtgt   1440

aataaaagta cggttgtttg gtcctcgatt ctggtagtga tgcttctcga tttgacgaag   1500

ctatcctttg tttattccct attgaacaaa aataatccaa ctttgaagac ggtcccgttg   1560

atgagattga atgattgatt cttaagcctg tccaaaattt cgcagctggc ttgtttagat   1620

acagtagtcc ccatcacgaa attcatggaa acagttataa tcctcaggaa caggggattc   1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcacccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 55
<211> 1827
<212> DNA
<213> Oryza sativa

<400> 55
gcttgagtca tagggagaaa acaaatcgat catatttgac tcttttccct ccatctctct 60

taccggcaaa aaaagtagta ctggtttata tgtaaagtaa gattctttaa ttatgtgaga 120

tccggcttaa tgcttttctt ttgtcacata tactgcattg caacaattgc catatattca 180

cttctgccat cccattatat agcaactcaa gaatggattg atatatcccc tattactaat 240

ctagacatgt taaggctgag ttgggcagtc catcttccca acccaccacc ttcgtttttc 300

gcgcacatac ttttcaaact actaaatggt gtgtttttta aaaatatttt caatacaaaa 360

gttgctttaa aaaattatat tgatccattt ttttaaaaaa aatagctaat acttaattaa 420

tcacgtgtta aaagaccgct ccgttttgcg tgcaggaggg ataggttcac atcctgcatt 480

accgaacaca gcctaaatct tgttgtctag attcgtagta ctggatatat taaatcatgt 540

tctaagttac tatatactga gatgaataga ataagtaaaa ttagacccac cttaagtctt 600

gatgaagtta ctactagctg cgtttgggag gacttcccaa aaaaaaaagt attagccatt 660

agcacgtgat taattaagta ctagtttaaa aaacttaaaa aataaattaa tatgattctc 720

ttaagtaact ctcctataga aaacttttac aaaattacac cgtttaatag tttggaaaat 780

atgtcagtaa aaaataagag agtagaagtt atgaaagtta gaaaaagaat tgttttagta 840

gtatacagtt ataaactatt ccctctgttc taaaacataa gggattatgg atggattcga 900

catgtaccag taccatgaat cgaatccaga caagtttttt atgcatattt attctactat 960

aatatatcac atctgctcta aatatcttat atttcgaggt ggagactgtc gctatgtttt 1020

tctgcccgtt gctaagcaca cgccaccccc gatgcgggga cgcctctggc cttcttgcca 1080

cgataattga atggaacttc cacattcaga ttcgataggt gaccgtcgac tccaagtgct 1140

ttgcacaaaa caactccggc ctcccggcca ccagtcacac gactcacggc actaccaccc 1200

ctgactccct gaggcggacc tgccactgtt ctgcatgcga agctatctaa aattctgaag 1260

caaagaaagc acagcacatg ctccgggaca cgcgccaccc ggcggaaaag ggctcggtgt 1320

ggcgatctca cagccgcata tcgcatttca caagccgccc atctccaccg gcttcacgag 1380

gctcatcgcg gcacgaccgc gcacggaacg cacgcggccg acccgcgcgc ctcgatgcgc 1440

gagcccatcc gccgcgtcct ccctttgcct ttgccgctat cctctcggtc gtatcccgtt 1500

tctctgtctt ttgctccccg gcgcgcgcca gttcggagta ccagcgaaac ccggacacct 1560

ggtacacctc cgccggccac aacgcgtgtc cccctacgtg gccgcgcagc acatgcccat 1620

gcgcgacacg tgcacctcct catccaaact ctcaagtctc aacggtccta taaatgcacg 1680

gatagcctca agctgctcgt cacaaggcaa gaggcaagag gcaagagcat ccgtattaac 1740

cagccttttg agacttgaga gtgtgtgtga ctcgatccag cgtagtttca gttcgtgtgt 1800

tggtgagtga ttccagccaa gtttgcg 1827

<210> 56
<211> 2194
<212> DNA
<213> Oryza sativa

<400> 56
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 57
<211> 46
<212> PRT
<213> Artificial sequence

<220>
<223> SNH domain

<220>
<221> VARIANT
<222> (3)..(3)
<223> /replace = "Lys"

<220>
<221> UNSURE
<222> (4)..(4)
<223> Xaa can be any naturally occurring amino acid

<220>
<221> VARIANT
<222> (6)..(6)
<223> /replace = "Glu"

<220>
<221> VARIANT

<222> (7)..(7)
<223> /replace = "Asp"

<220>
<221> VARIANT
<222> (9)..(9)
<223> /replace = "Asn"

<220>
<221> UNSURE
<222> (10)..(10)
<223> Xaa can be any naturally occurring amino acid

<220>
<221> UNSURE
<222> (13)..(13)
<223> Xaa can be any naturally occurring amino acid

<220>
<221> VARIANT
<222> (14)..(14)
<223> /replace = "Ala" /replace = "Lys"

<220>
<221> VARIANT
<222> (16)..(16)
<223> /replace = "Val" /replace = "Met"

<220>
<221> VARIANT
<222> (17)..(17)
<223> /replace = "Asp" /replace = "Ser"

<220>
<221> VARIANT
<222> (18)..(18)
<223> /replace = "Asn"

<220>
<221> VARIANT
<222> (19)..(19)
<223> /replace = "Leu"

<220>
<221> UNSURE
<222> (21)..(21)
<223> Xaa can be any naturally occurring amino acid

<220>
<221> VARIANT
<222> (23)..(23)
<223> /replace = "Arg"

<220>
<221> UNSURE
<222> (24)..(25)
<223> Xaa can be any naturally occurring amino acid

<220>
<221> VARIANT
<222> (28)..(28)
<223> /replace = "Asp" /replace = "Ser"

<220>
<221> UNSURE

<222> (29)..(30)
<223> Xaa can be any naturally occurring amino acid

<220>
<221> VARIANT
<222> (32)..(32)
<223> /replace = "Ser" /replace = "Gln"

<220>
<221> UNSURE
<222> (33)..(33)
<223> Xaa can be any naturally occurring amino acid

<220>
<221> VARIANT
<222> (35)..(35)
<223> /replace = "His"

<220>
<221> UNSURE
<222> (36)..(36)
<223> Xaa can be any naturally occurring amino acid

<220>
<221> VARIANT
<222> (39)..(39)
<223> /replace = "Leu" /replace = "Val"

<220>
<221> VARIANT
<222> (43)..(43)
<223> /replace = "Thr"

<400> 57

```
Ile Gln Gln Xaa Leu Asp Glu Asn Lys Xaa Leu Ile Xaa Cys Ile Leu
1               5                   10                  15

Glu Ser Gln Asn Xaa Gly Lys Xaa Xaa Glu Cys Ala Xaa Xaa Gln Ala
            20                  25                  30

Xaa Leu Gln Xaa Asn Leu Met Tyr Leu Ala Ala Ile Ala Asp
        35                  40                  45
```

<210> 58
<211> 46
<212> PRT
<213> Arabidopsis thaliana

<400> 58

```
Ile Gln Gln Tyr Leu Asp Glu Asn Lys Ser Leu Ile Leu Lys Ile Val
1               5                   10                  15

Glu Ser Gln Asn Ser Gly Lys Leu Ser Glu Cys Ala Glu Asn Gln Ala
            20                  25                  30

Arg Leu Gln Arg Asn Leu Met Tyr Leu Ala Ala Ile Ala Asp
        35                  40                  45
```

<210> 59
<211> 633
<212> DNA
<213> Arabidopsis thaliana

<400> 59
atgcaacagc acctgatgca gatgcagccc atgatggctg gttactaccc cagcaatgtt 60

acctctgatc atatccaaca gtacttggac gaaaacaaat cgttgattct gaagattgtt 120

gagtctcaaa actctggaaa gcttagcgaa tgcgccgaga atcaagcaag gcttcaacgc 180

aacctaatgt acctagctgc aatagcagat tctcagcctc agccaccaag tgtgcatagc 240

cagtatggat ctgctggtgg tgggatgatt cagggagaag gagggtcaca ctatttgcag 300

cagcaacaag cgactcaaca gcaacagatg actcagcagt ctctaatggc ggctcgatct 360

tcaatgttgt atgctcagca acagcagcag cagcagcctt acgcgacgct tcagcatcag 420

caattgcacc atagccagct tggaatgagc tcgagcagcg gaggaggagg aagcagtggt 480

ctccatatcc ttcagggaga ggctggtggg tttcatgatt ttggccgtgg gaagccggaa 540

atgggaagtg gtggtggcgg tgaaggcaga ggaggaagtt caggggatgg tggagaaacc 600

ctttacttga aatcatcaga tgatgggaat tga 633

<210> 60
<211> 210
<212> PRT
<213> Arabidopsis thaliana

<400> 60

Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Met Ile Gln Gly Glu Gly Gly Ser
                85                  90                  95

His Tyr Leu Gln Gln Gln Gln Ala Thr Gln Gln Gln Gln Met Thr Gln
            100                 105                 110

Gln Ser Leu Met Ala Ala Arg Ser Ser Met Leu Tyr Ala Gln Gln Gln
115 120 125

Gln Gln Gln Gln Pro Tyr Ala Thr Leu Gln His Gln Gln Leu His His
130 135 140

Ser Gln Leu Gly Met Ser Ser Ser Ser Gly Gly Gly Gly Ser Ser Gly
145 150 155 160

Leu His Ile Leu Gln Gly Glu Ala Gly Gly Phe His Asp Phe Gly Arg
165 170 175

Gly Lys Pro Glu Met Gly Ser Gly Gly Gly Gly Glu Gly Arg Gly Gly
180 185 190

Ser Ser Gly Asp Gly Gly Glu Thr Leu Tyr Leu Lys Ser Ser Asp Asp
195 200 205

Gly Asn
210

<210> 61
<211> 588
<212> DNA
<213> Arabidopsis thaliana

<400> 61
atgcagcagc agcagtctcc gcaaatgttt ccgatggttc cgtcgattcc ccctgctaac 60

aacatcacta ccgaacagat ccaaaagtac cttgatgaga acaagaagct gattatggcc 120

atcatggaaa accagaatct cggtaaactt gctgagtgcg cccagtacca agctcttctc 180

cagaagaact tgatgtatct tgctgcaatt gctgatgctc aacccccacc acctacgcca 240

ggaccttcac catctacagc tgtcgctgcc cagatggcaa caccgcattc tgggatgcaa 300

ccacctagct acttcatgca acacccacaa gcatcccctg cagggatttt cgctccaagg 360

ggtcctttac agtttggtag cccactccag tttcaggatc cgcaacagca gcagcagata 420

catcagcaag ctatgcaagg acacatgggg attagaccaa tgggtatgac caacaacggg 480

atgcagcatg cgatgcaaca accagaaacc ggtcttggag gaaacgtggg gcttagagga 540

ggaaagcaag atggagcaga tggacaagga aaagatgatg gcaagtga 588

<210> 62
<211> 195
<212> PRT
<213> Arabidopsis thaliana

<400> 62

Met Gln Gln Gln Gln Ser Pro Gln Met Phe Pro Met Val Pro Ser Ile
1 5 10 15

```
Pro Pro Ala Asn Asn Ile Thr Thr Glu Gln Ile Gln Lys Tyr Leu Asp
            20                  25                  30

Glu Asn Lys Lys Leu Ile Met Ala Ile Met Glu Asn Gln Asn Leu Gly
        35                  40                  45

Lys Leu Ala Glu Cys Ala Gln Tyr Gln Ala Leu Leu Gln Lys Asn Leu
    50                  55                  60

Met Tyr Leu Ala Ala Ile Ala Asp Ala Gln Pro Pro Pro Pro Thr Pro
65                  70                  75                  80

Gly Pro Ser Pro Ser Thr Ala Val Ala Ala Gln Met Ala Thr Pro His
                85                  90                  95

Ser Gly Met Gln Pro Pro Ser Tyr Phe Met Gln His Pro Gln Ala Ser
            100                 105                 110

Pro Ala Gly Ile Phe Ala Pro Arg Gly Pro Leu Gln Phe Gly Ser Pro
        115                 120                 125

Leu Gln Phe Gln Asp Pro Gln Gln Gln Gln Gln Ile His Gln Gln Ala
    130                 135                 140

Met Gln Gly His Met Gly Ile Arg Pro Met Gly Met Thr Asn Asn Gly
145                 150                 155                 160

Met Gln His Ala Met Gln Gln Pro Glu Thr Gly Leu Gly Gly Asn Val
                165                 170                 175

Gly Leu Arg Gly Gly Lys Gln Asp Gly Ala Asp Gly Gln Gly Lys Asp
            180                 185                 190

Asp Gly Lys
        195
```

<210> 63
<211> 672
<212> DNA
<213> Arabidopsis thaliana

<400> 63

```
atgcagcaat ctccacagat gattccgatg gttcttcctt catttccgcc caccaataat      60

atcaccaccg aacagatcca aaagtatctt gatgagaaca agaagctgat aatggcgatc     120

ttggaaaatc agaacctcgg taaacttgca gaatgtgctc agtatcaagc tcttctccag     180

aagaatttga tgtatctcgc tgcaattgcg gatgctcaac ctcagccacc agcagctaca     240

ctaacatcag gagccatgac tccccaagca atggctccta atccgtcatc aatgcagcca     300
```

ccaccaagct acttcatgca gcaacatcaa gctgtgggaa tggctcaaca aatacctcct 360

gggattttcc ctcctagagg tccattgcaa tttggtagcc cgcatcagtt tctggatccg 420

cagcaacagt tacatcaaca agctatgcaa gggcacatgg ggattagacc aatgggtttg 480

aataataaca acggactgca acatcaaatg caccaccatg aaactgctct tgccgcaaac 540

aatgcgggtc ctaacgatgc tagtggagga ggtaaaccgg atgggaccaa tatgagccag 600

agtggagctg atgggcaagg tggctcagcc gctagacatg gcggtggtga tgcaaaaact 660

gaaggaaaat ga 672

<210> 64
<211> 223
<212> PRT
<213> Arabidopsis thaliana

<400> 64

Met Gln Gln Ser Pro Gln Met Ile Pro Met Val Leu Pro Ser Phe Pro
1 5 10 15

Pro Thr Asn Asn Ile Thr Thr Glu Gln Ile Gln Lys Tyr Leu Asp Glu
20 25 30

Asn Lys Lys Leu Ile Met Ala Ile Leu Glu Asn Gln Asn Leu Gly Lys
35 40 45

Leu Ala Glu Cys Ala Gln Tyr Gln Ala Leu Leu Gln Lys Asn Leu Met
50 55 60

Tyr Leu Ala Ala Ile Ala Asp Ala Gln Pro Gln Pro Pro Ala Ala Thr
65 70 75 80

Leu Thr Ser Gly Ala Met Thr Pro Gln Ala Met Ala Pro Asn Pro Ser
85 90 95

Ser Met Gln Pro Pro Pro Ser Tyr Phe Met Gln Gln His Gln Ala Val
100 105 110

Gly Met Ala Gln Gln Ile Pro Pro Gly Ile Phe Pro Pro Arg Gly Pro
115 120 125

Leu Gln Phe Gly Ser Pro His Gln Phe Leu Asp Pro Gln Gln Gln Leu
130 135 140

His Gln Gln Ala Met Gln Gly His Met Gly Ile Arg Pro Met Gly Leu
145 150 155 160

Asn Asn Asn Asn Gly Leu Gln His Gln Met His His His Glu Thr Ala
165 170 175

```
Leu Ala Ala Asn Asn Ala Gly Pro Asn Asp Ala Ser Gly Gly Gly Lys
            180                 185                 190

Pro Asp Gly Thr Asn Met Ser Gln Ser Gly Ala Asp Gly Gln Gly Gly
        195                 200                 205

Ser Ala Ala Arg His Gly Gly Gly Asp Ala Lys Thr Glu Gly Lys
    210                 215                 220
```

<210> 65
<211> 633
<212> DNA
<213> Aspergillus officinalis

<400> 65

```
atgcagcagc acctgatgca gatgcagccc atgatggcaa cctacggttc accgaatcag      60

gtcaccaccg atatcattca gcagtatctg gacgagaaca agcagttgat tctggctatt     120

cttgaaaacc aaaattcagg aaaagctgat gaatgtgctg agaatcaggc taagcttcag     180

aggaatctga tgtatcttgc agccattgcg gatagccagc cccaagttcc taccattgct     240

cagtatcctc ccaacgctgt tgctgctatg caatcgagtg ctcgctacat gcaacaacac     300

caagcagctc aacagatgac ccctcaatct ctcatggctg ctcgctcctc aatgctctac     360

tcacagtccc caatgtctgc actccagcag caacagcagc aagcagcaat gcatagccag     420

ctcgccatga gctccggagg caacaacagc agcaccggag gattcaccat tcttcatggt     480

gaagctagca taggaggcaa tggctcaatg aattctggtg gagtctttgg agattttgga     540

cggagcagcg gtgggaagca agagactggg agcgaagggc acgggacaga gactcctatg     600

tacctgaaag gctctgaaga agaaggaaac tga                                 633
```

<210> 66
<211> 210
<212> PRT
<213> Aspergillus officinalis

<400> 66

```
Met Gln Gln His Leu Met Gln Met Gln Pro Met Met Ala Thr Tyr Gly
1               5                   10                  15

Ser Pro Asn Gln Val Thr Thr Asp Ile Ile Gln Gln Tyr Leu Asp Glu
            20                  25                  30

Asn Lys Gln Leu Ile Leu Ala Ile Leu Glu Asn Gln Asn Ser Gly Lys
        35                  40                  45

Ala Asp Glu Cys Ala Glu Asn Gln Ala Lys Leu Gln Arg Asn Leu Met
    50                  55                  60
```

```
Tyr Leu Ala Ala Ile Ala Asp Ser Gln Pro Gln Val Pro Thr Ile Ala
65                  70                  75                  80

Gln Tyr Pro Pro Asn Ala Val Ala Ala Met Gln Ser Ser Ala Arg Tyr
                85                  90                  95

Met Gln Gln His Gln Ala Ala Gln Gln Met Thr Pro Gln Ser Leu Met
            100                 105                 110

Ala Ala Arg Ser Ser Met Leu Tyr Ser Gln Ser Pro Met Ser Ala Leu
        115                 120                 125

Gln Gln Gln Gln Gln Gln Ala Ala Met His Ser Gln Leu Ala Met Ser
    130                 135                 140

Ser Gly Gly Asn Asn Ser Ser Thr Gly Gly Phe Thr Ile Leu His Gly
145                 150                 155                 160

Glu Ala Ser Ile Gly Gly Asn Gly Ser Met Asn Ser Gly Gly Val Phe
                165                 170                 175

Gly Asp Phe Gly Arg Ser Ser Gly Gly Lys Gln Glu Thr Gly Ser Glu
            180                 185                 190

Gly His Gly Thr Glu Thr Pro Met Tyr Leu Lys Gly Ser Glu Glu Glu
        195                 200                 205

Gly Asn
    210

<210>  67
<211>  591
<212>  DNA
<213>  Brassica napus

<400>  67
atgcagccca tgatggctgg ttactacccc agcaatgtca cctctgatca tatccagcag      60

tacttggatg agaacaagtc tttgattctg aagatagttg agtctcaaaa ctcaggaaag     120

ctcagcgagt gtgccgagaa tcaggcaagg cttcaacgca acctcatgta cttggctgca     180

atagcagatt ctcagcctca acctccaagc gtgcatagcc agtatggatc tgctggtggt     240

gggttgattc agggagaagg agcgtcacac tatttgcagc agcaacaggc gactcaacag     300

cagcagatga ctcagcagtc tcttatggca gctcgttctt caatgatgta tcagcagcag     360

caacagcctt atgcaacgct tcagcatcag cagttgcacc atagccagct tgggatgagc     420

tctagcagcg gaggaggaag cagtggtctc catatccttc agggagaggc tggtgggttt     480

catgaatttg gccgtgggaa gccggagatg ggaagtggtg aaggcagggg tggaagctca     540

ggggatggtg gagaaacact ctacttgaag tcatcagatg atgggaactg a             591
```

<210> 68
<211> 203
<212> PRT
<213> Brassica napus

<400> 68

```
Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Leu Ile Gln Gly Glu Gly Ala Ser
                85                  90                  95

His Tyr Leu Gln Gln Gln Gln Ala Thr Gln Gln Gln Gln Met Thr Gln
            100                 105                 110

Gln Ser Leu Met Ala Ala Arg Ser Ser Met Met Tyr Gln Gln Gln Gln
        115                 120                 125

Gln Pro Tyr Ala Thr Leu Gln His Gln Gln Leu His His Ser Gln Leu
    130                 135                 140

Gly Met Ser Ser Ser Ser Gly Gly Gly Ser Ser Gly Leu His Ile Leu
145                 150                 155                 160

Gln Gly Glu Ala Gly Gly Phe His Glu Phe Gly Arg Gly Lys Pro Glu
                165                 170                 175

Met Gly Ser Gly Glu Gly Arg Gly Gly Ser Ser Gly Asp Gly Gly Glu
            180                 185                 190

Thr Leu Tyr Leu Lys Ser Ser Asp Asp Gly Asn
        195                 200
```

<210> 69
<211> 663
<212> DNA
<213> Citrus sinensis

<400> 69
atgcaacagc acctgatgca gatgcagccc atgatggcag cttattatcc caacaacgtc 60

actactgacc acattcaaca gtatctagat gagaacaaat cattgatttt gaagattgtt 120

gagagccaga attcagggaa actgagcgag tgtgcagaga accaggcaag attgcagcgg 180

aatctcatgt acctggctgc tattgctgat gctcaacccc aaccacctag cgttcatgcc 240

cagttctctt ctggtggcat tatgcagcca ggagctcact atatgcaaca ccagcaatct 300

cagccaatga caccacagtc acttatggct gcacgctcat ccatggtgta ctctcaacag 360

caattttcag tgcttcagca acagcaagcc ttgcatggtc agcttggcat gagctctggt 420

ggtagctcag gacttcacat gctgcaaagt gagggtagta ctgcaggagg tagtggttca 480

cttgggggtg gggattccc tgattttggc cgtggctcat ctggtgaagg cttgcactca 540

aggggaatgg ggagcaagca tgatataggc agttctggat ctgctgaagg acgaggaggg 600

agctcaggaa gccaagatgg aggcgaaact ctctacttga aaggggctga tgatggaaat 660

taa 663

<210> 70
<211> 219
<212> PRT
<213> Citrus sinensis

<400> 70

Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ala Gln Pro Gln Pro Pro Ser Val His Ala
65                  70                  75                  80

Gln Phe Ser Ser Gly Gly Ile Met Gln Pro Gly Ala His Tyr Met Gln
                85                  90                  95

His Gln Gln Ser Gln Pro Met Thr Pro Gln Ser Leu Met Ala Ala Arg
            100                 105                 110

Ser Ser Met Val Tyr Ser Gln Gln Gln Phe Ser Val Leu Gln Gln Gln
        115                 120                 125

Gln Ala Leu His Gly Gln Leu Gly Met Ser Ser Gly Gly Ser Ser Gly
130 135 140

Leu His Met Leu Gln Ser Glu Gly Ser Thr Ala Gly Gly Ser Gly Ser
145 150 155 160

Leu Gly Gly Gly Gly Phe Pro Asp Phe Gly Arg Gly Ser Ser Gly Glu
165 170 175

Gly Leu His Ser Arg Gly Met Gly Ser Lys His Asp Ile Gly Ser Ser
180 185 190

Gly Ser Ala Glu Gly Arg Gly Gly Ser Ser Gly Ser Gln Asp Gly Gly
195 200 205

Glu Thr Leu Tyr Leu Lys Gly Ala Asp Asp Gly
210 215

<210> 71
<211> 660
<212> DNA
<213> Gossypium arboreum

<220>
<221> misc_feature
<222> (309)..(309)
<223> n is a, c, g, or t

<400> 71
atgcagcagc acctgatgca gatgcagccc atgatggcag cttattatcc caacaacgtc 60

actactgatc atattcaaca gtatctcgat gagaacaagt cattgatctt aaagattgtt 120

gagagccaga attctgggaa attgagtgaa tgtgctgaga accaagcaag gctgcagcga 180

aacctcatgt acctggctgc cattgcggat tctcaacccc aaccacccac cgtgcatgca 240

cagtttccat ctggtggtat catgcagcaa ggagctgggc actacatgca gcaccaacaa 300

gctcaacana tgacacaaca gtcgcttatg gctgctcggt cctcaatgtt gtattctcag 360

caaccatttt ctgcactgca acaacaacaa caacaaggct ttgcacagtc agcttggcat 420

gagctctggc gggagcacag gcctttcata tgctgcaaac tgaatctagt actgcagggg 480

gcagtgagac accttgggcc cgagggttgt cctgatttgg acgggggtct tttggagagg 540

catccctggt ggcaggccaa tggccggggg aacaaccaaa aatccgggga ggccggctca 600

cctaagggcc gggaggagcc cttggggcag gggggggtga tgggggaac ctcttcttaa 660

<210> 72
<211> 219
<212> PRT
<213> Gossypium arboreum

<220>
<221> UNSURE
<222> (103)..(103)
<223> Xaa can be any naturally occurring amino acid

<400> 72

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Thr Val His Ala
65                  70                  75                  80

Gln Phe Pro Ser Gly Gly Ile Met Gln Gln Gly Ala Gly His Tyr Met
                85                  90                  95

Gln His Gln Gln Ala Gln Xaa Met Thr Gln Gln Ser Leu Met Ala Ala
            100                 105                 110

Arg Ser Ser Met Leu Tyr Ser Gln Gln Pro Phe Ser Ala Leu Gln Gln
        115                 120                 125

Gln Gln Gln Gln Gly Phe Ala Gln Ser Ala Trp His Glu Leu Trp Arg
    130                 135                 140

Glu His Arg Pro Phe Ile Cys Cys Lys Leu Asn Leu Val Leu Gln Gly
145                 150                 155                 160

Ala Val Arg His Leu Gly Pro Glu Gly Cys Pro Asp Leu Asp Gly Gly
                165                 170                 175

Leu Leu Glu Arg His Pro Trp Trp Gln Ala Asn Gly Arg Gly Asn Asn
            180                 185                 190

Gln Lys Ser Gly Glu Ala Gly Ser Pro Lys Gly Arg Glu Glu Pro Leu
        195                 200                 205

Gly Gln Gly Gly Val Met Gly Gly Thr Ser Ser
    210                 215
```

<210> 73

<211> 636
<212> DNA
<213> Medicago trunculata

<400> 73
atgcagcagc acctgatgca gatgcagccc atgatggcag cttactatcc taacaacgtc 60

actactgatc atattcaaca gtatcttgat gagaacaagt ccttgattct caagattgtt 120

gaaagccaga acactggcaa gctcaccgag tgtgctgaga accaatcaag gcttcagaga 180

aatctcatgt acctagctgc aatagctgat tctcaacccc aaccacctac tatgcctggc 240

cagtaccctt caagtggaat gatgcagcag ggaggacact acatgcaggc tcaacaagct 300

cagcagatga cacaacaaca attaatggct gcacgttcct ctcttatgta tgctcaacag 360

cttcaacagc agcaagcctt gcaaagccaa cttggtatga attccagtgg aagtcaaggc 420

cttcacatgt tgcatagtga aggggctaat gttggaggca attcatctct aggggctggt 480

tttcctgatt ttggccgtag ctcagccggt gatggtttgc acggcagtgg taagcaagac 540

attggaagca ctgatggccg cggtggaagc tctagtggtc actctggtga tggcggcgaa 600

acactttacc tgaaatcttc tggtgatggg aattag 636

<210> 74
<211> 211
<212> PRT
<213> Medicago trunculata

<400> 74

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Thr Gly Lys Leu
35 40 45

Thr Glu Cys Ala Glu Asn Gln Ser Arg Leu Gln Arg Asn Leu Met Tyr
50 55 60

Leu Ala Ala Ile Ala Asp Ser Gln Pro Gln Pro Pro Thr Met Pro Gly
65 70 75 80

Gln Tyr Pro Ser Ser Gly Met Met Gln Gln Gly Gly His Tyr Met Gln
85 90 95

Ala Gln Gln Ala Gln Gln Met Thr Gln Gln Gln Leu Met Ala Ala Arg
100 105 110

Ser Ser Leu Met Tyr Ala Gln Gln Leu Gln Gln Gln Gln Ala Leu Gln
115 120 125

Ser Gln Leu Gly Met Asn Ser Ser Gly Ser Gln Gly Leu His Met Leu
    130                 135                 140

His Ser Glu Gly Ala Asn Val Gly Gly Asn Ser Ser Leu Gly Ala Gly
145                 150                 155                 160

Phe Pro Asp Phe Gly Arg Ser Ser Ala Gly Asp Gly Leu His Gly Ser
                165                 170                 175

Gly Lys Gln Asp Ile Gly Ser Thr Asp Gly Arg Gly Gly Ser Ser Ser
            180                 185                 190

Gly His Ser Gly Asp Gly Gly Glu Thr Leu Tyr Leu Lys Ser Ser Gly
        195                 200                 205

Asp Gly Asn
    210

<210> 75
<211> 684
<212> DNA
<213> Oryza sativa

<400> 75
atgcagcagc aacacctgat gcagatgaac cagggcatga tgggggggata tgcttcccct 60

accaccgtca ccactgatct cattcagcag tatctggatg agaacaagca gctgatcctg 120

gccatccttg acaaccagaa caatgggaag gtggaagagt gcgctcggaa ccaagctaag 180

ctccagcaca atctcatgta cctcgccgcc atcgccgaca gccagccgcc gcagacggcc 240

gccatgtccc agtatccgtc gaacctgatg atgcagtccg gggcgaggta catgccgcag 300

cagtcggcgc agatgatggc gccgcagtcg ctgatggcgg cgaggtcttc gatgatgtac 360

gcgcagccgg cgctgtcgcc gctccagcag cagcagcagc agcaggcggc ggcggcgcac 420

gggcagctgg gcatgggctc gggggggcacc accagcgggt tcagcatcct ccacggcgag 480

gccagcatgg gcggcggcgg cggcggcggt ggcgccggta acagcatgat gaacgccggc 540

gtgttctccg acttcggacg cggcggcggc ggcggcggca aggaggggtc cacctcgctg 600

tccgtcgacg tccgggggcgc caactccggc gcccagagcg gcgacgggga gtacctcaag 660

ggcaccgagg aggaaggcag ctag 684

<210> 76
<211> 227
<212> PRT
<213> Oryza sativa

<400> 76

Met Gln Gln Gln His Leu Met Gln Met Asn Gln Gly Met Met Gly Gly

1 5 10 15

Tyr Ala Ser Pro Thr Thr Val Thr Thr Asp Leu Ile Gln Gln Tyr Leu
20 25 30

Asp Glu Asn Lys Gln Leu Ile Leu Ala Ile Leu Asp Asn Gln Asn Asn
35 40 45

Gly Lys Val Glu Glu Cys Ala Arg Asn Gln Ala Lys Leu Gln His Asn
50 55 60

Leu Met Tyr Leu Ala Ala Ile Ala Asp Ser Gln Pro Pro Gln Thr Ala
65 70 75 80

Ala Met Ser Gln Tyr Pro Ser Asn Leu Met Met Gln Ser Gly Ala Arg
85 90 95

Tyr Met Pro Gln Gln Ser Ala Gln Met Met Ala Pro Gln Ser Leu Met
100 105 110

Ala Ala Arg Ser Ser Met Met Tyr Ala Gln Pro Ala Leu Ser Pro Leu
115 120 125

Gln Gln Gln Gln Gln Gln Gln Ala Ala Ala Ala His Gly Gln Leu Gly
130 135 140

Met Gly Ser Gly Gly Thr Thr Ser Gly Phe Ser Ile Leu His Gly Glu
145 150 155 160

Ala Ser Met Gly Gly Gly Gly Gly Gly Gly Gly Ala Gly Asn Ser Met
165 170 175

Met Asn Ala Gly Val Phe Ser Asp Phe Gly Arg Gly Gly Gly Gly Gly
180 185 190

Gly Lys Glu Gly Ser Thr Ser Leu Ser Val Asp Val Arg Gly Ala Asn
195 200 205

Ser Gly Ala Gln Ser Gly Asp Gly Glu Tyr Leu Lys Gly Thr Glu Glu
210 215 220

Glu Gly Ser
225

<210> 77
<211> 558
<212> DNA
<213> Oryza sativa

<400> 77

atgcagcagc agccgatgcc gatgcccgcg caggcgccgc cgacggccgg aatcaccacc 60

gagcagatcc aaaagtatct ggatgaaaac aagcagctta ttttggctat tttggaaaat 120

cagaatctgg gaaagttggc agaatgtgct cagtatcaag cgcagcttca gaagaatctc 180

ttgtacttgg ctgcaattgc tgatactcaa ccgcagacca ctataagccg tccccagatg 240

gtgccgcatg gtgcatcgcc ggggttaggg gggcaataca tgtcgcaggt gccaatgttc 300

ccccccagga cccctctaac gccccagcag atgcaggagc agcagctgca gcaacagcaa 360

gcccagctgc tctcgttcgg cggtcagatg gttatgaggc ctggcgttgt gaatggcatt 420

cctcagcttc tgcaaggcga aatgcaccgc ggagcagatc accagaacgc tggcggggcc 480

acctcggagc cttccgagag ccacaggagc accggcaccg aaaatgacgg tggaagcgac 540

ttcggcgatc aatcctaa 558

<210> 78
<211> 185
<212> PRT
<213> Oryza sativa

<400> 78

Met Gln Gln Gln Pro Met Pro Met Pro Ala Gln Ala Pro Pro Thr Ala
1 5 10 15

Gly Ile Thr Thr Glu Gln Ile Gln Lys Tyr Leu Asp Glu Asn Lys Gln
20 25 30

Leu Ile Leu Ala Ile Leu Glu Asn Gln Asn Leu Gly Lys Leu Ala Glu
35 40 45

Cys Ala Gln Tyr Gln Ala Gln Leu Gln Lys Asn Leu Leu Tyr Leu Ala
50 55 60

Ala Ile Ala Asp Thr Gln Pro Gln Thr Thr Ile Ser Arg Pro Gln Met
65 70 75 80

Val Pro His Gly Ala Ser Pro Gly Leu Gly Gly Gln Tyr Met Ser Gln
85 90 95

Val Pro Met Phe Pro Pro Arg Thr Pro Leu Thr Pro Gln Gln Met Gln
100 105 110

Glu Gln Gln Leu Gln Gln Gln Gln Ala Gln Leu Leu Ser Phe Gly Gly
115 120 125

Gln Met Val Met Arg Pro Gly Val Val Asn Gly Ile Pro Gln Leu Leu
130 135 140

Gln Gly Glu Met His Arg Gly Ala Asp His Gln Asn Ala Gly Gly Ala

145 150 155 160

Thr Ser Glu Pro Ser Glu Ser His Arg Ser Thr Gly Thr Glu Asn Asp
165 170 175

Gly Gly Ser Asp Phe Gly Asp Gln Ser
180 185

<210> 79
<211> 618
<212> DNA
<213> Oryza sativa

<400> 79
atgcagcagc agatggccat gccggcgggg gccgccgccg ccgcggtgcc gccggcggcc 60

ggcatcacca ccgagcagat ccaaaagtat ttggatgaaa ataaacagct aattttggcc 120

atcctggaaa atcaaaacct agggaagttg gctgaatgtg ctcagtacca agctcagctt 180

caaaagaatc tcttgtatct ggctgccatt gcagatgccc aaccacctca gaatccagga 240

agtcgccctc agatgatgca gcctggtgct accccaggtg ctgggcatta catgtcccaa 300

gtaccgatgt tccctccaag aactccctta accccacaac agatgcaaga gcagcagcag 360

cagcaactcc agcaacagca agctcaggct ctagccttcc ccggccagat gctaatgaga 420

ccaggtactg tcaatggcat gcaatctatc ccagttgctg accctgctcg cgcagccgat 480

cttcagacgg cagcaccggg ctcggtagat ggccgaggaa acaagcagga tgcaacctcg 540

gagccttccg ggaccgagag ccacaagagt gcgggagcag ataacgacgc aggcggtgac 600

atagcggaga agtcctga 618

<210> 80
<211> 205
<212> PRT
<213> Oryza sativa

<400> 80

Met Gln Gln Gln Met Ala Met Pro Ala Gly Ala Ala Ala Ala Ala Val
1 5 10 15

Pro Pro Ala Ala Gly Ile Thr Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Ala Gln Pro Pro Gln Asn Pro Gly
65 70 75 80

Ser Arg Pro Gln Met Met Gln Pro Gly Ala Thr Pro Gly Ala Gly His
85 90 95

Tyr Met Ser Gln Val Pro Met Phe Pro Pro Arg Thr Pro Leu Thr Pro
100 105 110

Gln Gln Met Gln Glu Gln Gln Gln Gln Gln Leu Gln Gln Gln Gln Ala
115 120 125

Gln Ala Leu Ala Phe Pro Gly Gln Met Leu Met Arg Pro Gly Thr Val
130 135 140

Asn Gly Met Gln Ser Ile Pro Val Ala Asp Pro Ala Arg Ala Ala Asp
145 150 155 160

Leu Gln Thr Ala Ala Pro Gly Ser Val Asp Gly Arg Gly Asn Lys Gln
165 170 175

Asp Ala Thr Ser Glu Pro Ser Gly Thr Glu Ser His Lys Ser Ala Gly
180 185 190

Ala Asp Asn Asp Ala Gly Gly Asp Ile Ala Glu Lys Ser
195 200 205

<210> 81
<211> 540
<212> DNA
<213> Solanum tuberosum

<400> 81
atgcagcagc agcacctgat gcagatgcag cccatgatgg cagcctatta tcccaacaat 60

gtcactactg atcatattca acagttcctg gatgagaaca aatcacttat tctgaagatt 120

gttgagagcc agaactctgg gaaaataagt gaatgtgcag agtcccaagc taaacttcag 180

agaaatctta tgtaccttgc agctattgct gattcacagc cccagcctcc tagtatgcat 240

tcacagttag cttctggtgg gatgatgcag ggaggggcac attatatgca gcaacaacaa 300

gctcaacaac tcacaacgca atcgcttatg gctgcagcaa gatcctcctc ctcaatgctc 360

tatggacaac aacaacaaca acaacaacaa caactatcat cattgcaaca acagcaagca 420

gcctttcata gccagcaact cggaatgagc agctctggtg gaggaagcag tagtggactt 480

cacatgctac aaagcgaaaa cactcatagt gctagcactg gtggtgggtg gtttccctga 540

<210> 82
<211> 179
<212> PRT
<213> Solanum tuberosum

<400> 82

Met Gln Gln Gln His Leu Met Gln Met Gln Pro Met Met Ala Ala Tyr
1               5                   10                  15

Tyr Pro Asn Asn Val Thr Thr Asp His Ile Gln Gln Phe Leu Asp Glu
            20                  25                  30

Asn Lys Ser Leu Ile Leu Lys Ile Val Glu Ser Gln Asn Ser Gly Lys
        35                  40                  45

Ile Ser Glu Cys Ala Glu Ser Gln Ala Lys Leu Gln Arg Asn Leu Met
    50                  55                  60

Tyr Leu Ala Ala Ile Ala Asp Ser Gln Pro Gln Pro Pro Ser Met His
65                  70                  75                  80

Ser Gln Leu Ala Ser Gly Gly Met Met Gln Gly Gly Ala His Tyr Met
                85                  90                  95

Gln Gln Gln Gln Ala Gln Gln Leu Thr Thr Gln Ser Leu Met Ala Ala
            100                 105                 110

Ala Arg Ser Ser Ser Ser Met Leu Tyr Gly Gln Gln Gln Gln Gln Gln
        115                 120                 125

Gln Gln Gln Leu Ser Ser Leu Gln Gln Gln Gln Ala Ala Phe His Ser
    130                 135                 140

Gln Gln Leu Gly Met Ser Ser Ser Gly Gly Gly Ser Ser Ser Gly Leu
145                 150                 155                 160

His Met Leu Gln Ser Glu Asn Thr His Ser Ala Ser Thr Gly Gly Gly
                165                 170                 175

Trp Phe Pro

<210> 83
<211> 684
<212> DNA
<213> Zea mays

<400> 83
atgcagcagc aacacctgat gcagatgaac cagaacatga tggggggcta cacctctcct 60

gccgccgtga ccaccgatct catccagcag cacctggacg agaacaagca gctgatcctg 120

gccatcctcg acaaccagaa caatggcaag gcggaggagt gcgaacggca ccaagctaag 180

ctccagcaca acctcatgta cctggccgcc atcgctgaca gccagccgcc acagaccgcg 240

ccactatcac agtacccgtc caacctgatg atgcagccgg gccctcggta catgccaccg 300

```
cagtccgggc agatgatgaa cccgcagtcg ctgatggcgg cgcggtcctc catgatgtac     360

gcgcaccccgt ccctgtcgcc actccagcag cagcaggcgg cgcacggaca gctgggtatg    420

gctccagggg gcggcggtgg cggcacgacc agcgggttca gcatcctcca cggcgaggcc     480

agcatgggcg gtggtggtgc tggcgcaggc gccggcaaca acatgatgaa cgccggcatg     540

ttctcgggct ttggccgcag cggcagtggc gccaaggaag ggtcgacctc tctgtcggtt     600

gacgtccggg gtggaaccag ctccggcgcg cagagcgggg acggcgagta cctcaaagtc     660

ggcaccgagg aagaaggcag ttag                                            684
```

<210> 84
<211> 227
<212> PRT
<213> Zea mays

<400> 84

```
Met Gln Gln Gln His Leu Met Gln Met Asn Gln Asn Met Met Gly Gly
1               5                   10                  15

Tyr Thr Ser Pro Ala Ala Val Thr Thr Asp Leu Ile Gln Gln His Leu
            20                  25                  30

Asp Glu Asn Lys Gln Leu Ile Leu Ala Ile Leu Asp Asn Gln Asn Asn
        35                  40                  45

Gly Lys Ala Glu Glu Cys Glu Arg His Gln Ala Lys Leu Gln His Asn
    50                  55                  60

Leu Met Tyr Leu Ala Ala Ile Ala Asp Ser Gln Pro Pro Gln Thr Ala
65                  70                  75                  80

Pro Leu Ser Gln Tyr Pro Ser Asn Leu Met Met Gln Pro Gly Pro Arg
                85                  90                  95

Tyr Met Pro Pro Gln Ser Gly Gln Met Met Asn Pro Gln Ser Leu Met
            100                 105                 110

Ala Ala Arg Ser Ser Met Met Tyr Ala His Pro Ser Leu Ser Pro Leu
        115                 120                 125

Gln Gln Gln Gln Ala Ala His Gly Gln Leu Gly Met Ala Pro Gly Gly
    130                 135                 140

Gly Gly Gly Gly Thr Thr Ser Gly Phe Ser Ile Leu His Gly Glu Ala
145                 150                 155                 160

Ser Met Gly Gly Gly Gly Ala Gly Ala Gly Ala Gly Asn Asn Met Met
                165                 170                 175
```

Asn Ala Gly Met Phe Ser Gly Phe Gly Arg Ser Gly Ser Gly Ala Lys
            180                 185                 190

Glu Gly Ser Thr Ser Leu Ser Val Asp Val Arg Gly Gly Thr Ser Ser
        195                 200                 205

Gly Ala Gln Ser Gly Asp Gly Glu Tyr Leu Lys Val Gly Thr Glu Glu
    210                 215                 220

Glu Gly Ser
225

<210> 85
<211> 549
<212> DNA
<213> Zea mays

<400> 85
atgcagcagc cgatgcacat gcagccacag gcgccggcga taaccccagc tgccggaatc 60

agcacggagc agatccaaaa gtatctggat gagaataagc agcttatttt ggctattttg 120

gaaaatcaga acctaggaaa attggcagaa tgtgctcagt atcaatcaca acttcagaag 180

aacctcttgt atctcgctgc aatcgcagat gctcaaccgc agactgctgt aagccgccct 240

cagatggcgc cgcctggtgg atcgcctgga gtagggcagt acatgtcaca ggtgcctatg 300

ttcccaccga ggacacctct tacaccccag cagatgcagg agcagcagct tcagcagcag 360

caggctcagt tgctaaactt cagtggccaa atggttgcta gaccaggcat ggtcaacggc 420

atggctcagt ccatgcaagc tcagctacca ccgggtgtga acaagcagga tgctggtggg 480

gtcgcctctg agccctcggg caccgagagc cacaggagca ctggtggtga cgatggtgga 540

agcgactag 549

<210> 86
<211> 182
<212> PRT
<213> Zea mays

<400> 86

Met Gln Gln Pro Met His Met Gln Pro Gln Ala Pro Ala Ile Thr Pro
1               5                   10                  15

Ala Ala Gly Ile Ser Thr Glu Gln Ile Gln Lys Tyr Leu Asp Glu Asn
            20                  25                  30

Lys Gln Leu Ile Leu Ala Ile Leu Glu Asn Gln Asn Leu Gly Lys Leu
        35                  40                  45

Ala Glu Cys Ala Gln Tyr Gln Ser Gln Leu Gln Lys Asn Leu Leu Tyr
    50                  55                  60

Leu Ala Ala Ile Ala Asp Ala Gln Pro Gln Thr Ala Val Ser Arg Pro
65                  70                  75                  80

Gln Met Ala Pro Pro Gly Gly Ser Pro Gly Val Gly Gln Tyr Met Ser
                85                  90                  95

Gln Val Pro Met Phe Pro Pro Arg Thr Pro Leu Thr Pro Gln Gln Met
            100                 105                 110

Gln Glu Gln Gln Leu Gln Gln Gln Gln Ala Gln Leu Leu Asn Phe Ser
        115                 120                 125

Gly Gln Met Val Ala Arg Pro Gly Met Val Asn Gly Met Ala Gln Ser
    130                 135                 140

Met Gln Ala Gln Leu Pro Pro Gly Val Asn Lys Gln Asp Ala Gly Gly
145                 150                 155                 160

Val Ala Ser Glu Pro Ser Gly Thr Glu Ser His Arg Ser Thr Gly Gly
                165                 170                 175

Asp Asp Gly Gly Ser Asp
            180

<210> 87
<211> 1173
<212> DNA
<213> Homo sapiens

<400> 87
atgggcggca acatgtctgt ggctttcgcg gccccgaggc agcgaggcaa gggggagatc 60

actcccgctg cgattcagaa gatgttggat gacaataacc atcttattca gtgtataatg 120

gactctcaga ataaaggaaa gacctcagag tgttctcagt atcagcagat gttgcacaca 180

aacttggtat accttgctac aatagcagat tctaatcaaa atatgcagtc tcttttacca 240

gcaccaccca cacagaatat gcctatgggt cctggaggga tgaatcagag cggccctccc 300

ccacctccac gctctcacaa catgccttca gatggaatgg taggtggggg tcctcctgca 360

ccgcacatgc agaaccagat gaacggccag atgcctgggc ctaaccatat gcctatgcag 420

ggacctggac ccaatcaact caatatgaca aacagttcca tgaatatgcc ttcaagtagc 480

catggatcca tgggaggtta caaccattct gtgccatcat cacagagcat gccagtacag 540

aatcagatga caatgagtca gggacaacca atgggaaact atggtcccag accaaatatg 600

agtatgcagc caaaccaagg tccaatgatg catcagcagc ctccttctca gcaatacaat 660

atgccacagg gaggcggaca gcattaccaa ggacagcagc cacctatggg aatgatgggt 720

caagttaacc aaggcaatca tatgatgggt cagagacaga ttcctcccta tagacctcct 780

caacagggcc caccacagca gtactcaggc caggaagact attacgggga ccaatacagt 840

catggtggac aaggtcctcc agaaggcatg aaccagcaat attaccctga tggaaattca 900

cagtatggcc aacagcaaga tgcataccag ggaccacctc cacaacaggg atatccaccc 960

cagcagcagc agtacccagg gcagcaaggt tacccaggac agcagcaggg ctacggtcct 1020

tcacagggtg gtccaggtcc tcagtatcct aactacccac agggacaagg tcagcagtat 1080

ggaggatata gaccaacaca gcctggacca ccacagccac cccagcagag gccttatgga 1140

tatgaccagg gacagtatgg aaattaccag cag 1173

<210> 88
<211> 391
<212> PRT
<213> Homo sapiens

<400> 88

Met Gly Gly Asn Met Ser Val Ala Phe Ala Ala Pro Arg Gln Arg Gly
1 5 10 15

Lys Gly Glu Ile Thr Pro Ala Ala Ile Gln Lys Met Leu Asp Asp Asn
20 25 30

Asn His Leu Ile Gln Cys Ile Met Asp Ser Gln Asn Lys Gly Lys Thr
35 40 45

Ser Glu Cys Ser Gln Tyr Gln Gln Met Leu His Thr Asn Leu Val Tyr
50 55 60

Leu Ala Thr Ile Ala Asp Ser Asn Gln Asn Met Gln Ser Leu Leu Pro
65 70 75 80

Ala Pro Pro Thr Gln Asn Met Pro Met Gly Pro Gly Gly Met Asn Gln
85 90 95

Ser Gly Pro Pro Pro Pro Pro Arg Ser His Asn Met Pro Ser Asp Gly
100 105 110

Met Val Gly Gly Gly Pro Pro Ala Pro His Met Gln Asn Gln Met Asn
115 120 125

Gly Gln Met Pro Gly Pro Asn His Met Pro Met Gln Gly Pro Gly Pro
130 135 140

Asn Gln Leu Asn Met Thr Asn Ser Ser Met Asn Met Pro Ser Ser Ser
145 150 155 160

His Gly Ser Met Gly Gly Tyr Asn His Ser Val Pro Ser Ser Gln Ser
165 170 175

```
Met Pro Val Gln Asn Gln Met Thr Met Ser Gln Gly Gln Pro Met Gly
            180                 185                 190

Asn Tyr Gly Pro Arg Pro Asn Met Ser Met Gln Pro Asn Gln Gly Pro
        195                 200                 205

Met Met His Gln Gln Pro Pro Ser Gln Gln Tyr Asn Met Pro Gln Gly
    210                 215                 220

Gly Gly Gln His Tyr Gln Gly Gln Gln Pro Pro Met Gly Met Met Gly
225                 230                 235                 240

Gln Val Asn Gln Gly Asn His Met Met Gly Gln Arg Gln Ile Pro Pro
                245                 250                 255

Tyr Arg Pro Pro Gln Gln Gly Pro Pro Gln Gln Tyr Ser Gly Gln Glu
            260                 265                 270

Asp Tyr Tyr Gly Asp Gln Tyr Ser His Gly Gly Gln Gly Pro Pro Glu
        275                 280                 285

Gly Met Asn Gln Gln Tyr Tyr Pro Asp Gly Asn Ser Gln Tyr Gly Gln
    290                 295                 300

Gln Gln Asp Ala Tyr Gln Gly Pro Pro Pro Gln Gln Gly Tyr Pro Pro
305                 310                 315                 320

Gln Gln Gln Gln Tyr Pro Gly Gln Gln Gly Tyr Pro Gly Gln Gln Gln
                325                 330                 335

Gly Tyr Gly Pro Ser Gln Gly Gly Pro Gly Pro Gln Tyr Pro Asn Tyr
            340                 345                 350

Pro Gln Gly Gln Gly Gln Gln Tyr Gly Gly Tyr Arg Pro Thr Gln Pro
        355                 360                 365

Gly Pro Pro Gln Pro Pro Gln Gln Arg Pro Tyr Gly Tyr Asp Gln Gly
    370                 375                 380

Gln Tyr Gly Asn Tyr Gln Gln
385                 390
```

<210> 89
<211> 627
<212> DNA
<213> Allium cepa

<400> 89
atgcagcagc cgcagccagc gatgggaacc atgggctcgg tgccacctac tagcatcacc 60

accgaacaga ttcaaaggta cttggatgag aacaaacagt taatattggc aattttggat 120

aatcaaaatt taggaagact gaatgagtgt gctcaatatc aagctcagct tcaaaagaat 180

ctgctttacc tggcagcaat agctgatgct cagcctcagt ctcctgcggt gcgtctgcag 240

atgatgcctc aaggtgcagc tgccacgcct caagctggaa accaatttat gcagcagcag 300

agccctaatt tccctcccaa aacaggaatg caatttactc ctcaacaagt acaagaattg 360

cagcagcaac agctacaaca tcagccacat atgatgcctc catttcaagg tcaaatgggt 420

atgagaccta tgaatggaat gcaggcagca atgcatgcag attcatctct tgcttataac 480

actaacaata agcaagatgc aggaaacgca gcttatgaaa atactgctgc caacacagat 540

ggttccattc aaaagaaaac agcaaatgat gatttagacc cttctgcagc aaaccctaga 600

aggtctgaag atgccaaatc atcatga 627

<210> 90
<211> 208
<212> PRT
<213> Allium cepa

<400> 90

Met Gln Gln Pro Gln Pro Ala Met Gly Thr Met Gly Ser Val Pro Pro
1 5 10 15

Thr Ser Ile Thr Thr Glu Gln Ile Gln Arg Tyr Leu Asp Glu Asn Lys
20 25 30

Gln Leu Ile Leu Ala Ile Leu Asp Asn Gln Asn Leu Gly Arg Leu Asn
35 40 45

Glu Cys Ala Gln Tyr Gln Ala Gln Leu Gln Lys Asn Leu Leu Tyr Leu
50 55 60

Ala Ala Ile Ala Asp Ala Gln Pro Gln Ser Pro Ala Val Arg Leu Gln
65 70 75 80

Met Met Pro Gln Gly Ala Ala Ala Thr Pro Gln Ala Gly Asn Gln Phe
85 90 95

Met Gln Gln Gln Ser Pro Asn Phe Pro Pro Lys Thr Gly Met Gln Phe
100 105 110

Thr Pro Gln Gln Val Gln Glu Leu Gln Gln Gln Gln Leu Gln His Gln
115 120 125

Pro His Met Met Pro Pro Phe Gln Gly Gln Met Gly Met Arg Pro Met
130 135 140

Asn Gly Met Gln Ala Ala Met His Ala Asp Ser Ser Leu Ala Tyr Asn
145                 150                 155                 160

Thr Asn Asn Lys Gln Asp Ala Gly Asn Ala Ala Tyr Glu Asn Thr Ala
                165                 170                 175

Ala Asn Thr Asp Gly Ser Ile Gln Lys Lys Thr Ala Asn Asp Asp Leu
            180                 185                 190

Asp Pro Ser Ala Ala Asn Pro Arg Arg Ser Glu Asp Ala Lys Ser Ser
        195                 200                 205

<210> 91
<211> 633
<212> DNA
<213> Aquilegia formosa x Aquilegia pubescens

<400> 91
atgcaacaca tgcagatgca gcccatgatg ccaccttata gtgccaacag cgtcactact 60

gatcatatcc aacagtactt ggatgaaaat aaggcgttga ttctgaagat acttgagaac 120

caaaattcgg gaaaagttag tgaatgtgca gagaaccaag caagacttca acgaaatctt 180

atgtatctgg ctgcaattgc tgattctcaa ccacagcctc ccaatatgca tgctcagtac 240

tctaatgcgg gtataccacc tggtgcacat tacctacaac accaacaggc ccaacagatg 300

acacaacagt cgctcatggc tgctcgatca aatatgctgt atgctcagcc aatcacagga 360

atgcagcaac agcaagcaat gcatagccag cttggcatga gctctggtgg taacagtgga 420

ctccacatga tgcacaatga gggcagcatg ggaggtagtg gggcacttgg aagctattct 480

gattatggcc gtggcagtgg tggtggagta actatcgcta gcaaacaaga tggtggaagt 540

ggttctggtg aaggacgagg tggaaactct ggaggccaaa gtgcagatgg aggtgaatct 600

ctttacctga aaaacagtga cgaagggaac taa 633

<210> 92
<211> 210
<212> PRT
<213> Aquilegia formosa x Aquilegia pubescens

<400> 92

Met Gln His Met Gln Met Gln Pro Met Met Pro Pro Tyr Ser Ala Asn
1               5                   10                  15

Ser Val Thr Thr Asp His Ile Gln Gln Tyr Leu Asp Glu Asn Lys Ala
            20                  25                  30

Leu Ile Leu Lys Ile Leu Glu Asn Gln Asn Ser Gly Lys Val Ser Glu
        35                  40                  45

Cys Ala Glu Asn Gln Ala Arg Leu Gln Arg Asn Leu Met Tyr Leu Ala

```
        50                  55                  60

Ala Ile Ala Asp Ser Gln Pro Gln Pro Pro Asn Met His Ala Gln Tyr
65                  70                  75                  80

Ser Asn Ala Gly Ile Pro Pro Gly Ala His Tyr Leu Gln His Gln Gln
                85                  90                  95

Ala Gln Gln Met Thr Gln Gln Ser Leu Met Ala Ala Arg Ser Asn Met
            100                 105                 110

Leu Tyr Ala Gln Pro Ile Thr Gly Met Gln Gln Gln Gln Ala Met His
        115                 120                 125

Ser Gln Leu Gly Met Ser Ser Gly Gly Asn Ser Gly Leu His Met Met
    130                 135                 140

His Asn Glu Gly Ser Met Gly Gly Ser Gly Ala Leu Gly Ser Tyr Ser
145                 150                 155                 160

Asp Tyr Gly Arg Gly Ser Gly Gly Gly Val Thr Ile Ala Ser Lys Gln
                165                 170                 175

Asp Gly Gly Ser Gly Ser Gly Glu Gly Arg Gly Gly Asn Ser Gly Gly
            180                 185                 190

Gln Ser Ala Asp Gly Gly Glu Ser Leu Tyr Leu Lys Asn Ser Asp Glu
        195                 200                 205

Gly Asn
    210
```

<210> 93
<211> 615
<212> DNA
<213> Brachypodium distachyon

<400> 93

```
atgcagcagg cgatgtccat gtccccgggg tcggccggcg cggtgccgcc tccggccggc      60

atcaccacag agcagatcca aaagtatttg gatgaaaata agcaacttat tttggccatc     120

ctggaaaatc agaacctagg aaagttgact gaatgtgctc agtatcaagc tcaacttcag     180

aagaatctct tgtatctggc tgccattgcg gatgcccaac caccacagaa ccctggaagt     240

cgcccccaga tggtgcagcc tggtggtatg ccaggtgcag ggcattacat gtcgcaagta     300

ccaatgttcc ctccaagaac ccctttaacc ccacaacaga tgcaagagca acagcaccag     360

cagcttcagc agcagcaagc acaggctctt gctttcccca gccagatggt catgagacca     420

ggtactgtga acggcatgca gcctatgcaa gctgatctcc aagcagcagc agcagcacct     480
```

ggcctggcag acagccgagg aagtaagcag gacgcagcgg tagctggggc catctcggaa 540

ccttctggca ccgagagtca caagagtaca ggagcggatc atgaggcagg tggcgatgta 600

gctgagcaat cctaa 615

<210> 94
<211> 204
<212> PRT
<213> Brachypodium distachyon

<400> 94

Met Gln Gln Ala Met Ser Met Ser Pro Gly Ser Ala Gly Ala Val Pro
1 5 10 15

Pro Pro Ala Gly Ile Thr Thr Glu Gln Ile Gln Lys Tyr Leu Asp Glu
20 25 30

Asn Lys Gln Leu Ile Leu Ala Ile Leu Glu Asn Gln Asn Leu Gly Lys
35 40 45

Leu Thr Glu Cys Ala Gln Tyr Gln Ala Gln Leu Gln Lys Asn Leu Leu
50 55 60

Tyr Leu Ala Ala Ile Ala Asp Ala Gln Pro Pro Gln Asn Pro Gly Ser
65 70 75 80

Arg Pro Gln Met Val Gln Pro Gly Gly Met Pro Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln His Gln Gln Leu Gln Gln Gln Gln Ala Gln
115 120 125

Ala Leu Ala Phe Pro Ser Gln Met Val Met Arg Pro Gly Thr Val Asn
130 135 140

Gly Met Gln Pro Met Gln Ala Asp Leu Gln Ala Ala Ala Ala Ala Pro
145 150 155 160

Gly Leu Ala Asp Ser Arg Gly Ser Lys Gln Asp Ala Ala Val Ala Gly
165 170 175

Ala Ile Ser Glu Pro Ser Gly Thr Glu Ser His Lys Ser Thr Gly Ala
180 185 190

Asp His Glu Ala Gly Gly Asp Val Ala Glu Gln Ser
195 200

<210> 95
<211> 636
<212> DNA
<213> Brassica napus

<400> 95
atgcagcagc agcagcagca gcagcagcag cctccgcaaa tgtttccgat ggctccttcg 60

atgccgccaa ctaacatcac caccgaacag atccaaaagt accttgagga gaacaagaag 120

ctgataatgg caatcatgga aaatcagaat cttggcaagc ttgcagagtg tgcacagtac 180

caagctcttc tccagaagaa cttaatgtac ctcgctgcta ttgctgatgc tcaacctcct 240

ccatctaccg ctggagctac accaccacca gctatggctt cccagatggg ggcaccgcat 300

cctgggatgc aaccgccgag ctactttatg caacacccac aagcttcagg gatggctcaa 360

caagcaccac ccgctggtat cttccctccg agaggtcctt tgcagtttgg tagcccacac 420

cagcttcagg atccgcaaca gcagcatatg catcaacagg ctatgcaagg acacatgggg 480

atgcgaccaa tgggtatcaa caacaacaat gggatgcagc atcagatgca gcaacaacaa 540

ccagaaacct ctcttggagg aagcgctgca aacgtggggc ttagaggtgg aaagcaagat 600

ggagcagatg gacaaggaaa agatgatggc aaatga 636

<210> 96
<211> 203
<212> PRT
<213> Brassica napus

<400> 96

Met Gln Gln His Leu Met Gln Met Gln Pro Met Met Ala Gly Tyr Tyr
1 5 10 15

Pro Ser Asn Val Thr Ser Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Ser Val His Ser
65 70 75 80

Gln Tyr Gly Ser Ala Gly Gly Gly Leu Ile Gln Gly Glu Gly Ala Ser
85 90 95

His Tyr Leu Gln Gln Gln Gln Ala Thr Gln Gln Gln Gln Met Thr Gln
100 105 110

Gln Ser Leu Met Ala Ala Arg Ser Ser Met Met Tyr Gln Gln Gln Gln
115 120 125

Gln Pro Tyr Ala Thr Leu Gln His Gln Gln Leu His His Ser Gln Leu
130 135 140

Gly Met Ser Ser Ser Ser Gly Gly Gly Ser Ser Gly Leu His Ile Leu
145 150 155 160

Gln Gly Glu Ala Gly Gly Phe His Glu Phe Gly Arg Gly Lys Pro Glu
165 170 175

Met Gly Ser Gly Glu Gly Arg Gly Gly Ser Ser Gly Asp Gly Gly Glu
180 185 190

Thr Leu Tyr Leu Lys Ser Ser Asp Asp Gly Asn
195 200

<210> 97
<211> 636
<212> DNA
<213> Citrus sinensis

<400> 97
atgcagcagc caccgcaaat gatccctgtt atgccttcat ttccacccac caacatcacc 60

acagagcaga ttcaaaagta ccttgatgag aacaaaaagt tgattttggc aattttggac 120

aatcaaaatc ttggaaagct tacagaatgt gcccactatc aagctcagct tcaaaagaat 180

ttaatgtatt tagctgcaat tgctgatgca caaccacaag caccaacaat gcctcctcag 240

atggctccac atcctgcaat gcaagctagt gggtattaca tgcaacatcc tcaggcggca 300

gcaatggctc agcaacaagg aatctttccc caaaagatgc cattacaatt caataaccct 360

catcaactac aggatcctca acagcagcta caccaacatc aagccatgca agcacaaatg 420

ggaatgagac cgggtgccac taacaatggt atgcatccca tgcatgctga aagctctctt 480

ggaggtggca gcagtggagg accccccttca gcatcaggcc caggtgacat acgtggtgga 540

aataagcaag atgcctcgga ggctgggact actggtgctg atggccaggg cagttcggct 600

ggtgggcatg gtggggatgg agaggaggca aagtga 636

<210> 98
<211> 211
<212> PRT
<213> Citrus sinensis

<400> 98

Met Gln Gln Pro Pro Gln Met Ile Pro Val Met Pro Ser Phe Pro Pro
1 5 10 15

Thr Asn Ile Thr Thr Glu Gln Ile Gln Lys Tyr Leu Asp Glu Asn Lys

20 25 30

Lys Leu Ile Leu Ala Ile Leu Asp Asn Gln Asn Leu Gly Lys Leu Thr
35 40 45

Glu Cys Ala His Tyr Gln Ala Gln Leu Gln Lys Asn Leu Met Tyr Leu
50 55 60

Ala Ala Ile Ala Asp Ala Gln Pro Gln Ala Pro Thr Met Pro Pro Gln
65 70 75 80

Met Ala Pro His Pro Ala Met Gln Ala Ser Gly Tyr Tyr Met Gln His
85 90 95

Pro Gln Ala Ala Ala Met Ala Gln Gln Gln Gly Ile Phe Pro Gln Lys
100 105 110

Met Pro Leu Gln Phe Asn Asn Pro His Gln Leu Gln Asp Pro Gln Gln
115 120 125

Gln Leu His Gln His Gln Ala Met Gln Ala Gln Met Gly Met Arg Pro
130 135 140

Gly Ala Thr Asn Asn Gly Met His Pro Met His Ala Glu Ser Ser Leu
145 150 155 160

Gly Gly Gly Ser Ser Gly Gly Pro Pro Ser Ala Ser Gly Pro Gly Asp
165 170 175

Ile Arg Gly Gly Asn Lys Gln Asp Ala Ser Glu Ala Gly Thr Thr Gly
180 185 190

Ala Asp Gly Gln Gly Ser Ser Ala Gly Gly His Gly Gly Asp Gly Glu
195 200 205

Glu Ala Lys
210

<210> 99
<211> 597
<212> DNA
<213> Euphorbia esula

<400> 99
atgcagcagc aaccgcagat gatgcctatg atgccttcat atccaccagc aaacattacc 60

acggagcaaa tccaaaagta tcttgatgaa aataaaaaat tgattttggc gatcttggat 120

aatcaaaatc ttggaaaact cgctgagtgt gcacagtatc aagccctgct gcaaaaaaat 180

ctgatgtatt tagccgcaat tgctgatgca caaccccaga ccccacccat gccacctcag 240

atgtccccac atccggctat gcaacaagga gcatattaca tgcaacatcc tcaggctgca 300

gcagcagcaa tggctcatca gtcgggtatt ttcccaccaa agatgtctcc gttacaattc 360

aataatcctc atcaaataca ggacccccag cagttacatc aagcagccct ccaagggcaa 420

atgggaatga ggcccatggg gcccaataac gggatgcatc cgatgcaccc cgaggcaaat 480

cttggaggat ctaatgatgg tcgtggagga aacaaacagg atgctccgga gacgggagca 540

tcgggaggtg atgggcaagg caattctggt ggtgatgggg ctgaagatgg gaaatga 597

<210> 100
<211> 198
<212> PRT
<213> Euphorbia esula

<400> 100

```
Met Gln Gln Gln Pro Gln Met Met Pro Met Met Pro Ser Tyr Pro Pro
1               5                   10                  15

Ala Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Leu Leu Gln Lys Asn Leu Met Tyr Leu
    50                  55                  60

Ala Ala Ile Ala Asp Ala Gln Pro Gln Thr Pro Pro Met Pro Pro Gln
65                  70                  75                  80

Met Ser Pro His Pro Ala Met Gln Gln Gly Ala Tyr Tyr Met Gln His
                85                  90                  95

Pro Gln Ala Ala Ala Ala Ala Met Ala His Gln Ser Gly Ile Phe Pro
            100                 105                 110

Pro Lys Met Ser Pro Leu Gln Phe Asn Asn Pro His Gln Ile Gln Asp
        115                 120                 125

Pro Gln Gln Leu His Gln Ala Ala Leu Gln Gly Gln Met Gly Met Arg
    130                 135                 140

Pro Met Gly Pro Asn Asn Gly Met His Pro Met His Pro Glu Ala Asn
145                 150                 155                 160

Leu Gly Gly Ser Asn Asp Gly Arg Gly Gly Asn Lys Gln Asp Ala Pro
                165                 170                 175

Glu Thr Gly Ala Ser Gly Gly Asp Gly Gln Gly Asn Ser Gly Gly Asp
```

```
        180                 185                 190

Gly Ala Glu Asp Gly Lys
        195


<210>  101
<211>  642
<212>  DNA
<213>  Glycine max

<400>  101
atgcagcaga caccgccaat gattcctatg atgccttctt tcccacctac gaacataacc      60

accgagcaga ttcaaaaata ccttgatgag aacaagaagc tgattctggc aatattggac     120

aatcaaaatc ttggaaaact tgcagaatgt gcccagtacc aagctcagct tcaaaagaat     180

ttgatgtatt tagctgcaat tgctgatgcc cagcctcaaa ccccggccat gcctccgcag     240

atggcaccgc accctgccat gcaaccagga ttctatatgc aacatcctca ggctgctgca     300

gcagcaatgg ctcagcagca gcaaggaatg ttcccccaga aaatgccatt gcaatttggc     360

aatccacatc aaatgcagga acaacaacag cagctacacc agcaggccat ccaaggtcaa     420

atgggactta gacctggaga tataaataat ggcatgcatc caatgcacag tgaggctgct     480

cttggaggtg gaaacagcgg tggtccacct tcggctactg gtccaaacga tgcacgtggt     540

ggaagcaagc aagatgcctc tgaggctgga acagctggtg gagacggcca aggcagctcc     600

gcggctgctc ataacagtgg agatggtgaa gaggcaaagt ga                        642


<210>  102
<211>  213
<212>  PRT
<213>  Glycine max

<400>  102

Met Gln Gln Thr Pro Pro Met Ile Pro Met Met Pro Ser Phe Pro Pro
1               5                   10                  15


Thr Asn Ile Thr Thr Glu Gln Ile Gln Lys Tyr Leu Asp Glu Asn Lys
            20                  25                  30


Lys Leu Ile Leu Ala Ile Leu Asp Asn Gln Asn Leu Gly Lys Leu Ala
        35                  40                  45


Glu Cys Ala Gln Tyr Gln Ala Gln Leu Gln Lys Asn Leu Met Tyr Leu
    50                  55                  60


Ala Ala Ile Ala Asp Ala Gln Pro Gln Thr Pro Ala Met Pro Pro Gln
65                  70                  75                  80


Met Ala Pro His Pro Ala Met Gln Pro Gly Phe Tyr Met Gln His Pro
                85                  90                  95
```

Gln Ala Ala Ala Ala Ala Met Ala Gln Gln Gln Gln Gly Met Phe Pro
100 105 110

Gln Lys Met Pro Leu Gln Phe Gly Asn Pro His Gln Met Gln Glu Gln
115 120 125

Gln Gln Gln Leu His Gln Gln Ala Ile Gln Gly Gln Met Gly Leu Arg
130 135 140

Pro Gly Asp Ile Asn Asn Gly Met His Pro Met His Ser Glu Ala Ala
145 150 155 160

Leu Gly Gly Gly Asn Ser Gly Gly Pro Pro Ser Ala Thr Gly Pro Asn
165 170 175

Asp Ala Arg Gly Gly Ser Lys Gln Asp Ala Ser Glu Ala Gly Thr Ala
180 185 190

Gly Gly Asp Gly Gln Gly Ser Ser Ala Ala Ala His Asn Ser Gly Asp
195 200 205

Gly Glu Glu Ala Lys
210

<210> 103
<211> 633
<212> DNA
<213> Glycine soya

<400> 103
atgcagcaga caccgcctat gattcctatg atgccttcgt tcccacctac gaacataacc 60

accgagcaga ttcaaaaata ccttgatgag aacaagaagc tgattctggc aatattggac 120

aatcaaaatc ttggaaaact tgcagaatgt gcccagtacc aagctcagct tcaaaagaat 180

ttgatgtatt tagctgcaat tgctgatgcc cagcctcaaa caccagccat gcctccacag 240

atggcaccac accctgccat gcaaccagga ttctatatgc aacatcctca ggctgcagca 300

gcagcaatgg ctcagcagca gcagcaagga atgttccccc agaaaatgcc attgcaattt 360

ggcaatccac atcaaatgca ggaacaacag cagcagctac accagcaagc catccaaggt 420

caaatgggac tgagacctgg aggaataaat aatggcatgc atccaatgca caatgagggc 480

ggcaacagcg gtggtccacc ctcggctacc ggtccgaacg acgcacgtgg tggaagcaag 540

caagatgctt ctgaggctgg aacagctggt ggagatggcc aaggcagctc tgcagctgct 600

cataacagtg gagatggtga agaggcaaag tga 633

<210> 104
<211> 210

<212> PRT
<213> Glycine soya

<400> 104

Met Gln Gln Thr Pro Pro Met Ile Pro Met Met Pro Ser Phe Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Thr Pro Ala Met Pro Pro Gln
65 70 75 80

Met Ala Pro His Pro Ala Met Gln Pro Gly Phe Tyr Met Gln His Pro
85 90 95

Gln Ala Ala Ala Ala Ala Met Ala Gln Gln Gln Gln Gln Gly Met Phe
100 105 110

Pro Gln Lys Met Pro Leu Gln Phe Gly Asn Pro His Gln Met Gln Glu
115 120 125

Gln Gln Gln Gln Leu His Gln Gln Ala Ile Gln Gly Gln Met Gly Leu
130 135 140

Arg Pro Gly Gly Ile Asn Asn Gly Met His Pro Met His Asn Glu Gly
145 150 155 160

Gly Asn Ser Gly Gly Pro Pro Ser Ala Thr Gly Pro Asn Asp Ala Arg
165 170 175

Gly Gly Ser Lys Gln Asp Ala Ser Glu Ala Gly Thr Ala Gly Gly Asp
180 185 190

Gly Gln Gly Ser Ser Ala Ala Ala His Asn Ser Gly Asp Gly Glu Glu
195 200 205

Ala Lys
210

<210> 105
<211> 690
<212> DNA
<213> Gossypium hirsutum

<400> 105
atgcagcagc acctgatgca gatgcagccc atgatggcag cttattatcc caacaacgtc 60

actactgatc atattcaaca gtatctcgat gagaacaagt cattgatctt aaagattgtt 120

gagagccaga attctgggaa attgagtgaa tgtgctgaga accaagcaag gctgcagcga 180

aacctcatgt acctggctgc cattgcggat tctcaacccc aaccacccac cgtgcatgca 240

cagtttccat ctggtggtat catgcagcca ggagctgggc actacatgca gcaccaacaa 300

gctcaacaaa tgacacaaca gtcgcttatg gctgctcggt cctcaatgtt gtattctcag 360

caaccatttt ctgcactgca acaacaacag cagcaagctt tgcacagtca gcttggcatg 420

agctctggcg gaagcacagg ccttcatatg ctgcaaactg aatctagtac tgcaggtggc 480

agtggagcac ttggggccgg agggtttcct gattttggac gtggttcttc tggagaaggc 540

atccatggtg gcaggccaat ggcaggtgga agcaagcaag atatcgggag tgccggctca 600

gctgaaggtc gtggaggaag ctctggtggt cagggtggtg gtgatggggg tgaaaccctt 660

tacttaaaag cagccgatga tgggaactga 690

<210> 106
<211> 229
<212> PRT
<213> Gossypium hirsutum

<400> 106

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Val His Ala
65 70 75 80

Gln Phe Pro Ser Gly Gly Ile Met Gln Pro Gly Ala Gly His Tyr Met
85 90 95

Gln His Gln Gln Ala Gln Gln Met Thr Gln Gln Ser Leu Met Ala Ala
100 105 110

Arg Ser Ser Met Leu Tyr Ser Gln Gln Pro Phe Ser Ala Leu Gln Gln
115 120 125

Gln Gln Gln Gln Ala Leu His Ser Gln Leu Gly Met Ser Ser Gly Gly
    130                 135                 140

Ser Thr Gly Leu His Met Leu Gln Thr Glu Ser Ser Thr Ala Gly Gly
145                 150                 155                 160

Ser Gly Ala Leu Gly Ala Gly Gly Phe Pro Asp Phe Gly Arg Gly Ser
                165                 170                 175

Ser Gly Glu Gly Ile His Gly Gly Arg Pro Met Ala Gly Gly Ser Lys
            180                 185                 190

Gln Asp Ile Gly Ser Ala Gly Ser Ala Glu Gly Arg Gly Gly Ser Ser
        195                 200                 205

Gly Gly Gln Gly Gly Gly Asp Gly Gly Glu Thr Leu Tyr Leu Lys Ala
    210                 215                 220

Ala Asp Asp Gly Asn
225

<210> 107
<211> 642
<212> DNA
<213> Gossypium hirsutum

<400> 107
atgccgcagc caccgcaaat gattcctgtg atgccttcat atccacctac taatatcact 60

actgaacaga ttcagaagta ccttgatgag aataagaagt tgattttggc aattttggac 120

aatcagaatc ttggaaaact cgctgaatgc gcccagtatc aagctcagct gcaaaagaat 180

ttgatgtatt tagctgcaat tgcggatgct caacctcaat caacgccagc aatgtcgcct 240

cagatggcac cgcatccagc aatgcaaccc ggaggatatt ttatgcaaca tcctcaagct 300

gctgcaatgt cacagcaacc tggcatgtac cctcaaaagg tgccattgca attcaatagt 360

ccgcatcaaa tgcaggaccc tcagcacctc ctatatcagc agcatcaaca agcaatgcaa 420

ggtcaaatgg gaatcaggcc tgggggaccc aataatagca tgcatcccat gcattcagag 480

gctagccttg gaggcggcag cagtggtggt ccccctcaac cttcaggccc aagtgatgga 540

cgtgctggaa acaagcaaga gggctccgaa gctggtggta atgggcaggg cagcacaact 600

ggtgggcatg gtggcggtga tggagcggat gaggcaaagt ga 642

<210> 108
<211> 213
<212> PRT
<213> Gossypium hirsutum

<400> 108

Met Pro Gln Pro Pro Gln Met Ile Pro Val Met Pro Ser Tyr Pro Pro
1               5                   10                  15

Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Gln Leu Gln Lys Asn Leu Met Tyr Leu
    50                  55                  60

Ala Ala Ile Ala Asp Ala Gln Pro Gln Ser Thr Pro Ala Met Ser Pro
65                  70                  75                  80

Gln Met Ala Pro His Pro Ala Met Gln Pro Gly Gly Tyr Phe Met Gln
                85                  90                  95

His Pro Gln Ala Ala Ala Met Ser Gln Gln Pro Gly Met Tyr Pro Gln
            100                 105                 110

Lys Val Pro Leu Gln Phe Asn Ser Pro His Gln Met Gln Asp Pro Gln
        115                 120                 125

His Leu Leu Tyr Gln Gln His Gln Gln Ala Met Gln Gly Gln Met Gly
    130                 135                 140

Ile Arg Pro Gly Gly Pro Asn Asn Ser Met His Pro Met His Ser Glu
145                 150                 155                 160

Ala Ser Leu Gly Gly Gly Ser Ser Gly Gly Pro Pro Gln Pro Ser Gly
                165                 170                 175

Pro Ser Asp Gly Arg Ala Gly Asn Lys Gln Glu Gly Ser Glu Ala Gly
            180                 185                 190

Gly Asn Gly Gln Gly Ser Thr Thr Gly Gly His Gly Gly Gly Asp Gly
        195                 200                 205

Ala Asp Glu Ala Lys
    210

<210> 109
<211> 561
<212> DNA
<213> Hordeum vulgare

<400> 109
atgcagcaag cgatgcccat gccgccggcg gcggcggcgc ctgggatgcc tccttctgcc 60

ggcctcagca ccgagcagat ccaaaagtac ctggatgaaa ataaacaact aattttggct 120

atcttggaaa atcagaacct gggaaagttg gcggaatgtg ctcagtatca agctcagctt 180

cagaagaatc ttttgtattt ggctgcgatt gctgatactc agccacagac ctctgtaagc 240

cgtcctcaga tggcaccacc tgctgcatcc ccaggggcag ggcattacat gtcacaggtg 300

ccaatgttcc ctccgaggac ccctctaacg cctcagcaga tgcaggagca gcaactacag 360

caacaacagg ctcagatgct tccgtttgct ggtcaaatgg ttgcgagacc cggggctgtc 420

aatggcattc cccaggcccc tcaagttgaa caaccagcct atgcagcagg tggggccagt 480

tccgagcctt ctggcaccga gagccacagg agcactggcg ccgataacga tggtgggagc 540

ggcttggctg accagtccta a 561

<210> 110
<211> 186
<212> PRT
<213> Hordeum vulgare

<400> 110

Met Gln Gln Ala Met Pro Met Pro Pro Ala Ala Ala Ala Pro Gly Met
1 5 10 15

Pro Pro Ser Ala Gly Leu Ser Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Thr Gln Pro Gln Thr Ser Val Ser
65 70 75 80

Arg Pro Gln Met Ala Pro Pro Ala Ala Ser Pro Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln Leu Gln Gln Gln Gln Ala Gln Met Leu Pro
115 120 125

Phe Ala Gly Gln Met Val Ala Arg Pro Gly Ala Val Asn Gly Ile Pro
130 135 140

Gln Ala Pro Gln Val Glu Gln Pro Ala Tyr Ala Ala Gly Gly Ala Ser
145 150 155 160

Ser Glu Pro Ser Gly Thr Glu Ser His Arg Ser Thr Gly Ala Asp Asn
165 170 175

Asp Gly Gly Ser Gly Leu Ala Asp Gln Ser
180 185

<210> 111
<211> 555
<212> DNA
<213> Lactuca serriola

<220>
<221> misc_feature
<222> (253)..(253)
<223> n is a, c, g, or t

<400> 111
atgaagcagc cgatgatgcc gaatccaatg atgtcttctt cgtttcctcc tacaaacatc 60

accaccgatc agatccaaaa gttccttgat gaaaacaagc aactaattat agcaataatg 120

agcaacctaa atcttggaaa gcttgctgaa tgtgcccagt accaagctct actccaaaaa 180

aatttgatgt atctagcagc cattgcagat gctcaaccac ctacacctac accaacacta 240

aatatctctt atnagatggg cccggttcca catccaggga tgccacagca aggtggattt 300

tacatggcgc agcagcaccc tcaggcggct gtaatgacgg ctcagccacc ttctggtttt 360

ccacaaccga tgcctggtat gcaatttaac agcccacagg ctattcaagg gcagatgggc 420

gggaggtccg gtgggccgcc aagctcagcc gctagtgatg tctggagagg aagcatgcaa 480

gatggtggtg gtggtgctgc tgctgatggt ggtaaggatg gtcatgctgg cggtggacct 540

gaggaagcaa agtaa 555

<210> 112
<211> 184
<212> PRT
<213> Lactuca serriola

<220>
<221> UNSURE
<222> (85)..(85)
<223> Xaa can be any naturally occurring amino acid

<400> 112

Met Lys Gln Pro Met Met Pro Asn Pro Met Met Ser Ser Ser Phe Pro
1 5 10 15

Pro Thr Asn Ile Thr Thr Asp Gln Ile Gln Lys Phe Leu Asp Glu Asn
20 25 30

Lys Gln Leu Ile Ile Ala Ile Met Ser Asn Leu Asn Leu Gly Lys Leu
35 40 45

Ala Glu Cys Ala Gln Tyr Gln Ala Leu Leu Gln Lys Asn Leu Met Tyr
    50                  55                  60

Leu Ala Ala Ile Ala Asp Ala Gln Pro Pro Thr Pro Thr Pro Thr Leu
65                  70                  75                  80

Asn Ile Ser Tyr Xaa Met Gly Pro Val Pro His Pro Gly Met Pro Gln
                85                  90                  95

Gln Gly Gly Phe Tyr Met Ala Gln Gln His Pro Gln Ala Ala Val Met
            100                 105                 110

Thr Ala Gln Pro Pro Ser Gly Phe Pro Gln Pro Met Pro Gly Met Gln
        115                 120                 125

Phe Asn Ser Pro Gln Ala Ile Gln Gly Gln Met Gly Gly Arg Ser Gly
    130                 135                 140

Gly Pro Pro Ser Ser Ala Ala Ser Asp Val Trp Arg Gly Ser Met Gln
145                 150                 155                 160

Asp Gly Gly Gly Gly Ala Ala Ala Asp Gly Gly Lys Asp Gly His Ala
                165                 170                 175

Gly Gly Gly Pro Glu Glu Ala Lys
            180

<210> 113
<211> 627
<212> DNA
<213> Lycopersicon esculentum

<400> 113
atgcagcagc acctgatgca gatgcagccc atgatggcag cttactatcc aacgaacgtc 60

actactgacc atattcaaca gtatttggat gaaaacaaat cactcattct gaagattgtt 120

gagagccaga actctgggaa actcagtgaa tgtgcggaga accaagctag gcttcagagg 180

aatctgatgt accttgctgc gattgctgat tcacaacctc aaccttctag catgcattct 240

cagttctctt ctggtgggat gatgcagcca gggacacaca gttacttgca gcagcagcag 300

cagcaacaac aagcgcaaca aatggcaaca caacaactca tggctgcaag atcctcgtcg 360

atgctctatg gacaacagca gcagcaatct cagttatcgc aatatcaaca aggcttgcat 420

agtagccaac tcggcatgag ttctggcagt ggcggaagca ctggacttca tcacatgctt 480

caaagtgaat catcacctca tggtggtggt ttctctcatg acttcggccg cgcaaataag 540

caagacattg ggagtagtat gtctgctgaa gggcgcggcg gaagttcagg tggtgagaat 600

ctttatctga aagcttctga ggattga 627

<210> 114
<211> 208
<212> PRT
<213> Lycopersicon esculentum

<400> 114

Met Gln Gln His Leu Met Gln Met Gln Pro Met Met Ala Ala Tyr Tyr
1 5 10 15

Pro Thr Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Ser Ser Met His Ser
65 70 75 80

Gln Phe Ser Ser Gly Gly Met Met Gln Pro Gly Thr His Ser Tyr Leu
85 90 95

Gln Gln Gln Gln Gln Gln Gln Gln Ala Gln Gln Met Ala Thr Gln Gln
100 105 110

Leu Met Ala Ala Arg Ser Ser Ser Met Leu Tyr Gly Gln Gln Gln Gln
115 120 125

Gln Ser Gln Leu Ser Gln Tyr Gln Gln Gly Leu His Ser Ser Gln Leu
130 135 140

Gly Met Ser Ser Gly Ser Gly Gly Ser Thr Gly Leu His His Met Leu
145 150 155 160

Gln Ser Glu Ser Ser Pro His Gly Gly Gly Phe Ser His Asp Phe Gly
165 170 175

Arg Ala Asn Lys Gln Asp Ile Gly Ser Ser Met Ser Ala Glu Gly Arg
180 185 190

Gly Gly Ser Ser Gly Gly Glu Asn Leu Tyr Leu Lys Ala Ser Glu Asp
195 200 205

<210> 115
<211> 624
<212> DNA
<213> Malus domestica

<400> 115
atgcagcagc caccacaaat gatccccgtc atgccttcat ttcctcccac caacatcacc 60

accgaacaaa ttcagaagta ccttgatgac aacaaaaagt tgattctggc aatattggat 120

aatcaaaatc ttggaaaact tgctgagtgt gctcagtacc aggctctgct tcaaaagaat 180

ctgatgtatt tagcagcaat tgccgatgcg caaccacagg caccagctgc ccctccccag 240

atggccccac atcctgctat gcaacaggca ggatattaca tgcaacatcc tcaggcagca 300

gcaatggctc agcaacaggg tattttctcc ccaaagatgc cgatgcaatt caataacatg 360

catcaaatgc acgatccaca gcagcaccaa caagccatgc aagggcaaat gggaatgaga 420

cctggagggc ctaacggcat gccttccatg cttcatactg aggccacaca tggtggtggt 480

agtggcggcc caaattcagc tggagaccca aatgatgggc gtggaggaag caagcaagac 540

gcctctgagt ctggggcagg tggtgatggc caggggacct cagccggcgg gcgtggaact 600

ggtgatggag aggacggcaa gtga 624

<210> 116
<211> 207
<212> PRT
<213> Malus domestica

<400> 116

Met Gln Gln Pro Pro Gln Met Ile Pro Val Met Pro Ser Phe Pro Pro
1               5                   10                  15

Thr Asn Ile Thr Thr Glu Gln Ile Gln Lys Tyr Leu Asp Asp Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Leu Leu Gln Lys Asn Leu Met Tyr Leu
    50                  55                  60

Ala Ala Ile Ala Asp Ala Gln Pro Gln Ala Pro Ala Ala Pro Pro Gln
65                  70                  75                  80

Met Ala Pro His Pro Ala Met Gln Gln Ala Gly Tyr Tyr Met Gln His
                85                  90                  95

Pro Gln Ala Ala Ala Met Ala Gln Gln Gln Gly Ile Phe Ser Pro Lys
            100                 105                 110

Met Pro Met Gln Phe Asn Asn Met His Gln Met His Asp Pro Gln Gln
        115                 120                 125

His Gln Gln Ala Met Gln Gly Gln Met Gly Met Arg Pro Gly Gly Pro
    130                 135                 140

```
Asn Gly Met Pro Ser Met Leu His Thr Glu Ala Thr His Gly Gly Gly
145                 150                 155                 160

Ser Gly Gly Pro Asn Ser Ala Gly Asp Pro Asn Asp Gly Arg Gly Gly
                165                 170                 175

Ser Lys Gln Asp Ala Ser Glu Ser Gly Ala Gly Gly Asp Gly Gln Gly
            180                 185                 190

Thr Ser Ala Gly Gly Arg Gly Thr Gly Asp Gly Glu Asp Gly Lys
        195                 200                 205
```

<210> 117
<211> 639
<212> DNA
<213> Medicago trunculata

<400> 117

```
atgcagcaga cacctcaaat gattcctatg atgccttcat tcccacaaca aacaaacata      60

accactgagc agattcaaaa atatcttgat gagaacaaga agctgatcct ggcaatattg     120

gacaatcaaa atcttggaaa acttgcagaa tgtgcccagt accaagctca gcttcagaag     180

aatttgatgt atttagctgc aattgctgac gcgcagccac aaacaccggc cttgcctcca     240

cagatggccc cgcaccctgc gatgcaacaa ggattctata tgcaacatcc tcaggctgca     300

gcaatggctc agcaacaagg aatgttcccc caaaaaatgc caatgcagtt cggtaatccg     360

catcaaatgc aggatcagca gcatcagcag caacaacagc agctacatca gcaagctatg     420

caaggtcaaa tgggacttag acctggaggg ataaataacg gcatgcatcc aatgcacaac     480

gaggctgctc tcggaggtag cggcagtggt ggtcaaatga cgggcgtggt ggtggagcaa     540

gcaagatgct tcggagctgg gacagccggc ggtgatggtc aaggaacctc tgccgcagct     600

gcgcacaaca gtggagatgc ttcagaagaa ggaaagtaa                            639
```

<210> 118
<211> 213
<212> PRT
<213> Medicago trunculata

<400> 118

```
Met Gln Gln Thr Pro Gln Met Ile Pro Met Met Pro Ser Phe Pro Gln
1               5                   10                  15

Gln Thr Asn Ile Thr Thr Glu Gln Ile Gln Lys Tyr Leu Asp Glu Asn
            20                  25                  30

Lys Lys Leu Ile Leu Ala Ile Leu Asp Asn Gln Asn Leu Gly Lys Leu
        35                  40                  45
```

Ala Glu Cys Ala Gln Tyr Gln Ala Gln Leu Gln Lys Asn Leu Met Tyr
    50                  55                  60

Leu Ala Ala Ile Ala Asp Ala Gln Pro Gln Thr Pro Ala Leu Pro Pro
65                  70                  75                  80

Gln Met Ala Pro His Pro Ala Met Gln Gln Gly Phe Tyr Met Gln His
                85                  90                  95

Pro Gln Ala Ala Ala Met Ala Gln Gln Gln Gly Met Phe Pro Gln Lys
            100                 105                 110

Met Pro Met Gln Phe Gly Asn Pro His Gln Met Gln Asp Gln Gln His
        115                 120                 125

Gln Gln Gln Gln Gln Gln Leu His Gln Gln Ala Met Gln Gly Gln Met
    130                 135                 140

Gly Leu Arg Pro Gly Gly Ile Asn Asn Gly Met His Pro Met His Asn
145                 150                 155                 160

Glu Ala Ala Leu Gly Gly Ser Gly Ser Gly Gly Pro Asn Asp Gly Arg
                165                 170                 175

Gly Gly Gly Ser Lys Gln Asp Ala Ser Glu Ala Gly Thr Ala Gly Gly
            180                 185                 190

Asp Gly Gln Gly Thr Ser Ala Ala Ala Ala His Asn Ser Gly Asp Ala
        195                 200                 205

Ser Glu Glu Gly Lys
    210

<210> 119
<211> 624
<212> DNA
<213> Panicum virgatum

<400> 119
atgcagcagc agatgcccat gcagtcggcg cccccggcga ccggcatcac caccgagcag 60

atccaaaagt atttggatga aaataagcag cttattttgg ccatcctgga aaatcagaac 120

ttaggaaagt tggctgaatg tgctcagtat caagctcagc ttcaaaagaa tctcttgtac 180

ctggctgcga ttgcagatgc ccaaccccaa ccaccacaga accctgcaag tcgcccacag 240

atgatgcaac ctggcatggt accaggtgca gggcattaca tgtcccaagt accaatgttc 300

ccgccaagaa caccattaac cccgcaacag atgcaagaac agcagcagca gcagcagcag 360

cttcaacagc agcaagcaca ggctcttgct ttcccgggac agatggtcat gagacctacc 420

attaatggca tgcagcctat gcaagccgac cctgctgccg ccgccgccag cctacagcag 480

tcagcacctg gccctactga tgggcgagga ggcaagcaag atgcaactgc tggggtgagc 540

acagagcctt ctggcaccga gagccacaag agcacaaccg cagcagatca cgatgtgggc 600

actgatgtcg cggagaaatc ctaa 624

<210> 120
<211> 207
<212> PRT
<213> Panicum virgatum

<400> 120

```
Met Gln Gln Gln Met Pro Met Gln Ser Ala Pro Pro Ala Thr Gly Ile
1               5                   10                  15

Thr Thr Glu Gln Ile Gln Lys Tyr Leu Asp Glu Asn Lys Gln Leu Ile
            20                  25                  30

Leu Ala Ile Leu Glu Asn Gln Asn Leu Gly Lys Leu Ala Glu Cys Ala
        35                  40                  45

Gln Tyr Gln Ala Gln Leu Gln Lys Asn Leu Leu Tyr Leu Ala Ala Ile
    50                  55                  60

Ala Asp Ala Gln Pro Gln Pro Pro Gln Asn Pro Ala Ser Arg Pro Gln
65                  70                  75                  80

Met Met Gln Pro Gly Met Val Pro Gly Ala Gly His Tyr Met Ser Gln
                85                  90                  95

Val Pro Met Phe Pro Pro Arg Thr Pro Leu Thr Pro Gln Gln Met Gln
            100                 105                 110

Glu Gln Gln Gln Gln Gln Gln Gln Leu Gln Gln Gln Gln Ala Gln Ala
        115                 120                 125

Leu Ala Phe Pro Gly Gln Met Val Met Arg Pro Thr Ile Asn Gly Met
    130                 135                 140

Gln Pro Met Gln Ala Asp Pro Ala Ala Ala Ala Ala Ser Leu Gln Gln
145                 150                 155                 160

Ser Ala Pro Gly Pro Thr Asp Gly Arg Gly Gly Lys Gln Asp Ala Thr
                165                 170                 175

Ala Gly Val Ser Thr Glu Pro Ser Gly Thr Glu Ser His Lys Ser Thr
            180                 185                 190

Thr Ala Ala Asp His Asp Val Gly Thr Asp Val Ala Glu Lys Ser
```

195 200 205

<210> 121
<211> 747
<212> DNA
<213> Picea sitchensis

<400> 121
atgcagcagc atctcatgca aatgcagccc atgatggcgg catacgcctc caacaacatc 60

accactgatc acatccagaa gtacctggat gagaacaagc agttgattct ggcaattctg 120

gacaaccaaa atcttggaaa gctcaatgag tgtgctcagt accaagcaaa acttcagcag 180

aatttgatgt atctggctgc gattgctgat tctcaaccac aagcacaaac tgcacatgct 240

cagattcctc ctaatgcagt gatgcagtct ggtgggcatt acatgcagca ccagcaggca 300

cagcaacaag tgactcctca gtctctgatg gcagctagat cttccatgct gtattctcag 360

cagccgatgg ctgctttgca tcaagctcag caacaacagc agcagcagca tcagcagcaa 420

caacaatctc ttcacagcca gcttggcata aattctggag gaagcagtgg attgcatatg 480

ttgcatggtg agacaaacat gggatgtaat gggcctctct catctggggg cttccctgaa 540

tttgggcgtg ggtctgctac ctctgctgaa ggtatgcagg ccaacagggg cttcactata 600

gatcgtggtt caaataagca ggatggagta ggatcagaga atgcccatcc aggtgctggt 660

gatggaagag ggagttcaac tggagggcag aatgcagatg agtcagaacc atcatacctg 720

aaagcctccg aagaagaagg aaactag 747

<210> 122
<211> 248
<212> PRT
<213> Picea sitchensis

<400> 122

Met Gln Gln His Leu Met Gln Met Gln Pro Met Met Ala Ala Tyr Ala
1 5 10 15

Ser Asn Asn Ile Thr Thr Asp His Ile Gln Lys Tyr Leu Asp Glu Asn
20 25 30

Lys Gln Leu Ile Leu Ala Ile Leu Asp Asn Gln Asn Leu Gly Lys Leu
35 40 45

Asn Glu Cys Ala Gln Tyr Gln Ala Lys Leu Gln Gln Asn Leu Met Tyr
50 55 60

Leu Ala Ala Ile Ala Asp Ser Gln Pro Gln Ala Gln Thr Ala His Ala
65 70 75 80

Gln Ile Pro Pro Asn Ala Val Met Gln Ser Gly Gly His Tyr Met Gln
85 90 95

His Gln Gln Ala Gln Gln Gln Val Thr Pro Gln Ser Leu Met Ala Ala
100 105 110

Arg Ser Ser Met Leu Tyr Ser Gln Gln Pro Met Ala Ala Leu His Gln
115 120 125

Ala Gln Gln Gln Gln Gln Gln Gln His Gln Gln Gln Gln Gln Ser Leu
130 135 140

His Ser Gln Leu Gly Ile Asn Ser Gly Gly Ser Ser Gly Leu His Met
145 150 155 160

Leu His Gly Glu Thr Asn Met Gly Cys Asn Gly Pro Leu Ser Ser Gly
165 170 175

Gly Phe Pro Glu Phe Gly Arg Gly Ser Ala Thr Ser Ala Glu Gly Met
180 185 190

Gln Ala Asn Arg Gly Phe Thr Ile Asp Arg Gly Ser Asn Lys Gln Asp
195 200 205

Gly Val Gly Ser Glu Asn Ala His Pro Gly Ala Gly Asp Gly Arg Gly
210 215 220

Ser Ser Thr Gly Gly Gln Asn Ala Asp Glu Ser Glu Pro Ser Tyr Leu
225 230 235 240

Lys Ala Ser Glu Glu Glu Gly Asn
245

<210> 123
<211> 735
<212> DNA
<213> Pinus taeda

<400> 123
atgcagcagc acctcatgca aatgcagccc atgatggcgg cctacgcctc caacaatatc 60

accactgatc acatccagaa gtacctggat gagaacaagc agttgattct ggcaattttg 120

gacaaccaaa atctcggaaa gctcaatgag tgtgctcaat accaagcaaa acttcagcag 180

aatttgatgt atctggctgc tattgctgat tctcaacctc aagcacaaac tgcacatgct 240

cagattcctc caaatgcggt gatgcagtct ggtgggcatt acatgcagca tcaacaggca 300

cagcaacaag ttactcctca gtctctgatg gcagctagat cttccatact gtatgctcag 360

caacaacagc agcagcagca tcagcagcat cagcagcaac agcagcaaca acagtctctt 420

cacagccagc ttggcataaa ttctggagga agcagcggtt tgcatatgtt gcatggtgag 480

acaaacatgg gatgtaatgg gcctctgtca tctgggggat tccctgaatt tgggcgtggg 540

tctgctacct ctgctgatgg tatgcaggtg aacaggggct ttgctataga tcgtggttca 600

aacaagcagg atggagttgg atcagagaat gcccatgctg gtgctggtga tggaagaggg 660

agttcaactg gagggcagaa tgcagatgag tcagaaccat catacctgaa ggcctccgag 720

gaagaaggaa actag 735

<210> 124
<211> 244
<212> PRT
<213> Pinus taeda

<400> 124

Met Gln Gln His Leu Met Gln Met Gln Pro Met Met Ala Ala Tyr Ala
1 5 10 15

Ser Asn Asn Ile Thr Thr Asp His Ile Gln Lys Tyr Leu Asp Glu Asn
20 25 30

Lys Gln Leu Ile Leu Ala Ile Leu Asp Asn Gln Asn Leu Gly Lys Leu
35 40 45

Asn Glu Cys Ala Gln Tyr Gln Ala Lys Leu Gln Gln Asn Leu Met Tyr
50 55 60

Leu Ala Ala Ile Ala Asp Ser Gln Pro Gln Ala Gln Thr Ala His Ala
65 70 75 80

Gln Ile Pro Pro Asn Ala Val Met Gln Ser Gly Gly His Tyr Met Gln
85 90 95

His Gln Gln Ala Gln Gln Gln Val Thr Pro Gln Ser Leu Met Ala Ala
100 105 110

Arg Ser Ser Ile Leu Tyr Ala Gln Gln Gln Gln Gln Gln Gln His Gln
115 120 125

Gln His Gln Gln Gln Gln Gln Gln Gln Gln Ser Leu His Ser Gln Leu
130 135 140

Gly Ile Asn Ser Gly Gly Ser Ser Gly Leu His Met Leu His Gly Glu
145 150 155 160

Thr Asn Met Gly Cys Asn Gly Pro Leu Ser Ser Gly Gly Phe Pro Glu
165 170 175

Phe Gly Arg Gly Ser Ala Thr Ser Ala Asp Gly Met Gln Val Asn Arg
180 185 190

Gly Phe Ala Ile Asp Arg Gly Ser Asn Lys Gln Asp Gly Val Gly Ser
195 200 205

Glu Asn Ala His Ala Gly Ala Gly Asp Gly Arg Gly Ser Ser Thr Gly
210 215 220

Gly Gln Asn Ala Asp Glu Ser Glu Pro Ser Tyr Leu Lys Ala Ser Glu
225 230 235 240

Glu Glu Gly Asn

<210> 125
<211> 663
<212> DNA
<213> Populus tremula

<400> 125
atgcaacagc acctgatgca gatgcagccc atgatggcag cctattaccc cagcaacgtc 60

actactgatc atattcaaca gtatctggac gaaaacaagt cattgatttt gaagattgtt 120

gagagccaga attcagggaa actcagtgag tgtgcagaga accaagcaag actgcaacaa 180

aatctcatgt acttggctgc aattgctgat tgtcagcccc aaccacctac catgcatgcc 240

cagttccctt ccagcggcat tatgcagcca ggagcacatt acatgcagca tcaacaagct 300

caacagatga caccacaagc ccttatggct gcacgctctt ctatgctgca gtatgctcaa 360

cagccattct cagcgcttca acaacagcaa gccttacaca gccagctcgg catgagctct 420

ggtggaagcg caggacttca tatgatgcaa agcgaggcta acactgcagg aggcagtgga 480

gctcttggtg ctggacgatt tcctgatttt ggcatggatg cctccagtag aggaatcgca 540

agtgggagca agcaagatat tcggagtgca gggtctagtg aagggcgagg aggaagctct 600

ggaggccagg gtggtgatgg aggtgaaacc ctttacttga aatctgctga tgatgggaac 660

tga 663

<210> 126
<211> 220
<212> PRT
<213> Populus tremula

<400> 126

Met Gln Gln His Leu Met Gln Met Gln Pro Met Met Ala Ala Tyr Tyr
1 5 10 15

Pro Ser Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Gln Asn Leu Met Tyr
    50                  55                  60

Leu Ala Ala Ile Ala Asp Cys Gln Pro Gln Pro Pro Thr Met His Ala
65                  70                  75                  80

Gln Phe Pro Ser Ser Gly Ile Met Gln Pro Gly Ala His Tyr Met Gln
                85                  90                  95

His Gln Gln Ala Gln Gln Met Thr Pro Gln Ala Leu Met Ala Ala Arg
            100                 105                 110

Ser Ser Met Leu Gln Tyr Ala Gln Gln Pro Phe Ser Ala Leu Gln Gln
        115                 120                 125

Gln Gln Ala Leu His Ser Gln Leu Gly Met Ser Ser Gly Gly Ser Ala
    130                 135                 140

Gly Leu His Met Met Gln Ser Glu Ala Asn Thr Ala Gly Gly Ser Gly
145                 150                 155                 160

Ala Leu Gly Ala Gly Arg Phe Pro Asp Phe Gly Met Asp Ala Ser Ser
                165                 170                 175

Arg Gly Ile Ala Ser Gly Ser Lys Gln Asp Ile Arg Ser Ala Gly Ser
            180                 185                 190

Ser Glu Gly Arg Gly Gly Ser Ser Gly Gly Gln Gly Gly Asp Gly Gly
        195                 200                 205

Glu Thr Leu Tyr Leu Lys Ser Ala Asp Asp Gly Asn
    210                 215                 220

<210> 127
<211> 678
<212> DNA
<213> Saccharum officinarum

<400> 127
atgcagcagc aacacctgat gcagatgaac cagaacatga ttgggggcta cacctctcct 60

gccgctgtga caaccgatct catccagcag tacctggatg agaacaagca gctgatcctg 120

gccatcctcg acaaccagaa caatggcaag gtggaggagt gcgaacggca ccaagctaag 180

ctccagcaca acctcatgta cctggccgcc atcgccgaca gccagccacc acagactgca 240

ccactatcac aatacccgtc caacctgatg atgcagccgg gccctcggta catgccaccg 300

cagtccgggc agatgatgag cccgcagtcg ctaatggcgg cgcggtcctc catgatgtac 360

gcgcacccgt ccatgtcacc actccagcag cagcaggcag cgcacgggca gctgggcatg 420

gcttcagggg gcggcggtgg cacgaccagt gggttcaaca tcctccatgg cgaggccagt 480

atgggcggtg ctggtggcgc ttgtgccggc aacaacatga tgaacgccgg catgttctca 540

ggctttggcc gcagcggcag tggcgccaag gagggatcga cctcgctgtc ggttgacgtc 600

cgtggtggca ccagctccgg cgcgcaaagc ggggacggcg agtacctgaa agcaggcacc 660

gaggaagaag gcagttaa 678

<210> 128
<211> 225
<212> PRT
<213> Saccharum officinarum

<400> 128

Met Gln Gln Gln His Leu Met Gln Met Asn Gln Asn Met Ile Gly Gly
1 5 10 15

Tyr Thr Ser Pro Ala Ala Val Thr Thr Asp Leu Ile Gln Gln Tyr Leu
20 25 30

Asp Glu Asn Lys Gln Leu Ile Leu Ala Ile Leu Asp Asn Gln Asn Asn
35 40 45

Gly Lys Val Glu Glu Cys Glu Arg His Gln Ala Lys Leu Gln His Asn
50 55 60

Leu Met Tyr Leu Ala Ala Ile Ala Asp Ser Gln Pro Pro Gln Thr Ala
65 70 75 80

Pro Leu Ser Gln Tyr Pro Ser Asn Leu Met Met Gln Pro Gly Pro Arg
85 90 95

Tyr Met Pro Pro Gln Ser Gly Gln Met Met Ser Pro Gln Ser Leu Met
100 105 110

Ala Ala Arg Ser Ser Met Met Tyr Ala His Pro Ser Met Ser Pro Leu
115 120 125

Gln Gln Gln Gln Ala Ala His Gly Gln Leu Gly Met Ala Ser Gly Gly
130 135 140

Gly Gly Gly Thr Thr Ser Gly Phe Asn Ile Leu His Gly Glu Ala Ser
145 150 155 160

Met Gly Gly Ala Gly Gly Ala Cys Ala Gly Asn Asn Met Met Asn Ala
165 170 175

Gly Met Phe Ser Gly Phe Gly Arg Ser Gly Ser Gly Ala Lys Glu Gly
180 185 190

Ser Thr Ser Leu Ser Val Asp Val Arg Gly Gly Thr Ser Ser Gly Ala
195 200 205

Gln Ser Gly Asp Gly Glu Tyr Leu Lys Ala Gly Thr Glu Glu Glu Gly
210 215 220

Ser
225

<210> 129
<211> 561
<212> DNA
<213> Saccharum officinarum

<400> 129
atgcagcagc cgatgcccat gcagccgcag gcgccggaga tgaccccggc cgccggaatc 60

accacggagc agatccaaaa gtatctggat gagaataagc agcttatttt ggctattttg 120

gaaaatcaga acctaggaaa attggcagaa tgtgctcagt atcaatcaca acttcagaag 180

aacctcttgt atctcgctgc aatcgcagat gcccaaccac agactgctgt aagccgccct 240

cagatggcgc cgcctggtgc attgcctgga gtagggcagt acatgtcaca ggtgcctatg 300

ttcccaccga ggacacctct aacaccccag cagatgcagg agcagcaact tcagcagcag 360

caggctcagc tgctaaattt cagtggccta atggttgcta gacctggcat ggtcaacggc 420

atgcctcagt ccattcaagt tcagcaagct cagccaccac cagcagggaa caaacaggat 480

gctggtgggg tcgcctcgga gccctcgggc attgagaacc acaggagcac tggtggtgat 540

aatgatggtg gaagcgacta g 561

<210> 130
<211> 186
<212> PRT
<213> Saccharum officinarum

<400> 130

Met Gln Gln Pro Met Pro Met Gln Pro Gln Ala Pro Glu Met Thr Pro
1 5 10 15

Ala Ala Gly Ile Thr Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65 70 75 80

```
Gln Met Ala Pro Pro Gly Ala Leu Pro Gly Val Gly Gln Tyr Met Ser
                85                  90                  95

Gln Val Pro Met Phe Pro Pro Arg Thr Pro Leu Thr Pro Gln Gln Met
            100                 105                 110

Gln Glu Gln Gln Leu Gln Gln Gln Gln Ala Gln Leu Leu Asn Phe Ser
        115                 120                 125

Gly Leu Met Val Ala Arg Pro Gly Met Val Asn Gly Met Pro Gln Ser
    130                 135                 140

Ile Gln Val Gln Gln Ala Gln Pro Pro Pro Ala Gly Asn Lys Gln Asp
145                 150                 155                 160

Ala Gly Gly Val Ala Ser Glu Pro Ser Gly Ile Glu Asn His Arg Ser
                165                 170                 175

Thr Gly Gly Asp Asn Asp Gly Gly Ser Asp
            180                 185
```

```
<210>  131
<211>  642
<212>  DNA
<213>  Saccharum officinarum

<400>  131
atgcagcagc agatgcccat gccgccggcg cccgctgcgg cggcggcgcc cccggcggcc      60

ggcatcacca ccgagcagat ccaaaagtat ttggacgaaa ataagcaact tattttggcc     120

atcctggaaa atcagaactt aggaaagttg gctgaatgtg ctcagtatca agctcaactt     180

caaaagaacc tcttgtacct ggctgcgatt gctgatgccc aaccccagcc accacaaaac     240

cctgcaggtc gccctcagat gatgcaacct ggtatagtgc caggtgcggg gcattacatg     300

tcacaagtac caatgttccc tccaagaact ccattaaccc cacagcagat gcaagagcag     360

cagcagcaac agcttcagca gcagcaagcg caggctctta cattccctgg acagatggtc     420

atgagaccag ctaccatcaa cggcatacag cagcctatgc aagctgaccc tgcccgggca     480

gcggagctgc aacaaccacc acctatccca gctgacgggc gagtaagcaa gcagcaggac     540

acaacggctg gcgtgagctc agagccttct gccaatgaga gccacaagac cacaactgga     600

gcagatagtg aggcaggtgg tgacgtggcg gagaaatcct aa                        642

<210>  132
<211>  213
<212>  PRT
<213>  Saccharum officinarum

<400>  132
```

Met Gln Gln Gln Met Pro Met Pro Pro Ala Pro Ala Ala Ala Ala Ala
1               5                   10                  15

Pro Pro Ala Ala Gly Ile Thr Thr Glu Gln Ile Gln Lys Tyr Leu Asp
            20                  25                  30

Glu Asn Lys Gln Leu Ile Leu Ala Ile Leu Glu Asn Gln Asn Leu Gly
        35                  40                  45

Lys Leu Ala Glu Cys Ala Gln Tyr Gln Ala Gln Leu Gln Lys Asn Leu
    50                  55                  60

Leu Tyr Leu Ala Ala Ile Ala Asp Ala Gln Pro Gln Pro Pro Gln Asn
65                  70                  75                  80

Pro Ala Gly Arg Pro Gln Met Met Gln Pro Gly Ile Val Pro Gly Ala
                85                  90                  95

Gly His Tyr Met Ser Gln Val Pro Met Phe Pro Pro Arg Thr Pro Leu
            100                 105                 110

Thr Pro Gln Gln Met Gln Glu Gln Gln Gln Gln Gln Leu Gln Gln Gln
        115                 120                 125

Gln Ala Gln Ala Leu Thr Phe Pro Gly Gln Met Val Met Arg Pro Ala
    130                 135                 140

Thr Ile Asn Gly Ile Gln Gln Pro Met Gln Ala Asp Pro Ala Arg Ala
145                 150                 155                 160

Ala Glu Leu Gln Gln Pro Pro Pro Ile Pro Ala Asp Gly Arg Val Ser
                165                 170                 175

Lys Gln Gln Asp Thr Thr Ala Gly Val Ser Ser Glu Pro Ser Ala Asn
            180                 185                 190

Glu Ser His Lys Thr Thr Thr Gly Ala Asp Ser Glu Ala Gly Gly Asp
        195                 200                 205

Val Ala Glu Lys Ser
    210

<210> 133
<211> 645
<212> DNA
<213> Solanum tuberosum

<400> 133
atgcagcagc acctgatgca gatgcagccc atgatggcag cttactatcc aacgaacgtc 60

actactgacc atattcaaca gtatttggat gagaacaaat cactcattct gaaaattgtt 120

gagagccaaa actcgggaaa actcagtgaa tgtgcagaga accaagctag gcttcagagg 180

aatctgatgt accttgctgc tattgctgat tcacaacctc agccttctag catgcattct 240

cagttctctt ctggtgggat gatgcagcca gggacacaca gttacctgca gcagcagcag 300

cagcaacaac aagcgcaaca aatggcaaca caacaactca tggctgcaag atcctcatca 360

atgctctatg gacaacaaca gcagcagcag cagcagtctc agttatcaca atttcaacaa 420

ggcttgcata gtagccaact tggcatgagt tctggcagtg gtggaagcac tggacttcat 480

cacatgcttc aaagtgaatc atcacctcat ggtggtggtt tctctcatga cttcggccgt 540

gcaaataagc aagacattgg gagtagtatg tctgctgaag ggcgcggcgg aagctcaggt 600

ggtgatggtg gtgagaatct ttatctgaaa gcttctgagg attga 645

<210> 134
<211> 214
<212> PRT
<213> Solanum tuberosum

<400> 134

```
Met Gln Gln His Leu Met Gln Met Gln Pro Met Met Ala Ala Tyr Tyr
1               5                   10                  15

Pro Thr Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Ser Ser Met His Ser
65                  70                  75                  80

Gln Phe Ser Ser Gly Gly Met Met Gln Pro Gly Thr His Ser Tyr Leu
                85                  90                  95

Gln Gln Gln Gln Gln Gln Gln Gln Ala Gln Gln Met Ala Thr Gln Gln
            100                 105                 110

Leu Met Ala Ala Arg Ser Ser Ser Met Leu Tyr Gly Gln Gln Gln Gln
        115                 120                 125

Gln Gln Gln Gln Ser Gln Leu Ser Gln Phe Gln Gln Gly Leu His Ser
    130                 135                 140

Ser Gln Leu Gly Met Ser Ser Gly Ser Gly Gly Ser Thr Gly Leu His
```

```
145                 150                 155                 160

His Met Leu Gln Ser Glu Ser Ser Pro His Gly Gly Gly Phe Ser His
                165                 170                 175

Asp Phe Gly Arg Ala Asn Lys Gln Asp Ile Gly Ser Ser Met Ser Ala
            180                 185                 190

Glu Gly Arg Gly Gly Ser Ser Gly Gly Asp Gly Gly Glu Asn Leu Tyr
        195                 200                 205

Leu Lys Ala Ser Glu Asp
    210


<210>  135
<211>  645
<212>  DNA
<213>  Sorghum bicolor

<400>  135
atgcagcagc agatgcccat gccgccggcg cccgctgcgg cggcggcgac ggcgccccgg      60

gcggccggca tcaccaccga gcagatccag aagtatttgg acgaaaataa gcaacttatt     120

ttggccatcc tagaaaatca gaacttagga aagttggctg aatgtgctca gtatcaagct     180

caacttcaaa agaacctctt gtacctggct gcgattgctg atgcccaacc ccgaccaccg     240

caaaaccctg caggtcgccc tcagatgatg caacctggta tagtgccagg tgcagggcat     300

tacatgtcac aagtaccaat gttccctcca agaactccat taaccccaca gcaaatgcaa     360

gagcagcagc agcaacagct tcagcagcag caagcgcagg ctcttgcatt ccctgggcag     420

atggtcatga gaccagctac catcaacggc atgcagcagc ctatgcaggc tgaccctgcc     480

cgggcagcgg agctgcaaca gccagcatct gtcccagccg acgggcgagt aagcaagcag     540

gacacagcgg ctggggtgag ctcagagcct tctgccaatg agagccacaa gaccacaacc     600

ggagcagata gtgaggcagg tggagacgtg gcggagaaat cctaa                     645


<210>  136
<211>  214
<212>  PRT
<213>  Sorghum bicolor

<400>  136

Met Gln Gln Gln Met Pro Met Pro Pro Ala Pro Ala Ala Ala Ala Ala
1               5                   10                  15

Thr Ala Pro Pro Ala Ala Gly Ile Thr Thr Glu Gln Ile Gln Lys Tyr
            20                  25                  30

Leu Asp Glu Asn Lys Gln Leu Ile Leu Ala Ile Leu Glu Asn Gln Asn
        35                  40                  45
```

Leu Gly Lys Leu Ala Glu Cys Ala Gln Tyr Gln Ala Gln Leu Gln Lys
    50                  55                  60

Asn Leu Leu Tyr Leu Ala Ala Ile Ala Asp Ala Gln Pro Arg Pro Pro
65                  70                  75                  80

Gln Asn Pro Ala Gly Arg Pro Gln Met Met Gln Pro Gly Ile Val Pro
                85                  90                  95

Gly Ala Gly His Tyr Met Ser Gln Val Pro Met Phe Pro Pro Arg Thr
            100                 105                 110

Pro Leu Thr Pro Gln Gln Met Gln Glu Gln Gln Gln Gln Gln Leu Gln
        115                 120                 125

Gln Gln Gln Ala Gln Ala Leu Ala Phe Pro Gly Gln Met Val Met Arg
    130                 135                 140

Pro Ala Thr Ile Asn Gly Met Gln Gln Pro Met Gln Ala Asp Pro Ala
145                 150                 155                 160

Arg Ala Ala Glu Leu Gln Gln Pro Ala Ser Val Pro Ala Asp Gly Arg
                165                 170                 175

Val Ser Lys Gln Asp Thr Ala Ala Gly Val Ser Ser Glu Pro Ser Ala
            180                 185                 190

Asn Glu Ser His Lys Thr Thr Thr Gly Ala Asp Ser Glu Ala Gly Gly
        195                 200                 205

Asp Val Ala Glu Lys Ser
    210

<210> 137
<211> 558
<212> DNA
<213> Triticum aestivum

<400> 137
atgcagcaag cgatgcccat gccgccggcg gcggcggcgc cggggatgcc tccgtctgct 60

ggcctcagca ccgagcagat ccaaaagtac ctggatgaaa ataagcaact aattttggct 120

atcttggaaa atcagaacct gggaaagttg gcggaatgtg ctcagtatca agctcagctt 180

cagaagaatc ttttgtattt ggctgcaatc gctgatactc agccacagac cactgtaagc 240

cgtcctcaga tggcaccacc tagtgcatcc ccaggggcag ggcattacat gtcacaggtg 300

ccaatgttcc ctccgaggac ccctctaacg cctcagcaga tgcaggagca gcaactacag 360

cagcaacagg ctcagatgct tccgtttgct ggtcaaatgg ttgcgagacc tggggctgtc 420

aatggcatgc ctcaggcccc tcaagttgaa ccagcctatg cagcaggtgg ggccagttct 480

gagccttctg gcactgagag ccacaggagc actggtgccg ataatgacgg ggggagcggc 540

tgggctgatc agtcctaa 558

<210> 138
<211> 185
<212> PRT
<213> Triticum aestivum

<400> 138

Met Gln Gln Ala Met Pro Met Pro Pro Ala Ala Ala Ala Pro Gly Met
1 5 10 15

Pro Pro Ser Ala Gly Leu Ser Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Thr Gln Pro Gln Thr Thr Val Ser
65 70 75 80

Arg Pro Gln Met Ala Pro Pro Ser Ala Ser Pro Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln Leu Gln Gln Gln Gln Ala Gln Met Leu Pro
115 120 125

Phe Ala Gly Gln Met Val Ala Arg Pro Gly Ala Val Asn Gly Met Pro
130 135 140

Gln Ala Pro Gln Val Glu Pro Ala Tyr Ala Ala Gly Gly Ala Ser Ser
145 150 155 160

Glu Pro Ser Gly Thr Glu Ser His Arg Ser Thr Gly Ala Asp Asn Asp
165 170 175

Gly Gly Ser Gly Trp Ala Asp Gln Ser
180 185

<210> 139
<211> 603

<212> DNA
<213> Triticum aestivum

<400> 139
atgcagcagg cgatgtcctt gcccccggga gcggtcggcg cggtgtcctc gccggccggc 60

atcaccaccg agcagatcca aaagtatttg gatgaaaata agcaacttat tttggccatc 120

cttgaaaatc agaacctagg aaagttggct gaatgtgctc agtatcaagc tcaactccaa 180

aagaatctct tgtatctagc tgctatcgcg gatgcccaac caccacagaa ccctacaagt 240

caccctcaga tggtgcagcc tggtagtatg caaggtgcag ggcattacat gtcacaagta 300

ccaatgttcc ctccaagaac gcctttaacc ccacagcaga tgcaagagca gcagcaccag 360

cagcttcagc agcagcaagc ccaggcccttt tctttccccg cccaggtggt catgagacca 420

ggcaccgtca acggcatgca gcagcctatg caagcagccg gcgacctcca gccagcagca 480

gcacctggag ggagcaagca ggacgccgca gtggctgggg ccagctcgga accatctggc 540

accaagagcc acaagaacgc gggagcagag gaggtgggcg ctgatgtagc agaacaatcc 600

taa 603

<210> 140
<211> 200
<212> PRT
<213> Triticum aestivum

<400> 140

Met Gln Gln Ala Met Ser Leu Pro Pro Gly Ala Val Gly Ala Val Ser
1 5 10 15

Ser Pro Ala Gly Ile Thr Thr Glu Gln Ile Gln Lys Tyr Leu Asp Glu
20 25 30

Asn Lys Gln Leu Ile Leu Ala Ile Leu Glu Asn Gln Asn Leu Gly Lys
35 40 45

Leu Ala Glu Cys Ala Gln Tyr Gln Ala Gln Leu Gln Lys Asn Leu Leu
50 55 60

Tyr Leu Ala Ala Ile Ala Asp Ala Gln Pro Pro Gln Asn Pro Thr Ser
65 70 75 80

His Pro Gln Met Val Gln Pro Gly Ser Met Gln Gly Ala Gly His Tyr
85 90 95

Met Ser Gln Val Pro Met Phe Pro Pro Arg Thr Pro Leu Thr Pro Gln
100 105 110

Gln Met Gln Glu Gln Gln His Gln Gln Leu Gln Gln Gln Gln Ala Gln
115 120 125

Ala Leu Ser Phe Pro Ala Gln Val Val Met Arg Pro Gly Thr Val Asn
    130                 135                 140

Gly Met Gln Gln Pro Met Gln Ala Ala Gly Asp Leu Gln Pro Ala Ala
145                 150                 155                 160

Ala Pro Gly Gly Ser Lys Gln Asp Ala Ala Val Ala Gly Ala Ser Ser
                165                 170                 175

Glu Pro Ser Gly Thr Lys Ser His Lys Asn Ala Gly Ala Glu Glu Val
            180                 185                 190

Gly Ala Asp Val Ala Glu Gln Ser
        195                 200

<210>  141
<211>  672
<212>  DNA
<213>  Vitis vinifera

<400>  141
atgcagcagc acctgatgca gatgcagccc atgatggcag cctattaccc cagcaacgtc     60

accactgatc acattcagca gtatcttgat gaaaacaagt cattgattct gaagattgtt    120

gagagccaga attcaggaaa attgactgaa tgtgcagaga accaggcaag actacagaga    180

aacctcatgt acctggctgc aattgctgat tctcaacccc aaccacccac catgcatgct    240

cagttccctc ctagtggcat tgttcagcca ggagctcact acatgcaaca ccaacaagct    300

caacaaatga caccacagtc gctcctggct gcacgctcct ccatgctgta cacccaacaa    360

ccattttcgg ccctgcaaca acaacaagcc atccatagcc agcttggcat gggctctggt    420

ggaagtgcag gacttcacat gctgcaaagc gaggggagta atccaggagg caatggaaca    480

ctggggactg gtgggtttcc tgatttcagc cgtggaactt ctggagaagg cctgcaggct    540

gcaggcaggg gaatggctgg tgggagcaag caagatatgg gaaatgcaga agggcgagga    600

gggaactcag gaggtcaggg tggggatgga ggtgagactc tttacttgaa agctgctgaa    660

gatgggaatt ga                                                        672

<210>  142
<211>  223
<212>  PRT
<213>  Vitis vinifera

<400>  142

Met Gln Gln His Leu Met Gln Met Gln Pro Met Met Ala Ala Tyr Tyr
1               5                   10                  15

Pro Ser Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
35 40 45

Thr Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Met His Ala
65 70 75 80

Gln Phe Pro Pro Ser Gly Ile Val Gln Pro Gly Ala His Tyr Met Gln
85 90 95

His Gln Gln Ala Gln Gln Met Thr Pro Gln Ser Leu Leu Ala Ala Arg
100 105 110

Ser Ser Met Leu Tyr Thr Gln Gln Pro Phe Ser Ala Leu Gln Gln Gln
115 120 125

Gln Ala Ile His Ser Gln Leu Gly Met Gly Ser Gly Gly Ser Ala Gly
130 135 140

Leu His Met Leu Gln Ser Glu Gly Ser Asn Pro Gly Gly Asn Gly Thr
145 150 155 160

Leu Gly Thr Gly Gly Phe Pro Asp Phe Ser Arg Gly Thr Ser Gly Glu
165 170 175

Gly Leu Gln Ala Ala Gly Arg Gly Met Ala Gly Gly Ser Lys Gln Asp
180 185 190

Met Gly Asn Ala Glu Gly Arg Gly Gly Asn Ser Gly Gly Gln Gly Gly
195 200 205

Asp Gly Gly Glu Thr Leu Tyr Leu Lys Ala Ala Glu Asp Gly Asn
210 215 220

<210> 143
<211> 663
<212> DNA
<213> Zea mays

<400> 143
atgcagcagc agatgcccat gccgccggcg cccgctgccg ccgcggcggc ggcgcccccg 60

gcggcaggca tcactaccga gcagatccag aagtatttgg acgaaaataa gcaacttatt 120

ttggccatcc tggaaaatca gaacttaggg aagttggctg aatgtgctca gtatcaagct 180

caacttcaaa agaacctctt gtacctggct gcgattgctg atgcccaacc ccagcctccg 240

caaaaccctg caggtcgccc tcagatgatg cagcctggta tagtgccagg tgcggggcat 300

tacatgtcac aagtaccaat gttccctcca agaaccccat taaccccaca gcagatgcag 360

gagcagcagc aacaacaaca gtttcagcag cagcagcagc aagtgcaggc tcttacattt 420

cctggacaga tggtcatgag accaggcacc atcaacggca tgcagcagca gcagcctatg 480

caggctgacc ctgcccgggc agcagcggag ctgcagcagg cagcacctat cccagctgac 540

gggcgaggaa gcaagcagga caccgcgggt ggggcgagct cagagccttc tgccaatgag 600

agccacaaga gcgccaccgg agcagatacc gaggcaggtg gcgacgtggc cgagaaatcc 660

taa 663

<210> 144
<211> 220
<212> PRT
<213> Zea mays

<400> 144

Met Gln Gln Gln Met Pro Met Pro Pro Ala Pro Ala Ala Ala Ala Ala
1 5 10 15

Ala Ala Pro Pro Ala Ala Gly Ile Thr Thr Glu Gln Ile Gln Lys Tyr
20 25 30

Leu Asp Glu Asn Lys Gln Leu Ile Leu Ala Ile Leu Glu Asn Gln Asn
35 40 45

Leu Gly Lys Leu Ala Glu Cys Ala Gln Tyr Gln Ala Gln Leu Gln Lys
50 55 60

Asn Leu Leu Tyr Leu Ala Ala Ile Ala Asp Ala Gln Pro Gln Pro Pro
65 70 75 80

Gln Asn Pro Ala Gly Arg Pro Gln Met Met Gln Pro Gly Ile Val Pro
85 90 95

Gly Ala Gly His Tyr Met Ser Gln Val Pro Met Phe Pro Pro Arg Thr
100 105 110

Pro Leu Thr Pro Gln Gln Met Gln Glu Gln Gln Gln Gln Gln Gln Phe
115 120 125

Gln Gln Gln Gln Gln Gln Val Gln Ala Leu Thr Phe Pro Gly Gln Met
130 135 140

Val Met Arg Pro Gly Thr Ile Asn Gly Met Gln Gln Gln Gln Pro Met
145 150 155 160

Gln Ala Asp Pro Ala Arg Ala Ala Ala Glu Leu Gln Gln Ala Ala Pro
165 170 175

Ile Pro Ala Asp Gly Arg Gly Ser Lys Gln Asp Thr Ala Gly Gly Ala
180 185 190

Ser Ser Glu Pro Ser Ala Asn Glu Ser His Lys Ser Ala Thr Gly Ala
195 200 205

Asp Thr Glu Ala Gly Gly Asp Val Ala Glu Lys Ser
210 215 220

<210> 145
<211> 2193
<212> DNA
<213> Oryza sativa

<400> 145
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtacctttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcacccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 146
<211> 12
<212> PRT
<213> Artificial sequence

<220>
<223> Box I

<220>
<221> VARIANT
<222> (3)..(3)
<223> /replace = "Lys"

<220>
<221> VARIANT
<222> (4)..(4)
<223> /replace = "Met" /replace = "Phe" /replace = "His"

<220>
<221> VARIANT
<222> (6)..(6)
<223> /replace = "Glu"

<220>
<221> VARIANT
<222> (7)..(7)
<223> /replace = "Asp"

<220>
<221> VARIANT
<222> (9)..(9)
<223> /replace = "Asn"

<220>

<221> VARIANT
<222> (10)..(10)
<223> Xaa can be any naturally occurring amino acid

<400> 146

Ile Gln Gln Tyr Leu Asp Glu Asn Lys Xaa Leu Ile
1 5 10

<210> 147
<211> 10
<212> PRT
<213> Artificial sequence

<220>
<223> Box II

<220>
<221> VARIANT
<222> (3)..(3)
<223> /replace= "Leu" /replace= "Val"

<220>
<221> VARIANT
<222> (7)..(7)
<223> /replace= "Thr"

<400> 147

Asn Leu Met Tyr Leu Ala Ala Ile Ala Asp
1 5 10

<210> 148
<211> 53
<212> DNA
<213> Artificial sequence

<220>
<223> primer: prm06681

<400> 148
ggggacaagt ttgtacaaaa aagcaggctt aaacaatgca acagcacctg atg 53

<210> 149
<211> 50
<212> DNA
<213> Artificial sequence

<220>
<223> primer: prm06682

<400> 149
ggggaccact ttgtacaaga aagctgggtc atcattaaga ttccttgtgc 50

<210> 150
<211> 615
<212> DNA
<213> Brassica napus

<400> 150

```
atgcaacagc acctgatgca gatgcagccc atgatggctg gttactaccc cagcaatgtc     60
acctctgatc atattcagca gtacttggac gagaacaaat cgttgattct gaagatagtt    120
gaatctcaaa actcgggaaa gctcagcgag tgtgccgaga accaggcaag gcttcaacgc    180
aacttaatgt acttagctgc aattgcagat tctcagcctc aacctccaag catgcatagc    240
cagtatggaa ctgctggtgg tggtgggttg atgcagggag aaggagggtc acactatttg    300
caacagcaac aggcaattca acagcagcag agtcagcagt ctctaatggc ggctcgatct    360
tcaatgttgt atgctcagca gcagcaacag ccttatgcaa cgcttcagca gcagcaattg    420
caccatagcc agcttgggat gagctcaagc agcggaggag gaagcagcgg tctccatatg    480
ctacagggag aggctggtgg gtttcatgat tttggccgtg agaagttgga aatgggaagt    540
ggtgaaggca gaggaggaag ctcaggggat ggtggagaaa ccctttactt gaagtcatca    600
gatgatggga actga                                                     615
```

```
<210>  151
<211>  204
<212>  PRT
<213>  Brassica napus

<400>  151

Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Met His Ser
65                  70                  75                  80

Gln Tyr Gly Thr Ala Gly Gly Gly Gly Leu Met Gln Gly Glu Gly Gly
                85                  90                  95

Ser His Tyr Leu Gln Gln Gln Gln Ala Ile Gln Gln Gln Gln Ser Gln
            100                 105                 110

Gln Ser Leu Met Ala Ala Arg Ser Ser Met Leu Tyr Ala Gln Gln Gln
        115                 120                 125

Gln Gln Pro Tyr Ala Thr Leu Gln Gln Gln Gln Leu His His Ser Gln
    130                 135                 140
```

Leu Gly Met Ser Ser Ser Ser Gly Gly Gly Ser Ser Gly Leu His Met
145 150 155 160

Leu Gln Gly Glu Ala Gly Gly Phe His Asp Phe Gly Arg Glu Lys Leu
165 170 175

Glu Met Gly Ser Gly Glu Gly Arg Gly Gly Ser Ser Gly Asp Gly Gly
180 185 190

Glu Thr Leu Tyr Leu Lys Ser Ser Asp Asp Gly Asn
195 200

<210> 152
<211> 639
<212> DNA
<213> Glycine max

<400> 152
atgcagcagc acctgatgca gatgcagccc atgatggctg cctactaccc caacaacgtc 60

accactgatc acattcaaca gtacctggat gagaacaagt ccttgattct gaagattgtt 120

gaaagccaga attctggcaa gctgagcgag tgtgccgaga accaatcaag gctgcagaga 180

aatctcatgt acctagctgc aatagctgat tctcaaccac aaccatctcc attggctggt 240

cagtatcctt ctagtggact tgtgcagcag ggagcacact acatgcaggc tcaacaggct 300

cagcagatgt cacaacaaca gctaatggct tcgcgctcct cgctcctgta ctcccaacag 360

cctttctcag tgcttcaaca gcagcaaggc atgcacagcc aacttggcat gagctccagt 420

ggaagtcaag gcctccacat gctgcaaagt gaagccacta atgttggagg caatgcaacc 480

ataggaaccg gaggagggtt tccggacttt gtacgcattg gtagtggcaa gcaagatatt 540

ggaatctctg gtgaaggcag aggaggaaac tctagtggcc actctggtga tggtggtgag 600

acacttaatt acctgaaagc tgctggtgat ggaaactga 639

<210> 153
<211> 212
<212> PRT
<213> Glycine max

<400> 153

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ser Arg Leu Gln Arg Asn Leu Met Tyr
    50              55              60

Leu Ala Ala Ile Ala Asp Ser Gln Pro Gln Pro Ser Pro Leu Ala Gly
65              70              75              80

Gln Tyr Pro Ser Ser Gly Leu Val Gln Gln Gly Ala His Tyr Met Gln
                85              90              95

Ala Gln Gln Ala Gln Gln Met Ser Gln Gln Gln Leu Met Ala Ser Arg
            100             105             110

Ser Ser Leu Leu Tyr Ser Gln Gln Pro Phe Ser Val Leu Gln Gln Gln
        115             120             125

Gln Gly Met His Ser Gln Leu Gly Met Ser Ser Ser Gly Ser Gln Gly
    130             135             140

Leu His Met Leu Gln Ser Glu Ala Thr Asn Val Gly Gly Asn Ala Thr
145             150             155             160

Ile Gly Thr Gly Gly Gly Phe Pro Asp Phe Val Arg Ile Gly Ser Gly
                165             170             175

Lys Gln Asp Ile Gly Ile Ser Gly Glu Gly Arg Gly Gly Asn Ser Ser
            180             185             190

Gly His Ser Gly Asp Gly Gly Glu Thr Leu Asn Tyr Leu Lys Ala Ala
        195             200             205

Gly Asp Gly Asn
    210

<210> 154
<211> 1248
<212> DNA
<213> Chlamydomonas reinhardtii

<400> 154
atggccgcca ccatgctccg ctccagcacc cagtcgggca ttgccgctaa ggccggccgc 60

aaggaggctg tcagcgtccg cgcggtcgcc cagccccagc gccaggctgg tgctgccagc 120

gtcttctcct cgtcgtcgtc gggcgctgct gctcgccgcg gggtcgtcgc tcaggccacc 180

gctgttgcca cccccgcggc caagcctgcg gccaagacca gccagtatga gctgttcacg 240

ctcaccacct ggctgctgaa ggaggagatg aagggcacaa tcgatggcga gcttgtgacc 300

gtcatctcgt cggtgtcgct ggcctgcaag caaatcgcgt cgctggtgaa ccgcgctggt 360

atctccaacc tgaccggtgt ggctggcaac cagaacgtgc agggtgagga ccagaagaag 420

ctggacgtgg tgtccaacga ggtcttcaag aactgcctgg cctcctgcgg ccgcacgggt 480

gtgatcgcct ccgaggagga ggaccagccc gtggccgtgg aggagaccta ctcgggcaac 540

tacatcgtgg tgttcgaccc cctggacggc tcgtccaaca tcgacgccgg catctccgtc 600

ggctccatct tcggcatcta cgagcccagc gaggagtgcc ccattgacgc catggacgac 660

ccccagaaga tgatggagca gtgcgtcatg aacgtgtgcc agcccggctc gcgcctcaag 720

tgcgccggct actgcctgta cagcagcagc accatcatgg tgctgaccat cggcaacggt 780

gtgttcggct tcacgctgga ccccctggtc ggcgagttcg tgctgaccca ccccaacgtg 840

cagatccccg aggtgggcaa gatctacccg ttcaacgagg gcaactacgg cctgtgggac 900

gacagcgtta aggcttacat ggacagcctg aaggacccca agaagtggga cggcaagccc 960

tactcggccc gctacatcgg ctccctggtc ggtgacttcc accgcaccct gctgtacgga 1020

ggcatctacg gctaccccgg cgacgccaag aacaagaacg gcaagctccg cctgctgtac 1080

gagtgcgcgc ccatgtcgtt cattgccgag caggccggcg gcctcggctc caccggccag 1140

gagcgcgtgc tggacgtgaa ccccgagaag gtgcaccagc gcgtgccgct gttcatcggc 1200

tccaagaagg aggtcgagta cctggagtcc ttcaccaaga agcactaa 1248

<210> 155
<211> 415
<212> PRT
<213> Chlamydomonas reinhardtii

<400> 155

Met Ala Ala Thr Met Leu Arg Ser Ser Thr Gln Ser Gly Ile Ala Ala
1 5 10 15

Lys Ala Gly Arg Lys Glu Ala Val Ser Val Arg Ala Val Ala Gln Pro
20 25 30

Gln Arg Gln Ala Gly Ala Ala Ser Val Phe Ser Ser Ser Ser Ser Gly
35 40 45

Ala Ala Ala Arg Arg Gly Val Val Ala Gln Ala Thr Ala Val Ala Thr
50 55 60

Pro Ala Ala Lys Pro Ala Ala Lys Thr Ser Gln Tyr Glu Leu Phe Thr
65 70 75 80

Leu Thr Thr Trp Leu Leu Lys Glu Glu Met Lys Gly Thr Ile Asp Gly
85 90 95

Glu Leu Val Thr Val Ile Ser Ser Val Ser Leu Ala Cys Lys Gln Ile
100 105 110

Ala Ser Leu Val Asn Arg Ala Gly Ile Ser Asn Leu Thr Gly Val Ala
115 120 125

```
Gly Asn Gln Asn Val Gln Gly Glu Asp Gln Lys Lys Leu Asp Val Val
    130                 135                 140

Ser Asn Glu Val Phe Lys Asn Cys Leu Ala Ser Cys Gly Arg Thr Gly
145                 150                 155                 160

Val Ile Ala Ser Glu Glu Glu Asp Gln Pro Val Ala Val Glu Glu Thr
                165                 170                 175

Tyr Ser Gly Asn Tyr Ile Val Val Phe Asp Pro Leu Asp Gly Ser Ser
            180                 185                 190

Asn Ile Asp Ala Gly Ile Ser Val Gly Ser Ile Phe Gly Ile Tyr Glu
        195                 200                 205

Pro Ser Glu Glu Cys Pro Ile Asp Ala Met Asp Asp Pro Gln Lys Met
    210                 215                 220

Met Glu Gln Cys Val Met Asn Val Cys Gln Pro Gly Ser Arg Leu Lys
225                 230                 235                 240

Cys Ala Gly Tyr Cys Leu Tyr Ser Ser Ser Thr Ile Met Val Leu Thr
                245                 250                 255

Ile Gly Asn Gly Val Phe Gly Phe Thr Leu Asp Pro Leu Val Gly Glu
            260                 265                 270

Phe Val Leu Thr His Pro Asn Val Gln Ile Pro Glu Val Gly Lys Ile
        275                 280                 285

Tyr Pro Phe Asn Glu Gly Asn Tyr Gly Leu Trp Asp Asp Ser Val Lys
    290                 295                 300

Ala Tyr Met Asp Ser Leu Lys Asp Pro Lys Lys Trp Asp Gly Lys Pro
305                 310                 315                 320

Tyr Ser Ala Arg Tyr Ile Gly Ser Leu Val Gly Asp Phe His Arg Thr
                325                 330                 335

Leu Leu Tyr Gly Gly Ile Tyr Gly Tyr Pro Gly Asp Ala Lys Asn Lys
            340                 345                 350

Asn Gly Lys Leu Arg Leu Leu Tyr Glu Cys Ala Pro Met Ser Phe Ile
        355                 360                 365

Ala Glu Gln Ala Gly Gly Leu Gly Ser Thr Gly Gln Glu Arg Val Leu
    370                 375                 380
```

Asp Val Asn Pro Glu Lys Val His Gln Arg Val Pro Leu Phe Ile Gly
385 390 395 400

Ser Lys Lys Glu Val Glu Tyr Leu Glu Ser Phe Thr Lys Lys His
405 410 415

<210> 156
<211> 1239
<212> DNA
<213> Bigelowiella natans

<400> 156
atggctgccg tgctctttcg cggaggtgtg ctgtcttcca ccatgactgc ggctcgcaca 60

ttcgcagcgc catctgtgca cacccgtggc atgcacatgt cagtcaagag cagtaaccct 120

ttctctcagg ctggtcgtcg tgctgctgtt agatccagtg tcgcaccctc ccctgtccaa 180

gactctgctg gtactatcgt tgatgacgga actgtcacat taacaagatt catgattgag 240

gaggcaatga agagcaagac tcctgggcag gaggacatgg ttcgtttgat ttcttccatc 300

tcagttgcat gcaaacgcat tgcatccatg gtgcaaactg caggaatctc cggatctaca 360

ggtctggctg aaggtggtgg atcagtcaac gttcaaggcg aggaacagaa gaaacttgat 420

gtcatttcta acgatgtact aaagtctgcc cttcgtcctt ctggaaaact tggagtaatt 480

gcctctgagg aggaagataa tccagttgtc gttgatgaac tttactccgg cgaatatgtt 540

gctactttcg atccgctcga tggatcttcc aatattgatg ctgcaatttc cactggaact 600

atcttcggag tgttcaaagc tccagaagag tgcttgatcg gggattctga taatctcagt 660

attgcagagc agcaatgttt ggaggcaaca cttcaacctg gaactaacct tgttgctgct 720

ggatactgca tgtattcgtc ctccaccatc cttgttctca ccaccggaga cgggctcaat 780

ggatttactc ttgacccttc cattggagag ttcatcctca cccatcctaa tatccagatt 840

cctagccgtg gaaagatcta ttctatgaac gaagcaaact acttcgattg ggaccctaag 900

cttcagacct acgttgataa ccttaagaag gcagaaggtc aaactggaga aaagtacagc 960

tctcgctaca tcggatccat ggtcggagat gtgcaccgta cccttctcta tggaggcatc 1020

ttcgcttacc ctggagataa gaagaacgtc aacggaaaac ttcgccttct gtacgaagct 1080

gctccaatgt cccttatctt cgaacaagcg ggtggaaaat ctattactgg acctggtgga 1140

cgtgtgttgg acttagttcc tgataaggtt caccagcgtt gtcctgtgtt cattggatcc 1200

cctgatgatg tggatgaagt tgaaaaggct ctcgcataa 1239

<210> 157
<211> 412
<212> PRT
<213> Bigelowiella natans

<400> 157

```
Met Ala Ala Val Leu Phe Arg Gly Gly Val Leu Ser Ser Thr Met Thr
1               5                   10                  15

Ala Ala Arg Thr Phe Ala Ala Pro Ser Val His Thr Arg Gly Met His
            20                  25                  30

Met Ser Val Lys Ser Ser Asn Pro Phe Ser Gln Ala Gly Arg Arg Ala
        35                  40                  45

Ala Val Arg Ser Ser Val Ala Pro Ser Pro Val Gln Asp Ser Ala Gly
    50                  55                  60

Thr Ile Val Asp Asp Gly Thr Val Thr Leu Thr Arg Phe Met Ile Glu
65                  70                  75                  80

Glu Ala Met Lys Ser Lys Thr Pro Gly Gln Glu Asp Met Val Arg Leu
                85                  90                  95

Ile Ser Ser Ile Ser Val Ala Cys Lys Arg Ile Ala Ser Met Val Gln
            100                 105                 110

Thr Ala Gly Ile Ser Gly Ser Thr Gly Leu Ala Glu Gly Gly Gly Ser
        115                 120                 125

Val Asn Val Gln Gly Glu Glu Gln Lys Lys Leu Asp Val Ile Ser Asn
    130                 135                 140

Asp Val Leu Lys Ser Ala Leu Arg Pro Ser Gly Lys Leu Gly Val Ile
145                 150                 155                 160

Ala Ser Glu Glu Glu Asp Asn Pro Val Val Val Asp Glu Leu Tyr Ser
                165                 170                 175

Gly Glu Tyr Val Ala Thr Phe Asp Pro Leu Asp Gly Ser Ser Asn Ile
            180                 185                 190

Asp Ala Ala Ile Ser Thr Gly Thr Ile Phe Gly Val Phe Lys Ala Pro
        195                 200                 205

Glu Glu Cys Leu Ile Gly Asp Ser Asp Asn Leu Ser Ile Ala Glu Gln
    210                 215                 220

Gln Cys Leu Glu Ala Thr Leu Gln Pro Gly Thr Asn Leu Val Ala Ala
225                 230                 235                 240

Gly Tyr Cys Met Tyr Ser Ser Ser Thr Ile Leu Val Leu Thr Thr Gly
                245                 250                 255

Asp Gly Leu Asn Gly Phe Thr Leu Asp Pro Ser Ile Gly Glu Phe Ile
```

260 265 270

Leu Thr His Pro Asn Ile Gln Ile Pro Ser Arg Gly Lys Ile Tyr Ser
275 280 285

Met Asn Glu Ala Asn Tyr Phe Asp Trp Asp Pro Lys Leu Gln Thr Tyr
290 295 300

Val Asp Asn Leu Lys Lys Ala Glu Gly Gln Thr Gly Glu Lys Tyr Ser
305 310 315 320

Ser Arg Tyr Ile Gly Ser Met Val Gly Asp Val His Arg Thr Leu Leu
325 330 335

Tyr Gly Gly Ile Phe Ala Tyr Pro Gly Asp Lys Lys Asn Val Asn Gly
340 345 350

Lys Leu Arg Leu Leu Tyr Glu Ala Ala Pro Met Ser Leu Ile Phe Glu
355 360 365

Gln Ala Gly Gly Lys Ser Ile Thr Gly Pro Gly Gly Arg Val Leu Asp
370 375 380

Leu Val Pro Asp Lys Val His Gln Arg Cys Pro Val Phe Ile Gly Ser
385 390 395 400

Pro Asp Asp Val Asp Glu Val Glu Lys Ala Leu Ala
405 410

<210> 158
<211> 1230
<212> DNA
<213> Aquilegia formosa x Aquilegia pubescens

<400> 158
atggttgcag cagctatccc tacttcttgt caactgctct tctccacctc ttcttcgacc 60

acttctcgtc tatatcctcc ttatcttgat gccaaaactc tcttctcatt tcctacaaac 120

aagagacatg taagcattgt tagaactccg gcaggtgtac ggtgtcaagc attaggagca 180

gaggtagtag tgaccaagag gagtgcattt gagatacaaa ctctaacagg atggttattg 240

aaacaagaac aaacaggggt tatagatgca gagttaacta tagttttgtc tagcatttca 300

atggcctgta agcagattgc ttcattggtg cagagggcaa gtatttccaa cttaactgga 360

gttcaaggag ctgttaatat tcaaggtgaa gatcagaaga agcttgatgt catctctaac 420

gaggtgttct ctaattgcct tagatcaagt ggaagaacag ggattatagc atcagaagaa 480

gaggatgtac cagttgcagt ggaggaaagc tactctggca actatattgt cgtttttgat 540

cctcttgacg ggtcatcaaa cattgatgcc gctgtgtcaa ctggatccat atttggaatt 600

tatagtccga acgatgagtg tcttactgaa gtcgatgata atgccacagt gcttcagcaa 660

gtggaacaga agtgcatcat caatgtgtgt caacctggca acaacttgtt ggcagctggc 720

tactgcatgt actcaagctc tgtaatcttt gtgctttcca ttggacaagg ggttttctca 780

tttaccttag accctatgta cggagaattc gtcttgacac aggaaaacat tcaaatacct 840

aaatcaggta aaatctactc attcaacgaa ggcaactacc aattatggga tgataagttg 900

aagaagtata tcgatgacct taaggaccct ggtcctagtg gcaaaccata ctcttccaga 960

tacattggaa gcttggttgg cgatttccac cggacccttc tttatggagg gatatatgga 1020

taccctaggg ataagaatag aaaaaatggg aagctaaggc tactgtatga gtgtgcacct 1080

atgagttatt tagttgaaca agcaggagga aaaggatcag atggacacca aagagtactc 1140

gatattgaac cggtggagat tcatcagcgt gttccacttt tcattggcag cgttgaagaa 1200

gttgagaaac tagagaagtt cttggcttga 1230

<210> 159
<211> 409
<212> PRT
<213> Aquilegia formosa x Aquilegia pubescens

<400> 159

Met Val Ala Ala Ala Ile Pro Thr Ser Cys Gln Leu Leu Phe Ser Thr
1 5 10 15

Ser Ser Ser Thr Thr Ser Arg Leu Tyr Pro Pro Tyr Leu Asp Ala Lys
20 25 30

Thr Leu Phe Ser Phe Pro Thr Asn Lys Arg His Val Ser Ile Val Arg
35 40 45

Thr Pro Ala Gly Val Arg Cys Gln Ala Leu Gly Ala Glu Val Val Val
50 55 60

Thr Lys Arg Ser Ala Phe Glu Ile Gln Thr Leu Thr Gly Trp Leu Leu
65 70 75 80

Lys Gln Glu Gln Thr Gly Val Ile Asp Ala Glu Leu Thr Ile Val Leu
85 90 95

Ser Ser Ile Ser Met Ala Cys Lys Gln Ile Ala Ser Leu Val Gln Arg
100 105 110

Ala Ser Ile Ser Asn Leu Thr Gly Val Gln Gly Ala Val Asn Ile Gln
115 120 125

Gly Glu Asp Gln Lys Lys Leu Asp Val Ile Ser Asn Glu Val Phe Ser
130 135 140

```
Asn Cys Leu Arg Ser Ser Gly Arg Thr Gly Ile Ile Ala Ser Glu Glu
145                 150                 155                 160

Glu Asp Val Pro Val Ala Val Glu Glu Ser Tyr Ser Gly Asn Tyr Ile
                165                 170                 175

Val Val Phe Asp Pro Leu Asp Gly Ser Ser Asn Ile Asp Ala Ala Val
            180                 185                 190

Ser Thr Gly Ser Ile Phe Gly Ile Tyr Ser Pro Asn Asp Glu Cys Leu
        195                 200                 205

Thr Glu Val Asp Asp Asn Ala Thr Val Leu Gln Gln Val Glu Gln Lys
    210                 215                 220

Cys Ile Ile Asn Val Cys Gln Pro Gly Asn Asn Leu Leu Ala Ala Gly
225                 230                 235                 240

Tyr Cys Met Tyr Ser Ser Ser Val Ile Phe Val Leu Ser Ile Gly Gln
                245                 250                 255

Gly Val Phe Ser Phe Thr Leu Asp Pro Met Tyr Gly Glu Phe Val Leu
            260                 265                 270

Thr Gln Glu Asn Ile Gln Ile Pro Lys Ser Gly Lys Ile Tyr Ser Phe
        275                 280                 285

Asn Glu Gly Asn Tyr Gln Leu Trp Asp Asp Lys Leu Lys Lys Tyr Ile
    290                 295                 300

Asp Asp Leu Lys Asp Pro Gly Pro Ser Gly Lys Pro Tyr Ser Ser Arg
305                 310                 315                 320

Tyr Ile Gly Ser Leu Val Gly Asp Phe His Arg Thr Leu Leu Tyr Gly
                325                 330                 335

Gly Ile Tyr Gly Tyr Pro Arg Asp Lys Asn Arg Lys Asn Gly Lys Leu
            340                 345                 350

Arg Leu Leu Tyr Glu Cys Ala Pro Met Ser Tyr Leu Val Glu Gln Ala
        355                 360                 365

Gly Gly Lys Gly Ser Asp Gly His Gln Arg Val Leu Asp Ile Glu Pro
    370                 375                 380

Val Glu Ile His Gln Arg Val Pro Leu Phe Ile Gly Ser Val Glu Glu
385                 390                 395                 400
```

Val Glu Lys Leu Glu Lys Phe Leu Ala
405

<210> 160
<211> 1254
<212> DNA
<213> Arabidopsis thaliana

<400> 160
atggcagcat cggccgcaac aacgacgtcg tctcaccttc ttctctcaag ctcacgtcac 60

gtggcttcat catcccagcc ttcaatcctt tcaccgagat ctctcttctc caacaacggg 120

aaacgagcac caactggagt gagaaaccat cagtatgcga gtggagtgag gtgtatggcc 180

gtagcggcgg atgcttctga gacgaaaacg gcggcgagga agaagagtgg atacgaactt 240

caaacgttga cgggctggtt gctgagacaa gagatgaaag gagagataga tgcagagctg 300

acgatagtga tgtcgagtat atcattggct tgtaagcaga tcgcttcact tgttcaacgt 360

gccggaatat ctaacctgac cggagttcaa ggcgccatta atattcaggg agaggatcag 420

aagaagcttg acgtcatctc taatgaggtg ttttccaact gtttgagatc aagtggaaga 480

acgggaatca tagcctcgga ggaagaggac gtgccagttg cggtggagga gagttactcc 540

ggcaactacg tcgtcgtgtt tgaccctctt gatggttcct ccaacattga cgctgccgtc 600

tctactggtt ctatcttcgg tatctatagc cccaatgacg aatgcattgt cgacgactcc 660

gacgatatct cagctcttgg gtcagaagaa caaaggtgta tagtaaacgt gtgccagcca 720

gggaacaact tgttagcagc cggctactgt atgtactcga gctcggtcat cttcgttctt 780

actctaggca aaggcgtttt ctccttcacg cttgatccaa tgtacggtga gtttgtcctc 840

acgcaagaaa acattgagat ccccaaagcc gggagaatct actctttcaa cgaagggaat 900

taccagatgt gggacgataa actaaagaag tacattgatg accttaagga ccctggtcca 960

actgggaagc cttactcggc aaggtacatt ggaagtttgg ttggagattt tcacaggact 1020

ttgttgtacg gtgggattta cgggtaccct cgtgacgcaa agagcaaaaa tggaaagctt 1080

aggcttttgt atgagtgtgc accaatgagt ttcattgttg aacaagctgg agggaaaggt 1140

tctgatggac attcgagagt actagatatc caaccgactg agatacatca gagggttcct 1200

ctatacattg gaagcacaga ggaagtagag aagttggaga agtacttggc ttga 1254

<210> 161
<211> 417
<212> PRT
<213> Arabidopsis thaliana

<400> 161

Met Ala Ala Ser Ala Ala Thr Thr Thr Ser Ser His Leu Leu Leu Ser
1 5 10 15

Ser Ser Arg His Val Ala Ser Ser Ser Gln Pro Ser Ile Leu Ser Pro

```
            20                  25                  30

Arg Ser Leu Phe Ser Asn Asn Gly Lys Arg Ala Pro Thr Gly Val Arg
        35                  40                  45

Asn His Gln Tyr Ala Ser Gly Val Arg Cys Met Ala Val Ala Ala Asp
    50                  55                  60

Ala Ser Glu Thr Lys Thr Ala Ala Arg Lys Lys Ser Gly Tyr Glu Leu
65                  70                  75                  80

Gln Thr Leu Thr Gly Trp Leu Leu Arg Gln Glu Met Lys Gly Glu Ile
                85                  90                  95

Asp Ala Glu Leu Thr Ile Val Met Ser Ser Ile Ser Leu Ala Cys Lys
            100                 105                 110

Gln Ile Ala Ser Leu Val Gln Arg Ala Gly Ile Ser Asn Leu Thr Gly
        115                 120                 125

Val Gln Gly Ala Ile Asn Ile Gln Gly Glu Asp Gln Lys Lys Leu Asp
    130                 135                 140

Val Ile Ser Asn Glu Val Phe Ser Asn Cys Leu Arg Ser Ser Gly Arg
145                 150                 155                 160

Thr Gly Ile Ile Ala Ser Glu Glu Glu Asp Val Pro Val Ala Val Glu
                165                 170                 175

Glu Ser Tyr Ser Gly Asn Tyr Val Val Val Phe Asp Pro Leu Asp Gly
            180                 185                 190

Ser Ser Asn Ile Asp Ala Ala Val Ser Thr Gly Ser Ile Phe Gly Ile
        195                 200                 205

Tyr Ser Pro Asn Asp Glu Cys Ile Val Asp Asp Ser Asp Asp Ile Ser
    210                 215                 220

Ala Leu Gly Ser Glu Glu Gln Arg Cys Ile Val Asn Val Cys Gln Pro
225                 230                 235                 240

Gly Asn Asn Leu Leu Ala Ala Gly Tyr Cys Met Tyr Ser Ser Ser Val
                245                 250                 255

Ile Phe Val Leu Thr Leu Gly Lys Gly Val Phe Ser Phe Thr Leu Asp
            260                 265                 270

Pro Met Tyr Gly Glu Phe Val Leu Thr Gln Glu Asn Ile Glu Ile Pro
        275                 280                 285
```

Lys Ala Gly Arg Ile Tyr Ser Phe Asn Glu Gly Asn Tyr Gln Met Trp
290 295 300

Asp Asp Lys Leu Lys Lys Tyr Ile Asp Asp Leu Lys Asp Pro Gly Pro
305 310 315 320

Thr Gly Lys Pro Tyr Ser Ala Arg Tyr Ile Gly Ser Leu Val Gly Asp
325 330 335

Phe His Arg Thr Leu Leu Tyr Gly Gly Ile Tyr Gly Tyr Pro Arg Asp
340 345 350

Ala Lys Ser Lys Asn Gly Lys Leu Arg Leu Leu Tyr Glu Cys Ala Pro
355 360 365

Met Ser Phe Ile Val Glu Gln Ala Gly Gly Lys Gly Ser Asp Gly His
370 375 380

Ser Arg Val Leu Asp Ile Gln Pro Thr Glu Ile His Gln Arg Val Pro
385 390 395 400

Leu Tyr Ile Gly Ser Thr Glu Glu Val Glu Lys Leu Glu Lys Tyr Leu
405 410 415

Ala

<210> 162
<211> 1254
<212> DNA
<213> Brassica napus

<400> 162
atggcagcaa ccgccgcgac aacgtcatcg tctcatcttc ttctctcaag ctctcgccac 60

gtggcagcct catctcagcc acaaatcctt ttccagagat ctctgttttc cggcgggaaa 120

cgatcggcag ctggaaaaaa ccatcatgct agtggtggag tgaggtgtat ggcggttgca 180

gcggatgctt cggcggaggc taagccggcg gcagcgagga agaagagtgg gtacgagctt 240

cagacgctga cgggttggtt gctgagacaa gagcagaaag gagagataga tacagagctg 300

acgatagtga tgtcgagcat agcgatggct tgtaagcaga tcgcttcgct tgtacagcgc 360

gccggaatct ctaatctgac cggcgttcaa ggagccgtta acattcaggg agaggatcag 420

aaaaagctcg acgtcgtctc taatgaggta ttttcaaact gtttgagatc aagtggaagg 480

acagggatca ttgcgtcaga ggaagaggac gtccccgtag cagttgaaga gagttactcc 540

ggaaactaca ttgtcgtctt tgatcctctc gacggttcct ccaacatcga cgctgcagtc 600

tccactggtt caatcttcgg tatctacagc cccaatgacg agtgtatcgt tgacgactcc 660

```
gacgatatct cctctcttgg ttcagaagaa caaaggtgta tagtaaacgt gtgtcaacca     720

gggaacaact tgctcgcagc tggctactgt atgtactcga gctcagtcat cttcgttctc     780

accttaggca agggcgtttt ctccttcaca ctcgacccaa tgtacggcga gttcgtcctc     840

actcaagaga acattgagat ccccaaagca gggaaaatct actctttcaa cgaagggaac     900

taccagatgt gggacgagaa actgaagaag tacattgatg atcttaagga ccctggtcca     960

agtgggaagc cttactctgc aaggtacatt ggtagtttgg tcggagactt tcacagaact    1020

ttgttgtacg gtgggattta cgggtaccct cgtgacgcca agagcaaaaa cggtaagctt    1080

aggcttttgt atgagtgtgc accgatgagt ttcatcgttg aacaagctgg aggaaaagga    1140

tcagatggcc accaaagagt actagatatc caacccaccg agatacatca gagggttcca    1200

ctttacattg gaagcaaaga agaagtagag aagctggaga agtacttggc ttga          1254


<210>  163
<211>  417
<212>  PRT
<213>  Brassica napus

<400>  163

Met Ala Ala Thr Ala Ala Thr Thr Ser Ser Ser His Leu Leu Leu Ser
1               5                   10                  15


Ser Ser Arg His Val Ala Ala Ser Ser Gln Pro Gln Ile Leu Phe Gln
            20                  25                  30


Arg Ser Leu Phe Ser Gly Gly Lys Arg Ser Ala Ala Gly Lys Asn His
        35                  40                  45


His Ala Ser Gly Gly Val Arg Cys Met Ala Val Ala Ala Asp Ala Ser
    50                  55                  60


Ala Glu Ala Lys Pro Ala Ala Ala Arg Lys Lys Ser Gly Tyr Glu Leu
65                  70                  75                  80


Gln Thr Leu Thr Gly Trp Leu Leu Arg Gln Glu Gln Lys Gly Glu Ile
                85                  90                  95


Asp Thr Glu Leu Thr Ile Val Met Ser Ser Ile Ala Met Ala Cys Lys
            100                 105                 110


Gln Ile Ala Ser Leu Val Gln Arg Ala Gly Ile Ser Asn Leu Thr Gly
        115                 120                 125


Val Gln Gly Ala Val Asn Ile Gln Gly Glu Asp Gln Lys Lys Leu Asp
    130                 135                 140
```

Val Val Ser Asn Glu Val Phe Ser Asn Cys Leu Arg Ser Ser Gly Arg
145 150 155 160

Thr Gly Ile Ile Ala Ser Glu Glu Glu Asp Val Pro Val Ala Val Glu
165 170 175

Glu Ser Tyr Ser Gly Asn Tyr Ile Val Val Phe Asp Pro Leu Asp Gly
180 185 190

Ser Ser Asn Ile Asp Ala Ala Val Ser Thr Gly Ser Ile Phe Gly Ile
195 200 205

Tyr Ser Pro Asn Asp Glu Cys Ile Val Asp Asp Ser Asp Asp Ile Ser
210 215 220

Ser Leu Gly Ser Glu Glu Gln Arg Cys Ile Val Asn Val Cys Gln Pro
225 230 235 240

Gly Asn Asn Leu Leu Ala Ala Gly Tyr Cys Met Tyr Ser Ser Ser Val
245 250 255

Ile Phe Val Leu Thr Leu Gly Lys Gly Val Phe Ser Phe Thr Leu Asp
260 265 270

Pro Met Tyr Gly Glu Phe Val Leu Thr Gln Glu Asn Ile Glu Ile Pro
275 280 285

Lys Ala Gly Lys Ile Tyr Ser Phe Asn Glu Gly Asn Tyr Gln Met Trp
290 295 300

Asp Glu Lys Leu Lys Lys Tyr Ile Asp Asp Leu Lys Asp Pro Gly Pro
305 310 315 320

Ser Gly Lys Pro Tyr Ser Ala Arg Tyr Ile Gly Ser Leu Val Gly Asp
325 330 335

Phe His Arg Thr Leu Leu Tyr Gly Gly Ile Tyr Gly Tyr Pro Arg Asp
340 345 350

Ala Lys Ser Lys Asn Gly Lys Leu Arg Leu Leu Tyr Glu Cys Ala Pro
355 360 365

Met Ser Phe Ile Val Glu Gln Ala Gly Gly Lys Gly Ser Asp Gly His
370 375 380

Gln Arg Val Leu Asp Ile Gln Pro Thr Glu Ile His Gln Arg Val Pro
385 390 395 400

Leu Tyr Ile Gly Ser Lys Glu Glu Val Glu Lys Leu Glu Lys Tyr Leu

405 410 415

Ala

<210> 164
<211> 1130
<212> DNA
<213> Cyanidioschyzon merolae

<400> 164
atggcgaagc aagcaccgca cgctttcgtt agccttgggg ctcctaagct gcgctcaaca 60

aacatatggg gaagggtatc ggtgtcggtc ttgccgctgg aaacaaggca tcaacgtcgc 120

tttggtgcag tgagactgcg cgcagcttta gacctaccca tgacactcgg acagtgggtt 180

ctccaacagg agaggcagca tccagagacc gctgatttgg ctcctttgat aacagccaca 240

gcgacagctg gcaagcagat tgcctcactg gtcgcccgag cgggcgtgag caacttgact 300

ggccttcagg gctcggtcaa cgtgcagggc gaggagcaga aaaagctgga cgtgttgacg 360

aacgaggtgc tcaagaacgc gctacgctca acagggaaac taggtgtctt ggcttcggaa 420

gaggaaaacg agccagtgtc gctcatggaa gaggcgtata caggtgactt tattgctgcg 480

tttgacccgt tggacggatc ctcgaatttg gatgcagcga tcgccacggg tacgattttt 540

ggtgtaatgc gcagcggcga gcactgtctg acgggacccg aggacgcgga tataagtcag 600

cgacaaatgc agtgcctcgt gcagacgctg caaccgggtg cgaatctggt ggcagcgggc 660

tacattctct actcctcatc ggtgattttc atgctgtcta ttggtcacgg agtgcagggt 720

ttttctctgg acccagcgat tggcgagttt gttttgacgc acccagatgt tcgtgttccg 780

gagcgtggca agatctactc cttcaacgag gcaaactcgg ataactggga ccctgttctt 840

caagaatata ttcgatcgat gaagaaaaag ggctattctt cgcgctacat aggatctctc 900

gttggcgatg tccatcgcac gctgatttac ggcggcgttt ttggataccc tggtgataag 960

aagaacccga acggcaagtt gcgtcttctg tacgagtgcg cacctatggc gtatctcatg 1020

gagcaggccg gcggtatagc gaccacggga aagcaaagaa ttctggatat tgttccgcga 1080

gatgtccatc agcgagagcc gttcatttgc ggaagcccca gggatgttga 1130

<210> 165
<211> 391
<212> PRT
<213> Cyanidioschyzon merolae

<400> 165

Met Ala Lys Gln Ala Pro His Ala Phe Val Ser Leu Gly Ala Pro Lys
1 5 10 15

Leu Arg Ser Thr Asn Ile Trp Gly Arg Val Ser Val Ser Val Leu Pro
20 25 30

```
Leu Glu Thr Arg His Gln Arg Arg Phe Gly Ala Val Arg Leu Arg Ala
        35                  40                  45

Ala Leu Asp Leu Pro Met Thr Leu Gly Gln Trp Val Leu Gln Gln Glu
    50                  55                  60

Arg Gln His Pro Glu Thr Ala Asp Leu Ala Pro Leu Ile Thr Ala Thr
65                  70                  75                  80

Ala Thr Ala Gly Lys Gln Ile Ala Ser Leu Val Ala Arg Ala Gly Val
                85                  90                  95

Ser Asn Leu Thr Gly Leu Gln Gly Ser Val Asn Val Gln Gly Glu Glu
            100                 105                 110

Gln Lys Lys Leu Asp Val Leu Thr Asn Glu Val Leu Lys Asn Ala Leu
        115                 120                 125

Arg Ser Thr Gly Lys Leu Gly Val Leu Ala Ser Glu Glu Glu Asn Glu
    130                 135                 140

Pro Val Ser Leu Met Glu Glu Ala Tyr Thr Gly Asp Phe Ile Ala Ala
145                 150                 155                 160

Phe Asp Pro Leu Asp Gly Ser Ser Asn Leu Asp Ala Ala Ile Ala Thr
                165                 170                 175

Gly Thr Ile Phe Gly Val Met Arg Ser Gly Glu His Cys Leu Thr Gly
            180                 185                 190

Pro Glu Asp Ala Asp Ile Ser Gln Arg Gln Met Gln Cys Leu Val Gln
        195                 200                 205

Thr Leu Gln Pro Gly Ala Asn Leu Val Ala Ala Gly Tyr Ile Leu Tyr
    210                 215                 220

Ser Ser Ser Val Ile Phe Met Leu Ser Ile Gly His Gly Val Gln Gly
225                 230                 235                 240

Phe Ser Leu Asp Pro Ala Ile Gly Glu Phe Val Leu Thr His Pro Asp
                245                 250                 255

Val Arg Val Pro Glu Arg Gly Lys Ile Tyr Ser Phe Asn Glu Ala Asn
            260                 265                 270

Ser Asp Asn Trp Asp Pro Val Leu Gln Glu Tyr Ile Arg Ser Met Lys
        275                 280                 285
```

Lys Lys Gly Tyr Ser Ser Arg Tyr Ile Gly Ser Leu Val Gly Asp Val
290 295 300

His Arg Thr Leu Ile Tyr Gly Gly Val Phe Gly Tyr Pro Gly Asp Lys
305 310 315 320

Lys Asn Pro Asn Gly Lys Leu Arg Leu Leu Tyr Glu Cys Ala Pro Met
325 330 335

Ala Tyr Leu Met Glu Gln Ala Gly Gly Ile Ala Thr Thr Gly Lys Gln
340 345 350

Arg Ile Leu Asp Ile Val Pro Arg Asp Val His Gln Arg Glu Pro Phe
355 360 365

Ile Cys Gly Ser Pro Arg Asp Val Glu Asp Leu Leu Lys Ile Tyr Gln
370 375 380

Gly Ser Met Ala Met Arg Gly
385 390

<210> 166
<211> 1218
<212> DNA
<213> Glycine max

<400> 166
atggttgcaa tggcagcagc aacagcatcc acccagttga ttttctcaaa gccttgttcc 60

ccttcacgtc tatgcccctt ccaactatgt gtctttgaca ctaaacaagt gctatcaagt 120

ggcaggagaa ggcatgtggg gggttctgga gttaggtgca tggctgtggg ggaagcagca 180

accactggga caaagaagag aagtggatat gagcttcaaa cactcactag ctggttgctg 240

aagcaggagc aagctggggt gattgatgca gaactcacta ttgtgctgtc tagcatttcc 300

atggcatgca aacagattgc ttctttggtg caaagagcta acatttccaa cctcactggg 360

gttcaaggtg ctgtcaatgt tcaaggggaa gaccagaaaa agcttgatgt tgtttcaaat 420

gaggttttct caaactgctt gaggtcaagt gggaggacag ggataatagc atcagaggag 480

gaagatgtgc cagtggcagt agaagagagt tattctggaa actacattgt ggtgtttgac 540

ccacttgatg ggtcatccaa tattgatgct gcagcgtcaa ctgggtccaa tttttggata 600

tacagcccca atgatgagtg tcttgctgac attgatgatg accccaccct tgacacaaca 660

gaacaaagat gtattgtgaa cgtgtgccaa cctggaagca accttcttgc agctggttac 720

tgcatgtatt ctagctcaat aatctttgtt ctcacacttg gaaatggagt gtttgtgttt 780

acattggacc cgatgtatgg cgaattcgtt ttgactcagg aaaacctcca gatacctaga 840

gcaggcaaaa tctatgcatt caatgaaggt aattatcagt tgtgggatga gaagctaaag 900

aaatatattg atgatctcaa ggacccaggt caaagcggca agccttattc tgcaaggtac 960

attggtagct tggtaggaga tttccacagg acactgctat atggtggcat ttacgggtac 1020

cccagggaca agaaaagcaa gaatgggaaa ctaaggctcc tgtatgaatg tgctcctatt 1080

aacttcattg tagaacaagc tggtggaaaa ggtacagatg gccttcaagt actccggctt 1140

caagggacag agattcatca acgtgtgcca ctgtacattg ggaagaggt agagaaggtg 1200

gaaaagtact tggcttaa 1218

<210> 167
<211> 405
<212> PRT
<213> Glycine max

<400> 167

Met Val Ala Met Ala Ala Ala Thr Ala Ser Thr Gln Leu Ile Phe Ser
1 5 10 15

Lys Pro Cys Ser Pro Ser Arg Leu Cys Pro Phe Gln Leu Cys Val Phe
20 25 30

Asp Thr Lys Gln Val Leu Ser Ser Gly Arg Arg Arg His Val Gly Gly
35 40 45

Ser Gly Val Arg Cys Met Ala Val Gly Glu Ala Ala Thr Thr Gly Thr
50 55 60

Lys Lys Arg Ser Gly Tyr Glu Leu Gln Thr Leu Thr Ser Trp Leu Leu
65 70 75 80

Lys Gln Glu Gln Ala Gly Val Ile Asp Ala Glu Leu Thr Ile Val Leu
85 90 95

Ser Ser Ile Ser Met Ala Cys Lys Gln Ile Ala Ser Leu Val Gln Arg
100 105 110

Ala Asn Ile Ser Asn Leu Thr Gly Val Gln Gly Ala Val Asn Val Gln
115 120 125

Gly Glu Asp Gln Lys Lys Leu Asp Val Val Ser Asn Glu Val Phe Ser
130 135 140

Asn Cys Leu Arg Ser Ser Gly Arg Thr Gly Ile Ile Ala Ser Glu Glu
145 150 155 160

Glu Asp Val Pro Val Ala Val Glu Glu Ser Tyr Ser Gly Asn Tyr Ile
165 170 175

Val Val Phe Asp Pro Leu Asp Gly Ser Ser Asn Ile Asp Ala Ala Ala

```
            180                 185                 190

Ser Thr Gly Ser Asn Phe Trp Ile Tyr Ser Pro Asn Asp Glu Cys Leu
        195                 200                 205

Ala Asp Ile Asp Asp Asp Pro Thr Leu Asp Thr Thr Glu Gln Arg Cys
    210                 215                 220

Ile Val Asn Val Cys Gln Pro Gly Ser Asn Leu Leu Ala Ala Gly Tyr
225                 230                 235                 240

Cys Met Tyr Ser Ser Ser Ile Ile Phe Val Leu Thr Leu Gly Asn Gly
                245                 250                 255

Val Phe Val Phe Thr Leu Asp Pro Met Tyr Gly Glu Phe Val Leu Thr
            260                 265                 270

Gln Glu Asn Leu Gln Ile Pro Arg Ala Gly Lys Ile Tyr Ala Phe Asn
        275                 280                 285

Glu Gly Asn Tyr Gln Leu Trp Asp Glu Lys Leu Lys Lys Tyr Ile Asp
    290                 295                 300

Asp Leu Lys Asp Pro Gly Gln Ser Gly Lys Pro Tyr Ser Ala Arg Tyr
305                 310                 315                 320

Ile Gly Ser Leu Val Gly Asp Phe His Arg Thr Leu Leu Tyr Gly Gly
                325                 330                 335

Ile Tyr Gly Tyr Pro Arg Asp Lys Lys Ser Lys Asn Gly Lys Leu Arg
            340                 345                 350

Leu Leu Tyr Glu Cys Ala Pro Ile Asn Phe Ile Val Glu Gln Ala Gly
        355                 360                 365

Gly Lys Gly Thr Asp Gly Leu Gln Val Leu Arg Leu Gln Gly Thr Glu
    370                 375                 380

Ile His Gln Arg Val Pro Leu Tyr Ile Gly Glu Glu Val Glu Lys Val
385                 390                 395                 400

Glu Lys Tyr Leu Ala
                405
```

<210> 168
<211> 1212
<212> DNA
<213> Lycopersicon esculentum

<400> 168

atggcagcaa cagcaacaac ttcatattta agtgctctag acaaaaagac tccattttta 60
tttgccttgg acaaaaagac tccattttta tgcccaaaaa acagcacaaa gagaaggtca 120
tttaatggag gagttaagtg catggcaata gagacagctt caggtgttac acaaaccaag 180
aaaaagagtg gctatgagtt acaaacttta acaagttggc tattgagaca agaacaagct 240
ggagttattg atgctgaact taccatagta atttcaagta tttcaatggc ttgtaaacag 300
attgcttctt tggtccaaag agctggaatt tctaacctta ctggagttca aggtgctgtc 360
aatattcaag gagaagatca gaagaaactt gatgttgtct ctaatgaggt tttctcgaat 420
tgtctaagat caagtggaag gactgggatt atagcatcag aagaagagga tgtacctgtg 480
gcagtggaag agagttactc aggcaactac attgtggtgt ttgatcctct tgatggatca 540
tcaaacattg atgctgctgt atctaccggt tctatctttg gaatatacag cccgaatgat 600
gagtgcctag ctgatcttgg agatgattcc acgcttgaca atatagagca aaagtgcatc 660
gtgaatgtat gtcaaccagg gacaaacctt cttgcagcag gatactgcat gtactcaagc 720
tctgtgattt tcgtactcac cttgggaaat ggcgtttttt cctttaactt ggatccaatg 780
tatggagaat ttgttctgac tcaagaaaat gtccaaatac caaagtctgg aaagatctat 840
tcattcaatg aaggaaacta ccagctctgg gatgacaagt tgaaaaaata tatcgatgac 900
ttgaaagacc ctggtcctag tggcaagcct tactctgcaa ggtacattgg tagtttggta 960
ggtgacttcc atagaactct tctatatggt ggcatttatg gttatcctag agacagaaag 1020
agcaagaatg gaaagttgag gcttttgtac gaatgtgctc ccatgagctt cattgtggaa 1080
caagctggtg gcaaaggatc cgatggtcac caaagagttc tcgatatcca accaactgag 1140
atacatcaac gagttccatt gtacattgga agcacagaag aagttgaaaa attggagaag 1200
tacttgtctt aa 1212

<210> 169
<211> 403
<212> PRT
<213> Lycopersicon esculentum

<400> 169

Met Ala Ala Thr Ala Thr Thr Ser Tyr Leu Ser Ala Leu Asp Lys Lys
1 5 10 15

Thr Pro Phe Leu Phe Ala Leu Asp Lys Lys Thr Pro Phe Leu Cys Pro
20 25 30

Lys Asn Ser Thr Lys Arg Arg Ser Phe Asn Gly Gly Val Lys Cys Met
35 40 45

Ala Ile Glu Thr Ala Ser Gly Val Thr Gln Thr Lys Lys Lys Ser Gly
50 55 60

```
Tyr Glu Leu Gln Thr Leu Thr Ser Trp Leu Leu Arg Gln Glu Gln Ala
65                  70                  75                  80

Gly Val Ile Asp Ala Glu Leu Thr Ile Val Ile Ser Ser Ile Ser Met
                85                  90                  95

Ala Cys Lys Gln Ile Ala Ser Leu Val Gln Arg Ala Gly Ile Ser Asn
            100                 105                 110

Leu Thr Gly Val Gln Gly Ala Val Asn Ile Gln Gly Glu Asp Gln Lys
        115                 120                 125

Lys Leu Asp Val Val Ser Asn Glu Val Phe Ser Asn Cys Leu Arg Ser
    130                 135                 140

Ser Gly Arg Thr Gly Ile Ile Ala Ser Glu Glu Glu Asp Val Pro Val
145                 150                 155                 160

Ala Val Glu Glu Ser Tyr Ser Gly Asn Tyr Ile Val Val Phe Asp Pro
                165                 170                 175

Leu Asp Gly Ser Ser Asn Ile Asp Ala Ala Val Ser Thr Gly Ser Ile
            180                 185                 190

Phe Gly Ile Tyr Ser Pro Asn Asp Glu Cys Leu Ala Asp Leu Gly Asp
        195                 200                 205

Asp Ser Thr Leu Asp Asn Ile Glu Gln Lys Cys Ile Val Asn Val Cys
    210                 215                 220

Gln Pro Gly Thr Asn Leu Leu Ala Ala Gly Tyr Cys Met Tyr Ser Ser
225                 230                 235                 240

Ser Val Ile Phe Val Leu Thr Leu Gly Asn Gly Val Phe Ser Phe Asn
                245                 250                 255

Leu Asp Pro Met Tyr Gly Glu Phe Val Leu Thr Gln Glu Asn Val Gln
            260                 265                 270

Ile Pro Lys Ser Gly Lys Ile Tyr Ser Phe Asn Glu Gly Asn Tyr Gln
        275                 280                 285

Leu Trp Asp Asp Lys Leu Lys Lys Tyr Ile Asp Asp Leu Lys Asp Pro
    290                 295                 300

Gly Pro Ser Gly Lys Pro Tyr Ser Ala Arg Tyr Ile Gly Ser Leu Val
305                 310                 315                 320
```

Gly Asp Phe His Arg Thr Leu Leu Tyr Gly Gly Ile Tyr Gly Tyr Pro
325 330 335

Arg Asp Arg Lys Ser Lys Asn Gly Lys Leu Arg Leu Leu Tyr Glu Cys
340 345 350

Ala Pro Met Ser Phe Ile Val Glu Gln Ala Gly Gly Lys Gly Ser Asp
355 360 365

Gly His Gln Arg Val Leu Asp Ile Gln Pro Thr Glu Ile His Gln Arg
370 375 380

Val Pro Leu Tyr Ile Gly Ser Thr Glu Glu Val Glu Lys Leu Glu Lys
385 390 395 400

Tyr Leu Ser

<210> 170
<211> 1236
<212> DNA
<213> Medicago truncatula

<400> 170
atggttgcaa tggcagcagc aacagcatca tcacaattaa tattctcaaa accttgttct 60

ccctcacgtc tatgtccctt ccaactttgt gttttcgaca cgaaatcagt gttatcaagt 120

tcaagaagaa agcatgtgag tggctctgga ggagtgagat gcatggctgt tggtgaagta 180

gctgctgaga caaagaaaag aagtagttat gagcttataa cattgactag ttggttgttg 240

aagcaagaac aaacaggggt tattgatgct gaacttacta ttgttttgaa tagtatttcg 300

ttggcatgta aacagattgc ttctttggtt caaagagcta atatttctaa ccttactggt 360

gttcaaggtg ctgtaaatat tcaaggggag gatcagaaaa aacttgatgt tgtctcaaat 420

gaggtattct caaattgttt gaggtcaagt gggaggacag ggattatagc atcagaggaa 480

gaggatgtgc cagtggcagt agaagagagt tattcaggaa actacattgt ggtctttgat 540

ccacttgatg gttcatcgaa tattgatgct gcagtttcaa ctggttctat ttttgggatt 600

tacagcccca atgatgagtg tcttgctgac gtaggcaatg agtccgatga ccccacactt 660

ggcacagaag aacaacgatg cattgtgaat gtgtgccaac caggaagcaa ccttctagca 720

gccggttact gcatgtattc tagctcagta atcttcgttc taacaatcgg caaaggagtt 780

ttcgtattca cattagatcc aatgtatggg gaatttgttt tgactcaaga aaatctccaa 840

ataccaaaat cagggaaaat ttattctttc aatgaaggaa attacaagtt atgggatgac 900

aacttgaaga aatacatcga tgatctcaag gaaccgggtg ctaatggcaa accttattca 960

gcaaggtata ttggtagttt ggtaggtgat ttccatagga cactgctata tggtggcatt 1020

tatggttacc ctagggacaa gaaaagtaag aatggaaggc ttaggctttt atatgagtgt 1080

gctccaatga gtttcattgt agaacaggct ggtggaaaag gttcagatgg tcatcaaaga 1140

gtacttgaca ttcaacccac cgaaattcat caacgtgttc cactgtacat tgggagcaca 1200

gaggaagtgg agaaggttga aaagtacttg gcttaa 1236

<210> 171
<211> 411
<212> PRT
<213> Medicago truncatula

<400> 171

Met Val Ala Met Ala Ala Ala Thr Ala Ser Ser Gln Leu Ile Phe Ser
1 5 10 15

Lys Pro Cys Ser Pro Ser Arg Leu Cys Pro Phe Gln Leu Cys Val Phe
20 25 30

Asp Thr Lys Ser Val Leu Ser Ser Ser Arg Arg Lys His Val Ser Gly
35 40 45

Ser Gly Gly Val Arg Cys Met Ala Val Gly Glu Val Ala Ala Glu Thr
50 55 60

Lys Lys Arg Ser Ser Tyr Glu Leu Ile Thr Leu Thr Ser Trp Leu Leu
65 70 75 80

Lys Gln Glu Gln Thr Gly Val Ile Asp Ala Glu Leu Thr Ile Val Leu
85 90 95

Asn Ser Ile Ser Leu Ala Cys Lys Gln Ile Ala Ser Leu Val Gln Arg
100 105 110

Ala Asn Ile Ser Asn Leu Thr Gly Val Gln Gly Ala Val Asn Ile Gln
115 120 125

Gly Glu Asp Gln Lys Lys Leu Asp Val Val Ser Asn Glu Val Phe Ser
130 135 140

Asn Cys Leu Arg Ser Ser Gly Arg Thr Gly Ile Ile Ala Ser Glu Glu
145 150 155 160

Glu Asp Val Pro Val Ala Val Glu Glu Ser Tyr Ser Gly Asn Tyr Ile
165 170 175

Val Val Phe Asp Pro Leu Asp Gly Ser Ser Asn Ile Asp Ala Ala Val
180 185 190

Ser Thr Gly Ser Ile Phe Gly Ile Tyr Ser Pro Asn Asp Glu Cys Leu
195 200 205

Ala Asp Val Gly Asn Glu Ser Asp Asp Pro Thr Leu Gly Thr Glu Glu
    210                 215                 220

Gln Arg Cys Ile Val Asn Val Cys Gln Pro Gly Ser Asn Leu Leu Ala
225                 230                 235                 240

Ala Gly Tyr Cys Met Tyr Ser Ser Ser Val Ile Phe Val Leu Thr Ile
                245                 250                 255

Gly Lys Gly Val Phe Val Phe Thr Leu Asp Pro Met Tyr Gly Glu Phe
            260                 265                 270

Val Leu Thr Gln Glu Asn Leu Gln Ile Pro Lys Ser Gly Lys Ile Tyr
        275                 280                 285

Ser Phe Asn Glu Gly Asn Tyr Lys Leu Trp Asp Asp Asn Leu Lys Lys
    290                 295                 300

Tyr Ile Asp Asp Leu Lys Glu Pro Gly Ala Asn Gly Lys Pro Tyr Ser
305                 310                 315                 320

Ala Arg Tyr Ile Gly Ser Leu Val Gly Asp Phe His Arg Thr Leu Leu
                325                 330                 335

Tyr Gly Gly Ile Tyr Gly Tyr Pro Arg Asp Lys Lys Ser Lys Asn Gly
            340                 345                 350

Arg Leu Arg Leu Leu Tyr Glu Cys Ala Pro Met Ser Phe Ile Val Glu
        355                 360                 365

Gln Ala Gly Gly Lys Gly Ser Asp Gly His Gln Arg Val Leu Asp Ile
    370                 375                 380

Gln Pro Thr Glu Ile His Gln Arg Val Pro Leu Tyr Ile Gly Ser Thr
385                 390                 395                 400

Glu Glu Val Glu Lys Val Glu Lys Tyr Leu Ala
                405                 410

<210> 172
<211> 1236
<212> DNA
<213> Nicotiana tabacum

<400> 172
atggcagcat catcagcaac agcaacagca acaacttcat ttttatgtgc tttagacaaa 60

aagactccat ttttatgtac tctagacaaa aaaggtactc catttttatg cccaaaaggc 120

agcagcacaa caaagagaag gtcatttaat ggaggagtga agtgcatggc aatagagaca 180

acagcaggag ctacagagac caagaaaaga agtggctatg agttacaaac tttaacaagc 240
tggctattaa ggcaagaaca agctgggagt attgatgctg aacttaccat agtgatttca 300
agtatttcta tggcttgtaa gcagattgct tctttggttc agagagctgg aatttctaac 360
cttactggag ttcaaggtgc tgtcaatatt caaggagaag accagaagaa gcttgatgtt 420
gtctctaacg aggttttctc gaattgtcta aggtcgagtg gaaggactgg gattatagca 480
tcagaggaag aggatgtacc agtggcagtg gaagagagtt actcaggaaa ctacattgtg 540
gtgtttgacc ctcttgatgg atcatcaaac attgatgctg ctgtttctac tggttctatt 600
ttcggaatat acagcccaaa tgatgagtgc ctcgcagatc atggagatga ttccacgctt 660
gacaatgttg aacagaggtg tattgtgaat gtatgccaac cagggagcaa ccttcttgca 720
gcaggctact gcatgtactc aagctctgtg atttttgtgg tcaccttggg aaacggagtc 780
tttgccttca acttggatcc gatgtatgga gaattcgttc tgacccaaga aaacatccaa 840
ataccaaaat ctggaaagat ctattcgttc aacgaaggaa actaccagct ttgggatgac 900
aaactgaaga aatacatcga tgacttgaag gaccctggtc ctagcggcaa gccttactct 960
gctaggtaca ttggtagttt ggttggtgac ttccatagaa ctcttctata tggtggcatt 1020
tatggctatc ctagagataa aaagagtaag aatggaaagt tgaggctttt gtatgagtgt 1080
gcccctatga gcttcctcgt ggaacaagct ggtggcaaag gatccgatgg ccaccaaaga 1140
gttcttgata tccaaccaac tgagatacac cagcgagtcc cattgtacat tggaagcaca 1200
gaagaagttg aaaagttgga gaagtattta tcttaa 1236

<210> 173
<211> 411
<212> PRT
<213> Nicotiana tabacum

<400> 173

Met Ala Ala Ser Ser Ala Thr Ala Thr Ala Thr Thr Ser Phe Leu Cys
1 5 10 15

Ala Leu Asp Lys Lys Thr Pro Phe Leu Cys Thr Leu Asp Lys Lys Gly
20 25 30

Thr Pro Phe Leu Cys Pro Lys Gly Ser Ser Thr Thr Lys Arg Arg Ser
35 40 45

Phe Asn Gly Gly Val Lys Cys Met Ala Ile Glu Thr Thr Ala Gly Ala
50 55 60

Thr Glu Thr Lys Lys Arg Ser Gly Tyr Glu Leu Gln Thr Leu Thr Ser
65 70 75 80

Trp Leu Leu Arg Gln Glu Gln Ala Gly Ser Ile Asp Ala Glu Leu Thr
85 90 95

Ile Val Ile Ser Ser Ile Ser Met Ala Cys Lys Gln Ile Ala Ser Leu
100 105 110

Val Gln Arg Ala Gly Ile Ser Asn Leu Thr Gly Val Gln Gly Ala Val
115 120 125

Asn Ile Gln Gly Glu Asp Gln Lys Lys Leu Asp Val Val Ser Asn Glu
130 135 140

Val Phe Ser Asn Cys Leu Arg Ser Ser Gly Arg Thr Gly Ile Ile Ala
145 150 155 160

Ser Glu Glu Glu Asp Val Pro Val Ala Val Glu Glu Ser Tyr Ser Gly
165 170 175

Asn Tyr Ile Val Val Phe Asp Pro Leu Asp Gly Ser Ser Asn Ile Asp
180 185 190

Ala Ala Val Ser Thr Gly Ser Ile Phe Gly Ile Tyr Ser Pro Asn Asp
195 200 205

Glu Cys Leu Ala Asp His Gly Asp Asp Ser Thr Leu Asp Asn Val Glu
210 215 220

Gln Arg Cys Ile Val Asn Val Cys Gln Pro Gly Ser Asn Leu Leu Ala
225 230 235 240

Ala Gly Tyr Cys Met Tyr Ser Ser Ser Val Ile Phe Val Val Thr Leu
245 250 255

Gly Asn Gly Val Phe Ala Phe Asn Leu Asp Pro Met Tyr Gly Glu Phe
260 265 270

Val Leu Thr Gln Glu Asn Ile Gln Ile Pro Lys Ser Gly Lys Ile Tyr
275 280 285

Ser Phe Asn Glu Gly Asn Tyr Gln Leu Trp Asp Asp Lys Leu Lys Lys
290 295 300

Tyr Ile Asp Asp Leu Lys Asp Pro Gly Pro Ser Gly Lys Pro Tyr Ser
305 310 315 320

Ala Arg Tyr Ile Gly Ser Leu Val Gly Asp Phe His Arg Thr Leu Leu
325 330 335

Tyr Gly Gly Ile Tyr Gly Tyr Pro Arg Asp Lys Lys Ser Lys Asn Gly

340 345 350

Lys Leu Arg Leu Leu Tyr Glu Cys Ala Pro Met Ser Phe Leu Val Glu
355 360 365

Gln Ala Gly Gly Lys Gly Ser Asp Gly His Gln Arg Val Leu Asp Ile
370 375 380

Gln Pro Thr Glu Ile His Gln Arg Val Pro Leu Tyr Ile Gly Ser Thr
385 390 395 400

Glu Glu Val Glu Lys Leu Glu Lys Tyr Leu Ser
405 410

<210> 174
<211> 1221
<212> DNA
<213> Oryza sativa

<400> 174
atggccgccg cagccacgac atcctcccac ctgctcctgc tctcccgcca gcaggcggcg 60
gcctcgctcc aatgcggcct ctccttccgg aggcagcccg gcaggctcgc cggtgggtcg 120
tcggccccga gcgtgcggtg catggcggcc gtcgacacgg cctcggcgcc cgccgcgacg 180
gaggctagca agaagagcag ctacgagatc accacgctga cgacgtggct gctgaagcag 240
gagcaggccg ggaccatcga cggcgagatg accatcgtgc tggccagcat ctccacggcg 300
tgcaagcaga tcgcctcgct ggtgcagcgc gcgcccatct ccaacctcac cggcgtccag 360
ggcgccgtca acgtgcaggg cgaggaccag aaaaaactcg acgtcgtctc caacgaggtg 420
ttctccaact gcctcaaatc gagcgggcgc accggcgtga tcgcgtcgga ggaggaggac 480
gtgccggtgg ccgtggagga gagctactcg ggcaactaca tcgtggtgtt cgacccgctc 540
gacggctcct ccaacatcga cgccgccgtc tccaccggct ccatcttcgg catctacagc 600
cccaacgacg agtgcctagc cgacatcgcc gacgaccaaa atcttgacca ggtggagcag 660
aggtgcatcg tgagcgtgtg ccagccgggg agcaacctgc tcgccgccgg ctactgcatg 720
tactcgagct cggtcatctt cgtgctcacc atcgggacag ggtgtacgt gttcacgctg 780
gacccgatgt acggcgagtt cgtgctgacg caggagaagg tgcagatccc caaggcaggc 840
aagatctatg ccttcaacga gggcaactac gcgctctggg acgacaagct caagagctac 900
atggacagcc tcaaggagcc cgggccgtcc gggaagccat actccgcgcg ctacatcggc 960
agcctcgtcg gcgacttcca ccgcacgctg ctctacggcg gcatctacgg ctaccccagg 1020
gaccagaaga gcaagaacgg caagctgcgg ctgctgtacg agtgcgcgcc gatgagcttc 1080
atcgtcgagc aggccggcgg caagggctcc gatggccacc agaggatact tgacatcatg 1140
cctacggaga tccatcagag agtgccgctg tacatcggga gcgtggagga agtggagaag 1200

gtcgagaagt tcttggcttg a 1221

<210> 175
<211> 406
<212> PRT
<213> Oryza sativa

<400> 175

Met Ala Ala Ala Ala Thr Thr Ser Ser His Leu Leu Leu Leu Ser Arg
1 5 10 15

Gln Gln Ala Ala Ala Ser Leu Gln Cys Gly Leu Ser Phe Arg Arg Gln
20 25 30

Pro Gly Arg Leu Ala Gly Gly Ser Ser Ala Pro Ser Val Arg Cys Met
35 40 45

Ala Ala Val Asp Thr Ala Ser Ala Pro Ala Ala Thr Glu Ala Ser Lys
50 55 60

Lys Ser Ser Tyr Glu Ile Thr Thr Leu Thr Thr Trp Leu Leu Lys Gln
65 70 75 80

Glu Gln Ala Gly Thr Ile Asp Gly Glu Met Thr Ile Val Leu Ala Ser
85 90 95

Ile Ser Thr Ala Cys Lys Gln Ile Ala Ser Leu Val Gln Arg Ala Pro
100 105 110

Ile Ser Asn Leu Thr Gly Val Gln Gly Ala Val Asn Val Gln Gly Glu
115 120 125

Asp Gln Lys Lys Leu Asp Val Val Ser Asn Glu Val Phe Ser Asn Cys
130 135 140

Leu Lys Ser Ser Gly Arg Thr Gly Val Ile Ala Ser Glu Glu Glu Asp
145 150 155 160

Val Pro Val Ala Val Glu Glu Ser Tyr Ser Gly Asn Tyr Ile Val Val
165 170 175

Phe Asp Pro Leu Asp Gly Ser Ser Asn Ile Asp Ala Ala Val Ser Thr
180 185 190

Gly Ser Ile Phe Gly Ile Tyr Ser Pro Asn Asp Glu Cys Leu Ala Asp
195 200 205

Ile Ala Asp Asp Gln Asn Leu Asp Gln Val Glu Gln Arg Cys Ile Val
210 215 220

Ser Val Cys Gln Pro Gly Ser Asn Leu Leu Ala Ala Gly Tyr Cys Met
225 230 235 240

Tyr Ser Ser Ser Val Ile Phe Val Leu Thr Ile Gly Thr Gly Val Tyr
245 250 255

Val Phe Thr Leu Asp Pro Met Tyr Gly Glu Phe Val Leu Thr Gln Glu
260 265 270

Lys Val Gln Ile Pro Lys Ala Gly Lys Ile Tyr Ala Phe Asn Glu Gly
275 280 285

Asn Tyr Ala Leu Trp Asp Asp Lys Leu Lys Ser Tyr Met Asp Ser Leu
290 295 300

Lys Glu Pro Gly Pro Ser Gly Lys Pro Tyr Ser Ala Arg Tyr Ile Gly
305 310 315 320

Ser Leu Val Gly Asp Phe His Arg Thr Leu Leu Tyr Gly Gly Ile Tyr
325 330 335

Gly Tyr Pro Arg Asp Gln Lys Ser Lys Asn Gly Lys Leu Arg Leu Leu
340 345 350

Tyr Glu Cys Ala Pro Met Ser Phe Ile Val Glu Gln Ala Gly Gly Lys
355 360 365

Gly Ser Asp Gly His Gln Arg Ile Leu Asp Ile Met Pro Thr Glu Ile
370 375 380

His Gln Arg Val Pro Leu Tyr Ile Gly Ser Val Glu Glu Val Glu Lys
385 390 395 400

Val Glu Lys Phe Leu Ala
405

<210> 176
<211> 1143
<212> DNA
<213> Ostreococcus lucimarinus

<400> 176
atgtctgccg ccacctccgc ctgcgctcgc gcgatcgtcg cgacgaagaa gaccaccgcg 60

gcgcgccgcg cgggcaagtc ggcgacgcgg gcgacgccgc gatccgtcgc ggcgcgcgcg 120

gggggcgcgg gcacgccgtt cacgacgtgg atcttgcaac aagagatgga agaaaagatc 180

gacggcgaac tcgcggtcgt gttgtcgagc atcggcttgg cgtgcaagca aatcgcgagc 240

ttggtgcaac gcgccgggct tcaaggcatg acgggtttgg cgggcgagac gaacgtgcaa 300

ggtgaagacc aaaagaagct cgacgtcatc tccaacgatg tgttctgcga tgtcttgcgt 360

caaaccggcc gcacgggcgt gattgcgtcc gaggaagaag acgtgccggt cgccgtcgaa 420

gaaaccttcg gcggtaacta cgtcgtcgtc ttcgatccgc tcgatggctc ttccaacatc 480

gacgccgccg tttccacggg ttccatctgg ggcatctacg agtccgactc cacgtgcatc 540

ccggattttg gcaccgaaga tgcggccaag attgaagaga agtgcgtcat gaacgtgtgc 600

caaccgggga acaacttgtt gtgcgcgggc tactgcatgt actcctcttc caccatcctc 660

gtcttgaccc tcggtgaggg tgtgtacggt ttcaccctcg acccgaccgt cggcgagttc 720

atcatgtccc acgacaacat caaggttccg gaatctggta agatttactc cttcaacgaa 780

ggcaactacg acatgtggac tccgggcttg aagaagtaca tggactccct caagactggc 840

ggcgcggaac aaggcaccaa gccgtacagc gcccgttaca tcggttccct cgtcggtgac 900

ttccacagaa ccatcttgta cggaggcatc tacggctacc cgggcgactc caagaacccg 960

aacggtaagc ttcgcctctt gtacgagtgc gcgccgatgt ccttcatcgc cgaacaagcc 1020

ggcggtatgg gttccaccgg taaggagcgc gtccttgacg ttgtcccgga aaagtttcac 1080

caacgtgtgc cgttcttcac cggctccaag aaggaagtgc agtacttgga atccttcatg 1140

tag 1143

<210> 177
<211> 380
<212> PRT
<213> Ostreococcus lucimarinus

<400> 177

Met Ser Ala Ala Thr Ser Ala Cys Ala Arg Ala Ile Val Ala Thr Lys
1 5 10 15

Lys Thr Thr Ala Ala Arg Arg Ala Gly Lys Ser Ala Thr Arg Ala Thr
20 25 30

Pro Arg Ser Val Ala Ala Arg Ala Gly Gly Ala Gly Thr Pro Phe Thr
35 40 45

Thr Trp Ile Leu Gln Gln Glu Met Glu Glu Lys Ile Asp Gly Glu Leu
50 55 60

Ala Val Val Leu Ser Ser Ile Gly Leu Ala Cys Lys Gln Ile Ala Ser
65 70 75 80

Leu Val Gln Arg Ala Gly Leu Gln Gly Met Thr Gly Leu Ala Gly Glu
85 90 95

Thr Asn Val Gln Gly Glu Asp Gln Lys Lys Leu Asp Val Ile Ser Asn
100 105 110

```
Asp Val Phe Cys Asp Val Leu Arg Gln Thr Gly Arg Thr Gly Val Ile
        115                 120                 125

Ala Ser Glu Glu Glu Asp Val Pro Val Ala Val Glu Glu Thr Phe Gly
    130                 135                 140

Gly Asn Tyr Val Val Val Phe Asp Pro Leu Asp Gly Ser Ser Asn Ile
145                 150                 155                 160

Asp Ala Ala Val Ser Thr Gly Ser Ile Trp Gly Ile Tyr Glu Ser Asp
                165                 170                 175

Ser Thr Cys Ile Pro Asp Phe Gly Thr Glu Asp Ala Ala Lys Ile Glu
            180                 185                 190

Glu Lys Cys Val Met Asn Val Cys Gln Pro Gly Asn Asn Leu Leu Cys
        195                 200                 205

Ala Gly Tyr Cys Met Tyr Ser Ser Ser Thr Ile Leu Val Leu Thr Leu
    210                 215                 220

Gly Glu Gly Val Tyr Gly Phe Thr Leu Asp Pro Thr Val Gly Glu Phe
225                 230                 235                 240

Ile Met Ser His Asp Asn Ile Lys Val Pro Glu Ser Gly Lys Ile Tyr
                245                 250                 255

Ser Phe Asn Glu Gly Asn Tyr Asp Met Trp Thr Pro Gly Leu Lys Lys
            260                 265                 270

Tyr Met Asp Ser Leu Lys Thr Gly Gly Ala Glu Gln Gly Thr Lys Pro
        275                 280                 285

Tyr Ser Ala Arg Tyr Ile Gly Ser Leu Val Gly Asp Phe His Arg Thr
    290                 295                 300

Ile Leu Tyr Gly Gly Ile Tyr Gly Tyr Pro Gly Asp Ser Lys Asn Pro
305                 310                 315                 320

Asn Gly Lys Leu Arg Leu Leu Tyr Glu Cys Ala Pro Met Ser Phe Ile
                325                 330                 335

Ala Glu Gln Ala Gly Gly Met Gly Ser Thr Gly Lys Glu Arg Val Leu
            340                 345                 350

Asp Val Val Pro Glu Lys Phe His Gln Arg Val Pro Phe Phe Thr Gly
        355                 360                 365
```

Ser Lys Lys Glu Val Gln Tyr Leu Glu Ser Phe Met
370 375 380

<210> 178
<211> 1134
<212> DNA
<213> Ostreococcus tauri

<400> 178
atgtctgcgt gtatttctgc ctctgtgacg cgcgcgcgaa cggcgcgcgc gcccgcggct 60

cgccgcgcgg cgagtaaggc gcgggcgtcg ccgcgcgccg tcgcggcgcg cgcggggggc 120

gttggtacgc cgtacacgac gtggattctc cagcaagaga tgcaagagaa cattgatggt 180

gaattggcgg tggttttgtc ctccatcggt ctcgcgtgca agcaaatcgc gagcctcgtc 240

cagcgcgcgg gtctcgcggg catgactggt ttggcgggcg agcaaaacgt gcaaggcgag 300

gaccagaaga agctcgacgt catctccaac gatgttttct gcaacgtatt gcgccaatcc 360

ggccgcacgg gcgtcatcgc ctccgaagaa gaagacgttc cggtcgccgt cgaggaaacg 420

tacggcggta actacgtcgt cgtcttcgat ccgcttgatg gttcctccaa tatcgacgcc 480

gccgtctcca cgggatccat ctggggtatc tacgagtccg actcctcgtg tattcccgac 540

ttcggctccg atgactccgc caaggttgaa gagaagtgcg tcatgaacgt ttgccaaccg 600

ggcagcaact tgctctgcgc gggttactgc atgtactcct cgtccaccat cctcgtcatc 660

accatcggcc aaggcgtgtt cggttttacc ctcgacccga ccgtcggtga gttcatcatg 720

tcccacgaga acatcaaggt tccggactct ggcaagattt actctttcaa cgaaggcaac 780

tacgccatgt ggtccgacgg tttgaagaag tacatggact ccctcaagac gggcggcaag 840

gacggtggca agccgtacag cgcccgctac atcggttcgc tcgtcggtga cttccaccgc 900

acgatccttt acggaggcat ctacggttac ccgggtgacg cgaagaaccc gaacggtaag 960

ctccgcctcc tgtacgagtg cgcgccgatg tccatgatcg ctgaacaagc cggtggtaag 1020

ggctccaccg gtgtcgcgcg tgtcctggac atcgttccgg aaaaggttca ccagcgcgtg 1080

ccgttcttcg ttggctccaa gaacgaggtt gcctacttgg agtccttcat gtga 1134

<210> 179
<211> 377
<212> PRT
<213> Ostreococcus tauri

<400> 179

Met Ser Ala Cys Ile Ser Ala Ser Val Thr Arg Ala Arg Thr Ala Arg
1 5 10 15

Ala Pro Ala Ala Arg Arg Ala Ala Ser Lys Ala Arg Ala Ser Pro Arg
20 25 30

Ala Val Ala Ala Arg Ala Gly Gly Val Gly Thr Pro Tyr Thr Thr Trp

```
            35                  40                  45

Ile Leu Gln Gln Glu Met Gln Glu Asn Ile Asp Gly Glu Leu Ala Val
    50                  55                  60

Val Leu Ser Ser Ile Gly Leu Ala Cys Lys Gln Ile Ala Ser Leu Val
65                  70                  75                  80

Gln Arg Ala Gly Leu Ala Gly Met Thr Gly Leu Ala Gly Glu Gln Asn
                85                  90                  95

Val Gln Gly Glu Asp Gln Lys Lys Leu Asp Val Ile Ser Asn Asp Val
            100                 105                 110

Phe Cys Asn Val Leu Arg Gln Ser Gly Arg Thr Gly Val Ile Ala Ser
        115                 120                 125

Glu Glu Glu Asp Val Pro Val Ala Val Glu Glu Thr Tyr Gly Gly Asn
    130                 135                 140

Tyr Val Val Val Phe Asp Pro Leu Asp Gly Ser Ser Asn Ile Asp Ala
145                 150                 155                 160

Ala Val Ser Thr Gly Ser Ile Trp Gly Ile Tyr Glu Ser Asp Ser Ser
                165                 170                 175

Cys Ile Pro Asp Phe Gly Ser Asp Asp Ser Ala Lys Val Glu Glu Lys
            180                 185                 190

Cys Val Met Asn Val Cys Gln Pro Gly Ser Asn Leu Leu Cys Ala Gly
        195                 200                 205

Tyr Cys Met Tyr Ser Ser Ser Thr Ile Leu Val Ile Thr Ile Gly Gln
    210                 215                 220

Gly Val Phe Gly Phe Thr Leu Asp Pro Thr Val Gly Glu Phe Ile Met
225                 230                 235                 240

Ser His Glu Asn Ile Lys Val Pro Asp Ser Gly Lys Ile Tyr Ser Phe
                245                 250                 255

Asn Glu Gly Asn Tyr Ala Met Trp Ser Asp Gly Leu Lys Lys Tyr Met
            260                 265                 270

Asp Ser Leu Lys Thr Gly Gly Lys Asp Gly Gly Lys Pro Tyr Ser Ala
        275                 280                 285

Arg Tyr Ile Gly Ser Leu Val Gly Asp Phe His Arg Thr Ile Leu Tyr
    290                 295                 300
```

Gly Gly Ile Tyr Gly Tyr Pro Gly Asp Ala Lys Asn Pro Asn Gly Lys
305 310 315 320

Leu Arg Leu Leu Tyr Glu Cys Ala Pro Met Ser Met Ile Ala Glu Gln
325 330 335

Ala Gly Gly Lys Gly Ser Thr Gly Val Ala Arg Val Leu Asp Ile Val
340 345 350

Pro Glu Lys Val His Gln Arg Val Pro Phe Phe Val Gly Ser Lys Asn
355 360 365

Glu Val Ala Tyr Leu Glu Ser Phe Met
370 375

<210> 180
<211> 1254
<212> DNA
<213> Phaeodactylum tricornutum

<400> 180
atgtttattt tgaagtcccc ggcgctttgg ttgcttcttt acccagttgt tgcttttacg 60

gcggcgaggg cgaactcgat tcgtccagcg gccgcgttat ctgtcttcga tctgtcgagc 120

gtggaggcag taccgtctag aaagaccaag gccccaattt tcgatgaagt ttgcgacaca 180

actggagtca ctctcaaacg cttcatgacc gaggtttctc tactgaatcc tgaaatcgaa 240

gagctgacga cgctgtttgg tgcgattgaa accgcttgca aagccattgc aaatcttgtc 300

aagcgatcgc cgcttccctc cagtgacacg ttgggtcttc agggagaaat caacgttcaa 360

ggcgaagacc aaaagaaatt agatgtgatc gccaacgata ttttgaagcg agcactccgc 420

ttcacgggcc gcctcggagt cttagcctcg gaagaagagg atactcccgt cgatttgatg 480

ccaagggatc ctagtaccaa aaaagttcta atcgatgagg gagaaaagta tgtcgctgtc 540

ttcgatccgc tcgatggtag ctcaaacgtt gatgcaggca taccgacagg cacaataatt 600

gggatatacg agcacgacga aacttgcaag attgatcctg atgctttgga agaggatcgg 660

accaaacaag aaaacctatg cctcgcaaat actctgcagc ccggcaccaa cttggtagca 720

gcggcgtact gtttatattc ttcgtcaaca tttttggtgt tgacgctggg agctggaaca 780

tatggattca cgctagatga gactatcggt gaatttgtct tgagccatcc aaacattaag 840

attcctgaat gctcatccat tatgtcgttc aacgaggcaa atactcccag ctgggatcgt 900

ccgcttcaag acactttcgc aaagtggagg acagggacag gaaagagcgg caagaaattt 960

tcaagtcgct acattggttc tatggtaggg gatgtccatc ggacgctcct gtacggagga 1020

gtttttgggt atcctggcga caaaaagaat cccaacggga agctacgcct gctttatgaa 1080

ggagctccaa tgtcattcat catggaacag gcgggtggat tgtcgaccac tggcacgcag 1140

cgtgtcatgg aaatctcccc agatacggtc catcaacgtg tgccgattat catgggatcc 1200

agacaagatg tcgaagaggt catggacgcc tataaaaact ttggcatcga ataa 1254

<210> 181
<211> 417
<212> PRT
<213> Phaeodactylum tricornutum

<400> 181

Met Phe Ile Leu Lys Ser Pro Ala Leu Trp Leu Leu Leu Tyr Pro Val
1 5 10 15

Val Ala Phe Thr Ala Ala Arg Ala Asn Ser Ile Arg Pro Ala Ala Ala
20 25 30

Leu Ser Val Phe Asp Leu Ser Ser Val Glu Ala Val Pro Ser Arg Lys
35 40 45

Thr Lys Ala Pro Ile Phe Asp Glu Val Cys Asp Thr Thr Gly Val Thr
50 55 60

Leu Lys Arg Phe Met Thr Glu Val Ser Leu Leu Asn Pro Glu Ile Glu
65 70 75 80

Glu Leu Thr Thr Leu Phe Gly Ala Ile Glu Thr Ala Cys Lys Ala Ile
85 90 95

Ala Asn Leu Val Lys Arg Ser Pro Leu Pro Ser Ser Asp Thr Leu Gly
100 105 110

Leu Gln Gly Glu Ile Asn Val Gln Gly Glu Asp Gln Lys Lys Leu Asp
115 120 125

Val Ile Ala Asn Asp Ile Leu Lys Arg Ala Leu Arg Phe Thr Gly Arg
130 135 140

Leu Gly Val Leu Ala Ser Glu Glu Glu Asp Thr Pro Val Asp Leu Met
145 150 155 160

Pro Arg Asp Pro Ser Thr Lys Lys Val Leu Ile Asp Glu Gly Glu Lys
165 170 175

Tyr Val Ala Val Phe Asp Pro Leu Asp Gly Ser Ser Asn Val Asp Ala
180 185 190

Gly Ile Pro Thr Gly Thr Ile Ile Gly Ile Tyr Glu His Asp Glu Thr
195 200 205

Cys Lys Ile Asp Pro Asp Ala Leu Glu Glu Asp Arg Thr Lys Gln Glu
210 215 220

Asn Leu Cys Leu Ala Asn Thr Leu Gln Pro Gly Thr Asn Leu Val Ala
225 230 235 240

Ala Ala Tyr Cys Leu Tyr Ser Ser Ser Thr Phe Leu Val Leu Thr Leu
245 250 255

Gly Ala Gly Thr Tyr Gly Phe Thr Leu Asp Glu Thr Ile Gly Glu Phe
260 265 270

Val Leu Ser His Pro Asn Ile Lys Ile Pro Glu Cys Ser Ser Ile Met
275 280 285

Ser Phe Asn Glu Ala Asn Thr Pro Ser Trp Asp Arg Pro Leu Gln Asp
290 295 300

Thr Phe Ala Lys Trp Arg Thr Gly Thr Gly Lys Ser Gly Lys Lys Phe
305 310 315 320

Ser Ser Arg Tyr Ile Gly Ser Met Val Gly Asp Val His Arg Thr Leu
325 330 335

Leu Tyr Gly Gly Val Phe Gly Tyr Pro Gly Asp Lys Lys Asn Pro Asn
340 345 350

Gly Lys Leu Arg Leu Leu Tyr Glu Gly Ala Pro Met Ser Phe Ile Met
355 360 365

Glu Gln Ala Gly Gly Leu Ser Thr Thr Gly Thr Gln Arg Val Met Glu
370 375 380

Ile Ser Pro Asp Thr Val His Gln Arg Val Pro Ile Ile Met Gly Ser
385 390 395 400

Arg Gln Asp Val Glu Glu Val Met Asp Ala Tyr Lys Asn Phe Gly Ile
405 410 415

Glu

<210> 182
<211> 1233
<212> DNA
<213> Pisum sativa

<400> 182
atggttgcaa tggcagcagc aacagcctca tctcagttaa tattctcaaa accttactct 60

ccttcacgtc tttgcccctt ccaactctgt gtctttgatg caaaatcagt gttatcaagt 120

tcaaggagaa agcatgtgaa tggctctggt gttagatgta tggctgtgaa ggaagcaact 180

agtgagacaa agaaaagaag tggatatgag attataacac tgactagttg gttgttgcag 240

caagaacaaa aagggattat tgatgcagaa cttactattg tactttctag tatttctatg 300

gcatgtaaac aaattgcttc tttggttcaa agagccaata tttctaacct cactggtact 360

caaggtgctg taaatattca aggggaagac cagaaaaaac ttgatgttat ctcaaatgag 420

gtattctcaa attgcttgag gtcaagtggg aggacaggga taattgcgtc ggaggaagag 480

gatgtcgcgg tggcagtaga agagagttat tcaggaaact acattgttgt atttgatcca 540

cttgatggtt catccaatct tgatgctgca gtctcaaccg gttccatttt cgggatttac 600

agccccaatg acgagtgtct tcctgatttt ggtgatgact ctgatgacaa cacacttggc 660

acagaagaac aaaggtgcat tgtgaatgtg tgtcaaccag gaagcaacct tctagcagct 720

ggctactgca tgtattctag ttcagtagct tttgttctta ccataggcaa aggagtgttt 780

gtattcacat tagatccatt gtacggagaa ttcgttttga ctcaagagaa tctccaaata 840

ccgaaatcag ggaaatcta ttctttcaat gaagggaatt acaagttgtg ggatgaaaac 900

ttgaagaaat atattgatga tcttaaggaa ccaggtccta gtggcaagcc ttattcagca 960

aggtatattg gtagtttggt tggtgatttt cacaggacac tgttatatgg tggcatttat 1020

ggataccta gggacaagaa aagtaagaat gggaagctta ggcttttata tgaatgtgct 1080

ccaatgagct tcattgttga acaggctggt ggaaaaggtt cagatggtca tcaaagagta 1140

cttgacattc aacccacaga aattcatcaa cgtgttccac tttacattgg gagcacagaa 1200

gaggtggaga aggttgaaaa gtacttagct taa 1233

<210> 183
<211> 410
<212> PRT
<213> Pisum sativa

<400> 183

Met Val Ala Met Ala Ala Ala Thr Ala Ser Ser Gln Leu Ile Phe Ser
1 5 10 15

Lys Pro Tyr Ser Pro Ser Arg Leu Cys Pro Phe Gln Leu Cys Val Phe
20 25 30

Asp Ala Lys Ser Val Leu Ser Ser Ser Arg Arg Lys His Val Asn Gly
35 40 45

Ser Gly Val Arg Cys Met Ala Val Lys Glu Ala Thr Ser Glu Thr Lys
50 55 60

Lys Arg Ser Gly Tyr Glu Ile Ile Thr Leu Thr Ser Trp Leu Leu Gln
65 70 75 80

```
Gln Glu Gln Lys Gly Ile Ile Asp Ala Glu Leu Thr Ile Val Leu Ser
                85                  90                  95

Ser Ile Ser Met Ala Cys Lys Gln Ile Ala Ser Leu Val Gln Arg Ala
            100                 105                 110

Asn Ile Ser Asn Leu Thr Gly Thr Gln Gly Ala Val Asn Ile Gln Gly
        115                 120                 125

Glu Asp Gln Lys Lys Leu Asp Val Ile Ser Asn Glu Val Phe Ser Asn
    130                 135                 140

Cys Leu Arg Ser Ser Gly Arg Thr Gly Ile Ile Ala Ser Glu Glu Glu
145                 150                 155                 160

Asp Val Ala Val Ala Val Glu Glu Ser Tyr Ser Gly Asn Tyr Ile Val
                165                 170                 175

Val Phe Asp Pro Leu Asp Gly Ser Ser Asn Leu Asp Ala Ala Val Ser
            180                 185                 190

Thr Gly Ser Ile Phe Gly Ile Tyr Ser Pro Asn Asp Glu Cys Leu Pro
        195                 200                 205

Asp Phe Gly Asp Asp Ser Asp Asp Asn Thr Leu Gly Thr Glu Glu Gln
    210                 215                 220

Arg Cys Ile Val Asn Val Cys Gln Pro Gly Ser Asn Leu Leu Ala Ala
225                 230                 235                 240

Gly Tyr Cys Met Tyr Ser Ser Ser Val Ala Phe Val Leu Thr Ile Gly
                245                 250                 255

Lys Gly Val Phe Val Phe Thr Leu Asp Pro Leu Tyr Gly Glu Phe Val
            260                 265                 270

Leu Thr Gln Glu Asn Leu Gln Ile Pro Lys Ser Gly Glu Ile Tyr Ser
        275                 280                 285

Phe Asn Glu Gly Asn Tyr Lys Leu Trp Asp Glu Asn Leu Lys Lys Tyr
    290                 295                 300

Ile Asp Asp Leu Lys Glu Pro Gly Pro Ser Gly Lys Pro Tyr Ser Ala
305                 310                 315                 320

Arg Tyr Ile Gly Ser Leu Val Gly Asp Phe His Arg Thr Leu Leu Tyr
                325                 330                 335
```

Gly Gly Ile Tyr Gly Tyr Pro Arg Asp Lys Lys Ser Lys Asn Gly Lys
340 345 350

Leu Arg Leu Leu Tyr Glu Cys Ala Pro Met Ser Phe Ile Val Glu Gln
355 360 365

Ala Gly Gly Lys Gly Ser Asp Gly His Gln Arg Val Leu Asp Ile Gln
370 375 380

Pro Thr Glu Ile His Gln Arg Val Pro Leu Tyr Ile Gly Ser Thr Glu
385 390 395 400

Glu Val Glu Lys Val Glu Lys Tyr Leu Ala
405 410

<210> 184
<211> 1242
<212> DNA
<213> Poncirus trifoliata

<400> 184
atggttgcag cagcagcgac aacatcatca cagcttctct tttcaagctc tcactctttc 60
tctcggctct ctccttacca aatatgtgtc ttcgattcca aagcactcgt gtcgtcatgt 120
cccagcaacg ttatgaagag aagacatgtt ggtgttgctg ctggggttcg gtgcatggct 180
gttgggacaa catcggaggt tgcaaccaag aagagaagtt cgtatgagat tgaaacgctg 240
acaaactggc tgttgaagca agaacagtct ggcgttattg atgctgagct cactattgtg 300
ctttccagca tttcaacggc gtgcaagcag attgcttctt tggtgcaaag agctggcatt 360
tccaacttga ctggaattca gggtgctgtc aatgttcaag gcgaggacca gaagaagctc 420
gacgtcgttt caaatgaggt gttctcaaac tgtttgagat caagtgggcg aactgggatt 480
atagcatcag aggaagagga tgtaccagtg gcggtagaag agagctactc tggaaactat 540
attgtagttt ttgatccact tgatggatca tccaacattg atgctgcagt gtctactgga 600
tccatctttg gcatatacag cccaaatgat gagtgtcttg ccgatattgg tgatgattct 660
actctgggca atactgaaca aagatgtgta gtgaacgtgt gccagccagg aagcaacctt 720
cttgctgcag gctattgcat gtattcaagc tctgtaatct ttgtgataac tttaggcaac 780
ggagtctttg cattcaccct ggatcccatg tatggagaat ttgttttgac acaagagaac 840
attcagatac ccaaaaccgg aaagatctat gcttttaatg aaggcaacta ccagctttgg 900
gatgacaagt tgaagaagta cattgacgat cttaaggatc caggacccag cggcaagccc 960
tattctgcta ggtacattgg cagcttggtt ggtgatttcc atcgaactct gctctacggc 1020
ggcatttatg gctatccaag agacaagaag agcaagaatg gaaagctgag gctcttgtat 1080
gaatgtgcac caatgagctt catagtggaa caagcaggag gcaaaggatc tgacggccat 1140

caaagagtac ttgacattca acctactgag attcaccagc gtattccttt aaagattgga 1200

agccaggagg aagtggagaa actggagaag tatttggcct aa 1242

<210> 185
<211> 413
<212> PRT
<213> Poncirus trifoliata

<400> 185

Met Val Ala Ala Ala Ala Thr Thr Ser Ser Gln Leu Leu Phe Ser Ser
1 5 10 15

Ser His Ser Phe Ser Arg Leu Ser Pro Tyr Gln Ile Cys Val Phe Asp
20 25 30

Ser Lys Ala Leu Val Ser Ser Cys Pro Ser Asn Val Met Lys Arg Arg
35 40 45

His Val Gly Val Ala Ala Gly Val Arg Cys Met Ala Val Gly Thr Thr
50 55 60

Ser Glu Val Ala Thr Lys Lys Arg Ser Ser Tyr Glu Ile Glu Thr Leu
65 70 75 80

Thr Asn Trp Leu Leu Lys Gln Glu Gln Ser Gly Val Ile Asp Ala Glu
85 90 95

Leu Thr Ile Val Leu Ser Ser Ile Ser Thr Ala Cys Lys Gln Ile Ala
100 105 110

Ser Leu Val Gln Arg Ala Gly Ile Ser Asn Leu Thr Gly Ile Gln Gly
115 120 125

Ala Val Asn Val Gln Gly Glu Asp Gln Lys Lys Leu Asp Val Val Ser
130 135 140

Asn Glu Val Phe Ser Asn Cys Leu Arg Ser Ser Gly Arg Thr Gly Ile
145 150 155 160

Ile Ala Ser Glu Glu Glu Asp Val Pro Val Ala Val Glu Glu Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Ser Pro
195 200 205

Asn Asp Glu Cys Leu Ala Asp Ile Gly Asp Asp Ser Thr Leu Gly Asn

210 215 220

Thr Glu Gln Arg Cys Val Val Asn Val Cys Gln Pro Gly Ser Asn Leu
225 230 235 240

Leu Ala Ala Gly Tyr Cys Met Tyr Ser Ser Ser Val Ile Phe Val Ile
245 250 255

Thr Leu Gly Asn Gly Val Phe Ala Phe Thr Leu Asp Pro Met Tyr Gly
260 265 270

Glu Phe Val Leu Thr Gln Glu Asn Ile Gln Ile Pro Lys Thr Gly Lys
275 280 285

Ile Tyr Ala Phe Asn Glu Gly Asn Tyr Gln Leu Trp Asp Asp Lys Leu
290 295 300

Lys Lys Tyr Ile Asp Asp Leu Lys Asp Pro Gly Pro Ser Gly Lys Pro
305 310 315 320

Tyr Ser Ala Arg Tyr Ile Gly Ser Leu Val Gly Asp Phe His Arg Thr
325 330 335

Leu Leu Tyr Gly Gly Ile Tyr Gly Tyr Pro Arg Asp Lys Lys Ser Lys
340 345 350

Asn Gly Lys Leu Arg Leu Leu Tyr Glu Cys Ala Pro Met Ser Phe Ile
355 360 365

Val Glu Gln Ala Gly Gly Lys Gly Ser Asp Gly His Gln Arg Val Leu
370 375 380

Asp Ile Gln Pro Thr Glu Ile His Gln Arg Ile Pro Leu Lys Ile Gly
385 390 395 400

Ser Gln Glu Glu Val Glu Lys Leu Glu Lys Tyr Leu Ala
405 410

<210> 186
<211> 1245
<212> DNA
<213> Populus tremuloides

<400> 186
atggttgcac aagcagcagc aataacaact tcatcatcac atcttctctt ctcaacctca 60

cgctcactgt ctcgcccatc tccttcccag ttatgtgtct ttgactcaaa aacacttgtg 120

tcatacccca acagcactag tacttacaag aaaaaacgtg gtggtgatgg gctcaagtgc 180

atggctgtga gcacagcctc ggatgctaaa acaaagaaga gtacgtttga gattcaaaca 240

ctgactggtt ggctgttgaa gcaagaacaa gctggtgtta ttgatgctga gctcactatt 300

gttatatcaa gcatttcaat ggcatgtaag cagattgctt ctttggtgca aagagctagc 360

atttctaact taactggagt tcaaggttct gttaacgttc aaggagaaga tcagaagaag 420

cttgacgtgg tctctaatga ggtgttctct agctgcttga gatcaagtgg gaggacagga 480

atcatagcat cagaggaaga ggacgtgcca gtggcagtgg aggagagtta ctctggaaac 540

tatatagtgg tttttgaccc actcgatgga tcatccaaca ttgatgctgc agtgtctact 600

ggttccatct ttggaatata cagccccaat gacgaatgcc tggccgatat tggagatgac 660

tccactcttg atcaaacgga acagaggtgt attgtgaatg tgtgccagcc aggaaataac 720

ctccttgttg ctggctactg catgtattca agctcagtga tttttgtgct aactattgga 780

aaaggcgtgt tctctttcag cttggatcca atgtatggag agtttgtttt aactcaagaa 840

aacatccaga taccaaaggc tggaaagatt tattcattta atgaaggaaa ctaccagttg 900

tgggatgaca agctgaagaa gtacattgat gaccttaaag accctggtcc gagtggcaag 960

ccctactccg ctagatacat tggaagcttg gtcggtgact tccaccggac gctgctgtac 1020

ggtggcattt atgggtaccc cagggacaag aagagcaaga atgggaagct gaggcttctg 1080

tatgagtgtg caccgatgag ctttatagtg gaacaagctg gtgggaaagg atcagacggg 1140

catcagagag tactggatat cactcctact gagatacacc agcgtgttcc gctttacata 1200

gggagcgtgg aggaagtgga gaaattggag aagtatttgg cttga 1245

<210> 187
<211> 414
<212> PRT
<213> Populus tremuloides

<400> 187

Met Val Ala Gln Ala Ala Ala Ile Thr Thr Ser Ser Ser His Leu Leu
1 5 10 15

Phe Ser Thr Ser Arg Ser Leu Ser Arg Pro Ser Pro Ser Gln Leu Cys
20 25 30

Val Phe Asp Ser Lys Thr Leu Val Ser Tyr Pro Asn Ser Thr Ser Thr
35 40 45

Tyr Lys Lys Lys Arg Gly Gly Asp Gly Leu Lys Cys Met Ala Val Ser
50 55 60

Thr Ala Ser Asp Ala Lys Thr Lys Lys Ser Thr Phe Glu Ile Gln Thr
65 70 75 80

Leu Thr Gly Trp Leu Leu Lys Gln Glu Gln Ala Gly Val Ile Asp Ala
85 90 95

```
Glu Leu Thr Ile Val Ile Ser Ser Ile Ser Met Ala Cys Lys Gln Ile
            100                 105                 110

Ala Ser Leu Val Gln Arg Ala Ser Ile Ser Asn Leu Thr Gly Val Gln
        115                 120                 125

Gly Ser Val Asn Val Gln Gly Glu Asp Gln Lys Lys Leu Asp Val Val
    130                 135                 140

Ser Asn Glu Val Phe Ser Ser Cys Leu Arg Ser Ser Gly Arg Thr Gly
145                 150                 155                 160

Ile Ile Ala Ser Glu Glu Glu Asp Val Pro Val Ala Val Glu Glu Ser
                165                 170                 175

Tyr Ser Gly Asn Tyr Ile Val Val Phe Asp Pro Leu Asp Gly Ser Ser
            180                 185                 190

Asn Ile Asp Ala Ala Val Ser Thr Gly Ser Ile Phe Gly Ile Tyr Ser
        195                 200                 205

Pro Asn Asp Glu Cys Leu Ala Asp Ile Gly Asp Asp Ser Thr Leu Asp
    210                 215                 220

Gln Thr Glu Gln Arg Cys Ile Val Asn Val Cys Gln Pro Gly Asn Asn
225                 230                 235                 240

Leu Leu Val Ala Gly Tyr Cys Met Tyr Ser Ser Ser Val Ile Phe Val
                245                 250                 255

Leu Thr Ile Gly Lys Gly Val Phe Ser Phe Ser Leu Asp Pro Met Tyr
            260                 265                 270

Gly Glu Phe Val Leu Thr Gln Glu Asn Ile Gln Ile Pro Lys Ala Gly
        275                 280                 285

Lys Ile Tyr Ser Phe Asn Glu Gly Asn Tyr Gln Leu Trp Asp Asp Lys
    290                 295                 300

Leu Lys Lys Tyr Ile Asp Asp Leu Lys Asp Pro Gly Pro Ser Gly Lys
305                 310                 315                 320

Pro Tyr Ser Ala Arg Tyr Ile Gly Ser Leu Val Gly Asp Phe His Arg
                325                 330                 335

Thr Leu Leu Tyr Gly Gly Ile Tyr Gly Tyr Pro Arg Asp Lys Lys Ser
            340                 345                 350
```

Lys Asn Gly Lys Leu Arg Leu Leu Tyr Glu Cys Ala Pro Met Ser Phe
355 360 365

Ile Val Glu Gln Ala Gly Gly Lys Gly Ser Asp Gly His Gln Arg Val
370 375 380

Leu Asp Ile Thr Pro Thr Glu Ile His Gln Arg Val Pro Leu Tyr Ile
385 390 395 400

Gly Ser Val Glu Glu Val Glu Lys Leu Glu Lys Tyr Leu Ala
405 410

<210> 188
<211> 1227
<212> DNA
<213> Solanum tuberosum

<400> 188
atggcagcat cagcagccac agcaacagca acaacttcat atttaagtgc tctagacaaa 60

aagactccat ttttatttgc cttagacaaa aagactccat ttttatgccc aaaaagcagc 120

acgaagagaa ggtcatttaa tggaggagta aagtgcatgg caatagagac aacttcaggt 180

tttacagcaa ccaagaaaag gagtggctat gagctgcaaa ctttaacaag ttggctatta 240

agacaagaac aagctggagt gattgatgct gaacttacca tagttatttc aagtatttca 300

atggcttgta aacagattgc ttccttagtc caaagagctg gaatttctaa ccttactgga 360

gttcaaggtg ctgtcaatat tcaaggagaa gatcagaaga aacttgatgt tgtctctaat 420

gaggttttct caaattgtct aagatcaagt ggaaggactg ggattatagc atcagaagaa 480

gaggatgtac ctgtggcagt ggaagagagt tactcaggca actacattgt ggtgtttgat 540

cctcttgatg gatcatcaaa cattgatgct gctgtgtcta ccggttctat ctttggaata 600

tacaacccga atgatgagtg cctcgctgat catggagatg attccacgct tgacaatata 660

gagcagaagt gcattgtgaa tgtatgtcaa ccagggacaa accttcttgc agcaggatac 720

tgcatgtact caagctctgt gatattcgta ctcaccttgg gaaatggcgt tttttccttt 780

aacttggatc cgatgtacgg agaatttgtt ctgactcaag aaaatgtcca aataccaaag 840

tctggaaaga tctattcatt caatgaagga aactaccagc tctgggatga caagttgaag 900

aaatatatcg atgacttgaa ggaccctggc cctagtggca agccttactc tgcaaggtac 960

attggtagtt tggttggtga cttccataga actcttctat atggtggcat ttatggttat 1020

cctagagacc aaaagagcaa gaatggaaag ttgaggcttt tgtacgagtg tgctcccatg 1080

agcttcattg tggaacaagc tggtggtaaa ggatccgatg gtcaccaaag agttctcgat 1140

atccaaccaa ctgaggtaca tcaacgagtt ccattgtaca ttggaagcac agaagaagtt 1200

gaaaaattgg agaagtactt gtcttaa 1227

<210> 189
<211> 408
<212> PRT
<213> Solanum tuberosum

<400> 189

Met Ala Ala Ser Ala Ala Thr Ala Thr Ala Thr Thr Ser Tyr Leu Ser
1 5 10 15

Ala Leu Asp Lys Lys Thr Pro Phe Leu Phe Ala Leu Asp Lys Lys Thr
20 25 30

Pro Phe Leu Cys Pro Lys Ser Ser Thr Lys Arg Arg Ser Phe Asn Gly
35 40 45

Gly Val Lys Cys Met Ala Ile Glu Thr Thr Ser Gly Phe Thr Ala Thr
50 55 60

Lys Lys Arg Ser Gly Tyr Glu Leu Gln Thr Leu Thr Ser Trp Leu Leu
65 70 75 80

Arg Gln Glu Gln Ala Gly Val Ile Asp Ala Glu Leu Thr Ile Val Ile
85 90 95

Ser Ser Ile Ser Met Ala Cys Lys Gln Ile Ala Ser Leu Val Gln Arg
100 105 110

Ala Gly Ile Ser Asn Leu Thr Gly Val Gln Gly Ala Val Asn Ile Gln
115 120 125

Gly Glu Asp Gln Lys Lys Leu Asp Val Val Ser Asn Glu Val Phe Ser
130 135 140

Asn Cys Leu Arg Ser Ser Gly Arg Thr Gly Ile Ile Ala Ser Glu Glu
145 150 155 160

Glu Asp Val Pro Val Ala Val Glu Glu Ser Tyr Ser Gly Asn Tyr Ile
165 170 175

Val Val Phe Asp Pro Leu Asp Gly Ser Ser Asn Ile Asp Ala Ala Val
180 185 190

Ser Thr Gly Ser Ile Phe Gly Ile Tyr Asn Pro Asn Asp Glu Cys Leu
195 200 205

Ala Asp His Gly Asp Asp Ser Thr Leu Asp Asn Ile Glu Gln Lys Cys
210 215 220

Ile Val Asn Val Cys Gln Pro Gly Thr Asn Leu Leu Ala Ala Gly Tyr
225 230 235 240

Cys Met Tyr Ser Ser Ser Val Ile Phe Val Leu Thr Leu Gly Asn Gly
245 250 255

Val Phe Ser Phe Asn Leu Asp Pro Met Tyr Gly Glu Phe Val Leu Thr
260 265 270

Gln Glu Asn Val Gln Ile Pro Lys Ser Gly Lys Ile Tyr Ser Phe Asn
275 280 285

Glu Gly Asn Tyr Gln Leu Trp Asp Asp Lys Leu Lys Lys Tyr Ile Asp
290 295 300

Asp Leu Lys Asp Pro Gly Pro Ser Gly Lys Pro Tyr Ser Ala Arg Tyr
305 310 315 320

Ile Gly Ser Leu Val Gly Asp Phe His Arg Thr Leu Leu Tyr Gly Gly
325 330 335

Ile Tyr Gly Tyr Pro Arg Asp Gln Lys Ser Lys Asn Gly Lys Leu Arg
340 345 350

Leu Leu Tyr Glu Cys Ala Pro Met Ser Phe Ile Val Glu Gln Ala Gly
355 360 365

Gly Lys Gly Ser Asp Gly His Gln Arg Val Leu Asp Ile Gln Pro Thr
370 375 380

Glu Val His Gln Arg Val Pro Leu Tyr Ile Gly Ser Thr Glu Glu Val
385 390 395 400

Glu Lys Leu Glu Lys Tyr Leu Ser
405

<210> 190
<211> 1248
<212> DNA
<213> Spinacia oleracea

<400> 190
atggcatcaa taggaccagc aacaacaact gcagtaaaac tgcgtagctc aatcttcaat 60

cctcagtcat ctactctttc cccgtctcaa caatgcatta catttacaaa gtcccttcac 120

tcgtttccta ctgccacccg acataatgtg gcttccgggg ttcgttgtat ggcagccgta 180

ggagaggcgg ctacagaaac aaaggcaagg actagaagta agtacgaaat tgaaacacta 240

acaggctggc tgcttaaaca agaaatggca ggtgttattg atgctgaact taccatcgtt 300

ctttctagca tttcattggc ttgtaaacaa attgcttcct tggttcaacg agctggtatt 360

tctaacttga ctggaattca aggtgctgtc aatatccaag gagaggatca gaagaaactt 420

gatgttgtct ccaatgaggt gttttcgagc tgcttgagat cgagtggaag aacaggaata 480

atagcatcag aagaagagga tgtaccagtg gcagtggaag agagttactc tggaaactat 540

attgttgtgt ttgatccact tgatggttca tccaacattg atgcagctgt ctccactggt 600

tccatctttg gcatttatag ccctaacgat gagtgcattg ttgactctga tcacgacgat 660

gagtcacagc taagtgcaga agaacagagg tgtgtagtga atgtatgtca accaggggat 720

aacctattag cagcagggta ttgtatgtac tcaagctctg ttatcttcgt acttacaatt 780

ggtaaaggtg tgtatgcatt cacattagat ccaatgtatg gtgaattcgt actcacttca 840

gagaaaatcc aaatcccaaa agctgggaag atctattcat tcaatgaagg taactacaaa 900

atgtgggatg ataaattgaa gaagtacatg gatgatctta aagagccagg agagtcacag 960

aaaccgtact cgtctcgtta catagggagt ttagttgggg actttcatag aacactttta 1020

tatggtggga tttatggtta cccaagagat gcaaagagta agaatgggaa attgaggctt 1080

ttgtatgaat gtgcacctat gagttttatt gttgaacaag ctggtggtaa aggttctgat 1140

ggtcatcaaa gaattcttga cattcaaccc accgagatac atcaacgtgt gccactgtac 1200

atcgggagtg tggaggaagt agagaaatta gagaagtact tagcataa 1248

<210> 191
<211> 415
<212> PRT
<213> Spinacia oleracea

<400> 191

Met Ala Ser Ile Gly Pro Ala Thr Thr Thr Ala Val Lys Leu Arg Ser
1 5 10 15

Ser Ile Phe Asn Pro Gln Ser Ser Thr Leu Ser Pro Ser Gln Gln Cys
20 25 30

Ile Thr Phe Thr Lys Ser Leu His Ser Phe Pro Thr Ala Thr Arg His
35 40 45

Asn Val Ala Ser Gly Val Arg Cys Met Ala Ala Val Gly Glu Ala Ala
50 55 60

Thr Glu Thr Lys Ala Arg Thr Arg Ser Lys Tyr Glu Ile Glu Thr Leu
65 70 75 80

Thr Gly Trp Leu Leu Lys Gln Glu Met Ala Gly Val Ile Asp Ala Glu
85 90 95

Leu Thr Ile Val Leu Ser Ser Ile Ser Leu Ala Cys Lys Gln Ile Ala
100 105 110

Ser Leu Val Gln Arg Ala Gly Ile Ser Asn Leu Thr Gly Ile Gln Gly
115 120 125

Ala Val Asn Ile Gln Gly Glu Asp Gln Lys Lys Leu Asp Val Val Ser
130 135 140

Asn Glu Val Phe Ser Ser Cys Leu Arg Ser Ser Gly Arg Thr Gly Ile
145 150 155 160

Ile Ala Ser Glu Glu Glu Asp Val Pro Val Ala Val Glu Glu Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Ser Pro
195 200 205

Asn Asp Glu Cys Ile Val Asp Ser Asp His Asp Asp Glu Ser Gln Leu
210 215 220

Ser Ala Glu Glu Gln Arg Cys Val Val Asn Val Cys Gln Pro Gly Asp
225 230 235 240

Asn Leu Leu Ala Ala Gly Tyr Cys Met Tyr Ser Ser Ser Val Ile Phe
245 250 255

Val Leu Thr Ile Gly Lys Gly Val Tyr Ala Phe Thr Leu Asp Pro Met
260 265 270

Tyr Gly Glu Phe Val Leu Thr Ser Glu Lys Ile Gln Ile Pro Lys Ala
275 280 285

Gly Lys Ile Tyr Ser Phe Asn Glu Gly Asn Tyr Lys Met Trp Asp Asp
290 295 300

Lys Leu Lys Lys Tyr Met Asp Asp Leu Lys Glu Pro Gly Glu Ser Gln
305 310 315 320

Lys Pro Tyr Ser Ser Arg Tyr Ile Gly Ser Leu Val Gly Asp Phe His
325 330 335

Arg Thr Leu Leu Tyr Gly Gly Ile Tyr Gly Tyr Pro Arg Asp Ala Lys
340 345 350

Ser Lys Asn Gly Lys Leu Arg Leu Leu Tyr Glu Cys Ala Pro Met Ser
355 360 365

Phe Ile Val Glu Gln Ala Gly Gly Lys Gly Ser Asp Gly His Gln Arg

370 375 380

Ile Leu Asp Ile Gln Pro Thr Glu Ile His Gln Arg Val Pro Leu Tyr
385 390 395 400

Ile Gly Ser Val Glu Glu Val Glu Lys Leu Glu Lys Tyr Leu Ala
405 410 415

<210> 192
<211> 1230
<212> DNA
<213> Triticum aestivum

<400> 192
atggccgccg cgaccaccac cacctcccgc ccgcttctgc tgtcccgcca gcaggcggcg 60

gctagctccc tccaatgccg cctccccagg aggcccggaa gcagcctctt tgccggccag 120

ggccaggcgt cgactccgaa tgtgcggtgc atggcagtcg tggacacggc ctcggcgccg 180

gcgccggcgg cggctaggaa gaggagcagc tacgacatga tcacgctgac gacgtggctg 240

ctgaagcagg agcaggaggg ggtcatcgac aacgagatga ccatcgtgct gtccagcata 300

tccacggcgt gcaagcagat cgcctcgttg gtgcagcgcg cgcccatctc caacctcacc 360

ggcgtccagg gcgccaccaa cgtgcagggc gaggaccaga agaagctcga cgtcatctcc 420

aacgaggtgt tctcgaactg cctgaggtgg agtggccgca ccggcgtgat cgcatcggag 480

gaggaggacg tgccggtggc ggtggaggag agctactcgg gcaactacat cgtggtgttc 540

gacccgctcg acggctcctc caacatcgac gccgccgtct ccaccggctc catcttcggc 600

atctacagcc catccgacga gtgccacatt ggcgacgacg caacccttga cgaagtgacg 660

cagatgtgca tagtgaacgt gtgccagcca gggagcaacc tgctcgccgc cggctactgc 720

atgtactcga gctcggtcat cttcgtgctc accatcggca ccggggtgta cgtgttcacg 780

ctggacccga tgtacggcga gttcgtgctg acgcaggaga aggtgcagat cccaaagtcg 840

ggcaagatct actccttcaa cgagggcaac tacgcgctct gggacgacaa gctcaagaag 900

tacatggaca gcctcaagga gcccggcacc tccggcaagc cctactccgc gcgctacatc 960

ggcagcctcg tcggcgactt ccaccgcacc atgctctacg gcggcatcta cgggtacccc 1020

agcgaccaga agagcaagaa cggcaagctg cggctgctct acgagtgcgc gcccatgagc 1080

ttcatcgccg agcaggccgg cggcaaaggc tccgacggcc accagagggt actcgacatc 1140

atgcccacag cggtccatca gagagtgcct ctgtacgtcg ggagcgtgga ggaagtggag 1200

aaggtggaga aattcttgtc ttcagagtag 1230

<210> 193
<211> 409
<212> PRT
<213> Triticum aestivum

<400> 193

Met Ala Ala Ala Thr Thr Thr Thr Ser Arg Pro Leu Leu Leu Ser Arg
1               5                   10                  15

Gln Gln Ala Ala Ala Ser Ser Leu Gln Cys Arg Leu Pro Arg Arg Pro
            20                  25                  30

Gly Ser Ser Leu Phe Ala Gly Gln Gly Gln Ala Ser Thr Pro Asn Val
        35                  40                  45

Arg Cys Met Ala Val Val Asp Thr Ala Ser Ala Pro Ala Pro Ala Ala
    50                  55                  60

Ala Arg Lys Arg Ser Ser Tyr Asp Met Ile Thr Leu Thr Thr Trp Leu
65                  70                  75                  80

Leu Lys Gln Glu Gln Glu Gly Val Ile Asp Asn Glu Met Thr Ile Val
                85                  90                  95

Leu Ser Ser Ile Ser Thr Ala Cys Lys Gln Ile Ala Ser Leu Val Gln
            100                 105                 110

Arg Ala Pro Ile Ser Asn Leu Thr Gly Val Gln Gly Ala Thr Asn Val
        115                 120                 125

Gln Gly Glu Asp Gln Lys Lys Leu Asp Val Ile Ser Asn Glu Val Phe
    130                 135                 140

Ser Asn Cys Leu Arg Trp Ser Gly Arg Thr Gly Val Ile Ala Ser Glu
145                 150                 155                 160

Glu Glu Asp Val Pro Val Ala Val Glu Glu Ser Tyr Ser Gly Asn Tyr
                165                 170                 175

Ile Val Val Phe Asp Pro Leu Asp Gly Ser Ser Asn Ile Asp Ala Ala
            180                 185                 190

Val Ser Thr Gly Ser Ile Phe Gly Ile Tyr Ser Pro Ser Asp Glu Cys
        195                 200                 205

His Ile Gly Asp Asp Ala Thr Leu Asp Glu Val Thr Gln Met Cys Ile
    210                 215                 220

Val Asn Val Cys Gln Pro Gly Ser Asn Leu Leu Ala Ala Gly Tyr Cys
225                 230                 235                 240

Met Tyr Ser Ser Ser Val Ile Phe Val Leu Thr Ile Gly Thr Gly Val
                245                 250                 255

Tyr Val Phe Thr Leu Asp Pro Met Tyr Gly Glu Phe Val Leu Thr Gln
260 265 270

Glu Lys Val Gln Ile Pro Lys Ser Gly Lys Ile Tyr Ser Phe Asn Glu
275 280 285

Gly Asn Tyr Ala Leu Trp Asp Asp Lys Leu Lys Lys Tyr Met Asp Ser
290 295 300

Leu Lys Glu Pro Gly Thr Ser Gly Lys Pro Tyr Ser Ala Arg Tyr Ile
305 310 315 320

Gly Ser Leu Val Gly Asp Phe His Arg Thr Met Leu Tyr Gly Gly Ile
325 330 335

Tyr Gly Tyr Pro Ser Asp Gln Lys Ser Lys Asn Gly Lys Leu Arg Leu
340 345 350

Leu Tyr Glu Cys Ala Pro Met Ser Phe Ile Ala Glu Gln Ala Gly Gly
355 360 365

Lys Gly Ser Asp Gly His Gln Arg Val Leu Asp Ile Met Pro Thr Ala
370 375 380

Val His Gln Arg Val Pro Leu Tyr Val Gly Ser Val Glu Glu Val Glu
385 390 395 400

Lys Val Glu Lys Phe Leu Ser Ser Glu
405

<210> 194
<211> 1242
<212> DNA
<213> Zea mays

<400> 194
atggccgccg ccgccaccac ctcctcatcc tcccacttgc tcctcctctc ccgccagcag 60

gcggcctccc tacgatgccg cctctccttc ctcggccagc ccgccagaag gtccggcagg 120

gtcacggccc aggcgccggc cgctaaggac gtgcggtgca tggcggccgt ggacactgcg 180

gcgtccgcgg cggcggcgga gacgagcccc aagtcgagca gctacgagat cgtgacgctc 240

acgacgtggc tgctgcagca ggagcggacc ggcgcgatcg acaacgagat gaccatcgtg 300

ctggccagca tatccacggc gtgcaagcag atcgccgcgc tggtgcagcg cgcgcccatc 360

tccaacctca cgggcgttca gggcgccgtc aacgtgcagg gcgaggacca gaagaagctc 420

gatgtcgtct ccaacgaggt gttctccaac tgcctcaagt cgagcgggcg caccggcgtg 480

atcgcctcgg aggaggagga cgtgcccgta gcggtggagc agagctactc cggcaactac 540

atcgtcgtgt tcgaccctct cgacggctcc tccaacatcg acgccgccgt ctccactggc 600

tccatcttcg gcatctacaa ccccaacgac gagtgcctcg ccgacgtcga cgacaacgac 660

acccttgatt cggtggagca gaggtgcatc gtgaacgtgt gccagccggg gagcaacctg 720

ctggccgccg gctactgcat gtactcgagc tcggtgatct tcgtgctcac cgtcggcacc 780

ggggtgtacg tgttcacgct ggaccccatg tacggcgagt tcgtgctgac gcaggagaag 840

gtgcagatcc ccaaggcggg caagatctac gccttcaacg agggcaacta cgcgctctgg 900

gacgacaagc tgaagctgta catggacagc ctcaaggagc ccggcgactc ggggaagccc 960

tactccgcgc ggtacatcgg cagcctcgtc ggcgacttcc accgcactct gctctacgga 1020

gggatctacg ggtaccccag ggacaagaag agcaagaacg gcaagctgcg gcttctctac 1080

gagtgcgccc ccatgagctt catcgtcgag caggccggtg gcaagggctc tgacggccac 1140

cagagaattc ttgacatcac acctacagag atccaccaaa gagtgcctct gtacattggg 1200

agcgtggagg aagtggacaa ggtggagaaa ttcctggctt ga 1242

<210> 195
<211> 413
<212> PRT
<213> Zea mays

<400> 195

Met Ala Ala Ala Ala Thr Thr Ser Ser Ser Ser His Leu Leu Leu Leu
1 5 10 15

Ser Arg Gln Gln Ala Ala Ser Leu Arg Cys Arg Leu Ser Phe Leu Gly
20 25 30

Gln Pro Ala Arg Arg Ser Gly Arg Val Thr Ala Gln Ala Pro Ala Ala
35 40 45

Lys Asp Val Arg Cys Met Ala Ala Val Asp Thr Ala Ala Ser Ala Ala
50 55 60

Ala Ala Glu Thr Ser Pro Lys Ser Ser Ser Tyr Glu Ile Val Thr Leu
65 70 75 80

Thr Thr Trp Leu Leu Gln Gln Glu Arg Thr Gly Ala Ile Asp Asn Glu
85 90 95

Met Thr Ile Val Leu Ala Ser Ile Ser Thr Ala Cys Lys Gln Ile Ala
100 105 110

Ala Leu Val Gln Arg Ala Pro Ile Ser Asn Leu Thr Gly Val Gln Gly
115 120 125

Ala Val Asn Val Gln Gly Glu Asp Gln Lys Lys Leu Asp Val Val Ser

130 135 140

Asn Glu Val Phe Ser Asn Cys Leu Lys Ser Ser Gly Arg Thr Gly Val
145 150 155 160

Ile Ala Ser Glu Glu Glu Asp Val Pro Val Ala Val Glu Gln Ser Tyr
165 170 175

Ser Gly Asn Tyr Ile Val Val Phe Asp Pro Leu Asp Gly Ser Ser Asn
180 185 190

Ile Asp Ala Ala Val Ser Thr Gly Ser Ile Phe Gly Ile Tyr Asn Pro
195 200 205

Asn Asp Glu Cys Leu Ala Asp Val Asp Asp Asn Asp Thr Leu Asp Ser
210 215 220

Val Glu Gln Arg Cys Ile Val Asn Val Cys Gln Pro Gly Ser Asn Leu
225 230 235 240

Leu Ala Ala Gly Tyr Cys Met Tyr Ser Ser Ser Val Ile Phe Val Leu
245 250 255

Thr Val Gly Thr Gly Val Tyr Val Phe Thr Leu Asp Pro Met Tyr Gly
260 265 270

Glu Phe Val Leu Thr Gln Glu Lys Val Gln Ile Pro Lys Ala Gly Lys
275 280 285

Ile Tyr Ala Phe Asn Glu Gly Asn Tyr Ala Leu Trp Asp Asp Lys Leu
290 295 300

Lys Leu Tyr Met Asp Ser Leu Lys Glu Pro Gly Asp Ser Gly Lys Pro
305 310 315 320

Tyr Ser Ala Arg Tyr Ile Gly Ser Leu Val Gly Asp Phe His Arg Thr
325 330 335

Leu Leu Tyr Gly Gly Ile Tyr Gly Tyr Pro Arg Asp Lys Lys Ser Lys
340 345 350

Asn Gly Lys Leu Arg Leu Leu Tyr Glu Cys Ala Pro Met Ser Phe Ile
355 360 365

Val Glu Gln Ala Gly Gly Lys Gly Ser Asp Gly His Gln Arg Ile Leu
370 375 380

Asp Ile Thr Pro Thr Glu Ile His Gln Arg Val Pro Leu Tyr Ile Gly
385 390 395 400

Ser Val Glu Glu Val Asp Lys Val Glu Lys Phe Leu Ala
405 410

<210> 196
<211> 1266
<212> DNA
<213> Physicomitrella patens

<400> 196
atggcgacca cacaagcgat tctctctgcc acccttgcca tagccccggc ttccagctgc 60

gagacttcgt cacggagccc ggcgtccacc aaaacttgtc tctcagtggc aggatcgtcg 120

ctgcatggct cagtggccgg actcggagct gggaaacaga ttgtgagcgt gcagaggaag 180

agcgttgccg tgagggccgc cgttgcagct gagactgccg ctcccaagca gcaggcgaag 240

agccagtatg acatcactac cctgacgacg tggttgctga agaaagagca ggcgggcgtc 300

atcgatggcg agctcaccat tgtgctctcc agcatcgccc tggcttgcaa gcaaattgcg 360

tctctggtgc agagggctgg catctccaac atgactgggt tgcaaggagc tgctaacatt 420

caaggggagg accagaagaa gctagacgtt atttcgaacg aggtgttctc aagctgtctg 480

cgctcaagcg gacggacagg catcatcgct tctgaggaag aagacacccc ggttgcagtg 540

gaggagagct actctggcaa ctacattgtg gtgttcgacc ctcttgacgg ctcttccaac 600

atcgatgctg ctgtttccac ggggtcaatc tggggaatct acaagcccaa cgaggaatgc 660

ctgaccaatc tcggagagga gccaactatt gatgagatcg ccgaaaactg cgtcgtcaat 720

gtctgtcaac cagggagcaa tttgttgtcc gccgggtact gcatgtactc gagctccgtc 780

atcctcgttc tctcagtcgg tgatggagtc tacggcttca ctctggaccc tctctacgga 840

gaattcgtcc tctcgcacga caacatccaa attccaaaat ctggtaagat ctattccatg 900

aatgaaggca actacgccct ctgggatgac aacctcaaga agtacgtcga cagcttgaag 960

gaccccggtc ccagcggcaa gccctactcc gctcgttaca tcggcagtct ggtgggcgac 1020

ttccacagga caatgctgta tggtggcatc tatggctacc ccagggattc caagagcaag 1080

aacgggaagt tgaggttgct ttacgagtgt gctcccatga gctacctcgc tgaacaagca 1140

ggtgggaagg gctccgacgg tcaccagagg attctggaca tccaacctga gcaggttcac 1200

caacgtgtgc cattgtacgt tggaagcacg gaggaggtgg agaagttgga gaagttctta 1260

gcttaa 1266

<210> 197
<211> 421
<212> PRT
<213> Physicomitrella patens

<400> 197

Met Ala Thr Thr Gln Ala Ile Leu Ser Ala Thr Leu Ala Ile Ala Pro

```
1               5                   10                  15

Ala Ser Ser Cys Glu Thr Ser Ser Arg Ser Pro Ala Ser Thr Lys Thr
            20                  25                  30

Cys Leu Ser Val Ala Gly Ser Ser Leu His Gly Ser Val Ala Gly Leu
        35                  40                  45

Gly Ala Gly Lys Gln Ile Val Ser Val Gln Arg Lys Ser Val Ala Val
    50                  55                  60

Arg Ala Ala Val Ala Ala Glu Thr Ala Ala Pro Lys Gln Gln Ala Lys
65                  70                  75                  80

Ser Gln Tyr Asp Ile Thr Thr Leu Thr Thr Trp Leu Leu Lys Lys Glu
                85                  90                  95

Gln Ala Gly Val Ile Asp Gly Glu Leu Thr Ile Val Leu Ser Ser Ile
            100                 105                 110

Ala Leu Ala Cys Lys Gln Ile Ala Ser Leu Val Gln Arg Ala Gly Ile
        115                 120                 125

Ser Asn Met Thr Gly Leu Gln Gly Ala Ala Asn Ile Gln Gly Glu Asp
    130                 135                 140

Gln Lys Lys Leu Asp Val Ile Ser Asn Glu Val Phe Ser Ser Cys Leu
145                 150                 155                 160

Arg Ser Ser Gly Arg Thr Gly Ile Ile Ala Ser Glu Glu Glu Asp Thr
                165                 170                 175

Pro Val Ala Val Glu Glu Ser Tyr Ser Gly Asn Tyr Ile Val Val Phe
            180                 185                 190

Asp Pro Leu Asp Gly Ser Ser Asn Ile Asp Ala Ala Val Ser Thr Gly
        195                 200                 205

Ser Ile Trp Gly Ile Tyr Lys Pro Asn Glu Glu Cys Leu Thr Asn Leu
    210                 215                 220

Gly Glu Glu Pro Thr Ile Asp Glu Ile Ala Glu Asn Cys Val Val Asn
225                 230                 235                 240

Val Cys Gln Pro Gly Ser Asn Leu Leu Ser Ala Gly Tyr Cys Met Tyr
                245                 250                 255

Ser Ser Ser Val Ile Leu Val Leu Ser Val Gly Asp Gly Val Tyr Gly
            260                 265                 270
```

Phe Thr Leu Asp Pro Leu Tyr Gly Glu Phe Val Leu Ser His Asp Asn
275 280 285

Ile Gln Ile Pro Lys Ser Gly Lys Ile Tyr Ser Met Asn Glu Gly Asn
290 295 300

Tyr Ala Leu Trp Asp Asp Asn Leu Lys Lys Tyr Val Asp Ser Leu Lys
305 310 315 320

Asp Pro Gly Pro Ser Gly Lys Pro Tyr Ser Ala Arg Tyr Ile Gly Ser
325 330 335

Leu Val Gly Asp Phe His Arg Thr Met Leu Tyr Gly Gly Ile Tyr Gly
340 345 350

Tyr Pro Arg Asp Ser Lys Ser Lys Asn Gly Lys Leu Arg Leu Leu Tyr
355 360 365

Glu Cys Ala Pro Met Ser Tyr Leu Ala Glu Gln Ala Gly Gly Lys Gly
370 375 380

Ser Asp Gly His Gln Arg Ile Leu Asp Ile Gln Pro Glu Gln Val His
385 390 395 400

Gln Arg Val Pro Leu Tyr Val Gly Ser Thr Glu Glu Val Glu Lys Leu
405 410 415

Glu Lys Phe Leu Ala
420

<210> 198
<211> 1263
<212> DNA
<213> Galderia sulphuraria

<400> 198
ggcacgagga aagatgtgca agtatacttt attcaagtcc tactatatct ttacaaagaa 60

gagtgtcaag aaataaccac tgcaaaagat atcttcctta taagatggat atccatcaaa 120

atgatagctg ttcaacccca acccaaaaag actcccgtag cagaatattt acaaacctca 180

caagacacac caacttcact cactcgttac ttgttggaag tagccaaaca aaataaggat 240

atgggagata tggtggcatt gataaacggt attcaatttg cttgcaaaaa gatagcttca 300

ttggttggta aagcaggggt cactgacttg atgggaatct atcaacaagg catagtcaac 360

gttcacggag aagaacagaa aaagttggat gttctttcta atgaagtatt gaagaacgct 420

ctcaaatatt cgggaaaaat ggctgtcata gcttcggaag aagaagacgt tcctatcatg 480

gtagaagaaa gttattccgg taactatgta gtcgtgtttg atccattaga tggttcttcc 540

aatttggatg ctggattgcc tactggaact atatttggag tgtttcaaca acaattctcg 600

tgtctcattc atgactatga ggagtccatc gataacatgg aattggcttg tttacaaaac 660

actttacaac caggacgtag attgattgcc gctggatatt gtatctattc ttcttctacc 720

atgttggtac tctcattggg taatggtctt cattgtttta ctttggatac ggaagttggt 780

gaatttgtat tgactcgtgc taacatccaa attccacaaa gaggtaatat ttattctttc 840

aacgagtcca acttttatca atgggataaa ggagttcaag attatataga gaggttaaaa 900

aaaggaaaca atcaaacaaa ttgtcgttat tccgcaagat atgttggctc catggttgct 960

gatgtacatc gtacaatatt gtatggcgga atatttggtt atccagcaga taaaaagaat 1020

gtctctggta aattgagatt ggtatatgaa tgtgcaccga tggcatattt ggttgaacaa 1080

gcaggaggta aagcaaccac gggaatagaa aatattttag atttgacacc aaaagatatt 1140

cacgaaagga agcctcttat attaggttct ccagcagata tcgaagaatt tctgcaagta 1200

tatggaatgt cacgagttga tagagaagat atgcatatgt gggataactt gagtagttta 1260

taa 1263

<210> 199
<211> 420
<212> PRT
<213> Galderia sulphuraria

<400> 199

Gly Thr Arg Lys Asp Val Gln Val Tyr Phe Ile Gln Val Leu Leu Tyr
1 5 10 15

Leu Tyr Lys Glu Glu Cys Gln Glu Ile Thr Thr Ala Lys Asp Ile Phe
20 25 30

Leu Ile Arg Trp Ile Ser Ile Lys Met Ile Ala Val Gln Pro Gln Pro
35 40 45

Lys Lys Thr Pro Val Ala Glu Tyr Leu Gln Thr Ser Gln Asp Thr Pro
50 55 60

Thr Ser Leu Thr Arg Tyr Leu Leu Glu Val Ala Lys Gln Asn Lys Asp
65 70 75 80

Met Gly Asp Met Val Ala Leu Ile Asn Gly Ile Gln Phe Ala Cys Lys
85 90 95

Lys Ile Ala Ser Leu Val Gly Lys Ala Gly Val Thr Asp Leu Met Gly
100 105 110

Ile Tyr Gln Gln Gly Ile Val Asn Val His Gly Glu Glu Gln Lys Lys
115 120 125

```
Leu Asp Val Leu Ser Asn Glu Val Leu Lys Asn Ala Leu Lys Tyr Ser
    130                 135                 140

Gly Lys Met Ala Val Ile Ala Ser Glu Glu Glu Asp Val Pro Ile Met
145                 150                 155                 160

Val Glu Glu Ser Tyr Ser Gly Asn Tyr Val Val Val Phe Asp Pro Leu
                165                 170                 175

Asp Gly Ser Ser Asn Leu Asp Ala Gly Leu Pro Thr Gly Thr Ile Phe
            180                 185                 190

Gly Val Phe Gln Gln Gln Phe Ser Cys Leu Ile His Asp Tyr Glu Glu
        195                 200                 205

Ser Ile Asp Asn Met Glu Leu Ala Cys Leu Gln Asn Thr Leu Gln Pro
    210                 215                 220

Gly Arg Arg Leu Ile Ala Ala Gly Tyr Cys Ile Tyr Ser Ser Ser Thr
225                 230                 235                 240

Met Leu Val Leu Ser Leu Gly Asn Gly Leu His Cys Phe Thr Leu Asp
                245                 250                 255

Thr Glu Val Gly Glu Phe Val Leu Thr Arg Ala Asn Ile Gln Ile Pro
            260                 265                 270

Gln Arg Gly Asn Ile Tyr Ser Phe Asn Glu Ser Asn Phe Tyr Gln Trp
        275                 280                 285

Asp Lys Gly Val Gln Asp Tyr Ile Glu Arg Leu Lys Lys Gly Asn Asn
    290                 295                 300

Gln Thr Asn Cys Arg Tyr Ser Ala Arg Tyr Val Gly Ser Met Val Ala
305                 310                 315                 320

Asp Val His Arg Thr Ile Leu Tyr Gly Gly Ile Phe Gly Tyr Pro Ala
                325                 330                 335

Asp Lys Lys Asn Val Ser Gly Lys Leu Arg Leu Val Tyr Glu Cys Ala
            340                 345                 350

Pro Met Ala Tyr Leu Val Glu Gln Ala Gly Gly Lys Ala Thr Thr Gly
        355                 360                 365

Ile Glu Asn Ile Leu Asp Leu Thr Pro Lys Asp Ile His Glu Arg Lys
    370                 375                 380
```

Pro Leu Ile Leu Gly Ser Pro Ala Asp Ile Glu Glu Phe Leu Gln Val
385 390 395 400

Tyr Gly Met Ser Arg Val Asp Arg Glu Asp Met His Met Trp Asp Asn
405 410 415

Leu Ser Ser Leu
420

<210> 200
<211> 710
<212> DNA
<213> Marchantia polymorpha

<400> 200
gaggaggata ccccagtcgc cgtcgaagag agctactcag gaaactacgt cgttgtcttc 60

gatcctctcg atggatcctc caacatcgac gctgcggtat ctaccggatc catctttgga 120

atctacaggc ccactgagga gtgtcttgca gacatggacg acgactcaca gctcggtatg 180

gtggaacaaa actgcatcgt gaacgtatgc cagcccggta gcaacctcct atccgctggg 240

tactgcatgt actccagctc cgtcattttg gtgttgtcag tcggagacgg tgtctatgga 300

ttcacactgg atcccctgta cggtgaattc gtcatgaccc acgacaacat caagatccca 360

aagaagggat cgatctactc gttcaatgaa ggtaactacg cactctggga tgacaagctc 420

aagaagtaca tcgactcact gaaggacccc gaacccaccg gcaagcctta ctctgctcgt 480

tacatcggta gtctggtcgg tgacttccac agaaccatgc tctatggtgg catctacgga 540

taccctgccg acaagaaaag caagaacgga aagttgagac ttctgtacga gtgtgctcct 600

atgagctact tggcagagca ggccggagga aagggttctg atggttaccg cagagttctg 660

gaaatcgagc ctgagcaggt acatcagcga gtgccacttt tcgtcggaag 710

<210> 201
<211> 236
<212> PRT
<213> Marchantia polymorpha

<400> 201

Glu Glu Asp Thr Pro Val Ala Val Glu Glu Ser Tyr Ser Gly Asn Tyr
1 5 10 15

Val Val Val Phe Asp Pro Leu Asp Gly Ser Ser Asn Ile Asp Ala Ala
20 25 30

Val Ser Thr Gly Ser Ile Phe Gly Ile Tyr Arg Pro Thr Glu Glu Cys
35 40 45

Leu Ala Asp Met Asp Asp Asp Ser Gln Leu Gly Met Val Glu Gln Asn
50 55 60

```
Cys Ile Val Asn Val Cys Gln Pro Gly Ser Asn Leu Leu Ser Ala Gly
65                  70                  75                  80

Tyr Cys Met Tyr Ser Ser Ser Val Ile Leu Val Leu Ser Val Gly Asp
                85                  90                  95

Gly Val Tyr Gly Phe Thr Leu Asp Pro Leu Tyr Gly Glu Phe Val Met
            100                 105                 110

Thr His Asp Asn Ile Lys Ile Pro Lys Lys Gly Ser Ile Tyr Ser Phe
        115                 120                 125

Asn Glu Gly Asn Tyr Ala Leu Trp Asp Asp Lys Leu Lys Lys Tyr Ile
    130                 135                 140

Asp Ser Leu Lys Asp Pro Glu Pro Thr Gly Lys Pro Tyr Ser Ala Arg
145                 150                 155                 160

Tyr Ile Gly Ser Leu Val Gly Asp Phe His Arg Thr Met Leu Tyr Gly
                165                 170                 175

Gly Ile Tyr Gly Tyr Pro Ala Asp Lys Lys Ser Lys Asn Gly Lys Leu
            180                 185                 190

Arg Leu Leu Tyr Glu Cys Ala Pro Met Ser Tyr Leu Ala Glu Gln Ala
        195                 200                 205

Gly Gly Lys Gly Ser Asp Gly Tyr Arg Arg Val Leu Glu Ile Glu Pro
    210                 215                 220

Glu Gln Val His Gln Arg Val Pro Leu Phe Val Gly
225                 230                 235


<210>  202
<211>  765
<212>  DNA
<213>  Tagetes patula

<400>  202
atcattgcat cggaggaaga agacgtgccg gtggctgtgg aagagagtta ctccggaaac      60

tacattgtcg tgtttgatcc ccttgatggg tcatctaata ttgatgctgc tgtttcaacc     120

ggttctattt tcggaatcta tagccccaat gatgagtgtc tcgctgatat tagtgacgac     180

tccacgctag acagtgtgga acaaaaatgt attgtcaacg tatgccagcc cggaagcaat     240

ctactagcag ccggttactg tatgtactca agctccgtaa tcttcgtgct ctcgatcggt     300

actggtgtct acgcgttcac attagaccca atgtacggtg agtttgtact cactcaagaa     360

aagattcaaa tcccgaaatc ggggaagatt tactcgttta acgaaggaaa ctatcaacta     420
```

tgggacgata aattgaagaa gtacatggat gatctaaagg acccgggccc cacgggcaag 480

ccgtattcgg ctcgctacat tggtagcttg gttggtgatt ttcataggac attattgtac 540

ggagggattt acgggtaccc acgtgacaaa aagagcaaga acgggaagct tcggttgttg 600

tatgagtgtg caccgatgag ttacttggtt gaacaagcgg gtggaaaggg gtcggatgga 660

catcaacgag tgctcgacat tcaaccaacc gagattcatc agcgcgttcc gctatacatt 720

gggagcgtag aggaagtgga aaaattggag aagtatttgg cttga 765

<210> 203
<211> 254
<212> PRT
<213> Tagetes patula

<400> 203

Ile Ile Ala Ser Glu Glu Glu Asp Val Pro Val Ala Val Glu Glu Ser
1 5 10 15

Tyr Ser Gly Asn Tyr Ile Val Val Phe Asp Pro Leu Asp Gly Ser Ser
20 25 30

Asn Ile Asp Ala Ala Val Ser Thr Gly Ser Ile Phe Gly Ile Tyr Ser
35 40 45

Pro Asn Asp Glu Cys Leu Ala Asp Ile Ser Asp Asp Ser Thr Leu Asp
50 55 60

Ser Val Glu Gln Lys Cys Ile Val Asn Val Cys Gln Pro Gly Ser Asn
65 70 75 80

Leu Leu Ala Ala Gly Tyr Cys Met Tyr Ser Ser Ser Val Ile Phe Val
85 90 95

Leu Ser Ile Gly Thr Gly Val Tyr Ala Phe Thr Leu Asp Pro Met Tyr
100 105 110

Gly Glu Phe Val Leu Thr Gln Glu Lys Ile Gln Ile Pro Lys Ser Gly
115 120 125

Lys Ile Tyr Ser Phe Asn Glu Gly Asn Tyr Gln Leu Trp Asp Asp Lys
130 135 140

Leu Lys Lys Tyr Met Asp Asp Leu Lys Asp Pro Gly Pro Thr Gly Lys
145 150 155 160

Pro Tyr Ser Ala Arg Tyr Ile Gly Ser Leu Val Gly Asp Phe His Arg
165 170 175

```
Thr Leu Leu Tyr Gly Gly Ile Tyr Gly Tyr Pro Arg Asp Lys Lys Ser
            180                 185                 190

Lys Asn Gly Lys Leu Arg Leu Leu Tyr Glu Cys Ala Pro Met Ser Tyr
        195                 200                 205

Leu Val Glu Gln Ala Gly Gly Lys Gly Ser Asp Gly His Gln Arg Val
    210                 215                 220

Leu Asp Ile Gln Pro Thr Glu Ile His Gln Arg Val Pro Leu Tyr Ile
225                 230                 235                 240

Gly Ser Val Glu Glu Val Glu Lys Leu Glu Lys Tyr Leu Ala
                245                 250


<210>  204
<211>  795
<212>  DNA
<213>  Linum usitatissimum


<220>
<221>  misc_feature
<222>  (738)..(738)
<223>  n is a, c, g, or t

<400>  204
gaggaggaac aacgtcgccg ggggttctgg tttcaaatgc tctgccgtcg ggacgcgccg      60

tcggaggcgg caacgacgac gaagaagagg agtagctacg agattctgac gctgacgacc     120

tggcttctgc agcaggaaca ggccggagtg atcgacgccg agctcacgat tgtgctctcc     180

agcatctcca cggcgtgtaa gcagatcgct tctctagttc agcgatccgg gatttctaac     240

ctcaccggcg tccagggcgc cgtcaatgtc cagggtgagg atcagaagaa gctcgacgtc     300

gtttcgaacg aggtgttttc aaattgcttg aggtcgagcg gcaggacggg gatcatagcg     360

tcggaggaag aagacgtgcc ggtcgccgtg gaggaaagct actccggtaa ctatatcgta     420

gtgttcgatc cacttgatgg atcgtcaaac atcgatgccg cagtctccac cggctcaatc     480

ttcggaatct acagtcccaa cgatgagtgc ctggccgaca tcggagacgg agacgagtca     540

aatctagata ctcaggagca gaagtgtgtg gtgagcgtgt gccagccggg gagcaaccta     600

ctcgccgccg gctactgcat gtactcaagc tcggtgatct tcgtcctcac gatcggaaac     660

ggcgttttcg ccttcaatct ggacccaatg tacggcgagt tggtgttgac tcaagagaac     720

attcagatcc cgaaagcngg aaagatctac tcattcaacg aaggggacta ccaaatgtgg     780

gatgagaaat tgaag                                                      795


<210>  205
<211>  265
<212>  PRT
<213>  Linum usitatissimum
```

<400> 205

```
Glu Glu Glu Gln Arg Arg Arg Gly Phe Trp Phe Gln Met Leu Cys Arg
1               5                   10                  15

Arg Asp Ala Pro Ser Glu Ala Ala Thr Thr Thr Lys Lys Arg Ser Ser
            20                  25                  30

Tyr Glu Ile Leu Thr Leu Thr Thr Trp Leu Leu Gln Gln Glu Gln Ala
        35                  40                  45

Gly Val Ile Asp Ala Glu Leu Thr Ile Val Leu Ser Ser Ile Ser Thr
    50                  55                  60

Ala Cys Lys Gln Ile Ala Ser Leu Val Gln Arg Ser Gly Ile Ser Asn
65                  70                  75                  80

Leu Thr Gly Val Gln Gly Ala Val Asn Val Gln Gly Glu Asp Gln Lys
                85                  90                  95

Lys Leu Asp Val Val Ser Asn Glu Val Phe Ser Asn Cys Leu Arg Ser
            100                 105                 110

Ser Gly Arg Thr Gly Ile Ile Ala Ser Glu Glu Glu Asp Val Pro Val
        115                 120                 125

Ala Val Glu Glu Ser Tyr Ser Gly Asn Tyr Ile Val Val Phe Asp Pro
    130                 135                 140

Leu Asp Gly Ser Ser Asn Ile Asp Ala Ala Val Ser Thr Gly Ser Ile
145                 150                 155                 160

Phe Gly Ile Tyr Ser Pro Asn Asp Glu Cys Leu Ala Asp Ile Gly Asp
                165                 170                 175

Gly Asp Glu Ser Asn Leu Asp Thr Gln Glu Gln Lys Cys Val Val Ser
            180                 185                 190

Val Cys Gln Pro Gly Ser Asn Leu Leu Ala Ala Gly Tyr Cys Met Tyr
        195                 200                 205

Ser Ser Ser Val Ile Phe Val Leu Thr Ile Gly Asn Gly Val Phe Ala
    210                 215                 220

Phe Asn Leu Asp Pro Met Tyr Gly Glu Leu Val Leu Thr Gln Glu Asn
225                 230                 235                 240

Ile Gln Ile Pro Lys Ala Gly Lys Ile Tyr Ser Phe Asn Glu Gly Asp
                245                 250                 255
```

Tyr Gln Met Trp Asp Glu Lys Leu Lys
260 265

<210> 206
<211> 50
<212> DNA
<213> Artificial sequence

<220>
<223> primer: prm08448

<400> 206
ggggacaagt ttgtacaaaa aagcaggctt aaacaatggc cgccaccatg 50

<210> 207
<211> 50
<212> DNA
<213> Artificial sequence

<220>
<223> primer: prm08449

<400> 207
ggggaccact ttgtacaaga aagctgggta gctgcttagt gcttcttggt 50

<210> 208
<211> 1167
<212> DNA
<213> Oryza sativa

<400> 208
atccaagcca agaagaggga gagcaccaag gacacgcgac tagcagaagc cgagcgaccg 60

ccttcttcga tccatatctt ccggtcgagt tcttggtcga tctcttccct cctccacctc 120

ctcctcacag ggtatgtgcc cttcggttgt tcttggattt attgttctag gttgtgtagt 180

acgggcgttg atgttaggaa aggggatctg tatctgtgat gattcctgtt cttggatttg 240

ggatagaggg gttcttgatg ttgcatgtta tcggttcggt ttgattagta gtatggtttt 300

caatcgtctg gagagctcta tggaaatgaa atggtttagg gtacggaatc ttgcgatttt 360

gtgagtacct tttgtttgag gtaaaatcag agcaccggtg attttgcttg gtgtaataaa 420

agtacggttg tttggtcctc gattctggta gtgatgcttc tcgatttgac gaagctatcc 480

tttgtttatt ccctattgaa caaaaataat ccaactttga agacggtccc gttgatgaga 540

ttgaatgatt gattcttaag cctgtccaaa atttcgcagc tggcttgttt agatacagta 600

gtccccatca cgaaattcat ggaaacagtt ataatcctca ggaacagggg attccctgtt 660

cttccgattt gctttagtcc cagaattttt tttcccaaat atcttaaaaa gtcactttct 720

ggttcagttc aatgaattga ttgctacaaa taatgctttt atagcgttat cctagctgta 780

gttcagttaa taggtaatac ccctatagtt tagtcaggag aagaacttat ccgatttctg 840

atctccattt ttaattatat gaaatgaact gtagcataag cagtattcat ttggattatt 900

tttttatta gctctcaccc cttcattatt ctgagctgaa agtctggcat gaactgtcct 960

caattttgtt ttcaaattca catcgattat ctatgcatta tcctcttgta tctacctgta 1020

gaagtttctt tttggttatt ccttgactgc ttgattacag aaagaaattt atgaagctgt 1080

aatcgggata gttatactgc ttgttcttat gattcatttc ctttgtgcag ttcttggtgt 1140

agcttgccac tttcaccagc aaagttc 1167

<210> 209
<211> 1629
<212> DNA
<213> Oryza sativa

<400> 209
atgatgggtt gcttcactgt cctgagatcc aagaagaaga agcctcttgc tctcaccaag 60

aaatcggttg atgcaaggga aagcacgtcc tcaagactcc cagagccaga agcgcatgtg 120

ccatcgttac aatctgctcc tcctagtttt aggaacaagg ctaaaatcca ccaatcggaa 180

aagaaagctt cttacagcag agcgcgcgtg ctgtctgctc cttccagcct aattgtggtt 240

gatcaggatg gtcttccata tgccgaattc gatgatcaag atgactccag gggcaaggga 300

ggttctataa agggccaccg tttctctaat ccactgcccc ttcctctccc atcaccagaa 360

ggaaaatcat tgaggaactt tggcagcttc aaagccatca atgcaagtgg accactcgat 420

gcttcaggcc ctctgccact tcctccaaag aagtgtgatg ggcttaagaa tttctcctat 480

gaggaacttt catcagcttg ccaatggttt tctggtgacc agtgtgtttc cgaaagtttg 540

acatcaacat catacaaggc gtcctttagg gatgatttta ccgacccaaa gaccattgaa 600

gcaatagtat ctcggttgct ctcctccact cagagtttga aagagtttaa aacacaagtg 660

aataccttgg catcacttca gcatcccaac ttatgtaaac taatcggctt tcacgcaaga 720

gaagaatcta atgaaaggat gttggtctat gagcgactcc atcatggcag cttagataaa 780

ctactctttg gaagatcgga tggtcgtttc atggactggt cagcacgttt gaaggttgct 840

cttggtgctg ctagaggcct ggctttccta catgatgaag ggccttttca ggccatgtac 900

aatgacttct caacctcaaa catccaaatt gacaaagatt tcactgcaaa gctatcagga 960

tatggatgtg ttggattcaa taccgaggag gaaatatcaa atgcatctgt ggctgctgca 1020

aacctctcag tggaaacctt ggagaaaggt gtactgactc ccaagagcaa cgtatggtgc 1080

tttggagttg tcctgctgga gctaataaca ggaaggaaga accttgatgt ccgttcctca 1140

aaagaagaac gcaatattgt caagtggagt aggcctttcc tcaccgatga tagtcgccta 1200

tcgttaatca tggactcccg tataaaagga cgcttcccta ccaaggctgc tcggattgta 1260

gcagatatca tattgagatg ccttaataaa gatccatcag agaggcctac catgagggcc 1320

gttgtggagt ccctagcaag cgtccaggac ataaaggttc catgtcgata tcctttgcaa 1380

gagccatctg ctgccccaag aaaggtgatg ttaaaatcta caagtctcaa tggcatcatt 1440

catcaccatc ctgtcgtaac cttctcaccg tcacctcctt cgcgaaacca acatttgctc 1500

tcgccaaggt catccacgtc tgcactgctt cctccaagga ccagctgtgc tctggatgac 1560

cctagagtaa gctctatcaa gaaatcgcct tcccctattt tacggagatc tggtgttgag 1620

ggtttttga 1629

<210> 210
<211> 542
<212> PRT
<213> Oryza sativa

<400> 210

Met Met Gly Cys Phe Thr Val Leu Arg Ser Lys Lys Lys Lys Pro Leu
1 5 10 15

Ala Leu Thr Lys Lys Ser Val Asp Ala Arg Glu Ser Thr Ser Ser Arg
20 25 30

Leu Pro Glu Pro Glu Ala His Val Pro Ser Leu Gln Ser Ala Pro Pro
35 40 45

Ser Phe Arg Asn Lys Ala Lys Ile His Gln Ser Glu Lys Lys Ala Ser
50 55 60

Tyr Ser Arg Ala Arg Val Leu Ser Ala Pro Ser Ser Leu Ile Val Val
65 70 75 80

Asp Gln Asp Gly Leu Pro Tyr Ala Glu Phe Asp Asp Gln Asp Asp Ser
85 90 95

Arg Gly Lys Gly Gly Ser Ile Lys Gly His Arg Phe Ser Asn Pro Leu
100 105 110

Pro Leu Pro Leu Pro Ser Pro Glu Gly Lys Ser Leu Arg Asn Phe Gly
115 120 125

Ser Phe Lys Ala Ile Asn Ala Ser Gly Pro Leu Asp Ala Ser Gly Pro
130 135 140

Leu Pro Leu Pro Pro Lys Lys Cys Asp Gly Leu Lys Asn Phe Ser Tyr
145 150 155 160

Glu Glu Leu Ser Ser Ala Cys Gln Trp Phe Ser Gly Asp Gln Cys Val
165 170 175

Ser Glu Ser Leu Thr Ser Thr Ser Tyr Lys Ala Ser Phe Arg Asp Asp
180 185 190

Phe Thr Asp Pro Lys Thr Ile Glu Ala Ile Val Ser Arg Leu Leu Ser
195 200 205

Ser Thr Gln Ser Leu Lys Glu Phe Lys Thr Gln Val Asn Thr Leu Ala
210 215 220

Ser Leu Gln His Pro Asn Leu Cys Lys Leu Ile Gly Phe His Ala Arg
225 230 235 240

Glu Glu Ser Asn Glu Arg Met Leu Val Tyr Glu Arg Leu His His Gly
245 250 255

Ser Leu Asp Lys Leu Leu Phe Gly Arg Ser Asp Gly Arg Phe Met Asp
260 265 270

Trp Ser Ala Arg Leu Lys Val Ala Leu Gly Ala Ala Arg Gly Leu Ala
275 280 285

Phe Leu His Asp Glu Gly Pro Phe Gln Ala Met Tyr Asn Asp Phe Ser
290 295 300

Thr Ser Asn Ile Gln Ile Asp Lys Asp Phe Thr Ala Lys Leu Ser Gly
305 310 315 320

Tyr Gly Cys Val Gly Phe Asn Thr Glu Glu Glu Ile Ser Asn Ala Ser
325 330 335

Val Ala Ala Ala Asn Leu Ser Val Glu Thr Leu Glu Lys Gly Val Leu
340 345 350

Thr Pro Lys Ser Asn Val Trp Cys Phe Gly Val Val Leu Leu Glu Leu
355 360 365

Ile Thr Gly Arg Lys Asn Leu Asp Val Arg Ser Ser Lys Glu Glu Arg
370 375 380

Asn Ile Val Lys Trp Ser Arg Pro Phe Leu Thr Asp Asp Ser Arg Leu
385 390 395 400

Ser Leu Ile Met Asp Ser Arg Ile Lys Gly Arg Phe Pro Thr Lys Ala
405 410 415

Ala Arg Ile Val Ala Asp Ile Ile Leu Arg Cys Leu Asn Lys Asp Pro
420 425 430

Ser Glu Arg Pro Thr Met Arg Ala Val Val Glu Ser Leu Ala Ser Val
435 440 445

Gln Asp Ile Lys Val Pro Cys Arg Tyr Pro Leu Gln Glu Pro Ser Ala

450 455 460

Ala Pro Arg Lys Val Met Leu Lys Ser Thr Ser Leu Asn Gly Ile Ile
465 470 475 480

His His His Pro Val Val Thr Phe Ser Pro Ser Pro Pro Ser Arg Asn
485 490 495

Gln His Leu Leu Ser Pro Arg Ser Ser Thr Ser Ala Leu Leu Pro Pro
500 505 510

Arg Thr Ser Cys Ala Leu Asp Asp Pro Arg Val Ser Ser Ile Lys Lys
515 520 525

Ser Pro Ser Pro Ile Leu Arg Arg Ser Gly Val Glu Gly Phe
530 535 540

<210> 211
<211> 1674
<212> DNA
<213> Arabidopsis thaliana

<400> 211
atggttttgg ggtgtttccc tttgaaaagc aagaagaaac gtggctctgt ttctatgaag 60

cggttggatc ttgaagaaag caagccaact gctttacctg agccaccaaa gattccaagt 120

cgtaatttac aatcaggtcc tccgagtttc agaaatcgtg tgaagccaat tcaatcaaac 180

aacggtggaa acggagagat gagtagccga gcaagagtca tgtttgctcc gtcaagcatc 240

cacggtgcag cggaacggga tttgcttgct ggtgtttacc acgacgagca agatgaacaa 300

ccaagagatc cacgtacttc tactaaagaa tctagccctc aaccacttcc gttaccgtca 360

ccaagaactg gttcttcatt gaagaattgg ggaagcttta agtcgtttaa cggaagcagc 420

ggtcggttat catcatccgc agctgtatct ggacctttac ctttgccacc tagcgggtca 480

gttaggagct tttcatatga tgaagtaatg gctgcgtgta acgctttttc ttcagaccga 540

tgtgtcatgg aaggtctttc atctgttatg tacatggctt cctttggtga tgaggcttcg 600

acctcaggtt taaagaaggt tgacgcaact gttgtacgac ttcacgtaac tactcagagt 660

attagggagt tcattaatga agtcaacaca ttggcgtcgc tgcaacacca gaacctttgt 720

aagctggtag gctatcatgc tcgtgacggt tctgacacaa gaatgttggt gtacgagagg 780

cttgctctgg gcagcttgga ccgtttactg catgggagat cagatgggcc tcctcttgat 840

tggaacacta gaatgaagat tgcactatgt gcagctcagg gtctaacctt cttgcacgaa 900

gaaggccctt ttcaggcaat gtacaatgaa ttttcgacgg caaatatcca agtcgataaa 960

gatttcagcg ccaagctatc aggatacggt tgtgcaggcc atgcgcctga gacagagaca 1020

tctaatagtt cggcacttgc taatctctct gtcgagactc tagagagagg gcttttgacc 1080

ccgaagagca atgtgtggag ctatggaata gttcttcttg agatgttaac gggtcggaaa 1140

aatatggacg ggtcttaccc gaaagaagag aggaacttag tgaaatggag cagagctttt 1200

ctagcagatg attgcaggct ctcgcttata atggatcctc agcttaaagg tcggtttccg 1260

gcaaaagcgg cgaggagcat agcagatata gcacagaaat gtctgcaggt ggagccatca 1320

gagcgtccaa ccatgagaaa catcgtggat caactcaaga tcatacagga catgaagtac 1380

tcgtgtaggt tcccgttaag agaacccgca ccagtcgcgg caaggaaaca tatgggaaga 1440

tcaagcagtc tcaacacgat tatttggacc ccggcatcag tgccaccaag gtcaagtttt 1500

tcaccgtcac ctccaccacg acgaccgtct gtttcaccca caaggggacg gacgctcgtg 1560

tttcccccag tgtttccgcc gcgagcgtgt tcatctttgg aggaaatggc tcgggaagag 1620

gttcgaagat cgtcttcagc cagtggtagg agaactagcc tcgaagggtt ttga 1674

<210> 212
<211> 557
<212> PRT
<213> Arabidopsis thaliana

<400> 212

Met Val Leu Gly Cys Phe Pro Leu Lys Ser Lys Lys Lys Arg Gly Ser
1 5 10 15

Val Ser Met Lys Arg Leu Asp Leu Glu Glu Ser Lys Pro Thr Ala Leu
20 25 30

Pro Glu Pro Pro Lys Ile Pro Ser Arg Asn Leu Gln Ser Gly Pro Pro
35 40 45

Ser Phe Arg Asn Arg Val Lys Pro Ile Gln Ser Asn Asn Gly Gly Asn
50 55 60

Gly Glu Met Ser Ser Arg Ala Arg Val Met Phe Ala Pro Ser Ser Ile
65 70 75 80

His Gly Ala Ala Glu Arg Asp Leu Leu Ala Gly Val Tyr His Asp Glu
85 90 95

Gln Asp Glu Gln Pro Arg Asp Pro Arg Thr Ser Thr Lys Glu Ser Ser
100 105 110

Pro Gln Pro Leu Pro Leu Pro Ser Pro Arg Thr Gly Ser Ser Leu Lys
115 120 125

Asn Trp Gly Ser Phe Lys Ser Phe Asn Gly Ser Ser Gly Arg Leu Ser
130 135 140

Ser Ser Ala Ala Val Ser Gly Pro Leu Pro Leu Pro Pro Ser Gly Ser

```
145                 150                 155                 160

Val Arg Ser Phe Ser Tyr Asp Glu Val Met Ala Ala Cys Asn Ala Phe
                165                 170                 175

Ser Ser Asp Arg Cys Val Met Glu Gly Leu Ser Ser Val Met Tyr Met
            180                 185                 190

Ala Ser Phe Gly Asp Glu Ala Ser Thr Ser Gly Leu Lys Lys Val Asp
        195                 200                 205

Ala Thr Val Val Arg Leu His Val Thr Thr Gln Ser Ile Arg Glu Phe
    210                 215                 220

Ile Asn Glu Val Asn Thr Leu Ala Ser Leu Gln His Gln Asn Leu Cys
225                 230                 235                 240

Lys Leu Val Gly Tyr His Ala Arg Asp Gly Ser Asp Thr Arg Met Leu
                245                 250                 255

Val Tyr Glu Arg Leu Ala Leu Gly Ser Leu Asp Arg Leu Leu His Gly
            260                 265                 270

Arg Ser Asp Gly Pro Pro Leu Asp Trp Asn Thr Arg Met Lys Ile Ala
        275                 280                 285

Leu Cys Ala Ala Gln Gly Leu Thr Phe Leu His Glu Glu Gly Pro Phe
    290                 295                 300

Gln Ala Met Tyr Asn Glu Phe Ser Thr Ala Asn Ile Gln Val Asp Lys
305                 310                 315                 320

Asp Phe Ser Ala Lys Leu Ser Gly Tyr Gly Cys Ala Gly His Ala Pro
                325                 330                 335

Glu Thr Glu Thr Ser Asn Ser Ser Ala Leu Ala Asn Leu Ser Val Glu
            340                 345                 350

Thr Leu Glu Arg Gly Leu Leu Thr Pro Lys Ser Asn Val Trp Ser Tyr
        355                 360                 365

Gly Ile Val Leu Leu Glu Met Leu Thr Gly Arg Lys Asn Met Asp Gly
    370                 375                 380

Ser Tyr Pro Lys Glu Glu Arg Asn Leu Val Lys Trp Ser Arg Ala Phe
385                 390                 395                 400

Leu Ala Asp Asp Cys Arg Leu Ser Leu Ile Met Asp Pro Gln Leu Lys
                405                 410                 415
```

Gly Arg Phe Pro Ala Lys Ala Ala Arg Ser Ile Ala Asp Ile Ala Gln
420 425 430

Lys Cys Leu Gln Val Glu Pro Ser Glu Arg Pro Thr Met Arg Asn Ile
435 440 445

Val Asp Gln Leu Lys Ile Ile Gln Asp Met Lys Tyr Ser Cys Arg Phe
450 455 460

Pro Leu Arg Glu Pro Ala Pro Val Ala Ala Arg Lys His Met Gly Arg
465 470 475 480

Ser Ser Ser Leu Asn Thr Ile Ile Trp Thr Pro Ala Ser Val Pro Pro
485 490 495

Arg Ser Ser Phe Ser Pro Ser Pro Pro Pro Arg Arg Pro Ser Val Ser
500 505 510

Pro Thr Arg Gly Arg Thr Leu Val Phe Pro Pro Val Phe Pro Pro Arg
515 520 525

Ala Cys Ser Ser Leu Glu Glu Met Ala Arg Glu Glu Val Arg Arg Ser
530 535 540

Ser Ser Ala Ser Gly Arg Arg Thr Ser Leu Glu Gly Phe
545 550 555

<210> 213
<211> 2069
<212> DNA
<213> Sorghum bicolor

<400> 213
ctactggatt aacccccgc gtgcgttccc tccctctgtc agtctgtctc tctcttggcg 60

tcgactgggc gaccgtcgca gccgccctaa gccaggtacc cagaccatct tcgggccctt 120

cgccggcgac gagcaccacc aggaattttc ccagctgtag caatgatggg ttgcttcact 180

gttctcagat ccaagaagaa gaaaatccct tttgataatc ctcttcttcc aagcaagaaa 240

tcggttgatg caagggaaag tacgtcgtct agacttccgg aaccagaagt tcatgtgcca 300

tctttgcaat cagctctctc ctagttttag gaacaggact aagatatccc agtcgtcaaa 360

taaagtttca aacagcagag ctcgcgtgtt gtctgctccg tcaaccctta ttgtggttga 420

tcagtttggc tttccatatg ctgaataccg agatcaagac gactccagag ataaggaagg 480

ttcaacaaag gggcatcgct tttcaaatcc tttgcccctt cctcttcctt caccggaagg 540

acattctttt aggaactctg gtagcttcaa agctagcaac gtaagtgggc cattggagat 600

gtccagccct ctcccattgc ctccaaagaa atgtgatgga cttagaatct tttcttacga 660

ggaggttttg tctgcttgcc aatggttttc aagtgatcag tgtgtttcag aagcacttgg 720

ttcaacatca tacaaggcaa catttaggga tgaatttatt gatacaaaga ccactgaagc 780

aacggtagct cgattactcc cctccactca gagtttgaag gagtttaaaa cacaagcaac 840

tacattggca ttgcttcagc atcccaattt atgtaaactg attggctatt atgcaaaaga 900

agattctaat gaaaggatgt tggtctatga acggcttcat catgggagct tagataagct 960

actctttgga agaccagatg gtcgtttcat ggactggtct aaacgtttga aggttgccct 1020

tggtgcggcc agaggtcttg ctttcttgca tgatgaagga ccctttcagg ccatgtacag 1080

cgagttctca actttgaaca tccaaataga taaagatttc actgctaagc tttcaggata 1140

tggttgtgtt ggcttcaata ctgaggagat atctaatgca cctgcgtctg cagcaaacct 1200

ttcggtggag accttggaga agggtttact cactcccaag agcaatgtct ggagctttgg 1260

agttgtgtta ctggagctaa taactggaag gaagaacctt gatgccaatt cttctaaaga 1320

agaacgcaat attgtcaggt ggagtaggcc tttccttact gatgatagtc gcctatctct 1380

gatcatggac tcccgtataa aagggcgctt tcctactaag gctgctcgga tagtagcaga 1440

catcattctg aaatgcttgc ataatgatcc atccgagagg ccgaccatga gggatgttgt 1500

ggaggctcta gcaagagtcc aggagataaa ggttccgtgc agatatcctt tgcaagaacc 1560

ttctgctgca ccaagaaaag taatgctaaa atctacatcc ctcaatggaa ttgtgcctca 1620

tcatcctgtc ataaccttct ctccatcgcc gccttcgcat aaccagcact tgatttcacc 1680

aaggtcatca acatctgcct tgtttcatcc aaggacatgc tcttctactc tggatgatcc 1740

cggtgtaagc tctataaaga aaacacctcc tattatgcgt aggtctagcg ttgaaggctt 1800

ttgattgtcc atattgttct tttatttctt tttctcggat ttatttgttt ctttgttagc 1860

ctagtgattt tagctgtgtt ctgtattgta agacaagtgg ccttatgtag tgcccttgag 1920

tctcgagtaa taactaagca gagcaggatg aattgtaaat agtatcaggt tgtagatacc 1980

tttttgtgct ctaattgggt ggaagcagcg tgctgagtct gagtaggcct tgtaacctca 2040

taaataaact cgtttcccag tttctgtcc 2069

<210> 214
<211> 1360
<212> DNA
<213> Saccharum officinarum

<400> 214
agaaacactt ggttcaacat catacaaggc aacatttagg gatgaattta ttgatacaaa 60

gaccactgaa gcaacggtag ctcgattact cccctccact cagagtttga aggagtttaa 120

aacacaagcg accacattgg catcacttca gcatcccaat ttgtgtaaac tgattggcta 180

ttatgcaaaa gaagattcta atgaaaggat gttggtctat gaacggcttc atcatgggag 240

cttagataag ctactctttg gaagaccaga tggtcgtttc atggactggt ctaaacgttt 300

gaaggtttcc cttggtgctg cccgaggtct agctttcttg catgatgaag gaccctttca 360

ggccatgtac agtgagttct caactttgaa catccaaata gataaagatt tcactgctaa 420

gctttcagga tatggttgtg ttggcttcaa tactgaggag atatctaatg cacctgcgtc 480

tgcagcaaac ctttcggtgg agaccttgga gaagggttta ctcactccca agagcaacgt 540

ctggagcttt ggagttgtgt tactggagct aataactgga aggaagaacc ttgatgccaa 600

ttcttccaaa gaagaacgca atattgtcag gtggagtagg cctttcctta ctgatgatag 660

tcgcctatct ctgatcatgg actcccgtat aaaagggcgc tttccaacta aggctgctcg 720

gatagtagca gacatcattc tgaaatgctt gcataaagat ccatcagaga ggccgaccat 780

gagggatgtt gtggaggccc tagcaagagt ccaggaaata aaggttccat gcagatatcc 840

tctgcaagaa ccttttgctg caccaagaaa aataatgtta aaatctacat ccctcaatgg 900

aattgtgcct catcatcctg tcataacctt ctccccttcg ccgccttcac ataaccaaca 960

cttgatttca ccaaggtcat caacatctgc cttgtttcat ccaaggacat gctcttctac 1020

tctggatgat cccggtgtaa gctctataaa gaaaacacct cctattatgc gtaggtctag 1080

cgtcgaaggc ttttgattgt ccatattgtt cttttatttc cttttcttgg atttattagt 1140

ttctttggta gcctagtgat tctagctatg ttctgtattg caagacaagc ggccttaatg 1200

tagtgccctt gggtctagag taataactaa gcagagcagg atgaattgta aataggatca 1260

ggttgtagat accttttgt gctctaattg ggtggaagca gcgtgctaag tgagccttgt 1320

aacctcccaa tgcaataaac tcgtttttca gtttttgttc 1360

<210> 215
<211> 2062
<212> DNA
<213> Medicago truncatula

<400> 215
acgaggctat tctctctctt tctctctcta catttctcaa cattcttcaa atttctctct 60

ccaaaacctt cacttcgcag ctttttcaat ctgctttctt tgactctgta acagtttttt 120

tcgtggaaga atcagaacca caaaaaagtt tcgattttta cccatttctt caacccgtga 180

tcgcttcact gtaattggca tgagcttcca tttctcgttc tgacaaaaac aggtatctat 240

aacatcctag ttctgtacat agaatggggt gttttactat attgaagaga aagaagaaaa 300

agcctgatca gattgtgtat gtaaaacgcg taagccctgg cgaagattca cctacagtac 360

tgcccgaacc ccaaactcat acccgctcac tccagtctgc gcctcctagt tttaagatca 420

gagtgaaacc cattcaacct agcaataaag ccactaacaa tagaatacga gcattgtctg 480

ctccatcgag tcttgatgat gcagaacaag atgcattggc caccattgag tatgaagaac 540

aagaagggtc aaaataccga actggatcat ggaaggagca gcgttcgcct agtccacaac 600

cattaccgct tccatctcct aagggtggtg gtacattgaa gactgttgga agctttaagt 660

tggggatagc tagtagtcct ttatatgctt ctggaccttt gccgcttcca ccaactgggt 720

cactgagaaa cttttcttat gatgaacttg ctgctgcttg cctcaatttc tcttcagatc 780

gatacatgtc agaatctctt tcatccacca tgtataaagc ttcctttggt gatgatacct 840

caacttcaag ttcaaagaag tttgaagcta ctgtcacacg ccttcgccca tcatctcagg 900

gcctgaagga attcataaat gaggttaata ctcttgcatc attgcagcat ccgaacctct 960

gtagattgct aggatttcac gcaggtgatg gttcagagca tagaatgttg gtttatgaga 1020

ggctatacca tggaagcttg gaccgcttat tgtatgggag atctgatggg ccatcaattg 1080

attggaatac aagaatgaaa attgcaatat gtgctgcact aggtctttct ttcttgcatg 1140

aagaagggcc ttttcaggca atgtataatg aattttcaac agctaacatt cagattgaca 1200

aagatttcag tgcaaagctt tcaggatatg gttgtgttgg acatgttccg aaggaagaga 1260

tttcaagcag ttcatctgcc gttggaaacc tatcgatgga gacactggag aaaggaatgc 1320

ttactccgaa aagcaatgta tggagtttcg gaattttcct tctagagcta cttacaggaa 1380

gaaagaatct cgatagcccg tcacccaaag gaagagagaa atttggtgaa gtggagcagg 1440

cctttcctgt ctgataatca tcgtttgtca atgatcatgg atcctcaact taaaggtcgc 1500

tttccttcta aagcggcgag tacaatagct aacattgcac aaagatgcct tcaaatggag 1560

ccatcagaac gaccaaccat gggaacagtt gttgagcagc tgaaaaagat acaagatttg 1620

aagcattcta gcagattccc gctgcaagaa cctgcacaaa tgtcgagatc accaagtctt 1680

aacggtatca accaccctgc accaaggccg agtttctctc catcaccatc atccagagcc 1740

ctggtatctg tctcacctcc aagatggtcg ggagtgtcaa ttcaacttcc acctcgcgcg 1800

ttttcttcaa ccctctattt ggaggagctt gataggcaag aaagccgcaa gtcagcttca 1860

gcctctagga aggctagtgt tgaaggattt tgattgtcga tagtgttcat tttttatttg 1920

ttattctttt tttggtgtag ttcaacagag atttatagga gataaagatt tgtaatcatc 1980

cctcagtgtg atgcagagag gatgctcttt tgtacatagt gcaaaggttg gtccccttgg 2040

ttcaattagt tactccattt tg 2062

<210> 216
<211> 2039
<212> DNA
<213> Zea mays

<400> 216
ctcccccggc gtgcgctccc tcctgtctct cttggcgacg actgggcaac ggtcgcagct 60

gtatgttcga gcccttcgcc gacgacgttc accaccagga attttctcag ctgtgccaat 120

gatgggttgc ttcactgttc tcagatccaa gaagaagaaa agccattttg ataatcctct 180

tgtcccaagc aagaaatcag ttgatgcaag ggaaagtacg tcctctagac ttccggagcc 240

agaagttcat gtgccatctt tgcaatcagc tcctcctagt tttaggaaca gggtcaagat 300

```
atccgagtca tcaaatgaag tttcaaacag aaacagcagg gctcgcgtgc tgtctgctcc    360
atcagccctt attgtggttg atcagtttgg ctttccatat tcggaatacc gagatcaaga    420
tgactccagg gataaggaag gtttgacaaa ggggcatcgc ttttcaaatc ctttgcccct    480
tcctcttcct tcacgcgaag gacattcttt taggaactct ggtagcttca aagccagcaa    540
cgtaagcggg ccattggaga tgtctggccc tctcccattg cctctaaaga aatgtgatgg    600
acttagaatc ttctcttatg aggagatttc atctgcctgc caacgatttt ctagtgatca    660
gtgtgtttca gaaacacttg gttcaacatc atacaaggca acatttagag atgaatttat    720
tgatacgagg accactgaag caacagtagc tcgattactc ccctccactc agagtttgaa    780
ggagtttaaa acgcaagcga ccacattggc atcgcttcag catcccaatt tatgtaaact    840
gattggctat tatgcaaaag aagattctaa tgaaaggatg ttggtctatg aacggcttta    900
tcatggaagc ttagataagc tactctttgg aagatcagat ggtcgtttca tggactggtc    960
taaacgtttg aaggttgccc tgggtgctgc cagaggtcta gctttcttgc atgatgaagg   1020
accctttcag gccatgtaca gcgagttctc aactttgaac atccaaatag ataaaggttt   1080
cactgctaag ctttcaggat atggttgtgt tggcttcaat accgaggaga tatctaatgc   1140
acctgtgtct gcagcaaacc tttcggtgga gaccttggag aagggtttac tcactcccaa   1200
gagcaacgtc tggagctttg gagtcgtgtt actggagcta ataactggaa ggaagaacct   1260
tgatcccaat tattccaaag aagaacgcaa tattgtcaac tggagtaggc ctttccttac   1320
tgatgacagt cgcttgtctc ttatcatgga ctcccgtata aaagggcgct ttcccactaa   1380
ggctgctcgg atagtagcag acatcatttt gaaatgcctg cgtaaggatc catcggagag   1440
gcctaccatg agggatgttg tggaggccct agcaagagtc caggaaataa aggttccgtg   1500
cagatatcct ctgcaagaac cttctgctgc accaagaaaa gtgatgttga aatctacatc   1560
cctcaatggg attgtgcctc accatcctgt cataaccttc tctccatcgc cgccttcaca   1620
taaccagcac ttgatttcac caaggtcgtc gacatctgcc ttgtttcatc caagggcatg   1680
ctcttctacc ctggacgatc cgagtgtaag ttctataaag aaaacaccac ctattatgcg   1740
aaggtctagc gtcgaaggct tttgattgtc catattgttc ctttatttcc ttttcttgga   1800
tttatttgtt tctttgttag tagtgtagcg gtttttagct gtgttctgta ttgtaagaca   1860
agtggcctca tatgtagtgc ccttgggtct ggagtaaagc agaacaggat gaattgtaaa   1920
taggaccagg ttgtagatac ctttttttg tgctcgaatt gggtggaagc agcgtgccga   1980
gtgggccttg taacctcata atgcaataaa ctccgtttcc cagtttctgt aaaaaaaaa    2039
```

```
<210>  217
<211>  1479
<212>  DNA
<213>  Hordeum vulgare
```

<400> 217
tgccgaattc ggcacgagaa agaggtccga ggggcttaag aacttctcgt acgatgagat 60

ttccactgct tgccagtggt tttctggcga tcatcgtgtc tcagaaactc tgacttcgac 120

atcatacaag gcattgttca gggatgattt cgttgaacca aagaagatgg aagcaatagt 180

agccaggtta ctcccttcca atcagagttt caaagaattt aaggcacaag taaatacgtt 240

ggcatcactt caacatccca atttatgtaa actcatcggc tatcacgcaa gagaagaatc 300

taatgaaagg atgttggtct atgagcggct ccatcatggc agtttagaca ggttactctt 360

tggaagaccg gaaggtcgtt tcatggactg gtctacacgt ttgaaggttg cccttggtgc 420

tgctaaaggt ctagctttcc tacacgatga aggacccttt caggcaatgt atgatgactt 480

ctcaacgtca aacatccaaa tagagaaaga tttcactgca aagctgtcgg gatatggttg 540

tgttggcttc aattctgacg aggaaagatc caaggcatct gtggctgcaa acctctcaga 600

ggaaaccttg gagaaaggcg tactgactcc gaagagcaac gtatggagct ttggagttgt 660

cttgctcgag ctaataacag gacggaagaa cctcgatgta cgttccacca aagaagaacg 720

taatattgtc aagtggggta ggcctttcct caccgacgat agtcgtctat ccctcatcat 780

ggatccccgt ataaaaggac gctttcctac caaggctgct cgaactgtgg cagacataat 840

tctgaagtgc cttcaaagag atccatcaga aaggcctacg atgagggccg tcgtggaggc 900

cctaacaagc gttcaggaca taaaggtccc ctgccggtat cctcttcaag taccgtcccg 960

ccgcacccga ggaaagtgat gctaaagtct acgagcctca atggcattgt tcctcagcat 1020

cccgtcatga ccttctcgcc gtcgcctcct tcgcgcaacc agcacctggc gtcgccaaga 1080

tcatcgactt ccgcgcttct tcccccaagg acctgctctt ccatcatcac cctggattac 1140

cccagggtaa gctcggtcaa gaagtcgcct tccggtatcc tgcggagacc tggcgtcgag 1200

ggtttttgat tgtccataca tggtcggatt tcttctcttt gccttggatt tatttgttgt 1260

tttggttagc ctagcgagtg gtctcagtgc tatgtgatat gtatatgttg gaaagacatc 1320

tgaaaaaagg gcagagtgaa gtgtagatag tagaaccagc tttgtagata cttgtcgttc 1380

tctgattgga tgggggtcat ggaagcagtg tgtgttagag tggggcttgt aaccccatta 1440

ttatgcaata aacgtgggtt ggtcggtcgt atttgctcg 1479

<210> 218
<211> 769
<212> DNA
<213> Glycine max

<400> 218
gtaaattgct aggatttcat gcacgagagg gttcagaaca tagaatgttg gtttatgaaa 60

ggctatacca tggaagcttg gatcgcttat tgtatgggag atctgatggg ccatcaattg 120

attggaatac aagaatgaaa attgctatat gtgctgcaca aggtctaacc ttcttgcacg 180

aagaggggcc ttttcaggct atgtataatg agttctcaac agccaacata cagattgaca 240

aagatttcag cgcaaagctt tcaggatatg gttgtgttgg acatattccc gaagaagaga 300

tttcaagcag ctcatctgct gttggaaacc tatcaatgga aacactggag aaaggaatgc 360

tcactccaaa gagcaacgta tggagttttg gaatttttct tctggagcta cttactggaa 420

gaaagaatct tgatagccgt caccccaagg aagagaggaa tttagtcaag tggagccggc 480

ctttcttagc cgataactac cgtctgtcgt tgatcatgga tcctcaactc aaaggccgct 540

ttccttctaa agcagcaaga acaatagctg atattgcaca aagatgcctt caaaaggagc 600

catcagacag acctaccatg aggactgttg ttgagcatct caagataata caggatttga 660

aatattcgtg ccggttccct ctgcaagaac cagcatcaaa ctctggaaaa catatgtcaa 720

gatcaccaag tctcaatggc atcatctgcc ctgcaccaag ggtgagttt 769

<210> 219
<211> 1150
<212> DNA
<213> Solanum tuberosum

<400> 219
ttcttagctc atcgaaacgt taaaccttac aatacttcaa acatcgatca atacaatttg 60

ctgaagcacg caatttttca aggactgtat aaacaacaaa gatgggttgt ttcacagttt 120

taaaaagtaa gaagaagaag tctgaacaga gtattcacat caaacgtgtg aatcctcagg 180

aacattctcc tactgcattg cccgagcctc aagtgcagac acggtcgttg cagtcggcac 240

ctccaagttt tagaactaga gtaaaacctg tacagtcgag taatagagtt acaagcagta 300

gggcgcgggc actctctgcc ccatctagcc tggactcagc agaacaagat gtagcatcaa 360

atgaatgtga ggaacatgat gagttcaaga gtcgtattgg ttcaatcaag gagtaccaat 420

caccaagtcc tcagcctctt cctcttccat ctccacagag tgccgctgcc actctcaaga 480

ctatgggaag ctttaaagtt ggcaacgcca gcggtccgtt aaatgcctct ggacccctgc 540

cactgcctcc tacactgcct tcaacattgc cttctactgg agcactcagg aacttctcat 600

ttgaagaact tgctgctgcc tgccaccgct tctctcctga acggtgtatg tcagaaggtc 660

tctcttctgt tatttacaga gcttcttttg gagatgatgc aactggtaca aagaagcttg 720

aagccactgt aacccggctt catccttctt cgcaggggtt gaaggaattt gtgactgagg 780

tgaacacact agcttctttg caacatccat cactttgtaa actgattggt ttccatgcca 840

cggggaaggt tccgagcaca gaatgttggt ttatgaaagg ctttttcatg gaagcttaga 900

ccggcttttg tttgggaggt ccgatggtcc cccgatagac tggaatgctc gaacgaaaat 960

tgctttatgt gctgctcaag gtctcacatt cctgcatgag gaaggacctt ttcaggcaat 1020

gttccatgaa ttttccactg caaatataca aattgataag gattgcagtg caaagctctc 1080

gggatatgga tgcattactc ctataccaga gacagacata tcatgcagtt cagctgccct 1140

ggcaatctct 1150

<210> 220
<211> 910
<212> DNA
<213> Lycopersicon esculentum

<400> 220
catggaagct tacaccggct tttatttggg aggtcggatg gtcccccaat agactggaat 60

gctcgaataa aaattgcttt atgtgctgct caaggtctca cattcctgca tgaggaagga 120

ccttttcagg caatgttcca tgaattttcc actgcaaata tacaaattga taaggattgc 180

agtgcaaagc tctcgggata tggatgcatt actcatatac aagagacaga catatcatgc 240

agttcagctg ccctggcaaa tctctctgag gagactctgg agcgaggatt ggtaactcca 300

aagagtaatg tttggagttt tgggattgtt cttcttgagc tgctgactgg ccggaagaat 360

ttaagcagtc ggcatccaaa ggaagagagg aatttagtga agtggagcaa gctttttcta 420

gctgatgaca gtagattatc gctaatcatg gaccctcagt taaaaggtcg gttccctgcc 480

aaagcagcaa gaactgtggc tgatattgct caaagatgtc tgcaaaagga tccatctgaa 540

aggcccacca tgagaactgt agtggagcaa ctcaagactg tacaagttat gaagtaccct 600

tctcggtttc ctcttcaaga gccaagggca gttggtgtaa aacacatgtc aaagtgtccg 660

agcctaaatg gaattattac cccaacatca agattgagct tctccccttc accaccaacc 720

caccctatat ctatttctcc gacaaggaca gctgctccac tgctgtctct accttcatgt 780

tcctccatcc tctctatgga ggactttgat cgattggaaa atcgaaggcc atcatcttca 840

tctgttcgga ggtctagtgt cgaaggattt tgatcgattg tagagcactt ttttcttcaa 900

attgcttttc 910

<210> 221
<211> 681
<212> DNA
<213> Lycopersicon esculentum

<400> 221
gagaggcttt ttcatggaag cttagacaga cttttgttcg gaagatcaga tggcccctct 60

atagattgga atgcgagaac caaaattgcc ctatgtgctg cacaaggtct tacatttcta 120

catgaggagg gaccttttca ggcaatgttc caagaatttt caactggaaa tatacaaatc 180

gacaaggatt ttagtgcaaa gctctcgggg tatggatgca ttacgaatat acaagagacg 240

gagatatctt gcaattcaat cgccctggcg aatctctcac aggagacact ggagagaggg 300

ttgataactc ctaagagcaa tgtttggagt ttcgggattg ttcttttaga actgctcact 360

ggccggaaga atcttgatgg tcggtattca aaggaagaga ggaatctagt caagtggagt 420

aggcctttcc ttgctgatga tggtagatta tcgcttatca tggatcctca gcttaaagga 480

cggtttcccg caaaagcagc ccgtacagtg gctgatattg cccaaagatg cctgcaaaag 540

gatccatctg aaaggcccac catgagaact atcttggacc aactcaaatc cgtacaagtg 600

atgaagtgcc cttcacggtt tcctctgcaa gaaccagcgg ttgttggtgg taaacacatg 660

tcaaagtctc caagcatgaa t 681

<210> 222
<211> 741
<212> DNA
<213> Triticum aestivum

<400> 222
tttcctccca aggctgctag gactgtggca gacatcattc tgaagtgcct tcatagggat 60

ccatccgaaa ggcctacgat gagggccgtc gtggagtctc tagcaagcgt ccaggacata 120

aaggtcccct gccggtatcc tcttcaagta ccgtccgccg caccgaggaa agtgatgcta 180

aaatccacga gtctcaacgg cattgttcct cagcatcctg tcatgacctt ctcgccgtcg 240

cctccttcgc gcaaccagca cctggtgtca ccgagatcat cgacgtctgc gctgcttccc 300

ccaaggaact gctcttccac catcaccctg gactacccca gggtaagctc ggtcaagaaa 360

tcgcccccca acatcatgcg gagacctggc gtcgagggtt tttgattgtc catatagtgt 420

cggattttct tctctttgcc ttgatttatt tgttgttttg gttagcctag cgagtgatct 480

cagtgctatg ggatatacta tgttgcaagg acatgtgaaa aagggcagag tgaagcgtag 540

atagtagaac cagcttgtag atacttgtcg ttctctgatt ggatgggggt catggaagaa 600

gtgtgtgttg agtggggctt gtaaccccac tatgcaataa acgtgggttg gttggtcggt 660

cggtatttgc tcgccgtcaa acatttcagc tgtttctttc ttctcttgaa tgctaagttt 720

tgttgctgct agtcgtggaa t 741

<210> 223
<211> 2039
<212> DNA
<213> Zea mays

<400> 223
ctcccccggc gtgcgctccc tcctgtctct cttggcgacg actgggcaac ggtcgcagct 60

gtatgttcga gcccttcgcc gacgacgttc accaccagga attttctcag ctgtgccaat 120

gatgggttgc ttcactgttc tcagatccaa gaagaagaaa agccattttg ataatcctct 180

tgtcccaagc aagaaatcag ttgatgcaag ggaaagtacg tcctctagac ttccggagcc 240

agaagttcat gtgccatctt tgcaatcagc tcctcctagt tttaggaaca gggtcaagat 300

atccgagtca tcaaatgaag tttcaaacag aaacagcagg gctcgcgtgc tgtctgctcc 360

atcagccctt attgtggttg atcagtttgg ctttccatat tcggaatacc gagatcaaga 420

tgactccagg gataaggaag gtttgacaaa ggggcatcgc ttttcaaatc ctttgcccct 480

tcctcttcct tcacgcgaag gacattcttt taggaactct ggtagcttca aagccagcaa 540

cgtaagcggg ccattggaga tgtctggccc tctcccattg cctctaaaga aatgtgatgg 600

acttagaatc ttctcttatg aggagatttc atctgcctgc caacgatttt ctagtgatca 660

gtgtgtttca gaaacacttg gttcaacatc atacaaggca acatttagag atgaatttat 720

tgatacgagg accactgaag caacagtagc tcgattactc ccctccactc agagtttgaa 780

ggagtttaaa acgcaagcga ccacattggc atcgcttcag catcccaatt tatgtaaact 840

gattggctat tatgcaaaag aagattctaa tgaaaggatg ttggtctatg aacggcttta 900

tcatggaagc ttagataagc tactctttgg aagatcagat ggtcgtttca tggactggtc 960

taaacgtttg aaggttgccc tgggtgctgc cagaggtcta gctttcttgc atgatgaagg 1020

acccttttcag gccatgtaca gcgagttctc aactttgaac atccaaatag ataaaggttt 1080

cactgctaag ctttcaggat atggttgtgt tggcttcaat accgaggaga tatctaatgc 1140

acctgtgtct gcagcaaacc tttcggtgga gaccttggag aagggtttac tcactcccaa 1200

gagcaacgtc tggagctttg gagtcgtgtt actggagcta ataactggaa ggaagaacct 1260

tgatcccaat tattccaaag aagaacgcaa tattgtcaac tggagtaggc ctttccttac 1320

tgatgacagt cgcttgtctc ttatcatgga ctcccgtata aaagggcgct ttcccactaa 1380

ggctgctcgg atagtagcag acatcatttt gaaatgcctg cgtaaggatc catcggagag 1440

gcctaccatg agggatgttg tggaggccct agcaagagtc caggaaataa aggttccgtg 1500

cagatatcct ctgcaagaac cttctgctgc accaagaaaa gtgatgttga aatctacatc 1560

cctcaatggg attgtgcctc accatcctgt cataaccttc tctccatcgc cgccttcaca 1620

taaccagcac ttgatttcac caaggtcgtc gacatctgcc ttgtttcatc caagggcatg 1680

ctcttctacc ctggacgatc cgagtgtaag ttctataaag aaaacaccac ctattatgcg 1740

aaggtctagc gtcgaaggct tttgattgtc catattgttc ctttatttcc ttttcttgga 1800

tttatttgtt tctttgttag tagtgtagcg gtttttagct gtgttctgta ttgtaagaca 1860

agtggcctca tatgtagtgc ccttgggtct ggagtaaagc agaacaggat gaattgtaaa 1920

taggaccagg ttgtagatac cttttttttg tgctcgaatt gggtggaagc agcgtgccga 1980

gtgggccttg taacctcata atgcaataaa ctccgtttcc cagtttctgt aaaaaaaaa 2039

<210> 224
<211> 768
<212> DNA
<213> Lactuca sativa

<400> 224
accgactgat ggccgctagt cggggaagaa cacgaagaat ccaaaacccg tggcagcggc 60

ggaggcggag gcgggttgcc gcctgtcccg cagccgctgc cgcttcctgc accgcattca 120

cgccaacttc gaaaaccatc gcggaatgct tttaaagatt gagtggcggt gagttgggca 180

tgctgccagc ctcttaatac ttccggaccc tctgccactt ccgccatcgg gaaccaccca 240

tcttccgcca atgattccgg catctttacc gacatctgga acacttaaga acttcacata 300

ttgaagaaat cgcagctgct tgccacaaca ttttcaccct gacagatgcg tgtctgaagg 360

tctttcttct gttatgtata gagcttcttt tggagaagat acttctaatt taaagaatct 420

tcaagccact gttaccagtc tccatccttc aactcagggt ttgaaggaat ttgtgagtga 480

agtgaacacg ctagcatcat tgcaacaccc ttatctctgt aaatagattg gtttccatgc 540

gcgtgaggga tctgatagaa gaatgttggt ttacgagagg ctttttcatg gaagcttaga 600

tcggctttta tatggtacaa cagatggccc acctattgat tgcaatgcaa gaatgaaagt 660

cgcactctgt gctgctcaag ggcttacttt tttgcatgag gaaggaccat ttcaggcgat 720

gtttcatgaa ttttccactg ctaatataca aattgataaa gattttag 768

<210> 225
<211> 1637
<212> DNA
<213> Gossypium spp.

<400> 225
ctttttcctc tcaaacctct ctcacactga taaacttttc tctccattac ctttctttga 60

cttgcaaagt tgttctcctc aaactaacca aacaagggat tctgggtttt cagctttctc 120

tgttttccta ctctctttcg cctttgcatg acctgcttac atagaaacat aaaagtccga 180

aatacaattt aggtggtttg tatataagat ggggtgtttt acagttttga gaagcaacaa 240

gaaaaagtcc aaacagtcag tttttgttaa acacattgct cataaagagc atattcctac 300

catgctgcct gagccccaaa ttcagacacg atcactgcaa tctgcacccc caagttttat 360

aaccagagta aaaccaattc aatctaataa caaggcaagc tgcaatagga cacgtgcatt 420

atctgctccg tcaagtcttg atgctgctga gcaagatgac cttgcgtcag ttgaatttga 480

agaacaagaa gagttgaaga gtcgtgttgg tttagtcaag gaacagaagt catcaagtcc 540

acagcctctt ccacttccat ctccccacag tactgcgctg aagacaatgg gaagttttaa 600

agcagggaat gttagtggcc ctctttttgc ttcgggacca ttacccctgc ctccctctgg 660

aacactacgt aacttcgcct acgaagaaat tgcagctgct tgccatcatt tctcttctga 720

tcgatgcaca tctgagggtc tatcttctgt tatgtacaag gcatccttcg gagatgacac 780

atcaagttca aagaagtttg aagctactgt tactcgcctt cacccatcca ctcagggttt 840

aagggaattt ataaacgaag taaacactct tgcatcattg caacatccaa atctctgtaa 900

attgcttggg taccatgcac gtgataattc agaacaacga atgttggtct atgagaggtt 960

atttcatgga agcttagacc ggcttttata cgggagatcg gatggaccgc cacttgattg 1020

gaatactcgc atgaaaattg ctttatgttc tgcacagggt cttactttct tgcatgagga 1080

aggaccattt caggcaatgt acaatgaatt ttcgactgcc aacatacaga tcgacaagga 1140

tttcagtgca aagctctcag gatatgggtg cgttggtcat atcccagaga cagaagagat 1200

ctccagtaat tcagttgctg tggcaaatat atccgtagag actctggaga gagggcgact 1260

aactccaaag agcaatgttt ggagttttgg gatcattcta cttgaattac tcactggccg 1320

gaagaacctt gacaaccgtt atcccaagga agagaggaac ctagtgaagt ggagccggcc 1380

attcctagcc gacaattgta gattgtcact catcatggat cctcagctca aaggtcgctt 1440

tcctatgaaa gctgcccgta cggtggctga cattgcacaa aggtgtctcc agatggaccc 1500

atcagagagg ccaaccatga gaaccatcgt tgagcatctc aaaatcatcc aagacctgaa 1560

atactcttgt cggtttcctc tgcaagatcc agcagcaatt gctggaaaac agatgtcaag 1620

gtcaccgagt ctcaacg 1637

<210> 226
<211> 2193
<212> DNA
<213> Oryza sativa

<400> 226
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtaccttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga atttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcaccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 227
<211> 56
<212> DNA
<213> Artificial sequence

<220>
<223> primer: prm06729

<400> 227
ggggacaagt ttgtacaaaa aagcaggctt aaacaatgat gggttgcttc actgtc 56

<210> 228
<211> 50
<212> DNA
<213> Artificial sequence

<220>
<223> primer: prm06730

<400> 228
ggggaccact ttgtacaaga aagctgggta tggacaatca aaaaccctca 50

<210> 229
<211> 1176
<212> DNA
<213> Arabidopsis thaliana

<400> 229
cttcaactgc tgcgtctagc tgtccttcct tcttcatttg cttcttcttc tctagctcag 60

cgcggatcgc tgcagtagta ccctgacgta gcctttcttc ttcctttact ttctcatctt 120

ctatctctca aaagaaaagc agacaacttt atttgcaaaa acagagtttt tttttcttat 180

cttgagaaag ttcaacagaa gatgatgttc gagaaagacg atctgggtct aagcttaggc 240

ttgaattttc caaagaaaca gatcaatctc aaatcaaatc catctgtttc tgttactcct 300

tcttcttctt cttttggatt attcagaaga tcttcatgga acgagagttt tacttcttca 360

gttccaaact cagattcgtc acaaaaagaa acaagaactt tcatccgagg aatcgacgtg 420

aacagaccac cgtctacagc ggaatacggc gacgaagacg ctggagtatc ttcacctaac 480

agtacagtct caagctctac agggaaaaga agcgagagag aagaagacac agatccacaa 540

ggctcaagag gaatcagtga cgatgaagat ggtgataact ccaggaaaaa gcttagactt 600

tccaaagatc aatctgctat tcttgaagag accttcaaag atcacagtac tctcaatccg 660

aagcagaagc aagcattggc taaacaatta gggttacgag caagacaagt ggaagtttgg 720

tttcagaaca gacgagcaag aacaaagctg aagcaaacgg aggtagactg cgagttctta 780

cggagatgct gcgagaatct aacggaagag aaccgtcggc tacaaaaaga agtaacggaa 840

ttgagagtac ttaagctctc tcctcagttc tacatgcaca tgagcccacc cactactttg 900

accatgtgcc cttcatgtga acacgtgtcg gtcccgccac cacaacctca ggctgctacg 960

tcagcgcacc accggtcgtt gccggtcaat gcgtgggctc ctgctacgag gatatctcac 1020

ggcttgactt ttgacgctct tcgtcctagg tcctaagtct ttttacttgc aaccaaaggg 1080

cattttggtc gtttttttaag tttcatggac cagatatgca tgtagttgtt aacatgtatg 1140

tattttctta gaaagaaaga aaaacagatt aatatt 1176

<210> 230
<211> 284
<212> PRT
<213> Arabidopsis thaliana

<400> 230

Met Met Phe Glu Lys Asp Asp Leu Gly Leu Ser Leu Gly Leu Asn Phe
1 5 10 15

Pro Lys Lys Gln Ile Asn Leu Lys Ser Asn Pro Ser Val Ser Val Thr
20 25 30

Pro Ser Ser Ser Ser Phe Gly Leu Phe Arg Arg Ser Ser Trp Asn Glu
35 40 45

Ser Phe Thr Ser Ser Val Pro Asn Ser Asp Ser Ser Gln Lys Glu Thr
50 55 60

Arg Thr Phe Ile Arg Gly Ile Asp Val Asn Arg Pro Pro Ser Thr Ala
65 70 75 80

Glu Tyr Gly Asp Glu Asp Ala Gly Val Ser Ser Pro Asn Ser Thr Val
85 90 95

Ser Ser Ser Thr Gly Lys Arg Ser Glu Arg Glu Glu Asp Thr Asp Pro
100 105 110

Gln Gly Ser Arg Gly Ile Ser Asp Asp Glu Asp Gly Asp Asn Ser Arg
115 120 125

Lys Lys Leu Arg Leu Ser Lys Asp Gln Ser Ala Ile Leu Glu Glu Thr
130 135 140

Phe Lys Asp His Ser Thr Leu Asn Pro Lys Gln Lys Gln Ala Leu Ala
145 150 155 160

Lys Gln Leu Gly Leu Arg Ala Arg Gln Val Glu Val Trp Phe Gln Asn
165 170 175

Arg Arg Ala Arg Thr Lys Leu Lys Gln Thr Glu Val Asp Cys Glu Phe
180 185 190

Leu Arg Arg Cys Cys Glu Asn Leu Thr Glu Glu Asn Arg Arg Leu Gln
195 200 205

Lys Glu Val Thr Glu Leu Arg Ala Leu Lys Leu Ser Pro Gln Phe Tyr
210 215 220

Met His Met Ser Pro Pro Thr Thr Leu Thr Met Cys Pro Ser Cys Glu
225 230 235 240

His Val Ser Val Pro Pro Pro Gln Pro Gln Ala Ala Thr Ser Ala His
245 250 255

His Arg Ser Leu Pro Val Asn Ala Trp Ala Pro Ala Thr Arg Ile Ser
260 265 270

His Gly Leu Thr Phe Asp Ala Leu Arg Pro Arg Ser
275 280

<210> 231
<211> 3171
<212> DNA
<213> Arabidopsis thaliana

<400> 231
gacaatccga aattaggtaa ccatgttcat tgaatttatc gtttcaacac attcgaatca 60

ctgagtccta cgtttcagtt taattccctg atgcacattt tgaattcggt ttgcgtattg 120

gtcttcgagt tttccgtcgg cggccatgga cgcgttcaag gaggagatag aaatcgggag 180

ctcggtggag tctttaatgg agctattgga ttcgcagaag gtgctttttc atagccagat 240

cgatcagctc caagatgtcg tcgttgcgca atgcaaactt accggcgtta atccccttgc 300

gcaagaaatg gctgctggtg ctttgtccat taaaattgga aagcggccaa gagacttgtt 360

gaatcctaag gctgttaagt atctacaagc agttttcgca attaaagatg ctattagtaa 420

gagggaatct cgggagataa gtgctttatt tggcatcaca gtcgcccagg ttcgagaatt 480

ttttgttact caaaagacaa gagtgaggaa acaggtgagg ctttcaaggg agaaagtagt 540

tatgtccaat acgcatgctt tacaagatga tggtgttccg gaaaataaca atgccacaaa 600

tcatgttgaa cccgttccct tgaactctat acatccggag gcatgttcta taagctgggg 660

tgaaggtgaa acagtggcac ttattccacc tgaagatatt ccacctgaca tcagcgattc 720

agacaaatac tttgttgaga atatattttc tctgctgcgt aaagaggaaa cattttcagg 780

ccaggtgaaa ctaatggagt ggatcatgca gatacaagat gcttctgtgc tgatctggtt 840

tttatcaaaa ggaggggttt tgatacttac aacatggtta agtcaagctg ctagtgaaga 900

gcaaacaagt gtcttacttc ttatcctgaa ggttctttgt catttacctc tccacaaagc 960

atctcctgaa aatatgtctg ccatattaca aagcgttaat ggacttcgtt tctataggat 1020

atcagacata tcaaacaggg caaaaggttt gttatcaagg tggaccaagt tatttgcgaa 1080

aatccaagct atgaagaaac aaaatcgtaa cagttcgcaa attgattcgc agagtcaatt 1140

gcttctgaaa cagagtattg ctgaaatcat gggtgatagt agcaatcctg aagatattct 1200

tagtctctca aatggaaagt cagagaatgt caggaggatt gaatcgtcac agggtccaaa 1260

actgttgctt acttctgcag atgattccac caagaaacac atgcttggtt caaatccatc 1320

gtataacaaa gaacgcagga aagtacagat ggtggaacaa ccaggccaaa aagctgctgg 1380

aaagagtccg cagacagtaa gaataggaac ttcaggtcga agccgcccaa tgtctgctga 1440

tgatattcag aaagcaaaga tgcgtgccct ttatatgcag agcaagaaca gtaaaaagga 1500

tcctttacca agtgccattg gtgattcgaa aatcgttgct cctgagaagc ccttggctct 1560

tcattcagcc aaggattctc cacctattca gaacaatgaa gctaagactg aagacacacc 1620

tgtactctcg actgttcagc ccgtcaatgg attttcaact attcagcccg tcaatggacc 1680

ttcagctgtt cagcccgtca atggaccttt ggctgttcag cctgtcaatg gaccttcggc 1740

tcttcagccc gtcaatggac cttcggccgt aattgtcccg gtacaagctg atgaaattaa 1800

aaaaccttca acacctccta aaagcatttc tagtaaggtg ggagttatga tgaaaatgag 1860

ttcacaaact attctcaaga attgcaagag aaaacagatt gattggcatg taccaccagg 1920

aatggaactt gacgaactct ggagagtagc cgctggtggt aatagcaagg aggctgatgt 1980

tcagagaaac agaaaccggc gagaaagaga aacaacatat cagtctcttc aaactatacc 2040

gttgaaccct aaagaaccat gggataggga aatggactat gatgacagtt tgacccctga 2100

aattccatct caacagccac cagaagaaag tttaacggaa ccacaggatt cacttgatga 2160

acgaagaatt gctgctggtg ctgccacaac ctcttcatct ctaagcagtc ctgaacctga 2220

tctcgagtta ttagctgcgt tacttaagaa cccagatctt gtttatgcac taacttcggg 2280

aaaacccagt aatttagccg gccaagatat ggtaaaactg cttgatgtga ttaagactgg 2340

tgcaccaaac tcaagcagta gctcaaataa acaggttgaa gaaagggtcg aagtttccct 2400

tccatctccc actccatcaa ctaatcctgg aatgagtgga tggggacaag aagggattcg 2460

gaatccattt tcaaggcaaa accaagttgg tactgcagtt gctagatcgg gtacacagct 2520

tcgtgttggt tcaatgcaat ggcatcaaac aaatgaacaa tcaatcccac gacatgctcc 2580

atcagcatac agtaactcga tcacattggc tcacacagaa agagaacagc aacaatatat 2640

gcaaccaaaa cttcatcaca atttacattt tcaacaacaa caacaacaac caatctcaac 2700

aacctcgtat gcagttaggg aaccagtagg acaaatggga acaggtacat cgagttcatg 2760

gaggagtcag cagagtcaga acagttacta ctcacatcaa gaaaacgaga ttgcatcggc 2820

ttcacaagtt acttcatacc aagggaatag ccagtacatg agtagcaatc caggatatga 2880

atcatggagt cctgataata gcccaagtag gaaccagctt aacatgaggg gacaacaaca 2940

acaagcatca aggaaacatg attcttctac tcatccatat tggaaccaaa acaaaagatg 3000

gcgttaaacc atatataaga ctatggttct ttggtcctca atatttgctt gtcatggttg 3060

aattagcatt atactctgtt cagacacaga ttttcatttt taactcacaa cagattcatc 3120

agagataata aactaaaaga aagaatgaat ggataaagtt attactgtct c 3171

<210> 232
<211> 953
<212> PRT
<213> Arabidopsis thaliana

<400> 232

Met Asp Ala Phe Lys Glu Glu Ile Glu Ile Gly Ser Ser Val Glu Ser
1 5 10 15

Leu Met Glu Leu Leu Asp Ser Gln Lys Val Leu Phe His Ser Gln Ile
20 25 30

Asp Gln Leu Gln Asp Val Val Val Ala Gln Cys Lys Leu Thr Gly Val
35 40 45

Asn Pro Leu Ala Gln Glu Met Ala Ala Gly Ala Leu Ser Ile Lys Ile
50 55 60

Gly Lys Arg Pro Arg Asp Leu Leu Asn Pro Lys Ala Val Lys Tyr Leu
65 70 75 80

Gln Ala Val Phe Ala Ile Lys Asp Ala Ile Ser Lys Arg Glu Ser Arg
85 90 95

```
Glu Ile Ser Ala Leu Phe Gly Ile Thr Val Ala Gln Val Arg Glu Phe
            100                 105                 110

Phe Val Thr Gln Lys Thr Arg Val Arg Lys Gln Val Arg Leu Ser Arg
        115                 120                 125

Glu Lys Val Val Met Ser Asn Thr His Ala Leu Gln Asp Asp Gly Val
    130                 135                 140

Pro Glu Asn Asn Asn Ala Thr Asn His Val Glu Pro Val Pro Leu Asn
145                 150                 155                 160

Ser Ile His Pro Glu Ala Cys Ser Ile Ser Trp Gly Glu Gly Glu Thr
                165                 170                 175

Val Ala Leu Ile Pro Pro Glu Asp Ile Pro Pro Asp Ile Ser Asp Ser
            180                 185                 190

Asp Lys Tyr Phe Val Glu Asn Ile Phe Ser Leu Leu Arg Lys Glu Glu
        195                 200                 205

Thr Phe Ser Gly Gln Val Lys Leu Met Glu Trp Ile Met Gln Ile Gln
    210                 215                 220

Asp Ala Ser Val Leu Ile Trp Phe Leu Ser Lys Gly Gly Val Leu Ile
225                 230                 235                 240

Leu Thr Thr Trp Leu Ser Gln Ala Ala Ser Glu Glu Gln Thr Ser Val
                245                 250                 255

Leu Leu Leu Ile Leu Lys Val Leu Cys His Leu Pro Leu His Lys Ala
            260                 265                 270

Ser Pro Glu Asn Met Ser Ala Ile Leu Gln Ser Val Asn Gly Leu Arg
        275                 280                 285

Phe Tyr Arg Ile Ser Asp Ile Ser Asn Arg Ala Lys Gly Leu Leu Ser
    290                 295                 300

Arg Trp Thr Lys Leu Phe Ala Lys Ile Gln Ala Met Lys Lys Gln Asn
305                 310                 315                 320

Arg Asn Ser Ser Gln Ile Asp Ser Gln Ser Gln Leu Leu Leu Lys Gln
                325                 330                 335

Ser Ile Ala Glu Ile Met Gly Asp Ser Ser Asn Pro Glu Asp Ile Leu
            340                 345                 350
```

```
Ser Leu Ser Asn Gly Lys Ser Glu Asn Val Arg Arg Ile Glu Ser Ser
        355                 360                 365

Gln Gly Pro Lys Leu Leu Leu Thr Ser Ala Asp Asp Ser Thr Lys Lys
    370                 375                 380

His Met Leu Gly Ser Asn Pro Ser Tyr Asn Lys Glu Arg Arg Lys Val
385                 390                 395                 400

Gln Met Val Glu Gln Pro Gly Gln Lys Ala Ala Gly Lys Ser Pro Gln
                405                 410                 415

Thr Val Arg Ile Gly Thr Ser Gly Arg Ser Arg Pro Met Ser Ala Asp
            420                 425                 430

Asp Ile Gln Lys Ala Lys Met Arg Ala Leu Tyr Met Gln Ser Lys Asn
        435                 440                 445

Ser Lys Lys Asp Pro Leu Pro Ser Ala Ile Gly Asp Ser Lys Ile Val
    450                 455                 460

Ala Pro Glu Lys Pro Leu Ala Leu His Ser Ala Lys Asp Ser Pro Pro
465                 470                 475                 480

Ile Gln Asn Asn Glu Ala Lys Thr Glu Asp Thr Pro Val Leu Ser Thr
                485                 490                 495

Val Gln Pro Val Asn Gly Phe Ser Thr Ile Gln Pro Val Asn Gly Pro
            500                 505                 510

Ser Ala Val Gln Pro Val Asn Gly Pro Leu Ala Val Gln Pro Val Asn
        515                 520                 525

Gly Pro Ser Ala Leu Gln Pro Val Asn Gly Pro Ser Ala Val Ile Val
    530                 535                 540

Pro Val Gln Ala Asp Glu Ile Lys Lys Pro Ser Thr Pro Pro Lys Ser
545                 550                 555                 560

Ile Ser Ser Lys Val Gly Val Met Met Lys Met Ser Ser Gln Thr Ile
                565                 570                 575

Leu Lys Asn Cys Lys Arg Lys Gln Ile Asp Trp His Val Pro Pro Gly
            580                 585                 590

Met Glu Leu Asp Glu Leu Trp Arg Val Ala Ala Gly Gly Asn Ser Lys
        595                 600                 605

Glu Ala Asp Val Gln Arg Asn Arg Asn Arg Arg Glu Arg Glu Thr Thr
```

610 615 620

Tyr Gln Ser Leu Gln Thr Ile Pro Leu Asn Pro Lys Glu Pro Trp Asp
625 630 635 640

Arg Glu Met Asp Tyr Asp Asp Ser Leu Thr Pro Glu Ile Pro Ser Gln
645 650 655

Gln Pro Pro Glu Glu Ser Leu Thr Glu Pro Gln Asp Ser Leu Asp Glu
660 665 670

Arg Arg Ile Ala Ala Gly Ala Ala Thr Thr Ser Ser Ser Leu Ser Ser
675 680 685

Pro Glu Pro Asp Leu Glu Leu Leu Ala Ala Leu Leu Lys Asn Pro Asp
690 695 700

Leu Val Tyr Ala Leu Thr Ser Gly Lys Pro Ser Asn Leu Ala Gly Gln
705 710 715 720

Asp Met Val Lys Leu Leu Asp Val Ile Lys Thr Gly Ala Pro Asn Ser
725 730 735

Ser Ser Ser Ser Asn Lys Gln Val Glu Glu Arg Val Glu Val Ser Leu
740 745 750

Pro Ser Pro Thr Pro Ser Thr Asn Pro Gly Met Ser Gly Trp Gly Gln
755 760 765

Glu Gly Ile Arg Asn Pro Phe Ser Arg Gln Asn Gln Val Gly Thr Ala
770 775 780

Val Ala Arg Ser Gly Thr Gln Leu Arg Val Gly Ser Met Gln Trp His
785 790 795 800

Gln Thr Asn Glu Gln Ser Ile Pro Arg His Ala Pro Ser Ala Tyr Ser
805 810 815

Asn Ser Ile Thr Leu Ala His Thr Glu Arg Glu Gln Gln Gln Tyr Met
820 825 830

Gln Pro Lys Leu His His Asn Leu His Phe Gln Gln Gln Gln Gln Gln
835 840 845

Pro Ile Ser Thr Thr Ser Tyr Ala Val Arg Glu Pro Val Gly Gln Met
850 855 860

Gly Thr Gly Thr Ser Ser Ser Trp Arg Ser Gln Gln Ser Gln Asn Ser
865 870 875 880

Tyr Tyr Ser His Gln Glu Asn Glu Ile Ala Ser Ala Ser Gln Val Thr
885 890 895

Ser Tyr Gln Gly Asn Ser Gln Tyr Met Ser Ser Asn Pro Gly Tyr Glu
900 905 910

Ser Trp Ser Pro Asp Asn Ser Pro Ser Arg Asn Gln Leu Asn Met Arg
915 920 925

Gly Gln Gln Gln Gln Ala Ser Arg Lys His Asp Ser Ser Thr His Pro
930 935 940

Tyr Trp Asn Gln Asn Lys Arg Trp Arg
945 950

<210> 233
<211> 501
<212> DNA
<213> Oryza sativa

<400> 233
atggagcagc agcttcctct tcttgcacct gactctaagg ctgccacgtc gtccccttg 60

tgcctcacct tggacaaccc aacctccaca tccacctcgc cggcggtgcc gtcgtcggcg 120

ccgccgccgg cagccgcctt ggaaccttct agacaatctt tccatgagag ggaaacggat 180

gcaatcaaag ccaagatcat gtcgcacccc ctctacccgg ctctcctcag agccttcata 240

gattgccaga aggtcggagc tccgccggag gtcgtcggcc ggctttccgc cctcgccggc 300

gagctcgact cgcgtgcaga agacaggtac cttcaagggc agtcgtcaga cccggagctc 360

gacgagttta tggaaaccta cattgatatg ctggtgagct acaggcagga gctgacaaga 420

ccaattcaag aggccgacca gttcttcaga aacatggagg cacagatcga ctcgtttaca 480

ctagagatgt gcagtttctg a 501

<210> 234
<211> 166
<212> PRT
<213> Oryza sativa

<400> 234

Met Glu Gln Gln Leu Pro Leu Leu Ala Pro Asp Ser Lys Ala Ala Thr
1 5 10 15

Ser Ser Pro Leu Cys Leu Thr Leu Asp Asn Pro Thr Ser Thr Ser Thr
20 25 30

Ser Pro Ala Val Pro Ser Ser Ala Pro Pro Pro Ala Ala Ala Leu Glu
35 40 45

Pro Ser Arg Gln Ser Phe His Glu Arg Glu Thr Asp Ala Ile Lys Ala
50 55 60

Lys Ile Met Ser His Pro Leu Tyr Pro Ala Leu Leu Arg Ala Phe Ile
65 70 75 80

Asp Cys Gln Lys Val Gly Ala Pro Pro Glu Val Val Gly Arg Leu Ser
85 90 95

Ala Leu Ala Gly Glu Leu Asp Ser Arg Ala Glu Asp Arg Tyr Leu Gln
100 105 110

Gly Gln Ser Ser Asp Pro Glu Leu Asp Glu Phe Met Glu Thr Tyr Ile
115 120 125

Asp Met Leu Val Ser Tyr Arg Gln Glu Leu Thr Arg Pro Ile Gln Glu
130 135 140

Ala Asp Gln Phe Phe Arg Asn Met Glu Ala Gln Ile Asp Ser Phe Thr
145 150 155 160

Leu Glu Met Cys Ser Phe
165

<210> 235
<211> 879
<212> DNA
<213> Oryza sativa

<400> 235
atggctcagg aggacgtcgg tcacctgagc gacgccggcc tggcgctggg cctgtccctc 60

ggcgggggag gaggagggac gaccgacgcg gcggcggcgc accgtggcgg ctgccggcgg 120

ccgtcgccgt cgtcgcagtg cccgccgctg gagccgtcgc tgaccctgag cttgcccgac 180

gacgcggcgg ccggcgcggc cgcgaccgcg accgcgaccg cgtccggcgg gggcggccct 240

gcgcacagcg tgtcgtcgct gtccgtcggc gcggcggcgg cggcggccgt gaagagggag 300

cgcgcggagg aggccgacgg cgagagggtg tcgtcgacgg cggccgggcg tgacgacgac 360

gacgacggga gcacccgcaa gaagctccgg ctgaccaagg agcagtccgc gctcctggag 420

gaccgcttcc gggagcacag cacgctcaac ccgaagcaga aagtcgcttt agcgaagcaa 480

ctgaacctca ggccaaggca ggtggaggtc tggttccaaa acagaagagc aaggacaaag 540

ctgaagcaga cggaggtgga ctgcgagttc ctgaagcgct gctgcgagac gctcaccgag 600

gagaaccgtc ggctgcagcg cgagctgcag gagctccgcg cgctcaagtt cgccccgccg 660

ccgccgtcct cggcggccca ccagccgtcg ccggcgccac cggcgccgtt ctacatgcaa 720

ctcccggccg ccacgctcac catctgcccg tcctgcgagc gcgtcggcgg gcccgcgtcc 780

gccgccaagg tcgtcgccgc cgacgggacc aaggccggcc ccggccggac caccacccac 840

cacttcttca accccttcac ccactccgcc gcctgctga 879

<210> 236
<211> 292
<212> PRT
<213> Oryza sativa

<400> 236

Met Ala Gln Glu Asp Val Gly His Leu Ser Asp Ala Gly Leu Ala Leu
1               5                   10                  15

Gly Leu Ser Leu Gly Gly Gly Gly Gly Gly Thr Thr Asp Ala Ala Ala
            20                  25                  30

Ala His Arg Gly Gly Cys Arg Arg Pro Ser Pro Ser Ser Gln Cys Pro
        35                  40                  45

Pro Leu Glu Pro Ser Leu Thr Leu Ser Leu Pro Asp Asp Ala Ala Ala
    50                  55                  60

Gly Ala Ala Ala Thr Ala Thr Ala Thr Ala Ser Gly Gly Gly Gly Pro
65                  70                  75                  80

Ala His Ser Val Ser Ser Leu Ser Val Gly Ala Ala Ala Ala Ala Ala
                85                  90                  95

Val Lys Arg Glu Arg Ala Glu Glu Ala Asp Gly Glu Arg Val Ser Ser
            100                 105                 110

Thr Ala Ala Gly Arg Asp Asp Asp Asp Asp Gly Ser Thr Arg Lys Lys
        115                 120                 125

Leu Arg Leu Thr Lys Glu Gln Ser Ala Leu Leu Glu Asp Arg Phe Arg
    130                 135                 140

Glu His Ser Thr Leu Asn Pro Lys Gln Lys Val Ala Leu Ala Lys Gln
145                 150                 155                 160

Leu Asn Leu Arg Pro Arg Gln Val Glu Val Trp Phe Gln Asn Arg Arg
                165                 170                 175

Ala Arg Thr Lys Leu Lys Gln Thr Glu Val Asp Cys Glu Phe Leu Lys
            180                 185                 190

Arg Cys Cys Glu Thr Leu Thr Glu Glu Asn Arg Arg Leu Gln Arg Glu
        195                 200                 205

Leu Gln Glu Leu Arg Ala Leu Lys Phe Ala Pro Pro Pro Pro Ser Ser

210 215 220

Ala Ala His Gln Pro Ser Pro Ala Pro Pro Ala Pro Phe Tyr Met Gln
225 230 235 240

Leu Pro Ala Ala Thr Leu Thr Ile Cys Pro Ser Cys Glu Arg Val Gly
245 250 255

Gly Pro Ala Ser Ala Ala Lys Val Val Ala Ala Asp Gly Thr Lys Ala
260 265 270

Gly Pro Gly Arg Thr Thr Thr His His Phe Phe Asn Pro Phe Thr His
275 280 285

Ser Ala Ala Cys
290

<210> 237
<211> 744
<212> DNA
<213> Oryza sativa

<400> 237
atggcgcaag atgatgagga cgtgggtcta gctctgggcc tctccctggg ctccggcggc 60

cacaggcggc agagagaaag tagagacgaa gcgccgtcgt cggcggcggc gtccctgctg 120

acgctgaggc tcccggcaga gagcgggggg cagccgcagg tggtggtgaa gagggaggtg 180

gtgcgggcgg aagaggagga gtacgaatac gagtacgaga gggcgctcta ctcgtcgtcg 240

gctgcggcgg cggacgacga cgagggctgc aacagccgga agaagctgag gctgagcaag 300

gagcagtcgg ctctgctgga ggatcgcttc aaggagcata gcactctcaa ccctaagcaa 360

aaggttgctt tggcgaagca gctaaacctg aggccaaggc aagttgaggt gtggttccaa 420

aacagaagag ccaggacgaa gctgaagcag acggaggtgg attgcgagct tctgaagcgc 480

tgctgcgaga cgctgacgga ggagaaccga cgcctccatc gtgagctcca gcagctcagg 540

gctctgaccc actccaccgc cgccggcttc ttcatggcca ccaccctccc cgtccccgcc 600

gccacgctct ccatctgccc ctcctgcgag cgcctcgcca ccgccgccgc cgccggagct 660

tccccccaccg ccgccgccga ccgcaccaac aagcccaccg ccccgcactt gttcagccct 720

ttcgccaagt ccgccgcctg ctag 744

<210> 238
<211> 247
<212> PRT
<213> Oryza sativa

<400> 238

Met Ala Gln Asp Asp Glu Asp Val Gly Leu Ala Leu Gly Leu Ser Leu
1 5 10 15

Gly Ser Gly Gly His Arg Arg Gln Arg Glu Ser Arg Asp Glu Ala Pro
20 25 30

Ser Ser Ala Ala Ala Ser Leu Leu Thr Leu Arg Leu Pro Ala Glu Ser
35 40 45

Gly Gly Gln Pro Gln Val Val Val Lys Arg Glu Val Val Arg Ala Glu
50 55 60

Glu Glu Glu Tyr Glu Tyr Glu Tyr Glu Arg Ala Leu Tyr Ser Ser Ser
65 70 75 80

Ala Ala Ala Ala Asp Asp Asp Glu Gly Cys Asn Ser Arg Lys Lys Leu
85 90 95

Arg Leu Ser Lys Glu Gln Ser Ala Leu Leu Glu Asp Arg Phe Lys Glu
100 105 110

His Ser Thr Leu Asn Pro Lys Gln Lys Val Ala Leu Ala Lys Gln Leu
115 120 125

Asn Leu Arg Pro Arg Gln Val Glu Val Trp Phe Gln Asn Arg Arg Ala
130 135 140

Arg Thr Lys Leu Lys Gln Thr Glu Val Asp Cys Glu Leu Leu Lys Arg
145 150 155 160

Cys Cys Glu Thr Leu Thr Glu Glu Asn Arg Arg Leu His Arg Glu Leu
165 170 175

Gln Gln Leu Arg Ala Leu Thr His Ser Thr Ala Ala Gly Phe Phe Met
180 185 190

Ala Thr Thr Leu Pro Val Pro Ala Ala Thr Leu Ser Ile Cys Pro Ser
195 200 205

Cys Glu Arg Leu Ala Thr Ala Ala Ala Ala Gly Ala Ser Pro Thr Ala
210 215 220

Ala Ala Asp Arg Thr Asn Lys Pro Thr Ala Pro His Leu Phe Ser Pro
225 230 235 240

Phe Ala Lys Ser Ala Ala Cys
245

<210> 239
<211> 1065
<212> DNA

<213> Oryza sativa

<400> 239
```
atggagctgg ggctgagctt gggggatgcg gtgaccgtgg cggatggcgg gaggctggag      60
ctggttcttg ggctcggggt tggggttggg gctggggtga ggagaggaga ggaggaggag     120
agggggagga gggaggatgt ggtgggagct gggaggtggg cggcgatggc ggcggcctcg     180
ccggagccgt cggtgcggct cagcctcgtg tcgagcctcg ggctccactg gccttccgag     240
accgggcgtt cggaggcggc ggcgcgtggg ttcgacgtga accgggcgcc gtcggtggcg     300
gcgggcgcgc cggggatgga ggacgacgag gagggcccgg gcgccgcgcc ggcgttgtcg     360
tcgtcgccca acgacagcgg gggatccttc ccgctggacc tctccgggca aggcctccgt     420
ggccacgccg aggcggcggc gcagggtggc ggcggcggcg gcggcggcga gcggtcgtcc     480
tcgcgcgcga gcgacgacga cgagggcgcg tccgcgcgca agaagctgcg actctccaag     540
gagcagtcgg cgttcctcga ggagagcttc aaggagcaca gcacgctgaa tcccaagcag     600
aaggtggcgc tggcgaagca gctcaacctc cggccgcggc aagtggaggt ctggttccag     660
aaccgccgag ccaggacgaa gctgaagcag acggaggtgg actgcgagta cctgaagcgc     720
tgctgcgaga cgctcaccga ggagaaccgg cggctgcaca aggagctcgc cgagctgcgc     780
gcgctcaaga cggcgcgccc cttctacatg cacctcccgg ccacgaccct ctccatgtgc     840
ccctcctgcg agcgtgtcgc ctccaacccg gccaccgctt cgacctccgc ccccgccgcg     900
gccacgtccc cggcggcggc gccgaccgcg gccgcaagaa ccgccgtcgc gtcgcccgag     960
ccgcaccggc cgtcgtcctt cgccgcgctg ttcgcggcac ccctcggctt cccgctgacc    1020
gccgcccagc cgcggccgcc gccgccggcg agcaactgcc tgtaa                    1065
```

<210> 240
<211> 354
<212> PRT
<213> Oryza sativa

<400> 240

```
Met Glu Leu Gly Leu Ser Leu Gly Asp Ala Val Thr Val Ala Asp Gly
1               5                   10                  15

Gly Arg Leu Glu Leu Val Leu Gly Leu Gly Val Gly Val Gly Ala Gly
            20                  25                  30

Val Arg Arg Gly Glu Glu Glu Glu Arg Gly Arg Arg Glu Asp Val Val
        35                  40                  45

Gly Ala Gly Arg Trp Ala Ala Met Ala Ala Ala Ser Pro Glu Pro Ser
    50                  55                  60

Val Arg Leu Ser Leu Val Ser Ser Leu Gly Leu His Trp Pro Ser Glu
65                  70                  75                  80
```

```
Thr Gly Arg Ser Glu Ala Ala Ala Arg Gly Phe Asp Val Asn Arg Ala
                85                  90                  95

Pro Ser Val Ala Ala Gly Ala Pro Gly Met Glu Asp Asp Glu Glu Gly
            100                 105                 110

Pro Gly Ala Ala Pro Ala Leu Ser Ser Ser Pro Asn Asp Ser Gly Gly
        115                 120                 125

Ser Phe Pro Leu Asp Leu Ser Gly Gln Gly Leu Arg Gly His Ala Glu
    130                 135                 140

Ala Ala Ala Gln Gly Gly Gly Gly Gly Gly Gly Gly Glu Arg Ser Ser
145                 150                 155                 160

Ser Arg Ala Ser Asp Asp Asp Glu Gly Ala Ser Ala Arg Lys Lys Leu
                165                 170                 175

Arg Leu Ser Lys Glu Gln Ser Ala Phe Leu Glu Glu Ser Phe Lys Glu
            180                 185                 190

His Ser Thr Leu Asn Pro Lys Gln Lys Val Ala Leu Ala Lys Gln Leu
        195                 200                 205

Asn Leu Arg Pro Arg Gln Val Glu Val Trp Phe Gln Asn Arg Arg Ala
    210                 215                 220

Arg Thr Lys Leu Lys Gln Thr Glu Val Asp Cys Glu Tyr Leu Lys Arg
225                 230                 235                 240

Cys Cys Glu Thr Leu Thr Glu Glu Asn Arg Arg Leu His Lys Glu Leu
                245                 250                 255

Ala Glu Leu Arg Ala Leu Lys Thr Ala Arg Pro Phe Tyr Met His Leu
            260                 265                 270

Pro Ala Thr Thr Leu Ser Met Cys Pro Ser Cys Glu Arg Val Ala Ser
        275                 280                 285

Asn Pro Ala Thr Ala Ser Thr Ser Ala Pro Ala Ala Ala Thr Ser Pro
    290                 295                 300

Ala Ala Ala Pro Thr Ala Ala Ala Arg Thr Ala Val Ala Ser Pro Glu
305                 310                 315                 320

Pro His Arg Pro Ser Ser Phe Ala Ala Leu Phe Ala Ala Pro Leu Gly
                325                 330                 335
```

```
Phe Pro Leu Thr Ala Ala Gln Pro Arg Pro Pro Pro Pro Ala Ser Asn
            340                 345                 350

Cys Leu


<210>  241
<211>  1089
<212>  DNA
<213>  Oryza sativa

<400>  241
atggagctgg ggttgagctt gggggaggct atggcggatg ctgggaggga gctggttctt      60

gggcttggga tggggaggag ggaggaggcg gcggaggcgg ggaggaggga tcatgaggtg     120

aggagggagc tggagttcgg gtcgatgtcg agcaggtgcg gtggttcttc gccggagccg     180

acggtgcggc tcacgcttct gcccatggtg cccggccttg gcctcccatg gccgccgccg     240

ccgccgccgt cgtccgagag caggcatttg gaggcgtcga cgcggggatt cgacgtgaac     300

cgcccgccgt cgtccggcgg cggcggcggc ggcggcggcg cggaggagga gcaggacgac     360

gtggccgggg cggcactctc gtcgtccccc aacaacagcg ccggatcctt cccgatggat     420

gacttctccg ggcacggcct cggcggcaac gacgcggccc ctggcggtgg cggcggcgac     480

cgctcgtgct cccgcgccag cgacgaggac gacggcggct ccgcgcgcaa gaagctccgc     540

ctctccaagg agcagtccgc gttcctcgag gagagcttca aggagcacag caccctaaac     600

cccaagcaga agctggcgct ggcgaagcag ctcaacctcc ggccgcgcca ggtggaggtg     660

tggttccaga accgccgcgc caggacgaag ctgaagcaga cggaggtgga ctgcgagtac     720

ctgaagcggt gctgcgagac gctgacggag gagaaccggc ggctgcagaa ggagctcgcc     780

gagctccggg cgctcaagac ggtgcacccc ttctacatgc acctcccggc gacgacactc     840

tccatgtgcc cctcctgcga gcgcgtcgcc tccaactccg ccccggccac cgcctcctcc     900

gccgcaacat cgtcgacggc cgcgccgccc gcggcaccct catccggcgg cattgcggcc     960

acctcctcct ccgccgccgc cgccgcggcg ccggaccaca ggccgtcgtc gttcgccgcg    1020

ctgttctcgt cgccgcgtgg cttcccgcta tccgtagccc cgcaggcgca gccgccgacg    1080

agctcgtga                                                            1089


<210>  242
<211>  362
<212>  PRT
<213>  Oryza sativa

<400>  242

Met Glu Leu Gly Leu Ser Leu Gly Glu Ala Met Ala Asp Ala Gly Arg
1               5                   10                  15
```

Glu Leu Val Leu Gly Leu Gly Met Gly Arg Arg Glu Glu Ala Ala Glu
20 25 30

Ala Gly Arg Arg Asp His Glu Val Arg Arg Glu Leu Glu Phe Gly Ser
35 40 45

Met Ser Ser Arg Cys Gly Gly Ser Ser Pro Glu Pro Thr Val Arg Leu
50 55 60

Thr Leu Leu Pro Met Val Pro Gly Leu Gly Leu Pro Trp Pro Pro Pro
65 70 75 80

Pro Pro Pro Ser Ser Glu Ser Arg His Leu Glu Ala Ser Thr Arg Gly
85 90 95

Phe Asp Val Asn Arg Pro Pro Ser Ser Gly Gly Gly Gly Gly Gly Gly
100 105 110

Gly Ala Glu Glu Glu Gln Asp Asp Val Ala Gly Ala Ala Leu Ser Ser
115 120 125

Ser Pro Asn Asn Ser Ala Gly Ser Phe Pro Met Asp Asp Phe Ser Gly
130 135 140

His Gly Leu Gly Gly Asn Asp Ala Ala Pro Gly Gly Gly Gly Gly Asp
145 150 155 160

Arg Ser Cys Ser Arg Ala Ser Asp Glu Asp Asp Gly Gly Ser Ala Arg
165 170 175

Lys Lys Leu Arg Leu Ser Lys Glu Gln Ser Ala Phe Leu Glu Glu Ser
180 185 190

Phe Lys Glu His Ser Thr Leu Asn Pro Lys Gln Lys Leu Ala Leu Ala
195 200 205

Lys Gln Leu Asn Leu Arg Pro Arg Gln Val Glu Val Trp Phe Gln Asn
210 215 220

Arg Arg Ala Arg Thr Lys Leu Lys Gln Thr Glu Val Asp Cys Glu Tyr
225 230 235 240

Leu Lys Arg Cys Cys Glu Thr Leu Thr Glu Glu Asn Arg Arg Leu Gln
245 250 255

Lys Glu Leu Ala Glu Leu Arg Ala Leu Lys Thr Val His Pro Phe Tyr
260 265 270

Met His Leu Pro Ala Thr Thr Leu Ser Met Cys Pro Ser Cys Glu Arg

275 280 285

Val Ala Ser Asn Ser Ala Pro Ala Thr Ala Ser Ser Ala Ala Thr Ser
290 295 300

Ser Thr Ala Ala Pro Pro Ala Ala Pro Ser Ser Gly Gly Ile Ala Ala
305 310 315 320

Thr Ser Ser Ser Ala Ala Ala Ala Ala Ala Pro Asp His Arg Pro Ser
325 330 335

Ser Phe Ala Ala Leu Phe Ser Ser Pro Arg Gly Phe Pro Leu Ser Val
340 345 350

Ala Pro Gln Ala Gln Pro Pro Thr Ser Ser
355 360

<210> 243
<211> 771
<212> DNA
<213> Oryza sativa

<400> 243
atggagaggc aaggcttgga tcttggcctg agcctcgggc taggcttgac gacggcggcg 60

acatggccgg ctgctgggtt ctgtctgaac tccggcatgg cggagcagga agtgatcagg 120

cgtgatgatg tggttgcggc gacggcggcg gaggatgaga ggttcgcgtg ctcacccggc 180

agcccggtgt cgagcggcag cgggaagcga ggcagcggca gcggcagcgg cgacgaggtc 240

gacgacgccg gctgcgacgt cggcggcggc ggcgcgcgca agaagctgcg gttgtccaag 300

gaccaggccg ccgtcctcga ggagtgcttc aagacgcacc acaccctcac tccgaagcag 360

aaggtggcgc tggcgaagag cttgaacctg cggccgcggc aggtggaggt gtggttccag 420

aaccgccgcg cgaggacgaa gctgaagcag acggaggtgg actgcgagca cctcaagcgg 480

tggtgcgacc agctcgccga cgacaaccgc cgcctccaca aggagctcgc cgagctcagg 540

gcgctcaagg ccacgcccac accgcccgcc gccgcgccgc cattgaccac cctcacaatg 600

tgcctctcct gcaagcgcgt cgccaatgcc ggcgtgccct cgccggcggc ggcgatattc 660

cccggccacc cccagttctt gtgcggattc agagatcacg ccggagcagc gtcgtcgtcg 720

tacggcggcg catcatctgg actcgcgaag gcggtcaggg cggcgaggta g 771

<210> 244
<211> 256
<212> PRT
<213> Oryza sativa

<400> 244

Met Glu Arg Gln Gly Leu Asp Leu Gly Leu Ser Leu Gly Leu Gly Leu
1 5 10 15

```
Thr Thr Ala Ala Thr Trp Pro Ala Ala Gly Phe Cys Leu Asn Ser Gly
            20                  25                  30

Met Ala Glu Gln Glu Val Ile Arg Arg Asp Asp Val Val Ala Ala Thr
        35                  40                  45

Ala Ala Glu Asp Glu Arg Phe Ala Cys Ser Pro Gly Ser Pro Val Ser
    50                  55                  60

Ser Gly Ser Gly Lys Arg Gly Ser Gly Ser Gly Ser Gly Asp Glu Val
65                  70                  75                  80

Asp Asp Ala Gly Cys Asp Val Gly Gly Gly Gly Ala Arg Lys Lys Leu
                85                  90                  95

Arg Leu Ser Lys Asp Gln Ala Ala Val Leu Glu Glu Cys Phe Lys Thr
            100                 105                 110

His His Thr Leu Thr Pro Lys Gln Lys Val Ala Leu Ala Lys Ser Leu
        115                 120                 125

Asn Leu Arg Pro Arg Gln Val Glu Val Trp Phe Gln Asn Arg Arg Ala
    130                 135                 140

Arg Thr Lys Leu Lys Gln Thr Glu Val Asp Cys Glu His Leu Lys Arg
145                 150                 155                 160

Trp Cys Asp Gln Leu Ala Asp Asp Asn Arg Arg Leu His Lys Glu Leu
                165                 170                 175

Ala Glu Leu Arg Ala Leu Lys Ala Thr Pro Thr Pro Pro Ala Ala Ala
            180                 185                 190

Pro Pro Leu Thr Thr Leu Thr Met Cys Leu Ser Cys Lys Arg Val Ala
        195                 200                 205

Asn Ala Gly Val Pro Ser Pro Ala Ala Ala Ile Phe Pro Gly His Pro
    210                 215                 220

Gln Phe Leu Cys Gly Phe Arg Asp His Ala Gly Ala Ala Ser Ser Ser
225                 230                 235                 240

Tyr Gly Gly Ala Ser Ser Gly Leu Ala Lys Ala Val Arg Ala Ala Arg
                245                 250                 255
```

<210> 245
<211> 927
<212> DNA

<213> Oryza sativa

<400> 245
atgatggatc tcggcctcag cctcggcctc ggcctcgcct cgcagggcag cctcacctcc 60

tccaccacca ccacctcctc ccccggcgcc ggatcatcct ccccgtgggc cgccgcgctc 120

aactccatcg tcggcgacgt gaggcgggat caggccgcgg cgcatgctgc cgcggcggtg 180

ggggttgggg ttgggggcga ggagatgtac caggggaggg cgtccacgtc gccggacagc 240

gcggcggcgc tgtcgagcgc gagcgggaag agggagaggg agctggagcg gtcgggatcc 300

ggggttgacg acgacgacgg cgcggacggc gccggcgggc ggaagaagct caggctgtcc 360

aaggaccagg ccgccgtgct cgaggagtgc ttcaagacgc actccactct caaccccaag 420

cagaaggtgg cgctggcgaa caggctgggg ctgcggccgc ggcaggtgga ggtgtggttc 480

cagaaccgga gggcgaggac gaagctgaag cagacggagg tggactgcga gtacctgaag 540

cggtggtgcg agcgcctcgc cgacgagaac aagcgcctcg agaaggagct cgccgacctc 600

agggcgctca aggccgcgcc ctcgccggcg tccgcgtccg cgatgcagcc ctcctcctcc 660

gccgccgcca cgctcaccat gtgcccctcc tgccgccgcg tcgccaccgc cggcgcgccg 720

caccagccta accaccaaca atgccatccc aaatctaaca ccaccatctc ctcctcctcc 780

accgccgccg ccgccgtcgc cgtcgccggc ggcaacgtgc tgcccagcca ctgccagttc 840

ttcccggccg ccgccgccgc cgccgaccgg acaagccaga gcacgtggaa cgccgccgcg 900

ccgctcgtca ccagagagct cttctag 927

<210> 246
<211> 308
<212> PRT
<213> Oryza sativa

<400> 246

Met Met Asp Leu Gly Leu Ser Leu Gly Leu Gly Leu Ala Ser Gln Gly
1 5 10 15

Ser Leu Thr Ser Ser Thr Thr Thr Thr Ser Ser Pro Gly Ala Gly Ser
20 25 30

Ser Ser Pro Trp Ala Ala Ala Leu Asn Ser Ile Val Gly Asp Val Arg
35 40 45

Arg Asp Gln Ala Ala Ala His Ala Ala Ala Ala Val Gly Val Gly Val
50 55 60

Gly Gly Glu Glu Met Tyr Gln Gly Arg Ala Ser Thr Ser Pro Asp Ser
65 70 75 80

Ala Ala Ala Leu Ser Ser Ala Ser Gly Lys Arg Glu Arg Glu Leu Glu
85 90 95

```
Arg Ser Gly Ser Gly Val Asp Asp Asp Asp Gly Ala Asp Gly Ala Gly
            100                 105                 110

Gly Arg Lys Lys Leu Arg Leu Ser Lys Asp Gln Ala Ala Val Leu Glu
        115                 120                 125

Glu Cys Phe Lys Thr His Ser Thr Leu Asn Pro Lys Gln Lys Val Ala
    130                 135                 140

Leu Ala Asn Arg Leu Gly Leu Arg Pro Arg Gln Val Glu Val Trp Phe
145                 150                 155                 160

Gln Asn Arg Arg Ala Arg Thr Lys Leu Lys Gln Thr Glu Val Asp Cys
                165                 170                 175

Glu Tyr Leu Lys Arg Trp Cys Glu Arg Leu Ala Asp Glu Asn Lys Arg
            180                 185                 190

Leu Glu Lys Glu Leu Ala Asp Leu Arg Ala Leu Lys Ala Ala Pro Ser
        195                 200                 205

Pro Ala Ser Ala Ser Ala Met Gln Pro Ser Ser Ser Ala Ala Ala Thr
    210                 215                 220

Leu Thr Met Cys Pro Ser Cys Arg Arg Val Ala Thr Ala Gly Ala Pro
225                 230                 235                 240

His Gln Pro Asn His Gln Gln Cys His Pro Lys Ser Asn Thr Thr Ile
                245                 250                 255

Ser Ser Ser Ser Thr Ala Ala Ala Ala Val Ala Val Ala Gly Gly Asn
            260                 265                 270

Val Leu Pro Ser His Cys Gln Phe Phe Pro Ala Ala Ala Ala Ala Ala
        275                 280                 285

Asp Arg Thr Ser Gln Ser Thr Trp Asn Ala Ala Ala Pro Leu Val Thr
    290                 295                 300

Arg Glu Leu Phe
305
```

<210> 247
<211> 936
<212> DNA
<213> Oryza sativa

<400> 247
atggagatga tggttcatgg gaggagagac gagcagtatg gcgggctcag gctcgggctt 60

```
gggcttgggc tcagcctcgg cgtcgccggt ggtgcagccg acgacgagca gccgccgccg    120
cgccgtggtg ccgcccgcc gccgcagcag cagctgtgcg gctggaacgg cggcggtctc    180
ttctcctcgt cttcctccga tcatcggggg aggtcggcga tgatggcgtg ccacgacgtc    240
atcgagatgc cgttcctgcg ggggatcgac gtgaaccgtg cgccggcggc agagacgacc    300
acgacgacgg cgaggggggcc cagctgcagc gaggaagacg aggagcccgg cgcgtcctcc   360
cccaacagca cgctctccag cctcagcggc aagcgcggcg caccatctgc cgccaccgcc    420
gccgccgccg ccgccagcga cgacgaggac tccggcggcg gatcccgcaa gaagctccgc    480
ctctccaagg accaagccgc cgtcctcgag gacaccttca aagagcacaa caccctcaat    540
cccaagcaga aggcggcgct ggcgaggcag ctgaatctga agccgcggca ggtggaggtg    600
tggttccaga acaggagggc gaggacgaag ctgaagcaga cggaggtgga ctgcgagctg    660
ctcaagcgct gctgcgagac gctcaccgac gagaaccgcc gcctccaccg cgagctccag    720
gagctccgcg ccctcaagct cgccaccgcc gccgccgcgc cgcaccacct ctacggcgcc    780
cgcgtcccgc cgcccaccac cctcaccatg tgcccctcct gcgagcgcgt cgcctccgca    840
gccaccacca cccgcaacaa ctccggcgcc gcccccgcgc ggccggtgcc cacccgcccg    900
tggccgccgg cggcggcgca gaggtcgtcg gcgtag                              936


<210>  248
<211>  311
<212>  PRT
<213>  Oryza sativa

<400>  248

Met Glu Met Met Val His Gly Arg Arg Asp Glu Gln Tyr Gly Gly Leu
1               5                   10                  15

Arg Leu Gly Leu Gly Leu Gly Leu Ser Leu Gly Val Ala Gly Gly Ala
            20                  25                  30

Ala Asp Asp Glu Gln Pro Pro Pro Arg Arg Gly Ala Ala Pro Pro Pro
        35                  40                  45

Gln Gln Gln Leu Cys Gly Trp Asn Gly Gly Gly Leu Phe Ser Ser Ser
    50                  55                  60

Ser Ser Asp His Arg Gly Arg Ser Ala Met Met Ala Cys His Asp Val
65                  70                  75                  80

Ile Glu Met Pro Phe Leu Arg Gly Ile Asp Val Asn Arg Ala Pro Ala
                85                  90                  95

Ala Glu Thr Thr Thr Thr Thr Ala Arg Gly Pro Ser Cys Ser Glu Glu
            100                 105                 110
```

Asp Glu Glu Pro Gly Ala Ser Ser Pro Asn Ser Thr Leu Ser Ser Leu
115 120 125

Ser Gly Lys Arg Gly Ala Pro Ser Ala Ala Thr Ala Ala Ala Ala Ala
130 135 140

Ala Ser Asp Asp Glu Asp Ser Gly Gly Gly Ser Arg Lys Lys Leu Arg
145 150 155 160

Leu Ser Lys Asp Gln Ala Ala Val Leu Glu Asp Thr Phe Lys Glu His
165 170 175

Asn Thr Leu Asn Pro Lys Gln Lys Ala Ala Leu Ala Arg Gln Leu Asn
180 185 190

Leu Lys Pro Arg Gln Val Glu Val Trp Phe Gln Asn Arg Arg Ala Arg
195 200 205

Thr Lys Leu Lys Gln Thr Glu Val Asp Cys Glu Leu Leu Lys Arg Cys
210 215 220

Cys Glu Thr Leu Thr Asp Glu Asn Arg Arg Leu His Arg Glu Leu Gln
225 230 235 240

Glu Leu Arg Ala Leu Lys Leu Ala Thr Ala Ala Ala Ala Pro His His
245 250 255

Leu Tyr Gly Ala Arg Val Pro Pro Pro Thr Thr Leu Thr Met Cys Pro
260 265 270

Ser Cys Glu Arg Val Ala Ser Ala Ala Thr Thr Thr Arg Asn Asn Ser
275 280 285

Gly Ala Ala Pro Ala Arg Pro Val Pro Thr Arg Pro Trp Pro Pro Ala
290 295 300

Ala Ala Gln Arg Ser Ser Ala
305 310

<210> 249
<211> 1674
<212> DNA
<213> Arabidopsis thaliana

<400> 249
aaaatggaaa actcagacac tgattcagaa gttttctttt ggtttcagaa ccaaaaccaa 60

aaccatagcc ataagtttcc ttcttcttgt tttcctccga gctctcactc tgctttttat 120

ggatctagct caatgatcaa cacagagact gcaactatgg acgaagaaga cgtatgtgag 180

```
agctacatga tgcgtgagat aaccaagaaa cggaagctaa caccgatcca attgcgttta    240
ctagaggaga gtttcgaaga agagaaaaga ctcgaaccag acaggaaact ctggctagcc    300
gagaagctag ggctgcagcc aagccaagtc gctgtctggt tccaaaacag aagagctaga    360
tacaagacca aacagctcga acacgactgt gactctctta aagctagcta tgctaagctc    420
aagactgact gggacattct ctttgtacaa aaccaaaccc tcaagagcaa ggtacagttt    480
cttaatagat taacaagcca ctattttcaa gagtctgttc aaaattttga tgacacattc    540
aaacaggtcg atcttctcaa agagaagctg aaaatgcaag agaatcttga aactcagtct    600
attgagagga aaagacttgg ggaagaaggt agttcggtga aaagcgataa cacgcagtac    660
agtgaagaag aaggtttgga gaatcaatac agtttcccgg agcttgcagt tctcggattt    720
tattatgatc caaccttaac tgcttcaaat ctaagacaag aacctttgaa agtcacgtgt    780
gcggatcaga tgactcagat ccaaatatct gacgtcacag aacctgcgag ctccgcacat    840
aaaaaaattg aagtgacaca gcgaagttcg agtatgagcc gcaagagaga taaaccctac    900
acaaaccgtc acacaccggc gcgaatttcg aaacgccggc gaccatgggc gccttcgtca    960
tcggagcacg atgagattat cgacaaaccg atcaccaaac ctccgccgcc accggcgttg   1020
gtagttatgg gacttcccgc caactgttca gttctggagc taaaatctcg attcgagatc   1080
tacggttcaa tctcccgaat ccgaatccat aaagacggaa ttggctccgt ttcgtatcga   1140
accgccgagt cagcagaagc cgccattgct ggtagtcacg agccttcttt cggtatctcc   1200
atcgattcca aaaagttgga ggtggtttgg gcgacggatc cattggtgaa gtggaaggaa   1260
ggtgtgacgg cgggagaagg aaaggagaga acgtcgtcgt tttcgtcgaa gcttttacga   1320
cctgtgatgc ctttgagaaa acatgggaga agcagtaggt tagcgtcagc tattgttaat   1380
ccgagaagtg ataatactaa aggaattagt ggagatggag ggatatcatc tccggcgacg   1440
acaagtgaag ttaaacagag aaatatagta acctacgacg atatcgttta acaccattct   1500
actacaatta tagtaacttt gattctttta catattttgg caattttatg agtttagcta   1560
atcctgttgg taatctcagt tgtcgaatgt tgtgaaatga gttgagtttt gtttatttgt   1620
atgattgtaa cttataagaa agagaaggat tccaaaatgg gtagaagatt caaa         1674
```

<210> 250
<211> 495
<212> PRT
<213> Arabidopsis thaliana

<400> 250

```
Met Glu Asn Ser Asp Thr Asp Ser Glu Val Phe Phe Trp Phe Gln Asn
1               5                   10                  15

Gln Asn Gln Asn His Ser His Lys Phe Pro Ser Ser Cys Phe Pro Pro
            20                  25                  30
```

Ser Ser His Ser Ala Phe Tyr Gly Ser Ser Ser Met Ile Asn Thr Glu
35 40 45

Thr Ala Thr Met Asp Glu Glu Asp Val Cys Glu Ser Tyr Met Met Arg
50 55 60

Glu Ile Thr Lys Lys Arg Lys Leu Thr Pro Ile Gln Leu Arg Leu Leu
65 70 75 80

Glu Glu Ser Phe Glu Glu Glu Lys Arg Leu Glu Pro Asp Arg Lys Leu
85 90 95

Trp Leu Ala Glu Lys Leu Gly Leu Gln Pro Ser Gln Val Ala Val Trp
100 105 110

Phe Gln Asn Arg Arg Ala Arg Tyr Lys Thr Lys Gln Leu Glu His Asp
115 120 125

Cys Asp Ser Leu Lys Ala Ser Tyr Ala Lys Leu Lys Thr Asp Trp Asp
130 135 140

Ile Leu Phe Val Gln Asn Gln Thr Leu Lys Ser Lys Val Gln Phe Leu
145 150 155 160

Asn Arg Leu Thr Ser His Tyr Phe Gln Glu Ser Val Gln Asn Phe Asp
165 170 175

Asp Thr Phe Lys Gln Val Asp Leu Leu Lys Glu Lys Leu Lys Met Gln
180 185 190

Glu Asn Leu Glu Thr Gln Ser Ile Glu Arg Lys Arg Leu Gly Glu Glu
195 200 205

Gly Ser Ser Val Lys Ser Asp Asn Thr Gln Tyr Ser Glu Glu Glu Gly
210 215 220

Leu Glu Asn Gln Tyr Ser Phe Pro Glu Leu Ala Val Leu Gly Phe Tyr
225 230 235 240

Tyr Asp Pro Thr Leu Thr Ala Ser Asn Leu Arg Gln Glu Pro Leu Lys
245 250 255

Val Thr Cys Ala Asp Gln Met Thr Gln Ile Gln Ile Ser Asp Val Thr
260 265 270

Glu Pro Ala Ser Ser Ala His Lys Lys Ile Glu Val Thr Gln Arg Ser
275 280 285

```
Ser Ser Met Ser Arg Lys Arg Asp Lys Pro Tyr Thr Asn Arg His Thr
    290                 295                 300

Pro Ala Arg Ile Ser Lys Arg Arg Arg Pro Trp Ala Pro Ser Ser Ser
305                 310                 315                 320

Glu His Asp Glu Ile Ile Asp Lys Pro Ile Thr Lys Pro Pro Pro Pro
                325                 330                 335

Pro Ala Leu Val Val Met Gly Leu Pro Ala Asn Cys Ser Val Leu Glu
            340                 345                 350

Leu Lys Ser Arg Phe Glu Ile Tyr Gly Ser Ile Ser Arg Ile Arg Ile
        355                 360                 365

His Lys Asp Gly Ile Gly Ser Val Ser Tyr Arg Thr Ala Glu Ser Ala
    370                 375                 380

Glu Ala Ala Ile Ala Gly Ser His Glu Pro Ser Phe Gly Ile Ser Ile
385                 390                 395                 400

Asp Ser Lys Lys Leu Glu Val Val Trp Ala Thr Asp Pro Leu Val Lys
                405                 410                 415

Trp Lys Glu Gly Val Thr Ala Gly Glu Gly Lys Glu Arg Thr Ser Ser
            420                 425                 430

Phe Ser Ser Lys Leu Leu Arg Pro Val Met Pro Leu Arg Lys His Gly
        435                 440                 445

Arg Ser Ser Arg Leu Ala Ser Ala Ile Val Asn Pro Arg Ser Asp Asn
    450                 455                 460

Thr Lys Gly Ile Ser Gly Asp Gly Gly Ile Ser Ser Pro Ala Thr Thr
465                 470                 475                 480

Ser Glu Val Lys Gln Arg Asn Ile Val Thr Tyr Asp Asp Ile Val
                485                 490                 495

<210>  251
<211>  1428
<212>  DNA
<213>  Arabidopsis thaliana

<400>  251
aaggccacaa cagatcttct tgttcctctc tctcaatctc tctctctaaa actcttattt      60

tcttcgtaag agatggaact agcgttgtct ctaggcgaca atactaagaa acagttctct     120

tttatggaga agaactcgaa gattaataat ccctctgtct cgtcgacatc tacttccgag     180
```

aaggatcttg ggttctgcat ggctttagat gttgcttttg gtggtcacag atcgttgtca 240

tcctcttcgt ctccgtcggt agaggatgag aagaagaaac cggcgcccag agcaaaaaaa 300

tctgacgaat ttagggtttc gtcttctgta gatccaccat tacagcttca gcttcacttc 360

cctaattggc tccctgagaa cagtaaaggt cgacaaggag gaagaatgcc cttaggagca 420

gctacggttg tggaggagga agaggaggag gaggaagcgg tgcctagtat gtcagtatcg 480

ccgccggata gtgtaacgtc gtcgtttcaa ttggactttg ggattaaaag ttatggttat 540

gagagaagaa gcaataagag agatattgat gatgaagtgg agagatcagc ttcaagagcc 600

agcaacgaag acaacgatga cgagaatgga tccactagga agaaacttag actctccaaa 660

gaccaatctg cttttcttga agacagcttc aaagaacaca gtacccttaa tcctgttcgt 720

gtcccattct ttacagtttt tatttattta aaatttgtct ttcttgaatt tattctattt 780

ttttagtacc aaattttagc cttaatgctt tttttttct ttctaaaaca ttgcagaaac 840

agaagattgc attggcgaag cagttgaatc ttcgtcctcg tcaggttgaa gtctggtttc 900

aaaacagacg agccaggaca aagctgaagc aaacggaagt ggactgtgaa tacctaaaga 960

gatgctgtga gtcactaacc gaagaaaacc ggaggcttca aaaagaggtt aaagaattga 1020

gaaccttgaa gacttccaca ccccttttaca tgcaacttcc ggccactact ctcactatgt 1080

gcccttcttg tgaacgtgtt gccacttcag cagcacagcc ctccacgtca gctgcccaca 1140

acctctgttt gtccacgtca tcattgattc cggttaagcc tcggccggcc aaacaagttt 1200

catgaaagca cctgcgaaat acagtttgag caaacggtcg agctagagtg gttttaaaag 1260

ttgtcttctt gtgtatatat ttattttact tttcatattt tattagagac cgctattttg 1320

aaagacgaat agattgatta tccggttagt gttttgtttt tcttagatag gaccggataa 1380

aaaacagatg gagcaaaagg gttgacatgt tttattgaat gatagagg 1428

<210> 252
<211> 237
<212> PRT
<213> Arabidopsis thaliana

<400> 252

Met Glu Leu Ala Leu Ser Leu Gly Asp Asn Thr Lys Lys Gln Phe Ser
1 5 10 15

Phe Met Glu Lys Asn Ser Lys Ile Asn Asn Pro Ser Val Ser Ser Thr
20 25 30

Ser Thr Ser Glu Lys Asp Leu Gly Phe Cys Met Ala Leu Asp Val Ala
35 40 45

Phe Gly Gly His Arg Ser Leu Ser Ser Ser Ser Ser Pro Ser Val Glu
50 55 60

Asp Glu Lys Lys Lys Pro Ala Pro Arg Ala Lys Lys Ser Asp Glu Phe
65 70 75 80

Arg Val Ser Ser Ser Val Asp Pro Pro Leu Gln Leu Gln Leu His Phe
85 90 95

Pro Asn Trp Leu Pro Glu Asn Ser Lys Gly Arg Gln Gly Gly Arg Met
100 105 110

Pro Leu Gly Ala Ala Thr Val Val Glu Glu Glu Glu Glu Glu Glu Glu
115 120 125

Ala Val Pro Ser Met Ser Val Ser Pro Pro Asp Ser Val Thr Ser Ser
130 135 140

Phe Gln Leu Asp Phe Gly Ile Lys Ser Tyr Gly Tyr Glu Arg Arg Ser
145 150 155 160

Asn Lys Arg Asp Ile Asp Asp Glu Val Glu Arg Ser Ala Ser Arg Ala
165 170 175

Ser Asn Glu Asp Asn Asp Asp Glu Asn Gly Ser Thr Arg Lys Lys Leu
180 185 190

Arg Leu Ser Lys Asp Gln Ser Ala Phe Leu Glu Asp Ser Phe Lys Glu
195 200 205

His Ser Thr Leu Asn Pro Val Arg Val Pro Phe Phe Thr Val Phe Ile
210 215 220

Tyr Leu Lys Phe Val Phe Leu Glu Phe Ile Leu Phe Phe
225 230 235

<210> 253
<211> 1167
<212> DNA
<213> Arabidopsis thaliana

<400> 253
tgcccccagc tagtcacata ctcatgattg caaaatctct ctctctctct gcctctctat 60

atattaacct ttcttcttcc tttactttct catcttctat ctctcaaaag aaaagcagac 120

aactttattt gcaaaaacag agtttttttt tcttatcttg agaaagttca acagaagatg 180

atgttcgaga aagacgatct gggtctaagc ttaggcttga attttccaaa gaaacagatc 240

aatctcaaat caaatccatc tgtttctgtt actccttctt cttcttcttt tggattattc 300

agaagatctt catggaacga gagttttact tcttcagttc caaactcaga ttcgtcacaa 360

aaagaaacaa gaactttcat ccgaggaatc gacgtgaaca gaccaccgtc tacagcggaa 420

tacggcgacg aagacgctgg agtatcttca cctaacagta cagtctcaag ctctacaggg 480

aaaagaagcg agagagaaga agacacagat ccacaaggct caagaggaat cagtgacgat 540

gaagatggtg ataactccag gaaaaagctt agactttcca aagatcaatc tgctattctt 600

gaagagacct tcaaagatca cagtactctc aatccgaagc agaagcaagc attggctaaa 660

caattagggt tacgagcaag acaagtggaa gtttggtttc agaacagacg agcaagaaca 720

aagctgaagc aaacggaggt agactgcgag ttcttacgga gatgctgcga gaatctaacg 780

gaagagaacc gtcggctaca aaaagaagta acggaattga gagcacttaa gctctctcct 840

cagttctaca tgcacatgag cccacccact actttgacca tgtgcccttc atgtgaacac 900

gtgtcggtcc cgccaccaca acctcaggct gctacgtcag cgcaccaccg gtcgttgccg 960

gtcaatgcgt gggctcctgc gacgaggata tctcacggct tgacttttga cgctcttcgt 1020

cctaggtcct aagtcttttt acttgcaacc aaagggcatt ttggtcgttt tttaagtttc 1080

atggaccaga tatgcatgta gttgttaaca tgtatgtatt ttcttagaaa gaaagaaaaa 1140

cagattaata tttttctagc ttaaacc 1167

<210> 254
<211> 284
<212> PRT
<213> Arabidopsis thaliana

<400> 254

Met Met Phe Glu Lys Asp Asp Leu Gly Leu Ser Leu Gly Leu Asn Phe
1 5 10 15

Pro Lys Lys Gln Ile Asn Leu Lys Ser Asn Pro Ser Val Ser Val Thr
20 25 30

Pro Ser Ser Ser Ser Phe Gly Leu Phe Arg Arg Ser Ser Trp Asn Glu
35 40 45

Ser Phe Thr Ser Ser Val Pro Asn Ser Asp Ser Ser Gln Lys Glu Thr
50 55 60

Arg Thr Phe Ile Arg Gly Ile Asp Val Asn Arg Pro Pro Ser Thr Ala
65 70 75 80

Glu Tyr Gly Asp Glu Asp Ala Gly Val Ser Ser Pro Asn Ser Thr Val
85 90 95

Ser Ser Ser Thr Gly Lys Arg Ser Glu Arg Glu Glu Asp Thr Asp Pro
100 105 110

Gln Gly Ser Arg Gly Ile Ser Asp Asp Glu Asp Gly Asp Asn Ser Arg
115 120 125

Lys Lys Leu Arg Leu Ser Lys Asp Gln Ser Ala Ile Leu Glu Glu Thr
    130                 135                 140

Phe Lys Asp His Ser Thr Leu Asn Pro Lys Gln Lys Gln Ala Leu Ala
145                 150                 155                 160

Lys Gln Leu Gly Leu Arg Ala Arg Gln Val Glu Val Trp Phe Gln Asn
                165                 170                 175

Arg Arg Ala Arg Thr Lys Leu Lys Gln Thr Glu Val Asp Cys Glu Phe
            180                 185                 190

Leu Arg Arg Cys Cys Glu Asn Leu Thr Glu Glu Asn Arg Arg Leu Gln
        195                 200                 205

Lys Glu Val Thr Glu Leu Arg Ala Leu Lys Leu Ser Pro Gln Phe Tyr
    210                 215                 220

Met His Met Ser Pro Pro Thr Thr Leu Thr Met Cys Pro Ser Cys Glu
225                 230                 235                 240

His Val Ser Val Pro Pro Pro Gln Pro Gln Ala Ala Thr Ser Ala His
                245                 250                 255

His Arg Ser Leu Pro Val Asn Ala Trp Ala Pro Ala Thr Arg Ile Ser
            260                 265                 270

His Gly Leu Thr Phe Asp Ala Leu Arg Pro Arg Ser
        275                 280

<210> 255
<211> 1194
<212> DNA
<213> Arabidopsis thaliana

<400> 255
atttcacatc tctctctctc tataagaacc ctagaagaag gtttctcttg tcctccatac 60

acttagcaca actgataaat cttttgaggt aaaatcagct ttagatcaag gtttttctag 120

tcatctctac tcataaagat caaagctttt gctattctca ttttctacca agagacaata 180

tcatgatgat gggtaaagag gatttgggtt taagtcttag cttgggattt gcacaaaacc 240

atcctctcca gctaaatctt aaacccactt cttcaccaat gtccaatctc cagatgtttc 300

catggaacca aacccttgtt tcttcctcag atcaacaaaa gcaacagttt cttaggaaaa 360

tcgacgtgaa cagcttgcca acaacggtgg atttggaaga ggagacagga gtttcgtctc 420

caaacagtac gatctcgagc acagtgagtg gaaagaggag gagtactgaa agagaaggta 480

cctccggtgg tggttgcgga gatgaccttg acatcactct agatagatct tcctcacgtg 540

gaacctccga tgaagaggaa gattacggag gtgagacttg taggaagaag cttagactat 600

ccaaagatca atccgcagtt ctcgaagaca ctttcaaaga gcacaatact ctcaatccca 660

aacagaagct ggctttggct aagaagctag gtttaacagc aagacaagtg gaagtgtggt 720

tccaaaacag aagagcaagg acaaagttaa agcagaccga agtggattgc gagtatttga 780

aaagatgtgt tgagaaatta acggaagaga atcggcggct cgagaaagag gcagcggaac 840

taagagcatt aaagctttca ccgcggttgt atggtcagat gagtccaccg accacacttt 900

tgatgtgtcc atcgtgtgaa cgtgtggccg gaccatcctc atctaaccac aaccagcgat 960

ctgtctcatt gagtccatgg ctccaaatgg cccatgggtc aacctttgat gtgatgcgtc 1020

ctaggtctta actttaatgc tgcttctatg ggttgtgtgt gggtcattgt actttttaga 1080

ttattgactc tcagctaatg tatccttaaa agcctttttc tacttttaaa tttactttaa 1140

tctaattaaa ttagttattc ttgtcttctt gataacaaac aaatttataa taat 1194

<210> 256
<211> 282
<212> PRT
<213> Arabidopsis thaliana

<400> 256

Met Met Met Gly Lys Glu Asp Leu Gly Leu Ser Leu Ser Leu Gly Phe
1 5 10 15

Ala Gln Asn His Pro Leu Gln Leu Asn Leu Lys Pro Thr Ser Ser Pro
20 25 30

Met Ser Asn Leu Gln Met Phe Pro Trp Asn Gln Thr Leu Val Ser Ser
35 40 45

Ser Asp Gln Gln Lys Gln Gln Phe Leu Arg Lys Ile Asp Val Asn Ser
50 55 60

Leu Pro Thr Thr Val Asp Leu Glu Glu Glu Thr Gly Val Ser Ser Pro
65 70 75 80

Asn Ser Thr Ile Ser Ser Thr Val Ser Gly Lys Arg Arg Ser Thr Glu
85 90 95

Arg Glu Gly Thr Ser Gly Gly Gly Cys Gly Asp Asp Leu Asp Ile Thr
100 105 110

Leu Asp Arg Ser Ser Ser Arg Gly Thr Ser Asp Glu Glu Glu Asp Tyr
115 120 125

Gly Gly Glu Thr Cys Arg Lys Lys Leu Arg Leu Ser Lys Asp Gln Ser
130 135 140

Ala Val Leu Glu Asp Thr Phe Lys Glu His Asn Thr Leu Asn Pro Lys
145 150 155 160

Gln Lys Leu Ala Leu Ala Lys Lys Leu Gly Leu Thr Ala Arg Gln Val
165 170 175

Glu Val Trp Phe Gln Asn Arg Arg Ala Arg Thr Lys Leu Lys Gln Thr
180 185 190

Glu Val Asp Cys Glu Tyr Leu Lys Arg Cys Val Glu Lys Leu Thr Glu
195 200 205

Glu Asn Arg Arg Leu Glu Lys Glu Ala Ala Glu Leu Arg Ala Leu Lys
210 215 220

Leu Ser Pro Arg Leu Tyr Gly Gln Met Ser Pro Pro Thr Thr Leu Leu
225 230 235 240

Met Cys Pro Ser Cys Glu Arg Val Ala Gly Pro Ser Ser Ser Asn His
245 250 255

Asn Gln Arg Ser Val Ser Leu Ser Pro Trp Leu Gln Met Ala His Gly
260 265 270

Ser Thr Phe Asp Val Met Arg Pro Arg Ser
275 280

<210> 257
<211> 1284
<212> DNA
<213> Arabidopsis thaliana

<400> 257
aataatgatg gattgtaatt agccacctga caaaatctct catcattcaa agtactatat 60

taatcccctc tcttcttcat taccatctca catctctctc tatttctctt ccacaaagag 120

tcctaacttc gagttgaaac aaacaccatt tctcatctct atctcagaaa gaacaaacca 180

tttcgtgttc tttctttctc tattctcata aggaaatata attcctgaaa ctgttgagtt 240

cttgtgaaag gaaataaaaa acatgatgat gggcaaagaa gatctaggtt tgagcctaag 300

cttagggttt tcacaaaatc acaatcctct tcagatgaat ctgaatccta actcttcatt 360

atcaaacaat ctccagagac tcccatggaa ccaaacattc gatcctacat cagatcttcg 420

caagatagac gtgaacagtt ttccatcaac ggttaactgc gaggaagaca caggagtttc 480

gtcaccaaac agtacgatct caagcaccat tagcgggaag agaagtgaga gagaaggaat 540

ctccggaacc ggcgttggct ccggcgacga tcacgacgag atcactccgg atcgagggta 600

ctcacgtgga acctcagatg aagaagaaga cgggggcgaa acgtcgagga agaagctcag 660

gttatcaaaa gatcagtctg cttttctcga agagactttc aaagaacaca acactctcaa 720

tcccaaacag aagctagctt tggctaagaa gctgaacttg acggcaagac aagtggaagt 780

gtggttccaa aacagaagag ctagaaccaa gttaaagcaa acggaggtag attgcgaata 840

cttgaaacgg tgcgtagaga agctaacgga agagaaccgg agacttcaga aagaggctat 900

ggagcttcga actctcaagc tgtctccaca attctacggt cagatgactc caccaactac 960

actcatcatg tgtccttcgt gcgagcgtgt gggtggccca tcatcatcga accatcacca 1020

caatcacagg cccgtttcta tcaatccgtg ggttgcttgt gctggtcagg tggctcatgg 1080

gctgaatttt gaagccttgc gtccacgatc gtgatttttt attttagtgg tgggaaaagg 1140

gtgttttggt atttttcgtt atcgttatat agtctatctg tgtggggtca ttgtaatttt 1200

ggatgattgg ccttctcatg aactagtcct atgtatgatg caaccttaaa aagatttaaa 1260

ttagcaaaaa ttagttacaa actt 1284

<210> 258
<211> 283
<212> PRT
<213> Arabidopsis thaliana

<400> 258

Met Met Met Gly Lys Glu Asp Leu Gly Leu Ser Leu Ser Leu Gly Phe
1 5 10 15

Ser Gln Asn His Asn Pro Leu Gln Met Asn Leu Asn Pro Asn Ser Ser
20 25 30

Leu Ser Asn Asn Leu Gln Arg Leu Pro Trp Asn Gln Thr Phe Asp Pro
35 40 45

Thr Ser Asp Leu Arg Lys Ile Asp Val Asn Ser Phe Pro Ser Thr Val
50 55 60

Asn Cys Glu Glu Asp Thr Gly Val Ser Ser Pro Asn Ser Thr Ile Ser
65 70 75 80

Ser Thr Ile Ser Gly Lys Arg Ser Glu Arg Glu Gly Ile Ser Gly Thr
85 90 95

Gly Val Gly Ser Gly Asp Asp His Asp Glu Ile Thr Pro Asp Arg Gly
100 105 110

Tyr Ser Arg Gly Thr Ser Asp Glu Glu Glu Asp Gly Gly Glu Thr Ser
115 120 125

Arg Lys Lys Leu Arg Leu Ser Lys Asp Gln Ser Ala Phe Leu Glu Glu
130 135 140

Thr Phe Lys Glu His Asn Thr Leu Asn Pro Lys Gln Lys Leu Ala Leu
145 150 155 160

Ala Lys Lys Leu Asn Leu Thr Ala Arg Gln Val Glu Val Trp Phe Gln
165 170 175

Asn Arg Arg Ala Arg Thr Lys Leu Lys Gln Thr Glu Val Asp Cys Glu
180 185 190

Tyr Leu Lys Arg Cys Val Glu Lys Leu Thr Glu Glu Asn Arg Arg Leu
195 200 205

Gln Lys Glu Ala Met Glu Leu Arg Thr Leu Lys Leu Ser Pro Gln Phe
210 215 220

Tyr Gly Gln Met Thr Pro Pro Thr Thr Leu Ile Met Cys Pro Ser Cys
225 230 235 240

Glu Arg Val Gly Gly Pro Ser Ser Ser Asn His His His Asn His Arg
245 250 255

Pro Val Ser Ile Asn Pro Trp Val Ala Cys Ala Gly Gln Val Ala His
260 265 270

Gly Leu Asn Phe Glu Ala Leu Arg Pro Arg Ser
275 280

<210> 259
<211> 957
<212> DNA
<213> Arabidopsis thaliana

<400> 259
atgggggaaa gagatgatgg gttgggtttg agtctaagct tgggaaatag tcaacaaaaa 60

gaaccatctc tgaggttgaa tcttatgccg ttgacaactt cttcttcttc ttcttcgttt 120

caacacatgc acaatcagaa taacaatagc catccccaga agattcataa catctcttgg 180

actcatctgt ttcaatcttc tgggattaaa cgtacaactg cagagagaaa ctccgacgcc 240

gggtcatttc taagaggttt caacgtgaac agagctcagt cttcggtggc ggtagtggac 300

ttggaagaag aagccgccgt cgtctcgtct ccaaacagcg ccgtttcgag tctgagtgga 360

aataaaaggg atcttgcggt ggcgagagga ggagatgaaa acgaggcgga gagagcttct 420

tgctcacgcg gagggggaag cggtggtagc gacgatgaag acggcggaaa cggcgacgga 480

tcaaggaaga aactacggtt atcgaaggat caagctcttg ttctcgagga gacttttaaa 540

gaacatagca ctcttaatcc gaagcaaaag ctggctctag caaaacagtt gaatctaagg 600

gcaagacaag ttgaagtgtg gtttcagaac cgtagggcaa ggacgaagct gaaacaaacg 660

gaggttgatt gtgagtattt aaagagatgt tgcgataatc tgaccgagga gaatcgacgg 720

ctgcagaaag aagtgtcgga gctgagggcg ttgaagttgt ctccacatct ctacatgcac 780

atgactcctc ctactactct caccatgtgc ccttcttgcg aacgtgtctc ctcctctgcc 840

gccactgtga ccgctgctcc ttccactact actactccta cggtggtggg gcggccaagt 900

ccacagcgat taactccttg gactgctatt tctctccagc aaaaatcagg tcgctag 957

<210> 260
<211> 318
<212> PRT
<213> Arabidopsis thaliana

<400> 260

Met Gly Glu Arg Asp Asp Gly Leu Gly Leu Ser Leu Ser Leu Gly Asn
1 5 10 15

Ser Gln Gln Lys Glu Pro Ser Leu Arg Leu Asn Leu Met Pro Leu Thr
20 25 30

Thr Ser Ser Ser Ser Ser Ser Phe Gln His Met His Asn Gln Asn Asn
35 40 45

Asn Ser His Pro Gln Lys Ile His Asn Ile Ser Trp Thr His Leu Phe
50 55 60

Gln Ser Ser Gly Ile Lys Arg Thr Thr Ala Glu Arg Asn Ser Asp Ala
65 70 75 80

Gly Ser Phe Leu Arg Gly Phe Asn Val Asn Arg Ala Gln Ser Ser Val
85 90 95

Ala Val Val Asp Leu Glu Glu Glu Ala Ala Val Val Ser Ser Pro Asn
100 105 110

Ser Ala Val Ser Ser Leu Ser Gly Asn Lys Arg Asp Leu Ala Val Ala
115 120 125

Arg Gly Gly Asp Glu Asn Glu Ala Glu Arg Ala Ser Cys Ser Arg Gly
130 135 140

Gly Gly Ser Gly Gly Ser Asp Asp Glu Asp Gly Gly Asn Gly Asp Gly
145 150 155 160

Ser Arg Lys Lys Leu Arg Leu Ser Lys Asp Gln Ala Leu Val Leu Glu
165 170 175

Glu Thr Phe Lys Glu His Ser Thr Leu Asn Pro Lys Gln Lys Leu Ala
180 185 190

Leu Ala Lys Gln Leu Asn Leu Arg Ala Arg Gln Val Glu Val Trp Phe
195 200 205

Gln Asn Arg Arg Ala Arg Thr Lys Leu Lys Gln Thr Glu Val Asp Cys
210 215 220

Glu Tyr Leu Lys Arg Cys Cys Asp Asn Leu Thr Glu Glu Asn Arg Arg
225 230 235 240

Leu Gln Lys Glu Val Ser Glu Leu Arg Ala Leu Lys Leu Ser Pro His
245 250 255

Leu Tyr Met His Met Thr Pro Pro Thr Thr Leu Thr Met Cys Pro Ser
260 265 270

Cys Glu Arg Val Ser Ser Ser Ala Ala Thr Val Thr Ala Ala Pro Ser
275 280 285

Thr Thr Thr Thr Pro Thr Val Val Gly Arg Pro Ser Pro Gln Arg Leu
290 295 300

Thr Pro Trp Thr Ala Ile Ser Leu Gln Gln Lys Ser Gly Arg
305 310 315

<210> 261
<211> 1357
<212> DNA
<213> Arabidopsis thaliana

<400> 261
ccacaacccc atatctcttt gtctgaaaaa actcttttgc atatataaat aaatataagc 60

catatctttt gaaactgtac tgctgcacaa gtgtagaggc agtggcacaa catctttaag 120

aaagagagag agaaagagtc atttattcat ttcctcgctt aaaaatcttg agcacccaga 180

cagaattctt ctttcttctt tctggggttg agaaaaatga gtgaaagaga tgatggattg 240

gggctaagtt tgagcttgag tttaggtttt aatcaaaagg acccgtcttc gaggttaaat 300

ccaatgcctc tggcttctta tgcatcttca tcacacatgc agcatatgca gcagagcaat 360

tataaccatc ctcaaaagat tcagaacact tggattaaca tgtttcagtc atcagagaga 420

aactcggaca tgagatcgtt tctccgggga atagacgtga acagagctcc atcgacggtg 480

gtggttgacg tggaggatga aggcgccgga gtttcgtctc cgaacagcac cgtctcaagc 540

gtgatgagcg ggaagaagag cgagcgagag ctaatggctg cggcaggtgc agttggagga 600

ggtagagtag aagataatga gattgagaga gcttcttgct cgctcggcgg tggtagcgac 660

gatgaagacg gtagcgggaa cggagatgac agttcgagga agaaactccg attgtctaaa 720

gaacaagctt tggttcttga agaaactttt aaagaacata gtacactcaa tccgaagcaa 780

aagatggctt tggctaagca attgaatctg aggacgagac aagttgaagt gtggttccaa 840

aaccgaaggg caaggacgaa gctgaagcaa acggaagtag actgtgaata tcttaagaga 900

tgttgcgaga atctaacgga tgagaatcgg agattgcaaa aggaagtgag tgagcttagg 960

gctttaaagc tttctccaca cttatacatg cacatgaaac ctcccactac tctcacaatg 1020

tgtccttctt gcgagcgagt cgctgttacg tcatcttcgt catcggtggc tcctcctgtg 1080

atgaattcat cgtctccgat gggtccgatg agtccgtggg ctgccatgcc tctacggcaa 1140

cgacctgctg ctggttctca ttagagttta attagtctaa agataatagg tttggtgatt 1200

tgtttttatt atattgttga acggacttgg aagttctttt caagagttgg tcccatgctc 1260

ttctttcctt tgttttattc agtttctatt tatagctgaa ttctggataa tggaaaatct 1320

actaatatta ttgtccaatt attagtttgg ggaaaga 1357

<210> 262
<211> 315
<212> PRT
<213> Arabidopsis thaliana

<400> 262

Met Ser Glu Arg Asp Asp Gly Leu Gly Leu Ser Leu Ser Leu Ser Leu
1 5 10 15

Gly Phe Asn Gln Lys Asp Pro Ser Ser Arg Leu Asn Pro Met Pro Leu
20 25 30

Ala Ser Tyr Ala Ser Ser Ser His Met Gln His Met Gln Gln Ser Asn
35 40 45

Tyr Asn His Pro Gln Lys Ile Gln Asn Thr Trp Ile Asn Met Phe Gln
50 55 60

Ser Ser Glu Arg Asn Ser Asp Met Arg Ser Phe Leu Arg Gly Ile Asp
65 70 75 80

Val Asn Arg Ala Pro Ser Thr Val Val Val Asp Val Glu Asp Glu Gly
85 90 95

Ala Gly Val Ser Ser Pro Asn Ser Thr Val Ser Ser Val Met Ser Gly
100 105 110

Lys Lys Ser Glu Arg Glu Leu Met Ala Ala Ala Gly Ala Val Gly Gly
115 120 125

Gly Arg Val Glu Asp Asn Glu Ile Glu Arg Ala Ser Cys Ser Leu Gly
130 135 140

Gly Gly Ser Asp Asp Glu Asp Gly Ser Gly Asn Gly Asp Asp Ser Ser

145 150 155 160

Arg Lys Lys Leu Arg Leu Ser Lys Glu Gln Ala Leu Val Leu Glu Glu
165 170 175

Thr Phe Lys Glu His Ser Thr Leu Asn Pro Lys Gln Lys Met Ala Leu
180 185 190

Ala Lys Gln Leu Asn Leu Arg Thr Arg Gln Val Glu Val Trp Phe Gln
195 200 205

Asn Arg Arg Ala Arg Thr Lys Leu Lys Gln Thr Glu Val Asp Cys Glu
210 215 220

Tyr Leu Lys Arg Cys Cys Glu Asn Leu Thr Asp Glu Asn Arg Arg Leu
225 230 235 240

Gln Lys Glu Val Ser Glu Leu Arg Ala Leu Lys Leu Ser Pro His Leu
245 250 255

Tyr Met His Met Lys Pro Pro Thr Thr Leu Thr Met Cys Pro Ser Cys
260 265 270

Glu Arg Val Ala Val Thr Ser Ser Ser Ser Ser Val Ala Pro Pro Val
275 280 285

Met Asn Ser Ser Ser Pro Met Gly Pro Met Ser Pro Trp Ala Ala Met
290 295 300

Pro Leu Arg Gln Arg Pro Ala Ala Gly Ser His
305 310 315

<210> 263
<211> 744
<212> DNA
<213> Oryza sativa

<400> 263
atgatggaga gggccgagga cctgcgcctg agcctcagtc tcagctcgcc gcttattgct 60

cctcgtactc accatgtcgc catgctgttc cacgctcctc cagagaaaag attcctggag 120

atgccgctgc tccctgctgc gaagcggagc gaggtcgtcg cggcagaaga ggagcgcgcg 180

ggcctgcgcg gcggcggcgg cagcgacgag gaggacggtg gctgcggcat cgacggctca 240

cgcaagaagc tccggctttc caaggaccag tccgccgtgc tcgaggacag cttccgggag 300

caccccaccc tcaaccccag gcagaaggca actttggcgc agcagctcgg gcttcggcct 360

cggcaggtcg aggtgtggtt tcagaacaga cgcgcaagga cgaagctgaa gcagacggag 420

gtggactgcg agttcctgaa gcgctgctgc gagacgctca cggaggagaa ccggaggctg 480

cagaaggagg tgcaggagct gcgagcgctc aagctcgtct cgccgcacct ctacatgaac 540

atgtccccgc ccaccacgct caccatgtgc ccctcctgcg agcgcgtctc caacaccaat 600

aacaactcca gcgccgccgc cgccgccgac cgccgcggca tcaggactac tactgccgca 660

ggcggcggca gcgtcgtcga caccgccgcc gacgggggca tcctctgcca ccgccccgatc 720

gccgtccggc cgcagcagtc atga 744

<210> 264
<211> 247
<212> PRT
<213> Oryza sativa

<400> 264

```
Met Met Glu Arg Ala Glu Asp Leu Arg Leu Ser Leu Ser Leu Ser Ser
1               5                   10                  15

Pro Leu Ile Ala Pro Arg Thr His His Val Ala Met Leu Phe His Ala
            20                  25                  30

Pro Pro Glu Lys Arg Phe Leu Glu Met Pro Leu Leu Pro Ala Ala Lys
        35                  40                  45

Arg Ser Glu Val Val Ala Ala Glu Glu Glu Arg Ala Gly Leu Arg Gly
    50                  55                  60

Gly Gly Gly Ser Asp Glu Glu Asp Gly Gly Cys Gly Ile Asp Gly Ser
65                  70                  75                  80

Arg Lys Lys Leu Arg Leu Ser Lys Asp Gln Ser Ala Val Leu Glu Asp
                85                  90                  95

Ser Phe Arg Glu His Pro Thr Leu Asn Pro Arg Gln Lys Ala Thr Leu
            100                 105                 110

Ala Gln Gln Leu Gly Leu Arg Pro Arg Gln Val Glu Val Trp Phe Gln
        115                 120                 125

Asn Arg Arg Ala Arg Thr Lys Leu Lys Gln Thr Glu Val Asp Cys Glu
    130                 135                 140

Phe Leu Lys Arg Cys Cys Glu Thr Leu Thr Glu Glu Asn Arg Arg Leu
145                 150                 155                 160

Gln Lys Glu Val Gln Glu Leu Arg Ala Leu Lys Leu Val Ser Pro His
                165                 170                 175

Leu Tyr Met Asn Met Ser Pro Pro Thr Thr Leu Thr Met Cys Pro Ser
            180                 185                 190
```

Cys Glu Arg Val Ser Asn Thr Asn Asn Asn Ser Ser Ala Ala Ala Ala
195 200 205

Ala Asp Arg Arg Gly Ile Arg Thr Thr Thr Ala Ala Gly Gly Gly Ser
210 215 220

Val Val Asp Thr Ala Ala Asp Gly Gly Ile Leu Cys His Arg Pro Ile
225 230 235 240

Ala Val Arg Pro Gln Gln Ser
245

<210> 265
<211> 846
<212> DNA
<213> Oryza sativa

<400> 265
atgaggtcgt acatggacgg cggcggcgcg gcggcgtacg aggaggagga ggaggaggtt 60

gaggacgacg acggcggcgg cggcggcggc ggcggcggcg gcggtggggg gctcggggag 120

aagaagcggc ggctggcggc ggagcaggtg cgggcgctgg agcggagctt cgaggcggac 180

aacaagctgg acccggagcg gaaggcccgg atcgcccgcg accttcgcct ccaccctcgc 240

caggtcgccg tctggttcca gaaccgccgc gcgaggtgga agaccaagca gatcgagcgc 300

gacttcgccg ccctccgctc ccgccacgac gccctccgcc tcgagtgcga cgccctccgc 360

cgcgacaagg acgccctcgc cgccgagatc gccgacctcc gggacagggt ggacggccag 420

atgtccgtca agctggaggc cgtggccgcg gacgaacacc agccgcctcc gccgccgccg 480

ccgccgccac tggcgtataa cagcaaggtg gtggacggct cgacggacag cgactcgagc 540

gcggtgttca acgaggaggc gtcgccgtac tccggcgcgg ccatcgacca ccaccaccac 600

caaactccgg cgagctacga cacggcgggg ttcacctcct tcttcgcgcc atccaccacg 660

ctcacctcgt ccctctcctt cccttccatg ttccacgcgt catcgcattt cgatggccac 720

caagaactcc tcgtcggcgg cggcggcgcc ggcgcagtgg ccgacgccga cctcggaggc 780

gccggattct tcgccggcga cgagcacgcc ggcggcctct cctggtacgg cgccgagggt 840

tggtag 846

<210> 266
<211> 281
<212> PRT
<213> Oryza sativa

<400> 266

Met Arg Ser Tyr Met Asp Gly Gly Gly Ala Ala Ala Tyr Glu Glu Glu
1 5 10 15

```
Glu Glu Glu Val Glu Asp Asp Asp Gly Gly Gly Gly Gly Gly Gly Gly
            20                  25                  30

Gly Gly Gly Gly Gly Leu Gly Glu Lys Lys Arg Arg Leu Ala Ala Glu
        35                  40                  45

Gln Val Arg Ala Leu Glu Arg Ser Phe Glu Ala Asp Asn Lys Leu Asp
    50                  55                  60

Pro Glu Arg Lys Ala Arg Ile Ala Arg Asp Leu Arg Leu His Pro Arg
65                  70                  75                  80

Gln Val Ala Val Trp Phe Gln Asn Arg Arg Ala Arg Trp Lys Thr Lys
                85                  90                  95

Gln Ile Glu Arg Asp Phe Ala Ala Leu Arg Ser Arg His Asp Ala Leu
            100                 105                 110

Arg Leu Glu Cys Asp Ala Leu Arg Arg Asp Lys Asp Ala Leu Ala Ala
        115                 120                 125

Glu Ile Ala Asp Leu Arg Asp Arg Val Asp Gly Gln Met Ser Val Lys
    130                 135                 140

Leu Glu Ala Val Ala Ala Asp Glu His Gln Pro Pro Pro Pro Pro Pro
145                 150                 155                 160

Pro Pro Pro Leu Ala Tyr Asn Ser Lys Val Val Asp Gly Ser Thr Asp
                165                 170                 175

Ser Asp Ser Ser Ala Val Phe Asn Glu Glu Ala Ser Pro Tyr Ser Gly
            180                 185                 190

Ala Ala Ile Asp His His His His Gln Thr Pro Ala Ser Tyr Asp Thr
        195                 200                 205

Ala Gly Phe Thr Ser Phe Phe Ala Pro Ser Thr Thr Leu Thr Ser Ser
    210                 215                 220

Leu Ser Phe Pro Ser Met Phe His Ala Ser Ser His Phe Asp Gly His
225                 230                 235                 240

Gln Glu Leu Leu Val Gly Gly Gly Gly Ala Gly Ala Val Ala Asp Ala
                245                 250                 255

Asp Leu Gly Gly Ala Gly Phe Phe Ala Gly Asp Glu His Ala Gly Gly
            260                 265                 270

Leu Ser Trp Tyr Gly Ala Glu Gly Trp
```

275 280

<210> 267
<211> 918
<212> DNA
<213> Oryza sativa

<400> 267
atgaagaggc ccagctgcag aggctcctcc atggccatca tccatgacac ctctgatcaa 60

caagaggaca acatgaggtc gtacatggac ggcggcggcg cggcggcgta cgaggaggag 120

gaggaggagg ttgaggacga cgacggcggc ggcggcggcg gcggcggcgg cggcggtggg 180

gggctcgggg agaagaagcg gcggctggcg gcggagcagg tgcgggcgct ggagcggagc 240

ttcgaggcgg acaacaagct ggacccggag cggaaggccc ggatcgcccg cgaccttcgc 300

ctccaccctc gccaggtcgc cgtctggttc cagaaccgcc gcgcgaggtg gaagaccaag 360

cagatcgagc gcgacttcgc cgccctccgc tcccgccacg acgccctccg cctcgagtgc 420

gacgccctcc gccgcgacaa ggacgccctc gccgccgaga tcgccgacct ccgggacagg 480

gtggacggcc agatgtccgt caagctggag gccgtggccg cggacgaaca ccagccgcct 540

ccgccgccgc cgccgccgcc actggcgtat aacagcaagg tggtggacgg ctcgacggac 600

agcgactcga gcgcggtgtt caacgaggag gcgtcgccgt actccggcgc ggccatcgac 660

caccaccacc accaaactcc ggcgagctac gacacggcgg ggttcacctc cttcttcgcg 720

ccatccacca cgctcacctc gtccctctcc ttcccttcca tgttccacgc gtcatcgcat 780

ttcgatggcc accaagaact cctcgtcggc ggcggcggcg ccggcgcagt ggccgacgcc 840

gacctcggag gcgccggatt cttcgccggc gacgagcacg ccggcggcct ctcctggtac 900

ggcgccgagg gttggtag 918

<210> 268
<211> 305
<212> PRT
<213> Oryza sativa

<400> 268

Met Lys Arg Pro Ser Cys Arg Gly Ser Ser Met Ala Ile Ile His Asp
1 5 10 15

Thr Ser Asp Gln Gln Glu Asp Asn Met Arg Ser Tyr Met Asp Gly Gly
20 25 30

Gly Ala Ala Ala Tyr Glu Glu Glu Glu Glu Glu Val Glu Asp Asp Asp
35 40 45

Gly Gly Gly Gly Gly Gly Gly Gly Gly Gly Gly Gly Gly Leu Gly Glu
50 55 60

```
Lys Lys Arg Arg Leu Ala Ala Glu Gln Val Arg Ala Leu Glu Arg Ser
65                  70                  75                  80

Phe Glu Ala Asp Asn Lys Leu Asp Pro Glu Arg Lys Ala Arg Ile Ala
                85                  90                  95

Arg Asp Leu Arg Leu His Pro Arg Gln Val Ala Val Trp Phe Gln Asn
            100                 105                 110

Arg Arg Ala Arg Trp Lys Thr Lys Gln Ile Glu Arg Asp Phe Ala Ala
        115                 120                 125

Leu Arg Ser Arg His Asp Ala Leu Arg Leu Glu Cys Asp Ala Leu Arg
    130                 135                 140

Arg Asp Lys Asp Ala Leu Ala Ala Glu Ile Ala Asp Leu Arg Asp Arg
145                 150                 155                 160

Val Asp Gly Gln Met Ser Val Lys Leu Glu Ala Val Ala Ala Asp Glu
                165                 170                 175

His Gln Pro Pro Pro Pro Pro Pro Pro Pro Pro Leu Ala Tyr Asn Ser
            180                 185                 190

Lys Val Val Asp Gly Ser Thr Asp Ser Asp Ser Ser Ala Val Phe Asn
        195                 200                 205

Glu Glu Ala Ser Pro Tyr Ser Gly Ala Ala Ile Asp His His His His
    210                 215                 220

Gln Thr Pro Ala Ser Tyr Asp Thr Ala Gly Phe Thr Ser Phe Phe Ala
225                 230                 235                 240

Pro Ser Thr Thr Leu Thr Ser Ser Leu Ser Phe Pro Ser Met Phe His
                245                 250                 255

Ala Ser Ser His Phe Asp Gly His Gln Glu Leu Leu Val Gly Gly Gly
            260                 265                 270

Gly Ala Gly Ala Val Ala Asp Ala Asp Leu Gly Gly Ala Gly Phe Phe
        275                 280                 285

Ala Gly Asp Glu His Ala Gly Gly Leu Ser Trp Tyr Gly Ala Glu Gly
    290                 295                 300

Trp
305

<210>  269
```

<211> 936
<212> DNA
<213> Oryza sativa

<400> 269
atgaagaggc ccaccagcag cagccgaaaa tccaaaaaac aaggagagga cctggcgttc 60

tctgaggagg gcagcttgcc cgcggtgacc atggagcaga aagatgaagc cgagatggag 120

gaggtggacg aggaggagga ggaggaggtc gacgaagaca tggccggcgg gcacgcggcg 180

cagtcgccgt cgccgtcgtg cgggctgggc gagaagaagc ggcggctggc gctggagcag 240

gtgcgcgctc tggagcggag cttcgacacg gacaacaagc tggacccgga ccgcaaggcc 300

cgcatcgcgc gcgacctcgg cctgcagccg cgccaggtcg ccgtctggtt ccagaaccgc 360

cgcgcccggt ggaagacgaa gcagctcgag cgcgacttcg ccgccctccg cgcccgccac 420

gacgccctcc gcgccgactg cgacgccctg cgccgcgaca aggacgccct cgccgccgag 480

attcgggagc tgagggagaa gctgcccacc aagccggcgg acacggcggc atcggtgaag 540

gtagaagccg gcaatgacgc ggcggccggc gccgcggccg ccacggtgtg caaggacggc 600

tcgtcggacg acagcgactc cagcgtggtg ttcaacgacg aggcgtcgcc gtactccggc 660

gcggccttca ttggattcgg cccgtcgttc ttggtcgacg acgcgtcggc ggcaaccgtg 720

ggctgctcgt cgtcgctccc cgcgctcgag tccaaatggc acggtccgta ctccgacgac 780

tcgtgcaaag gcggcgtcta tggcttcacg gaggaatggc tcgctgcctg ctccggcgag 840

atggccggca acgacgccgc cggcttcttc tccgacgagc acgcctccaa cctcaacttc 900

ggttggtgcg cgagtggtaa cgagggttgg gaatga 936

<210> 270
<211> 311
<212> PRT
<213> Oryza sativa

<400> 270

Met Lys Arg Pro Thr Ser Ser Ser Arg Lys Ser Lys Lys Gln Gly Glu
1               5                   10                  15

Asp Leu Ala Phe Ser Glu Glu Gly Ser Leu Pro Ala Val Thr Met Glu
            20                  25                  30

Gln Lys Asp Glu Ala Glu Met Glu Glu Val Asp Glu Glu Glu Glu Glu
        35                  40                  45

Glu Val Asp Glu Asp Met Ala Gly Gly His Ala Ala Gln Ser Pro Ser
    50                  55                  60

Pro Ser Cys Gly Leu Gly Glu Lys Lys Arg Arg Leu Ala Leu Glu Gln
65                  70                  75                  80

```
Val Arg Ala Leu Glu Arg Ser Phe Asp Thr Asp Asn Lys Leu Asp Pro
                85                  90                  95

Asp Arg Lys Ala Arg Ile Ala Arg Asp Leu Gly Leu Gln Pro Arg Gln
            100                 105                 110

Val Ala Val Trp Phe Gln Asn Arg Arg Ala Arg Trp Lys Thr Lys Gln
        115                 120                 125

Leu Glu Arg Asp Phe Ala Ala Leu Arg Ala Arg His Asp Ala Leu Arg
    130                 135                 140

Ala Asp Cys Asp Ala Leu Arg Arg Asp Lys Asp Ala Leu Ala Ala Glu
145                 150                 155                 160

Ile Arg Glu Leu Arg Glu Lys Leu Pro Thr Lys Pro Ala Asp Thr Ala
                165                 170                 175

Ala Ser Val Lys Val Glu Ala Gly Asn Asp Ala Ala Ala Gly Ala Ala
            180                 185                 190

Ala Ala Thr Val Cys Lys Asp Gly Ser Ser Asp Asp Ser Asp Ser Ser
        195                 200                 205

Val Val Phe Asn Asp Glu Ala Ser Pro Tyr Ser Gly Ala Ala Phe Ile
    210                 215                 220

Gly Phe Gly Pro Ser Phe Leu Val Asp Asp Ala Ser Ala Ala Thr Val
225                 230                 235                 240

Gly Cys Ser Ser Ser Leu Pro Ala Leu Glu Ser Lys Trp His Gly Pro
                245                 250                 255

Tyr Ser Asp Asp Ser Cys Lys Gly Gly Val Tyr Gly Phe Thr Glu Glu
            260                 265                 270

Trp Leu Ala Ala Cys Ser Gly Glu Met Ala Gly Asn Asp Ala Ala Gly
        275                 280                 285

Phe Phe Ser Asp Glu His Ala Ser Asn Leu Asn Phe Gly Trp Cys Ala
    290                 295                 300

Ser Gly Asn Glu Gly Trp Glu
305                 310
```

<210> 271
<211> 810
<212> DNA
<213> Oryza sativa

<400> 271
atgagaagtc cagcagcgct tctcccggtg gttgcagatg gtggtggtgg tgttggggtg 60

gaggaggaga tggatgtgga cgaggacatg gcgatgtgcg gcggccgcgg cggcggcggc 120

ggggagaaga agcggcggct gagcgtggag caggtgcgcg cgctggagcg gagcttcgag 180

acggagaaca agctggagcc ggagcggaag gcgcggctgg cgcgcgacct cgggctgcag 240

ccgcgccagg tcgccgtctg gttccagaac cgccgcgcgc ggtggaagac caagcagctc 300

gagcgcgact acgccgcgct ccgccaatcc tacgacgcgc tccgcgccga ccacgacgcg 360

cttcgccgcg acaaggacgc cctcctcgcc gagatcaagg agctgaaggg gaagctcggc 420

gacgaggacg ccgcggcgag cttctcgtcg gtgaaggagg aggaggaccc ggcggcgtcc 480

gacgccgacc ccccggccac cggcgcgccg cagggctcgt ccgagagcga ctcgagcgcg 540

gtgctgaacg acgcggagat ccttccacac aagccagcgc cggcggccgc cgccgacgcc 600

gcggcctcgg aggagacgga ggcggtggtg accggcgccg cgctgctcca ccacgccgag 660

gtgttcttcc acgggcagct tctcaaggtg gacgacgacg aggcggcgtt cctaggcgac 720

gacggcgcgg cgtgcggcgg cttcttcgcc gacgagcatc tcccgtcgct gccgtggtgg 780

gccgagccca ccgagcaatg gacgacctag 810

<210> 272
<211> 269
<212> PRT
<213> Oryza sativa

<400> 272

Met Arg Ser Pro Ala Ala Leu Leu Pro Val Val Ala Asp Gly Gly Gly
1               5                   10                  15

Gly Val Gly Val Glu Glu Glu Met Asp Val Asp Glu Asp Met Ala Met
            20                  25                  30

Cys Gly Gly Arg Gly Gly Gly Gly Gly Glu Lys Lys Arg Arg Leu Ser
        35                  40                  45

Val Glu Gln Val Arg Ala Leu Glu Arg Ser Phe Glu Thr Glu Asn Lys
    50                  55                  60

Leu Glu Pro Glu Arg Lys Ala Arg Leu Ala Arg Asp Leu Gly Leu Gln
65                  70                  75                  80

Pro Arg Gln Val Ala Val Trp Phe Gln Asn Arg Arg Ala Arg Trp Lys
                85                  90                  95

Thr Lys Gln Leu Glu Arg Asp Tyr Ala Ala Leu Arg Gln Ser Tyr Asp
            100                 105                 110

Ala Leu Arg Ala Asp His Asp Ala Leu Arg Arg Asp Lys Asp Ala Leu
115 120 125

Leu Ala Glu Ile Lys Glu Leu Lys Gly Lys Leu Gly Asp Glu Asp Ala
130 135 140

Ala Ala Ser Phe Ser Ser Val Lys Glu Glu Glu Asp Pro Ala Ala Ser
145 150 155 160

Asp Ala Asp Pro Pro Ala Thr Gly Ala Pro Gln Gly Ser Ser Glu Ser
165 170 175

Asp Ser Ser Ala Val Leu Asn Asp Ala Glu Ile Leu Pro His Lys Pro
180 185 190

Ala Pro Ala Ala Ala Ala Asp Ala Ala Ala Ser Glu Glu Thr Glu Ala
195 200 205

Val Val Thr Gly Ala Ala Leu Leu His His Ala Glu Val Phe Phe His
210 215 220

Gly Gln Leu Leu Lys Val Asp Asp Asp Glu Ala Ala Phe Leu Gly Asp
225 230 235 240

Asp Gly Ala Ala Cys Gly Gly Phe Phe Ala Asp Glu His Leu Pro Ser
245 250 255

Leu Pro Trp Trp Ala Glu Pro Thr Glu Gln Trp Thr Thr
260 265

<210> 273
<211> 834
<212> DNA
<213> Oryza sativa

<400> 273
atgaagcgac ccggcggtgc cggcggcggc ggaggcagcc catcgctcgt cacgatggct 60

aattctagtg atgatggata tggaggggtt gggatggagg cggaggggga cgtggaggag 120

gagatgatgg cgtgcggcgg cggcggggag aagaagcggc ggctgagcgt ggagcaggtt 180

cgcgcgctgg agcggagctt cgaggtggag aacaagcttg agcctgagcg gaaggcgcgg 240

ctggcgcgcg acctcggcct gcagccgcgc caggtcgccg tctggttcca gaaccgccgc 300

gcgcggtgga agaccaagca gctcgagcgc gactacgccg cgctccgcca ttcctacgac 360

tccctgcgcc tcgatcacga cgcgctccgc cgcgacaagg acgccctcct cgccgagatc 420

aaggagctga aggcgaagct cggggacgag gaggcggcgg cgagcttcac gtcggtgaag 480

gaggagccgg cggcctccga cgggccaccg gcggcgggat ttgggtcgtc cgacagcgac 540

tcaagcgcgg tgctgaacga cgtggacgcg gccggcgccg cgcccgcggc gacggacgcg 600

ctggctccgg aggcgtgcac gtttctcggc gcgccgcccg ccgcgggcgc gggcgcgggc 660

gcagcggcgg cggcgagcca cgaggaggtg ttcttccacg gcaatttcct caaggtggag 720

gaggacgaga cggggttcct cgacgacgac gagccgtgcg gcgggttctt cgccgacgat 780

cagccccccgc cgctgtcgtc gtggtgggcc gagcccacgg agcactggaa ctga 834

<210> 274
<211> 277
<212> PRT
<213> Oryza sativa

<400> 274

Met Lys Arg Pro Gly Gly Ala Gly Gly Gly Gly Gly Ser Pro Ser Leu
1 5 10 15

Val Thr Met Ala Asn Ser Ser Asp Asp Gly Tyr Gly Gly Val Gly Met
20 25 30

Glu Ala Glu Gly Asp Val Glu Glu Glu Met Met Ala Cys Gly Gly Gly
35 40 45

Gly Glu Lys Lys Arg Arg Leu Ser Val Glu Gln Val Arg Ala Leu Glu
50 55 60

Arg Ser Phe Glu Val Glu Asn Lys Leu Glu Pro Glu Arg Lys Ala Arg
65 70 75 80

Leu Ala Arg Asp Leu Gly Leu Gln Pro Arg Gln Val Ala Val Trp Phe
85 90 95

Gln Asn Arg Arg Ala Arg Trp Lys Thr Lys Gln Leu Glu Arg Asp Tyr
100 105 110

Ala Ala Leu Arg His Ser Tyr Asp Ser Leu Arg Leu Asp His Asp Ala
115 120 125

Leu Arg Arg Asp Lys Asp Ala Leu Leu Ala Glu Ile Lys Glu Leu Lys
130 135 140

Ala Lys Leu Gly Asp Glu Glu Ala Ala Ala Ser Phe Thr Ser Val Lys
145 150 155 160

Glu Glu Pro Ala Ala Ser Asp Gly Pro Pro Ala Ala Gly Phe Gly Ser
165 170 175

Ser Asp Ser Asp Ser Ser Ala Val Leu Asn Asp Val Asp Ala Ala Gly
180 185 190

```
Ala Ala Pro Ala Ala Thr Asp Ala Leu Ala Pro Glu Ala Cys Thr Phe
        195                 200                 205

Leu Gly Ala Pro Pro Ala Ala Gly Ala Gly Ala Gly Ala Ala Ala Ala
    210                 215                 220

Ala Ser His Glu Glu Val Phe Phe His Gly Asn Phe Leu Lys Val Glu
225                 230                 235                 240

Glu Asp Glu Thr Gly Phe Leu Asp Asp Asp Glu Pro Cys Gly Gly Phe
                245                 250                 255

Phe Ala Asp Asp Gln Pro Pro Pro Leu Ser Ser Trp Trp Ala Glu Pro
            260                 265                 270

Thr Glu His Trp Asn
        275


<210>  275
<211>  375
<212>  DNA
<213>  Oryza sativa

<400>  275
atgtcatcct ctctctttct tcttcctcct ctctttcttt ctctcttctc tccccttcgg      60

ctagccgcag ggagcacggg ctggggcggt agtgggcggg gacaagagag gcggagggag     120

ctgaagcggc gccggcggcg gcacacgctc tccagcctca gcggcaagcg tggtgcgcca     180

tctgccgctg ccgccgccgt cggcggcagc gacgacgagg actccgacga cggatcccgc     240

aagaagctcc gcctctccaa ggaccaagcc gccgtcctcg aggacacctt caacaagcac     300

aacaccctca accccaagca gaaggcggcg ctggcgaggc agctgaatct gaagccgcgg     360

caggtggagg tgtag                                                      375


<210>  276
<211>  124
<212>  PRT
<213>  Oryza sativa

<400>  276

Met Ser Ser Ser Leu Phe Leu Leu Pro Pro Leu Phe Leu Ser Leu Phe
1               5                   10                  15

Ser Pro Leu Arg Leu Ala Ala Gly Ser Thr Gly Trp Gly Gly Ser Gly
            20                  25                  30

Arg Gly Gln Glu Arg Arg Arg Glu Leu Lys Arg Arg Arg Arg Arg His
        35                  40                  45

Thr Leu Ser Ser Leu Ser Gly Lys Arg Gly Ala Pro Ser Ala Ala Ala
```

```
    50                  55                  60

Ala Ala Val Gly Gly Ser Asp Asp Glu Asp Ser Asp Asp Gly Ser Arg
65                  70                  75                  80

Lys Lys Leu Arg Leu Ser Lys Asp Gln Ala Ala Val Leu Glu Asp Thr
                85                  90                  95

Phe Asn Lys His Asn Thr Leu Asn Pro Lys Gln Lys Ala Ala Leu Ala
            100                 105                 110

Arg Gln Leu Asn Leu Lys Pro Arg Gln Val Glu Val
        115                 120

<210>  277
<211>  1767
<212>  DNA
<213>  Oryza sativa

<400>  277
atgttttatt caagtccctt ttgttcatct cctttgcaga ttccatttct gacccaaatt      60

ttggctattc ctcatgatga gtttgtctca aactggtgct ctgttaatct gccagtgata     120

gaagaagacg ctaatcttga ttatgatcca tttggtgcag ctgagctggc actggcagct     180

gctggtaata agttaactga agctaaagca aactattctt gccctttttcg ccctatcagt     240

atgccttcta tagcatatgc gcagacaaga acatcatgtg tggtgaaaat aatagcaaat     300

ttgcatgttt ttgtcccaaa catatgcgaa gagcaagaaa gagacctttt tcttcagaaa     360

tttcagaagt acttggtatc agggaatcct agatcatcag ttgatcatcc agcatcagct     420

gatctcaagg ccactacagt ttgcagaaac ttgggatctt tgtctgagta tgctagatcg     480

ttaattccta ataacttgtt aaacgaggaa gatgtgcaat tgttaagtga atttgcttat     540

aagttacaaa cttggtgtaa atcacatgtt ggacagagta catcccaggc agtaaagatt     600

gatccgtcat cagaaagcaa ggaagacttt aagccactgc agcatccctt gataccaagt     660

actgttgttc cagattccag tataaataac cttccgaaga acatggaaga gcctacacca     720

acaaacatgg aagagcctgc accaacaccc tcaacaaagc aagagggaaa tgccagggat     780

gagactccta gaagcactgt cgctttaaat ggtgggttcc tgcagaattc agtcggccag     840

gacttagtcc atcttggtgt ggcgagaact agttcaggct ttctaggggg aggtaccagt     900

acaagtacag gatccctgcg ctgcaaaatg gatcttgatc ctgcatccag cagtatggac     960

catttcaaaa caccagatag aaaagaaagt ggtcttcagg atgatgagaa aggagacact    1020

cacatgtatg atgagagaca acctaagaga aggaagcgaa ccattatgaa tgataggcaa    1080

ataaacgaaa tcgagaaggc tcttattgat gagcctgaga tgcataagaa tgctgcttta    1140

ctgcaggcat ggtcagagaa gttaagtggg cagggctcgg agattacgtc atctcagcta    1200
```

```
aagaattggc tgaacaacag aaaagctaag cttgctcgta ttgcgaaaga aagaggagta   1260
ctatctgagg gtgagaacgc agataagcca tctacgccag ctactcccca ccactgtgac   1320
tcctcagaaa gtgctggcga ggagagctac ttgccacctg cgagggtcat gagtgctcta   1380
ggcatatcca agggcagcag atttgtgagc ccagatggca atgagacaac atcacaggca   1440
gaattcaatc aaaatatcat gcttagccgc cctttcacaa gatcattctc atttgaacct   1500
ggccgtcttg tttcgctcat tgataatgat gggaaggagg ttggcagggg aaagatcttc   1560
caagttgagg ggagactgca agggaaggcc ctgacagata ctcgcgtttg catcgttgat   1620
gttattgagc tcaagattga gaaatggcgg gagctacctc atccctcgga agcatcagga   1680
agaacattcc aagaggcaga atcaaggaac ggtggtgtga tgagggtcgc gtgggatgtc   1740
atcagactat ctccagtggt tcagtaa                                       1767
```

<210> 278
<211> 588
<212> PRT
<213> Oryza sativa

<400> 278

```
Met Phe Tyr Ser Ser Pro Phe Cys Ser Ser Pro Leu Gln Ile Pro Phe
1               5                   10                  15

Leu Thr Gln Ile Leu Ala Ile Pro His Asp Glu Phe Val Ser Asn Trp
            20                  25                  30

Cys Ser Val Asn Leu Pro Val Ile Glu Glu Asp Ala Asn Leu Asp Tyr
        35                  40                  45

Asp Pro Phe Gly Ala Ala Glu Leu Ala Leu Ala Ala Ala Gly Asn Lys
    50                  55                  60

Leu Thr Glu Ala Lys Ala Asn Tyr Ser Cys Pro Phe Arg Pro Ile Ser
65                  70                  75                  80

Met Pro Ser Ile Ala Tyr Ala Gln Thr Arg Thr Ser Cys Val Val Lys
                85                  90                  95

Ile Ile Ala Asn Leu His Val Phe Val Pro Asn Ile Cys Glu Glu Gln
            100                 105                 110

Glu Arg Asp Leu Phe Leu Gln Lys Phe Gln Lys Tyr Leu Val Ser Gly
        115                 120                 125

Asn Pro Arg Ser Ser Val Asp His Pro Ala Ser Ala Asp Leu Lys Ala
    130                 135                 140

Thr Thr Val Cys Arg Asn Leu Gly Ser Leu Ser Glu Tyr Ala Arg Ser
```

145 150 155 160

Leu Ile Pro Asn Asn Leu Leu Asn Glu Glu Asp Val Gln Leu Leu Ser
165 170 175

Glu Phe Ala Tyr Lys Leu Gln Thr Trp Cys Lys Ser His Val Gly Gln
180 185 190

Ser Thr Ser Gln Ala Val Lys Ile Asp Pro Ser Ser Glu Ser Lys Glu
195 200 205

Asp Phe Lys Pro Leu Gln His Pro Leu Ile Pro Ser Thr Val Val Pro
210 215 220

Asp Ser Ser Ile Asn Asn Leu Pro Lys Asn Met Glu Glu Pro Thr Pro
225 230 235 240

Thr Asn Met Glu Glu Pro Ala Pro Thr Pro Ser Thr Lys Gln Glu Gly
245 250 255

Asn Ala Arg Asp Glu Thr Pro Arg Ser Thr Val Ala Leu Asn Gly Gly
260 265 270

Phe Leu Gln Asn Ser Val Gly Gln Asp Leu Val His Leu Gly Val Ala
275 280 285

Arg Thr Ser Ser Gly Phe Leu Gly Gly Gly Thr Ser Thr Ser Thr Gly
290 295 300

Ser Leu Arg Cys Lys Met Asp Leu Asp Pro Ala Ser Ser Ser Met Asp
305 310 315 320

His Phe Lys Thr Pro Asp Arg Lys Glu Ser Gly Leu Gln Asp Asp Glu
325 330 335

Lys Gly Asp Thr His Met Tyr Asp Glu Arg Gln Pro Lys Arg Arg Lys
340 345 350

Arg Thr Ile Met Asn Asp Arg Gln Ile Asn Glu Ile Glu Lys Ala Leu
355 360 365

Ile Asp Glu Pro Glu Met His Lys Asn Ala Ala Leu Leu Gln Ala Trp
370 375 380

Ser Glu Lys Leu Ser Gly Gln Gly Ser Glu Ile Thr Ser Ser Gln Leu
385 390 395 400

Lys Asn Trp Leu Asn Asn Arg Lys Ala Lys Leu Ala Arg Ile Ala Lys
405 410 415

Glu Arg Gly Val Leu Ser Glu Gly Glu Asn Ala Asp Lys Pro Ser Thr
420 425 430

Pro Ala Thr Pro His His Cys Asp Ser Ser Glu Ser Ala Gly Glu Glu
435 440 445

Ser Tyr Leu Pro Pro Ala Arg Val Met Ser Ala Leu Gly Ile Ser Lys
450 455 460

Gly Ser Arg Phe Val Ser Pro Asp Gly Asn Glu Thr Thr Ser Gln Ala
465 470 475 480

Glu Phe Asn Gln Asn Ile Met Leu Ser Arg Pro Phe Thr Arg Ser Phe
485 490 495

Ser Phe Glu Pro Gly Arg Leu Val Ser Leu Ile Asp Asn Asp Gly Lys
500 505 510

Glu Val Gly Arg Gly Lys Ile Phe Gln Val Glu Gly Arg Leu Gln Gly
515 520 525

Lys Ala Leu Thr Asp Thr Arg Val Cys Ile Val Asp Val Ile Glu Leu
530 535 540

Lys Ile Glu Lys Trp Arg Glu Leu Pro His Pro Ser Glu Ala Ser Gly
545 550 555 560

Arg Thr Phe Gln Glu Ala Glu Ser Arg Asn Gly Gly Val Met Arg Val
565 570 575

Ala Trp Asp Val Ile Arg Leu Ser Pro Val Val Gln
580 585

<210> 279
<211> 6
<212> PRT
<213> Artificial sequence

<220>
<223> motif I

<400> 279

Arg Lys Lys Leu Arg Leu
1 5

<210> 280
<211> 24
<212> PRT
<213> Artificial sequence

<220>
<223> motif II

<400> 280

Thr Lys Leu Lys Gln Thr Glu Val Asp Cys Glu Phe Leu Arg Arg Cys
1 5 10 15

Cys Glu Asn Leu Thr Glu Glu Asn
20

<210> 281
<211> 10
<212> PRT
<213> Artificial sequence

<220>
<223> motif III

<400> 281

Thr Leu Thr Met Cys Pro Ser Cys Glu Arg
1 5 10

<210> 282
<211> 2193
<212> DNA
<213> Oryza sativa

<400> 282
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtaccttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga attttttttc ccaaatatct taaaaagtca 1740

ctttctggtt cagttcaatg aattgattgc tacaaataat gcttttatag cgttatccta 1800

gctgtagttc agttaatagg taatacccct atagtttagt caggagaaga acttatccga 1860

tttctgatct ccatttttaa ttatatgaaa tgaactgtag cataagcagt attcatttgg 1920

attatttttt ttattagctc tcaccccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 283
<211> 58
<212> DNA
<213> Artificial sequence

<220>
<223> primer: p01294

<400> 283
ggggacaagt ttgtacaaaa aagcaggctt cacaatgatg ttcgagaaag acgatctg 58

<210> 284
<211> 52
<212> DNA
<213> Artificial sequence

<220>
<223> primer: p00402

<400> 284
ggggaccact ttgtacaaga aagctgggtt taggacctag gacgaagagc gt 52

<210> 285
<211> 622
<212> DNA
<213> Arabidopsis thaliana

<400> 285
atgagcagac ccggagattg gaactgcagg tcatgcagcc atctcaactt ccagcgccgt 60

gactcttgcc agcgatgcgg tgactctcgt tccggccccg gtggagttgg tggcttagac 120

tttggtaatt tcggtggcag agccatgtct gctttcggat tcaccaccgg ctccgacgtt 180

cgtcccggtg attggtactg caccgtggga aactgcggga cacacaactt cgccagtcgc 240

tccacctgct tcaaatgcgg cactttcaag gacgagaccg gcgctggagg cggaggtggt 300

ggcatcggcg gtccggccat gtttgacgcc gacattatgc ggtctagagt ccccggtaac 360

ggtggtcgct ctagctggaa atccggcgac tggatttgca ctaggattgg ttgcaatgag 420

cataactttg caagcagaat ggaatgcttc aggtgcaatg caccaaggga cttcagcaac 480

agaacctctt tctaagttat acaaactgct tttgaagtag ccttgtctac ccagctttct 540

tgtacaaagt tggcattata agaaagcatt gcttatcaat ttgttgcaac gaacaggtca 600

ctatcagtca aaataaaatc at 622

<210> 286
<211> 164
<212> PRT
<213> Arabidopsis thaliana

<400> 286

Met Ser Arg Pro Gly Asp Trp Asn Cys Arg Ser Cys Ser His Leu Asn
1 5 10 15

Phe Gln Arg Arg Asp Ser Cys Gln Arg Cys Gly Asp Ser Arg Ser Gly
20 25 30

Pro Gly Gly Val Gly Gly Leu Asp Phe Gly Asn Phe Gly Gly Arg Ala
35 40 45

Met Ser Ala Phe Gly Phe Thr Thr Gly Ser Asp Val Arg Pro Gly Asp
50 55 60

Trp Tyr Cys Thr Val Gly Asn Cys Gly Thr His Asn Phe Ala Ser Arg
65 70 75 80

Ser Thr Cys Phe Lys Cys Gly Thr Phe Lys Asp Glu Thr Gly Ala Gly
85 90 95

Gly Gly Gly Gly Gly Ile Gly Gly Pro Ala Met Phe Asp Ala Asp Ile
100 105 110

Met Arg Ser Arg Val Pro Gly Asn Gly Gly Arg Ser Ser Trp Lys Ser
115 120 125

Gly Asp Trp Ile Cys Thr Arg Ile Gly Cys Asn Glu His Asn Phe Ala
130 135 140

Ser Arg Met Glu Cys Phe Arg Cys Asn Ala Pro Arg Asp Phe Ser Asn
145 150 155 160

Arg Thr Ser Phe

<210> 287
<211> 717
<212> DNA
<213> Gossypium hirsutum

<400> 287
atgagcaggc caggagattg gaactgcagg tcatgccaac acctcaactt ccaaaggagg 60

gacaactgcc aacgttgcgg tgaatctcga tacggtgtta gagtcggctc gacattcggg 120

tttaccgctg gctcggacgt tcgacctggt gactggtatt gcacggctgg aaactgcggc 180

acccacaatt tcgccagccg gtctacttgt ttcaattgcg gcgcgttcaa ggacgagtcg 240

gctggaggtt tcgacttgga catgtctcga tcaagagggt tcggaggtaa ccgatccggc 300

tggaaatcag ggattggat atgtaccagg ttagggtgca atgaacataa ttttgctagc 360

agaatggaat gtttcagatg cagtgctcca agagaattca acaatagaac ttcatattaa 420

atcacatgca tgctactata tccatttttg ggtgttttga ggcaattaat aggagattat 480

aatgagagag tgttactggc tttgatgatg aacaccacaa gcattttgtc atgtttcttt 540

tataagattc atggcaatgt agtacttttt cttgtgatga ttatttatgg tttcaattct 600

atggttttat tgtcttttat tttagagagt tttatttata tttttgtaat ggtgctttgg 660

ctttattaaa agaagatgat tctcttctgt cttttcaaaa aaaaaaaaaa aaaaaaa 717

<210> 288
<211> 139
<212> PRT
<213> Gossypium hirsutum

<400> 288

Met Ser Arg Pro Gly Asp Trp Asn Cys Arg Ser Cys Gln His Leu Asn
1 5 10 15

Phe Gln Arg Arg Asp Asn Cys Gln Arg Cys Gly Glu Ser Arg Tyr Gly
20 25 30

Val Arg Val Gly Ser Thr Phe Gly Phe Thr Ala Gly Ser Asp Val Arg
35 40 45

Pro Gly Asp Trp Tyr Cys Thr Ala Gly Asn Cys Gly Thr His Asn Phe
    50                  55                  60

Ala Ser Arg Ser Thr Cys Phe Asn Cys Gly Ala Phe Lys Asp Glu Ser
65                  70                  75                  80

Ala Gly Gly Phe Asp Leu Asp Met Ser Arg Ser Arg Gly Phe Gly Gly
                85                  90                  95

Asn Arg Ser Gly Trp Lys Ser Gly Asp Trp Ile Cys Thr Arg Leu Gly
            100                 105                 110

Cys Asn Glu His Asn Phe Ala Ser Arg Met Glu Cys Phe Arg Cys Ser
        115                 120                 125

Ala Pro Arg Glu Phe Asn Asn Arg Thr Ser Tyr
    130                 135

<210>  289
<211>  937
<212>  DNA
<213>  Zea mays

<400>  289
cgggcacgac cagcaacaca acagccgagc tttgcttagg caagatgaac aggaagccag      60

gagactggga ctgcagggcg tgccagcacc tcaacttcag ccgccgagac atatgccagc     120

gctgcagcga gccacgtgga gttgctgatc gtggcagtgg cggcggcgga ggaggcgact     180

acgccagctt cggtggccgc ggtggctcct ccttcggcgg cggctttggc gctgctggct     240

ccgacgtccg ccctggtgac tggtactgct cctgcggcgc gcacaacttc gccagccgct     300

ccagctgctt caagtgctcc gcctacaagg aggaggccgc tgtgaacagt ggcgctggcg     360

gctttgatgg cgacatgtca cgctcacggg gctacggctt cggcagcggt gctgctgctg     420

ctgctggtgc tggcgctgcc cgtactacca accgccccgg ttggaagtcc ggagactgga     480

tctgcaccag atccggatgc aacgagcaca acttcgccag caggatggag tgcttcaggt     540

gcaacgcacc gcgggactct ggcactgagg tgtaggatcg agcaagttaa aaagtctgca     600

gcgccgaaga aagcgacgac aagaggagtc ctcatcacgt cgtaacgtaa gagagagagt     660

agtggatttg caacaaaaaa aaaaaaaaga cggccgcgat gctctgttac tagctagcta     720

gttttgttca accacccatg ccgtctcttc tcttttatta gatttggttt ggttctcata     780

gccctttaat taccatttgg gacctatgtt tgctgttctg tttcccgttc gtctcctccg     840

catgtttgcg cttggatcga gtcttgtgat gtaacccccc aaaaaacgct tgcttaacta     900

gtactgttgc ttcttaataa aaaaaaaaaa aaaaaaa                              937

<210> 290
<211> 176
<212> PRT
<213> Zea mays

<400> 290

Met Asn Arg Lys Pro Gly Asp Trp Asp Cys Arg Ala Cys Gln His Leu
1               5                   10                  15

Asn Phe Ser Arg Arg Asp Ile Cys Gln Arg Cys Ser Glu Pro Arg Gly
            20                  25                  30

Val Ala Asp Arg Gly Ser Gly Gly Gly Gly Gly Gly Asp Tyr Ala Ser
        35                  40                  45

Phe Gly Gly Arg Gly Gly Ser Ser Phe Gly Gly Gly Phe Gly Ala Ala
    50                  55                  60

Gly Ser Asp Val Arg Pro Gly Asp Trp Tyr Cys Ser Cys Gly Ala His
65                  70                  75                  80

Asn Phe Ala Ser Arg Ser Ser Cys Phe Lys Cys Ser Ala Tyr Lys Glu
                85                  90                  95

Glu Ala Ala Val Asn Ser Gly Ala Gly Gly Phe Asp Gly Asp Met Ser
            100                 105                 110

Arg Ser Arg Gly Tyr Gly Phe Gly Ser Gly Ala Ala Ala Ala Ala Gly
        115                 120                 125

Ala Gly Ala Ala Arg Thr Thr Asn Arg Pro Gly Trp Lys Ser Gly Asp
    130                 135                 140

Trp Ile Cys Thr Arg Ser Gly Cys Asn Glu His Asn Phe Ala Ser Arg
145                 150                 155                 160

Met Glu Cys Phe Arg Cys Asn Ala Pro Arg Asp Ser Gly Thr Glu Val
                165                 170                 175

<210> 291
<211> 501
<212> DNA
<213> Oryza sativa

<400> 291
atgaacagga agccaggaga ctgggactgc agggcgtgcc agcacctcaa cttcagccgc 60

cgggacctat gccagcgctg cggcgagccg cgtggcgccg ctgatcgcgg cagcggtggt 120

ggcggtgact acgccaactt cggcggccgt ggtggttcct ccttcggtgg aggctttggc 180

actggctctg atgtccgccc aggtgactgg tactgcaact gcggcgcgca caacttcgcc 240

agccgctcca gctgcttcaa gtgcgctgct ttcaaggacg atgctgccgt caacagtggc 300

ggcgctggtg cctttgatgg tggggacatg tcgcgctcgc ggggctacgg cttcggcagc 360

ggcgccgtcc gcgccagccg ccctggctgg aagtctggcg actggatttg caccaggtct 420

ggatgcaatg agcacaactt cgccagcagg atggagtgct tcaggtgcaa cgcaccgcgg 480

gactccggca ctgaggtgta a 501

<210> 292
<211> 166
<212> PRT
<213> Oryza sativa

<400> 292

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Glu Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Thr Gly Ser Asp
50 55 60

Val Arg Pro Gly Asp Trp Tyr Cys Asn Cys Gly Ala His Asn Phe Ala
65 70 75 80

Ser Arg Ser Ser Cys Phe Lys Cys Ala Ala Phe Lys Asp Asp Ala Ala
85 90 95

Val Asn Ser Gly Gly Ala Gly Ala Phe Asp Gly Gly Asp Met Ser Arg
100 105 110

Ser Arg Gly Tyr Gly Phe Gly Ser Gly Ala Val Arg Ala Ser Arg Pro
115 120 125

Gly Trp Lys Ser Gly Asp Trp Ile Cys Thr Arg Ser Gly Cys Asn Glu
130 135 140

His Asn Phe Ala Ser Arg Met Glu Cys Phe Arg Cys Asn Ala Pro Arg
145 150 155 160

Asp Ser Gly Thr Glu Val
165

<210> 293
<211> 859
<212> DNA

<213> Arabidopsis thaliana

<400> 293
ataagctctt acactcattt caactctctt tttcgtttcc attaccctca gaagaagatg 60

aataggccgg gagattggaa ctgcagattg tgtagccacc tcaacttcca gaggagggat 120

tcatgccaac gttgtagaga gcctagaccg ggcgggatca gtaccgattt actcagcggt 180

tttggtggcc gtccggttag tagctccttc ggtttcaaca ccgggcccga tgtgcgaccc 240

ggggattggt attgcaacct tggggattgt gggacacata attttgccaa taggtccagt 300

tgtttcaagt gtggtgccgc aaaagatgag ttttcatgct caagtgctgc tgcaacaacc 360

gggtttatgg acatgaatgt tggtccgaga cgtggccttt ttggttttgg cggcagcagt 420

agtggtggtg gtggtacggg ccgttctcct tggaaatctg gagattggat ttgcccaagg 480

tcaggctgta acgaacataa cttcgcaagc aggtcagagt gtttcaggtg taacgcacca 540

aaggaacttg ccaccgaacc accctattag tcatttagtc ctcctacctt cttcatcatt 600

tccaatactc tggaagcatt agaggagagc agaaaggtga agaatcgaag caagataccg 660

accgccctta atctcttgat ttgtttaatt tcttagattt gactcgtttt atatctcgta 720

gtagtcagac ctatgttaga atgtaagcat gtcgagagtt ttccgaagca ttttgttctg 780

atatcggtct cctagctagt atgtaagttt gtttgaatgt attcttattt ctgataaagt 840

tgtttccttc tgattccgc 859

<210> 294
<211> 170
<212> PRT
<213> Arabidopsis thaliana

<400> 294

Met Asn Arg Pro Gly Asp Trp Asn Cys Arg Leu Cys Ser His Leu Asn
1 5 10 15

Phe Gln Arg Arg Asp Ser Cys Gln Arg Cys Arg Glu Pro Arg Pro Gly
20 25 30

Gly Ile Ser Thr Asp Leu Leu Ser Gly Phe Gly Gly Arg Pro Val Ser
35 40 45

Ser Ser Phe Gly Phe Asn Thr Gly Pro Asp Val Arg Pro Gly Asp Trp
50 55 60

Tyr Cys Asn Leu Gly Asp Cys Gly Thr His Asn Phe Ala Asn Arg Ser
65 70 75 80

Ser Cys Phe Lys Cys Gly Ala Ala Lys Asp Glu Phe Ser Cys Ser Ser
85 90 95

```
Ala Ala Ala Thr Thr Gly Phe Met Asp Met Asn Val Gly Pro Arg Arg
            100                 105                 110

Gly Leu Phe Gly Phe Gly Gly Ser Ser Ser Gly Gly Gly Gly Thr Gly
        115                 120                 125

Arg Ser Pro Trp Lys Ser Gly Asp Trp Ile Cys Pro Arg Ser Gly Cys
    130                 135                 140

Asn Glu His Asn Phe Ala Ser Arg Ser Glu Cys Phe Arg Cys Asn Ala
145                 150                 155                 160

Pro Lys Glu Leu Ala Thr Glu Pro Pro Tyr
                165                 170


<210>  295
<211>  834
<212>  DNA
<213>  Oryza sativa

<400>  295
cctagtacta agaacagcaa ccacctctga aagctttaca agttcgcagg ggcttaactg      60

cgaatgaaca tccagaggaa gccaggagac tggaactgca aatcgtgcca gcatctcaac     120

ttcagccgcc gggactactg ccagcgctgc cataccccac gccaggacct gccgcttggc     180

gatggttatg tcccaggtgg tgtgctgtcc tccctggaca ttcgcccggg cgactggtac     240

tgcaactgcg gctatcacaa ctttgctagc cgagcaagct gcttcaaatg tggcgccatt     300

gtgaaggacc ttccagcagg ccaaggtggt ggtgttgcca acggtgactt tgcccgtgcc     360

ctcgacagca gcgcagttcg tgctgggtgg aaggcgggtg actggatttg cacaaggcct     420

ggttgcaacg tccacaactt tgcaagtagg attgagtgct ataggtgcaa tgcacctagg     480

gaagcaggta atgtgaagta agaaaagact gacgcgacca aagatcgtga gacgtgacga     540

gctggccgag gatgaataaa gtgactgctg tcagttgtca cattcacagg ctgcgatcca     600

gaaactatgg atgggactat gtagtaacgt gctgatatat tattgctaag tgttactact     660

gcatggttgc aagggtggta gaagtacctt agcttccaag atcgttgtaa tgtgtgagtt     720

taattttggc gttttaagta ataagtctag tgattgcagg attgtacggg gtaatgtact     780

tgcattatcc attataatct taagcatcca atataattat gcaaaaaaaa aaaa           834


<210>  296
<211>  145
<212>  PRT
<213>  Oryza sativa

<400>  296

Met Asn Ile Gln Arg Lys Pro Gly Asp Trp Asn Cys Lys Ser Cys Gln
1               5                   10                  15
```

His Leu Asn Phe Ser Arg Arg Asp Tyr Cys Gln Arg Cys His Thr Pro
20 25 30

Arg Gln Asp Leu Pro Leu Gly Asp Gly Tyr Val Pro Gly Gly Val Leu
35 40 45

Ser Ser Leu Asp Ile Arg Pro Gly Asp Trp Tyr Cys Asn Cys Gly Tyr
50 55 60

His Asn Phe Ala Ser Arg Ala Ser Cys Phe Lys Cys Gly Ala Ile Val
65 70 75 80

Lys Asp Leu Pro Ala Gly Gln Gly Gly Gly Val Ala Asn Gly Asp Phe
85 90 95

Ala Arg Ala Leu Asp Ser Ser Ala Val Arg Ala Gly Trp Lys Ala Gly
100 105 110

Asp Trp Ile Cys Thr Arg Pro Gly Cys Asn Val His Asn Phe Ala Ser
115 120 125

Arg Ile Glu Cys Tyr Arg Cys Asn Ala Pro Arg Glu Ala Gly Asn Val
130 135 140

Lys
145

<210> 297
<211> 958
<212> DNA
<213> Oryza sativa

<400> 297
aagcctccat ccatccatcc attcatcaga gctcaagctc aagcaagcaa gcttgctagc 60

tagctgctga gcagcattcc agtctgctcc agctagctca gctctctctg ctctgctctt 120

tgctcgataa cgaccatcat cttcttcttc ttcttcttct tcttcttctt cttcttcttg 180

tacattctat ttgctcgata gatagataga tagatagata ggtagataga gatggagacg 240

aaggcggcgg cgatggcgat gaggaagccg gggggactgga gctgcaggtc gtgccagtac 300

gtgaacttct gcaagaggga ggcgtgccag cggtgcgggg aggcgaagct cggggtggag 360

cggacggact acgccgccat gggcggcggg tgggaggtga agcccggcga ctggtgctgc 420

cgctgctgcg ccgtcaacaa ctacgccagc cgcggcagct gcttcaagtg cggcgccgcc 480

aagaacgact ccgccgccgc cgtcgcccag ggctggggct tctccgtcgc ctcccaggcc 540

ggctggaaga acggcgactg gatctgcccc agaatggaat gcaacgtgca gaactacgct 600

aacagaaccg agtgcttccg gtgcaatttc cccagatact acgttgattg atctgacata 660

tatgatatat gccttgcact gagaagaaat gaaagggaaa taaaatttac gagatcgaag 720

atcgacggac aagattgatc ggccaccggt catgcccttc agggttttct tttttctatt 780

ttttttaga ataaccctcg ataacctgag ggttttttt catttgtaag agcggtaacg 840

gtactaaaaa aacacaaaaa cggcacaaaa ttgtggctga tgactgatca gtcttcctct 900

tttttttccc aataaaaggc agataattaa gaaagcaaaa ttattaaaaa aaaaaaa 958

<210> 298
<211> 139
<212> PRT
<213> Oryza sativa

<400> 298

Met Glu Thr Lys Ala Ala Ala Met Ala Met Arg Lys Pro Gly Asp Trp
1 5 10 15

Ser Cys Arg Ser Cys Gln Tyr Val Asn Phe Cys Lys Arg Glu Ala Cys
20 25 30

Gln Arg Cys Gly Glu Ala Lys Leu Gly Val Glu Arg Thr Asp Tyr Ala
35 40 45

Ala Met Gly Gly Gly Trp Glu Val Lys Pro Gly Asp Trp Cys Cys Arg
50 55 60

Cys Cys Ala Val Asn Asn Tyr Ala Ser Arg Gly Ser Cys Phe Lys Cys
65 70 75 80

Gly Ala Ala Lys Asn Asp Ser Ala Ala Ala Val Ala Gln Gly Trp Gly
85 90 95

Phe Ser Val Ala Ser Gln Ala Gly Trp Lys Asn Gly Asp Trp Ile Cys
100 105 110

Pro Arg Met Glu Cys Asn Val Gln Asn Tyr Ala Asn Arg Thr Glu Cys
115 120 125

Phe Arg Cys Asn Phe Pro Arg Tyr Tyr Val Asp
130 135

<210> 299
<211> 519
<212> DNA
<213> Beta vulgaris

<400> 299
atgagtaggc caggtgattg gaattgtagg tcatgcagcc acttgaactt ccaaaggagg 60

gactcctgcc agcgctgtgg ggacgtacgt cctgacggcc gaggcggcgg agggggagga 120

ggagactttg gcagtagctt tggagggagg tcaggtgggt ccccctttgg tgggggtttt 180

```
gcagggcccg atgttaggcc cggtgattgg tattgtagca ttggcaactg tggggcccac     240

aactttgcca gcaggtctag ctgcttcaag tgtggggcct acaaagagga agctggctgt     300

ggtgatagca tgggccgttc acgtggagga ttttcctttg gtggcatcgg cggtggcggt     360

agcggtgccg ctaccggccg ctcaggctgg aaatccggtg actggatttg cactaggtcg     420

ggttgcaacg agcataactt cgctagtagg actgagtgct tcagatgcag ggaaccaagg     480

gactccggta atgcaatgct aaaggaagta ccatgctga                            519


<210>  300
<211>  172
<212>  PRT
<213>  Beta vulgaris

<400>  300

Met Ser Arg Pro Gly Asp Trp Asn Cys Arg Ser Cys Ser His Leu Asn
1               5                   10                  15


Phe Gln Arg Arg Asp Ser Cys Gln Arg Cys Gly Asp Val Arg Pro Asp
            20                  25                  30


Gly Arg Gly Gly Gly Gly Gly Gly Gly Asp Phe Gly Ser Ser Phe Gly
        35                  40                  45


Gly Arg Ser Gly Gly Ser Pro Phe Gly Gly Gly Phe Ala Gly Pro Asp
    50                  55                  60


Val Arg Pro Gly Asp Trp Tyr Cys Ser Ile Gly Asn Cys Gly Ala His
65                  70                  75                  80


Asn Phe Ala Ser Arg Ser Ser Cys Phe Lys Cys Gly Ala Tyr Lys Glu
                85                  90                  95


Glu Ala Gly Cys Gly Asp Ser Met Gly Arg Ser Arg Gly Gly Phe Ser
            100                 105                 110


Phe Gly Gly Ile Gly Gly Gly Gly Ser Gly Ala Ala Thr Gly Arg Ser
        115                 120                 125


Gly Trp Lys Ser Gly Asp Trp Ile Cys Thr Arg Ser Gly Cys Asn Glu
    130                 135                 140


His Asn Phe Ala Ser Arg Thr Glu Cys Phe Arg Cys Arg Glu Pro Arg
145                 150                 155                 160


Asp Ser Gly Asn Ala Met Leu Lys Glu Val Pro Cys
                165                 170
```

<210> 301
<211> 807
<212> DNA
<213> Arabidopsis thaliana

<400> 301
atgggagacg gaagagaagg agactgggaa tgtttaggat gcagaaacag gaattatgcg 60

tttagatcat tctgtaacag atgcaagcag cctcgtctta tcatggataa taatacttct 120

ccaaactcta agtggcttcc tcgtatcggc gattggatct gcactggttg tactaacaac 180

aattatgcat cacgagaaaa gtgcaagaaa tgtgggcaat ctaaggaagt agcagcattg 240

tcagcacttg ctatccctgg agcttctctt caaactcatc tccattactt cacccgtgga 300

cctgagtcac atgatcaacc tggttcttta ctcgcattct ctaacgctac aaatcaagct 360

tcggttcata aagaatggag gagtggtgac tggatttgca gatgtggttt tcacaattat 420

tcctctcgta tacagtgcaa aaagtgcaat gaaatagctc cactagccct tggtacaaag 480

agattagcat cagaagcttt ggctcatgaa tgggatagca aaagactgaa tcaaggatat 540

acaagcatgc aaacacagtc agcgatatat gcatcttttc ctggtatgag cctaggaagg 600

gtctcaaatt ggcaacttcc tctcccgttt ctacaacaac actcaacacc tgctttactt 660

ggaatgggag tgaaacaatg gcgtgatggc gactggatgt gtacaaattg caagaatcac 720

aattatgcat cacgagcaga gtgcaatagg tgcaagacta cacgagatat ccttgatcag 780

gacataactc caacagaaca atcttga 807

<210> 302
<211> 268
<212> PRT
<213> Arabidopsis thaliana

<400> 302

Met Gly Asp Gly Arg Glu Gly Asp Trp Glu Cys Leu Gly Cys Arg Asn
1               5                   10                  15

Arg Asn Tyr Ala Phe Arg Ser Phe Cys Asn Arg Cys Lys Gln Pro Arg
            20                  25                  30

Leu Ile Met Asp Asn Asn Thr Ser Pro Asn Ser Lys Trp Leu Pro Arg
        35                  40                  45

Ile Gly Asp Trp Ile Cys Thr Gly Cys Thr Asn Asn Asn Tyr Ala Ser
    50                  55                  60

Arg Glu Lys Cys Lys Lys Cys Gly Gln Ser Lys Glu Val Ala Ala Leu
65                  70                  75                  80

Ser Ala Leu Ala Ile Pro Gly Ala Ser Leu Gln Thr His Leu His Tyr
                85                  90                  95

Phe Thr Arg Gly Pro Glu Ser His Asp Gln Pro Gly Ser Leu Leu Ala
100 105 110

Phe Ser Asn Ala Thr Asn Gln Ala Ser Val His Lys Glu Trp Arg Ser
115 120 125

Gly Asp Trp Ile Cys Arg Cys Gly Phe His Asn Tyr Ser Ser Arg Ile
130 135 140

Gln Cys Lys Lys Cys Asn Glu Ile Ala Pro Leu Ala Leu Gly Thr Lys
145 150 155 160

Arg Leu Ala Ser Glu Ala Leu Ala His Glu Trp Asp Ser Lys Arg Leu
165 170 175

Asn Gln Gly Tyr Thr Ser Met Gln Thr Gln Ser Ala Ile Tyr Ala Ser
180 185 190

Phe Pro Gly Met Ser Leu Gly Arg Val Ser Asn Trp Gln Leu Pro Leu
195 200 205

Pro Phe Leu Gln Gln His Ser Thr Pro Ala Leu Leu Gly Met Gly Val
210 215 220

Lys Gln Trp Arg Asp Gly Asp Trp Met Cys Thr Asn Cys Lys Asn His
225 230 235 240

Asn Tyr Ala Ser Arg Ala Glu Cys Asn Arg Cys Lys Thr Thr Arg Asp
245 250 255

Ile Leu Asp Gln Asp Ile Thr Pro Thr Glu Gln Ser
260 265

<210> 303
<211> 1264
<212> DNA
<213> Arabidopsis thaliana

<400> 303
ctatttctcc taccgtcgaa ctaaatccta gggcacaggt gaaatcgccg agtgacgtct 60

agccaccgca ccgcctcctt ctccggttat ctcgctacta atcagactat tccacagcta 120

tgtcacaggt agacaacaga aattcatcag cagccaagcg tgctagaact gacgggggc 180

gtagagaaga tgattggatc tgcccaagtt gtggcaatgt caacttttca ttcaggacaa 240

cttgcaatat gcgtaattgc actcagccta gacctgcaga tcataatgga aagtctgctc 300

ccaaacctat gcaacatcaa caaggtttct catcacccgg ggcatactta ggatctgggg 360

gtccccctcc agtatatatg ggcgggtcac catatggatc tcctctcttt aatggatcat 420

```
ctatgcctcc ttatgacgtc ccattttctg ggggttcgcc ttaccatttt aactataata     480

gccgaatgcc tgccggagct cattacagac cattacatat gtctggacca ccaccatacc     540

atggcggatc tatgatggga agtggtggta tgtatggaat gcctccacca atagacaggt     600

atggccttgg tatggcaatg ggtcctggtt ctgccgctgc catgatgcca agaccaaggt     660

tttacccaga tgaaaaatca caaaagagag attcaactcg cgataatgat tggacatgtc     720

cgaattgtgg taatgtaaac ttctcattca gaactgtatg taacatgagg aagtgcaaca     780

ctccaaagcc tggttctcag cagggtggaa gctcagataa aatatccaaa caaaatgcac     840

cggaagggag ctggaagtgt gataactgtg gaaatataaa ctacccattc aggagcaaat     900

gcaacaggca aaactgtgga gctgataagc ctggggatcg gtcgaatgga tctccgtccc     960

gtgcaccaga agagaacgat cagtgagtcg tagacatgtt tgggggttga gaagggtgag    1020

tctaatccag cgggtgtcgt aatgtcagtc aatgtctcgt caggtcaggt cgtccatgtt    1080

gctgcgtcgt cagtcaagtt gcttctatgc atttgtctct ttacttccct tgcgtggttg    1140

tggattgtat taataatgca aaaattgttt atttactttg tcgtcctcgt ggatcttgtc    1200

ttgctataga atcctcctcc aggtccatac tgttttacat cctaatctta agtaagttgg    1260

cacc                                                                 1264
```

<210> 304
<211> 288
<212> PRT
<213> Arabidopsis thaliana

<400> 304

```
Met Ser Gln Val Asp Asn Arg Asn Ser Ser Ala Ala Lys Arg Ala Arg
1               5                   10                  15

Thr Asp Gly Gly Arg Arg Glu Asp Asp Trp Ile Cys Pro Ser Cys Gly
            20                  25                  30

Asn Val Asn Phe Ser Phe Arg Thr Thr Cys Asn Met Arg Asn Cys Thr
        35                  40                  45

Gln Pro Arg Pro Ala Asp His Asn Gly Lys Ser Ala Pro Lys Pro Met
    50                  55                  60

Gln His Gln Gln Gly Phe Ser Ser Pro Gly Ala Tyr Leu Gly Ser Gly
65                  70                  75                  80

Gly Pro Pro Pro Val Tyr Met Gly Gly Ser Pro Tyr Gly Ser Pro Leu
                85                  90                  95

Phe Asn Gly Ser Ser Met Pro Pro Tyr Asp Val Pro Phe Ser Gly Gly
            100                 105                 110
```

Ser Pro Tyr His Phe Asn Tyr Asn Ser Arg Met Pro Ala Gly Ala His
115 120 125

Tyr Arg Pro Leu His Met Ser Gly Pro Pro Pro Tyr His Gly Gly Ser
130 135 140

Met Met Gly Ser Gly Gly Met Tyr Gly Met Pro Pro Pro Ile Asp Arg
145 150 155 160

Tyr Gly Leu Gly Met Ala Met Gly Pro Gly Ser Ala Ala Ala Met Met
165 170 175

Pro Arg Pro Arg Phe Tyr Pro Asp Glu Lys Ser Gln Lys Arg Asp Ser
180 185 190

Thr Arg Asp Asn Asp Trp Thr Cys Pro Asn Cys Gly Asn Val Asn Phe
195 200 205

Ser Phe Arg Thr Val Cys Asn Met Arg Lys Cys Asn Thr Pro Lys Pro
210 215 220

Gly Ser Gln Gln Gly Gly Ser Ser Asp Lys Ile Ser Lys Gln Asn Ala
225 230 235 240

Pro Glu Gly Ser Trp Lys Cys Asp Asn Cys Gly Asn Ile Asn Tyr Pro
245 250 255

Phe Arg Ser Lys Cys Asn Arg Gln Asn Cys Gly Ala Asp Lys Pro Gly
260 265 270

Asp Arg Ser Asn Gly Ser Pro Ser Arg Ala Pro Glu Glu Asn Asp Gln
275 280 285

<210> 305
<211> 1853
<212> DNA
<213> Oryza sativa

<400> 305
cagtttccac tttcttccaa agccgaaaaa tcgagagaga aagagcaaaa ccctaaccgc 60

ggcgctccgc cccttccgc cgccggattc ctcctcctcc ttcgtccgcc ttcgccggcg 120

ccgccctgct ccgaaggagc ccaggccttg tcgccgcgtc gcttgccccg ccgccggttt 180

cagcagcagc caccccacc accatgtctt ctcaggtcga caaccgtagc caatctgctg 240

gaaagcgtgc ccgcaccgac ggtgggcggc gtgaggacga ctgggtttgc cccagctgcc 300

agaacgtcaa cttcgccttc cgcaccacct gcaacatgcg caattgcaac caatccaggc 360

ccaccgacta tacgaaggat atgcagaaac ctatgcagac accgccgccc catttcccca 420

tgtcaggggg atacatgagc ccagggacgc cgccatccat gtacctcggg ggtggtgctc 480

ccccttatgg cacctccctc tatggcggac ctgctttacc acgttatggt attgctcagt 540

tccctggggg ttctggatat ccatatggct atggtggccg cctgccaatg gggagcccct 600

atgggccgcc gatgcatatg gcagggcctc catattctgc tggatccatg atgggaccag 660

gtggaatgta tgggatgccc atggacagat atagcttggg cttacctgct ggtcctggtc 720

caatgggcgc gagggctggt tcatattccg aggaaggatc ccagaagaag cctgcaggag 780

ctgggcgtga taatgattgg aaatgcccta attgcaacaa cattaacttt gcattccgga 840

cagtctgtaa catgagaaag tgcaatacac caagacctga aaaccagggg tccaaacctg 900

atggtgcaag aggtccaaaa ccaaagatgc cagaaggtag ttggaagtgc gagaagtgca 960

ataacataaa ctatccattc cgcacaaaat gtaaccgtcc aagttgcgag gctgaaaagc 1020

catttcagac aaacaatgct aatgagtcat ctgctgatca ggacaatcag ttgttgtcat 1080

gtaatattgt gaagctttta tcaaagctgc aactttcaca tgactttcaa gacaataatg 1140

agcaaacaaa ggtggatcct cccggtggcc ctgctggagt accgacaagt tcgcacgccc 1200

tgcgaatggc agctctcagt gagttgcaga acagtgttaa gagctgatac ccttttcatt 1260

ttccaaacaa ggtgagaact ttgatccatg tggatgtgct tctatcagga gcagctgaca 1320

gtattgcatg cagtgatctt gctaaggaag ttggagatgg gaaacatcgt gtgatggcca 1380

gccttgatgt aagaatgcaa atcagtctta ctccgtacca tatctgtgtg gagtgtgcac 1440

accggaagca tagtcatttt ttgaatggtc gattgcttct tccacagtaa agtctataat 1500

accagtgtgc aggtgttttt ttccctttct ttcagtgtga aggtgttttt ttaccccttt 1560

ctttcgttca ttcattcttt ttctttttcc ttgttgagac ggtatcctgg agcactttgt 1620

aatactctaa tgtgcttcgg aaagtagaat ataagttgtg cttatgggcg ggccaaaatg 1680

ctttggccgt tagttgcatg acagcctttc caacatgctg ctgcctagcc gtagttctcc 1740

caaaaatgaa accatcttga gatccgtttg cggttgaact gaaattagac ggtatctctg 1800

gtttcaacat catcctttaa accctggatg aaagatttgc aatattcctg cgg 1853

<210> 306
<211> 347
<212> PRT
<213> Oryza sativa

<400> 306

Met Ser Ser Gln Val Asp Asn Arg Ser Gln Ser Ala Gly Lys Arg Ala
1 5 10 15

Arg Thr Asp Gly Gly Arg Arg Glu Asp Asp Trp Val Cys Pro Ser Cys
20 25 30

Gln Asn Val Asn Phe Ala Phe Arg Thr Thr Cys Asn Met Arg Asn Cys

35 40 45

Asn Gln Ser Arg Pro Thr Asp Tyr Thr Lys Asp Met Gln Lys Pro Met
50 55 60

Gln Thr Pro Pro Pro His Phe Pro Met Ser Gly Gly Tyr Met Ser Pro
65 70 75 80

Gly Thr Pro Pro Ser Met Tyr Leu Gly Gly Gly Ala Pro Pro Tyr Gly
85 90 95

Thr Ser Leu Tyr Gly Gly Pro Ala Leu Pro Arg Tyr Gly Ile Ala Gln
100 105 110

Phe Pro Gly Gly Ser Gly Tyr Pro Tyr Gly Tyr Gly Gly Arg Leu Pro
115 120 125

Met Gly Ser Pro Tyr Gly Pro Pro Met His Met Ala Gly Pro Pro Tyr
130 135 140

Ser Ala Gly Ser Met Met Gly Pro Gly Gly Met Tyr Gly Met Pro Met
145 150 155 160

Asp Arg Tyr Ser Leu Gly Leu Pro Ala Gly Pro Gly Pro Met Gly Ala
165 170 175

Arg Ala Gly Ser Tyr Ser Glu Glu Gly Ser Gln Lys Lys Pro Ala Gly
180 185 190

Ala Gly Arg Asp Asn Asp Trp Lys Cys Pro Asn Cys Asn Asn Ile Asn
195 200 205

Phe Ala Phe Arg Thr Val Cys Asn Met Arg Lys Cys Asn Thr Pro Arg
210 215 220

Pro Glu Asn Gln Gly Ser Lys Pro Asp Gly Ala Arg Gly Pro Lys Pro
225 230 235 240

Lys Met Pro Glu Gly Ser Trp Lys Cys Glu Lys Cys Asn Asn Ile Asn
245 250 255

Tyr Pro Phe Arg Thr Lys Cys Asn Arg Pro Ser Cys Glu Ala Glu Lys
260 265 270

Pro Phe Gln Thr Asn Asn Ala Asn Glu Ser Ser Ala Asp Gln Asp Asn
275 280 285

Gln Leu Leu Ser Cys Asn Ile Val Lys Leu Leu Ser Lys Leu Gln Leu
290 295 300

Ser His Asp Phe Gln Asp Asn Asn Glu Gln Thr Lys Val Asp Pro Pro
305 310 315 320

Gly Gly Pro Ala Gly Val Pro Thr Ser Ser His Ala Leu Arg Met Ala
325 330 335

Ala Leu Ser Glu Leu Gln Asn Ser Val Lys Ser
340 345

<210> 307
<211> 1078
<212> DNA
<213> Oryza sativa

<400> 307
ctggtctctc gctctcttcg ctcgcggcgg cttctccgcc tccgcctccg ccgccctctc 60

ctcctctcgc ctcgccgccg gcgccgccag ccaccgcgcg ggtcgggcgg gccgtatctt 120

cctttctgtt ccgatggctt ccgcgaaggt ggagaaccgc ggcggcggtg ggttcggttc 180

gaagaggtca cgcaacgacg tgtctgtaag ggagggggac tggacttgtc ctcagtgtgg 240

taatgtcaac ttcagtttta gaaatgtttg caaccgcgga gcctgtggtg caccccgtcc 300

atcaccgagt ctaagcccaa gagtgccacc tcctcctgct gctggatatg atcggccaca 360

tcttggatat gatcggccac atctgtttta tggtagtgct ggcaccccac ctcctattcc 420

tcttggatct ggtagctatg gtgcccccta tccacatctt ggcttgcggt atggatatgg 480

tccaccagta ggacctcctg cttcatatgg cctttttttct tcttatggtc aacctggacc 540

aatgggcagt ccgatgggag gcatgggcta tggccctgga cctgagctag gccgatatgg 600

ttatggtttt agaggatctc caatgccggt ttctagccca tggtctggtg gagcattagt 660

ggaaaataat gacagctctg cttcacgcaa gcgtcgtgga ggcccagatg gaatggctga 720

gaatgactgg atctgcccaa agtgtgagaa tgtcaacttt tccttcagaa acagttgcaa 780

tatgaagaaa tgtggagctc caaggccaag ccctggatct aatgctaccc catgtcgcaa 840

agacaaggac gctccggaag ggagctggac ctgcccggag tgcaacaacc tgaactaccc 900

tttccgcacg gcgtgcaatc ggaaaggctg cggaagcagc aggccggcag cggccacggc 960

gaactaggac cctactaact ttgcgccggc gattgggggc agagagagta ttgttgtatc 1020

ctagctatat acaagttaat ttaaactgta aacggctcaa atatattttc ttgtggcc 1078

<210> 308
<211> 277
<212> PRT
<213> Oryza sativa

<400> 308

Met Ala Ser Ala Lys Val Glu Asn Arg Gly Gly Gly Gly Phe Gly Ser

```
1               5                   10                  15

Lys Arg Ser Arg Asn Asp Val Ser Val Arg Glu Gly Asp Trp Thr Cys
            20                  25                  30

Pro Gln Cys Gly Asn Val Asn Phe Ser Phe Arg Asn Val Cys Asn Arg
        35                  40                  45

Gly Ala Cys Gly Ala Pro Arg Pro Ser Pro Ser Leu Ser Pro Arg Val
    50                  55                  60

Pro Pro Pro Pro Ala Ala Gly Tyr Asp Arg Pro His Leu Gly Tyr Asp
65                  70                  75                  80

Arg Pro His Leu Phe Tyr Gly Ser Ala Gly Thr Pro Pro Pro Ile Pro
                85                  90                  95

Leu Gly Ser Gly Ser Tyr Gly Ala Pro Tyr Pro His Leu Gly Leu Arg
            100                 105                 110

Tyr Gly Tyr Gly Pro Pro Val Gly Pro Pro Ala Ser Tyr Gly Leu Phe
        115                 120                 125

Ser Ser Tyr Gly Gln Pro Gly Pro Met Gly Ser Pro Met Gly Gly Met
    130                 135                 140

Gly Tyr Gly Pro Gly Pro Glu Leu Gly Arg Tyr Gly Tyr Gly Phe Arg
145                 150                 155                 160

Gly Ser Pro Met Pro Val Ser Ser Pro Trp Ser Gly Gly Ala Leu Val
                165                 170                 175

Glu Asn Asn Asp Ser Ser Ala Ser Arg Lys Arg Arg Gly Gly Pro Asp
            180                 185                 190

Gly Met Ala Glu Asn Asp Trp Ile Cys Pro Lys Cys Glu Asn Val Asn
        195                 200                 205

Phe Ser Phe Arg Asn Ser Cys Asn Met Lys Lys Cys Gly Ala Pro Arg
    210                 215                 220

Pro Ser Pro Gly Ser Asn Ala Thr Pro Cys Arg Lys Asp Lys Asp Ala
225                 230                 235                 240

Pro Glu Gly Ser Trp Thr Cys Pro Glu Cys Asn Asn Leu Asn Tyr Pro
                245                 250                 255

Phe Arg Thr Ala Cys Asn Arg Lys Gly Cys Gly Ser Ser Arg Pro Ala
            260                 265                 270
```

Ala Ala Thr Ala Asn
275

<210> 309
<211> 662
<212> DNA
<213> Brassica rapa

<400> 309
ggtcttgttt tctttcaata aacacataat aagatagctt ccaagttcat caagaaataa 60

acgtaagcgc acgcatacaa taacctatcg aagatgagca gacccggaga ctggaactgt 120

agatcatgca cccacctcaa cttccagcgc cgtgattctt gccagcgatg cggtgactcc 180

cgtttgggtg caggtggagt cggtggctta gagtttggtg atttcggcgg cagaggtatg 240

tctgcttttg gattcaccac gggctccgac gttcgtccag gtgactggta ctgcacagtt 300

ggaaactgcg ggacacataa ctttgccagc cgctccacct gcttcaaatg cggcactttc 360

aaggacgaat ccctcggtgg gggcggcggc ggtggcgtag gcgtaaggcg gtccggtcat 420

gttgacgctg acgttatgcg gtctagagtc tccggcaacg gtggccgctc cagctggaaa 480

tccggtgatt ggatttgcac caggcttggt tgcaatgagc ataactttgc aagcagaatg 540

gagtgcttca gatgcaatgc accaagggac ttcagcatga gaacctcttt ctaagttaca 600

caataatgcc tttgaagtag ccttgtttcc aatctcttgg gcaccgtttc aatcaatgga 660

aa 662

<210> 310
<211> 166
<212> PRT
<213> Brassica rapa

<400> 310

Met Ser Arg Pro Gly Asp Trp Asn Cys Arg Ser Cys Thr His Leu Asn
1 5 10 15

Phe Gln Arg Arg Asp Ser Cys Gln Arg Cys Gly Asp Ser Arg Leu Gly
20 25 30

Ala Gly Gly Val Gly Gly Leu Glu Phe Gly Asp Phe Gly Gly Arg Gly
35 40 45

Met Ser Ala Phe Gly Phe Thr Thr Gly Ser Asp Val Arg Pro Gly Asp
50 55 60

Trp Tyr Cys Thr Val Gly Asn Cys Gly Thr His Asn Phe Ala Ser Arg
65 70 75 80

Ser Thr Cys Phe Lys Cys Gly Thr Phe Lys Asp Glu Ser Leu Gly Gly

85 90 95

Gly Gly Gly Gly Gly Val Gly Val Arg Arg Ser Gly His Val Asp Ala
100 105 110

Asp Val Met Arg Ser Arg Val Ser Gly Asn Gly Gly Arg Ser Ser Trp
115 120 125

Lys Ser Gly Asp Trp Ile Cys Thr Arg Leu Gly Cys Asn Glu His Asn
130 135 140

Phe Ala Ser Arg Met Glu Cys Phe Arg Cys Asn Ala Pro Arg Asp Phe
145 150 155 160

Ser Met Arg Thr Ser Phe
165

<210> 311
<211> 749
<212> DNA
<213> Euphorbia esula

<400> 311
atttccctt cttcacccaa atatctctca ccttttaaca agagaaaagc aaaaagaaga 60

tgagcagacc aggagattgg aactgcaggt cctgccagca tctcaacttc cagagaaggg 120

actcgtgcca gcgctgtggg gactccaggt ccgggactgg aggttcagga gctgactttg 180

gcgggtttgg aagccgggtt gggtcctcat tcgggttcag caccgggtct gatgttcgac 240

ccggtgactg gtactgtact gctggcaact gtggggccca caactttgcc agccgggcaa 300

gttgcttcaa atgtggagtt tataaggatg attctggggc ccctggcggg tttgattccg 360

atatcctccg ggcctctaga ggttttggta gtggcagcaa tcgctcttct tggaaatctg 420

gtgattggat ctgcactcgg tggggatgta atgaacacaa ctttgcaagc agaatggagt 480

gtttcaaatg cagtgcccct agggacctta gtaacagaac ttcatactag acattttgga 540

tggtgcagca gccaagaaag agaattaaga ttactacttt taaattttat tttattttta 600

ctatttgttt tgggtgttat tagagtggaa aacaaacatg gcttaaaaac ccatgtttca 660

tccctaattt aagctaagga agcaaacccc ttttggcatt ttgtaaggca gatttaggat 720

tgtagtactt aattaattag tgggttgtt 749

<210> 312
<211> 156
<212> PRT
<213> Euphorbia esula

<400> 312

Met Ser Arg Pro Gly Asp Trp Asn Cys Arg Ser Cys Gln His Leu Asn
1 5 10 15

Phe Gln Arg Arg Asp Ser Cys Gln Arg Cys Gly Asp Ser Arg Ser Gly
20 25 30

Thr Gly Gly Ser Gly Ala Asp Phe Gly Gly Phe Gly Ser Arg Val Gly
35 40 45

Ser Ser Phe Gly Phe Ser Thr Gly Ser Asp Val Arg Pro Gly Asp Trp
50 55 60

Tyr Cys Thr Ala Gly Asn Cys Gly Ala His Asn Phe Ala Ser Arg Ala
65 70 75 80

Ser Cys Phe Lys Cys Gly Val Tyr Lys Asp Asp Ser Gly Ala Pro Gly
85 90 95

Gly Phe Asp Ser Asp Ile Leu Arg Ala Ser Arg Gly Phe Gly Ser Gly
100 105 110

Ser Asn Arg Ser Ser Trp Lys Ser Gly Asp Trp Ile Cys Thr Arg Trp
115 120 125

Gly Cys Asn Glu His Asn Phe Ala Ser Arg Met Glu Cys Phe Lys Cys
130 135 140

Ser Ala Pro Arg Asp Leu Ser Asn Arg Thr Ser Tyr
145 150 155

<210> 313
<211> 646
<212> DNA
<213> Gossypium hirsutum

<400> 313
ctccattctc ttatttaact caaactcacc accttttcca tttttctaat taagaaaaga 60
aaagatgagc aggccaggag attggaactg caggtcgtgc caacacctaa acttccaaag 120
gagggactcc tgccaacgct gcggggaatt ccggtcgggt gatcacttcg gtagctacgg 180
tggtggcagg ggtggctcct cctttggatt cgccaccggc tccgacgtcc gacctggtga 240
ttggtactgc actgcgggaa actgcggtac ccacaatttc gccagtcgtt ccagctgctt 300
caaatgtggt gcattcaagg acgaccctgc cggaggtttc gacagcgacg ttccgcgttc 360
tagaggattt ggcggcggta atcgatccgg ctggaaatcc ggcgactgga tatgtaccag 420
gtcgggatgc aatgagcata actttgctag ccgaatggaa tgtttcagat gcagtgcccc 480
aagagacttc accgctagaa cttcatacta aatacaagct atccagtttt gggtgttgca 540
aaccaagaga gactcaaatg agagaaaaaa ttttacgggg ttagggttac ccgggtaaat 600
atatggcttg ggggggtgtg gcaccagggt tgaaaacacc agggtg 646

<210> 314
<211> 148
<212> PRT
<213> Gossypium hirsutum

<400> 314

```
Met Ser Arg Pro Gly Asp Trp Asn Cys Arg Ser Cys Gln His Leu Asn
1               5                   10                  15

Phe Gln Arg Arg Asp Ser Cys Gln Arg Cys Gly Glu Phe Arg Ser Gly
            20                  25                  30

Asp His Phe Gly Ser Tyr Gly Gly Gly Arg Gly Gly Ser Ser Phe Gly
        35                  40                  45

Phe Ala Thr Gly Ser Asp Val Arg Pro Gly Asp Trp Tyr Cys Thr Ala
    50                  55                  60

Gly Asn Cys Gly Thr His Asn Phe Ala Ser Arg Ser Ser Cys Phe Lys
65                  70                  75                  80

Cys Gly Ala Phe Lys Asp Asp Pro Ala Gly Gly Phe Asp Ser Asp Val
                85                  90                  95

Pro Arg Ser Arg Gly Phe Gly Gly Gly Asn Arg Ser Gly Trp Lys Ser
            100                 105                 110

Gly Asp Trp Ile Cys Thr Arg Ser Gly Cys Asn Glu His Asn Phe Ala
        115                 120                 125

Ser Arg Met Glu Cys Phe Arg Cys Ser Ala Pro Arg Asp Phe Thr Ala
    130                 135                 140

Arg Thr Ser Tyr
145
```

<210> 315
<211> 613
<212> DNA
<213> Vitis vinifera

<400> 315

```
ggtcttcttc catctctgca cagttatcct gatatttcct tggaaaacat gagcaggcca     60

ggagattgga actgcaggtc atgccagcac atgaacttcc aaaggcgcga ttcctgccaa    120

cgctgcggtg acccaaaatc gggtgggggt gactttggaa gctttggtgg gaggggtgga    180

tcctcctttg ggttcacggg ctcggatgtc cgcccagggg actggtactg caatgcaggc    240

aactgtggag ctcacaactt tgctagccgc tctaactgct tcaagtgtgg tgcattcaaa    300
```

gatgagtctg ctgggggcta cgattccgac atgtcacgct cccgaggttt cgggttcggc 360

ggtggcagcg gccggtctgg gtggaaatcc ggtgattgga tatgcagcag gtctggatgc 420

aatgagcaca actttgctag cagaatggaa tgtttcagat gcaatgcccc gagggacttg 480

agtaacaaaa cttcatacta gacatatatc ttccattttt gggtactgca gccagccaag 540

agagaccaaa tcatagaata tctattaatc cttgtgttgt tattaatttc tttgctttgg 600

gtgctaggtt ctt 613

<210> 316
<211> 150
<212> PRT
<213> Vitis vinifera

<400> 316

Met Ser Arg Pro Gly Asp Trp Asn Cys Arg Ser Cys Gln His Met Asn
1 5 10 15

Phe Gln Arg Arg Asp Ser Cys Gln Arg Cys Gly Asp Pro Lys Ser Gly
20 25 30

Gly Gly Asp Phe Gly Ser Phe Gly Gly Arg Gly Gly Ser Ser Phe Gly
35 40 45

Phe Thr Gly Ser Asp Val Arg Pro Gly Asp Trp Tyr Cys Asn Ala Gly
50 55 60

Asn Cys Gly Ala His Asn Phe Ala Ser Arg Ser Asn Cys Phe Lys Cys
65 70 75 80

Gly Ala Phe Lys Asp Glu Ser Ala Gly Gly Tyr Asp Ser Asp Met Ser
85 90 95

Arg Ser Arg Gly Phe Gly Phe Gly Gly Gly Ser Gly Arg Ser Gly Trp
100 105 110

Lys Ser Gly Asp Trp Ile Cys Ser Arg Ser Gly Cys Asn Glu His Asn
115 120 125

Phe Ala Ser Arg Met Glu Cys Phe Arg Cys Asn Ala Pro Arg Asp Leu
130 135 140

Ser Asn Lys Thr Ser Tyr
145 150

<210> 317
<211> 844
<212> DNA
<213> Populus trichocarpa

<400> 317
tcagaccatt tccctttcat cttctagcta gcatagcttc tctcagaggt ttttgaaacc 60

cctttttgcc attcttctct cacttagtat acttagtctt ctctaatcaa ttcataagca 120

aatatgaaca ggccaggaga ctggaactgc aggtcatgcc aacacctcaa tttccagagg 180

cgtgactctt gccaacgttg tggggacccc aggtccgcag gtgattttgg gggtttcggt 240

gggcggggtg gctcatcact tgggttcacc gggtcggatg ttcgtcccgg tgattggtac 300

tgcactgccg gaaactgcgg ggcccacaac tttgctagcc gttctagttg cttcaaatgt 360

ggagtgtaca aggaaatgga ctccgccggg ggcttcgatt ctgatttttc tcgaactaga 420

gggtttggtg ggagcactgg aggtggcaat cgatctggat ggaaatccgg agactggatt 480

tgcactaggt ggggatgcaa cgaacataac tttgctagca gaatggagtg cttcaagtgc 540

aatgccccaa gagatcttag caacagaact tcatactaga tacaatttct ccatttcctg 600

ggactgcacc agccaagaac aaagacaaca tgattaaaaa atatatatca ctacttttca 660

ttttcttctt gatttctttt tgtattagct agctagggtc ttgaggcgag acatggttta 720

agaaccatgt ttactgcttt gagctatata taacccttgg cattttgtca ggcttcctgt 780

aggaagtata tcgttgtctt tgtgggcttt tgatctatta tattcattat tgtaaccaag 840

ggag 844

<210> 318
<211> 151
<212> PRT
<213> Populus trichocarpa

<400> 318

Met Asn Arg Pro Gly Asp Trp Asn Cys Arg Ser Cys Gln His Leu Asn
1 5 10 15

Phe Gln Arg Arg Asp Ser Cys Gln Arg Cys Gly Asp Pro Arg Ser Ala
20 25 30

Gly Asp Phe Gly Gly Phe Gly Gly Arg Gly Gly Ser Ser Leu Gly Phe
35 40 45

Thr Gly Ser Asp Val Arg Pro Gly Asp Trp Tyr Cys Thr Ala Gly Asn
50 55 60

Cys Gly Ala His Asn Phe Ala Ser Arg Ser Ser Cys Phe Lys Cys Gly
65 70 75 80

Val Tyr Lys Glu Met Asp Ser Ala Gly Gly Phe Asp Ser Asp Phe Ser
85 90 95

Arg Thr Arg Gly Phe Gly Gly Ser Thr Gly Gly Gly Asn Arg Ser Gly
100 105 110

Trp Lys Ser Gly Asp Trp Ile Cys Thr Arg Trp Gly Cys Asn Glu His
        115                 120                 125

Asn Phe Ala Ser Arg Met Glu Cys Phe Lys Cys Asn Ala Pro Arg Asp
    130                 135                 140

Leu Ser Asn Arg Thr Ser Tyr
145                 150

<210> 319
<211> 785
<212> DNA
<213> Gossypium hirsutum

<400> 319
ccttgcgtcc gctttcttct tcattgttcc tgtctttgct tactcacccc ttatatatct 60

gctttctcaa gtaggggaga aaatatgagc aggccaggag actggaattg caggtcatgc 120

caacacctca acttccaaag gagggactca tgccagcgct gtggagaacc aagacctggt 180

ggtggtgaca gaggcggcga ctatggaagc tttggtggca ggggtggctc atctttcggg 240

tttactggac ccgatgttag gcctggtgac tggtattgca ctgtgggcaa ctgcggtgct 300

cacaacttcg ccagcaggtc gagctgcttc aaatgtggtg cggccaaaga tgaatcatcc 360

ggaggatttg aaagtgacat cccacgtatg aggggttatg gttttagcac tggcagctct 420

agtcgctcta actggaaatc tggagactgg atttgcacca ggtcgggttg caatgaacac 480

aacttcgcca gcaggatgga atgtttcaga tgcaatgcac caagggactc cacccacaaa 540

tcttcatact aattaataaa attttcattt ttgggtactg cagtcgcaag agagagatca 600

gacaaggcaa tcccagatct tgctttcttt ttcttttgtt tgtttcttta ttttagatct 660

ttagatgaat catgtttgaa agacttcagt tgaagtactt aagctaatat caaagtacat 720

agggcatgca tgtaattgat gtatggtatc agcaatgtta tatcagtgtc tttgtgccaa 780

aaaaa 785

<210> 320
<211> 155
<212> PRT
<213> Gossypium hirsutum

<400> 320

Met Ser Arg Pro Gly Asp Trp Asn Cys Arg Ser Cys Gln His Leu Asn
1               5                   10                  15

Phe Gln Arg Arg Asp Ser Cys Gln Arg Cys Gly Glu Pro Arg Pro Gly
            20                  25                  30

Gly Gly Asp Arg Gly Gly Asp Tyr Gly Ser Phe Gly Gly Arg Gly Gly

35 40 45

Ser Ser Phe Gly Phe Thr Gly Pro Asp Val Arg Pro Gly Asp Trp Tyr
50 55 60

Cys Thr Val Gly Asn Cys Gly Ala His Asn Phe Ala Ser Arg Ser Ser
65 70 75 80

Cys Phe Lys Cys Gly Ala Ala Lys Asp Glu Ser Ser Gly Gly Phe Glu
85 90 95

Ser Asp Ile Pro Arg Met Arg Gly Tyr Gly Phe Ser Thr Gly Ser Ser
100 105 110

Ser Arg Ser Asn Trp Lys Ser Gly Asp Trp Ile Cys Thr Arg Ser Gly
115 120 125

Cys Asn Glu His Asn Phe Ala Ser Arg Met Glu Cys Phe Arg Cys Asn
130 135 140

Ala Pro Arg Asp Ser Thr His Lys Ser Ser Tyr
145 150 155

<210> 321
<211> 760
<212> DNA
<213> Lactuca serriola

<400> 321
gtattcggaa tcctcaaaga aacacacact tcctatccat cacaagaaag atgagcaggc 60

caggagattg gaactgcagg tcatgccagc acttgaactt ccagaggagg gactcttgcc 120

aaagatgtgg ggagacgagg tatggtggtg ggggtggtgt gtttggtggt agaggaagta 180

tcatcagccc ttcagcattt ggcttcacag gcccagatgt ccgaccgggt gattggtact 240

gcaatgttgg caactgcggg gctcacaact ttgctagccg ctcgagctgc ttcaagtgtg 300

gtgcgttcaa ggatgactta gcttgtagtg gtggtggtgg tggtggtggt ggcgtttttg 360

atggtgatat gtcacgtggc aggggtttcg ggtttggtgg aggaagtggt ggtggaggtg 420

gtggcagcag ccgttcaggg tggaagtccg gtgactggat atgcggcagg cctggttgca 480

atgagcacaa ctttgcaagc agaatggaat gttttaggtg caacgcacct cgggaatcgg 540

gtaacaagtc tccttattaa gcaggttgcg gcatttccag ttccgggtat catggacttg 600

ctatctacca aacaggaaga gagataagtg atcgatcaga cagaatcatg aagagcatcc 660

agttgatagg aaattctaaa ctgacccccct ttttgcccat atcataatat gcaatcctat 720

ctttgatttt ttttttcctt ttgttttatt tttttttttt 760

<210> 322

<211> 169
<212> PRT
<213> Lactuca serriola

<400> 322

Met Ser Arg Pro Gly Asp Trp Asn Cys Arg Ser Cys Gln His Leu Asn
1 5 10 15

Phe Gln Arg Arg Asp Ser Cys Gln Arg Cys Gly Glu Thr Arg Tyr Gly
20 25 30

Gly Gly Gly Gly Val Phe Gly Gly Arg Gly Ser Ile Ile Ser Pro Ser
35 40 45

Ala Phe Gly Phe Thr Gly Pro Asp Val Arg Pro Gly Asp Trp Tyr Cys
50 55 60

Asn Val Gly Asn Cys Gly Ala His Asn Phe Ala Ser Arg Ser Ser Cys
65 70 75 80

Phe Lys Cys Gly Ala Phe Lys Asp Asp Leu Ala Cys Ser Gly Gly Gly
85 90 95

Gly Gly Gly Gly Gly Val Phe Asp Gly Asp Met Ser Arg Gly Arg Gly
100 105 110

Phe Gly Phe Gly Gly Gly Ser Gly Gly Gly Gly Gly Gly Ser Ser Arg
115 120 125

Ser Gly Trp Lys Ser Gly Asp Trp Ile Cys Gly Arg Pro Gly Cys Asn
130 135 140

Glu His Asn Phe Ala Ser Arg Met Glu Cys Phe Arg Cys Asn Ala Pro
145 150 155 160

Arg Glu Ser Gly Asn Lys Ser Pro Tyr
165

<210> 323
<211> 517
<212> DNA
<213> Glycine max

<400> 323
tttctgcatc tctaatactt caactcactg tattatcttg agtaattttg cagagaagta 60

aaatatgagc aggccaggag actggaattg caggtcgtgc cagcacctga actttcagag 120

gagagactca tgccagcgat gtggggactc aaaatatgga gatagagttg ttgattttgg 180

tggttttgga ggaagaggag ggtcctcatt tggtttaact ggctcagatg ttcgccccgg 240

cgactggtac tgtgctgctg ctaactgtgg tgcacacaac tttgctagcc gctcaagctg 300

cttcaagtgt ggtgctttca aggatgactt ggctggagga ggctataaca gttctgacat 360

cttgcgctcc agagcttttg gtggcagtgg aagacctgga tggaaatctg gtgattggat 420

atgcagcaga tcaggatgca atgagcacaa ctttgctagc agaatggaat gttttaaatg 480

cagtgctccc agggacacgt actagaaaaa atgagtt 517

<210> 324
<211> 146
<212> PRT
<213> Glycine max

<400> 324

Met Ser Arg Pro Gly Asp Trp Asn Cys Arg Ser Cys Gln His Leu Asn
1 5 10 15

Phe Gln Arg Arg Asp Ser Cys Gln Arg Cys Gly Asp Ser Lys Tyr Gly
20 25 30

Asp Arg Val Val Asp Phe Gly Gly Phe Gly Gly Arg Gly Gly Ser Ser
35 40 45

Phe Gly Leu Thr Gly Ser Asp Val Arg Pro Gly Asp Trp Tyr Cys Ala
50 55 60

Ala Ala Asn Cys Gly Ala His Asn Phe Ala Ser Arg Ser Ser Cys Phe
65 70 75 80

Lys Cys Gly Ala Phe Lys Asp Asp Leu Ala Gly Gly Gly Tyr Asn Ser
85 90 95

Ser Asp Ile Leu Arg Ser Arg Ala Phe Gly Gly Ser Gly Arg Pro Gly
100 105 110

Trp Lys Ser Gly Asp Trp Ile Cys Ser Arg Ser Gly Cys Asn Glu His
115 120 125

Asn Phe Ala Ser Arg Met Glu Cys Phe Lys Cys Ser Ala Pro Arg Asp
130 135 140

Thr Tyr
145

<210> 325
<211> 889
<212> DNA
<213> Populus trichocarpa

<400> 325
gacaagttcc ttaaacaatc tattgctctt tcaactacta gctaggttac acttcatttc 60

```
tgtacgtgtt aattctccta tctttctcct ttctgctttc tcgagatgag cagaccagga     120

gattggaatt gcaggtcatg ccagcacttg aactttcaaa ggagggactc atgccagcgt     180

tgcggtgatc caaggcccgg agagagagat cactatggaa gtttcggtgg aagatcatca     240

gggggctcat tcggatttac gggccctgat gttaggcctg gtgattggta ttgcacggct     300

ggcaattgtg gagctcacaa ctttgctagt cgttcaagct gcttcaagtg tggtgtgtcc     360

aaggatgaat cctctggtgg tggacttgat gctgatatgt cacggatgag aggttatggc     420

ttcggcggag gcggaggcgg aggcagtggc tctagccgta attggaaatc cggagactgg     480

atttgcacca ggtccggttg caacgagcac aactttgcta gcaggactga atgctataga     540

tgcaatgcac caagagaatc tagcagcaac aagtcttcgt attaatcatc gatatcgata     600

gcatttttgt gtcctgcagt gctgcaagga gggaattaac gaagaaggct catgagaaat     660

cttggattca tctttttgct cttaattact tctatttttg ttcttttgga tagctgtact     720

cttaagctga gaagctttag aaggatcatg ggagtgaaat taagttcaag gagagctata     780

tatatatcat tagccagtaa tttgtcaggt tccctaaata tagacatgta gttctgtact     840

tggtatcaat gtctttgtgt tttgagacgg gtttaagccc ttgtcgttc                 889


<210>  326
<211>  159
<212>  PRT
<213>  Populus trichocarpa

<400>  326

Met Ser Arg Pro Gly Asp Trp Asn Cys Arg Ser Cys Gln His Leu Asn
1               5                   10                  15


Phe Gln Arg Arg Asp Ser Cys Gln Arg Cys Gly Asp Pro Arg Pro Gly
            20                  25                  30


Glu Arg Asp His Tyr Gly Ser Phe Gly Gly Arg Ser Ser Gly Gly Ser
        35                  40                  45


Phe Gly Phe Thr Gly Pro Asp Val Arg Pro Gly Asp Trp Tyr Cys Thr
    50                  55                  60


Ala Gly Asn Cys Gly Ala His Asn Phe Ala Ser Arg Ser Ser Cys Phe
65                  70                  75                  80


Lys Cys Gly Val Ser Lys Asp Glu Ser Ser Gly Gly Gly Leu Asp Ala
                85                  90                  95


Asp Met Ser Arg Met Arg Gly Tyr Gly Phe Gly Gly Gly Gly Gly Gly
            100                 105                 110


Gly Ser Gly Ser Ser Arg Asn Trp Lys Ser Gly Asp Trp Ile Cys Thr
```

115 120 125

Arg Ser Gly Cys Asn Glu His Asn Phe Ala Ser Arg Thr Glu Cys Tyr
130 135 140

Arg Cys Asn Ala Pro Arg Glu Ser Ser Ser Asn Lys Ser Ser Tyr
145 150 155

<210> 327
<211> 723
<212> DNA
<213> Populus fremontii x Populus angustifolia

<400> 327
agggaaaaag aaagcctaac cctcctgcct gcctcaagct taccccatta tcgaggtcta 60

aaacaagtat aaaaacagcc ttagccccaa tacgtaaatc acaagttcct taaacaatct 120

attgctcttt caactactag gtttcccact tcatttctgt gcctactgat tctcctctct 180

ttcaactttc tgccttctca agctagctag agaagggaag atgagcagac caggagattg 240

gaattgcagg tcatgccaac acttgaactt ccagaggagg gactcgtgcc agcgttgtgg 300

ggacccaagg cccggagaga gagatcatta tggaagtttt ggtggaagat caggggggctc 360

gttcggattt acggggcctg atgttaggcc cggtgactgg tattgctcgg ttggcaactg 420

tggagctcac aactttgcta gtcgttcaag ctgcttcaag tgtggtatgt ccaaggatga 480

atcctctggt ggtgggcttg atgctgacat ttcatggatg agaggttatg gcttcggcgg 540

aggcagcgcc tctagccgct ctaattggaa atccggagac tggatttgca ccaggtcagg 600

ttgcaacgag cacaactttg ctagcaggac tgagtgttac agatgcaatg caccaagaga 660

atcaggcagc aacaagtctt cgtattaatc atcaacatcg atcgcatttt tgtgtactgc 720

agt 723

<210> 328
<211> 155
<212> PRT
<213> Populus fremontii x Populus angustifolia

<400> 328

Met Ser Arg Pro Gly Asp Trp Asn Cys Arg Ser Cys Gln His Leu Asn
1 5 10 15

Phe Gln Arg Arg Asp Ser Cys Gln Arg Cys Gly Asp Pro Arg Pro Gly
20 25 30

Glu Arg Asp His Tyr Gly Ser Phe Gly Gly Arg Ser Gly Gly Ser Phe
35 40 45

Gly Phe Thr Gly Pro Asp Val Arg Pro Gly Asp Trp Tyr Cys Ser Val
50 55 60

Gly Asn Cys Gly Ala His Asn Phe Ala Ser Arg Ser Ser Cys Phe Lys
65 70 75 80

Cys Gly Met Ser Lys Asp Glu Ser Ser Gly Gly Gly Leu Asp Ala Asp
85 90 95

Ile Ser Trp Met Arg Gly Tyr Gly Phe Gly Gly Gly Ser Ala Ser Ser
100 105 110

Arg Ser Asn Trp Lys Ser Gly Asp Trp Ile Cys Thr Arg Ser Gly Cys
115 120 125

Asn Glu His Asn Phe Ala Ser Arg Thr Glu Cys Tyr Arg Cys Asn Ala
130 135 140

Pro Arg Glu Ser Gly Ser Asn Lys Ser Ser Tyr
145 150 155

<210> 329
<211> 920
<212> DNA
<213> Oryza sativa

<400> 329
cacctcacag cattttccac cttagagctc accaacacag cagctgatac ttgtttaggg 60

taagacaaga tgaacaggaa gccaggagac tgggactgca gggcgtgcca gcacctcaac 120

ttcagccgcc gggacctatg ccagcgctgc ggcgggccgc gtggcgccgc tgatcgcggc 180

agcggtggtg gcggtgacta cgccaacttc ggcggccgtg gtggttcctc cttcggtgga 240

ggctttggca ctggctctga tgtccgccca ggtgactggt actgcaactg cggcgcgcac 300

aacttcgcca gccgctccag ctgcttcaag tgcgctgctt tcaaggacga tgctgccgtc 360

aacagtggcg gcgctggtgc ctttgacggt ggggacatgt cgcgctcgcg gggctacggc 420

ttcggcagcg gcgccgtccg cgccagccgc cctggctgga agtctggcga ctggatttgc 480

accaggtctg gatgcaatga gcacaacttc gccagcagga tggagtgctt caggtgcaac 540

gcaccgcggg actccggcac tgaggtgtaa tttgccgtac gtgtccgatc gatctggatc 600

cgatgaggct tgcagcagtg acgacgagca gcagaagcag cgttaagagt tgtgatgtct 660

acataagaag aagaagaaag tagaatgcaa aagaaatctc cccatggttt tactagtttt 720

gtttcttccc gttttagatt tggttctgat tcccatttgg gaggacccgt cgacccctga 780

ttatctatgt tttacccgtt ttatttcctg tttctttcgg catgtttgct cttcgatcga 840

gtcgtgtaac ccgaaacgct tgcgcttgag aagtattatt attattaact agtatgttgc 900

ttcttaaaaa aaaaaaaaaa 920

<210> 330
<211> 166
<212> PRT
<213> Oryza sativa

<400> 330

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Gly Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Thr Gly Ser Asp
50 55 60

Val Arg Pro Gly Asp Trp Tyr Cys Asn Cys Gly Ala His Asn Phe Ala
65 70 75 80

Ser Arg Ser Ser Cys Phe Lys Cys Ala Ala Phe Lys Asp Asp Ala Ala
85 90 95

Val Asn Ser Gly Gly Ala Gly Ala Phe Asp Gly Gly Asp Met Ser Arg
100 105 110

Ser Arg Gly Tyr Gly Phe Gly Ser Gly Ala Val Arg Ala Ser Arg Pro
115 120 125

Gly Trp Lys Ser Gly Asp Trp Ile Cys Thr Arg Ser Gly Cys Asn Glu
130 135 140

His Asn Phe Ala Ser Arg Met Glu Cys Phe Arg Cys Asn Ala Pro Arg
145 150 155 160

Asp Ser Gly Thr Glu Val
165

<210> 331
<211> 703
<212> DNA
<213> Panicum virgatum

<400> 331
ccacgcgtcc gcagaactcc ttgtcctagg ccccagaaac acaaacgacc gctctttgtt 60

taggcaagat gaacaggaag cctggagact gggattgcag ggcttgccaa cacctcaact 120

tcagtcggcg ggacctatgc cagcgctgtg gtgagccacg tggagctgct gaccgtggca 180

gcggcggtgg aggtgactat gccaacttcg gtggccgtgg tggctcctcc ttcggcggtg 240

gctttggtgc tggctctgat gtccgccctg gtgactggtt atgttcctgc ggcgcgcaca 300

acttcgccag ccgctccaac tgcttcaagt gctctgcctt caaggaggag gctgctgtca 360

acagtggtgc tggtggcttt gatggtgaca tgtcacgctc gcgctacggc ttcggtggcg 420

gtgctgcccg caccaaccgc cctggttgga agtctggaga ctggatctgc accaggtccg 480

gatgcaacga gcacaacttt gccagcagga tggagtgttt caggtgcaac gcaccacggg 540

actccggcac tgaggtgtag gatcggagct ggtgcttcga acggaccccg acgagcagaa 600

gcagcaagaa acctgataag aagagtagta gatttgcaaa aggcatcccg gatgctctat 660

tactgttttg ttccacccct accgcttcct tttagaattt ggt 703

<210> 332
<211> 163
<212> PRT
<213> Panicum virgatum

<400> 332

Met Asn Arg Lys Pro Gly Asp Trp Asp Cys Arg Ala Cys Gln His Leu
1 5 10 15

Asn Phe Ser Arg Arg Asp Leu Cys Gln Arg Cys Gly Glu Pro Arg Gly
20 25 30

Ala Ala Asp Arg Gly Ser Gly Gly Gly Gly Asp Tyr Ala Asn Phe Gly
35 40 45

Gly Arg Gly Gly Ser Ser Phe Gly Gly Gly Phe Gly Ala Gly Ser Asp
50 55 60

Val Arg Pro Gly Asp Trp Leu Cys Ser Cys Gly Ala His Asn Phe Ala
65 70 75 80

Ser Arg Ser Asn Cys Phe Lys Cys Ser Ala Phe Lys Glu Glu Ala Ala
85 90 95

Val Asn Ser Gly Ala Gly Gly Phe Asp Gly Asp Met Ser Arg Ser Arg
100 105 110

Tyr Gly Phe Gly Gly Gly Ala Ala Arg Thr Asn Arg Pro Gly Trp Lys
115 120 125

Ser Gly Asp Trp Ile Cys Thr Arg Ser Gly Cys Asn Glu His Asn Phe
130 135 140

Ala Ser Arg Met Glu Cys Phe Arg Cys Asn Ala Pro Arg Asp Ser Gly
145 150 155 160

Thr Glu Val

<210> 333
<211> 435
<212> DNA
<213> Medicago truncatula

<400> 333
atgaacagga aaatgagctg gtctggagga gattggatgt gtggtgcttg cgagcacata 60

aatttcaaga agagagaagc atgccaaaat tgtggatacc caaagtatgg aggccctgac 120

ccatcgacct atagatataa caggactgaa acgttggcag gggactggtt ttgcacttct 180

atgaactgtg gagctcacaa ctatgcaagc cgatcaaact gctatagatg tggtgcattt 240

aaagatcctt attcttctgg atatgggggt aacatggtgg gttctggagg atatggatca 300

gattgtagtt ctcccccagg atggaaaagt ggagactgga tttgccctag aattggctgt 360

ggaatccata attatgcaag caggacagag tgctacaaat gcaaaatgcc aagggattat 420

ggtggtgcag actga 435

<210> 334
<211> 144
<212> PRT
<213> Medicago truncatula

<400> 334

Met Asn Arg Lys Met Ser Trp Ser Gly Gly Asp Trp Met Cys Gly Ala
1 5 10 15

Cys Glu His Ile Asn Phe Lys Lys Arg Glu Ala Cys Gln Asn Cys Gly
20 25 30

Tyr Pro Lys Tyr Gly Gly Pro Asp Pro Ser Thr Tyr Arg Tyr Asn Arg
35 40 45

Thr Glu Thr Leu Ala Gly Asp Trp Phe Cys Thr Ser Met Asn Cys Gly
50 55 60

Ala His Asn Tyr Ala Ser Arg Ser Asn Cys Tyr Arg Cys Gly Ala Phe
65 70 75 80

Lys Asp Pro Tyr Ser Ser Gly Tyr Gly Gly Asn Met Val Gly Ser Gly
85 90 95

Gly Tyr Gly Ser Asp Cys Ser Ser Pro Pro Gly Trp Lys Ser Gly Asp
100 105 110

Trp Ile Cys Pro Arg Ile Gly Cys Gly Ile His Asn Tyr Ala Ser Arg
115 120 125

Thr Glu Cys Tyr Lys Cys Lys Met Pro Arg Asp Tyr Gly Gly Ala Asp
    130                 135                 140

<210> 335
<211> 513
<212> DNA
<213> Medicago truncatula

<400> 335
atgagcagac caggagattg gaactgcagg acatgcaacc acctcaactt tcaaagaaga 60

gaatcttgcc aacgatgtgg ggagtcaaga atgacttctg gctgcggcgc cgttgatttt 120

ggtggctcct ttcttggtgg aagaggctct agctcccctt ttcctttcac caccggccct 180

gatgtccgtc ctggtgactg gtattgcact gttggaaact gtggagctca caactttgcc 240

agccgctcca gctgcttcaa atgtggtgcc cctaaggata ttgatacctt ctcctctgac 300

tcctcagaca tgccacgatt attgagatca ccatacggtt ttggagcagg cagcgctggt 360

ggcggtgcct ccactcgccc cggctggaaa tccggtgact ggatatgcac caggtctggg 420

tgtaacgagc ataacttcgc caatagaatg gaatgctacc gatgcaacgg tccaagggac 480

tctagtactg gaagatcttc ctatttatcg tga 513

<210> 336
<211> 170
<212> PRT
<213> Medicago truncatula

<400> 336

Met Ser Arg Pro Gly Asp Trp Asn Cys Arg Thr Cys Asn His Leu Asn
1               5                   10                  15

Phe Gln Arg Arg Glu Ser Cys Gln Arg Cys Gly Glu Ser Arg Met Thr
            20                  25                  30

Ser Gly Cys Gly Ala Val Asp Phe Gly Gly Ser Phe Leu Gly Gly Arg
        35                  40                  45

Gly Ser Ser Ser Pro Phe Pro Phe Thr Thr Gly Pro Asp Val Arg Pro
    50                  55                  60

Gly Asp Trp Tyr Cys Thr Val Gly Asn Cys Gly Ala His Asn Phe Ala
65                  70                  75                  80

Ser Arg Ser Ser Cys Phe Lys Cys Gly Ala Pro Lys Asp Ile Asp Thr
                85                  90                  95

Phe Ser Ser Asp Ser Ser Asp Met Pro Arg Leu Leu Arg Ser Pro Tyr
            100                 105                 110

Gly Phe Gly Ala Gly Ser Ala Gly Gly Gly Ala Ser Thr Arg Pro Gly

115 120 125

Trp Lys Ser Gly Asp Trp Ile Cys Thr Arg Ser Gly Cys Asn Glu His
130 135 140

Asn Phe Ala Asn Arg Met Glu Cys Tyr Arg Cys Asn Gly Pro Arg Asp
145 150 155 160

Ser Ser Thr Gly Arg Ser Ser Tyr Leu Ser
165 170

<210> 337
<211> 826
<212> DNA
<213> Yucca filamentosa

<400> 337
ggcacaagct ctcacgcaac cccttgtctc cattttactc ttccttcctc tttcaaggga 60

cttgagaaga tgaacaggaa gccaggagac tggaactgca ggtcatgcca gcaccttaat 120

ttcagccgca gggactcgtg ccagcgctgc ggcgaccccc ggtcgatcgg cagcgagcga 180

tcggactacc cgggcttcgt cgggggccgc gggggtcct cattcgggtt cagcggctcg 240

gacgtcaggc ccggggactg gtactgccag tgcggggccc acaacttcgc cagccgctcg 300

agctgcttca agtgcaatgc tttcaaggat gagtccgccg ctagcggcgg cctcgacggc 360

ggcgacatgc tgagatccag gggtttggc ttcggcggcg gcggcggcgc tcgcagtggc 420

tggaagtctg gtgactggat ttgcaacagg tctggctgca atgagcacaa cttcgctagc 480

aggatggaat gcttccgatg caatgcacct cgagattcgg gcactgaggt ttaaggagac 540

agccaagaga gaagtgacga gatgagatca gtgatgatga taccagtagt aatctcgagt 600

tattactagt tttgccacca gccccaactt tatctccttt tgatgttttt ttagagcccc 660

cttctaaacg gaggtgtctt ctctttttca tccgtttgtt tgcttttgaa agcttgcact 720

taccctttac acccccccgc ccccctccac cttgtagttt gtgttgagag tgtctttgta 780

agtctttgga gaagggctcc tttagcttct tgggggagtc ccttct 826

<210> 338
<211> 154
<212> PRT
<213> Yucca filamentosa

<400> 338

Met Asn Arg Lys Pro Gly Asp Trp Asn Cys Arg Ser Cys Gln His Leu
1 5 10 15

Asn Phe Ser Arg Arg Asp Ser Cys Gln Arg Cys Gly Asp Pro Arg Ser
20 25 30

Ile Gly Ser Glu Arg Ser Asp Tyr Pro Gly Phe Val Gly Gly Arg Gly
35 40 45

Gly Ser Ser Phe Gly Phe Ser Gly Ser Asp Val Arg Pro Gly Asp Trp
50 55 60

Tyr Cys Gln Cys Gly Ala His Asn Phe Ala Ser Arg Ser Ser Cys Phe
65 70 75 80

Lys Cys Asn Ala Phe Lys Asp Glu Ser Ala Ala Ser Gly Gly Leu Asp
85 90 95

Gly Gly Asp Met Leu Arg Ser Arg Gly Phe Gly Phe Gly Gly Gly Gly
100 105 110

Gly Ala Arg Ser Gly Trp Lys Ser Gly Asp Trp Ile Cys Asn Arg Ser
115 120 125

Gly Cys Asn Glu His Asn Phe Ala Ser Arg Met Glu Cys Phe Arg Cys
130 135 140

Asn Ala Pro Arg Asp Ser Gly Thr Glu Val
145 150

<210> 339
<211> 614
<212> DNA
<213> Hordeum vulgare

<400> 339
cggcacgagg cacttctcat cttggagctc agcagcacag catccaagct ttttcttcgg 60

caagatgaac aggaagccag gagactggga ctgcaggtcg tgccagcacc tcaacttcag 120

tcgccgggac ctatgccagc gctgcggtga gccacgtagt gccgctgacc gtggcagcgt 180

cggtggtgct cttggtggtg actacgccaa ctttggcggc cgtggcgggg gtggttcctc 240

atttggtgcc ggctttggtg ccggctctga cgtccgcccg ggtgactggt actgcacctg 300

tggagcgcac aacttcgcca gccgctccag ctgcttcaag tgtgctgctt tcaaggagga 360

agctgccgtc aatggtggcg ctggtggctt tgatggtgac atgtcacgct caaggggctt 420

tggctttggc gctgtcggtg gcatgggtgg cggcatggga gccggcgcag ccggtggtcg 480

tgccagtcgc cctggctgga agtctcgcga ctggatttgc accaggtctg gatgcaacga 540

gcacaacttc gccagcaggc aggagtgctt caggtgcaac gcgccgaggg actccggtag 600

cgccacacca tacg 614

<210> 340
<211> 183
<212> PRT
<213> Hordeum vulgare

<400> 340

Met Asn Arg Lys Pro Gly Asp Trp Asp Cys Arg Ser Cys Gln His Leu
1 5 10 15

Asn Phe Ser Arg Arg Asp Leu Cys Gln Arg Cys Gly Glu Pro Arg Ser
20 25 30

Ala Ala Asp Arg Gly Ser Val Gly Gly Ala Leu Gly Gly Asp Tyr Ala
35 40 45

Asn Phe Gly Gly Arg Gly Gly Gly Gly Ser Ser Phe Gly Ala Gly Phe
50 55 60

Gly Ala Gly Ser Asp Val Arg Pro Gly Asp Trp Tyr Cys Thr Cys Gly
65 70 75 80

Ala His Asn Phe Ala Ser Arg Ser Ser Cys Phe Lys Cys Ala Ala Phe
85 90 95

Lys Glu Glu Ala Ala Val Asn Gly Gly Ala Gly Gly Phe Asp Gly Asp
100 105 110

Met Ser Arg Ser Arg Gly Phe Gly Phe Gly Ala Val Gly Gly Met Gly
115 120 125

Gly Gly Met Gly Ala Gly Ala Ala Gly Gly Arg Ala Ser Arg Pro Gly
130 135 140

Trp Lys Ser Arg Asp Trp Ile Cys Thr Arg Ser Gly Cys Asn Glu His
145 150 155 160

Asn Phe Ala Ser Arg Gln Glu Cys Phe Arg Cys Asn Ala Pro Arg Asp
165 170 175

Ser Gly Ser Ala Thr Pro Tyr
180

<210> 341
<211> 54
<212> DNA
<213> Artificial sequence

<220>
<223> primer: prm5539

<400> 341
ggggacaagt ttgtacaaaa aagcaggctt aaacaatgag cagacccgga gatt 54

<210> 342
<211> 51

<212> DNA
<213> Artificial sequence

<220>
<223> primer: prm5540

<400> 342
ggggaccact ttgtacaaga aagctgggta gacaaggcta cttcaaaagc a 51

<210> 343
<211> 2193
<212> DNA
<213> Oryza sativa

<400> 343
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtaccttttg tttgaggtaa aatcagagca ccggtgattt tgcttggtgt 1440

aataaaagta cggttgtttg gtcctcgatt ctggtagtga tgcttctcga tttgacgaag 1500

```
ctatcctttg tttattccct attgaacaaa aataatccaa ctttgaagac ggtcccgttg   1560

atgagattga atgattgatt cttaagcctg tccaaaattt cgcagctggc ttgtttagat   1620

acagtagtcc ccatcacgaa attcatggaa acagttataa tcctcaggaa caggggattc   1680

cctgttcttc cgatttgctt tagtcccaga atttttttc ccaaatatct taaaaagtca   1740

ctttctggtt cagttcaatg aattgattgc tacaaataat gcttttatag cgttatccta   1800

gctgtagttc agttaatagg taatacccct atagtttagt caggagaaga acttatccga   1860

tttctgatct ccatttttaa ttatatgaaa tgaactgtag cataagcagt attcatttgg   1920

attatttttt ttattagctc tcaccccttc attattctga gctgaaagtc tggcatgaac   1980

tgtcctcaat tttgttttca aattcacatc gattatctat gcattatcct cttgtatcta   2040

cctgtagaag tttctttttg gttattcctt gactgcttga ttacagaaag aaatttatga   2100

agctgtaatc gggatagtta tactgcttgt tcttatgatt catttccttt gtgcagttct   2160

tggtgtagct tgccactttc accagcaaag ttc                                2193


<210>  344
<211>  3
<212>  PRT
<213>  Artificial sequence

<220>
<223>  sequence motif 1


<220>
<221>  VARIANT
<222>  (1)..(1)
<223>  /replace="Arg" /replace="Asp" /replace="Asn"

<400>  344

Gly Asp Trp
1


<210>  345
<211>  3
<212>  PRT
<213>  Artificial sequence

<220>
<223>  sequence motif 2

<400>  345

Gly Ser Trp
1


<210>  346
<211>  5
<212>  PRT
<213>  Artificial sequence
```

```
<220>
<223>  sequence motif 3


<220>
<221>  VARIANT
<222>  (3)..(3)
<223>  /replace="Cys" /replace="Ser"

<220>
<221>  VARIANT
<222>  (4)..(4)
<223>  /replace="Lys"

<400>  346

Asn Phe Gln Arg Arg
1               5


<210>  347
<211>  5
<212>  PRT
<213>  Artificial sequence

<220>
<223>  sequence motif 4


<220>
<221>  VARIANT
<222>  (2)..(2)
<223>  /replace="Tyr"

<220>
<221>  VARIANT
<222>  (3)..(3)
<223>  /replace="Ser" /replace="Pro"

<220>
<221>  VARIANT
<222>  (4)..(4)
<223>  /replace="Ser" /replace="Phe"

<400>  347

Asn Phe Ala Asn Arg
1               5
```